# EUROPEAN PATENT SPECIFICATION

(11) **EP 0 607 334 B1**
(45) Date of publication and mention of the grant of the patent: **30.07.1997**
(21) Application number: 92922262.8
(22) Date of filing: 13.10.1992
(51) Int. Cl.: C07D 243/04, C07D 239/10, A61K 31/55, A61K 31/505, C07D 401/14, C07D 413/14, C07D 413/06, C07D 401/06

(54) **CYCLIC UREAS AND ANALOGUES USEFUL AS RETROVIRAL PROTEASE INHIBITIORS**
CYCLISCHE HARNSTOFFE UND ANALOGA VERWENDBAR ALS RETROVIRALE PROTEASEHEMMER
UREES CYCLIQUES ET ANALOGUES UTILES EN TANT QU'INHIBITEURS DE LA PROTEASE RETROVIRALE

(30) Priority: 11.10.1991 US 776491; 15.05.1992 US 883944; 29.09.1992 US 953272
(43) Date of publication of application: 27.07.1994
(60) Divisional application: 96118182.3
(73) Proprietor: THE DU PONT MERCK PHARMACEUTICAL COMPANY, Wilmington, Delaware 19898 (US)
(72) Inventor: LAM, Patrick, Yuk-Sun, Chadds Ford, PA 19317 (US); EYERMANN, Charles, Joseph, Wilmington, DE 19809 (US); HODGE, Carl, Nicholas, Wilmington, DE 19803 (US); JADHAV, Prabhakar, Kondaji, Wilmington, DE 19808 (US); DeLUCCA, George, Vincent, Wilmington, DE 19810 (US)
(74) Representative: von Kreisler, Alek, Dipl.-Chem.
(86) International application number: US9208749
(87) International publication number: WO9307128

(56) References cited:
- JOURNAL OF MEDICINAL CHEMISTRY vol. 33, no. 10, October 1990, WASHINGTON US pages 2687 - 2689 D.J. KEMPF ET AL. 'Strcture-Based, C2 Symmetric Inhibitors of HIV Protease'
- Chem.Abs.,Chem.Subst.Index,1987-1991, p.2815CS,2890CS,32617CS,78099CS
- Chim.Ther.,3 (1968),321-324
- Monats.Chem.,92(1961),79-87
- Tetrahedron Lett.,30(1989),1253-1256
- J.Org.Chem.,56(1991),365-372
- J.Org.Chem.,44(1979),1391-1397
- J.Org.Chem.,37(1972),208-215
- LiebigsAnn.Chem.,1985,275-300
- Chem.Pharm.Bull.,33(1985),340-346
- J.Am.Chem.Soc.,107(1985),1797-1798
- Tetrahedron Lett.,27(1987),3103-3106
- Tetrahedron Lett.,28(1987)5129-5132
- J.Org.Chem.,47(1982),1371-1373
- Science,263(1994),380-384

## Description

### FIELD OF THE INVENTION

This invention relates to substituted cyclic carbonyls and derivatives thereof useful as retroviral protease inhibitors, to pharmaceutical compositions comprising such compounds, and to methods of using these compounds for treating viral infection.

### BACKGROUND OF THE INVENTION

Current treatments for viral diseases usually involve administration of compounds that inhibit viral DNA synthesis. Current treatments for AIDS (Dagani, *Chem. Eng. News,* November 23, 1987 pp. 41-49) involve administration of compounds such as 2',3'-dideoxycytidine, trisodium phosphonoformate, ammonium 21-tungsto-9-antimoniate, 1-b-D-ribofuranoxyl-1,2,4-triazole-3-carboxamide, 3'-azido-3'-deoxythymidine (AZT), and adriamycin that inhibit viral DNA synthesis; compounds such as AL-721 and polymannoacetate which may prevent HIV from penetrating the host cell; and compounds which treat the opportunistic infections caused by the immunosupression resulting from HIV infection. None of the current AIDS treatments have proven to be totally effective in treating and/or reversing the disease. In addition, many of the compounds currently used to treat AIDS cause adverse side effects including low platelet count, renal toxicity, and bone marrow cytopenia.

Proteases are enzymes which cleave proteins at specific peptide bonds. Many biological functions are controlled or mediated by proteases and their complementary protease inhibitors. For example, the protease renin cleaves the peptide angiotensinogen to produce the peptide angiotensin I. Angiotensin I is further cleaved by the protease angiotensin converting enzyme (ACE) to form the hypotensive peptide angiotensin II. Inhibitors of renin and ACE are known to reduce high blood pressure *in vivo.* However, no therapeutically useful renin protease inhibitors have been developed, due to problems of oral availability and *in vivo* stability.

The genomes of retroviruses encode a protease that is responsible for the proteolytic processing of one or more polyprotein precursors such as the pol and gag gene products. See Wellink, *Arch. Virol.* 98 1 (1988). Retroviral proteases most commonly process the gag precursor into the core proteins, and also process the pol precursor into reverse transcriptase and retroviral protease.

The correct processing of the precursor polyproteins by the retroviral protease is necessary for the assembly of the infectious virions. It has been shown that *in vitro* mutagenesis that produces protease-defective virus leads to the production of immature core forms which lack infectivity. See Crawford, *J. Virol.* 53 899 (1985); Katoh et al., *Virology* 145 280 (1985). Therefore, retroviral protease inhibition provides an attractive target for antiviral therapy. See Mitsuya, *Nature* 325 775 (1987).

Moore, *Biochem. Biophys. Res. Commun.,* 159 420 (1989) discloses peptidyl inhibitors of HIV protease. WO 89/10752 discloses derivatives of peptides which are inhibitors of HIV protease.

U.S. Patent No. 4,652,552 discloses methyl ketone derivatives of tetrapeptides as inhibitors of viral proteases. U.S. Patent No. 4,644,055 discloses halomethyl derivatives of peptides as inhibitors of viral proteases. WO 87/07836 discloses L-glutamic acid gamma-monohydroxamate as an antiviral agent.

The ability to inhibit a viral protease provides a method for blocking viral replication and therefore a treatment for viral diseases, such as AIDS, that may have fewer side effects, be more efficacious, and be less prone to drug resistance when compared to current treatments.

The topic of the present invention is substituted cyclic carbonyls and derivatives thereof, which compounds are capable of inhibiting viral protease and which compounds are believed to serve as a means of combating viral diseases, such as AIDS. The substituted cyclic carbonyls and derivatives thereof of this invention provide significant improvements over protease inhibitors that are known in the art. A large number of compounds have been reported to be inhibitors of proteases, such as renin, but these have suffered from lack of adequate bioavailability and are thus not useful as therapeutic agents, particularly if oral administration is desired. This poor activity has been ascribed to the relatively high molecular weight of most protease inhibitors, to inadequate solubility properties, and to the presence of a number of peptide bonds, which are vulnerable to cleavage by mammalian proteases in vivo and which generally cause the molecules to be extensively bound in human serum. The substituted cyclic carbonyls and derivatives described herein have a distinct advantage in this regard, in that they do not contain peptide bonds, are of low molecular weight, and can be hydrophilic yet still inhibit the viral protease enzyme.

Additionally, known inhibitors of other non-HIV proteases do not inhibit HIV protease. The structure-activity requirements of such inhibitors differ from those of HIV protease inhibitors. The substituted cyclic carbonyls and derivatives of the invention are particularly useful as inhibitors of HIV protease and similar retroviral proteases.

Other HIV protease inhibitors have been reported, but to date none have been shown to be clinically effective. This lack of utility is due in part to the factors discussed above for renin inhibitors, particularly low bioavailability. The compounds of the invention offer a valuable solution to this problem in that they are of low molecular weight and many, therefore, have good oral absorption properties in mammals, ranging from 1-100% absolute oral availability.

### DETAILED DESCRIPTION OF THE INVENTION

There is provided by this invention a compound of the formula (I): 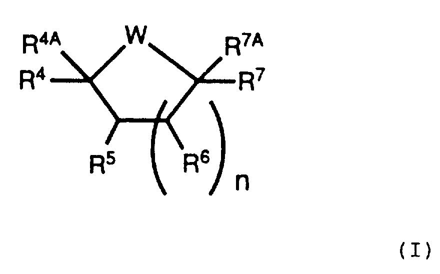 or a pharmaceutically acceptable salt or prodrug form thereof wherein:
R⁴ and R⁷ are independently selected from the following groups:
   hydrogen;
   C₁-C₈ alkyl substituted with 0-3 R¹¹;
   C₂-C₈ alkenyl substituted with 0-3 R¹¹;
   C₂-C₈ alkynyl substituted with 0-3 R¹¹;
   C₃-C₈ cycloalkyl substituted with 0-3 R¹¹;
   C₆-C₁₀ bicycloalkyl substituted with 0-3 R¹¹;
   aryl substituted with 0-3 R¹²;
   a C₆-C₁₄ carbocyclic residue substituted with 0-3 _{R}12_{;}
   a heterocyclic ring system substituted with 0-2 R¹², composed of 5 to 10 atoms including at least one nitrogen, oxygen or sulfur atom;
R^{4A} and R^{7A} are independently selected from the following groups:
   hydrogen;
   C₁-C₄ alkyl substituted with halogen or C₁-C₂ alkoxy;
   benzyl substituted with halogen or C₁-C₂ alkoxy;
R⁴ and R^{4A} can alternatively join to form a 5-7 membered carbocyclic ring substituted with 0-2 R¹²;
R⁷ and R^{7A} can alternatively join to form a 5-7 membered carbocyclic ring substituted with 0-2 R¹²;
n is 1;
R⁵ is selected from fluoro, difluoro, =O, C₁-C₃ alkyl or -OR²⁰;
R⁶ is selected from: hydrogen, =O, fluoro, difluoro, C₁-C₃ alkyl or -OR²¹;
R⁵ and R⁶ can alternatively join to form an epoxide ring; -OCH₂SCH₂O-; -OS(=O)O-; -OC(=O)O-; -OCH₂O-; -OC(=S)O-; -OC(=O)C(=O)O-; -OC(CH₃)₂O-; -OC(OCH₃) (CH₂CH₂CH₃)O-;
   or any hydrolysable group that, when administered to a mammalian subject, is metabolically cleaved to form the original diol wherein R⁵ and R⁶ are -OH
R²⁰ and R²¹ are independently selected from:
   hydrogen;
   C₁-C₆ alkyl substituted with 0-3 R¹¹;
   C₃-C₆ alkoxyalkyl substituted with 0-3 R¹¹;
   C₁-C₆ alkylcarbonyl substituted with 0-3 R¹¹;
   C₁-C₆ alkoxycarbonyl substituted with 0-3 R¹¹;
   benzoyl substituted with 0-3 R¹²;
   phenoxycarbonyl substituted with 0-3 R¹²;
   phenylaminocarbonyl substituted with 0-3 R¹²; or
   any group that, when administered to a mammalian subject, cleaves to form a free hydroxyl;
R¹¹ is selected from one or more of the following:
   keto, halogen, cyano, -CH₂NR¹³R¹⁴, -NR¹³R¹⁴, -CO₂R¹³, -OC(=O)R¹³, -OR¹³, C₂-C₆ alkoxyalkyl, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, C₁-C₄ alkyl, C₂-C₄ alkenyl, C₃-C₆ cycloalkyl, C₃-C₆ cycloalkylmethyl;
   1-3 amino acids, linked together via amide bonds and linked to R⁴ or R⁷ via the amine or carboxylate terminus;
   a C₅-C₁₄ carbocyclic residue substituted with 0-3 R¹²;
   aryl substituted with 0-3 R¹²; or
   a heterocyclic ring system substituted with 0-2 R¹², composed of 5 to 10 atoms including at least one nitrogen, oxygen or sulfur atom;
R¹², when a substituent on carbon, is selected from one or more of the following:
   phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -OCH₂CO₂H, 2-(1-morpholino)ethoxy; or
   a 5- or 6-membered heterocyclic ring containing from 1 to 4 heteroatoms selected from oxygen, nitrogen or sulfur;
   or R¹² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R¹² is attached to a saturated carbon atom, it may be carbonyl or thiocarbonyl;
R¹², when a substituent on nitrogen, is selected from one or more of the following:
   phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, -CO₂H, C₁-C₄ alkylcarbonyloxy, C₁-C₄ alkylcarbonyl;
R¹³ is H, phenyl, benzyl, C₁-C₆ alkyl, or C₃-C₆ alkoxyalkyl;
R¹⁴ is OH, H, C₁-C₄ alkyl, or benzyl;
R¹³ and R¹⁴ can alternatively join to form -(CH₂)₄-,
-(CH₂)₅-, -CH₂CH₂N(R¹⁵)CH₂CH₂-, or -CH₂CH₂OCH₂CH₂-;
R¹⁵ is H or CH₃;
m is 0, 1 or 2;
W is selected from:

   -N(R²²)C(=Z)N(R²³)-;

   -N(R²²)S(=O)N(R²³)-;
wherein:
Z is O, S, or NR²⁴;
R²² and R²³ are independently selected from the following:
   hydrogen;
   C₁-C₈ alkyl substituted with 0-3 R³¹;
   C₂-C₈ alkenyl substituted with 0-3 R³¹;
   C₂-C₈ alkynyl substituted with 0-3 R³¹;
   C₃-C₈ cycloalkyl substituted with 0-3 R³¹;
   C₆-C₁₀ bicycloalkyl substituted with 0-3 R³¹;
   aryl substituted with 0-3 R³²;
   a C₆-C₁₄ carbocyclic residue substituted with 0-3 R³²;
   a heterocyclic ring system substituted with 0-2 R³², composed of 5 to 10 atoms including at least one nitrogen, oxygen or sulfur atom;
R²⁴ is selected from: hydroxy; amino; C₁-C₄ alkyl; C₁-C₄ alkoxy; C₁-C₄ aminoalkyl; cyano; nitro; benzyloxy;
alternatively, R²² can join with R⁴ or R^{4A} to form a five- or six-membered fused heterocyclic, aromatic, or alicyclic ring substituted with 0-2 R¹²; and
alternatively, R²³ can join with R⁷ or R^{7A} to form a five- or six-membered fused heterocyclic, aromatic, or alicyclic ring substituted with 0-2 R¹²; and
alternatively, W can join with R⁵ or R⁶ to form a three-to seven-membered fused heterocyclic or carbocyclic ring substituted with 0-2 R¹²;
R³¹ is selected from one or more of the following:
   keto, halogen, cyano, -CH₂NR¹³R¹⁴, -NR¹³R¹⁴,
   -CO₂R¹³,
   -OC(=O)R¹³, -OR¹³, C₂-C₆ alkoxyalkyl, -S(O)ₘR¹³,
   -NHC(=NH)NHR¹³, -C(=NH)NHR¹³, -C(=O)NR¹³R¹⁴,
   -NR¹⁴C(=O)R¹³, =NOR¹⁴, -NR¹⁴C(=O)OR¹⁴,
   -OC(=O)NR¹³R¹⁴, -NR¹³C(=O)NR¹³R¹⁴, -NR¹⁴SO₂NR¹³R¹⁴,
   -NR¹⁴SO₂R¹³, -SO₂NR¹³R¹⁴, C₁-C₄ alkyl, C₂-C₄ alkenyl, C₃-C₆ cycloalkyl, C₃-C₆ cycloalkylmethyl;
   1-3 amino acids, linked together via amide bonds and linked to R⁴ or R⁷ via the amine or carboxylate terminus;
   a C₅-C₁₄ carbocyclic residue substituted with 0-3 R³²;
   aryl substituted with 0-3 R³²; or
   a heterocyclic ring system substituted with 0-2 R³², composed of 5 to 10 atoms including at least one nitrogen, oxygen or sulfur atom;
R³², when a substituent on carbon, is selected from one or more of the following:
   phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -OCH₂CO₂H, 2-(1-morpholino)ethoxy, -C(R¹⁴)=N(OR¹⁴); or
   a 5- or 6-membered heterocyclic ring containing from 1 to 4 heteroatoms selected from oxygen, nitrogen or sulfur;
   or R³² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R³² is attached to a saturated carbon atom, it may be carbonyl or thiocarbonyl;
R³², when a substituent on nitrogen, is selected from one or more of the following:
   phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, -CO₂H, C₁-C₄ alkylcarbonyloxy, C₁-C₄ alkylcarbonyl, -C(R¹⁴)=N(OR¹⁴);
provided that:
R⁴, R^{4A}, R⁷ and R^{7A} are not all hydrogen;
when R⁴ and R^{4A} are both hydrogen, at least one of the following is hydrogen: hydrogen: R²², R²³.

Preferred compounds of this invention are compounds of the formula (I): 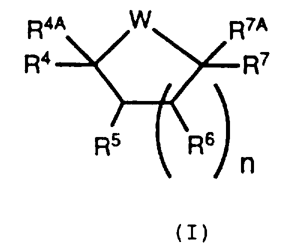 or a pharmaceutically acceptable salt or prodrug form thereof wherein:
R⁴ and R⁷ are independently selected from the following groups:
   hydrogen;
   C₁-C₄ alkyl substituted with 0-3 R¹¹;
   C₃-C₄ alkenyl substituted with 0-3 R¹¹;
   C₃-C₄ alkynyl substituted with 0-3 R¹¹;
R^{4A} and R^{7A} are hydrogen;
n is 1;
R⁵ is selected from fluoro, difluoro, =O, or -OR²⁰;
R⁶ is selected from: hydrogen, =O, fluoro, difluoro, or -OR²¹;
R⁵ and R⁶ can alternatively join to form an epoxide ring; -OCH₂SCH₂O-; -OS(=O)O-; -OC(=O)O-; -OCH₂O-; -OC(=S)O-; -OC(=O)C(=O)O-; -OC(CH₃)₂O-; -OC(OCH₃)(CH₂CH₂CH₃)O-;
   or any hydrolysable group that, when administered to a mammalian subject, is metabolically cleaved to form the original diol wherein R⁵ and R⁶ are -OH
R²⁰ and R²¹ are independently selected from:
   hydrogen;
   C₁-C₆ alkylcarbonyl;
   C₁-C₆ alkoxycarbonyl;
   benzoyl;
      or any hydrolysable group that, when administered to a mammalian subject, is metabolically cleaved to form the original diol wherein R⁵ and R⁶ are -OH
R¹¹ is selected from one or more of the following:
   keto, halogen, cyano, -CH₂NR¹³R¹⁴, -NR¹³R¹⁴,
   -CO₂R¹³,
   -OC(=O)R¹³, -OR¹³, C₂-C₄ alkoxyalkyl, -S(O)ₘR¹³, C₁-C₄ alkyl, C₂-C₄ alkenyl, C₃-C₆ cycloalkyl;
   a C₅-C₁₄ carbocyclic residue substituted with 0-3 R¹²;
   aryl substituted with 0-3 R¹²; or
   a heterocyclic ring system substituted with 0-2 R¹², composed of 5 to 10 atoms including at least one nitrogen, oxygen or sulfur atom;
R¹², when a substituent on carbon, is selected from one or more of the following:
   phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴; or
   a 5- or 6-membered heterocyclic ring containing from 1 to 4 heteroatoms selected from oxygen, nitrogen or sulfur;
   or R¹² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R¹² is attached to a saturated carbon atom, it may be carbonyl or thiocarbonyl;
R¹², when a substituent on nitrogen, is selected from one or more of the following:
   phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, C₁-C₄ alkylcarbonyloxy, C₁-C₄ alkylcarbonyl, -CO₂H;
R¹³ is H, C₁-C₆ alkyl, or C₃-C₆ alkoxyalkyl;
R¹⁴ is OH, H, C₁-C₄ alkyl, or benzyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
R¹⁵ is H or CH₃;
m is 0, 1 or 2;
W is selected from:

   -N(R²²)C(=Z)N(R²³)-;
wherein:
Z is O, S, N-CN, N-OH, N-OCH₃;
R²² and R²³ are independently selected from the following:
   hydrogen;
   C₁-C₈ alkyl substituted with 0-3 R³¹;
   C₃-C₈ alkenyl substituted with 0-3 R³¹;
   C₃-C₈ alkynyl substituted with 0-3 R³¹;
   C₃-C₆ cycloalkyl substituted with 0-3 R³¹;
R³¹ is selected from one or more of the following:
   keto, halogen, cyano, -CH₂NR¹³R¹⁴, -NR¹³R¹⁴, -CO₂R¹³, -OC(=O)R¹³ -OR¹³, C₂-C₄ alkoxyalkyl, -S(O)ₘR¹³, C₁-C₄ alkyl, C₂-C₄ alkenyl, C₃-C₆ cycloalkyl;
   a C₅-C₁₄ carbocyclic residue substituted with 0-3 _{R}12_{;}
   aryl substituted with 0-3 R³²; or
   a heterocyclic ring system substituted with 0-2 R³², composed of 5 to 10 atoms including at least one nitrogen, oxygen or sulfur atom;
R³², when a substituent on carbon, is selected from one or more of the following:
   phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -C(R¹⁴)=N(OR¹⁴); or
   a 5- or 6-membered heterocyclic ring containing from 1 to 4 heteroatoms selected from oxygen, nitrogen or sulfur;
   or R³² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or
   -NR¹³R¹⁴; or, when R³² is attached to a saturated carbon atom, it may be carbonyl or thiocarbonyl;
R³², when a substituent on nitrogen, is selected from one or more of the following:
   phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, C₁-C₄ alkylcarbonyloxy, C₁-C₄ alkylcarbonyl, -CO₂H, -C(R¹⁴)=N(OR¹⁴);
provided that:
R⁴, R^{4A}, R⁷, and R^{7A} are not all hydrogen;
when R⁴ and R^{4A} are hydrogen, at least one of the following is not hydrogen: R²², R²³.

Further preferred compounds of the invention of formula (I) are compounds of formula (II): 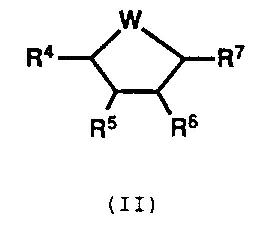
R⁴ and R⁷ are independently selected from the following groups:
   hydrogen;
   C₁-C₄ alkyl substituted with 0-3 R¹¹;
   C₃-C₄ alkenyl substituted with 0-3 R¹¹;
R⁵ is -OR²⁰;
R⁶ is hydrogen or -OR²¹;
R²⁰ and R²¹ are independently hydrogen or any hydrolysable group that, when administered to a mammalian subject, is metabolically cleaved to form the original diol wherein R⁵ and R⁶ are -OH
R¹¹ is selected from one or more of the following:
   keto, halogen, -CH₂NR¹³R¹⁴, -NR¹³R¹⁴, -OR¹³, C₂-C₄ alkoxyalkyl, C₁-C₄ alkyl, C₂-C₄ alkenyl, C₃-C₆ cycloalkyl;
   aryl substituted with 0-3 R¹²; or
   a heterocyclic ring system substituted with 0-2 R¹², composed of 5 to 10 atoms including at least one nitrogen, oxygen or sulfur atom;
R¹², when a substituent on carbon, is selected from one or more of the following:
   phenyl, benzyl, phenethyl, phenoxy, benzyloxy, halogen, C₁-C₄ alkyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³,
   C₁-C₄ alkyl substituted with -NR¹³R¹⁴, -N^{R13}R¹⁴, methylenedioxy, C₁-C₄ haloalkyl, C₁-C₄ alkylcarbonyl, C₁-C₄ alkylcarbonylamino, hydroxy, hydroxymethyl; or
   a 5- or 6-membered heterocyclic ring containing from 1 to 4 heteroatoms selected from oxygen, nitrogen or sulfur;
R¹², when a substituent on nitrogen, is selected from benzyl or methyl;
R¹³ is H, C₁-C₂ alkyl, or C₃-C₆ alkoxyalkyl;
R¹⁴ is OH, H or C₁-C₂ alkyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
W is selected from:

   -N(R²²)C(=Z)N(R²³)-;
wherein:
Z is O, S, or N-CN;
R²² and R²³ are independently selected from the following:
   hydrogen;
   C₁-C₄ alkyl substituted with 0-3 R³¹;
   C₃-C₄ alkenyl substituted with 0-3 R³¹;
R³¹ is selected from one or more of the following:
   keto, halogen, -CH₂NR¹³R¹⁴, -NR¹³R¹⁴, -OR¹³, C₂-C₄ alkoxyalkyl, C₁-C₄ alkyl, C₂-C₄ alkenyl, C₃-C₆ cycloalkyl;
   aryl substituted with 0-3 R³²; or
   a heterocyclic ring system substituted with 0-2 R³², composed of 5 to 10 atoms including at least one nitrogen, oxygen or sulfur atom;
R³², when a substituent on carbon, is selected from one or more of the following:
   phenyl, benzyl, phenethyl, phenoxy, benzyloxy, halogen, C₁-C₄ alkyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, methylenedioxy, C₁-C₄ haloalkyl, C₁-C₄ alkylcarbonyl, C₁-C₄ alkylcarbonylamino, hydroxy, hydroxymethyl, -C(R¹⁴)=N(OR¹⁴); or
   a 5- or 6-membered heterocyclic ring containing from 1 to 4 heteroatoms selected from oxygen, nitrogen or sulfur;
R³², when a substituent on nitrogen, is selected from benzyl or methyl;
provided that:
R⁴ and R⁷ are not both hydrogen;
when R⁴ is hydrogen, at least one of the following is not hydrogen: R²², R²³.

More preferred compounds of the present invention are compounds of the further preferred scope above, wherein:
R⁴ and R⁷ are independently selected from the following groups:
   hydrogen;
   C₁-C₃ alkyl substituted with 0-1 R¹¹;
R⁵ is -OR²⁰;
R⁶ is hydrogen or -OR²¹;
R²⁰ and R²¹ are independently hydrogen or any hydrolysable group that, when administered to a mammalian subject, is metabolically cleaved to form the original diol wherein R⁵ and R⁶ are -OH
R¹¹ is selected from one or more of the following:
   halogen, -OR¹³, C₁-C₄ alkyl, C₃-C₅ cycloalkyl;
   aryl substituted with 0-2 R¹²; or
   a heterocyclic ring system chosen from pyridyl, pyrimidinyl, triazinyl, furanyl, thienyl, pyrrolyl, pyrazolyl, imidazolyl, tetrazolyl, benzofuranyl, indolyl, quinolinyl, isoquinolinyl;
R¹², when a substituent on carbon, is selected from one or more of the following:
   benzyloxy, halogen, methyl, C₁-C₄ alkoxy, CF₃, 2-(1-morpholino)ethoxy, -CO₂H, hydroxamic acid, hydrazide, oxime, cyano, boronic acid, sulfonamide, formyl, C₃-C₆ cycloalkoxy, C₁-C₄ alkyl substituted with -NR¹³R¹⁴, -NR¹³R¹⁴, hydroxy, hydroxymethyl; or
R¹², when a substituent on nitrogen, is methyl;
R¹³ is H or methyl;
R¹⁴ is OH, H or methyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
W is -N(R²²)C(=O)N(R²³)- or -N(R²²)C(=N-CN)N(R²³)-;
R²² and R²³ are independently selected from the following:
   hydrogen;
   C₁-C₄ alkyl substituted with 0-1 R³¹;
   C₃-C₄ alkenyl substituted with 0-1 R³¹;
R³¹ is selected from one or more of the following:
   halogen, -OR¹³, C₁-C₄ alkyl, C₃-C₅ cycloalkyl;
   aryl substituted with 0-2 R³²; or
   a heterocyclic ring system chosen from pyridyl, pyrimidinyl, triazinyl, furanyl, thienyl, pyrrolyl, pyrazolyl, imidazolyl, tetrazolyl, benzofuranyl, indolyl, quinolinyl, isoquinolinyl;
R³², when a substituent on carbon, is selected from one or more of the following:
   benzyloxy, halogen, methyl, C₁-C₄ alkoxy, CF₃, 2-(1-morpholino)ethoxy, -CO₂H, hydroxamic acid, hydrazide, oxime, cyano, boronic acid, sulfonamide, formyl, C₃-C₆ cycloalkoxy, C₁-C₄ alkyl substituted with -NR¹³R¹⁴, -NR¹³R¹⁴, hydroxy, hydroxymethyl, -C(R¹⁴)=N(OR¹⁴); or
R³², when a substituent on nitrogen, is methyl;
provided that:
when R⁴ is hydrogen, R⁷ is not hydrogen;
when R⁴ is hydrogen, at least one of the following is not hydrogen: R²² and R²³.

Still more preferred compounds of the present invention are compounds of the further preferred scope above, wherein:
R⁴ and R⁷ are benzyl;
R⁵ is -OH;
R⁶ is hydrogen or -OH;
R¹³ is H or methyl;
R¹⁴ is H or methyl;
W is -N(R²²)C(=O)N(R²³)- or -N(R²²)C(=N-CN)N(R²³)-;
R²² and R²³ are independently selected from the following:
   hydrogen;
   C₁-C₄ alkyl substituted with 0-1 R³¹;
R³¹ is selected from one or more of the following:
   C₃-C₅ cycloalkyl;
   aryl substituted with 0-2 R³²; or
   a heterocyclic ring system chosen from pyridyl, thienyl, quinolinyl, or isoquinolinyl;
R³², when a substituent on carbon, is selected from one or more of the following:
   -CONH₂, -CO₂H, -CHO, -CH₂NHOH, -CH₂NR¹³R¹⁴, -NR¹³R¹⁴, hydroxy, hydroxymethyl, -C(R¹⁴)=N(OR¹⁴); or
R³², when a substituent on nitrogen, is methyl.

Also preferred are compounds of formula (IIa): 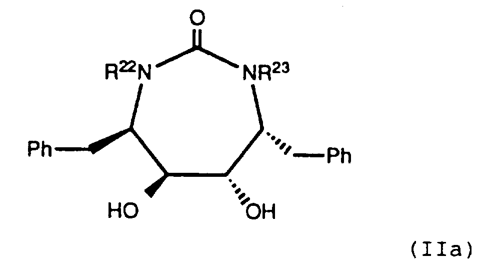 wherein R²² and R²³ are independently selected from the group consisting of:
hydrogen, allyl, propyl, cyclopropylmethyl, n-butyl, i-butyl, pyridylmethyl, methallyl, n-pentyl, i-pentyl, hexyl, benzyl, pyridylmethyl, isoprenyl, propargyl, picolinyl, methoxyethyl, cyclohexylmethyl, dimethyl-butyl, ethoxyethyl, methyl-oxazolinylmethyl, naphthylmethyl, methyloxazolinylmethyl, vinyloxyethyl, pentafluorobenzyl, quinolinylmethyl, carboxybenzyl, chloro-thienyl, picolinyl, benzyloxybenzyl, phenylbenzyl, adamantylethyl, cyclopropylmethoxybenzyl, ethoxybenzyl, hydroxybenzyl, hydroxymethylbenzyl, aminobenzyl, formylbenzyl, cyanobenzyl, cinnamyl, allyloxybenzyl, fluorobenzyl, cyclobutylmethyl, formaldoximebenzyl, cyclopentylmethyl, nitrobenzyl, nitrilobenzyl, carboxamidobenzyl, carbomethoxybenzyl, tetrazolylbenzyl, and dimethylallyl.

Specifically preferred are compounds of formula (IIa): 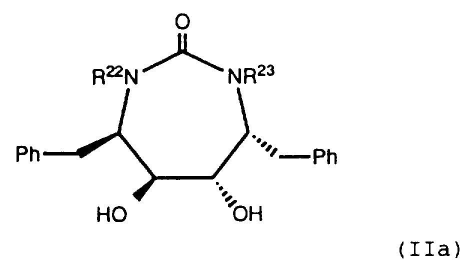 selected from the group consisting of:
the compound of formula (IIa) wherein R²² is allyl and R²³ is allyl;
the compound of formula (IIa) wherein R²² is propyl and R²³ is propyl;
the compound of formula (IIa) wherein R²² is cyclopropylmethyl and R²³ is cyclopropylmethyl;
the compound of formula (IIa) wherein R²² is n-butyl and R²³ is n-butyl;
the compound of formula (IIa) wherein R²² is i-pentyl and R²³ is i-pentyl;
the compound of formula (IIa) wherein R²² is 4-pyridylmethyl and R²³ is 4-pyridylmethyl;
the compound of formula (IIa) wherein R²² is 2-methallyl and R²³ is 2-methallyl;
the compound of formula (IIa) wherein R²² is n-pentyl and R²³ is n-pentyl;
the compound of formula (IIa) wherein R²² is i-butyl and R²³ is i-butyl;
the compound of formula (IIa) wherein R²² is benzyl and R²³ is benzyl;
the compound of formula (IIa) wherein R²² is 3-pyridylmethyl and R²³ is 3-pyridylmethyl;
the compound of formula (IIa) wherein R²² is allyl and R²³ is isoprenyl;
the compound of formula (IIa) wherein R²² is 3-propargyl and R²³ is 3-propargyl;
the compound of formula (IIa) wherein R²² is 2-picolinyl and R²³ is 2-picolinyl;
the compound of formula (IIa) wherein R²² is 2-methoxyethyl and R²³ is 2-methoxyethyl;
the compound of formula (IIa) wherein R²² is cyclohexylmethyl and R²³ is cyclohexylmethyl;
the compound of formula (IIa) wherein R²² is 3,3-dimethyl-1-butyl and R²³ is 3,3-dimethyl-1-butyl;
the compound of formula (IIa) wherein R²² is 2-ethoxyethyl and R²³ is 2-ethoxyethyl;
the compound of formula (IIa) wherein R²² is 3-methyl-5-oxazolinylmethyl and R²³ is hydrogen;
the compound of formula (IIa) wherein R²² is 1-naphthylmethyl and R²³ is 1-naphthylmethyl;
the compound of formula (IIa) wherein R²² is 3-methyloxazolinylmethyl and R²³ is 3-methyloxazolinylmethyl;
the compound of formula (IIa) wherein R²² is 2-vinyloxyethyl and R²³ is 2-vinyloxyethyl;
the compound of formula (IIa) wherein R²² is 2,3,4,5,6-pentafluorobenzyl and R²³ is 2,3,4,5,6-pentafluorobenzyl;
the compound of formula (IIa) wherein R²² is benzyl and R²³ is 2-quinolinylmethyl;
the compound of formula (IIa) wherein R²² is 4-carboxybenzyl and R²³ is 4-carboxybenzyl;
the compound of formula (IIa) wherein R²² is 5-chloro-2-thienyl and R²³ is 5-chloro-2-thienyl;
the compound of formula (IIa) wherein R²² is 2-quinolinylmethyl and R²³ is 2-quinolinylmethyl;
the compound of formula (IIa) wherein R²² is 2-propyl and R²³ is 2-picolinyl;
the compound of formula (IIa) wherein R²² is 3-benzyloxybenzyl and R²³ is 3-benzyloxybenzyl;
the compound of formula (IIa) wherein R²² is 4-phenylbenzyl and R²³ is phenylbenzyl;
the compound of formula (IIa) wherein R²² is 2-adamantylethyl and R²³ is 2-adamantylethyl;
the compound of formula (IIa) wherein R²² is hydrogen and R²³ is cyclopropylmethyl;
the compound of formula (IIa) wherein R²² is 2-picolinyl and R²³ is 2-naphthylmethyl;
the compound of formula (IIa) wherein R²² is 3-allyl and R²³ is hydrogen;
the compound of formula (IIa) wherein R²² is 3-allyl and R²³ is 2-picolinyl;
the compound of formula (IIa) wherein R²² is 3-allyl and R²³ is 4-picolinyl;
the compound of formula (IIa) wherein R²² is 3-benzyloxybenzyl and R²³ is 3-benzyloxybenzyl;
the compound of formula (IIa) wherein R²² is 3-cyclopropylmethoxybenzyl and R²³ is 3-cyclopropylmethoxybenzyl;
the compound of formula (IIa) wherein R²² is 3-ethoxybenzyl and R²³ is 3-ethoxybenzyl;
the compound of formula (IIa) wherein R²² is 4-benzyloxybenzyl and R²³ is 4-benzyloxybenzyl.
the compound of formula (IIa) wherein R²² is 3-hydroxybenzyl and R²³ is 3-hydroxybenzyl;
the compound of formula (IIa) wherein R²² is 4-hydroxybenzyl and R²³ is 4-hydroxybenzyl;
the compound of formula (IIa) wherein R²² is 3-hydroxymethylbenzyl and R²³ is 3-hydroxymethylbenzyl;
the compound of formula (IIa) wherein R²² is 4-hydroxymethylbenzyl and R²³ is 4-hydroxymethylbenzyl;
the compound of formula (IIa) wherein R²² is 3-aminobenzyl and R²³ is 3-aminobenzyl;
the compound of formula (IIa) wherein R²² is 3-carboxylbenzyl and R²³ is 3-carboxylbenzyl;
the compound of formula (IIa) wherein R²² is 3-formylbenzyl and R²³ is 3-formylbenzyl;
the compound of formula (IIa) wherein R²² is 3-cyanobenzyl and R²³ is 3-cyanobenzyl;
the compound of formula (IIa) wherein R²² is 2-naphthylmethyl and R²³ is 2-naphthylmethyl;
the compound of formula (IIa) wherein R²² is n-butyl and R²³ is benzyl;
the compound of formula (IIa) wherein R²² is allyl and R²³ is cyclopropylmethyl;
the compound of formula (IIa) wherein R²² is n-butyl and R²³ is cyclopropylmethyl
the compound of formula (IIa) wherein R²² is 3-methallyl and R²³ is benzyl;
the compound of formula (IIa) wherein R²² is benzyl and R²³ is ethyl;
the compound of formula (IIa) wherein R²² is benzyl and R²³ is 4-picolinyl;
the compound of formula (IIa) wherein R²² is cyclopropylmethyl and R²³ is 4-picolinyl;
the compound of formula (IIa) wherein R²² is benzyl and R²³ is cyclopentylmethyl;
the compound of formula (IIa) wherein R²² is cyclopropylmethyl and R²³ is cyclopentylmethyl;
the compound of formula (IIa) wherein R²² is benzyl and R²³ is n-propyl;
the compound of formula (IIa) wherein R²² is cyclopropylmethyl and R²³ is cinnamyl;
the compound of formula (IIa) wherein R²² is cyclopropylmethyl and R²³ is 2-naphthylmethyl;
the compound of formula (IIa) wherein R²² is cyclopentylmethyl and R²³ is 2-naphthylmethyl;
the compound of formula (IIa) wherein R²² is benzyl and R²³ is 2-naphthylmethyl;
the compound of formula (IIa) wherein R²² is cyclopropylmethyl and R²³ is 2-picolinyl;
the compound of formula (IIa) wherein R²² is 3-cyanobenzyl and R²³ is 3-cyanobenzyl;
the compound of formula (IIa) wherein R²² is 3-allyl and R²³ is 2-naphthylmethyl;
the compound of formula (IIa) wherein R²² is n-propyl and R²³ is 2-naphthylmethyl;
the compound of formula (IIa) wherein R²² is n-butyl and R²³ is 2-naphthylmethyl;
the compound of formula (IIa) wherein R²² is H and R²³ is 2-naphthylmethyl;
the compound of formula (IIa) wherein R²² is 4-picolinyl and R²³ is 2-naphthylmethyl;
the compound of formula (IIa) wherein R²² is 3-allyl and R²³ is cyclopentylmethyl;
the compound of formula (IIa) wherein R²² is 3-allyl and R²³ is 2-quinolinylmethyl;
the compound of formula (IIa) wherein R²² is 3-picolinyl and R²³ is cyclopropylmethyl;
the compound of formula (IIa) wherein R²² is 3-picolinyl and R²³ is 2-naphthylmethyl;
the compound of formula (IIa) wherein R²² is 3-allyloxybenzyl and R²³ is 3-allyloxybenzyl;
the compound of formula (IIa) wherein R²² is 3-allyloxybenzyl and R²³ is 3-hydroxybenzyl;
the compound of formula (IIa) wherein R²² is 3-picolinyl and R²³ is 3-picolinyl;
the compound of formula (IIa) wherein R²² is 2-naphthylmethyl and R²³ is 4-fluorobenzyl;
the compound of formula (IIa) wherein R²² is 3-carbomethoxybenzyl and R²³ is 3-carbomethyoxybenzyl;
the compound of formula (IIa) wherein R²² is 4-formylbenzyl and R²³ is 4-formylbenzyl;
the compound of formula (IIa) wherein R²² is 4-cyanobenzyl and R²³ is 4-cyanobenzyl;
the compound of formula (IIa) wherein R²² is 4-hydroxybenzyl and R²³ is n-propyl;
the compound of formula (IIa) wherein R²² is 3-hydroxybenzyl and R²³ is n-propyl;
the compound of formula (IIa) wherein R²² is 3-carboxybenzyl and R²³ is 3-carboxybenzyl;
the compound of formula (IIa) wherein R²² is cyclobutylmethyl and R²³ is cyclobutylmethyl;
the compound of formula (IIa) wherein R²² is cyclopentylmethyl and R²³ is cyclopentylmethyl;
the compound of formula (IIa) wherein R²² is n-butyl and R²³ is 3-methallyl;
the compound of formula (IIa) wherein R²² is n-butyl and R²³ is cyclopentylmethyl;
the compound of formula (IIa) wherein R²² is 3-formaldoximebenzyl and R²³ is 3-formaldoximebenzyl;
the compound of formula (IIa) wherein R²² is cyclopropylmethyl and R²³ is 3-hydroxybenzyl;
the compound of formula (IIa) wherein R²² is cyclopropylmethyl and R²³ is 4-hydroxybenzyl;
the compound of formula (IIa) wherein R²² is 3-(N-methylamino)benzyl and R²³ is 3-(N-methylamino)benzyl;
the compound of formula (IIa) wherein R²² is 3-acetylbenzyl and R²³ is 3-acetylbenzyl;
the compound of formula (IIa) wherein R²² is 3-hydroxylaminobenzyl and R²³ is 3-hydroxylaminobenzyl;
the compound of formula (IIa) wherein R²² is 2-naphthylmethyl and R²³ is 3-hydroxybenzyl;
the compound of formula (IIa) wherein R²² is 4-hydroxymethylbenzyl and R²³ is 3-hydroxybenzyl;
the compound of formula (IIa) wherein R²² is N-methyl-(3-amido)benzyl and R²³ is N-methyl-(3-amido)benzyl;
the compound of formula (IIa) wherein R²² is N-methyl-(3-amido)benzyl and R²³ is 3-(amidino)benzyl;
the compound of formula (IIa) wherein R²² is 3-(5-tetrazolyl)benzyl and R²³ is cyclopropylmethyl;
the compound of formula (IIa) wherein R²² is 3-(5-tetrazolyl)benzyl and R²³ is 3-(5-tetrazolyl)benzyl;
the compound of formula (IIa) wherein R²² is phenylmethyl-3-boronic acid and R²³ is phenylmethyl-3-boronic acid.

More specifically preferred are the following compounds of formula (I), which have IC₅₀ or Ki values of less than about 10 nM for inhibiting HIV protease:
the compound of formula (IIa) wherein R²² is allyl and R²³ is allyl;
the compound of formula (IIa) wherein R²² is propyl and R²³ is propyl;
the compound of formula (IIa) wherein R²² is cyclopropylmethyl and R²³ is cyclopropylmethyl;
the compound of formula (IIa) wherein R²² is n-butyl and R²³ is n-butyl;
the compound of formula (IIa) wherein R²² is i-pentyl and R²³ is i-pentyl;
the compound of formula (IIa) wherein R²² is 2-methallyl and R²³ is 2-methallyl;
the compound of formula (IIa) wherein R²² is n-pentyl and R²³ is n-pentyl;
the compound of formula (IIa) wherein R²² is benzyl and R²³ is benzyl;
the compound of formula (IIa) wherein R²² is allyl and R²³ is isoprenyl;
the compound of formula (IIa) wherein R²² is 3-hydroxybenzyl and R²³ is 3-hydroxybenzyl;
the compound of formula (IIa) wherein R²² is 4-hydroxybenzyl and R²³ is 4-hydroxybenzyl;
the compound of formula (IIa) wherein R²² is 3-hydroxymethylbenzyl and R²³ is 3-hydroxymethylbenzyl;
the compound of formula (IIa) wherein R²² is 4-hydroxymethylbenzyl and R²³ is 4-hydroxymethylbenzyl;
the compound of formula (IIa) wherein R²² is 3-aminobenzyl and R²³ is 3-aminobenzyl;
the compound of formula (IIa) wherein R²² is 3-carboxylbenzyl and R²³ is 3-carboxylbenzyl;
the compound of formula (IIa) wherein R²² is 3-formylbenzyl and R²³ is 3-formylbenzyl;
the compound of formula (IIa) wherein R²² is 3-cyanobenzyl and R²³ is 3-cyanobenzyl;
the compound of formula (IIa) wherein R²² is 2-naphthylmethyl and R²³ is 2-naphthylmethyl;
the compound of formula (IIa) wherein R²² is n-butyl and R²³ is benzyl;
the compound of formula (IIa) wherein R²² is allyl and R²³ is cyclopropylmethyl;
the compound of formula (IIa) wherein R²² is n-butyl and R²³ is cyclopropylmethyl
the compound of formula (IIa) wherein R²² is 3-methallyl and R²³ is benzyl;
the compound of formula (IIa) wherein R²² is benzyl and R²³ is ethyl;
the compound of formula (IIa) wherein R²² is benzyl and R²³ is 4-picolinyl;
the compound of formula (IIa) wherein R²² is cyclopropylmethyl and R²³ is 4-picolinyl;
the compound of formula (IIa) wherein R²² is benzyl and R²³ is cyclopentylmethyl;
the compound of formula (IIa) wherein R²² is cyclopropylmethyl and R²³ is cyclopentylmethyl;
the compound of formula (IIa) wherein R²² is benzyl and R²³ is n-propyl;
the compound of formula (IIa) wherein R²² is cyclopropylmethyl and R²³ is cinnamyl;
the compound of formula (IIa) wherein R²² is cyclopropylmethyl and R²³ is 2-naphthylmethyl;
the compound of formula (IIa) wherein R²² is cyclopentylmethyl and R²³ is 2-naphthylmethyl;
the compound of formula (IIa) wherein R²² is benzyl and R²³ is 2-naphthylmethyl;
the compound of formula (IIa) wherein R²² is cyclopropylmethyl and R²³ is 2-picolinyl;
the compound of formula (IIa) wherein R²² is 3-cyanobenzyl and R²³ is 3-cyanobenzyl;
the compound of formula (IIa) wherein R²² is 3-allyl and R²³ is 2-naphthylmethyl;
the compound of formula (IIa) wherein R²² is n-propyl and R²³ is 2-naphthylmethyl;
the compound of formula (IIa) wherein R²² is n-butyl and R²³ is 2-naphthylmethyl;
the compound of formula (IIa) wherein R²² is H and R²³ is 2-naphthylmethyl;
the compound of formula (IIa) wherein R²² is 4-picolinyl and R²³ is 2-naphthylmethyl;
the compound of formula (IIa) wherein R²² is 3-allyl and R²³ is cyclopentylmethyl;
the compound of formula (IIa) wherein R²² is 3-allyl and R²³ is 2-quinolinylmethyl;
the compound of formula (IIa) wherein R²² is 3-picolinyl and R²³ is cyclopropylmethyl;
the compound of formula (IIa) wherein R²² is 3-picolinyl and R²³ is 2-naphthylmethyl;
the compound of formula (IIa) wherein R²² is 3-allyloxybenzyl and R²³ is 3-allyloxybenzyl;
the compound of formula (IIa) wherein R²² is 3-allyloxybenzyl and R²³ is 3-hydroxybenzyl;
the compound of formula (IIa) wherein R²² is 3-picolinyl and R²³ is 3-picolinyl;
the compound of formula (IIa) wherein R²² is 2-naphthylmethyl and R²³ is 4-fluorobenzyl;
the compound of formula (IIa) wherein R²² is 3-carbomethoxybenzyl and R²³ is 3-carbomethyoxybenzyl;
the compound of formula (IIa) wherein R²² is 4-formylbenzyl and R²³ is 4-formylbenzyl;
the compound of formula (IIa) wherein R²² is 4-cyanobenzyl and R²³ is 4-cyanobenzyl;
the compound of formula (IIa) wherein R²² is 4-hydroxybenzyl and R²³ is n-propyl;
the compound of formula (IIa) wherein R²² is 3-hydroxybenzyl and R²³ is n-propyl;
the compound of formula (IIa) wherein R²² is 3-carboxybenzyl and R²³ is 3-carboxybenzyl;
the compound of formula (IIa) wherein R²² is cyclobutylmethyl and R²³ is cyclobutylmethyl;
the compound of formula (IIa) wherein R²² is cyclopentylmethyl and R²³ is cyclopentylmethyl;
the compound of formula (IIa) wherein R²² is n-butyl and R²³ is 3-methallyl;
the compound of formula (IIa) wherein R²² is n-butyl and R²³ is cyclopentylmethyl;
the compound of formula (IIa) wherein R²² is 3-formaldoximebenzyl and R²³ is 3-formaldoximebenzyl;
the compound of formula (IIa) wherein R²² is cyclopropylmethyl and R²³ is 3-hydroxybenzyl;
the compound of formula (IIa) wherein R²² is cyclopropylmethyl and R²³ is 4-hydroxybenzyl;
the compound of formula (IIa) wherein R²² is 3-(N-methylamino)benzyl and R²³ is 3-(N-methylamino)benzyl;
the compound of formula (IIa) wherein R²² is 3-acetylbenzyl and R²³ is 3-acetylbenzyl;
the compound of formula (IIa) wherein R²² is 3-hydroxylaminobenzyl and R²³ is 3-hydroxylaminobenzyl;
the compound of formula (IIa) wherein R²² is 2-naphthylmethyl and R²³ is 3-hydroxybenzyl;
the compound of formula (IIa) wherein R²² is 4-hydroxymethylbenzyl and R²³ is 3-hydroxybenzyl;
the compound of formula (IIa) wherein R²² is N-methyl-(3-amido)benzyl and R²³ is N-methyl-(3-amido)benzyl;
the compound of formula (IIa) wherein R²² is N-methyl-(3-amido)benzyl and R²³ is 3-(amidino)benzyl;
the compound of formula (IIa) wherein R²² is 3-(5-tetrazolyl)benzyl and R²³ is cyclopropylmethyl;
the compound of formula (IIa) wherein R²² is 3-(5-tetrazolyl)benzyl and R²³ is 3-(5-tetrazolyl)benzyl;
the compound of formula (IIa) wherein R²² is phenylmethyl-3-boronic acid and R²³ is phenylmethyl-3-boronic acid.

The following preferred compounds of formula (I) exhibit IC₉₀ values of less than about 10 mg/mL for the inhibition of HIV growth:
the compound of formula (IIa) wherein R²² is allyl and R²³ is allyl;
the compound of formula (IIa) wherein R²² is cyclopropylmethyl and R²³ is cyclopropylmethyl;
the compound of formula (IIa) wherein R²² is n-butyl and R²³ is n-butyl;
the compound of formula (IIa) wherein R²² is propyl and R²³ is propyl;
the compound of formula (IIa) wherein R²² is i-pentyl and R²³ is i-pentyl;
the compound of formula (IIa) wherein R²² is benzyl and R²³ is benzyl;
the compound of formula (IIa) wherein R²² is 3-hydroxybenzyl and R²³ is 3-hydroxybenzyl;
the compound of formula (IIa) wherein R²² is 4-hydroxybenzyl and R²³ is 4-hydroxybenzyl;
the compound of formula (IIa) wherein R²² is 3-hydroxymethylbenzyl and R²³ is 3-hydroxymethylbenzyl;
the compound of formula (IIa) wherein R²² is 4-hydroxymethylbenzyl and R²³ is 4-hydroxymethylbenzyl;
the compound of formula (IIa) wherein R²² is 3-aminobenzyl and R²³ is 3-aminobenzyl;
the compound of formula (IIa) wherein R²² is 3-carboxylbenzyl and R²³ is 3-carboxylbenzyl;
the compound of formula (IIa) wherein R²² is 3-formylbenzyl and R²³ is 3-formylbenzyl
the compound of formula (IIa) wherein R²² is 3-formaldoximebenzyl and R²³ is 3-formaldoximebenzyl;
the compound of formula (IIa) wherein R²² is 3-(N-methylamino)benzyl and R²³ is 3-(N-methylamino)benzyl;
the compound of formula (IIa) wherein R²² is 3-acetylbenzyl and R²³ is 3-acetylbenzyl;
the compound of formula (IIa) wherein R²² is 3-hydroxylaminobenzyl and R²³ is 3-hydroxylaminobenzyl;
the compound of formula (IIa) wherein R²² is 2-naphthylmethyl and R²³ is 3-hydroxybenzyl;
the compound of formula (IIa) wherein R²² is 4-hydroxymethylbenzyl and R²³ is 3-hydroxybenzyl;
the compound of formula (IIa) wherein R²² is N-methyl-(3-amido)benzyl and R²³ is N-methyl-(3-amido)benzyl;
the compound of formula (IIa) wherein R²² is N-methyl-(3-amido)benzyl and R²³ is 3-(amidino)benzyl;
the compound of formula (IIa) wherein R²² is 3-(5-tetrazoyl)benzyl and R²³ is cyclopropylmethyl;
the compound of formula (IIa) wherein R²² is 3-(5-tetrazolyl)benzyl and R²³ is 3-(5-tetrazolyl)benzyl;
the compound of formula (IIa) wherein R²² is phenylmethyl-3-boronic acid and R²³ is phenylmethyl-3-boronic acid.

Also preferred in the present invention are compounds of formula (Ib): 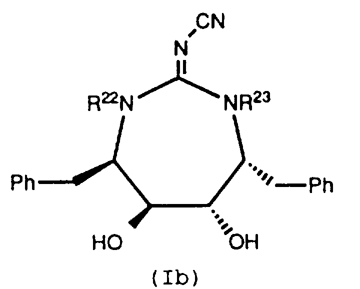 or a pharmaceutically acceptable salt or prodrug form thereof wherein:
R²² and R²³ are independently selected from the group consisting of: hydrogen, cyclopropylmethyl, CH₂(C₆H₄)-p-OCH₂C₆H₅, CH₂(C₆H₄)-p-OH, cyclopentylmethyl, allyl, n-butyl, beta-napthylmethyl, benzyl, CH₂(C₆H₄)-m-OCH₂C₆H₅, p-nitrobenzyl, m-nitrobenzyl, CH₂(C₆H₄)-m-OH, p-aminobenzyl, m-aminobenzyl, p-nitrilobenzyl, m-nitrilobenzyl, dimethylallyl, cyclohexylmethyl, cyclobutylmethyl, propyl, 3-methyl-1-butyl, carboxamidobenzyl, and formaldoximebenzyl.

More specifically preferred are the following compounds of formula (I), which have IC₅₀ or Ki values of less than about 10 nM for inhibiting HIV protease:
the compound of formula (Ib) wherein R²² is 3-formaldoximebenzyl and R²³ is 3-formaldoximebenzyl.

In the present invention it has been discovered that the compounds above are useful as inhibitors of HIV protease and similar retroviral proteases, and for the treatment of HIV infection and similar retrovirus infections.

The present invention also provides methods for the treatment of HIV infection by administering to a host infected with HIV a pharmaceutically effective amount of a compound of formula (I) as described above.

The compounds herein described may have asymmetric centers. All chiral, diastereomeric, and racemic forms are included in the present invention. Many geometric isomers of olefins, C=N double bonds, and the like can also be present in the compounds described herein, and all such stable isomers are contemplated in the present invention. 3

When any variable (for example, R¹ through R²³, R^{4A} and R^{7A}, m, n, W, Z, etc.) occurs more than one time in any constituent or in formula (I) or (II), or any other formula herein, its definition on each occurrence is independent of its definition at every other occurrence. Also, combinations of substituents and/or variables are permissible only if such combinations result in stable compounds.

As used herein, "alkyl" is intended to include both branched and straight-chain saturated aliphatic hydrocarbon groups having the specified number of carbon atoms; "alkoxy" represents an alkyl group of indicated number of carbon atoms attached through an oxygen bridge; "cycloalkyl" is intended to include saturated ring groups, such as cyclopropyl, cyclobutyl, cyclopentyl, cyclohexyl, cycloheptyl and cyclooctyl; and "biycloalkyl" is intended to include saturated bicyclic ring groups such as [3.3.0]bicyclooctane, [4.3.0]bicyclononane, [4.4.0]bicyclodecane (decalin), [2.2.2]bicyclooctane, and so forth. "Alkenyl" is intended to include hydrocarbon chains of either a straight or branched configuration and one or more unsaturated carbon-carbon bonds which may occur in any stable point along the chain, such as ethenyl, propenyl, and the like; and "alkynyl" is intended to include hydrocarbon chains of either a straight or branched configuration and one or more triple carbon-carbon bonds which may occur in any stable point along the chain, such as ethynyl, propynyl and the like. "Halo" as used herein refers to fluoro, chloro, bromo, and iodo; and "counterion" is used to represent a small, negatively charged species such as chloride, bromide, hydroxide, acetate, sulfate, and the like.

As used herein, "aryl" or "aromatic residue" is intended to mean phenyl or naphthyl; "carbocyclic" is intended to mean any stable 5- to 7- membered monocyclic or bicyclic or 7- to 14-membered bicyclic or tricyclic carbon ring, any of which may be saturated, partially unsaturated, or aromatic, for example, indanyl or tetrahydronaphthyl (tetralin).

As used herein, the term "heterocycle" is intended to mean a stable 5- to 7- membered monocyclic or bicyclic or 7- to 10-membered bicyclic heterocyclic ring which is either saturated or unsaturated, and which consists of carbon atoms and from 1 to 3 heteroatoms selected from the group consisting of N, O and S and wherein the nitrogen and sulfur heteroatoms may optionally be oxidized, and the nitrogen may optionally be quaternized, and including any bicyclic group in which any of the above-defined heterocyclic rings is fused to a benzene ring. The heterocyclic ring may be attached to its pendant group at any heteroatom or carbon atom which results in a stable structure. The heterocyclic rings described herein may be substituted on carbon or on a nitrogen atom if the resulting compound is stable. Examples of such heterocycles include, but are not limited to, pyridyl, pyrimidinyl, furanyl, thienyl, pyrrolyl, pyrazolyl, imidazolyl, tetrazolyl, benzofuranyl, benzothiophenyl, indolyl, indolenyl, quinolinyl, isoquinolinyl or benzimidazolyl, piperidinyl, 4-piperidonyl, pyrrolidinyl, 2-pyrrolidonyl, pyrrolinyl, tetrahydrofuranyl, tetrahydroquinolinyl, tetrahydroisoquinolinyl, decahydroquinolinyl or octahydroisoquinolinyl. The term "substituted", as used herein, means that an one or more hydrogen on the designated atom is replaced with a selection from the indicated group, provided that the designated atom's normal valency is not exceeded, and that the substitution results in a stable compound.

By "stable compound" or "stable structure" is meant herein a compound that is sufficiently robust to survive isolation to a useful degree of purity from a reaction mixture, and formulation into an efficacious therapeutic agent.

As used herein, "pharmaceutically acceptable salts and prodrugs" refer to derivatives of the disclosed compounds that are modified by making acid or base salts, or by modifying functional groups present in the compounds in such a way that the modifications are cleaved, either in routine manipulation or in vivo, to the parent compounds. Examples include, but are not limited to, mineral or organic acid salts of basic residues such as amines; alkali or organic salts of acidic residues such as carboxylic acids; acetate, formate and benzoate derivatives of alcohols and amines; and the like.

Pharmaceutically acceptable salts of the compounds of the invention can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418.

### Synthesis

The compounds of the present invention may be synthesized using the general synthetic procedures described below.

Compounds of the invention wherein:
W is -N(R²²)C(=Z)N(R²³)-
R⁵ is -OR²⁰ or H;
R⁶ is -OR²¹ or H; and
n is 1; can be formed from diamines of formula (III): 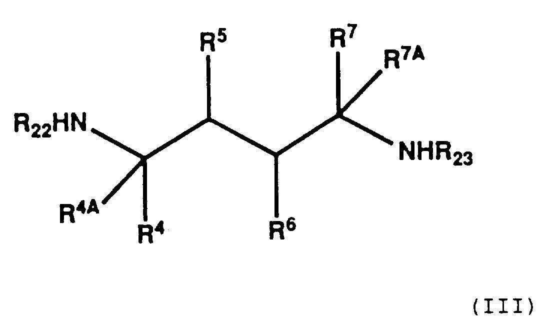
The diamines of formula (III) can be synthesized as described in WO 91/18866. Alternative methods which can be used to synthesize the compounds of structure (III) above are described in European Patent Application Publication Number 402646A1, U.S. Patent 4,837,204, and Canadian Patent Application 2,026,832 .

The compounds of formula (III) can be cyclized to form compounds of formula (IV) under conditions normally used to form cyclic ureas, as is known to one skilled in the art. Reagents J-(C=Z)-J', where J and J' are leaving groups, are employed, preferably under relatively dilute conditions (for example, less than about 0.1 M), to effect ring closure to provide compounds of formula (I). Many examples of J and J' are known; preferred are carbonyl diimidazole, thiocarbonyldiimidazole, phosgene, thiophosgene, diphenyl carbonate, or diphenyl thiocarbonate.

For compounds of the invention where R²⁰ or R²¹ is -OH, it is advantageous to protect the free hydroxyl before cyclization. Protecting groups used can include any of those listed in Greene, Protective Groups in Organic Synthesis, Chapter 2, Wiley, NY (1981). The preferred protecting groups are trimethylsilylethoxymethyl (SEM), methoxyethoxymethyl (MEM), or methoxymethyl (MOM).

Cyclization of compounds of formula (III) results in structure (IV).

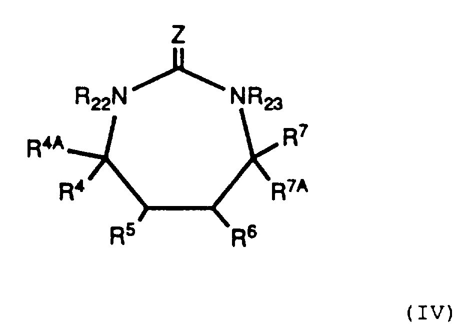

Another, preferred method to form compounds of formula (IV), in cases wherein R²² and R²³ are linked to their respective nitrogens by a CH2 residue, is to cyclize a compound of structure (III) where R²² and R²³ are hydrogen, and to alkylate the nitrogens using a base, a phase transfer catalyst, and an alkylating agent, using methods well known in the art. The preferred base is sodium hydride, and the preferred alkylating agents are R²²Y and R²³Y, wherein Y is a halogen, triflate, or mesylate, preferably a bromide or iodide. Preferred conditions are in polar aprotic solvents between 0 and 100 °C.

Cleavage of protecting groups, if employed, yields structures of formula (I) wherein R⁵ and R⁶ are hydroxyl.

When R⁵ and R⁶ are other than OR²⁰ and OR²¹, some chemical manipulation of functional groups may need to be performed in the preparation of the compounds of formula (III) or (IV), as is appreciated by one of skill in the art of organic synthesis. Described below are examples of such procedures.

Methods for obtaining compounds wherein R⁵ is OH and R⁶ is OR²¹ include protection of nitrogen, if necessary, followed by reaction of the diol with one equivalent of base and one equivalent of acyl halide, alkyl halide, alkoxyalkyl halide, alkoxycarbonyl halide, benzoyl halide, diphenyl carbonate or phenylisocyanate, and purification by column chromatography of the unwanted bis-alkylated and unreacted material.

Methods for obtaining compounds wherein R⁵ is OH and R⁶ is H include protection of nitrogen, if necessary, and reduction of the diol to the monool using techniques known in the art (see, for example, *Chem. Comm.* 1971, 1097; *J. Org. Chem.* 1969, 3923). The preferred method is formation of cyclic diol ester and reduction using hydride. Deprotection of nitrogen, if necessary, results in the desired compound.

Methods for obtaining compounds wherein R⁵ is OH and R⁶ is F include protection of nitrogen, if necessary, followed by formation of mono-protected diol as described above. Reaction with a fluorinating agent, preferably diethylaminosulfurtrifluoride (DAST) (Reagents for Organic Synthesis, Vol. 13, p. 110, Wiley Interscience, NY, 1988), provides the alkyl fluoride. Deprotection of nitrogen, if necessary, and hydroxyl results in the desired compound.

Methods for obtaining compounds wherein R⁵ is OH and R⁶ is =O include protection of nitrogen, if necessary, and standard conditions for oxidizing glycols to pinacols. The preferred oxidant is one equivalent of pyridinium dichromate in dichloromethane, or one equivalent of NaOCl in HOAc. Deprotection of nitrogen, if necessary, results in the desired compound. Alternatively, a monohydroxy compound described above can be oxidized to the ketone under standard conditions, preferably Swern oxidation using oxalyl chloride, DMSO and Et₃N, followed by alpha-hydroxylation of the ketone (see *Tet. Lett.* 1981, 607; *Tet. Lett.* 1982, 2917).

Methods for obtaining compounds wherein R⁵ is OH and R⁶ is difluoro include protection of nitrogen, if necessary, and hydroxyl of the above obtained pinacol, followed by reaction of the carbonyl with a fluorinating reagent, such as DAST. Deprotection of hydroxyl and nitrogen, if necessary, results in the desired compound.

Methods for obtaining compounds wherein R⁵ and R⁶ join to form an epoxide include protection of nitrogen, if necessary, followed by standard conditions for the formation of an epoxide from a glycol (see, for example, *J. Org. Chem.* 1981, 3361). Preferred is the reaction of the glycol with more than 2 equivalents of base and one equivalent of an activating group, such as methanesulfonyl chloride. Deprotection if necessary results in the desired compound.

Methods for obtaining compounds wherein R⁵ is OH and R⁶ is C₁-C₃ alkyl include protection of nitrogen, if necessary, and reaction of the epoxide prepared above with C₁-C₃ alkylmetal reagents. Preferred is the reaction of lithium dialkyl cuprates in aprotic solvents at low temperatures (-78 to -40 °C) (see Carruthers, Some Modern Methods in Organic Synthesis, p. 64, Cambridge University Press, 1978).

With a judicious selection of reagents, as is well appreciated to one skilled in the art, these manipulations can be performed in a straightforward manner to yield the claimed combinations of R⁵ and R⁶.

Protection, if necessary, cyclization, and functional group manipulation if desired is performed as described above to obtain compounds of structure (VI):

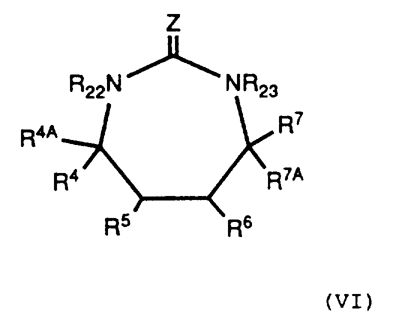

Functional group manipulation, if desired, may be performed as described above, followed by cyclization using standard conditions, preferably phosgene or thiophosgene in the presence of 2 equivalents of a base such as potassium hydride, to obtain compounds of structure (I).

Compounds of structure (I) described above wherein Z = O can be converted to the thio derivatives, Z = S, using standard conditions (March, Advanced Organic Chemistry, p. 792, Wiley, NY, 1985); preferred is the use of the disulfide described in Bull. Soc. Chim. Belges 1978, 223.

Structures (I) described above wherein Z = O can be converted to the imino derivatives, Z = NR²⁴, using standard conditions. When R²⁴ is OH or O-alkyl, the oximes can be formed and alkylated if desired as described in March, Advanced Organic Chemistry, pp. 359, 805, Wiley, NY, 1985. The hydrazones and imines can be formed similarly (ibid, pp. 533, 797).

It is expected that the compounds of the invention can also be prepared as shown in Scheme 1 (shown below). The intra-molecular coupling of the N-substituted or unsubstituted dialdehydes may be achieved by organometal reagents derived from vanadium, titanium, samarium etc. The dialdehyde precursors can be prepared from the commercially available materials by the methods known to those skilled in the art of organic synthesis, preferably by the techniques disclosed in WO 92/14696.

Compounds wherein W is -N(R²²)C(=O)N(R²³)- and n is 1 can likewise be synthesized as shown in Schemes 3, 4, 6, 7 (below). If necessary, intermediates described herein can be manipulated by methods known to those skilled in the art of organic synthesis to provide compounds within the scope of the invention.

Compounds wherein W is -N(R²²)C(=N-OR)N(R²³)- or -N(R²²)C(=S)N(R²³)- and n is 1 can be synthesized as shown in Scheme 5 (below). If necessary, intermediates described herein can be manipulated by methods known to those skilled in the art of organic synthesis to provide compounds within the scope of the invention.

The synthesis of compounds of the invention is described in further detail below.

It is to be noted that some of the following embodiments, e.g. examples, do not fall within the scope of the claims and are therefore not to be considered as being part of the invention, but are merely present for illustrative purposes.

### Procedure 1

Preparation of di-N-CBZ protected 1,4-diamino-2,3-diols (XIX):

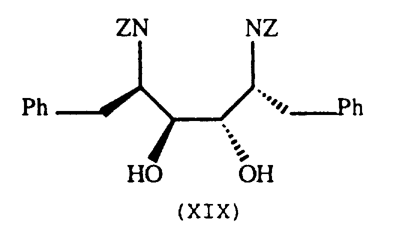

Detailed experimental procedures for the synthesis of compound (XIX) are described in WO 91/18866.

### Procedure 2

Preparation of di-O protected di-N-CBZ 1,4-diamino diols (XXa) and (XXb):

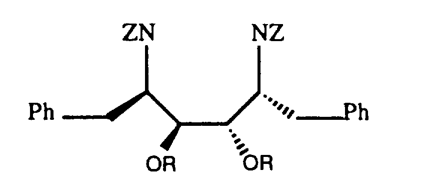

(XXa) R=SEM

(XXb) R=MOM

### A. Protection as 2-(trimethylsilyl) ethoxy methyl (SEM) ether (XXa):

Compound (XIX) (60 g, 105 mmol) was dissolved in dry DMF (600 mL). Diisopropylethylamine (75 mL) and SEMCl (66.8 g, 400mmol) were added and the mixture stirred for 16 h at room temperature under N₂. The solution was diluted with water (1L) and extracted with hexane (400mL). The organic layer was separated and washed with water (2x100mL). The aqueous layers were combined and extracted with hexane (2x300mL). The organic layers were combined, washed with water (2x100mL), dried over MgSO₄, filtered and evaporated. The residue was chromatographed on SiO₂ and eluted with 10-30% ethyl acetate/hexane to afford a white solid (91g, 100%). NMR(CDCl₃): δ 7.0-7.4 (m, 20H, Ph), 5,01 (br s, 4H, PhCH₂CO), 4.5-4.95 (m, 6H, NH, OCH₂O), 3.6-4.25 (m, 4H, CHOCH₂, CHNH), 3.5 (s, 4H, OCH₂CH₂), 2.76 (br d, 4H, PhCH₂), 0.8-1.0 (m, 4H, SiCH₂). MS: 846 (M+NH₄, 100), 695 (M-SEM, 40).

### B. Protection as methoxymethyl (MOM) ether (XXb).

Compound (XIX) (.50 g, 0.88 mmol) was dissolved in dry DMF (10mL). Diisopropylethylamine (.46 mL, 2.64 mmol) and methoxymethyl bromide (.165 mL, 2.02 mmol) were added and the solution stirred at 40 °C under nitrogen for four h. TLC (50/50 ethyl acetate /methylene chloride) showed that the reaction was complete. The mixture was partitioned between methylene chloride (50 mL) and 5% HCl (30 mL). The organic layer was separated , washed with water (5x20 mL), brine (20 mL), dried over MgSO₄, filtered and evaporated to a light yellow oil. Chromatography on SiO₂ and elution with 1-20% ethyl acetate/methylene chloride afforded (XXb) as a clear oil ( .29 g, 53%).
NMR (CDCl₃): δ 6.95-7.42 (m, 20H, Ph), 5.1-3.8 (m, complex), 3.35 (s, 6H, OCH₃), 2.8-2.95 (m, 4H, PhCH₂). MS: 657 (13, M+1), 674 (21, M+NH₄), 522 (84), 414 (100), 370 (34).

### Procedure 3

Deprotection of amines (XXa) and (XXb) via hydrogenation to afford (XXIa) and (XXIb):

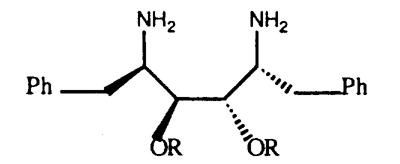

(XXIa) R=SEM

(XXIb) R=MOM

### A. Hydrogenation of SEM ether (XXa).

Compound (XXa) (90 g, 108.5 mmol) was dissolved in absolute ethanol (2.5 L). 5% Pd/C (6.5 g) was added and the solution was stirred under hydrogen for 1.5 h until hydrogen uptake ceased. TLC (20/80 ethyl acetate/hexane) showed that the reaction was complete. The solution was filtered through Celite and evaporated at reduced pressure to a colorless gum (60 g, 99%). NMR (CDCl₃) : δ 7.1-7.35 (m, 10H, Ph), 4.72 (br d, 4H, OCH₂O), 3.5-3.9 (m, 6H, NH₂, CHOCH₂), 3.15 (m, 2H, CHNH₂), 2.55-2.95 (m, 4H, PhCH₂), 0.95 (m, 4H, SiCH₂).

### B. Hydrogenation of MOM ether (XXb).

Compound (XXb) (.29 g, .441 mmol) was dissolved in ethyl acetate (6 mL) and methanol (3 mL). 10% Pd/C (70 mg) was added and the solution stirred under hydrogen until H₂ uptake ceased. TLC (20/80 methanol/ethyl acetate) showed that the reaction was complete. The solution was filtered through Celite and evaporated at reduced pressure to afford 3b as a clear oil (.132 g, 77.4%). NMR (CDCl₃): δ 7.1-7.35 ( m, 10H, Ph), 4.58 (s, 4H, OCH₂O), 3.75 (br s, 2H, CHOCH₂), 3.3-3.5 (m, 2H, CHNH₂), 3.23 (s, 6H, OCH₃), 2.85 (br d, 4H, PhCH₂), MS: 389 (M+1, 100), 345 (3.7), 280 (1.8), 120 (6.1).

### Procedure 4

Formation of cyclic ureas (XXIIa) and (XXIIb) :

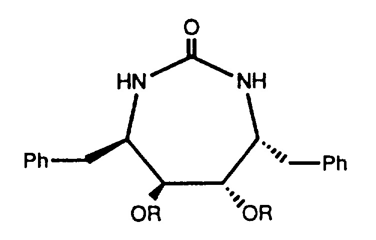

(XXIIa) R=SEM

(XXIIb) R=MOM

### A. Cyclization of SEM ether (XXIa).

Compound (XXIa) (40 g, 71.3 mmol) was dissolved in methylene chloride (200 mL). Carbonyl diimidazole (13.87 g, 85.6 mmol) was dissolved in methylene chloride (200 mL) in a separate flask. Each solution was then pumped into dry methylene chloride (6 L) at a rate of 90 mL/h. The mixture was then stirred for 18h at room temperature under nitrogen. TLC (60/40 ethyl acetate/hexane) showed the reaction was complete. The solvent was removed at reduced pressure and residue chromatographed on SiO₂ and eluted with 1-50% ethyl acetate/hexane to afford (XXIIa) as a white solid (38.82g, 93%). mp: 75-76°C. NMR (CDCl₃): δ 7.05-7.4 (m, 10H, Ph), 4.6-4.8 (dd, 4H, OCH₂O), 4.08 (s, 2H, CHOCH₂), 3.5-3.91 (m, 8H,
NH, CHNH, OCH₂CH₂), 2.86, (br d, 4H, PhCH₂), 0.8-0.95 (m, 4H, SiCH₂) . MS: 587 (M+1, 100).

### B. Cyclization of MOM ether (XXIb).

Compound (XXIb) (.53 g, 1.364 mmol) was dissolved in dry methylene chloride (20 mL). In a separate flask, carbonyl diimidazole (.265 g, 1.64 mmol) was dissolved in methylene chloride (20 mL). To a third flask containing pyridine (.22 mL, 2.73 mmol) in methylene chloride (100 mL) at room temperature under nitrogen were added the first two solutions via syringe pump at a rate of 1.7 mL/h. The solution was stirred overnight at room temperature. TLC (50/50 ethyl acetate/methylene chloride) showed that the reaction was complete. The solution was washed with 5% HCl (50 mL), NaHCO₃ (50 mL), brine (50 mL), dried over MgSO₄, filtered and evaporated. The residue was chromatographed on SiO₂ and eluted with 50-75% ethyl acetate/methylene chloride to afford (XXIIb) as a colorless gum (198 mg, 35%). NMR (CDCl₃): δ 7.1-7.4 (m, 10H, Ph), 4.65 (q, 4H, OCH₂O), 4.13 (s, 2H, NH), 3.89 (t, 2H, CHNH), 3.59 (s, 2H, CHOCH₂), 3.18 (s, 6H, OCH₃), 2.87 (m, 4H, PhCH₂). MS: 415 (M+1, 100), 102 (11).

### Synthesis of Dimem DiZ Intermediate:

DiZ Diol 507g(0.89mol) was stirred in 4L of dichloromethane. To the slurry was added N,N-Diisopropylethylamine 780g(6.05mol) in one portion at room temperature followed by the dropwise addition of B-methoxyethoxy methylchoride 500g(4mol) (1 hour addition, exothermic). Heated the solution at reflux for 12 hours. TLC (10:1:10 EtOAc:EtOH:Hexanes, Rf=0.56) indicated a complete reaction. The solution was worked up by quenching with ice water(3L). Washed the dichloromethane extract with water(2x 2L) and dried over magnesium sulfate. The filtrate was taken to dryness. The resultant semi-solid was dissolved in chlorobutane(1L). Passed the solution through a four inch pad of silica gel to remove most of the intense red color. To the chlorobutane extract was added hexane(2L) to precipitate the desired DiZ Dimem intermediate. Washed the white solid with hexanes(3x 350ml). Dried at room temperature. Recovered the desired DiZ Dimem intermediate as a white solid in a yield of 525g(79% yield). m.p. 52-54 C, 1H NMR(CDCl₃) : 2.80(m, 4H)-CH₂Ph, 3.38(s, 6H)-OCH₃, 3.58(m, 8H)-OCH₂CH₂O-, 3.80(m, 2H), 4.20(m, 2H), 4.6-5.2(m, 10H)NH, H₂CCO₂, -OCH₂O-, 7.25(m, 20H)C₆H₅

### Synthesis of Cyclic Urea Intermediate:

DiZ Dimem 20g(26.8mmol) was dissolved in 200ml of tetrahydrofuran. To the solution was added 2g of 10% Palladium on Carbon and the suspension stirred for 7 hours under hydrogen(1 atm). TLC (10:1:10 EtOAc:EtOH:Hex, Rf=0.05) indicated a complete reaction. The suspension was filtered through a bed of Celite to remove the catalyst. Washed the Celite bed with 150ml of tetrahydrofuran. Transferred the THF solution to a 500ml round bottom flask. To the THF solution was added 5.5g(33.3mmol) 1,1'-Carbonyldiimidazole in several portions as a solid. Stirred at room temperature for 12 hrs. TLC (10:1:10 EtOAc:EtOH:Hex, Rf=0.26) indicated a complete reaction. The mixture was worked up by quenching with ice-cold 0.5N HCl (150ml) and extracting with diethyl ether (2x50ml). The organic extract was washed with water (2x100ml) and dried over magnesium sulfate. The filtrate was taken to dryness. The residue was purified on silica gel(200g; 1:1 EtOAc:Hex followed by 10:1:10 EtOAc:EtOH:Hex) to provide 10.2g (75.7% yield over two steps) of the desired cyclic urea intermediate as a colorless oil. 1H NMR(CDCl₃): 2.90(m, 4H)-CH₂Ph, 3.36(s, 6H)-OCH₃, 3.40(m, 8H) -OCH₂CH₂O-, 3.60(m, 2H), 3.90(t, 2H), 4.10(s, 2H)NH, 4.80(q, 4H)-OCH₂O-, 7.30(m, 10H)C₆H₅

### Procedure 5

### General alkylation/hydrolysis procedure:

Compound (XXIIa) (1 mmol) in dry DMF (5 mL) was added to a flask containing sodium hydride (10 mmol, that had been washed with hexane, 3x20 mL) in DMF (5 mL). The solution was stirred at room temperature under nitrogen for 5 min. Evolution of hydrogen gas was observed. The appropriate alkyl bromide (5 mmol) was added and the solution was stirred at room temperature under nitrogen for lh. Hindered alkyl bromides required heating at 40-70 °C for up to 5 h. TLC (40/60 ethyl acetate/hexane) was used to ensure that no starting material remained. The solution was quenched with methanol (1 mL), partitioned between ether (60 mL) and water (50 mL) and the organic layer was removed. The aqueous layer was washed with ether (50 mL), the organic layers combined and washed with water (4x30 mL), brine (30 mL), dried over MgSO₄, filtered and evaporated. In cases where the alkyl bromide contained nitrogen, 1 N NaOH was used in place of water.

The crude product was hydrolyzed directly in methanol (10 mL) and 4 N HCl/dioxane (5 mL) for up to 16 h at room temperature. The solution was evaporated and chromatographed directly on SiO₂ to afford the bis-alkylated cyclic ureas. Where nitrogen was present, the solutions were first basified with 1 N NaOH and extracted with ethyl acetate, dried over MgSO₄, filtered, evaporated and chromatographed.

Hydrolysis of (XXIIb) under the same conditions gave 67% yield of the N,N-unsubstituted cyclic urea Example 1A, mp 170-174 °C.

### Example 1G

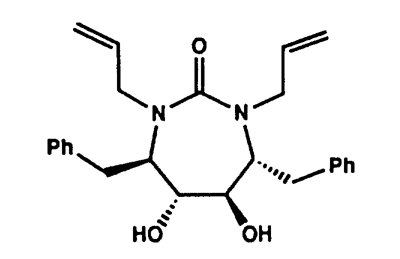

The experimental procedure is similar to the synthesis of Example 1E. The isomer, *(2R,3R,4R,5R)-*2,5-diamino-1,6-diphenyl-3,4-hexanediol, needed for the synthesis was isolated from the vanadium trichloride coupling reaction, as described in copending commonly assigned patent application Jadhav et al. USSN 07/714,042, filed 5/31/91 (see Procedure 1 above).

Example 1G: ¹³C NMR (CDCl3) : (75.48 MHz) 37.387, 51.51, 65.136, 72.779, 118.649, 126.540, 128.409, 129.714, 134.618, 137.757, 162.705.

### Example 1F

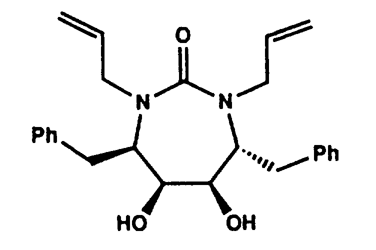

Compound (XXIIb) (0.85g) was heated with mixture of acetic acid (9.5 mL) and water (0.5 mL) at 85 °C for 4h. After extraction with dichloromethane, followed by washing the organic extract with saturated sodiumbicarbonate and brine, a mixture was provided which, on separation by column chromatography, furnished (XXIIb) (TLC 1:10 ethyl acetate/hexane Rf = 0.4; 0.54g), the desired mono-alcohol intermediate ( TLC 1:10 ethyl acetate/hexane Rf = 0.1; 0.13g), and overhydrolysed diol (0.05g).

The above mono-alcohol intermediate 0.25g (0.466 mmol), triphenylphosphine 183mg (0.7 mmol), diethylazadicarboxyalte 0.11 mL (0.7 mmol), and chloroacetic acid 66mg (0.7 mmol) were stirred in 5 mL anhydrous tetrahydrofuran at 0 °C for 15 minutes and then at room temperature for 18 h. The excess reagents were quenched with 0.5 mL methanol and the mixture allowed to stir for 20 minutes. The mixture was purified by silica gel column chromatography to provide the desired chloroacetate intermediate with inversion of configuration. ¹³C NMR (CDCl3) : (75.48 MHz) -1.373, 14.413, 14.487, 18.253, 25.591, 33.851, 35.741, 40.505, 48.824, 49.962, 57.507, 58.234, 66.589, 67.885, 73.179, 77.423, 95.454, 117.296, 118.554, 126.588, 126.887, 128.518, 128.610, 129.117, 129.199, 129.479, 133.686, 134.168, 136.324, 138.285, 155.698, 166.323.

The above chloroacetate intermediate 73mg (0.12 mmol) in 2mL dry methanol was treated with 0.25 mL (0.5M) sodium methoxide and stirred for 30 minutes at room temperature. The contents were then treated with 0.3 mL (4% HCl in methanol) and stirred for 4.5h at room temperature. The residue after removal of solvent was purified on silica gel column to provide Example 1F.

Example 1F: ¹³C NMR (CDCl3): (75.48 MHz) 34.075, 37.672, 48.941, 48.985, 58.071, 60.640, 65.861, 73.212, 177.975, 118.669, 126.535, 126.858, 128.603, 128.815, 129.225, 133.605, 134.172, 137.637, 138.273, 155.497.

### Synthesis of Monoalkyl Cyclic Urea:

The intermediate from previous step 2g(4mmol) was dissolved in 25ml toluene and placed in a 100ml round bottom flask. To the solution was added 85% KOH 0.82g(12mmol) and polyethylene glycol (M.W.=1000) 0.20g. With a Dean Stark trap in place the mixture was refluxed for 4 hours until the theoretical amount of water(0.20ml) was collected Cooled to room temperature and added (bromomethyl)cyclopropane 1.78g (13.2mmol). Stirred at 75 C for 17 hours. TLC(10:1:10 EtOAc:EtOH:Hex, Rf=0.52) indicated that the reaction was complete. Worked up by quenching with aqueous ammonium chloride(50ml) and extracting with ethyl acetate (2x35ml). Washed the organic layer with water(2x35ml) and dried over magnesium sulfate. The filtrate was taken to dryness. The residue was purified on silica gel(150g, 2:3 EtOAc:Hex) to provide 1.55g(70% yield) of the desired monoalkyl cyclic urea as a colorless oil. C13 NMR(CDCl₃): 3.331, 4.000, 10.619, 32.877, 34.159, 55.677, 58.294, 58.972, 64.085, 67.361, 67.437, 71.723, 71.753, 76.576, 78.023, 96.347, 96.519, 126.224, 126.316, 128.366, 128.563, 129.400, 129.447, 139.475, 139.555, 161.558.

### Examples 1A-1Z, 1AA-1AZ, and 1BA-1BD

The compounds listed in Table la (Examples 1A-1Z, 1AA-1AZ, and 1BA-1BD) were synthesized using the above-described procedures.

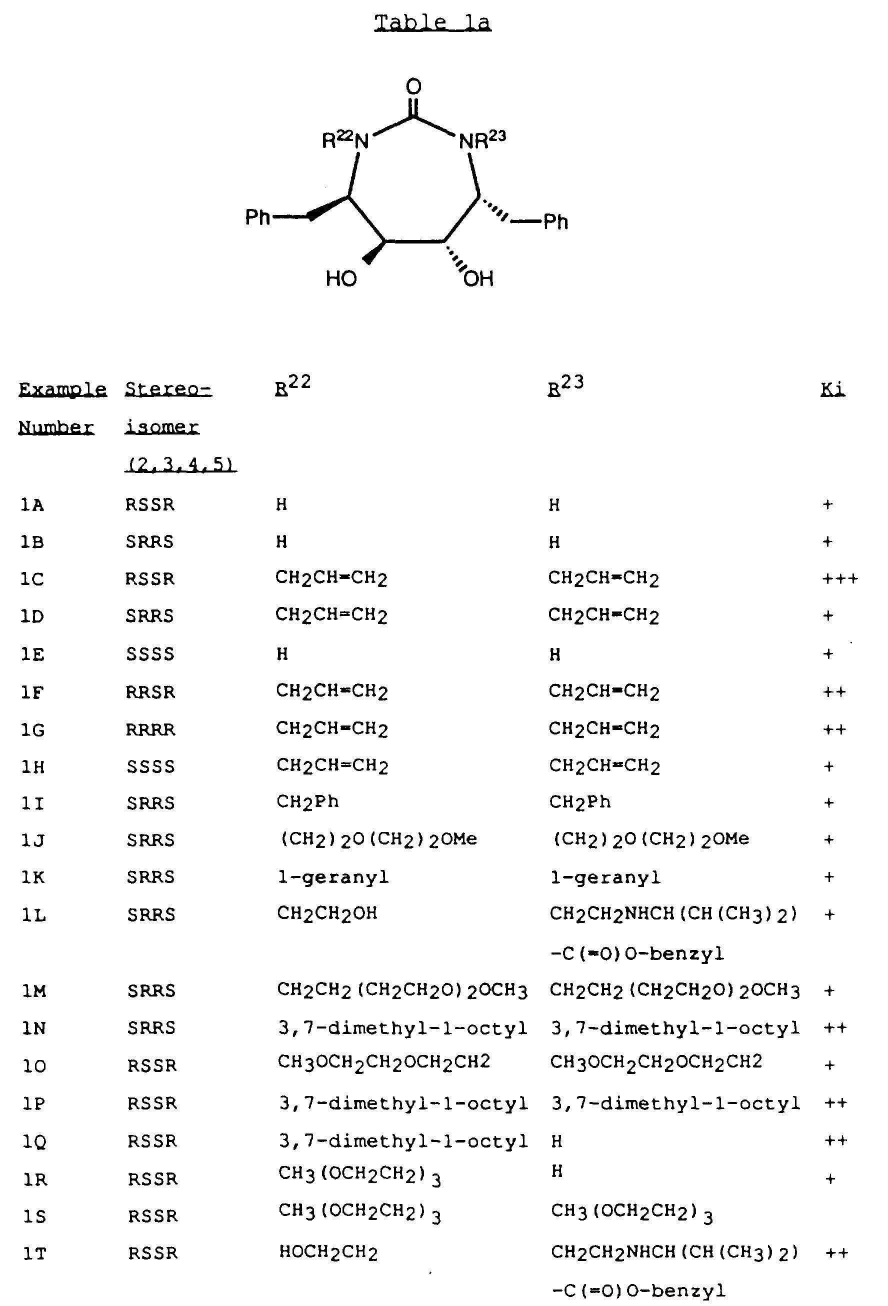

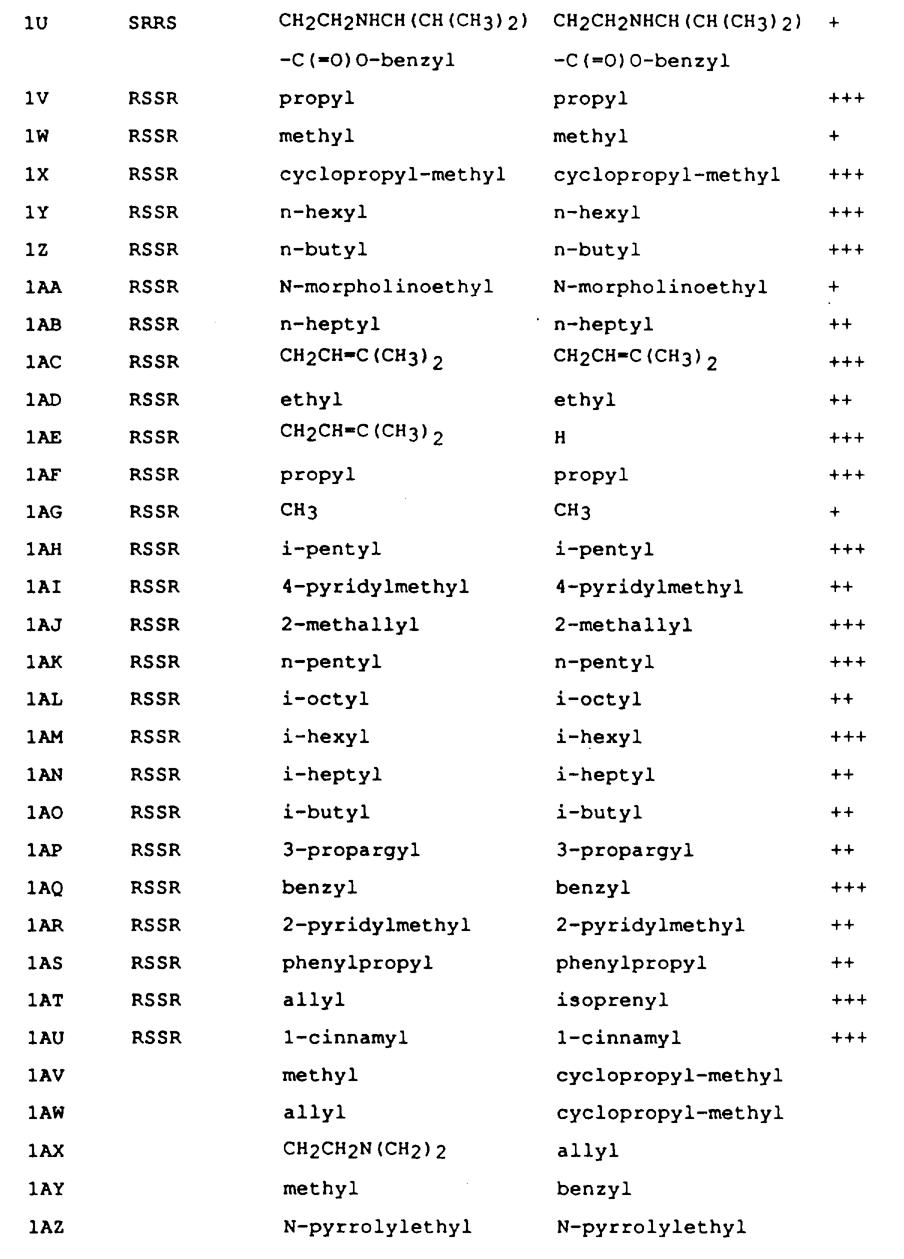

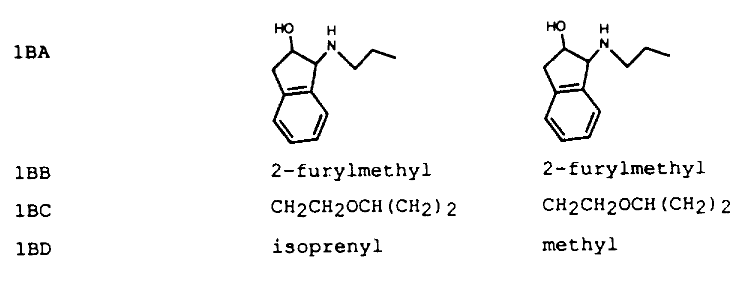

The Ki values in Table 1 were determined using the assay conditions described below under HIV Protease Inhibtion Assay. The Ki values are indicated as follows: +++ = <10 nM; ++ = 10 nM to 1 µM; + = >1 µM.

Listed below are physical data for representative compounds of the invention.

Example 1W: mp 170-174 °C (67% yield). MS: 355 (M+1, 100). NMR (CDCl₃)δ 7.1-7.35(m, 10H, Ph), 4.03(s,2H,CHOH, 3.5(d, 2H, NCH), 2.8-3.1(m, 6H, PhCH₂, OH), 2.58(s, 6H, NH₃).

Example 1AD: mp 214-215 °C. MS: 383(M+1, 100). NMR (CDCl₃)δ 7.1-7.35 (m, 10H, Ph), 3.95 (s, 2H, CHOH), 3.5 (d, 2H, NCH), 2.8-3.1 (m, 4H, PhCH₂, OH), 2.7 (br s, 2H, OH), 2.3 (m,2H, NCH), .95(t, 6H, CH₃).

Example 1AF: mp 180-182 °C. MS: 411 (M+1, 100). NMR (CDCl₃)δ 7.05-7.3(m, 10H, Ph), 4.0(s, 2H, CHOH), 3.68(m,2H, NCH₂), 3.52(d, 2H, NCH), 3.05(m, 4H, PhCH₂), 2.1(m, 2H, NCH₂), 1.6 (s, 2H, OH), 1.4 (m, 4H, MeCH₂), .79(t, 6H, CH₃).

Example 1Z: HRMS: Calc. 439.2960. Found: 439.2959.

Example 1AK: mp 125-127 °C. MS: 467(M+1, 100). NMR(CDCl₃)δ 7.15-7.35(m, 10H, Ph), 3.95(s, 2H, CHOH), 3.68(m, 2H, NCH₂),3.55(d, 2H, NCH), 2.9-3.15(m, 4H, PhCH₂), 2.75(s, 2H, OH), 2.18(m, 2H, NCH₂), >8-1.45(m, complex, pentyl).

Example 1Y: mp 110-112 °C. MS: 495(M+1,100). NMR(CDCl₃)δ 7.1-7.35(m, 10H, Ph), 4.0(s, 2H, CHOH), 3.65(m, 2H, NCH-H), 3.52(d, 2H, NCH), 2.8-3.2(m,4H,PhCH₂), 2.15(m,2H, OH), .9-1.45(m, 11H, hexyl).

Example 1AB: mp 100-101°C. MS: 523(M+1,100). NMR(CDCl₃)δ 7.1-7.35(m, 10H, Ph), 3.95(s, 2H, CHOH), 3.65(m, 2H, NCH-H), 3.5(d, 2H, NCH), 2.9-3.1(m, 4H, PhCH₂), 2.6(s, 2H, OH), 2.15(m, 2H, NCH), .8-1.4(m, complex, heptyl).

Example 1I: MS: 507(M+1,100). NMR(CDCl₃)δ 7.05-7.4(m, 20H, Ph), 4.91(d, 4H, PhCH₂N), 3.5-3.65(m, 4H, NCH, CHOH), 3.05(m, 4H, PhCH₂), 2.35( br s, 2H, OH).

Example 5i (R²² and R²³ = allyl; R²⁰ and R²¹ = C(=O)CH3): mp 164-166 °C. MS: 407(M+1,100). NMR(CDCl₃)δ 7.1-7.4(m, 10H, Ph), 5.65(m, 2H, CH₂CH), 5.01(m, 4H, CH₂CH), 4.26(m, 4H, NCH₂), 3.91(s, 2H, CHOH), 3.59(d, 2H, CHN), 3.1(m, 4H, PhCH₂), 2.7(m, 2H, CHCH₂)D 2.41(s, 2H, OH).

Example 1AO: MS 439(M+1,100). NMR(CDCl₃)δ 7.15-7.35(m, 10H, Ph), 4.05(s, 2H, CHOH), 3.7(m,2H, NCH₂), 3.55(d, 2H, NCH), 3.0-3.2(m, 4H, PhCH₂), 2.65(s, 2H, OH), 1.78(m, 2H, NCH), 1.6(s, 2H, CH₂CH), .82(d, 12H, CHMe₂).

Example 1AH: mp 194-195 °C. MS: 467(M+1,100). NMR(CDCl₃)δ 7.15-7.35(m, 10H, Ph), 3.95(s, 2H, CHOH), 3.65(m, 2H, NCH), 3.49(d, 2H, NCH), 2.9-3.1(m, 4H, PhCH₂), 2.61(s, 2H, OH), 2.2(m, 2H, NCH), 1.1-1.5(m, complex), .9(m, 12H, CHMe₂).

Example 1AM: mp 120-122 °C. MS: 495(M+1,100). NMR(CDCl₃)δ 7.1-7.35(m, 10H, Ph), 3.95(s, 2H, NCH), 3.45-3.7(m, 4H, NCH₂, NCH), 2.9-3.2(m, 4H, PhCH₂), 2.1(m, 2H, NCH₂), .78-1.45(m, complex, hexyl).

Example 1AN: mp 105-107 °C. MS: 523(M+1,100). NMR(CDCl₃)δ 7.18-7.31(m, 10H, Ph), 3.95(s, 2H, CHOH), 3.7(m, 2H, NCH₂), 3.56(d, 2H, NCH), 2.9-3.15(m, 4H, PhCH₂), 2.18(m, 2H, NCH₂), .9-1.45(m, complex, hexyl).

Example 1AL: mp 144-145 °C. MS: 551(M+1,100). NMR(CDCl₃)δ 7.15-7.35(m,10H, Ph), 3.95(s, 2H, CHOH), 3.65(m, 2H, NCH₂), 3.55(d, 2H, NCH), 2.9-3.15(m, 6H, PhCH₂, OH), 2.15(m, 2H, NCH₂), .9-1.5(m, complex, heptyl).

Example 1AC: mp 160-163 °C. MS: 463(M+1,100). NMR(CDCl₃)δ 7.15-7.35(m, 10H, Ph), 5.06(br t, 2H, NCH₂CH), 4.1(dd, 2H, NCH₂), 3.9(s, 2H, CHOH), 3.5(d, 2H, NCH), 2.8-3.1(m, 4H, PhCH₂), 1.65(s, 6H, CHCH₃), 1.38(s, 6H, CHCH₃).

Example 1AJ: mp 205-207 °C. MS: 435(M+1,100). NMR(CDCl₃)δ 7.1-7.35(m, 10H, Ph), 4.85(s, 2H, C=CH), 4.58(s, 2H, C=CH), 4.28(d, 2H, NCH₂), 3.95(s, 2H, CHOH), 3.7(br d, 2H, CHOH), 2.9-3.2(m, 4H, PhCH₂), 2.5(d, 2H, NCH₂), 1.75(s, 6H, CH₃).

Example 1AI: MS: 509(M+1,100). NMR(CDCl₃)δ 7.0-8.4(m, 18H, Ph, pyr.), 4.8-5.0(m, 4H, NCH₂), 3.7(s, 2H, CHOH), 3.5(d, 2H, NCH), 2.9-3.2(m, 8H, PhCH₂, pyrCH₂).

Example 1AP: mp 198-200 °C. MS: 403(M+1,100). NMR(CDCl₃)δ 7.2-7.35(m, 10H, Ph), 4.5(s, 2H, CCH), 4.42(s, 2H, NCH), 4.1(s, 2H, CHOH), 3.8(d, 2H, NCH), 2.9-3.2(m, 4H, PhCH₂), 2.7-2.85(m, 4H, NCH₂).

Example 1O: mp 105-106 °C. MS: 531(M+1,100). NMR(CDCl₃)δ 7.1-7.35(m, 10H, Ph), 3.0-4.15(m, complex), 2.25(m, 2H, OH).

Example 1S: MS: 619(M+1,100). NMR(CDCl₃)δ 7.18-7.3(m, 10H, Ph), 3.0-4.2(m, complex), 2.25(m,2H,OH).

Example 1P: mp 80-82 °C. MS: 607(M+1,100), 257(9.6). NMR(CDCl₃)δ 7.05-7.35(m,10H, Ph), 3.95(s, 2H, CHOH), 3.4-3.75(m, 4H, NCH, NCH₂), 2.9-3.15(m, 4H, PhCH₂), 2.1(m,2H, OH), .85-1.6(m, complex).

Example 1AA: mp 70-75 °C. MS: 553(M+1,100). NMR(CDCl₃)δ 7.05-7.4(m, 10H, Ph), 3.4-4.25(m, complex), 2.9-3.15(m, 4H, PhCH₂), 2.2-2.8(m, complex).

Example 1X: mp 210-212 °C. MS: 435(M+1,100). NMR(CDCl₃)δ 7.18-7.35(m, 10H, Ph), 4.06(s, 2H, CHOH), 3.68(br d, 2H, NCH), 3.55(q, 2H, NCHCH), 3.1(m, 4H, PhCH₂), 2.55(s, 2H, OH), 2.05(q, 2H, CHCH), .9(m, 2H, NCH₂CH), .42(m, 4H, -CH₂-), .008(m, 4H, -CH₂-).

| Example Number | MS (M+1) |
|---|---|
| 1B | 327.9 |
| 1D | 406.5 |
| 1H | 407 |
| 1J | 531 |
| 1K | 463 |
| 1L | 604.5 |
| 1M | 619 |
| 1N | 607 |
| 1O | 467 |
| 1R | 473 |
| 1T | 604 |
| 1U | 793 |
| 1V | 411 |
| 1AE | 395 |
| 1AG | 355 |
| 1AS | 563 |
| 1AT | 435 |
| 1BD | 409 |
| 1AQ | 507.26 |
| 1AU | 559.29 |

Example 1AR: mp 85-87 °C.
Example 1C: mp 164-166 °C.
Example 1A: mp 170-174 °C.

Using the above-described techniques or variations thereon appreciated by those of skill in the art of chemical synthesis, the compounds of Tables 1b can be prepared.

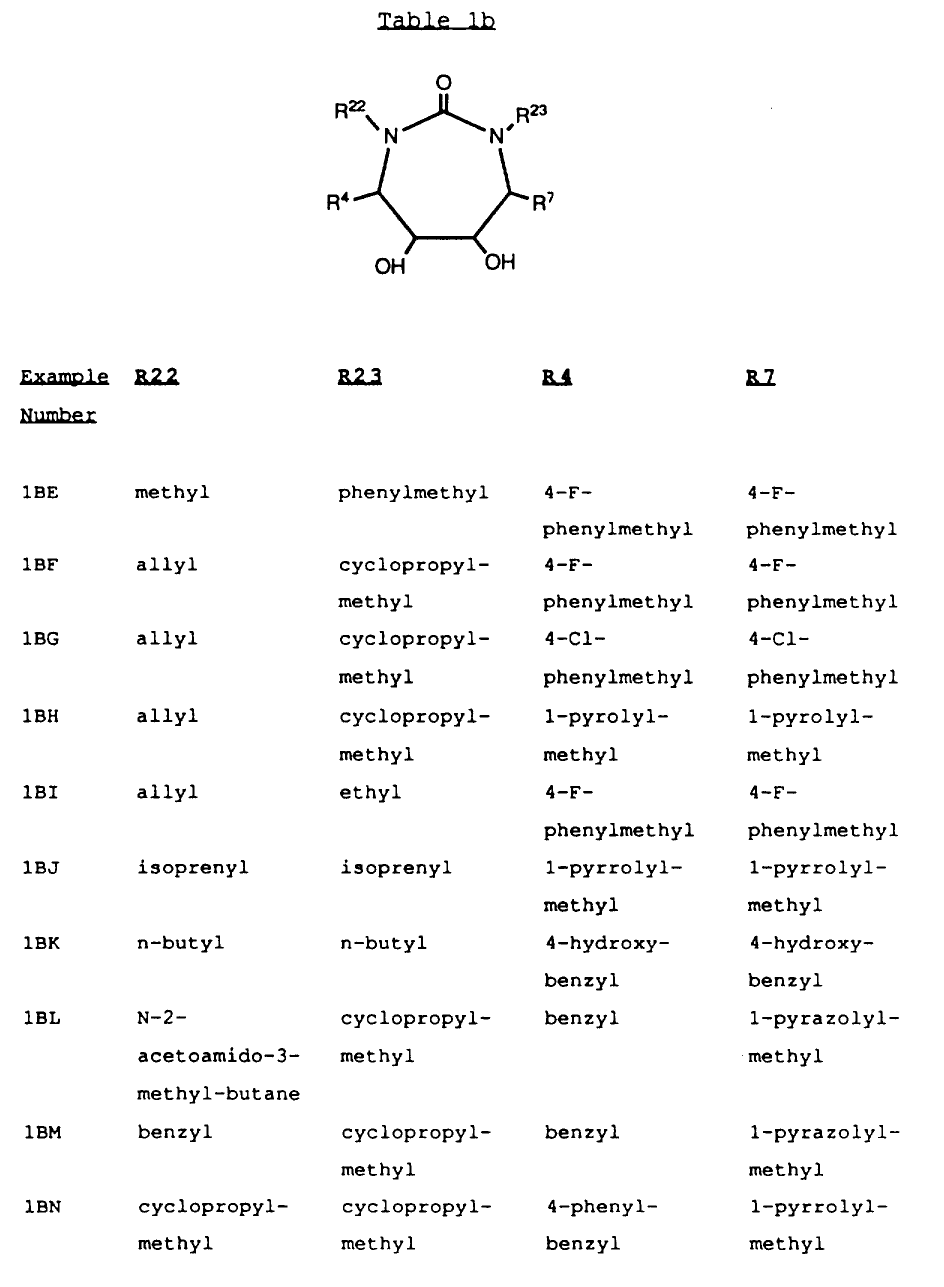

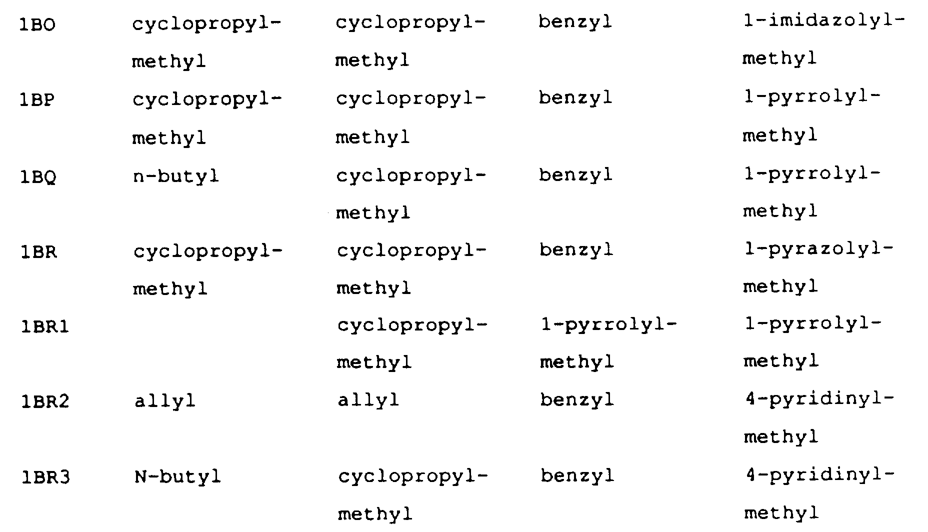

### Synthesis of Cyclic Guanidines

Cyclic guanidine compounds of the invention wherein W = NH(C=N-CN)NH, differ from the cyclic urea compounds of the invention wherein W = NH(C=O)NH. Described below are representative methods for the preparation of cyclic quanidine compounds of the invention.

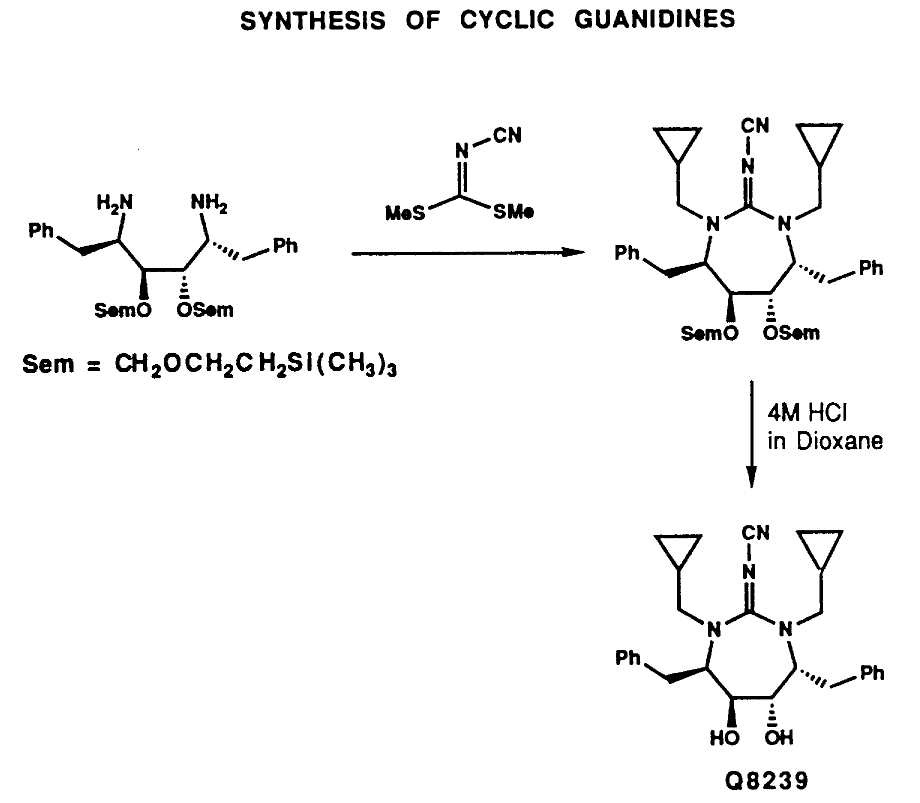

The structures of the Examples below are shown in Table 1c.

### Example 1BS (O8239)

SYNTHESIS OF INTERMEDIATE A: Diamino Disem 561mg (1mmol) was dissolved in 2ml pyridine and to this solution was added 175mg(1.2mmol) Dimethyl N-cyanodithioiminocarbonate. The contents were refluxed in a 125°C oil bath for 2 hours. (Caution: Methyl mercaptan is a by-product and the reaction should be vented to a Clorox™ scrubber). TLC(1:2 EtOAc:Hexane Rf=0.4) indicated a complete reaction. The reaction was diluted with 100ml dichloromethane. The organic layer was washed with 1N HCL(2x25ml) followed by sat. sodium bicarbonate solution (25ml). It was separated and dried over magnesium sulfate and the filtrate taken to dryness. The residue was purified on SiO₂ gel(55g; using 1:3 followed by 1:2 EtOAc:Hexane) to provide 372mg(60.9% yield) of the desired intermediate A as a colorless oil.

### SYNTHESIS OF INTERMEDIATE B: Intermediate A

305mg(0.5mmol) was dissolved in 2ml dimethylformamide and to this solution, cooled in a 0°C ice bath, was added NaH(60% in oil) 80mg(2mmol) slowly. The contents were stirred at room temperature for 30 minutes. The mixture was cooled in a 0°C ice bath and (bromomethyl)cyclopropane 0.19ml(2mmol) was added via syringe and stirred at room temperature for 18 hours. TLC(1:4 EtOAc:Hexane Rf=0.31) indicated a complete reaction. The reaction was worked up by diluting with water(50ml) and extracting with diethylether (2x25ml). The organic layer was dried over magnesium sulfate and the filtrate taken to dryness. The residue was purified on SiO₂ gel(33g; 1:5 followed by 1:4 EtOAc:Hexane) to provide 243mg(67.6% yield) of the desired intermediate B as a colorless oil.

### SYNTHESIS OF EXAMPLE 1BS: Intermediate B 110mg

(0.153mmol) was placed in a 10ml R.B. Flask and cooled in a 0°C ice bath. To the flask was added 4M HCl in dioxane 1ml (4mmol) and the mixture stirred at room temperature for 15 minutes. TLC(1:1 EtOAc:Hexane Rf=0.15) indicated a complete reaction. The mixture was worked up by quenching in sat. sodium bicarbonate(25ml) and extracting with dichloromethane (2x25ml). The organic extracts were dried over magnesium sulfate and the filtrated taken to dryness. The residue was purified on SiO₂ gel(33g; 1:1 EtOAc:Hexane followed by 10:1:10 EtOAc:EtOH:Hexane) to provide 27mg(38.5% yield) of the desired Q8239 as a white solid. m.p.211.2°C

### Example 1BT

### SYNTHESIS OF INTERMEDIATE C: Intermediate A

1.515g(2.48mmol) was dissolved in 7.5ml dimethylformamide and to this solution, cooled in a 0°C ice bath, was added NaH(60% in oil) 397mg(9.92mmol) slowly. The contents were stirred at room temperature for 30 minutes. The mixture was cooled in a 0°C ice bath and p-benzyloxybenzyl chloride 2.308g(9.92mmol) was added as a solid and the mixture stirred at room temperature for 18 hours. TLC(1:4 EtOAc:Hexane Rf=0.31) indicated a complete reaction. The reaction was worked up by diluting with water(100ml) and extracting with diethylether (2x50ml). The organic layer was dried over magnesium sulfate and the filtrate taken to dryness. The residue was purified on SiO₂ gel(130g; 1:4 followed by 1:3 EtOAc:Hexane)to provide 2.068g(83.1% yield) of the desired intermediate C as a colorless foam.

### SYNTHESIS OF EXAMPLE 1BT: Intermediate C 1.928g

(1.92mmol) was placed in a 100ml R.B. flask and cooled in a 0°C ice bath. To the flask was added 4M HCl in dioxane 15ml (60mmol) and the mixture stirred at room temperature for 15 minutes. TLC(1:1 EtOAc:Hexane Rf=0.25) indicated a complete reaction. The mixture was worked up by quenching in 0.5N sodium hydroxide solution(100ml) and washing with sat.sodium bicarbonate(50ml) and extracting with dichloromethane (3x50ml). The organic extracts were dried over magnesium sulfate and the filtrate taken to dryness. The residue was purified on SiO₂ gel(130g; 1:1 EtOAc:Hexane) to provide 1.284g(89.9% yield) of the desired Q8241 as a white solid. m.p.90.1°C

### Example 1BU

### SYNTHESIS OF EXAMPLE 1BU: Example Number 1BT

1.161g(1.56mmol) was dissolved in 15ml of ethanol. To the mixture was added 1.1g of 5% Palladium on Carbon and the suspension stirred for 18 hours under hydrogen(1 atm). TLC indicated an incomplete reaction. The mixture was treated with 1.1g of 10% Palladium Hydroxide on Carbon and stirred for 2 hours under hydrogen(1 atm). TLC(10:1:10 EtOAc:Hexane Rf=0.31) indicated a complete reaction. The suspension was filtered through a celite pad and the filtrate taken to dryness. The residue was purified on SiO₂ gel(130g; 10:1:10 followed by 10:2:10 EtOAc:EtOH:Hexane) to provide 458mg(52.2% yield) of the desired Q8242 as a white solid. m.p.103.3°C

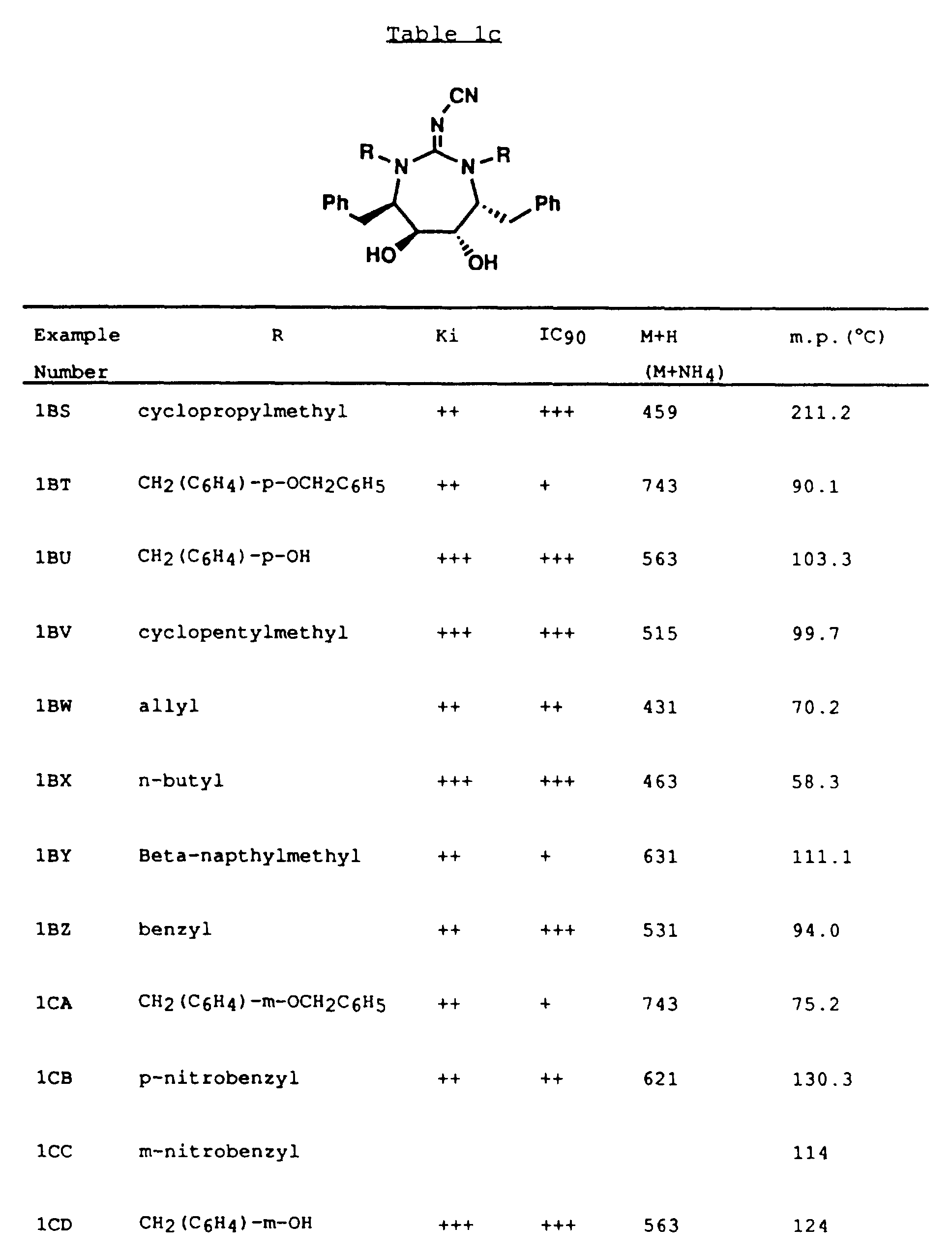

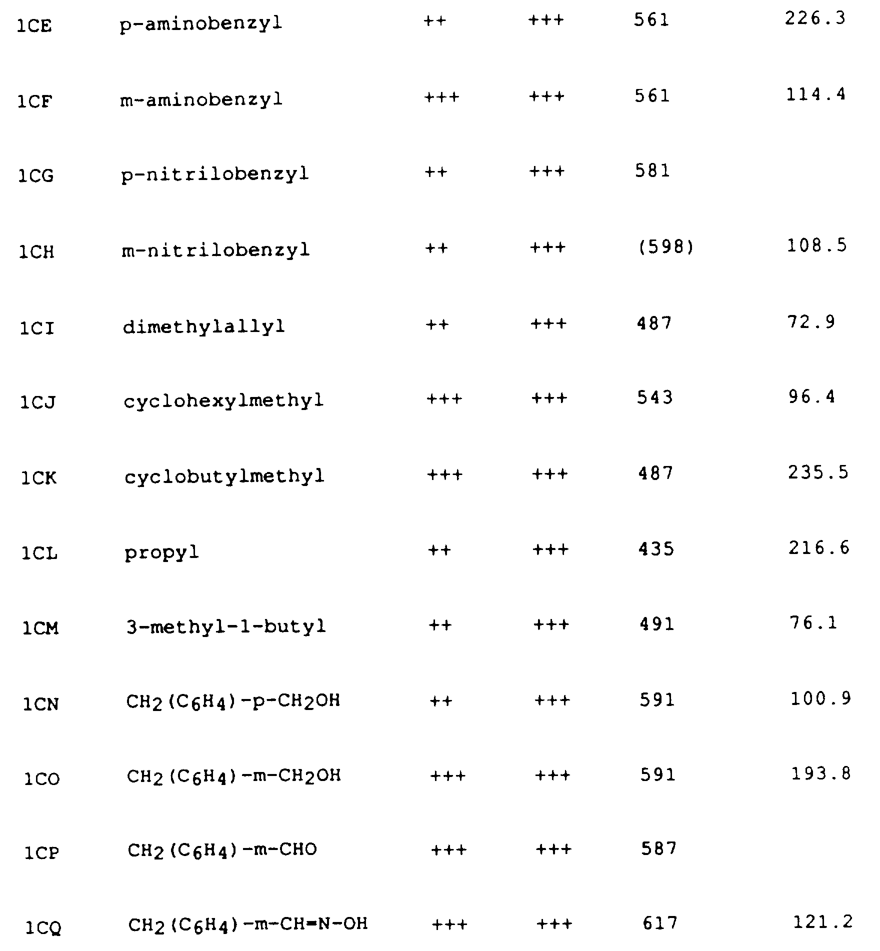

The structures of the Examples below are shown in Tables 1d and 1e.

### Example 7F

A solution of Example 1X (120 mg, 0.27 mmol) in methylene chloride (25 mL) was cooled in an ice bath at 0 °C and treated with triethylamine (110 mg, 1.1 mmol). Then a solution of thionyl chloride (150 mg, 1.3 mmol) in methylene chloride (10 mL) was added dropwise. The mixture was stirred for 10 minutes and then washed with sat'd NaHCO₃(aq), brine, and dried over MgSO₄. The solution is filtered and the solvent removed on a rotary evaporator and the resulting residue is HPLC chromatographed on silica gel (50% EtOAc/Hexanes) to give 100 mg of a white foam. HRMS calculated for C₂₇H₃₂N₂O₄S: 481.2161; found: 481.2152.

### Example 7G

A solution of Example 1X (120 mg, 0.27 mmol) in methylene chloride (25 mL) was cooled in an ice bath at 0 °C and treated with triethylamine (80 mg, 0.8 mmol). Then a solution of trichloromethyl chloroformate (DIPHOSGENE) (53 mg, 0.27 mmol) in methylene chloride (5 mL) was added dropwise. The mixture was stirred for 60 minutes and then washed with sat'd NaHCO₃(aq), brine, and dried over MgSO₄. The solution is filtered and the solvent removed on a rotary evaporator and the resulting residue is chromatographed on silica gel (50% EtOAc/Hexanes) to give 90 mg of a white foam. MS NH₃ (M+H)⁺ = 461.

### Example 7I

A solution of Ex. 1X (500 mg, 1.15 mmol) in THF (25 mL) was treated with thiocarbonyldiimidazole (410 mg, 2.3 mmol) and heated to reflux for 1.5 hours. The solvent is removed on a rotary evaporator and the resulting residue is chromatographed on silica gel (50% EtOAc/Hexanes) to give 320 mg of a white solid. MS NH₃ (M+H)⁺ = 477.3

### Example 7H and 7U

A solution of Example 71 (220 mg, 0.46 mmol) in dry toluene (25 mL) was heated to reflux and treated with Bu₃SnH (0.4 mL, 1.5 mL) and 20 mg of AIBN. The mixture was heated at reflux for 2.5 hours. The solvent is removed on a rotary evaporator and the resulting residue is chromatographed on silica gel (20% EtOAc/Hexanes) to give 70 mg of 7H as a white solid. MS NH₃ (M+H)⁺ = 447.2. A later fraction gave 30 mg of Example 7U as a white solid. MS (M+H)⁺ 419.2.

### Example 7V

A solution of Ex. 1X (112 mg, 0.26 mmol) in methylene chloride (5 mL) was cooled in an ice bath at 0 °C and treated with DAST (110 mg, 1.1 mmol). The mixture was stirred for 10 minutes and then washed with sat'd NaHCO₃(aq), brine, and dried over MgSO₄. The solution is filtered and the solvent removed on a rotary evaporator and the resulting residue is HPLC chromatographed on silica gel (65% EtOAc/Hexanes) to give 40 mg of a colorless residue. HRMS calculated for C₂₇H₃₃N₂O₂F: 437.2604; found: 437.2593.

### Example 7o

A solution of Ex. 3U (600 mg, 1.9 mmol) in pyridine (10 mL) is treated with methanesulfonyl chloride (170 mg, 1.5 mmol) and stirred at room temperature for 3 hours. The mixture is quenched with 5 mL of methanol. The solution is concentrated on a rotary evaporator and the resulting residue is dissolved in ethyl acetate and then washed with dilute HCl(aq), brine, and dried over MgSO₄. The solution is filtered and the solvent removed on a rotary evaporator and the resulting residue is chromatographed on silica gel (40% EtOAc/Hexanes) to give Example 7o (420 mg ) as a white solid. ms (M+H)⁺ = 685.

### Example 7Y

A solution of Example 7o (100 mg, 0.15 mmol) in DMF (4 mL) was treated with NaN₃ (100 mg, 1.5 mmol) and heated at 80 °C for 2 hrs and then stirred overnight at 40 °C. The solution was diluted with water and the resulting white solid is extracted into ethyl acetate. The organic extract is washed with water, brine, and dried over MgSO₄. The solution is filtered and the solvent removed on a rotary evaporator and the resulting residue is HPLC chromatographed on silica gel (50% EtOAc/Hexanes) to give Example 7Y (80 mg ) as a white solid. MS (M+H)⁺ = 632. IR (CHCl₃) 2214 cm⁻¹ for N₃.

### Example 7J and 7K

A solution of Ex. 3U (100 mg, 0.165 mmol) in pyridine (3 mL) is treated with acetic anhydride (84 mg, 0.824 mmol) and stirred at room temperature for 2 hours. The mixture is quenched with 5 mL of methanol. The solution is concentrated on a rotary evaporator and the resulting residue is dissolved in ethyl acetate and then washed with dilute HCl(aq), brine, and dried over MgSO₄. The solution is filtered and the solvent removed on a rotary evaporator and the resulting residue is HPLC chromatographed on silica gel (40% EtOAc/Hexanes) to give Example 7J (24 mg ) as a white solid. HRMS: (M+H)⁺ calculated for C₄₅H₄₃N₂O₅: 691.317198; found: 691.316252.
Also gives as a later fraction Example 7K (27 mg) as a white solid. HRMS: (M+H)⁺ calculated for C₄₃H₄₁N₂O₄: 649.306633; found: 649.304918.

### Example 7R

A solution of Ex. 3U (100 mg, 0.165 mmol) in methylene chloride (5 mL) is treated with 2,2dimethoxy propane (174 mg, 1.65 mmol), p-toluenesulfonic acid (10 mg) and stirred at room temperature for overnight. The mixture is diluted with methylene chloride and washed with saturated NaHCO₃, brine, and dried over MgSO₄. The solution is filtered and the solvent removed on a rotary evaporator and the resulting residue is HPLC chromatographed on silica gel (50% EtOAc/Hexanes) to give Example 7R (73 mg ) as a white solid. HRMS:(M+H)⁺ calculated for C₄₄H₄₃N₂O₃: 647.327369; found: 647.327531

### Example 7P

A solution of Ex. 3U (200 mg, 0.33mmol) in methylene chloride (5 mL) is treated with oxalyl chloride (249 mg, 2.0 mmol) and stirred at room temperature for overnight. The solvent removed on a rotary evaporator and the resulting residue is HPLC chromatographed on silica gel (50% EtOAc/Hexanes) to give a tan solid. This was recrystallized from CH₂Cl₂/hexane to give Example 7P (80 mg ) as a white solid. MS:(M+H)⁺ 661.4.

### Example 7S

A solution of Ex. 3U (100 mg, 0.165 mmol) in methylene chloride (5 mL) is treated with trimethylorthobutyrate (244 mg, 1.65 mmol) p-toluenesulfonic acid (10 mg) and stirred at room temperature for 30 minutes. The mixture is diluted with methylene chloride and washed with saturated NaHCO₃, brine, and dried over MgSO₄. The solution is filtered and the solvent removed on a rotary evaporator and the resulting residue is HPLC chromatographed on silica gel (50% EtOAc/Hexanes) to give Ex. 7S. MS: (M+H)⁺ 691.5

### Example 7B

A solution of the Di-MEM protected Ex. 1C (550 mg, 0.94 mmol) in pyridine (11 mL) is treated with P₂S₅ (420 mg, 0.94 mmol) and heated to reflux for 3 hrs. The pyridine is evaporated off on a rotary evaporator and the residue is taken up in methylene chloride and washed with water, NaHCO₃, and brine. The solution is dried over MgSO₄, filtered and the solvent removed on a rotary evaporator and the resulting residue is chromatographed on silica gel (40% EtOAc/Hexanes) to give Example 7B (120 mg) as a clear oil. HRMS: (M+H)⁺ calculated for C₂₇H₃₃N₂O₃S: 465.221190; found: 465.220899.

### Example 7A

A solution of Example 1C (330 mg, 0.8 mmol) in pyridine (5 mL) is treated with acetic anhydride (160 mg, 1.6 mmol) and stirred at room temperature for 4 hours. The mixture is quenched with 5 mL of methanol. The solution is concentrated on a rotary evaporator and the resulting residue is dissolved in ethyl acetate and then washed with dilute HCl (aq), brine, and dried over MgSO₄. The solution is filtered and the solvent removed on a rotary evaporator and the resulting residue is chromatographed on silica gel (50% EtOAc/Hex) to give Example 7A (120 mg) as a white solid. MS: (CI, NH₃) (M+H)⁺ = 449.1.

### Example 7W

A solution of example 1C (160 mg, 0.39 mmol) in methylene chloride (5 mL) was cooled in an ice bath at 0 °C and treated with DAST (63 mg, 0.4 mmol). The mixture was stirred for 10 minutes and then washed with sat'd NaHCO₃(aq), brine, and dried over MgSO₄. The solution is filtered and the solvent removed on a rotary evaporator and the resulting residue is HPLC chromatographed on silica gel (50% EtOAc/Hex) to give 80 mg of a colorless residue. HRMS calculated for C₂₅H₃₀N₂O₂F: 409.2291; found: 409.2291.

### Example 7X

A solution of example 3U (100 mg, 0.16 mmol) in methylene chloride (5 mL) was cooled in an ice bath at 0 °C and treated with DAST (26 mg, 0.16 mmol). The mixture was stirred for 10 minutes and then washed with sat'd NaHCO₃(aq), brine, and dried over MgSO₄. The solution is filtered and the solvent removed on a rotary evaporator and the resulting residue is HPLC chromatographed on silica gel (50% EtOAc/Hex) to give 35 mg of a white foam. HRMS calculated for C₄₁H₃₈N₂O₂F: 609.2917; found: 609.2911.

### Example 8A

A solution of example 1X (2.06 g, 4.74 mmol) in methylene chloride was treated with diisopropylethylamine (1.53 g, 11.8 mmol), MEM-Cl (0.71 g, 5.7 mmol) and heated to reflux for 5 hours and let stir overnight at rt. The solution is concentrated on a rotary evaporator the residue is HPLC chromatographed on silica gel (5 % MeOH/CHCl₃) to give 1.3 g of the mono-MEM mono-ol intermediate. MS: (CI, NH₃) (M+H)⁺ = 523.4.

A solution of mono-MEM mono-ol intermediate from above (1.0 g, 1.9 mmol) in THF was treated with triphenylphosphine (1.0 g, 3.8 mmol), diethylazadicarboxylate (DEAD) (0.7 g, 4.0 mmol), and chloroacetic acid (0.4 g, 4.2 mmol). The solution is stirred overnight at rt. The solvent is evaporated and the resulting residue is chromatographed on silica gel (50% EtOAc/Hex) to give 0.9 g of the chloroacetate intermediate. MS: (CI, NH₃) (M+H)⁺ = 599.3 (100%); 600 (39%).

The chloroacetate intermediate (0.9 g, 1.5 mmol) was dissolved in MeOH (15 ml) and treated with NaOH_{(aq)} (4 ml, 1N) and stirred at rt for 15 min. The solution was evaporated to dryness and the residue partitioned between water and ethyl acetate. The organic layer was washed with water and brine and then dried over MgSO₄. The solution is the filtered, concentrated and the residue is HPLC chromatographed on silica gel (85% EtOAc/Hex) to give 400 mg of example 8A. MS: (CI, NH₃) (M+H)⁺ = 523.4.

### Example 7Z

A solution of example 8A (100 mg, 0.2 mmol) in MeOH is cooled in an ice bath and treated with HCl _{(g)} for 20 min and then stirred for an additional 40 min at 0°C. The solution is then evaporated to dryness at rt and the residue is HPLC chromatographed on silica gel (80% EtOAc/Hex) to give 48 mg of example 7Z as a white foam. MS: (CI, NH₃) (M+H)⁺ = 435.2

### Example 8C

A solution of example 8A (160 mg, 0.3 mmol) in methylene chloride (10 ml) is cooled to 0°C in an ice bath and treated with DAST (50 mg, 0.3 mmol). The solution is stirred at rt for 15 min and then quenched with water. The organic layer is washed with water and brine and dried over MgSO₄. The solution is filtered, the solvent evaporated and the residue HPLC chromatographed on silica gel (50% EtOAc/Hex) to give 100 mg of example 8C. MS: (CI, NH₃) (M+H)⁺ = 525.4

### Example 8B

A solution of example 8C (70 mg, 0.13 mmol) in MeOH is cooled in an ice bath and treated with HCl (g) for 20 min and then stirred for an additional 40 min at 0°C. The solution is then evaporated to dryness at rt and the residue is HPLC chromatographed on silica gel (80% EtOAc/Hex) to give 40 mg of example 8B as a white foam. MS: (CI, NH₃) (M+H)⁺ = 437.3

### Example 7AA

To a stirred suspension of 750 mg (1.72 mmol) of the diol (1X) in 35 mL of methylene chloride was added 445 mg (3.45 mmol) of diisopropylethylamine and 322 mg (2.59 mmol) of MEM chloride. After stirring 5 days the resulting solution was washed with dilute HCl, brine and was dried with anhydrous MgSO₄. The solvent was removed under reduced pressure and the residue was chromatographed on silica gel. Elution with 50% ethyl acetate in hexanes gave 430 mg (48%) of the mono protected ether (XXVa). MS:523 (M+1,100); NMR (CDCl₃): δ 7.20 (m,10H), 4.96 (s,2H), 4.08 (m,1H), 3.90 (m,2H), 3.61 (m,7H), 3.42 (s,3H), 3.13 (m,4H), 1.99 (m,2H), 0.88 (m,2H), 0.40 (m,4H), 0.06 (m,4H),

To a stirred solution of 78mg (0.15mmol) of Compound (XXVa) in 3 ml of methylene chloride was added 60 mg (0.74 mmol) of sodium acetate and 95 mg (0.44 mmol) of PCC. The resulting suspension was stirred 3 days and was diluted with ether and filtered through florisil. The solvent was removed under reduced pressure and the residue was chromatographed on silica gel. Elution with 2.5% methanol in methylene chloride gave 68 mg (885) of example 7AA. MS:521 (m+1,100); NMR (CDCl₃): δ 7.21 (m,10H), 4.90 (d,1H), 4.70 (dd,2H), 4.10 (t,1H), 3.80-3.37 (m,8H), 3.36 (s,3H), 3.26-2.82 (m,4H), 2.22 (q,1H), 1.03 (m,2H), 0.51 (m,4H), 0.20 (m,4H),

### Oxidation of monoprotected diol: Preparation of (XXVb) :

To a stirred solution of 51 mg (0.10 mmol) of example 7AA in 4 mL of methanol was added 1 mL of concentrated HCl. The resulting solution was stirred 5h and the product was precipitated by adding water. The suspension was extracted with methylene chloride and the combined organic layers were dried with anhydrous MgSO₄. The solvent was removed under reduced pressure and the residue was chromatographed on silica gel. Elution with 33% ethyl acetate in hexanes gave 34 mg (80%) of the ketol (XXVb). MS:433 (m+1,100); NMR (CDCl₃): δ 7.21 (m,10H), 4.82 (m,1H), 4.24 (t,1H), 3.85 (m,1H), 3.74 (d,1H), 3.44 (m,3H), 3.22-2.73 (m,4H), 2.27 (q,1H), 1.01 (m,2H), 0.51 (m,4H), 0.20 (m,4H),

### Example 7AC

To a stirred solution of 37 mg (0.09 mmol) of the ketol (XXVb) in 4 mL of ethanol and 2 mL of water was added 40 mg (0.48 mmol) of methoxylamine hydrochloride. The resulting solution was stirred overnight and the product was precipitated by adding water. The suspension was extracted with methylene chloride and the combined organic layers were dried with anhydrous MgSO₄. The solvent was removed under reduced pressure to give 40 mg (100%) of example 7AC. MS: 462 (m+1,100); NMR (CDCl₃) δ 7.20 (m,10H), 5.30 (m,2H), 4.80 (t,1H), 4.24 (t,1H), 3.77 (m,4H), 3.40 (m,3H), 2.90 (m,4H), 2.25 (dd,1H), 1.03 (m,2H), 0.48 (m,4H), 0.23 (m,2H), 0.12 (m,2H).

### Example 7AB

By substituting hydroxylamine hydrochloride in the above procedure, the desired product can be obtained: MS: 448 (m+1,100); NMR (CDCl₃): δ 8.13 (s,1H), 7.20 (m,10H), 5.42 (t,1H), 4.83 (t,1H), 3.75 (m,2H), 3.40 (m,3H), 2.94 (m,4H), 2.22 (dd,1H), 1.17 (m,1H), 0.90 (m,1H), 0.47 (m,4H), 0.23 (m,2H), 0.11 (m,2H).

### Example 7AD

To a stirred solution of 98 mg (0.23 mmol) of ketol (XXVb) in 5 mL of butanol was added 57 mg (0.71 mmol) of formamidine hydrochloride and 40 mg (0.75 mmol) of sodium methoxide. The resulting suspension was stirred 30 min and was refluxed overnight. The butanol was removed under reduced pressure and water was added. The suspension was extracted with methylene chloride and the combined organic layers were dried with anhydrous MgSO₄. The solvent was removed under reduced pressure and the residue was chromatographed on silica gel. Elution with 25% ethyl acetate in hexanes gave 28 mg (28%) of example 7AD. MS:442 (m+1,100); NMR (CDCl₃): δ 7.58 (s,1H), 7.20 (m,10H), 4.55 (m,2H), 3.51 (m,2H), 3.24 (m,2H), 3.05 (m,2H), 2.53 (q,1H), 2.27 (q,1H), 0.91 (m,2H), 0.45 (m,4H), 0.11 (m,4H).

### Examples 7AE and 7AF

To a stirred solution of 65 mg (0.15 mmol) of the diol (1X) in 1mL of DMF was added 5.5 mg (0.18mmol) of 80% sodium hydride. The resulting suspension was stirred 20 min. and 68 mg (0.48 mmol) of methyl iodide was added. After stirring overnight, the suspension was quenched with water, extracted with ethyl acetate, and the combined organic layers were dried with anhydrous MgSO₄. The solvent was removed under reduced pressure and the residue was chromatographed on silica gel. Elution with 25% ethyl acetate in hexanes gave 19 mg (28%) of example 7AE along with 25 mg (37%) of Example 7AF.
Example 7AE: MS: 449 (m+1,100); NMR (CDCl₃): δ 7.26 (m,10H), 4.05 (dd,1H), 3.84 (m,1H), 3.67 (m,2H), 3.60 (s,3H), 3.53 (m,2H), 3.13 (m,3H), 2.90 (m,2H), 1.94 (dt,2H), 0.89 (m,2H), 0.41 (m,4H), 0.04 (m,4H),
Example 7AF: MS: 463 (m+1,100); NMR (CDCl₃): δ 7.21 (m,10H), 3.67 (m,4H), 3.62 (s,6H), 3.58 (s,2H), 3.10 (m,4H), 1.92 (dd,2H), 0.83 (m,2H), 0.41 (m,4H), 0.04 (m,4H).

### Examples 7AG and 7AH

By substituting benzyloxymethyl chloride in the above procedure, example 7AG and 7AH were obtained.
Example 7AG: MS: 615 (m+1,100); ¹H NMR (CDCl₃): δ 7.50-7.06 (m,20H), 4.88 (ab,4H), 3.92 (s,2H), 3.56 (m,2H), 3.47 (dd,2H), 3.14 (m,4H), 1.88 (dd,2H), 0.62 (m,2H), 0.34 (m,4H), 0.05 (m,4H).
Example 7AH: MS: 525 (m+1,100); ¹H NMR (CDCl₃): δ 7.45-7.10 (m,15H), 4.74 (ab,2H), 4.13 (dd,1H), 3.82-3.50 (m,5H), 3.09 (m,4H), 2.76 (s,1H), 1.95 (dt,2H), 0.92 (m,2H), 0.40 (m,4H), 0.03 (m,4H).

### Examples 7AI and 7AJ

By substituting allyl bromide in the above procedure, example 7AI and 7AJ were obtained.
Example 7AI: ¹H NMR (CDCl₃) δ 7.26 (m,10H), 6.05 (m,2H), 5.30 (dd,4H), 4.28 (m,2H), 3.76 (s,2H), 3.60 (m,4H), 3.10 (m,4H), 1.93 (dd,2H), 0.86 (m,2H), 0.40 (m,4H), 0.01 (m,4H).
Example 7AJ: MS: 475 (m+1,100); ¹H NMR (CDCl₃) δ 7.27 (m,10H), 6.01 (m,1H), 5.32 (dd,2H), 4.34 (dd,1H), 4.18 (dd.1H), 4.66 (m,5H), 3.10 (m,4H), 2.82 (s,1H), 1.95 (m,2H), 0.85 (m,2H), 0.40 (m,4H), 0.04 (m,4H).

### Example 8E

A solution of Example 5F (500 mg, 0.7 mmol) in methylene chloride (10 ml) is cooled to 0°C in an ice bath and treated with DAST (112 mg, 0.7 mmol). The solution is stirred at 0°C for 15 min and then quenched with sat'd NaHCO₃. The organic layer is washed with water and brine and dried over MgSO₄. The solution is filtered, the solvent evaporated and the residue HPLC chromatographed on silica gel (50% EtOAc/Hex) to give 250 mg of example 8E as a white foam. MS: (CI, NH₃) (M+H)⁺ = 721

### Example 8F

A solution of example 8E (200 mg, 0.28 mmol) in MeOH is cooled in an ice bath and treated with gaseous HCl for 20 min and then stirred for an additional 40 min at 0°C. The solution is then evaporated to dryness at rt and the residue is HPLC chromatographed on silica gel (5% MeOH/CHCl₃) to give 120 mg of example 8F as a white foam. MS: (CI, NH₃) (M+H)⁺ = 541

### Example 8G (via Alkene Intermediate XXIX):

A solution of Example 7O (150 mg, 0.22 mmol) in DMF was treated with sodium iodide (160 mg, 1.1 mmol) and heated at 90°C for 2 hrs. The mixture is cooled to room temperature, diluted with water and the precipitate is extracted into CH₂Cl₂. The extract is washed with water and brine, dried over MgSO₄ and evaporated to give a yellow oil. This is HPLC chromatographed on silica gel (50% EtOAc/Hex) to give 50 mg of alkene intermediate (XXIX) as a white solid. MS: (CI, NH₃) (M+H)⁺ = 589 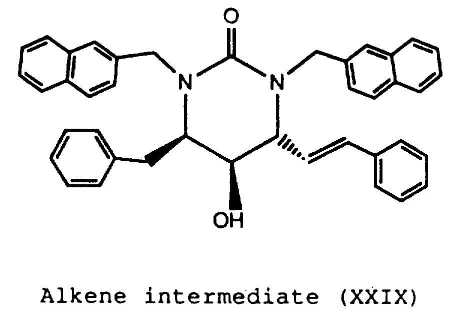 A solution of alkene intermediate (XXIX) (40 mg, 0.07 mmol) in THF was treated with 20 mg of 10% Pd/C and hydrogenated in a Parr Hydrogenator at 50 psi overnight. The catalyst was filtered off and the filtrate concentrated. The resulting residue was HPLC chromatographed on silica gel (70% EtOAc/Hex) to give 10 mg of Example 8G as a white solid. MS: (CI, NH₃) (M+H)⁺ = 591.5

### Example 8H

### Method 1.

### A. Synthesis of 6-membered ring cyclic urea (XXX):

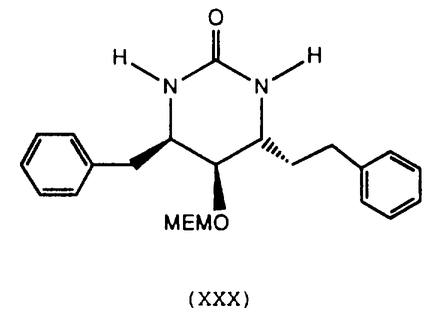

The synthesis of the six-membered cyclic urea (XXX) is outlined in Scheme 8. A solution of N-Cbz-D-phenylalanine N,O-dimethylhydroxylamide (33.5 g , 0.098 mol) in ether was cooled to 0°C and treated with 300 mL of a 1 M solution of vinyl magnesium bromide in THF. The mixture was stirred for 30 mins and then poured into an ice cold solution of 1 N HCl (500 mL). The mixture was extracted into ether and the extracts washed with water and brine. The organic layer was dried over MgSO₄, filtered and concentrated to give the desired vinyl ketone as a thick, light yellow residue which was used without further purification. MS: (CI, NH₃) (M+H)⁺ = 310 (77%); (M+NH₄)⁺ = 327.1 (100%).

The crude ketone was dissolved in methanol (350 mL) and treated with cerium trichloride heptahydrate (37.2 g, 0.1 mol) and cooled in an ice bath. While stirring vigorously sodium borohydride (3.78 g 0.1 mol) was added slowly, a small portion at a time, over a period of 30 min. After the addition was complete the mixture was allowed to warm to room temperature and stirred for an additional 1 hr. The solvent was removed under vacuum on a rotorary evaporator and the residue was partitioned between 1 N HCl and methylene chloride. The organic layer was washed with water, brine, and then dried over MgSO₄, filtered and concentrated to give the desired allylic alcohol as an off-white solid which was used without further purification.

A solution of the crude allylic alcohol and diisopropylethylamine (30 g, 0.23 mol) in methylene chloride was cooled in an ice bath and treated dropwise with methanesulfonyl chloride (28 g, 0.24 mol). The solution was stirred for 30 mins, then washed sequentially with 1 N HCl, water, brine and dried over MgSO₄. The solution was filtered and concentrated to give the crude mesylate as a thick oil. To a flamed-dried flask was added copper cyanide (12 g, 0.144 mol) and 100 mL of THF. The flask was cooled to -78 °C under nitrogen atmosphere. A solution of benzylmagnesium chloride (360 mL, 2M in THF, 0.72 mol) was added via syringe and the resulting thick solution was stirred at -60°C for 20 mins and at 0 °C for 30 mins. The solution was then cooled to - 78°C and a solution of the mesylate in 130 mL of THF was added via syringe. The solution was stirred at -60°C for 45 mins and then poured into a mixture of 1 N HCl/ice. This was extracted into ethyl acetate and the organic layer was washed sequentially with NH₄Cl (aq), NH₄OH, brine, dried over MgSO₄, filtered and concentrated. The resulting residue is chromatographed on silica gel (hexane, then 10% EtOAc/Hex) to give 11.7 g of the desire alkene as a white solid. MS: (CI, NH₃) (M+H)⁺ = 386.3 (98%); (M+NH₄)⁺ = 403.2 (100%).

A solution of the above alkene (11.0 g, 0.029 mol) in methylene chloride (75 mL) was cooled to 0°C in an ice bath and treated with 60% m-chloroperbenzoic acid (14.0 g, 0.049 mol). The solution was stirred 0°C for 7 hrs until TLC analysis showed no starting material remained. A precipitate formed during this time. The suspension was diluted with methylene chloride and washed sequentially with 1 N Na₂S₂O₃, 1 N sodium hydroxide, water, brine, dried over MgSO₄, filtered and concentrated to give the epoxide as a thick oil which was used without further purification.

To solution of crude epoxide in 80 mL of DMF was added sodium azide (20 g , 0.3 mol), ammonium chloride (2.5 g , 0.047 mol) and 20 mL of water. The mixture was heated at 90 °C for 3 hrs and then stirred at rt overnight. The solvent was removed under high vacuum on a rotorary evaporator and the residue was partitioned between water and methylene chloride. the organic layer was washed with water and brine, dried over MgSO₄, filtered and concentrated to give a residue. This was then chromatographed on silica gel (20% EtOAc/Hex) to give 7.4 g of the azide alcohol as a white solid. MS: (CI, NH₃) (M+H)⁺ = 445.0 (25%); (M+NH₄-BnOH)⁺ = 462.2 (100%).

A solution of the azide alcohol above (7.2 g, 0.016 mol) in methylene chloride was treated with diisopropylethylamine (4.2 g , 0.032 mol) and MEM-Cl (4.0 g 0.032 mol) and heated to reflux overnight (18 hrs). The mixture was concentrated and the residue chromatographed on silica gel (20% EtOAc/Hex - 35% EtOAc/Hex) to give 7.7 g of the MEM protected azido alcohol as a colorless oil. MS: (CI, NH₃) (M+H)⁺ = 533.2 (100%).

To a solution of MEM protected azido alcohol (5.7 g 0.0107 mol) in ethyl acetate was added 2 mL of acetic acid and 1 g of Pearlman's catalyst (10 % Pd(OH)₂ on Carbon) and the solution was hydrogenated at 55 psi for 22 hrs. The solution was filtered through Celite and the filtrate was extracted with 1 N HCl (organic layer turn orange). The acidic aqueous extract was made basic with 50% NaOH (while cooling in an ice bath) and the precipitate is extracted into ethyl acetate. The organic layer is washed with water, brine, dried over MgSO₄, filtered and concentrated to give 2.5 g of the MEM protected diamino alcohol as a colorless oil. MS: (CI, NH₃) (M+H)⁺ = 373.1 (100%).

To a solution of the MEM protected diamino alcohol (2.5 g 0.0067 mol) in THF was added 1,1-carbonyldiimidazole (1.1 g, 0.0067 mol) and stirred over night at room temperature. The solution was concentrated and the residue partitioned between 1 N HCl and CH₂Cl₂. The organic layer is washed with brine, dried over MgSO₄, filtered and concentrated. The residue is HPLC chromatographed on silica gel ( 5% MeOH/CHCl₃) to give 1.2 g of the MEM protected 6-membered ring cyclic urea (XXX) as a white solid. MS: (CI, NH₃) (M+H)⁺ = 399.1 (100%).

B. The MEM protected 6-membered ring cyclic urea (XXX) (100 mg, 0.27 mmol) was alkylated with cyclopropylmethylbromide (250 mg , 1.8 mmol) followed by removal of the MEM group, as described in general procedure 5, to give after chromatography on HPLC (silica gel, 10% MeOH/CHCl₃) 20 mg of Example 8H as a clear, viscous residue. MS: (CI, NH₃) (M+H)⁺ = 419.4 (100%).

### Method 2.

A solution of Example 8A (160 mg, 0.3 mmol) and thiocarbonyldiimidazole (55 mg, 0.3 mmol) in THF was heated to reflux for 4 hrs. The mixture was evaporated and the residue chromatographed on silica gel (50% EtOAc/Hex) to give 34 mg (0.055 mmol) of the corresponding thiocarbamate. The thiocarbamate was dissolved in 2 mL of toluene and heated to reflux. To the refluxing solution was added tributyltin hydride (32 mg, 0.1 mmol) and 2 mg of AIBN. The mixture was refluxed for 1 hour, concentrated, and the residue chromatography on HPLC (silica gel, 65% EtOAc/Hex) to give 20 mg of clear colorless oil. The oil was dissolved in MeOH, cooled in an ice bath and gaseous HCl was bubbled through the solution for 30 mins. The solution was then stirred at room temperature overnight, concentrated and the residue chromatography on HPLC (silica gel, 10% MeOH/CHCl₃) to give 10 mg of Example 8H as a clear, viscous residue. MS: (CI, NH₃) (M+H)⁺ = 419.2 (100%).

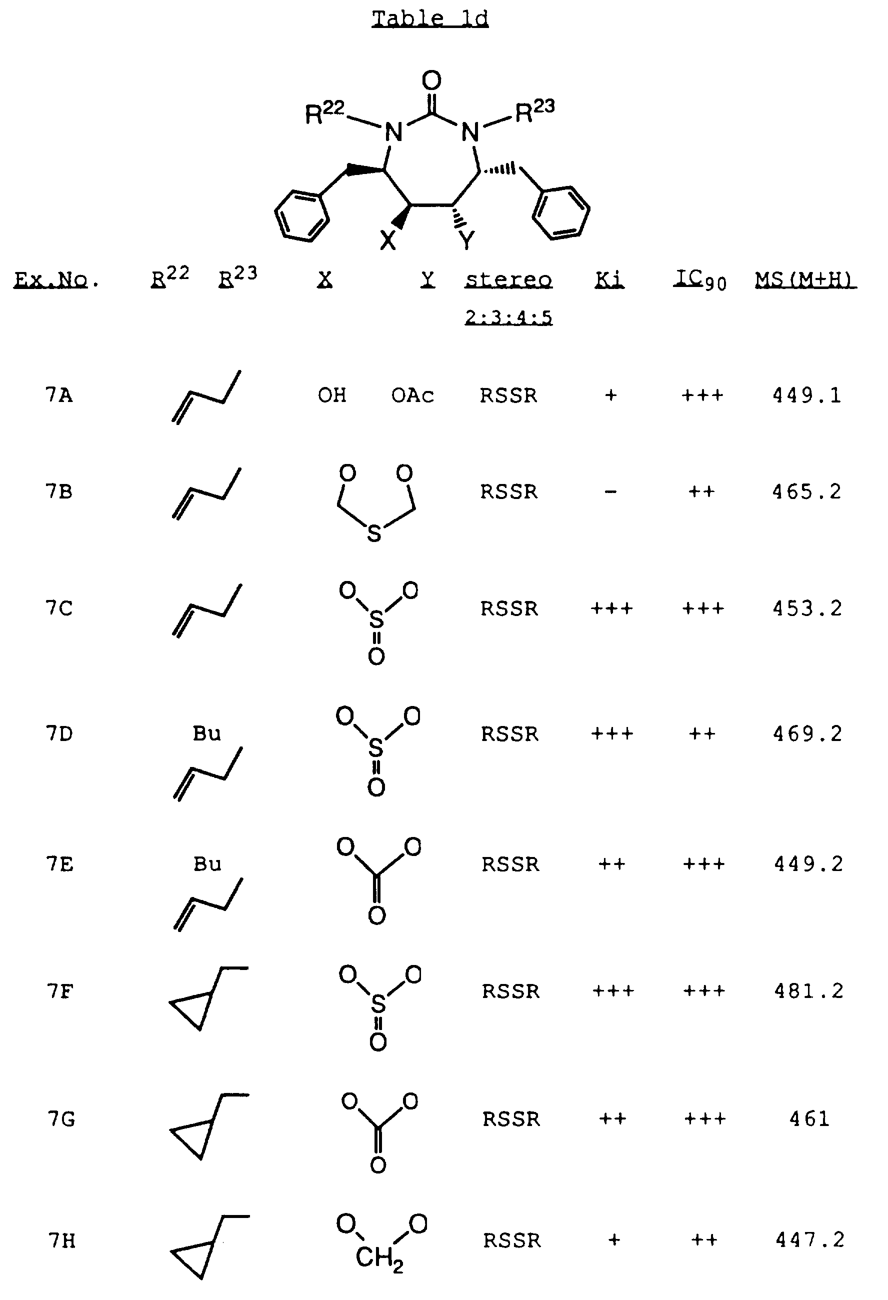

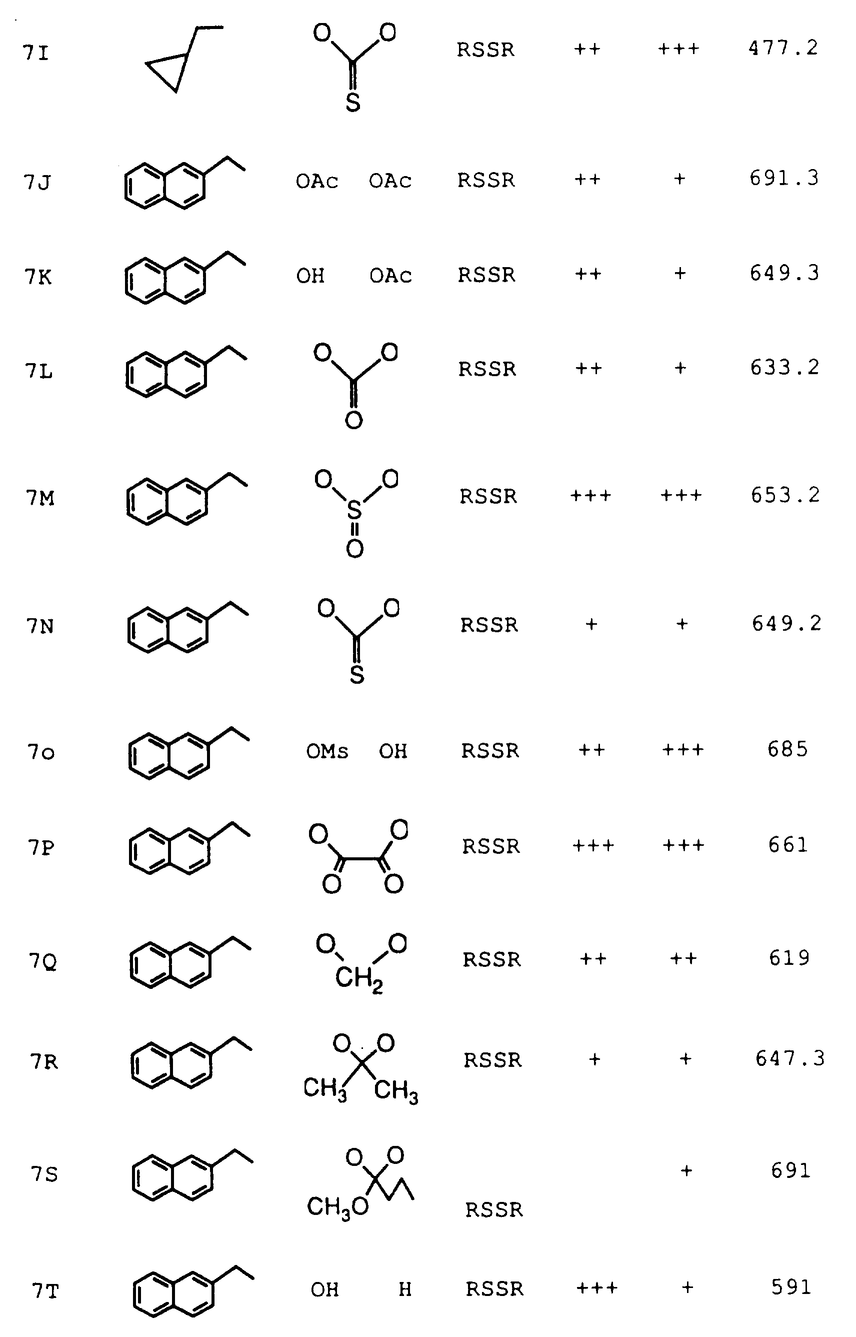

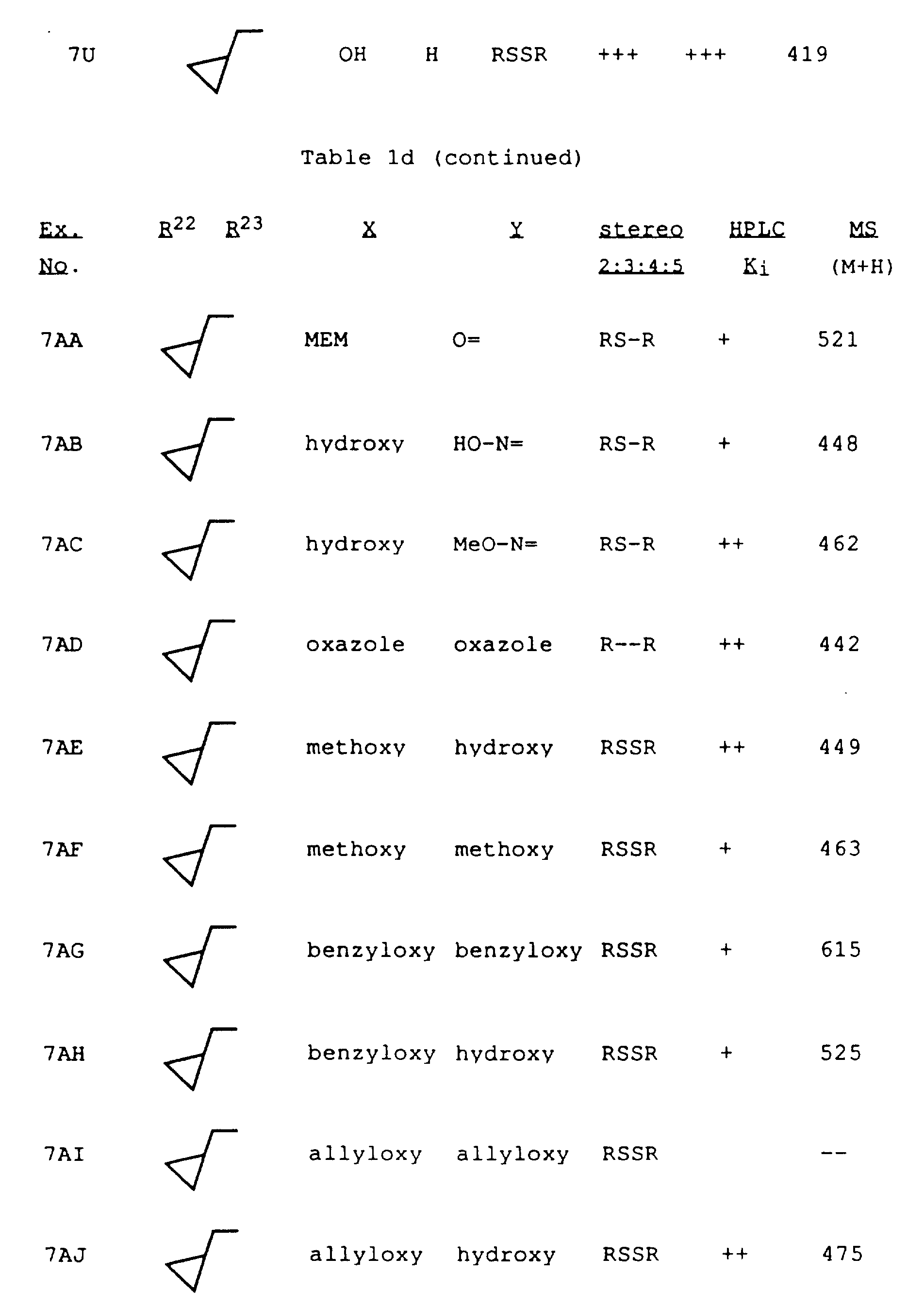

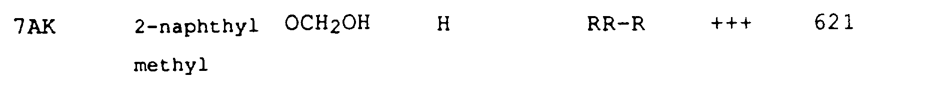

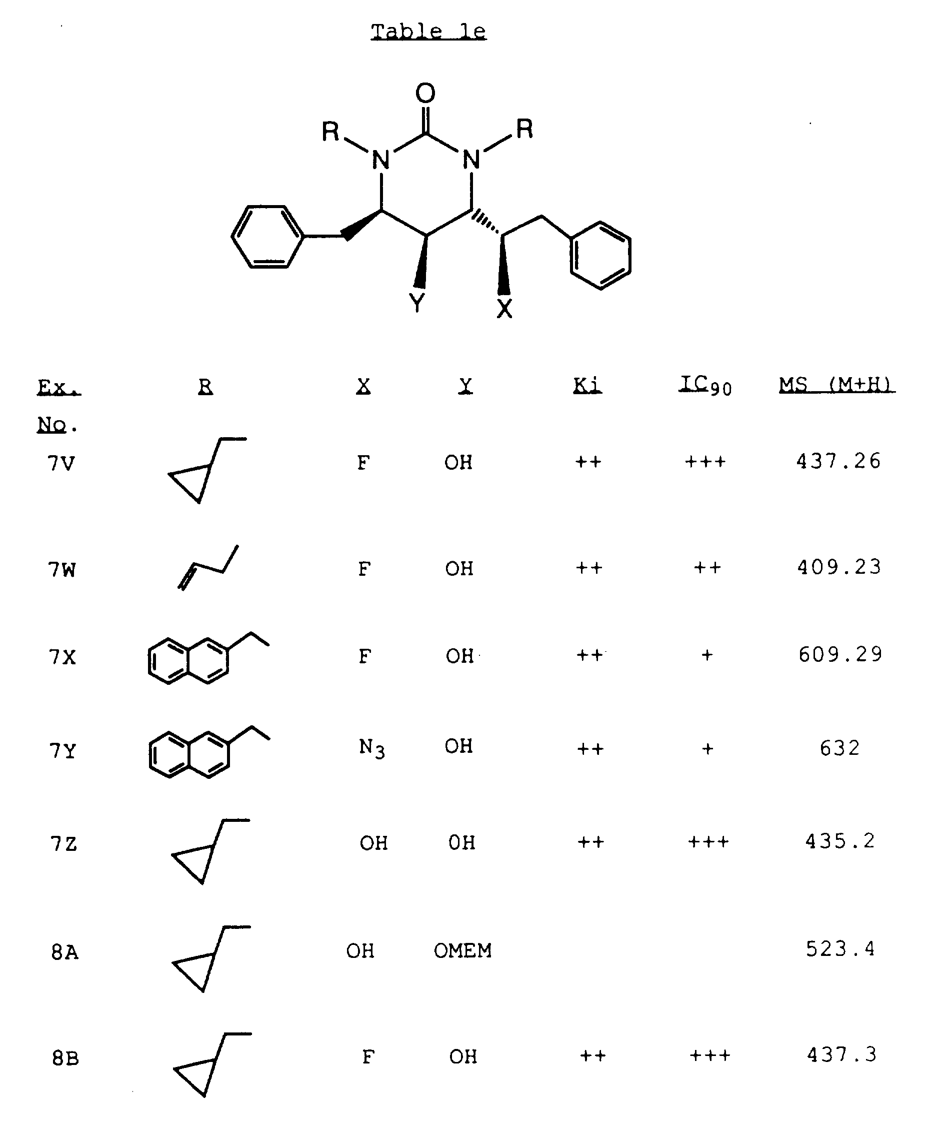

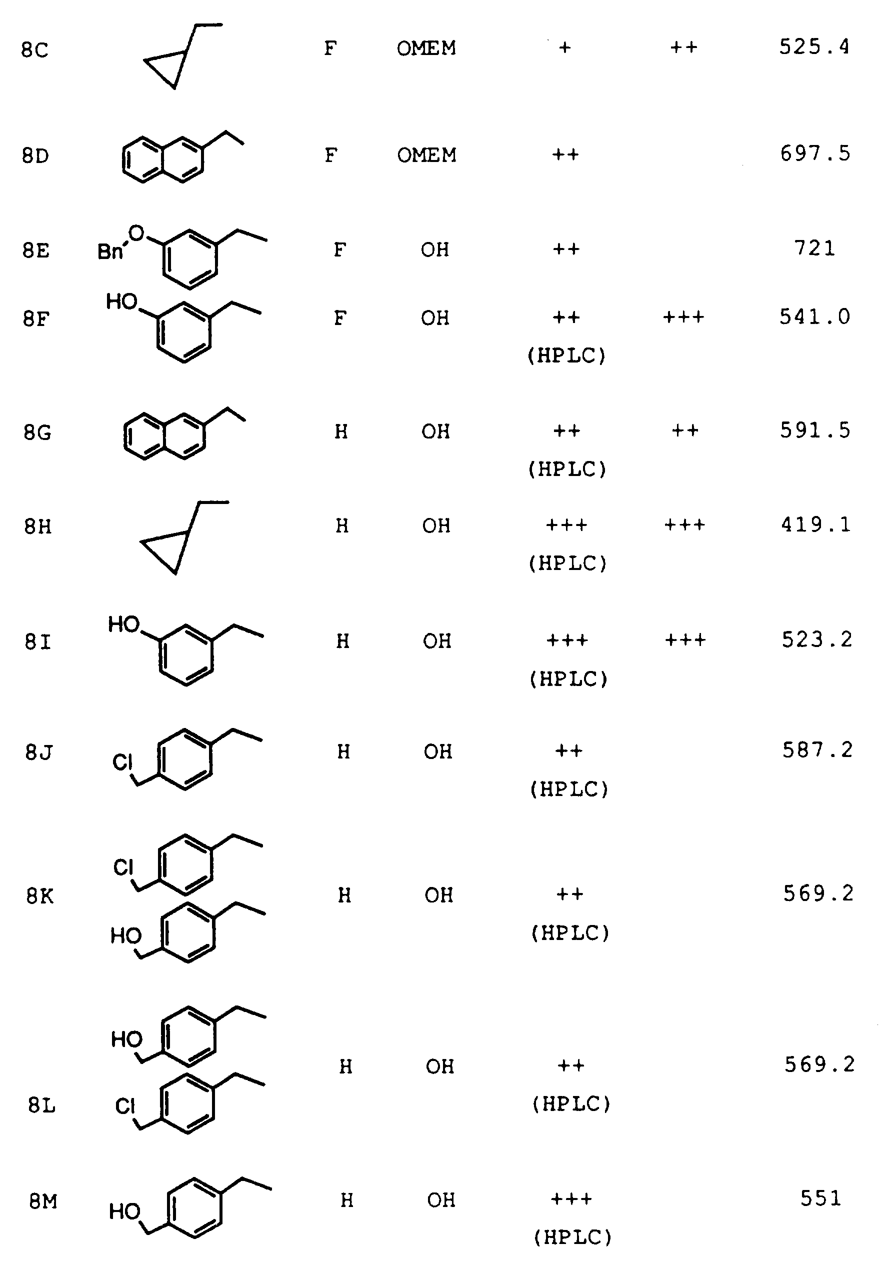

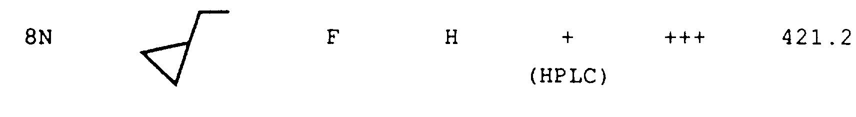

Using the above-described techniques or variations thereon appreciated by those of skill in the art of chemical synthesis, the compounds of Tables 3-13 (shown below) can also be prepared.

### Utility

The compounds of formula (I) possess retroviral protease inhibitory activity and are therefore useful as antiviral agents for the treatment of viral diseases. More particularly, the compounds of formula (I) possess HIV protease inhibitory activity and are effective as inhibitors of HIV growth. The protease inhibitory activity of the compounds of the present invention is demonstrated using standard assays of protease activity, for example, using the assay described below for assaying inhibitors of HIV protease activity. The ability of the compounds of the present invention to inhibit viral growth or infectivity is demonstrated in standard assay of viral growth or infectivity, for example, using the assays described below.

A compound is considered to be active if it has an IC₅₀ or Ki value of less than about 1 mM for the inhibition of HIV protease or HIV viral growth or infectivity.

### HIV Protease Inhibition Assay- Spectroscopic Method

### Materials:

Protease: Inclusion bodies of E. coli harboring plasmid containing HIV protease under the control of an inducible T7 promoter were prepared according to Cheng et. al (1990) Gene 87: 243. Inclusion bodies were solubilized in 8 M urea, 50 mM tris pH 8.0. Protease activity was recovered by dilution 20-fold into buffer containing 50 mM sodium acetate pH 5.5, 1 mM EDTA, 10% glycerol and 5% ethylene glycol. Enzyme was used at a final concentration of 1.0-10 µg/ml.

Substrate: Peptide of sequence: Ala-Thr-His-Gln-Val-Tyr-Phe(NO₂)-Val-Arg-Lys-Ala, containing ρ-nitrophenylalanine (Phe(NO₂)), was prepared by solid phase peptide synthesis as previously described by Cheng et al. (1990) Proc. Natl. Acad. Sci. USA 87: 9660. Stock solutions of 10 mM were prepared in DMSO.

Inhibitory compounds were dissolved in sufficient DMSO to make 2.5 or 25 mM stock solutions. All further dilutions were done in DMSO.

### Reactions:

Compound (1-5 µL) and HIV protease were mixed in buffer containing 50 mM MES, pH 6.5, 1 M NaCl, 1 mM EDTA, 1 mM dithiothreitol, 10% glycerol. Reactions were initiated by the addition of peptide substrate to a final concentration of 240 µM, and absorbance at 300 nM monitored for 10 min. Values of Ki for inhibitor binding were determined from percent activity measurements in the presence and absence of known concentration of inhibitor, using a value of 0.07 mM for the Km of the substrate (Cheng et al. (1990) Proc. Natl. Acad. Sci. USA 87: 9660).

The HIV-1 protease inhibitory activity of representative compounds of the invention is shown in Table 1 and 2.

### HIV Protease Inhibition Assay- HPLC Method

### Materials:

Protease: Inclusion bodies of E. coli harboring plasmid containing plasmid T1718R with a synthetic gene coding for a single-chain tethered dimer of HIV protease were prepared as described in Cheng et al. (Proc. Natl. Acad. Sci. USA, 87, 9660-9664, 1990). Active protease was prepared as described therein by extraction with 67% acetic acid, dilution 33-fold with water, dialysis against water and then against a "refolding buffer" consisting of 20 mM MES, 1 mM dithiothreitol and 10% glycerol. Protease was stored as a stock preparation at 10 µM in refolding buffer.

Substrate: Peptide of sequence: aminobenzoyl-Ala-Thr-His-Gln-Val-Tyr-Phe(NO₂)-Val-Arg-Lys-Ala containing p-nitrophenylalanine, was prepared by solid phase synthesis as previously described Cheng et al., op.cit. Stock solutions of 2 mM substrate were prepared in DMSO.

Inhibitory compounds were dissolved in sufficient DMSO to make 3 mM stock solutions. All further dilutions were prepared in "assay buffer": 1 M NaCl, 50 mM MES, pH 5.5, 1 mM EDTA, 1mM DTT, 20% glycerol.

### Reactions:

Enzyme reaction: In a 2 ml screw-cap centrifuge tube were added 50ul protease (final concentration 0.25 nM) and 0.1 ml inhibitory compound (final concentration 0.1-12,500). After 15 min preincubation at room temperature, the reaction was started with the addition of .05 ml substrate (final concentration 5 µM). Incubation was carried out at 30 C. for 1 hr. The reaction was stopped with 1 ml 0.1 M ammonium hydroxide.

HPLC measurement of product formation: The product (aminobenzoyl-Ala-Thr-His-Gln-Val-Tyr) was separated from substrate on a Pharmacia MonoQ anion exchange column. The injection volume was 0.2 ml. The mobile phases were: A (20 mM trisHCl, pH 9.0, 0.02% sodium Azide, 10% acetonitrile), B (20 mM tris HCl, pH 9.0, 0.02% sodium azide, 0.5 M ammonium formate, 10% acetonitrile). The mobile phases were pumped at 1 ml/min, with a gradient from 0 to 30% B in 5 min, 100 % B for 4 min to wash the column, and a re-equilibration for 4 min. The retention time of the product was 3.6 min. Detection with a Shimadzu model RF535 fluorescence monitor was at 330 nm (excitation) and 430 (emission). The Ki was calculated from the formula Ki = I/(((Km+S-FA*S)/(FA*Km))-1) where I = inhibitory concentration, S = substrate concentration, FA = fractional activity = cm peak height with inhibitor/cm peak height without inhibitor, and Km = Michaelis constant = 20 µM.

### HIV Yield Reduction Cell Assay

Materials: MT-2, a human T-cell line, was cultured in RPMI medium supplemented with 5% (v/v) heat inactivated fetal calf serum (FCS), L-glutamine and gentamycin. Human immunodeficiency virus strains, HIV (3B) and HIV(RF) were propagated in H-9 cells in RPMI with 5% FCS. Poly-L-lysine (Sigma) coated cell culture plates were prepared according to the method of Harada et al. (1985) Science 229: 563-566. MTT, 3-(4,5-dimethyl-thiazol-2yl)-2,5-diphenyltetrazolium bromide, was obtained from Sigma.

Method: Test compounds were dissolved in dimethylsulfoxide to 5 mg/mL and serially diluted into RPMI medium to ten times the desired final concentration. MT-2 cells (5 x 10⁵/mL) in 2.3 mL were mixed with 0.3 ml of the appropriate test compound solution and allowed to sit for 30 minutes at room temperature. HIV (3B) or HIV (RF) (~5 x 10⁵ plaque forming units/mL) in 0.375 ml was added to the cell and compound mixtures and incubated for one hour at 36°C. The mixtures were centrifuged at 1000 rpm for 10 minutes and the supernatants containing unattached virus were discarded. The cell pellets were suspended in fresh RPMI containing the appropriate concentrations of test compound and placed in a 36°C, 4% CO₂ incubator. Virus was allowed to replicate for 3 days. Cultures were centrifuged for 10 minutes at 1000 rpm and the supernatants containing cell free progeny virus were removed for plaque assay.

The virus titers of the progeny virus produced in the presence or absence of test compounds were determined by plaque assay. Progeny virus suspensions were serially diluted in RPMI and 1.0 mL of each dilution was added to 9 ml of MT-2 cells in RPMI. Cells and virus were incubated for 3 hours at 36°C to allow for efficient attachment of the virus to cells. Each virus and cell mixture was aliquoted equally to two wells of a six well poly-L-lysine coated culture plate and incubated overnight at 36°C, 4% CO₂. Liquid and unattached cells were removed prior to the addition of 1.5 mL of RPMI with 0.75% (w/v) Seaplaque agarose (FMC Corp.) and 5% FCS. Plates were incubated for 3 days and a second RPMI/agarose overlay was added. After an additional 3 days at 36°C, 4% CO₂, a final overlay of phosphate-buffered saline with 0.75% Seaplaque agarose and 1 mg MTT/mL was added. The plates were incubated overnight at 36°C. Clear plaques on a purple background were counted and the number of plaque forming units of virus was calculated for each sample. The antiviral activity of test compounds was determined by the percent reduction in the virus titer with respect to virus grown in the absence of any inhibitors.

### HIV Low Multiplicity Assay

Materials: MT-2, a human T-cell line, was cultured in RPMI medium supplemented with 5% (v/v) heat inactivated fetal calf serum (FCS), L-glutamine and gentamycin (GIBCO). Human immunodeficiency virus strains HIV (3B) and HIV (RF) were propagated in H-9 cells in RPMI with 5% FCS. XTT, benzene-sulfonic acid, 3,3'-[1-[(phenyl-amino)carbonyl]-3,4-tetrazolium]bis(4-methoxy-6-nitro)-, sodium salt, was obtained from Starks Associates, Inc.

Method: Test compounds were dissolved in dimethylsulfoxide to 5 mg/ml and serially diluted into RPMI medium to ten times the desired final concentration. MT-2 cells (5 x 10⁴/0.1 mL) were added to each well of a 96 well culture plate and 0.02 mL of the appropriate test compound solution was added to the cells such that each compound concentration was present in two wells. The cells and compounds were allowed to sit for 30 minutes at room temperature. HIV(3B) or HIV(RF) (∼5 x 10⁵ plaque forming units/mL) was diluted in medium and added to the cell and compound mixtures to give a multiplicity of infection of 0.01 plaque forming unit/cell. The mixtures were incubated for 7 days at 36°C, during which time the virus replicated and caused the death of unprotected cells. The percentage of cells protected from virus induced cell death was determined by the degree of metabolism of the tetrazolium dye, XTT. In living cells, XTT was metabolized to a colored formazan product which was quantitated spectrophotometrically at 450 nm. The amount of colored formazan was proportional to the number of cells protected from virus by the test compound. The concentration of compound protecting either 50% (IC₅₀) or 90% (IC₉₀) with respect to an uninfected cell culture was determined.

The HIV inhibitory activity of representative compounds of the present invention in the whole cell infectivity assay described above is shown in Table 2.

**Table 2**

| Example Number | IC₉₀ |
|---|---|
| 1C | +++ |
| 1X | +++ |
| 1Z | +++ |
| 1AC | +++ |
| 1AF | +++ |
| 1AH | +++ |
| 1AQ | +++ |
| The IC₉₀ values in Table 2 are indicated as: +++ = <10 µg/mL. | |

In the Tables herein the Ki values were determined using the assay conditions described above under HIV Protease Inhibtion Assay. The Ki values are indicated as follows: +++ = <10 nM; ++ = 10 nM to 1 µM; + = >1 µM.

In the Tables herein the IC₉₀ values were determined using the assay conditions described above under HIV Low Multiplicity Assay. The IC₉₀ values are indicated as follows: +++ = <10 µg/mL; ++ = 10 to 100 µg/mL; + = >100 µg/mL.

### Dosage and Formulation

The antiviral compounds of this invention can be administered as treatment for viral infections by any means that produces contact of the active agent with the agent's site of action, the viral protease, in the body of a mammal. They can be administered by any conventional means available for use in conjunction with pharmaceuticals, either as individual therapeutic agents or in a combination of therapeutic agents. They can be administered alone, but generally administered with a pharmaceutical carrier selected on the basis of the chosen route of administration and standard pharmaceutical practice.

The dosage administered will, of course, vary depending upon known factors, such as the pharmacodynamic characteristics of the particular agent and its mode and route of administration; the age, health and weight of the recipient; the nature and extent of the symptoms; the kind of concurrent treatment; the frequency of treatment; and the effect desired. A daily dosage of active ingredient can be expected to be about 0.001 to 1000 milligrams per kilogram of body weight, with the preferred dose being 0.1 to about 30 mg/kg.

Dosage forms (compositions suitable for administration contain from about 1 milligram to about 100 milligrams of active ingredient per unit. In these pharmaceutical compositions the active ingredient will ordinarily be present in an amount of about 0.5-95% by weight based on the total weight of the composition.

The active ingredient can be administered orally in solid dosage forms, such as capsules, tablets, and powders, or in liquid dosage forms, such as elixirs, syrups, and suspensions. It can also be administered parenterally, in sterile liquid dosage forms.

Gelatin capsules contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propyl-paraben, and chlorobutanol.

Suitable pharmaceutical carriers are described in Remington's Pharmaceutical Sciences, Mack Publishing Company, a standard reference text in this field.

Useful pharmaceutical dosage-forms for administration of the compounds of this invention can be illustrated as follows:

### Capsules

A large number of unit capsules are prepared by filling standard two-piece hard gelatin capsules each with 100 milligrams of powdered active ingredient, 150 milligrams of lactose, 50 milligrams of cellulose, and 6 milligrams magnesium stearate.

### Soft Gelatin Capsules

A mixture of active ingredient in a digestable oil such as soybean oil, cottonseed oil or olive oil is prepared and injected by means of a positive displacement pump into gelatin to form soft gelatin capsules containing 100 milligrams of the active ingredient. The capsules are washed and dried.

### Tablets

A large number of tablets are prepared by conventional procedures so that the dosage unit was 100 milligrams of active ingredient, 0.2 milligrams of colloidal silicon dioxide, 5 milligrams of magnesium stearate, 275 milligrams of microcrystalline cellulose, 11 milligrams of starch and 98.8 milligrams of lactose. Appropriate coatings may be applied to increase palatability or delay absorption.

Tables 3-16 below set forth representative compounds of the present invention.

In Tables 3-12, each of the Examples 1-5763 includes all stereoisomers of the indicated structure.

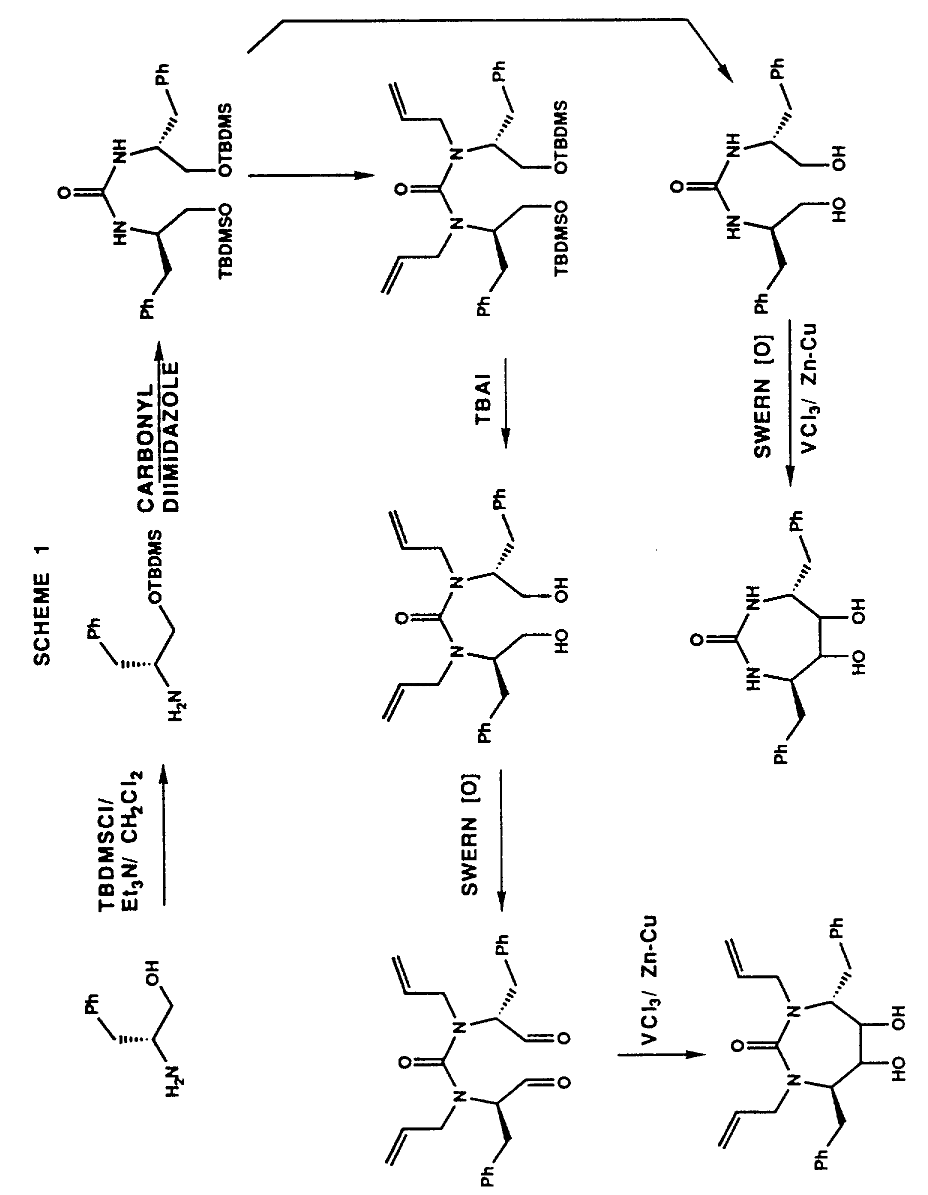

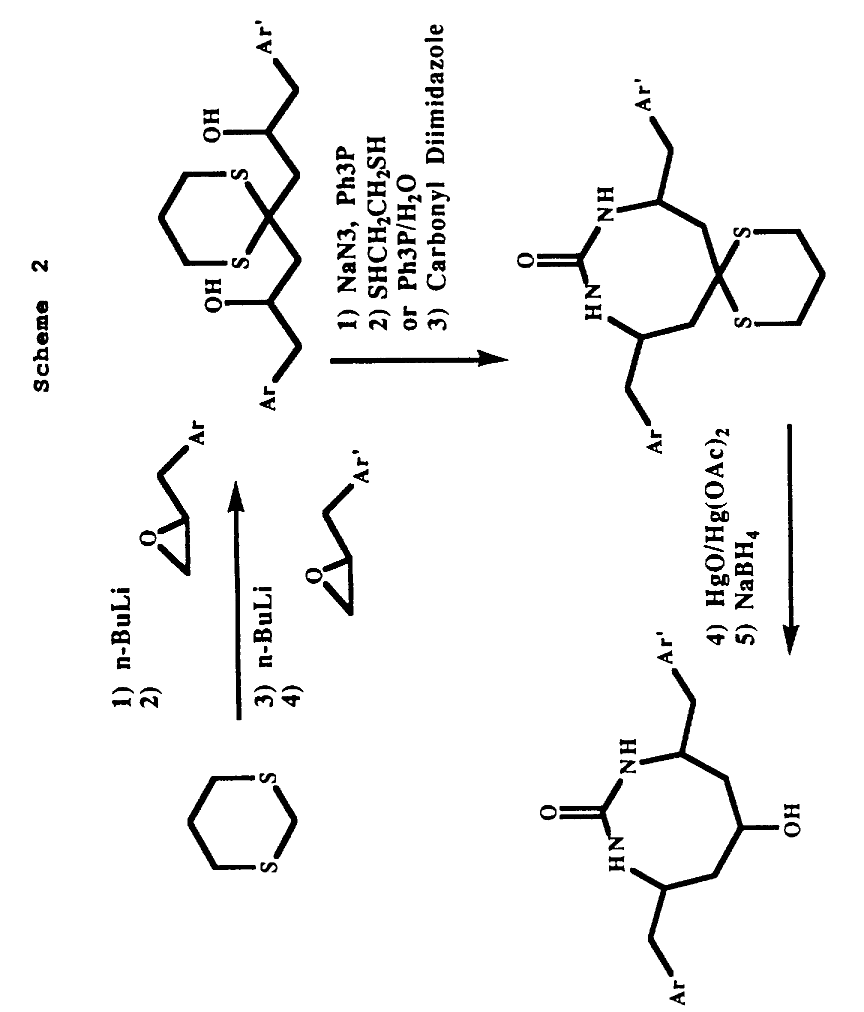

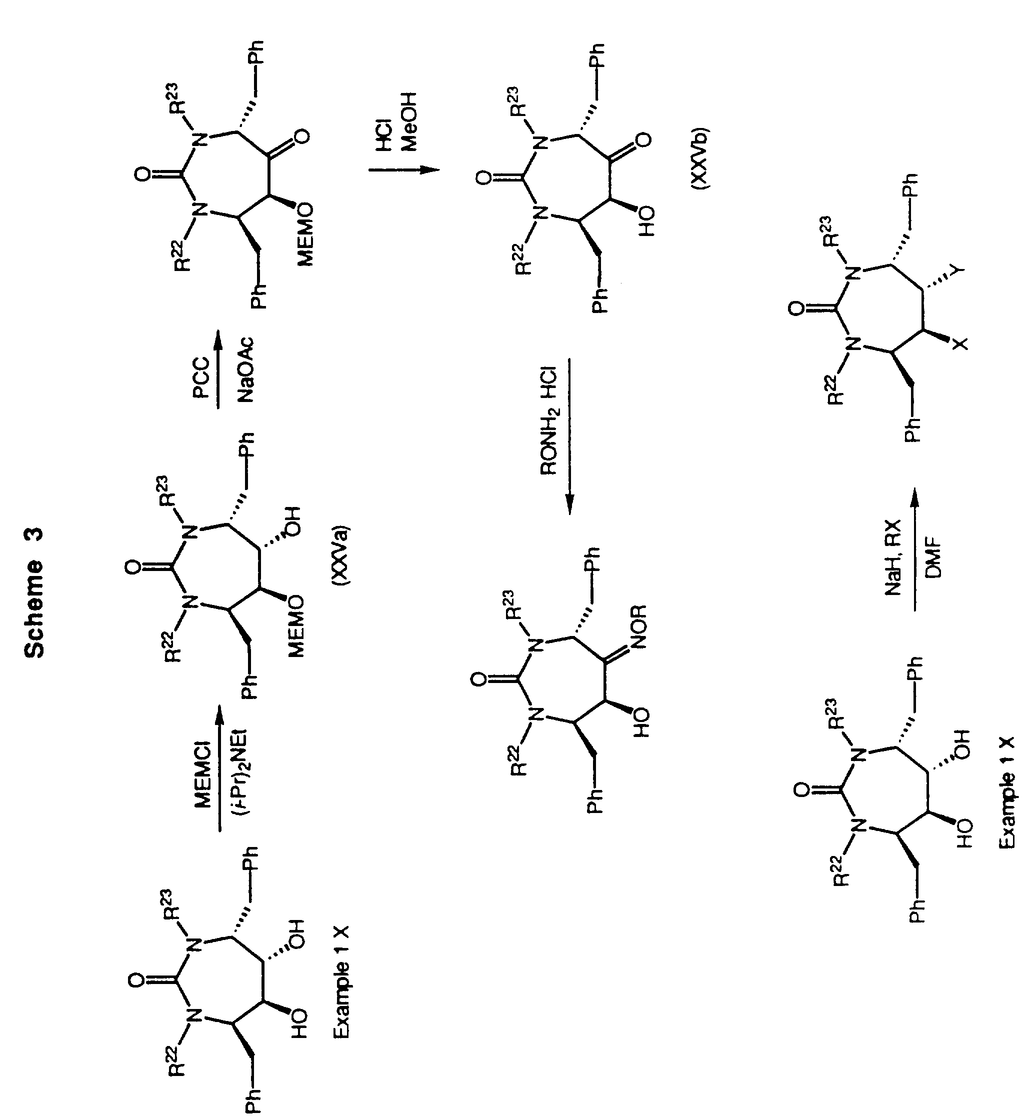

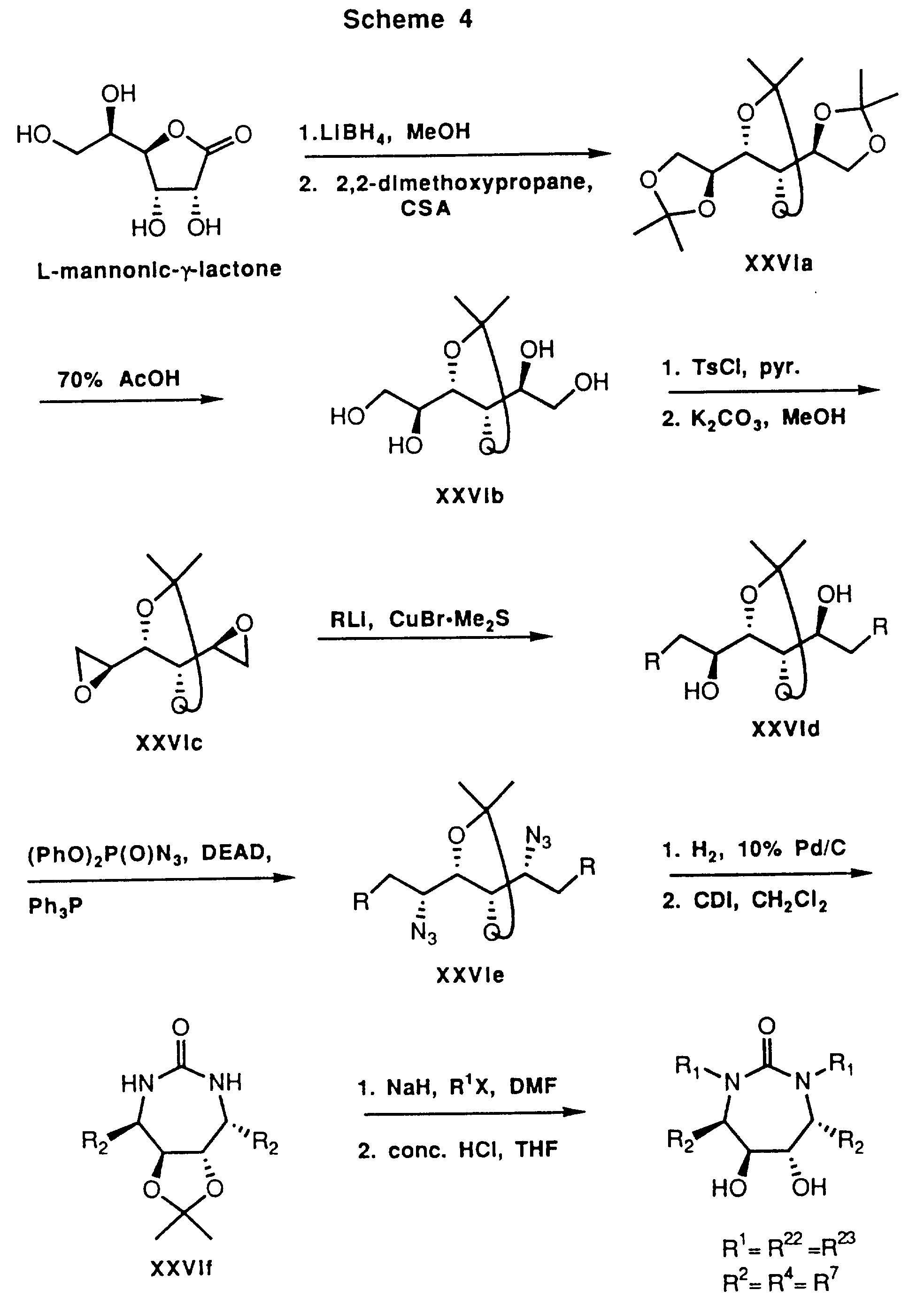

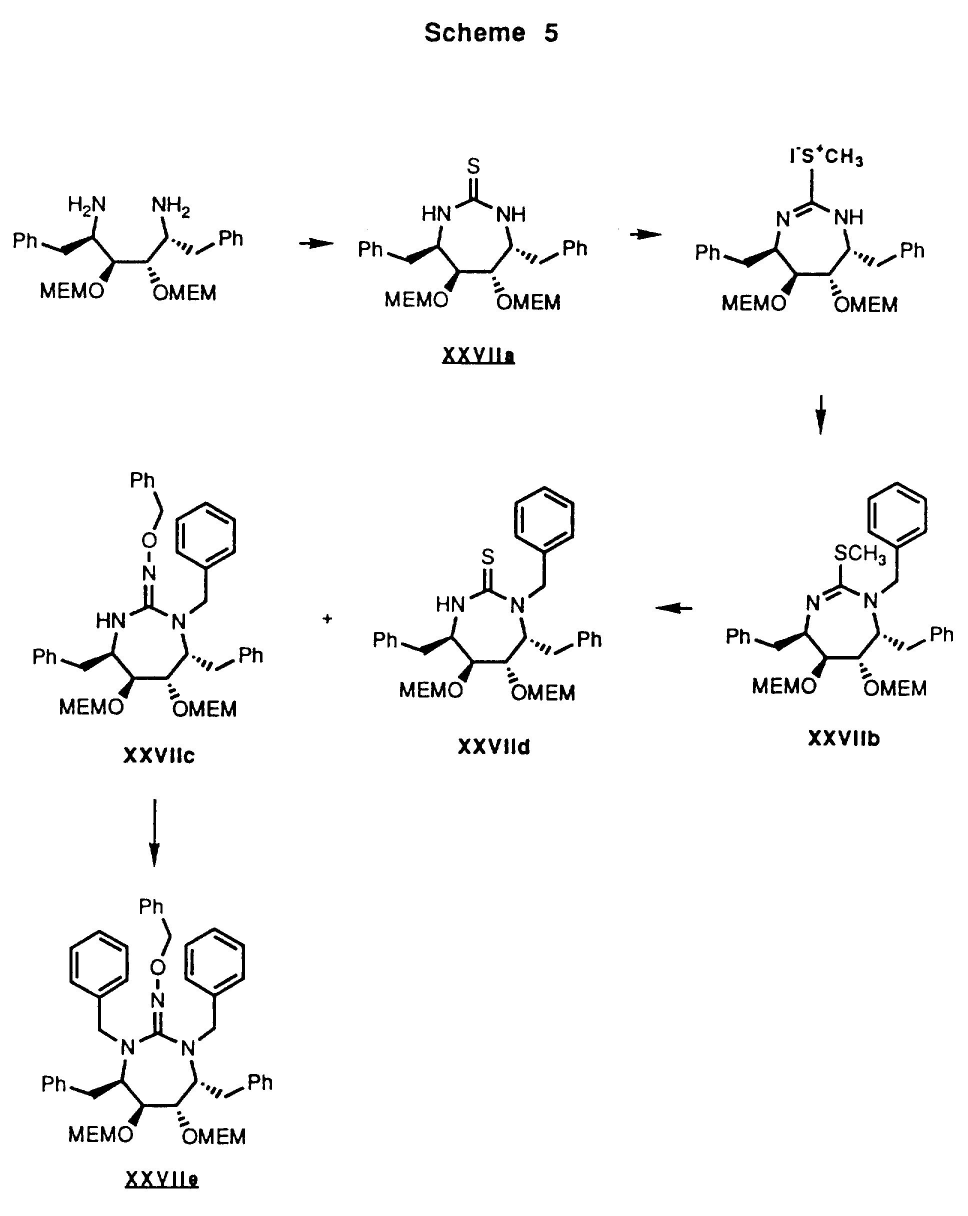

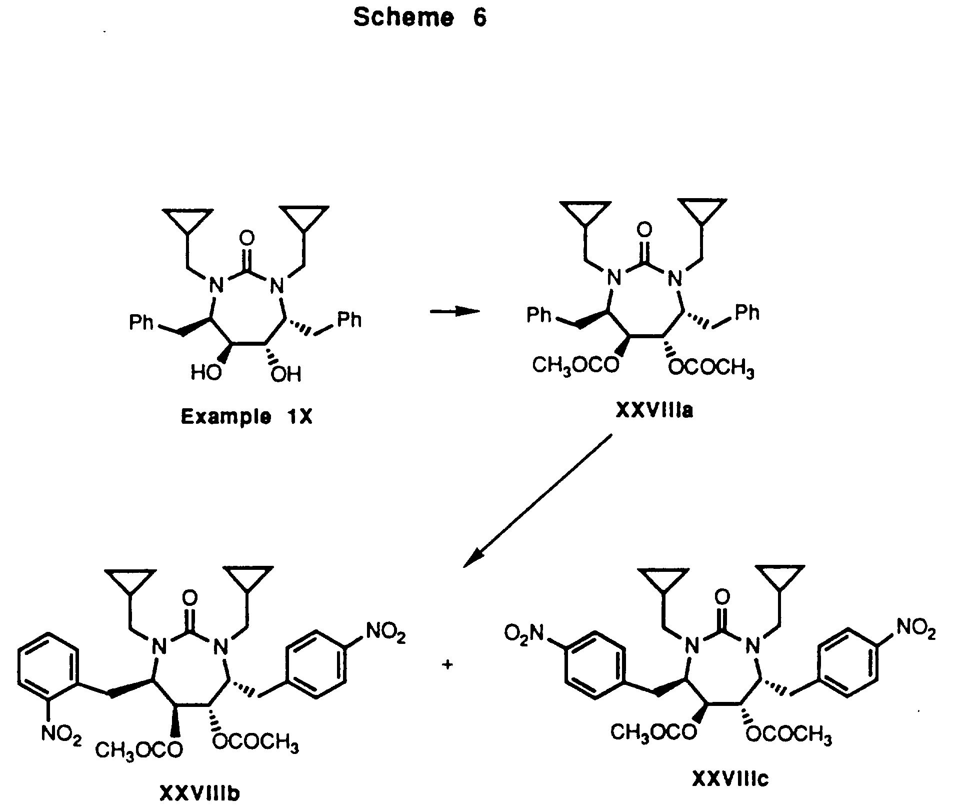

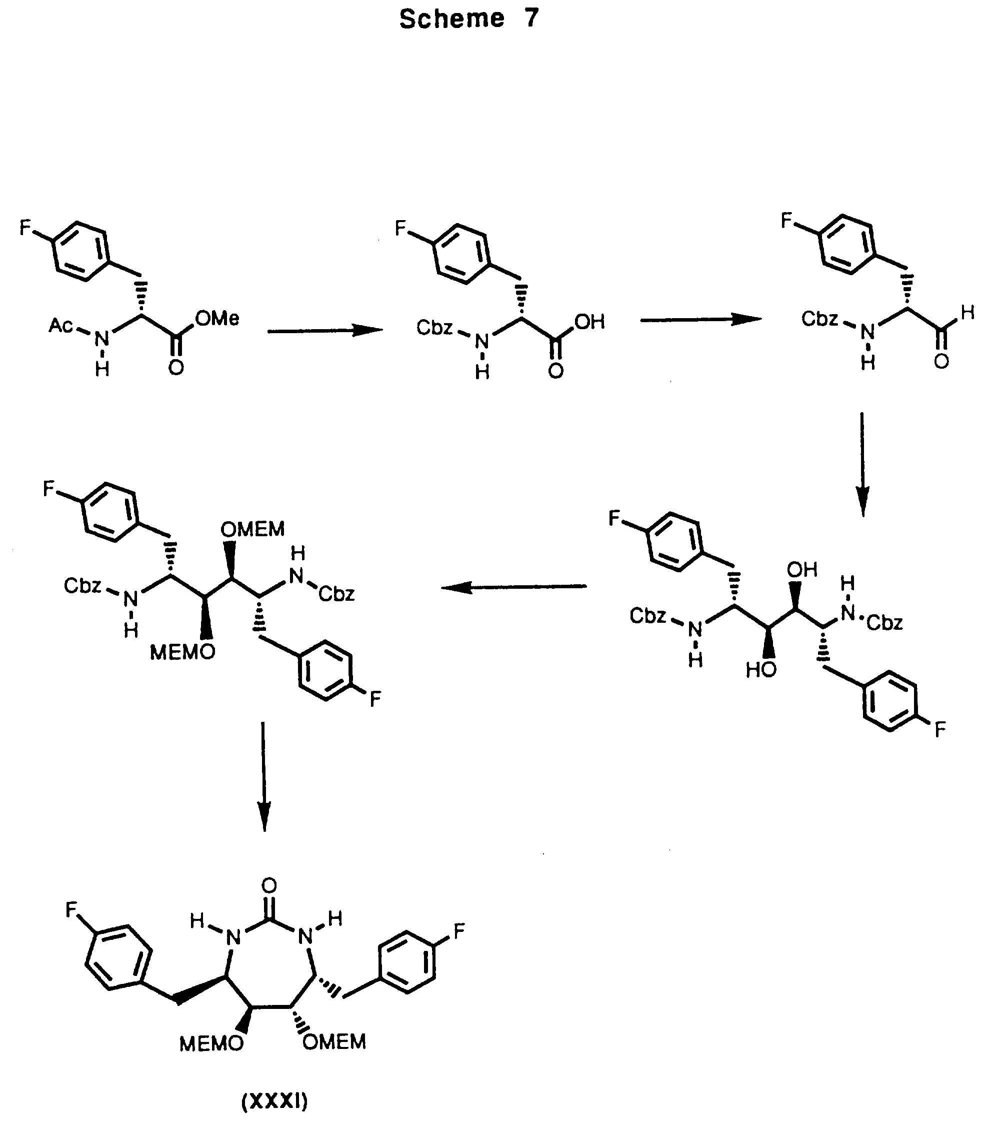

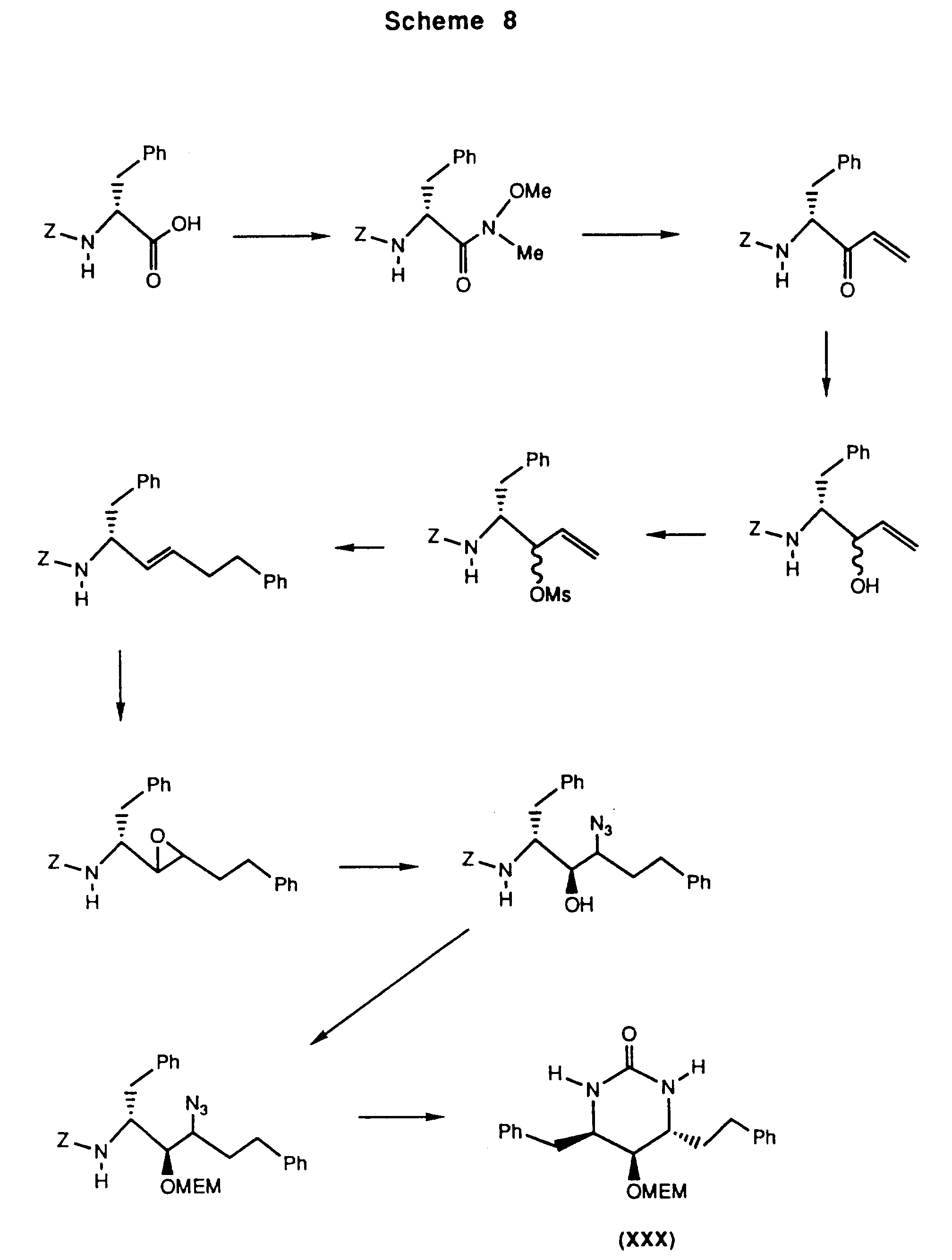

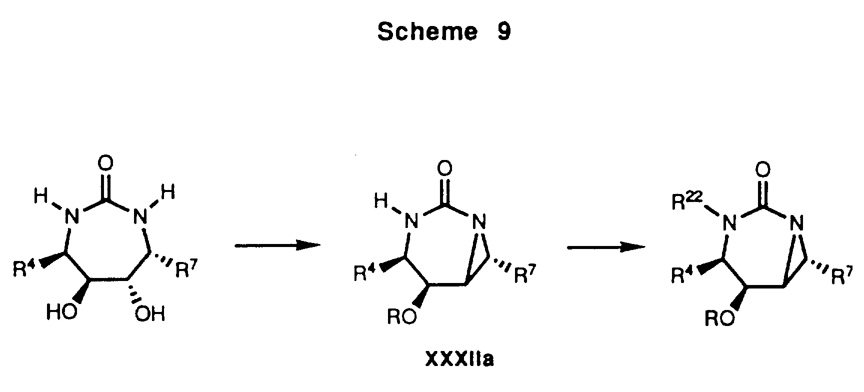

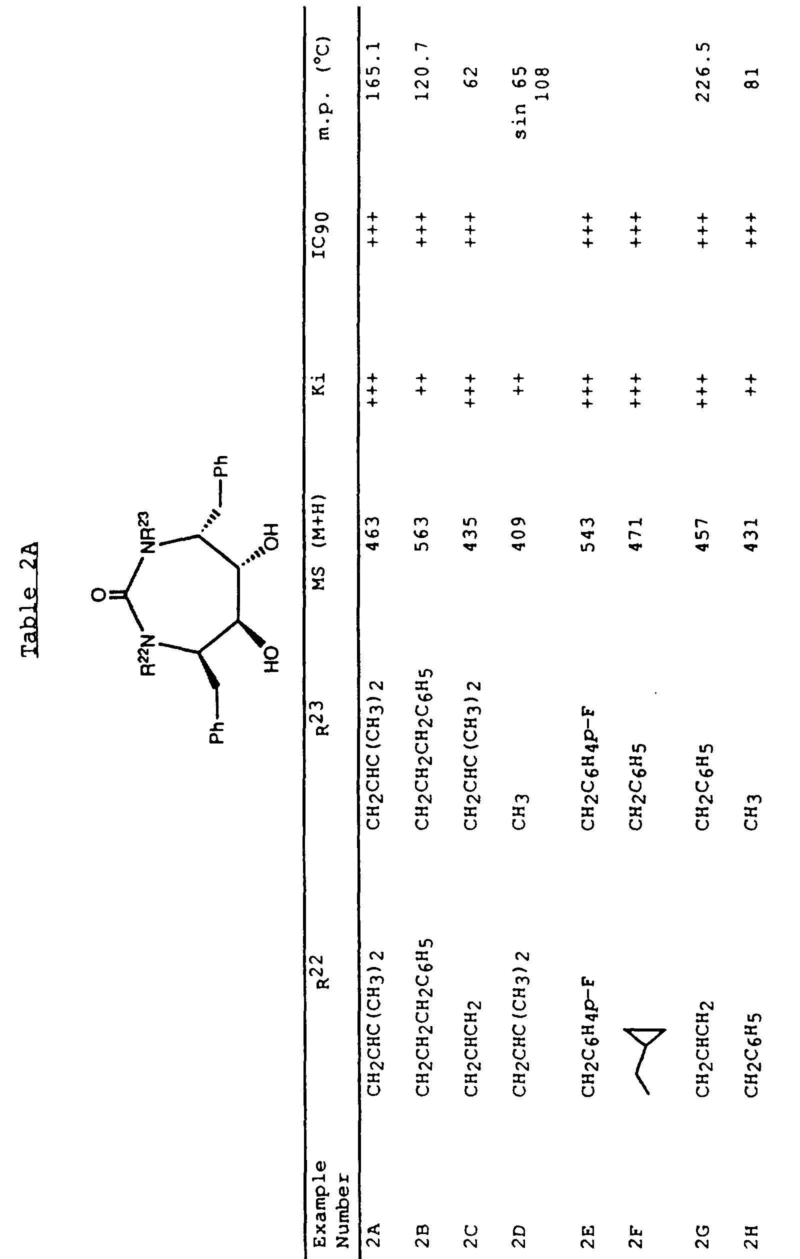

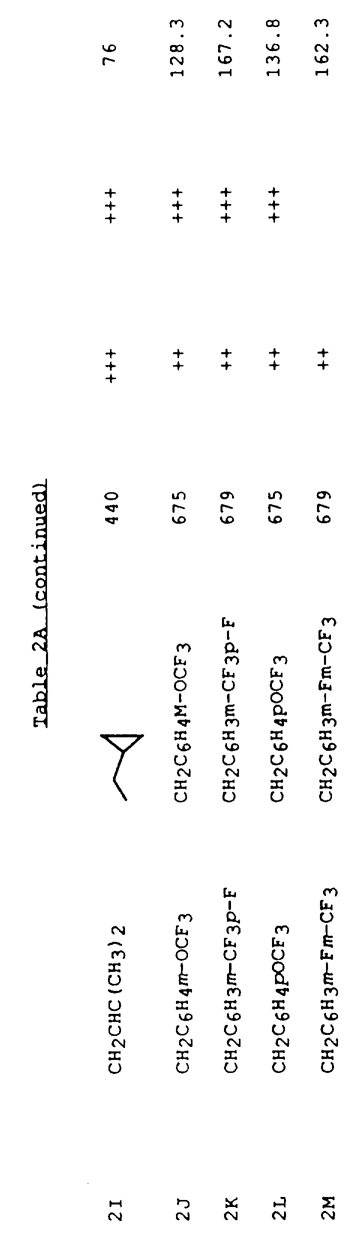

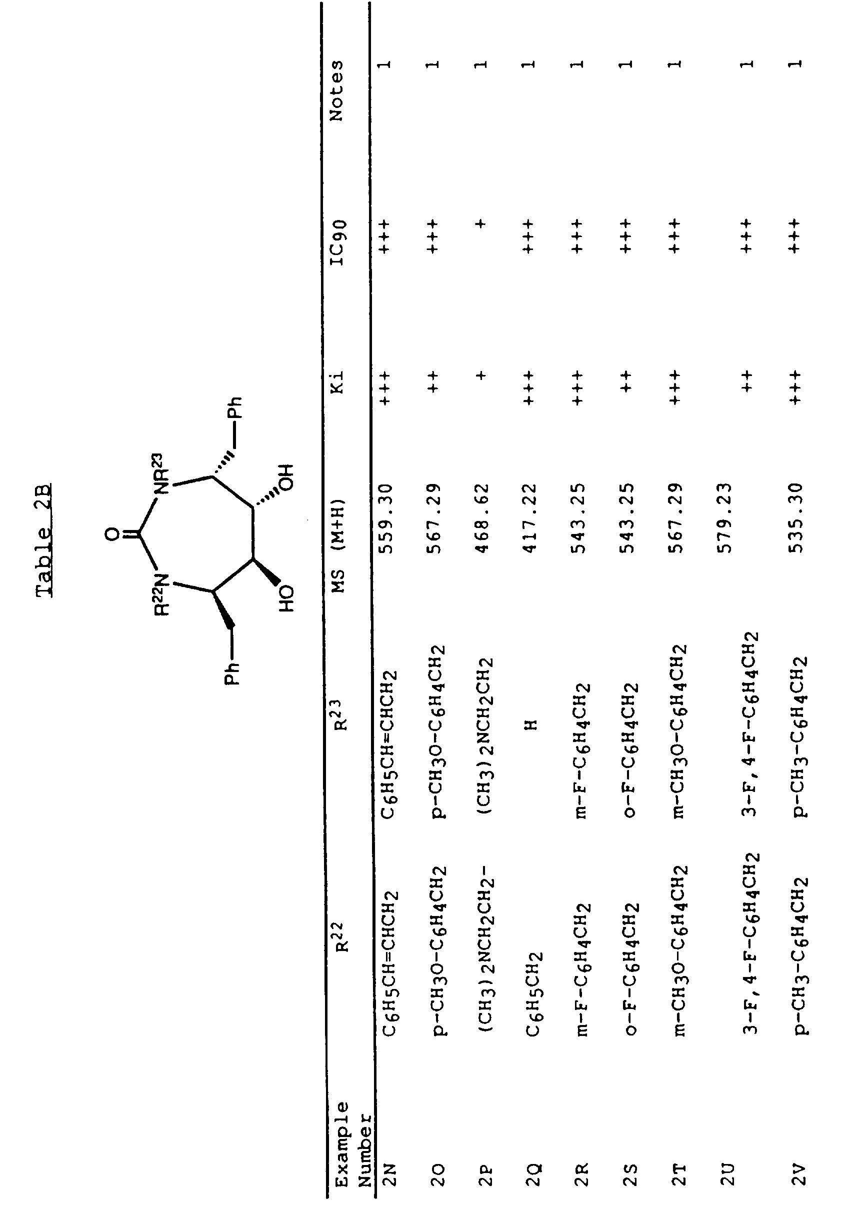

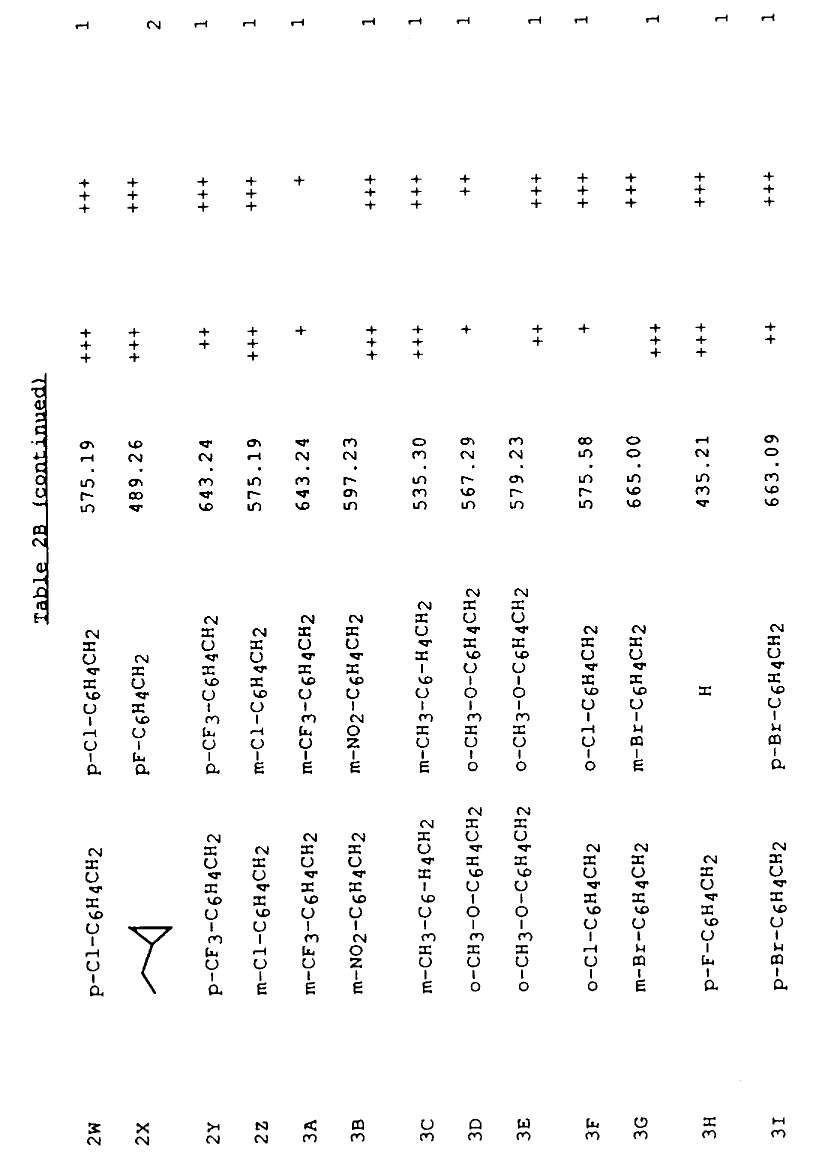

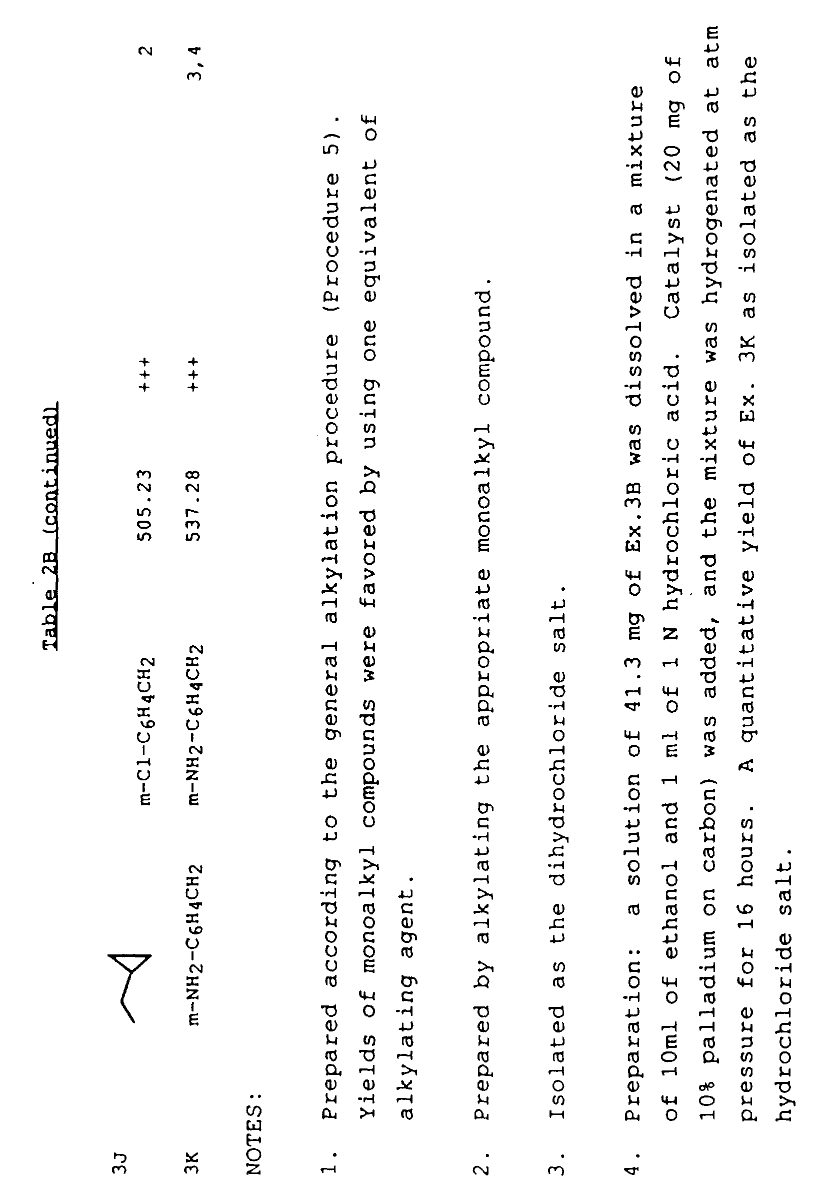

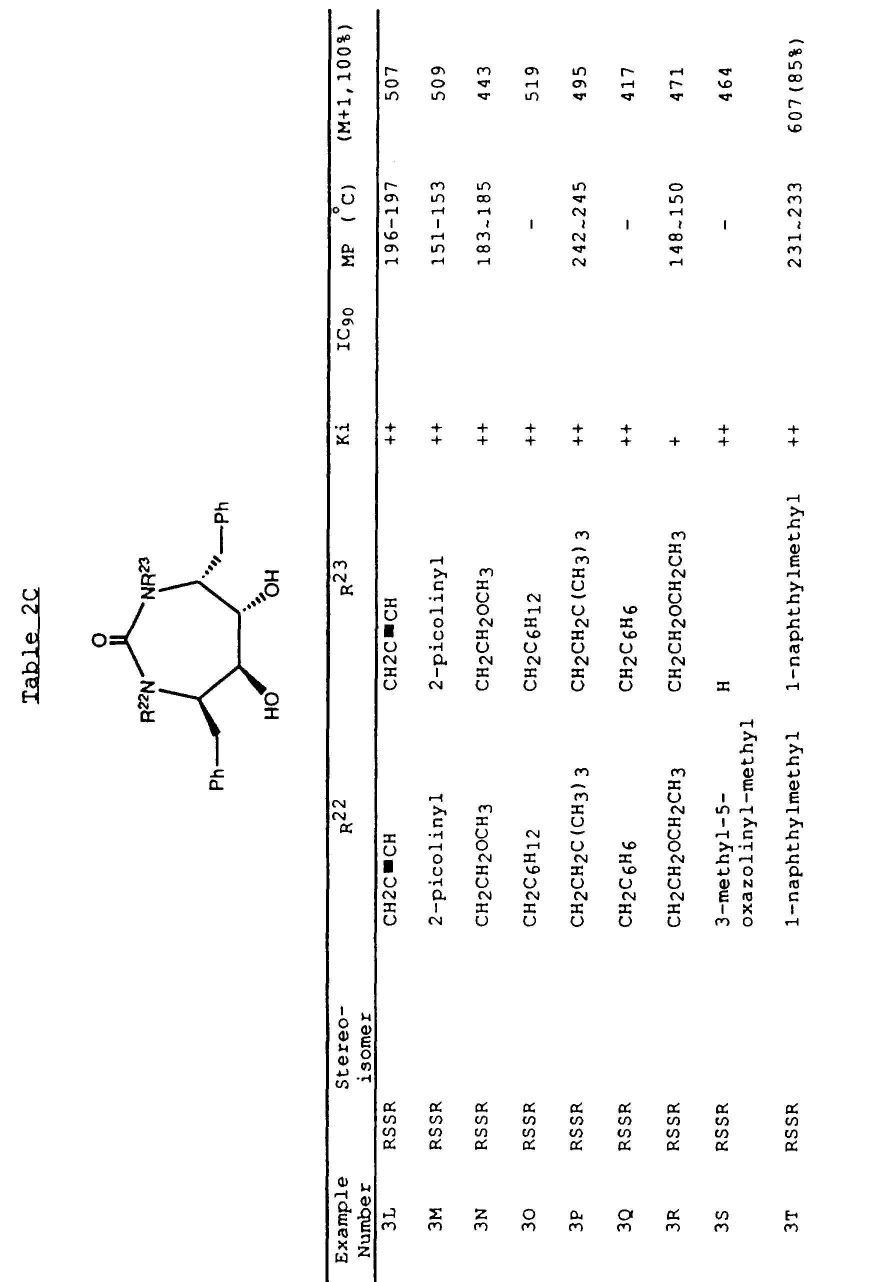

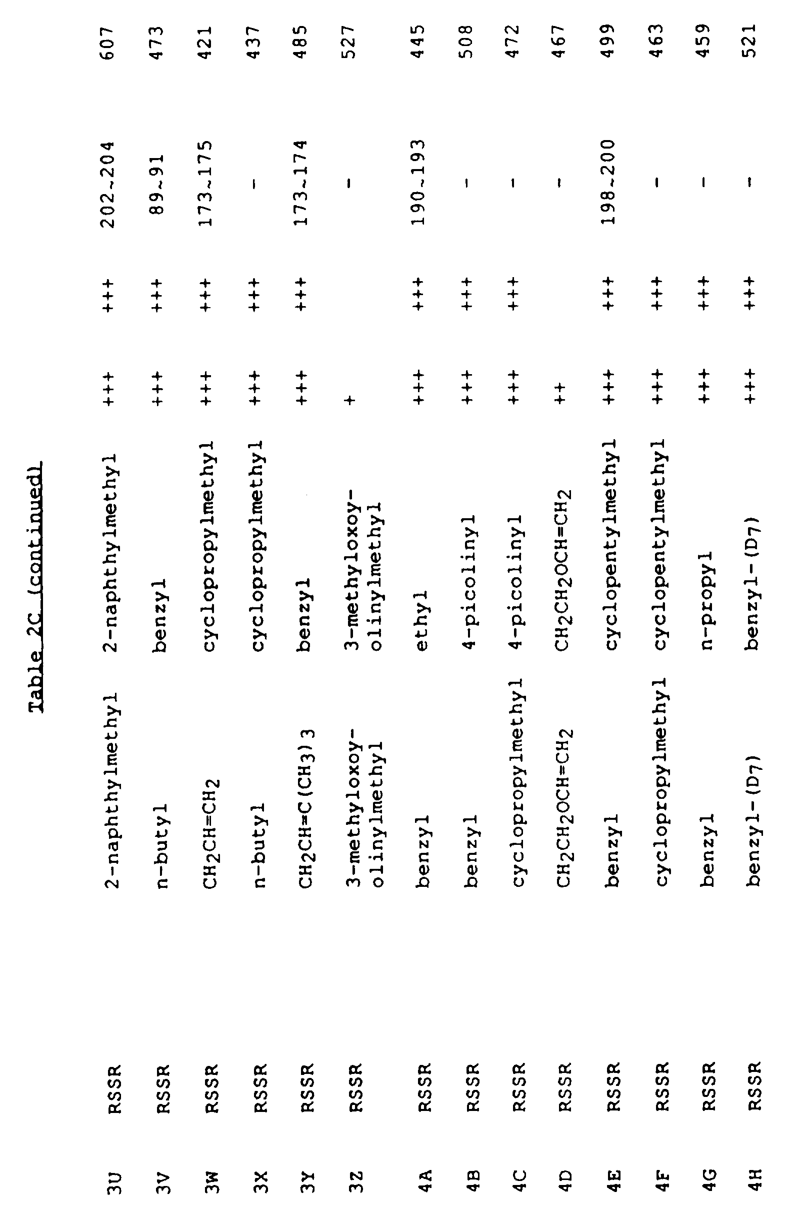

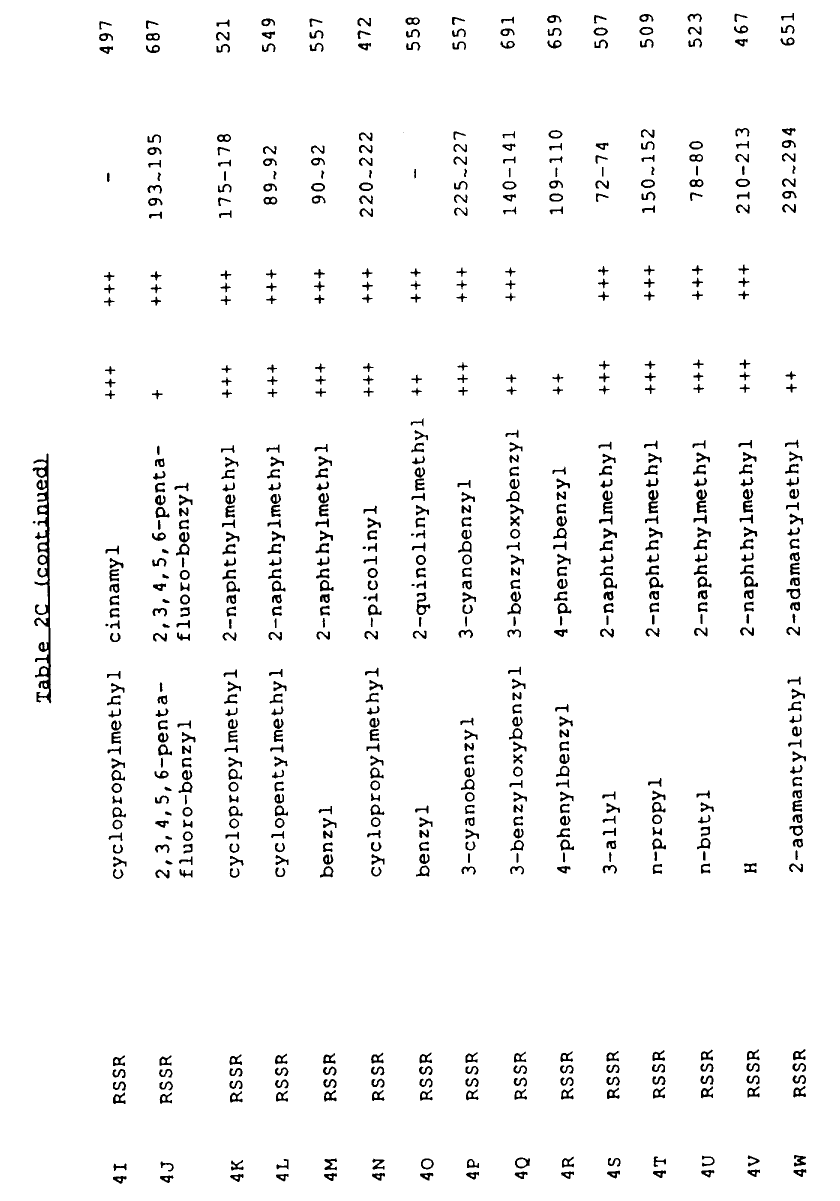

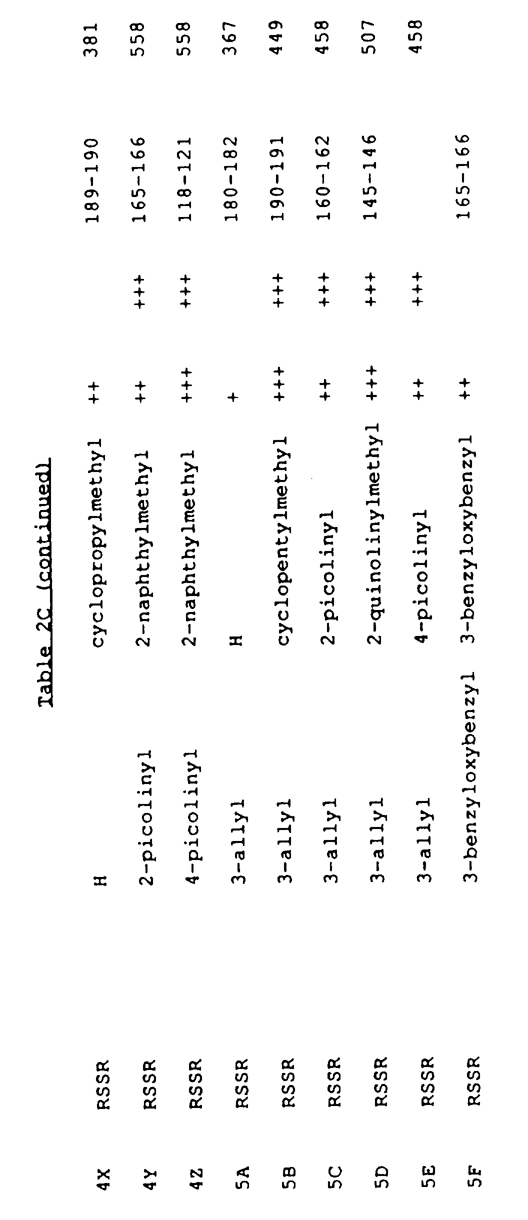

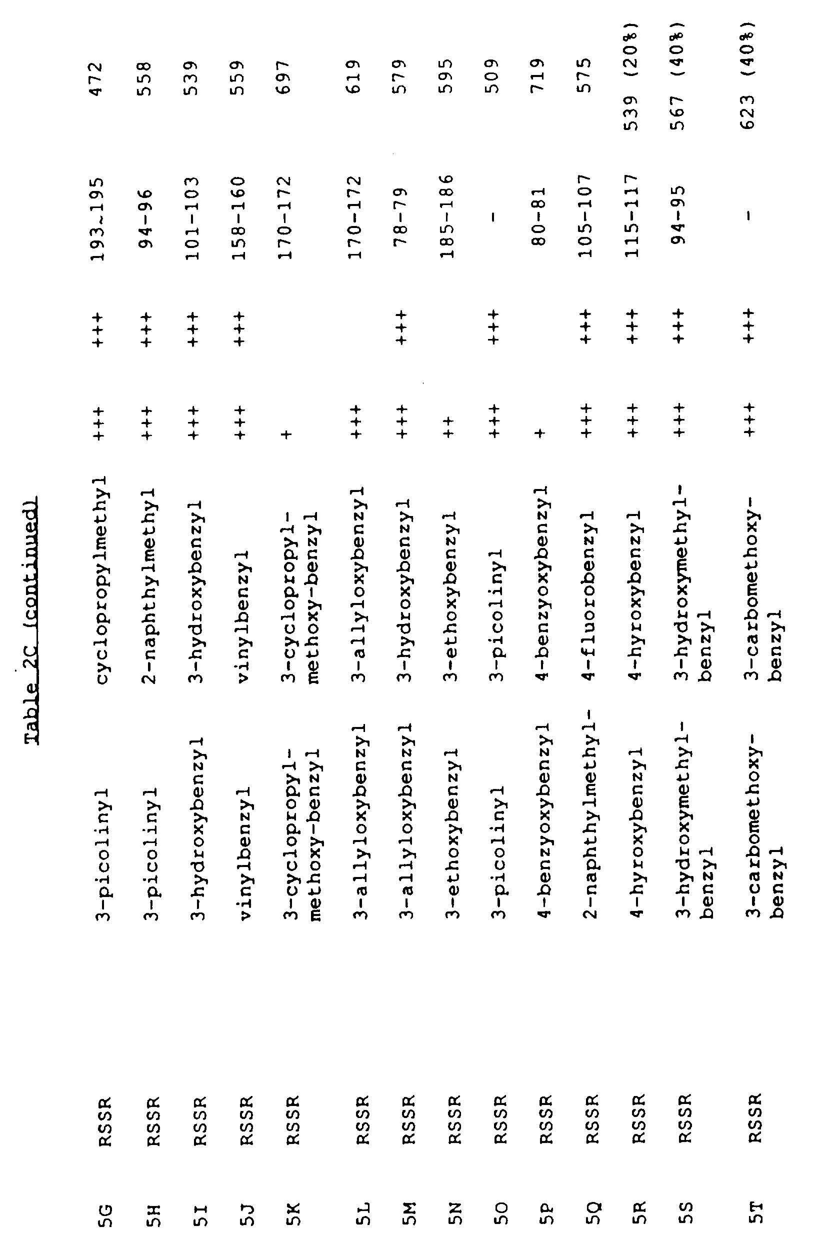

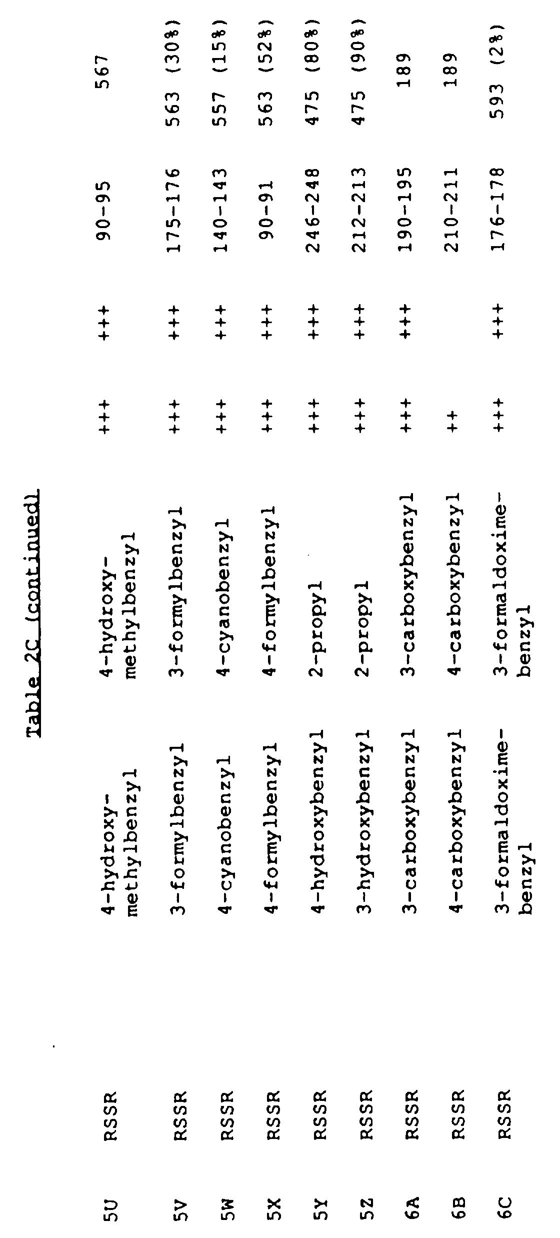

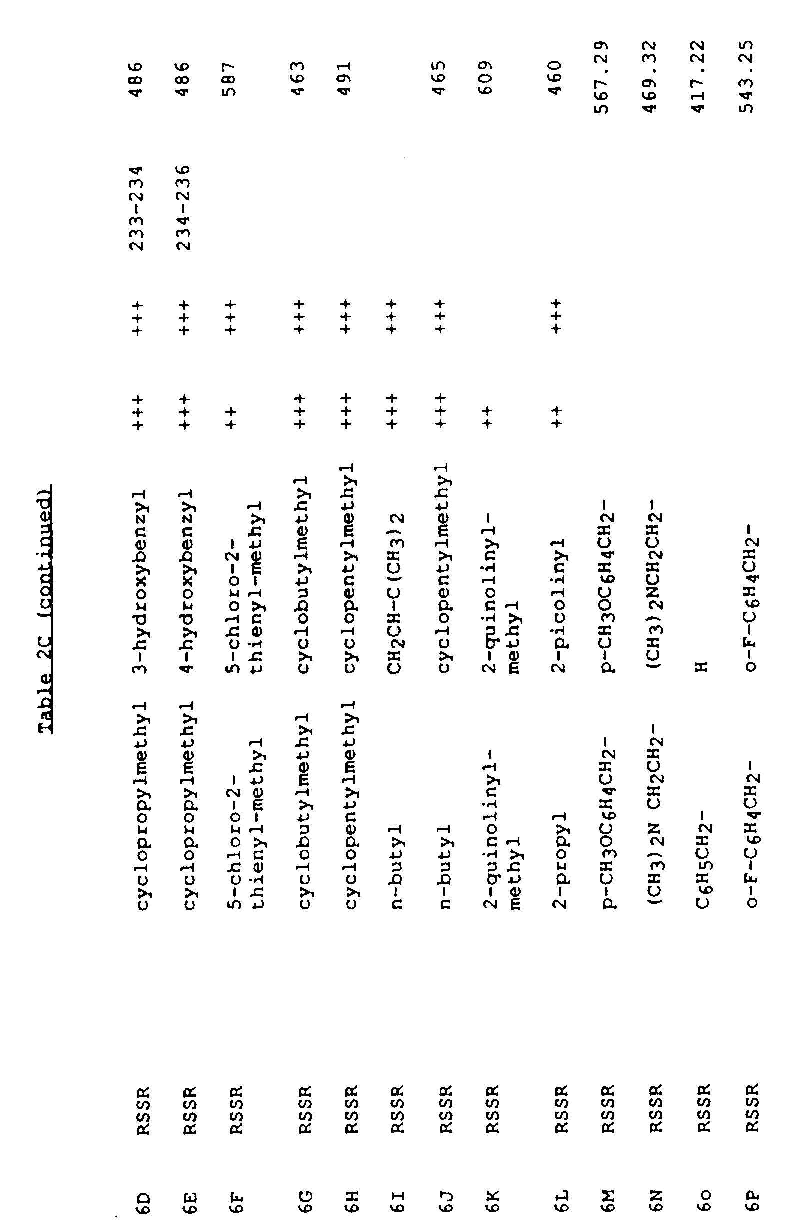

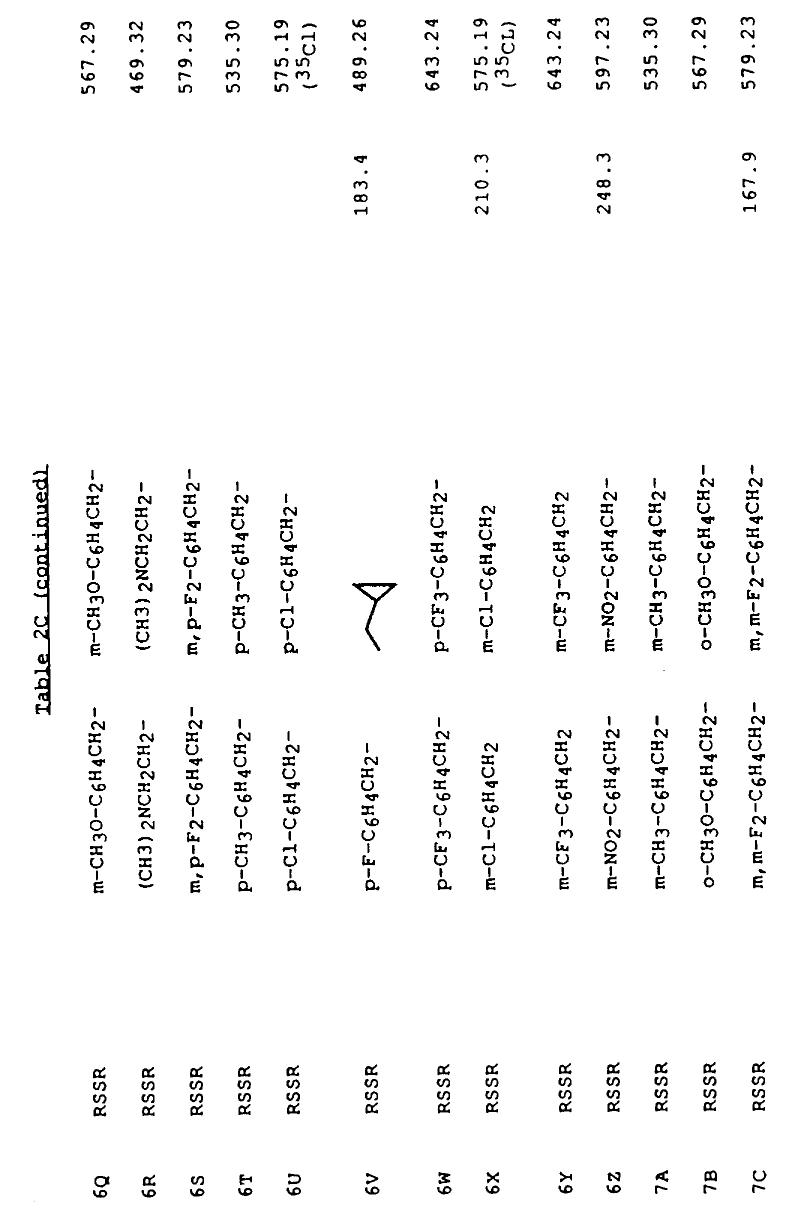

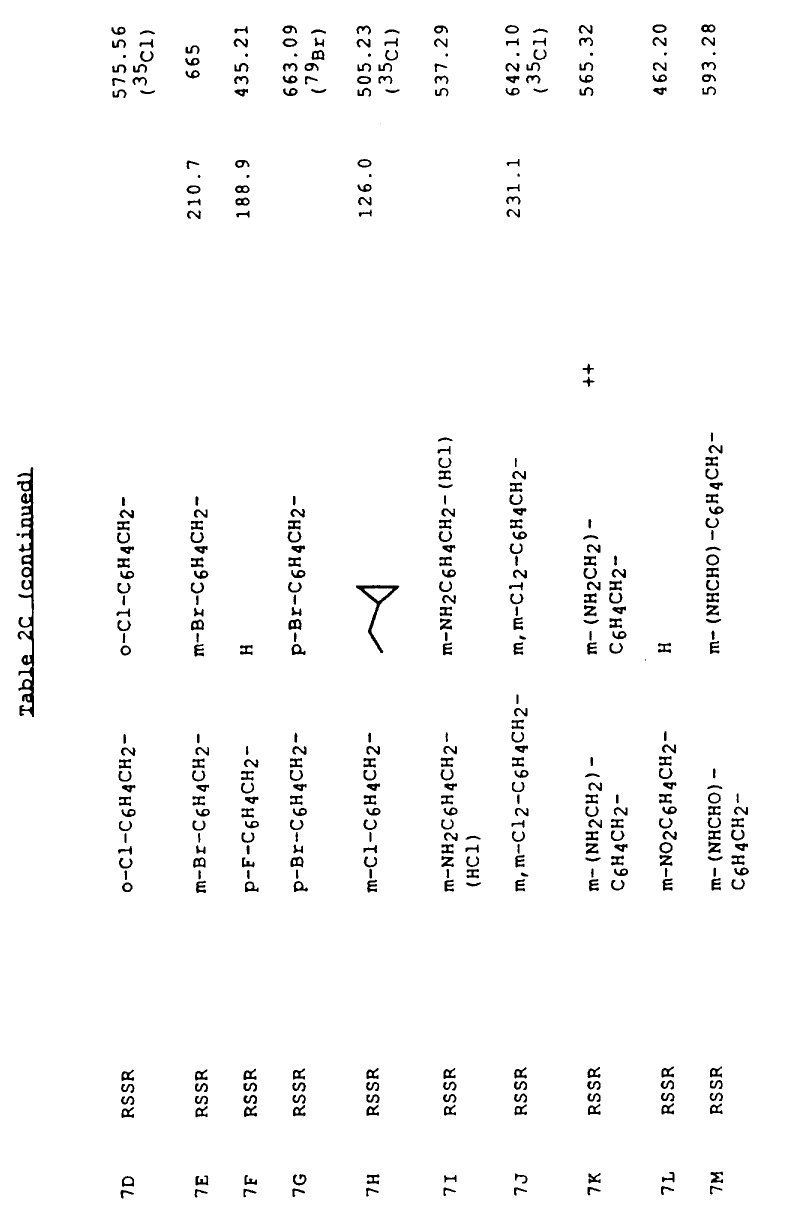

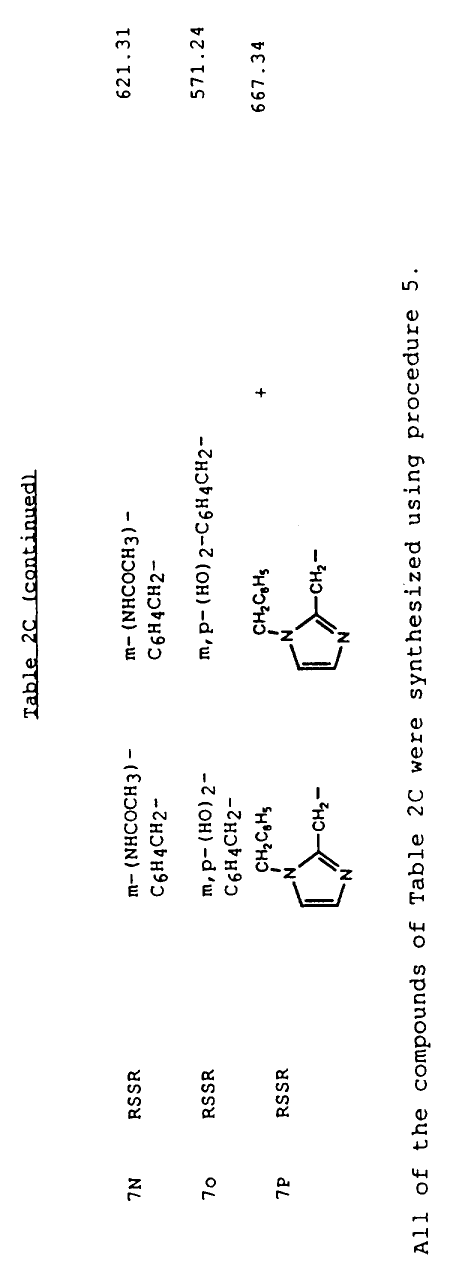

Synthesis of compounds of the invention in Table 2d is described in further detail below.

### Example 9A

To a solution of bis m-(HO-N=CH)-C₆H₄CH₂ cyclic urea (0.297, 0.502mmol) in methanol (5ml) was added borane-pyridine complex (0.464g, 5.02mmol) at -10°C and the resultant mixture was stirred for 15 minutes. After treated with 4M HCl in dioxane (5ml), the reaction mixture was allowed to stir to room temperature for additional 1.5 hours. The solution was neutralized with sat. NaHCO₃ to pH=8, washed with water and dried over MgSo₄. After removal of solvents, the residue was purified on silica gel plate with ethyl acetate: dichloromethane : methanol (50:50:2) to give 60mg of a solid, M.P. 214-216°C. ¹H NMR (CD₃OD): δ 8.03(s, 1H), 7.46 - 7.06 (m, 18H), 4.74 (d, *J*=13.9 Hz, 1H), 4.73 (d *J*=13.9Hz, 1H), 3.92(s,2H), 3.62-3.59 (bs, 4H), 3.07-2.91(m,6H) ¹³C NMR (CD₃OD): 163.88, 149.70, 141.26, 141.23, 139.92, 139.39, 138.73, 134.91, 131.50, 131.41, 130.64, 130.00 129.72, 129.64, 129.57, 129.53, 128.47, 127.45 127.42, 127.31, 71.96, 71.92, 67.42, 67.11, 58.65, 57.09, 57.03, 33.62, 33.57, MS: 594 (100%) 595 (M+H,60%).

### Example 9C

To a stirred solution of bis m-(HO-N=CH)-C₆H₄CH₂ cyclic urea (257 mg, 0.434 mmol), sodium cyanoborohydride (290 mg, 4.6mmol) and trace amount of methyl orange in methanol (10ml) at room temperature was added dropwise 2N HCl at a rate sufficient to maintain a pH of 3-4 over 3 hours. The methanol was removed by rotary evaporation. The residue was purified on a reverse phase TLC plate with 90% methanol in water to give the product. ¹H NMR (CD₃OD) : δ 7.33-7.09 (m, 18H), 4.75 (d, *J*=13.9Hz, 2H), 3.93 (s,4H), 3.61- 3.56 (m, 4H), 3.06- 2.94 (m, 6H); ¹³C NMR (CD₃OD): 162.42, 139.72, 137.88, 137.79, 129.92, 129.20, 128.31, 128.14, 128.06, 126.02, 70.36, 65.49, 57.40, 55.59, 32.09.

### Example 9E

A solution of Example 9C (30mg) in methanol was treated with 4M HCl in dioxane at room temperature. All solvents were removed under vacuum to give hydroxylamine hydrochloride. ¹H NMR (CD₃OD) : δ 7.44-7.23 (m,4H), 6.99 (d, *J*=6.2Hz, 4H), 4.65 (d, *J*=14.3 Hz,2H), 4.35(S, 4H), 3.70-3.66 (m, 4H), 3.12- 3.05 (m,4H), 2.89-2.85 (m,2H); ¹³C NMR(CD₃OD): 163.68, 141.13, 140.39, 123.09, 132.03, 131.06, 130.59, 130.50, 129.59, 127.48, 71.82, 68.23, 57.40, 56.12, 33.70.

### Example 9B

An unpurified sample of example 9C was purified on TLC plate using acetic acid: ethyl acetate: dichloromethane (5: 50:45) to give the acetic acid salt. ¹H NMR (CD₃OD): δ 7.35-7.08 (m, 18H), 4.73 (d, *J*=10.6Hz, 2H), 3.97(s, 4H), 3.62-3.53(m, 4H), 3.06-2.88 (m, 6H), 1.97 (s, 3H).

### Example 9D

A solution of bis (m-CHO-C₆H₄CH₂) cyclic urea (119 mg, 0.212 mml) and O-benzylhydroxylamine hydrochloride (203mg, 1.27mmol) in pyridine/ethanol (6ml 1:1) was refluxed for 3 hours. After removal of solvent, the residue was purified on T.L.C. plate with 15% ethyl acetate in dichloromethane to give the product (164mg) as a solid, M.P. 170.5-171°C. ¹H NMR(CDCl₃) : δ 8.01 (s, 2H), 7.39- 7.07 (m, 28H), 5.09 (s, 4H), 4.81 (d, *J*=14.3Hz, 2H), 3.59 (bs, 2H), 3.52 (d, *J*=11.0Hz, 2H), 3.07- 2.88 (m, 8H); ¹³C NMR(CDCl₃): 162.14, 148.55, 139.40, 138.62, 137.39, 132.50, 130.50, 129.43, 128.87, 128.62, 128.35, 128.27, 128.87, 127.57, 126.56, 126.46, 76.34, 71.32, 64.55, 55.57, 32.70; MS 790 (M+NH₄,100%).

### Example 9F

By the procedure given above Example 9C, a solution of Example 9D (60mg, 0.078mmol), sodium cyanoborohydride (70mg, 1.1mml), trace amounts of methyl orange in methanol was treated with 2N HCl (approx. 0.5 ml). Purification on T.L.C. plate with 40% ethyl acetate in methylene chloride gave the product (30mg). ¹H NMR (CDCl₃): δ 8.05 (s,1H), 7.43- 7.06 (m, 28H), 5.13 (s, 24), 4.90 (d, *J*=14.3Hz, 1H), 4.87(d, *J*=14.3Hz, 1H), 4.61(s, 2H), 3.98 (s, 2H), 3.59-3.49 (m, 4H), 3.07- 2.95 (m, 6H). ¹³C NMR(CDCl₃) : 161.98, 148.59, 139.48, 138.86, 138.32, 138.05, 137.74, 137.48, 132.56 130.61, 129.87, 129.51, 128.91, 128.67, 128.65, 128.44, 128.37, 128.11, 127.91, 127.86, 127.66, 126.59, 126.56, 126.40, 77.20, 76.41, 76.15, 71.52, 71.48, 64.33, 64.11, 56.26, 55.58, 55.57, 33.79, 32.47. MS: 792 (M+NH₄, 40%) 775 (M+H,100%).

### Example 9G

To a suspension solution of bis (m-(HO)-C₆H₄CH₂) cyclic urea (280mg, 0.52 mmol) and Cs₂CO₃ (1.5g, 4.6mmol) in THF (4ml) was added methylchloroformate (122.9mg, 1.3mm) and the resulting mixture was stirred at room temperature overnight. The mixture was filtered through celite and concentrated to give a residue which was purified on T.L.C. plate with 15% ethyl acetate in methylene chloride to give the product (220 mg). as a solid, M.P. 146-147°C. ¹H NMR (CD₃OD): d 7.34-6.95 (m, 18m), 4.78 (d.*J*=14.3Hz, 2H), 3.78 (s. 6H), 3.68 (bs, 2H), 3.61 (d, *J*=11.4Hz, 2H), 3.10- 2.83 (m, 6H). ¹³C NMR (CD₃OD): 162.22, 154.08, 151.28 139.74, 129.28, 129.14 128.11, 126.52, 125.99, 121.63, 119.85, 70.44, 66.14, 55.46, 54.51, 32.22; MS: 655 (M+H, 100%).

### Example 9I

To a solution of Example 9S (100mg) in tetrahydrofuran (1ml) was added methyl amine (0.4ml, 40% in water) and the resulting solution was stirred overnight. After concentration and purification on the plate, the product was obtained in good yield.

### Examples 9J and 9K

By the procedure described previously for preparation of Example 9D, and substituting b-ethanolamine and trace amounts of molecular sieves (powder) in ethanol. The reaction mixture was filtered through Celite and concentrated to give a residue, which was purified on reverse phrase T.L.C. plate with 90% methanol in water to the desired compounds.
Example 9J: ¹H NMR (CD₃OD): δ 8.31 (s, 2H), 7.67 (d, *J*=7.7Hz, 2H), 7.59 (s, 2H), 7.43- 7.03 (m, 14H), 4.74 (d, *J*=14Hz, 2H), 3.81-3.58 (m, 12H), 3.10- 2.86 (m, 6H); ¹³C NMR (CD₃OD): 164.97, 163.73, 141.22, 140.14 137.66, 133.06, 130.65, 130.53, 130.06, 129.56, 128.50, 127.46, 71.93, 67.90, 64.26, 62.29, 57.20, 33.68, MS: 649 (M+H, 100%).
Example 9K: ¹H NMR (CD₃OD) : δ 8.31 (s,1H), 7.68- 7.03 (m, 18H), 5.33 (s, 1H), 4.76 (d, *J*= 13.9Hz, 1H), 4.75 (d, *J*= 14.3Hz, 1H), 3.81- 3.55 (m, 10H), 3.12- 2.7 (m, 8H); ¹³C NMR (CD₃OD): 164,97, 163.78, 141.29, 141.24, 140.18, 139.39, 137.63, 133.06, 130.67, 130.64, 130.53, 130.48, 130.05, 129.55, 129.50, 128.86, 128.49, 127.47, 127.45, 127.20, 104.35, 71.98, 67.90, 67.40, 64.25 62.39, 57.22, 57.11, 53.23, 33.67; M.S.: 649 (M+H, 100%).

### Example 9L

To a solution of Example 51 (0.7g, 1.3 mmol) and triethylamine (0.263g, 2.6mmol) in THF (5ml) was added benzyl isocyanate (0.346g, 2.6mmol) and the resulting solution was stirred at room temperature overnight. After removal of all the volatiles, the residue was purified on T.L.C. plate to give 0.7g of a solid, M.P. 150°C (decompose). ¹H NMR (CD₃OD) : δ 7.33- 6.83 (m, 28H), 4.74 (d, *J*= 13.9Hz, 2H), 4.30 (s, 4H), 3.64 (bs, 2H), 3.59 (d, *J*=12.1 Hz, 2H), 3.13- 2.91 (m, 6H); ¹³C NMR (CD₃OD): 162.54, 155.68, 151.33, 139.88, 139.30, 138.64, 129.29, 129.20, 128.24, 128.18, 127.02, 126.90, 126.13, 125.81, 122.44, 120.55, 70.44, 65.28, 55.20, 44.31, 32.28; M.S: 805 (M+H, 100%).

### Example 9M

To a solution of Example 5I (100mg, 0.186mmol) triethylamine (38mg. 0.39mmol) in THF (1 ml) was added methyl isocyanate (27mg. 0.47mmol) at room temperature and the resulting mixture was stirred overnight. After removal of all volatile reagents, a residue was purified on T.L.C. plate with 40% ethyl acetate in dichloromehane to give 5lmgs of a solid, M.P. 150°C (decompose). ¹H NMR (CD₃OD) δ 7.44-6.99 (m, 18H), 4.82 (d, *J*=14.2Hz, 2H), 3.69- 3.65 (m, 4H), 3.15-2.95 (m,6H), 2.84 (s, 6H). ¹³C NMR (CD₃OD): 163.96, 157.56, 152.92, 141.31 140.83, 130.66, 130.55, 129.58, 127.46, 127.17, 123.70, 121.95, 71.88, 67.02, 56.67, 33.65, 27.57. M.S.: 670 (M+NH, 100%).

### Example 9N

A solution of bis (m-bromobenzyl) cyclic urea (MEM-protected) (0.84g, 1 mmol), propargyl alcohol (0.224g, 4mmol), tetrakis (triphenylphoshine) palladium (0.116g, 0.1mmol), copper iodide (0.019g, 0.1mmol) in triethylamine (5ml) was refluxed under nitrogen overnight. After evaporation of all volatiles, a residue was diluted with ether (20ml) and filtered through Celite. The filtrate was concentrated and purified on T.L.C. plate to give 400mg of MEM-protected-mono/coupling product. Deprotection of 170mg of the coupled product by the standard procedure gave 130 mg of the desired product. ¹H NMR (CD₃OD) : δ 7.50-7.09 (m, 18H), 4.72 (d, *J*=13.9Hz, 1H), 4.69 (d, *J*=13.4Hz, 1H), 4.46 (s, 2H), 3.75-3.68 (m, 4H), 3.18-2.86 (m, 6H). ¹³C NMR (CD₃OD): 163.61, 141.99, 141.07, 139.73, 133.64, 133.54, 131.91, 131.76, 131.47, 130.57, 130.50, 129.81, 129.59, 129.19, 127.51, 124.69, 123.46, 89.28, 85.09, 71.93, 71.91, 68.12, 67.75, 57.07, 57.03, 51.15, 33.60, 33.59;; M.S.: 639/641 (M+H, 100%), 656/658 (M+NH₄, 100%).

### Examples 9o and 9P

A solution of bis (m-Br-C₆H₄CH₂) cyclic urea (425,mg, 0.64 mmol), 1-ethoxy-1-trimethylstannyl ethylene (833mg, 3.84mmol) and Pd(PPh₃)₄ (37mg, 0.032mmol) in THF (5ml) was refluxed under N₂ overnight. After cooling to room temperature, the reaction mixture was diluted with ether (10ml) and filtered through silica gel to give two products. Further purification on T.L.C. plate with 20% ethyl acetate in methylene chloride gave Example 9Q (107mg, M.P. 190-191°C) and Example 9P (225 mg, M.P. 158-159°C).
Example 9o: ¹H NMR (CDCl₃): δ 7.78 (d, *J*=7.3Hz, 1H), 7.71 (s, 1H), 7.42- 7.02 (m, 16H), 4.81 (d, *J*= 13.9Hz, 1H), 4.77 (d, J=13.9H, 1H), 3.71 (bs, 2H), 3.62-3.54 (m, 2H), 3.20- 2.85(m, 8H), 2.50 (s, 3H); ¹³C NMR (CDCl₃): 198.27, 161.86, 140.38, 139.29, 139.22, 138.81, 137.22, 133.99, 132.36, 130.65, 130.09, 129.36, 129.32, 128.99, 128.86, 128.66, 127.70, 127.55, 126.61, 122.53, 71.38, 65.24, 65.12, 55.83, 55.59, 32.77, 26.58, M.S: 644/646 (M+NH₄, 100%).
Example 9P: ¹H NMR (CDCl₃): δ 7.78- 7.03 (m, 18H), 4.83 (d, *J*=14.3Hz, 2H), 3.73 (bs, 2H), 3.60 (d, *J*=10.6Hz, 2H), 3.17- 3.08 (m, 4H), 2.92-2.89 (m, 2H), 2.49 (s, 6H); ¹³C NMR (CDCl₃): 198.03, 161.83, 139.40, 138.78, 137.12, 133.87, 129.29, 129.04, 128.74, 128.52, 127.36, 126.46, 71.25, 71.15, 65.32, 55.82, 30.73, 26.51; M.S.: 608 (M+NH₄, 100%).

### Example 9Q

A solution of Example 9P (84mg, 0.142mmol) and hydroxylamine hydrochloride (59.4mg, 0.854mmol) in pyridine /ethanol (6ml, 1:1) was refluxed overnight. Evaporation of all solvents under vacuum gave a residue which was purified on preparative T.L.C. plates with ethyl acetate: methylene chloride: methanol (50:50:2) to give 71mg of a solid, M.P. 200 202°C. ¹H NMR (CD₃OD) : δ 7.67- 7.07 (m, 18H), 4.74 (d, *J*=13.9Hz, 2H), 3.64-3.62 (m, 4H), 3.09- 2.89 (m, 6H), 2.17 (s, 6H); ¹³C NMR(CD₃OD): 163.94, 155.43, 141.23, 139.46, 138.97, 130.77, 130.65, 129.69, 129.37, 128.10, 127.47, 126.32, 72.02, 67.16, 57.08, 33.62, 11.96; M.S.: 621, (M+H, 100%).

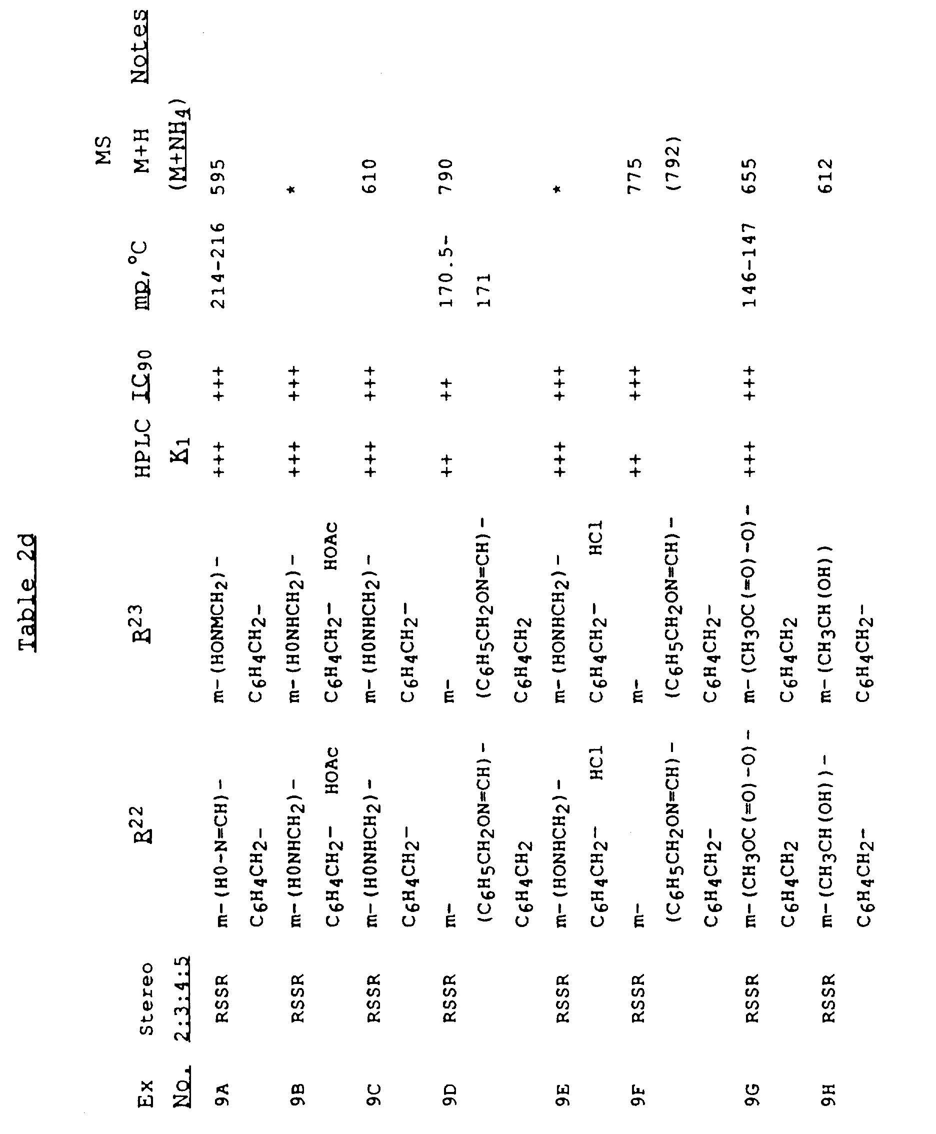

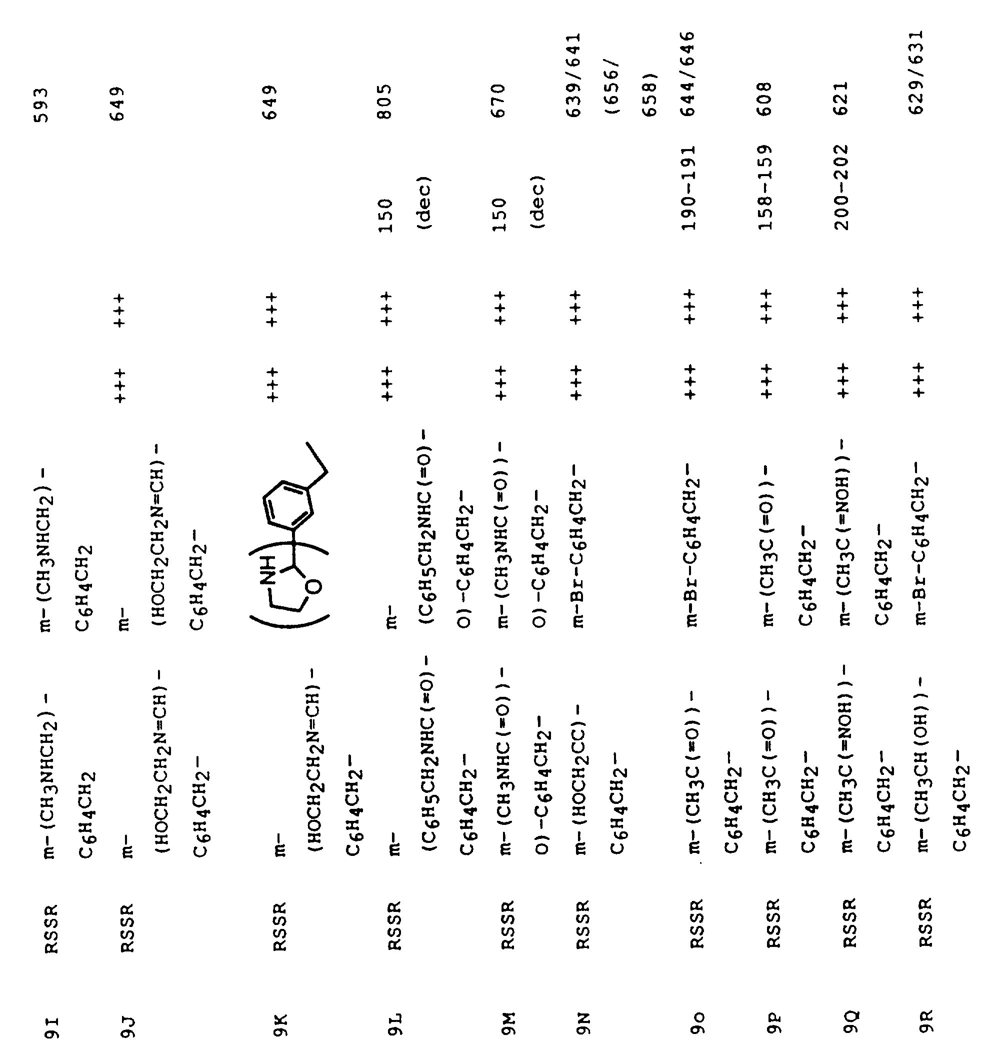

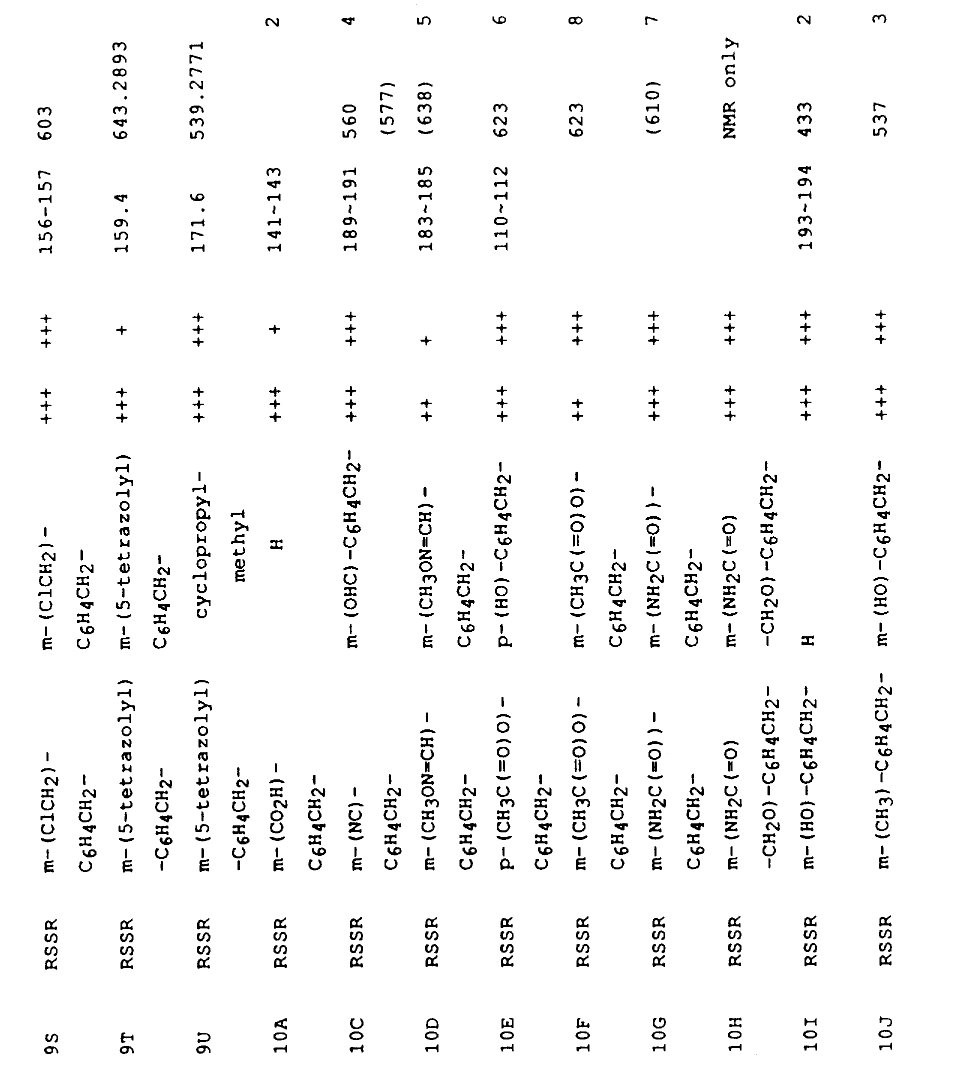

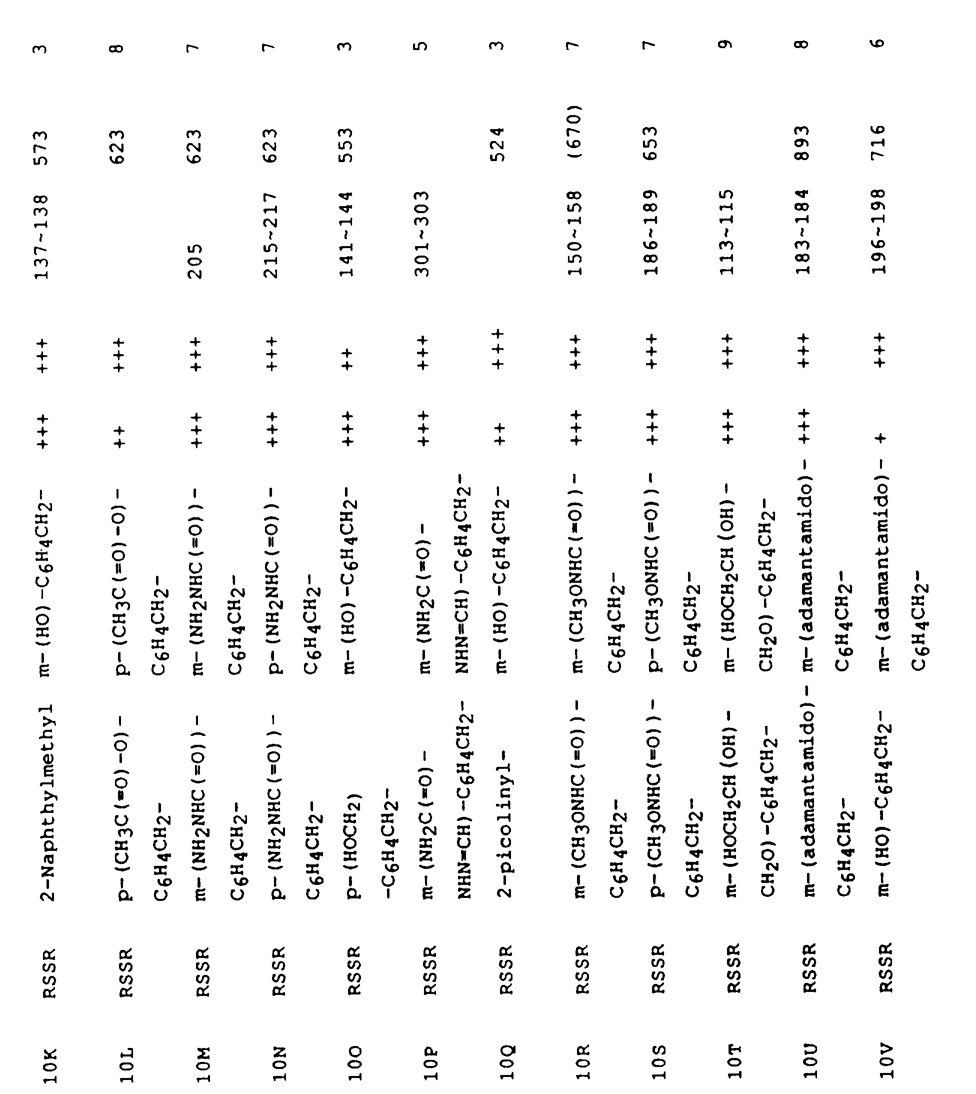

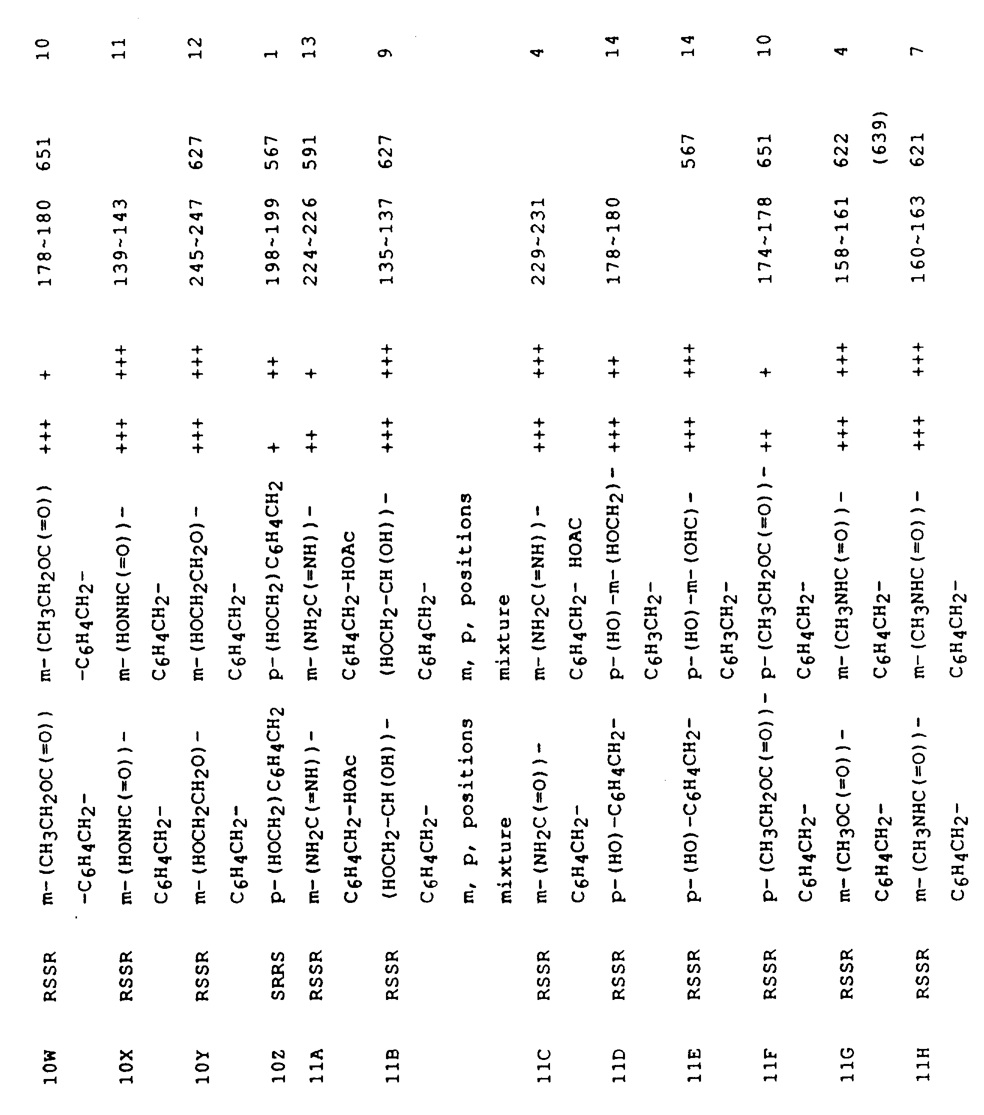

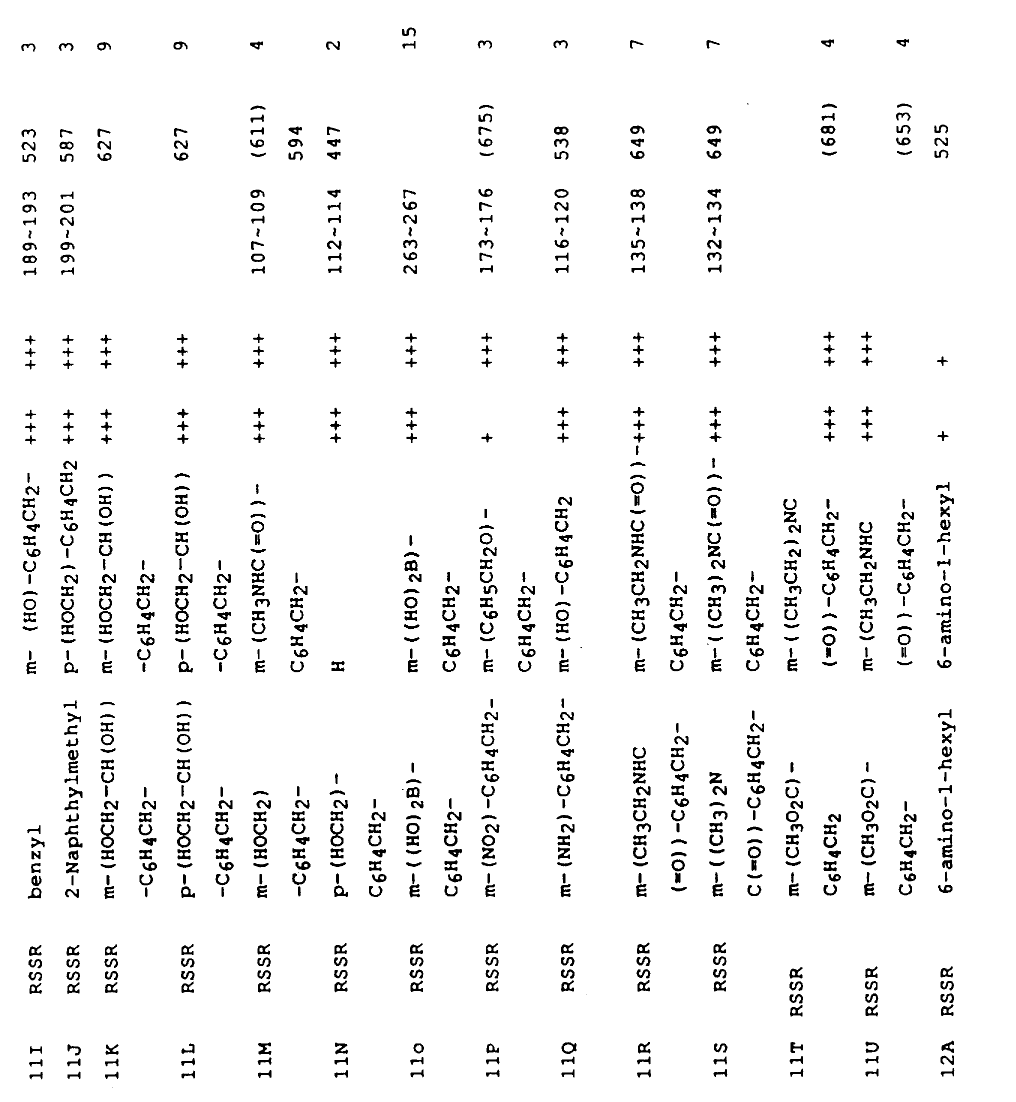

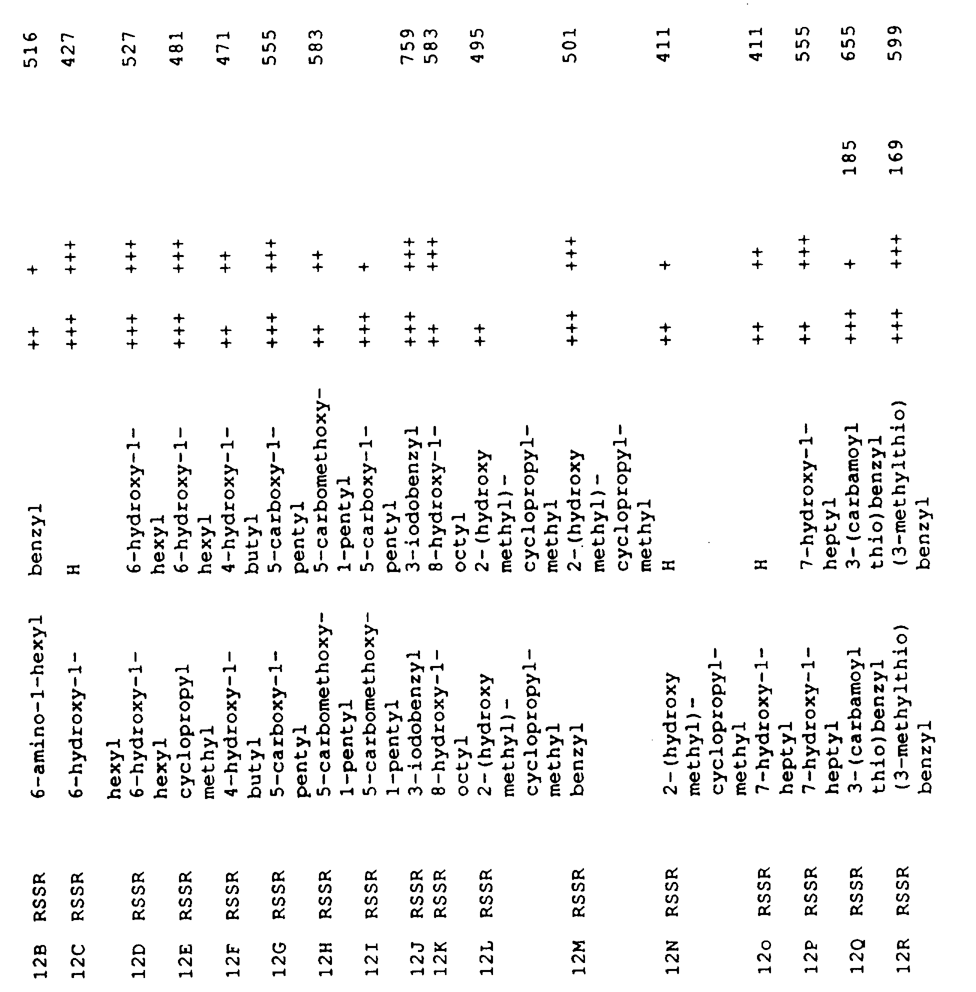

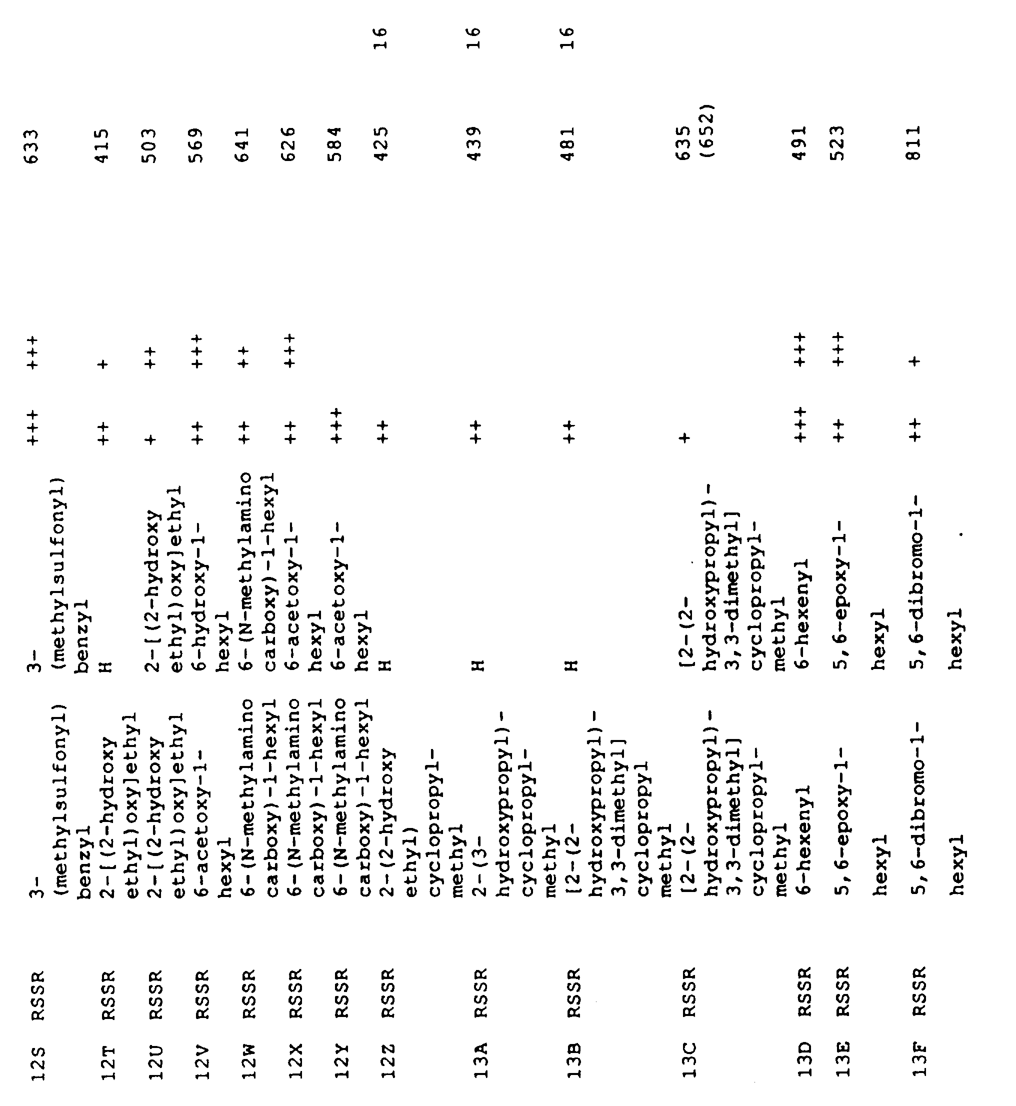

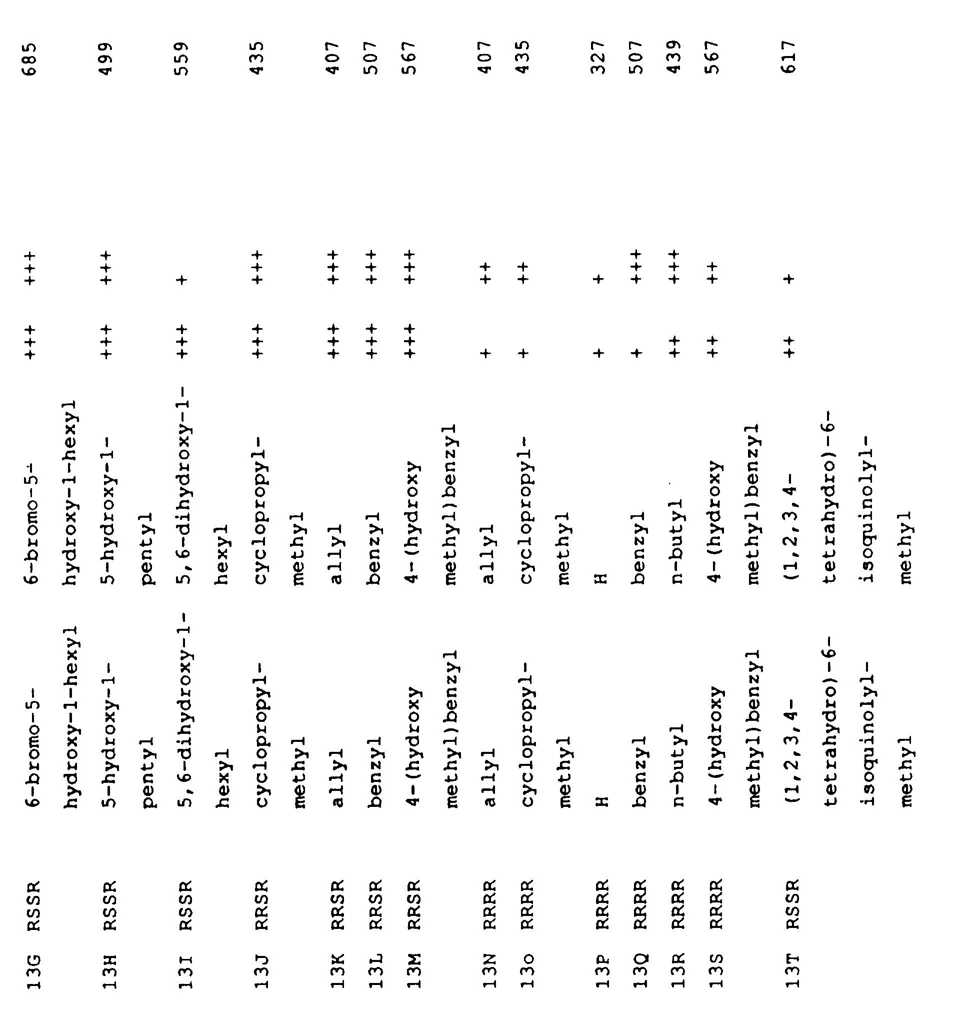

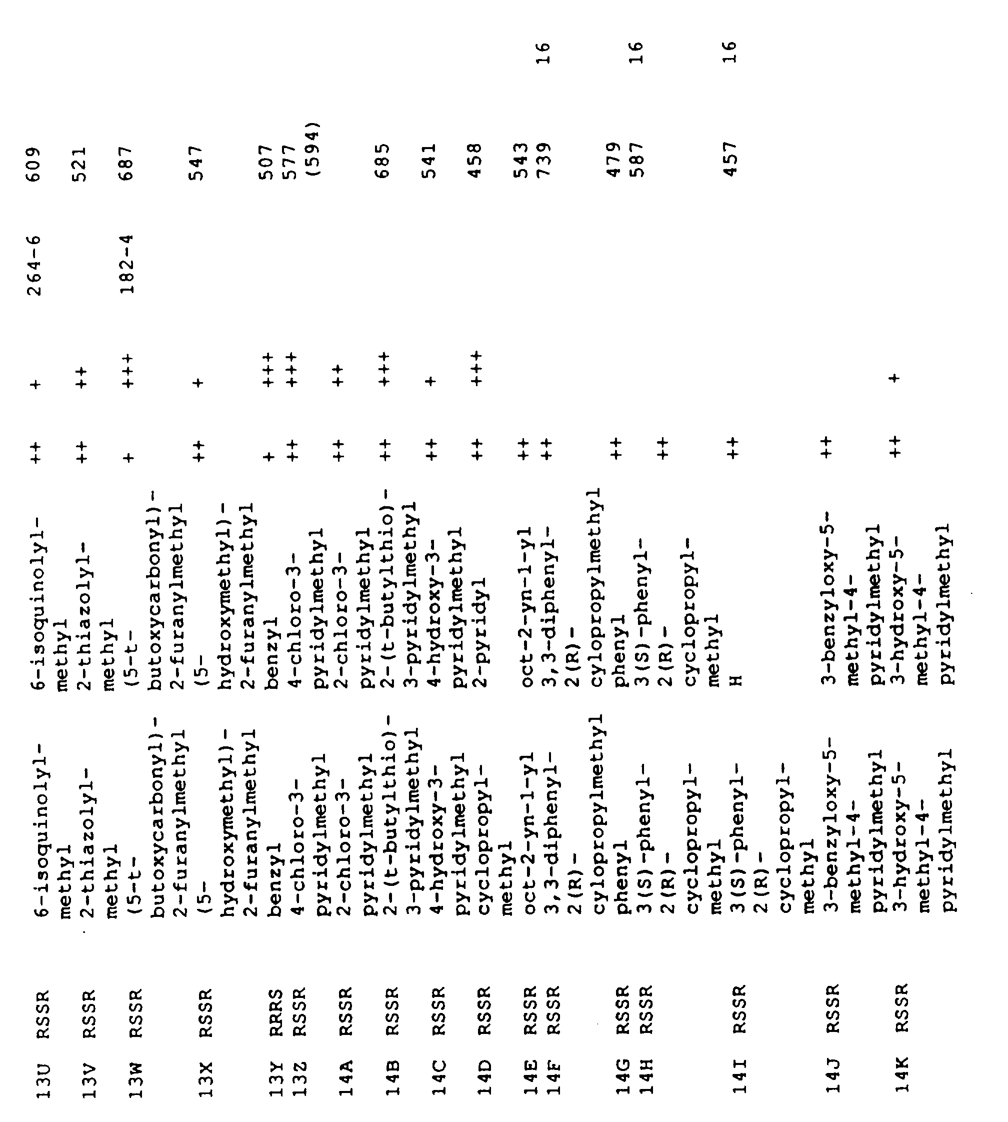

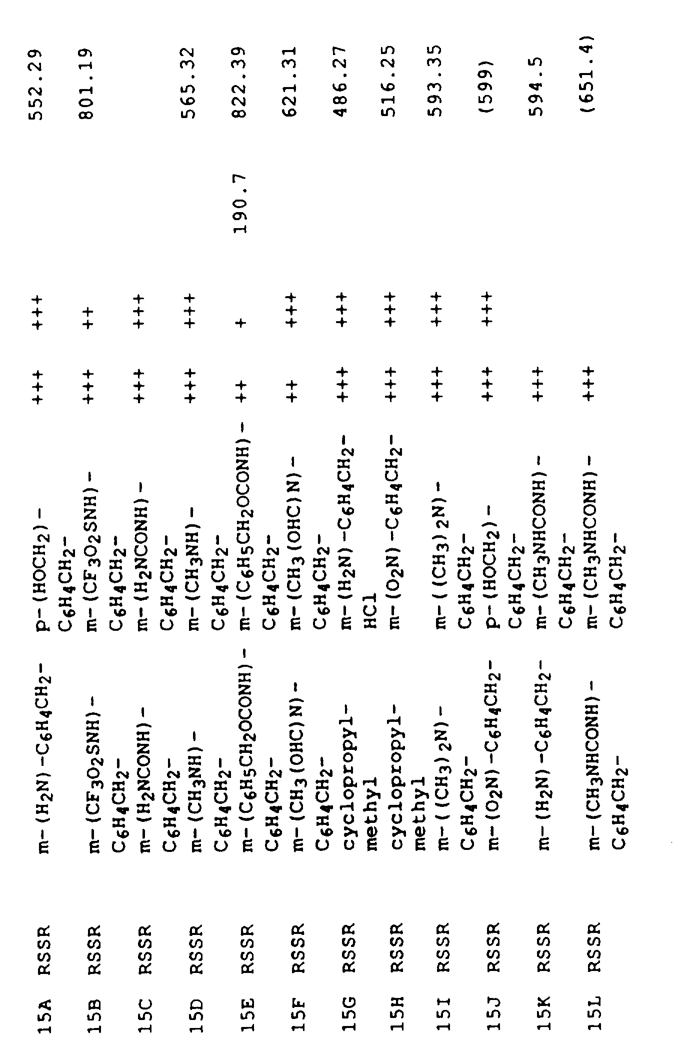

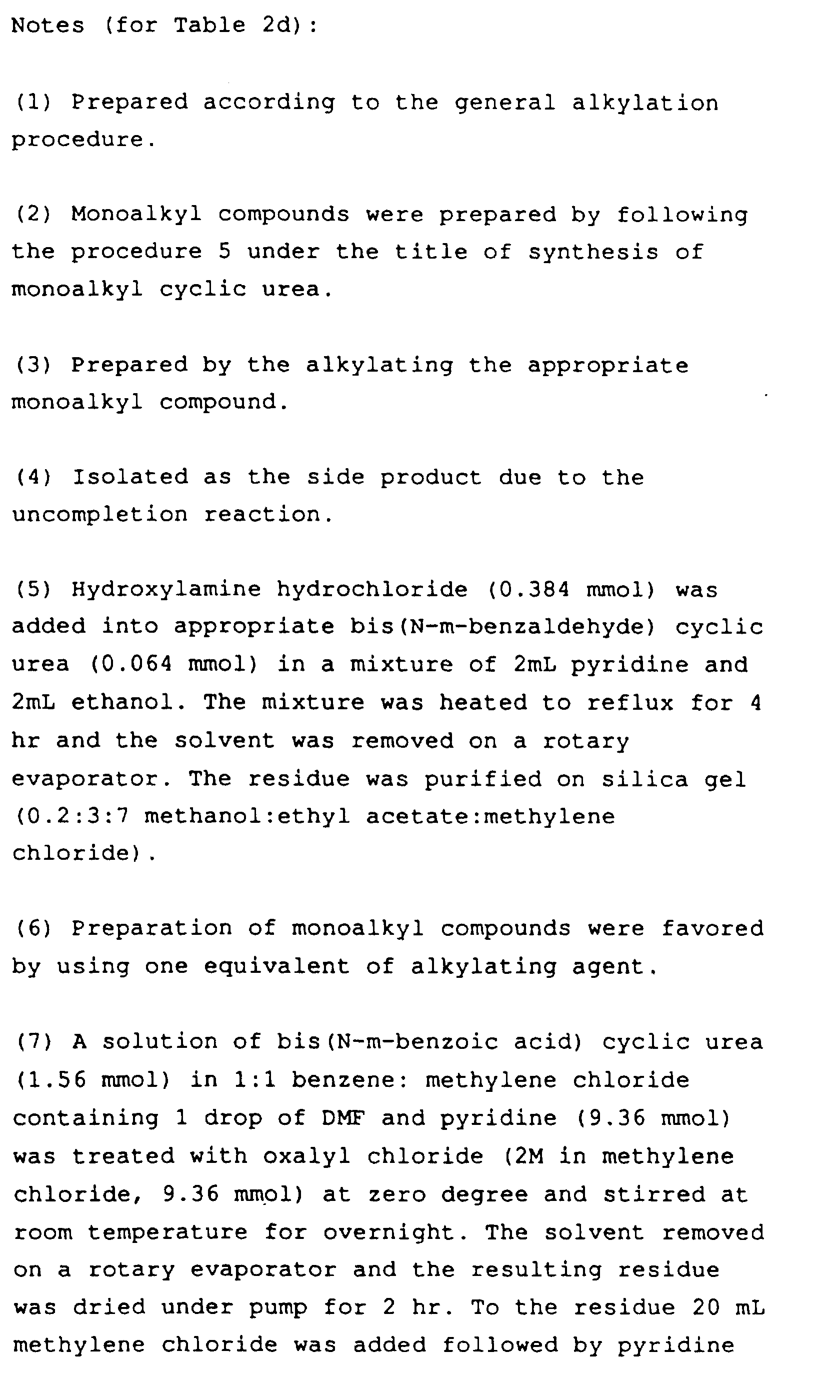

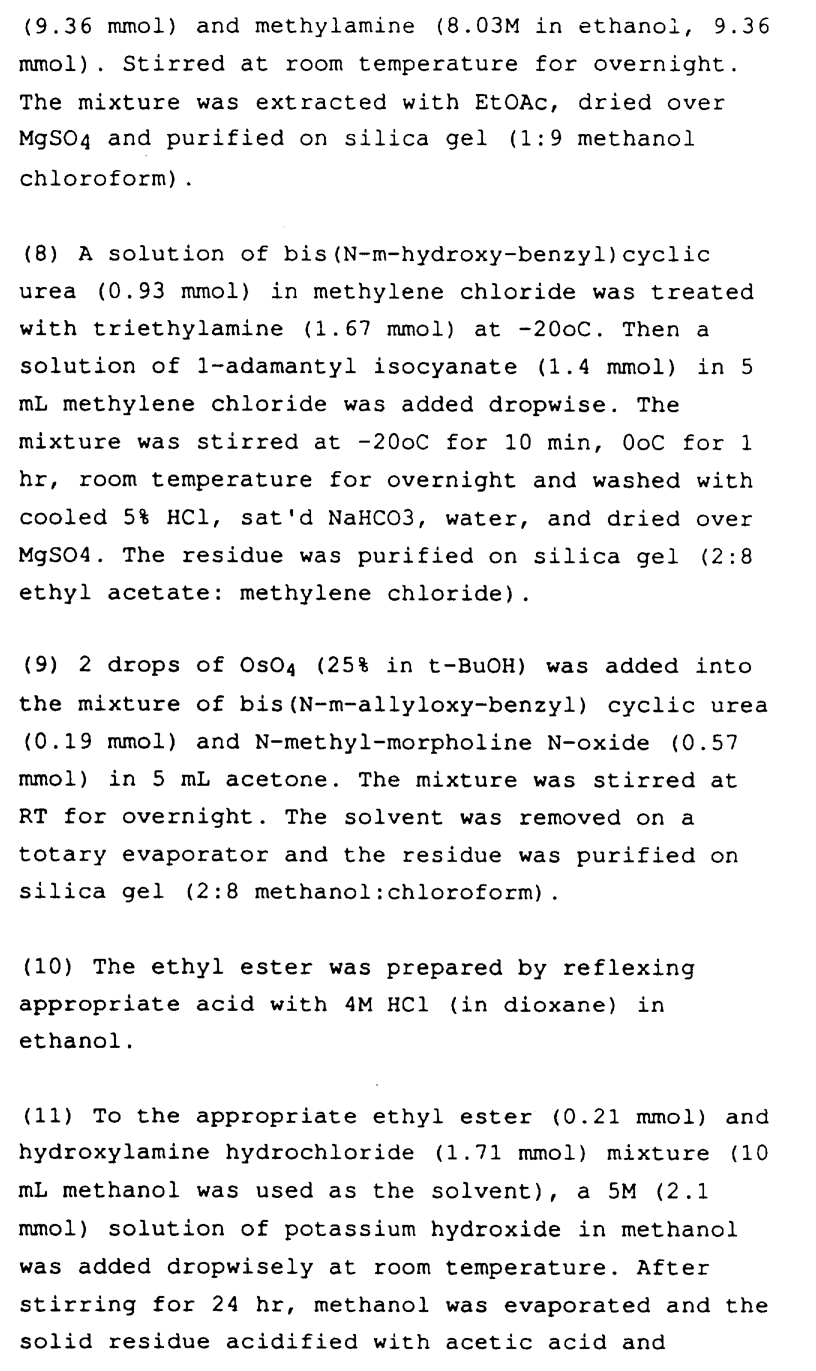

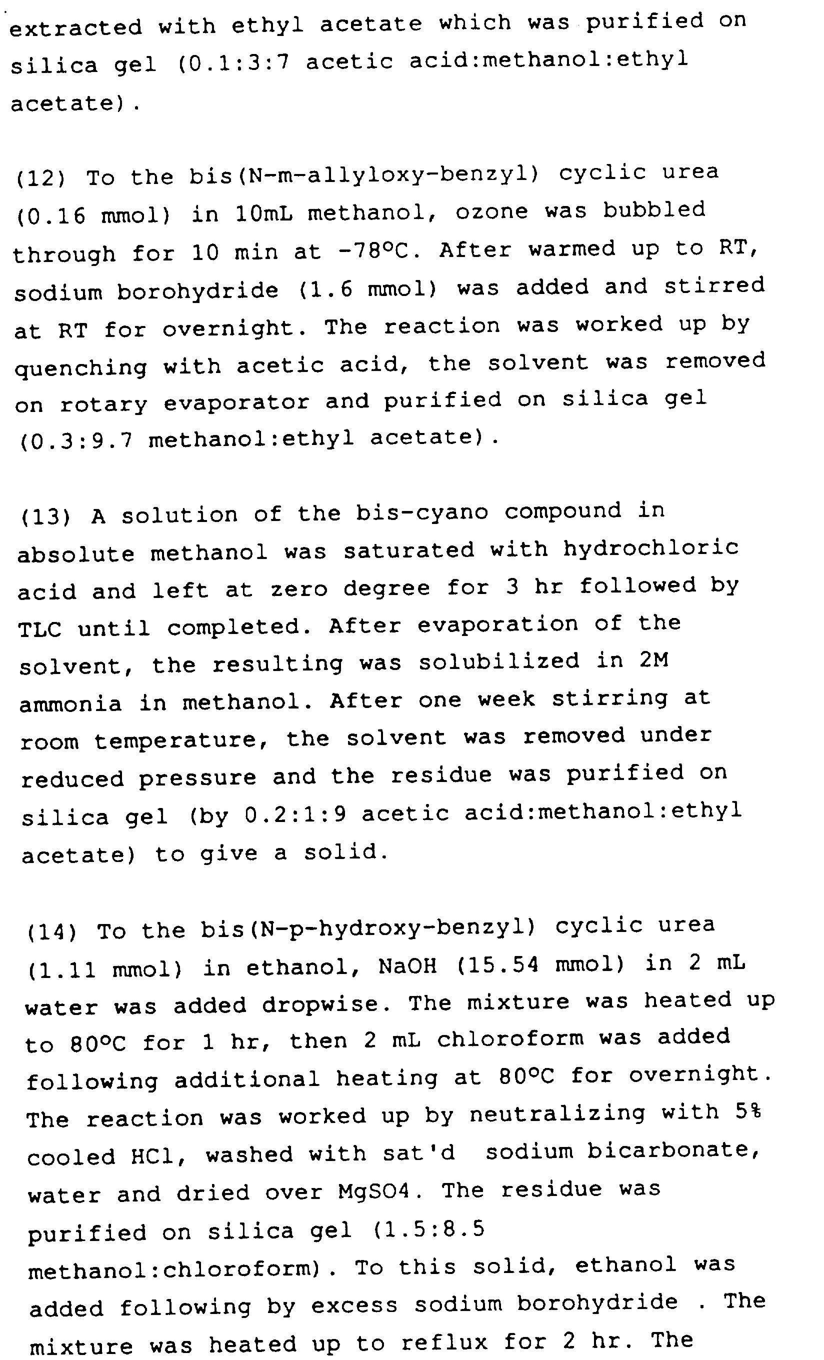

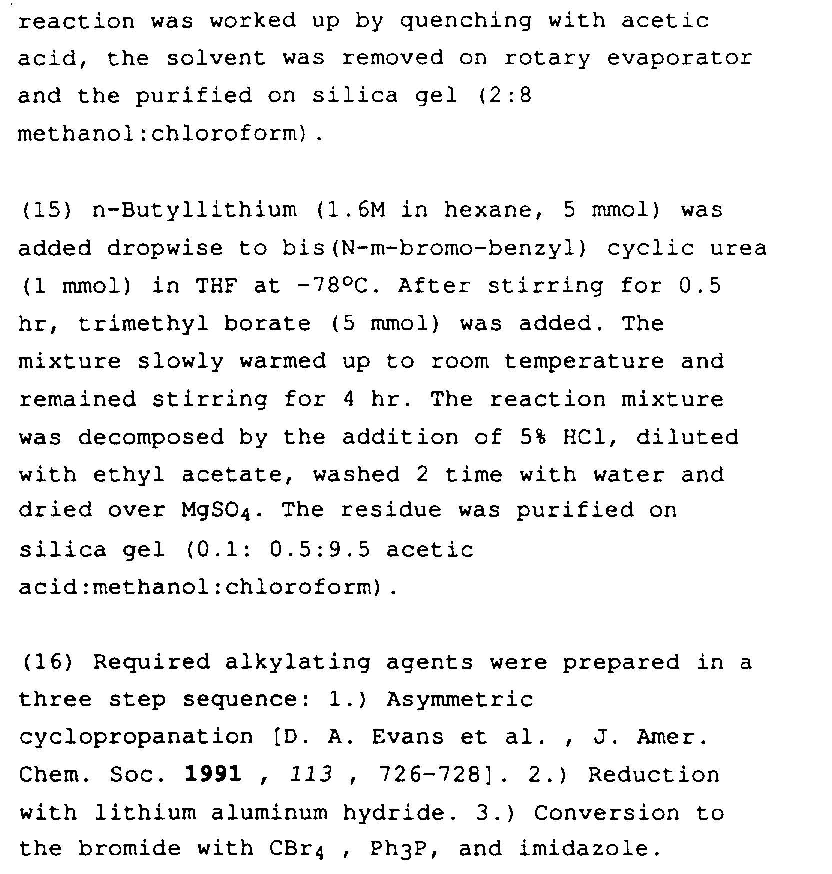

Listed below are a representative list of data for compounds listed in Table 2d:

Example 12A: MS: 525 (M + 1). NMR (CD₃OD): δ 7.30-7.10 (m, 10H), 3.96-3.88 (m, 2H), 3.58-3.42 (m, 4H), 3.37=3.28 (m, 4H), 3.17-3.10 (m, 2H), 2.94-2.78 (m, 6H), 2.20-2.08 (m, 2H), 1.72-1.10 (br m, 14H).

Example 12B: MS: 516 (M + 1, 100). NMR (CD₃OD): δ 6.96-7.72 (br m, 15H), 4.68 (d, 1H), 3.78-3.89 (m, 1H), 3.43-3.64 (m, 3H), 3.28-3.34 (m, 2H), 3.10-3.19 (m, 1H), 2.81-3.02 (m, 6H), 2.12-2.26 (m, 1H), 1.10-1.84 (br m, 9H).

Example 12C: MS: 427 (M + 1, 100). NMR (CDCl₃): δ 7.16-7.36 (m, 10H), 4.82 (d, 1H), 3.88-3.98 (m, 2H), 3.37-3.69 (m, 8H), 3.04-3.19 (m, 3H), 2.63-2.77 (m, 1H), 2.08-2.22 (m, 1H), 1.74-1.88 (m, 2H), 1.10-1.64 (br m, 6H).

Example 12D: MS: 527 (M + 1, 100). NMR (CDCl₃): δ 7.17-7.38 (m, 10H), 3.99 (s, 2H), 3.45-3.75 (m, 8H), 3.03-3.14 (m, 2H), 2.89-3.01 (m, 2H), 2.60-2.68 (m, 2H), 2.12-2.24 (m, 2H), 1.12-1.62 (br m, 18H).

Example 12E: MS: 481 (M + 1, 100). NMR (CDCl₃): δ 7.17-7.37 (m, 10H), 3.94-4.13 (m, 2H), 3.61-3.75 (m, 2H), 3.43-3.61 (m, 4H), 3.05-3.20 (m, 3H), 2.73-3.03 (m, 3H), 2.10-2.23 (m, 1H), 1.98-2.08 (m, 1H), 1.02-1.75 (br m, 9H), 0.82-0.97 (m, 1H), 0.30-0.47 (m, 2H), 0.02-0.13 (m, 2H).

Example 12F: MS: 471 (M + 1, 100). NMR (DMSO-d₆): δ 7.06-7.33 (m, 10H), 5.25 (s, 2H), 4.34 (t, 2H), 3.71 (s, 2H), 3.44-3.58 (m, 2H), 3.20-3.42 (m, 6H), 2.96-3.06 (m, 2H), 2.79-2.91 (m, 2H), 1.83-1.97 (m, 2H), 1.08-1.42 (br m, 8H).

Example 12G: FABMS: 555 (M + 1, 100). NMR (DMSO-d₆): δ 8.41 (s, 4H), 7.06-7.33 (m, 10H), 3.71 (s, 2H), 3.33-3.50 (m, 5H), 3.00-3.09 (m, 2H), 2.75-2.88 (m, 2H), 2.11-2.21 (m, 2H), 2.03-2.11 (m, 3H), 1.90-2.03 (m, 2H), 1.02-1.54 (br m, 10 H).

Example 12H: MS: 583 (M + 1, 100). NMR (CDCl₃): δ 7.12-7.35 (m, 10H), 3.98 (s, 2H), 3.61 (s, 6H), 3.60-3.74 (m, 2H), 3.45-3.58 (m, 2H), 3.03-3.13 (m, 2H), 2.84-3.03 (m, 4H), 2.07-2.28 (m, 6H), 1.08-1.81 (br m, 12H).

Example 12I: MS: 555 (M - CH₂ + 1, 100). NMR (CDCl₃): δ 7.12-7.34 (m, 10H), 3.98 (s, 2H), 3.62 (s, 3H), 3.45-3.76 (m, 6H), 3.04-3.13 (m, 2H), 2.85-2.99 (m, 3H), 2.79 (s, 1H), 2.10-2.28 (m, 4H), 1.08-1.64 (br m, 12H).

Example 12J: MS: 759 (M + 1, 100), 633 (M - I + 1, 33), 507 (M - 21 + 1, 17). NMR (CDCl₃): δ 7.00-7.64 (br m, 18H), 4.77 (d, 2H), 3.70 (s, 2H), 3.49-3.57 (m, 2H), 2.99-3.12 (m, 4H), 2.81-2.93 (m, 2H), 2.29 (s, 2H).

Example 12K: MS: 583 (M + 1, 100). NMR (CDCl₃) : δ 7.15-7.35 (m, 10H), 3.98 (s, 2H), 3.44-3.74 (m, 8H), 3.02-3.22 (m, 2H), 2.88-3.02 (m, 2H), 2.02-2.20 (m, 2H), 0.94-1.92 (br m, 28H).

Example 12L: MS: 495 (M+1,100) NMR (CDCl₃): δ 7.31 (m,10H) 4.22 (m. 4H) 3.76-4.05 (m. 6H) 3.60 (m, 2H) 3.03-3.37 (m, 6H) 1.89 (m, 2H) 0.95 (m, 2H) 0.80 (m, 2H) 0.45 (m, 2H) 0.30 (m, 2H).

Example 12M: MS: 501 (M+1,100) NMR (CDCl₃): δ 7.05-7.38 (m, 15H) 4.84 (dd,1H) 4.04 (m, 1H) 3.93 (m, 1H) 3.49-3.75 (m. 3H) 3.05 (m, 6H) 1.74 (m, 2H) 0.70-0.98 (m, 2H) 0.40 (m, 1H) 0.28 (m, 1H).

Example 12N: MS: 411 (M+1, 100) NMR (CDCl₃): δ 7.25 (m, 10H) 4.72 (bs, 1H) 3.95 (m, 5H) 3.53 (m. 2H) 3.11 (m. 6H) 2.94 (t, 1H) 2.76 (m. 1H) 0.84 (m. 2H) 0.39 (m, 1H) 0.24 (m. 1H).

Example 12o: MS: 441 (M + 1, 100). NMR (CDCl₃): δ 7.17-7.36 (m, 10H), 4.70 (d, 1H), 3.90-4.01 (m, 2H), 3.53-3.68 (m, 4H), 3.37-3.45 (m, 1H), 3.04-3.20 (m, 3H), 2.78-3.03 (m, 1H), 2.68-2.77 (m, 1H), 2.09-2.24 (m, 1H), 1.10-1.95 (br m, 12H).

Example 12P: MS: 555 (M + 1, 100). NMR (CDCl₃): δ 7.13-7.37 (m, 10H), 3.98 (s, 2H), 3.44-3.77 (m, 10H), 3.03-3.14 (m, 2H), 2.80-3.01 (m, 4H), 2.06-2.21 (m, 2H), 1.00-1.75 (br m, 20H).

Example 12Q: m. p. 185.7 ° C. FABMS: 655 (M + 1, 100), 613 (M - HNCNH + 1, 80). NMR (DMSO-d₆): δ 8.99 (br s, 8H), 6.87-7.62 (br m, 18H), 5.08-5.35 (m, 2H), 4.44-4.62 (m, 2H), 3.41-3.61 (m, 4H), 2.96-3.18 (m, 4H), 2.64-2.81 (m, 2H). Reference: K. Takagi, Chemistry Letters (1985), pp. 1307-1308.

Example 12R: m. p. 169.3° C. MS: 599 (M + 1, 100). NMR (CDCl₃): δ 6.90-7.40 (m, 18H), 4.88 (d, 2H), 3.63 (s, 2H), 3.49-3.58 (m, 2H), 2.91-3.12 (m, 6H), 2.42 (s, 6H), 2.22 (s, 2H).

Example 12S: MS: 663 (M + 1), 680 (M + NH₃ + 1). NMR (CDCl₃): δ 6.93-7.83 (br m, 18H), 4.78 (d, 2H), 3.71 (s, 2H), 3.53-3.59 (m, 2H), 3.19 (d, 2H), 3.00-3.14 (m, 4H), 2.98 (s, 6H), 2.75-2.83 (m, 2H).

Example 12T: MS: 415 (M + 1). NMR (Acetone-d₆) : δ 7.15-7.30 (m, 10H), 5.29 (s, 1H), 4.41 (s, 1H), 4.25 (s, 1H), 3.81-4.05 (m, 3H), 3.63-3.78 (m, 2H), 3.56-3.63 (m, 2H), 3.37-3.47 (m, 5H), 3.05-3.25 (m, 3H), 2.79-2.94 (m, 1H), 2.20 (br s, 1H).

Example 12U: MS: 503 (M + 1). NMR (Acetone-d₆) : δ 7.15-7.28 (m, 10H), 4.29 (s, 1H), 3.95-4.07 (m, 4H), 3.49-3.66 (m, 8H), 3.35-3.49 (m, 8H), 3.14-3.22 (m, 2H), 3.02-3.09 (m, 2H), 2.08-2.21 (m, 3H).

Example 12V: MS: 569 (M + 1). NMR (CDCl₃) : δ 7.14-7.34 (m, 10H), 3.90-4.03 (m, 4H), 3.42-3.74 (m, 8H), 3.03-3.14 (m, 2H), 2.86-3.01 (m, 2H), 2.68-2.86 (m, 2H), 2.05-2.23 (m, 2H), 2.01 (s, 3H), 0.98-1.75 (br m, 15 H).

Example 12W: MS: 641 (M + 1), 584 (M - CONHCH₂ + 1), 527 (M - 2CONHCH₂ + 1). NMR (CDCl₃): δ 7.10-7.35 (m, 10H), 4.77 (br s, 2H), 3.89-4.12 (m, 6H), 3.60-3.80 (m, 2H), 3.35-3.47 (m, 2H), 3.04-3.16 (m, 2H), 2.87-3.01 (m, 2H), 2.78 (d, 6H), 2.01-2.16 (m, 2H), 0.98-1.80 (br m, 18H).

Example 12X: MS: 626 (M + 1), 569 (M - CONHCH₂ + 1). NMR (CDCl₃): δ 7.09-7.35 (m, 10H), 4.79 (br s, 1H), 3.82-4.14 (m, 6H), 3.38-3.81 (m, 4H), 3.01-3.22 (m, 2H), 2.83-3.01 (m, 2H), 2.79 (d, 3H), 2.04-2.20 (m, 2H), 2.01 (s, 3H), 0.96-1.84 (br m, 18 H).

Example 12Y: MS: 584 (M + 1), 527 (M - CONHCH₂ + 1). NMR (CDCl₃): δ 7.11-7.41 (m, 10H), 4.81 (br s, 1H), 3.87-4.10 (m, 4H), 3.41-3.76 (m, 6H), 3.02-3.16 (m, 2H), 2.81-3.02 (m, 2H), 2.78 (d, 3H), 2.00-2.21 (m, 2H), 1.79 (br s, 1H), 0.95-1.63 (br m, 18H).

Example 12Z: MS: 425 (M+1, 100).

Example 13A: MS: 439 (M+1, 100).

Example 13B: MS: 481 (M+1, 100).

Example 13C: MS: 635 (M+1, 100) 652 (M+NH₄).

Example 13D: MS: 491 (M+1,100); NMR (CDCl₃): δ 7.24 (m,10H), 5.68 (m,2H), 4.90 (m,4H), 3.98 (s,2H), 3.66 (m,2H), 3.50 (m,2H), 3.04 (m,4H), 2.19 (m,2H), 1.95 (m,4H), 1.22 (m,8H).

Example 13E: MS: 523 (M+1,100); NMR (CDCl₃): δ 7.19 (m,10H), 3.98 (s,2H), 3.66 (m,2H), 3.53 (m,2H), 3.23 (s,2H), 3.14-2.66 (m,8H), 2.40 (m,2H), 2.21 (m,2H), 1.55-1.23 (m,12H).

Example 13F: MS: 811 (M+1,100); NMR (CDCl₃): δ 7.22 (m,10H), 4.00 (m,4H), 3.77 (dd,2H), 3.56 (m,6H), 3.13 (m,4H), 2.93 (m,2H), 2.23 (m,2H), 2.00 (m,2H), 1.65 (m,2H), 1.50-1.23 (m,8H).

Example 13G: MS: 685 (M+1,100); NMR (CDCl₃): δ 7.22 (m,10H), 4.01 (m,2H), 3.72-3.26 (m,12H), 3.14 (d,2H),2.96-2.70 (m,6H), 2.26 (m,2H), 1.43-1.18 (m,8H).

Example 13H: MS: 499 (M+1,100); NMR (CDCl₃): δ 7.23 (m,10H), 3.99 (s,2H), 3.54 (m,8H), 3.12 (m,2H), 2.94 (m,4H), 2.22 (m,2H), 1.47-1.20 (m,12H).

Example 13I: MS: 559 (M+1,100); NMR (CDCl₃/CD₃OD): δ 7.02 (m,10H), 3.79 (s,2H), 3.69 (s,2H), 3.28 (m,6H), 3.13 (m,4H), 2.86 (m,2H), 2.67 (m,2H), 1.95 (m,2H), 1.09-0.90 (m,12H).

Example 13J: MS: 435 (M+1,100); NMR (CDCl₃): δ 7.26 (m,10H), 4.15 (m,1H), 3.86 (m,1H), 3.76 (m,1H), 3.64 (m,1H), 3.53 (m,2H), 3.23-2.97 (m,4H), 2.78 (m,2H), 2.16 (d,1H), 1.92 (m,1H), 1.18 (m,1H), 0.88 (m,1H), 0.58 (m,2H), 0.46 (m,2H), 0.29 (d,1H), 0.07 (d,1H).

Example 13K: MS: 407 (M+1,100); NMR (CDCl₃): δ 7.23 (m,10H), 6.05 (m,1H), 5.67 (m,1H), 5.25 (m,2H), 5.05 (dd,2H), 4.17 (m,2H), 3.96 (m,1H), 3.83 (m,1H), 3.65 (m,1H), 3.55 (m,1H), 3.12-2.79 (m,5H), 2.59 (br s,1H), 2.35 (d,1H).

Example 13L: MS: 507 (M+1,100); NMR (CDCl₃): δ 7.28 (m,18H), 6.91 (d,2H), 4.10 (d,2H), 3.92 (d,1H), 3.58 (m,4H), 3.18-2.63 (m,5H).

Example 13M: MS: 567 (M+1,100); NMR (CDCl₃): δ 7.46-6.95 (m,18H), 4.90 (dd,2H), 4.67 (br d,4H), 3.84 (d,1H), 3.53 (m,4H), 3.09-2.75 (m,5H), 2.44 (d,1H).

Example 13N: MS: 407 (M+1,100); NMR (CDCl₃): δ 7.24 (m,10H), 5.55 (m,2H), 5.14 (m,4H), 4.07 (dd,2H), 3.74 (m,2H), 3.27 (m,2H), 3.12-2.84 (m,6H), 2.09 (s,2H).

Example 13o: MS: 435 (M+1,100); NMR (CDCl₃): δ 7.26 (m,10H), 3.85 (s,2H), 3.41 (m,4H), 3.01 (ddd,4H), 2.28 (dd,2H),2.19 (s,2H), 0.93 (m,2H), 0.50 (m,2H), 0.16 (m,2H).

Example 13P: MS:327 (M+1,100); NMR (CDCl₃): δ 7.28 (m,10H), 4.25 (m,2H), 3.35 (m,6H), 2.81 (m,2H), 2.65 (m,2H).

Example 13Q: MS: 507 (M+1,100); NMR (CDCl₃): δ 7.24 (m,20H), 4.74 (d,2H), 3.55 (m,4H), 3.20 (m,2H), 2.96 (m,2H), 2.77 (m,2H).

Example 13R: MS: 439 (M+1,100); NMR (CDCl₃): δ 7.26 (m,10H), 3.75 (s,2H), 3.62 (m,2H), 3.20 (m,2H), 3.08 (m,2H), 2.84 (m,2H), 2.38 (m,2H), 1.38-1.23 (m,8H), 0.90 (m,6H).

Example 13S: MS: 567 (M+1,100); NMR (CDCl₃): δ 7.34-7.07 (m,18H), 4.68 (m,6H), 3.57 (m,4H), 3.17 (m,2H), 2.96 (m,2H), 2.79 (m,2H).

Example 13T: MS: 617(M+H,100%); NMR (CDCl₃, 300MHz) : δ 7.3 (m, 10H), 6.7 (m,4H), 6.35(m,2H), 4.75(d,2H), 3.5(s,4H), 3.3(m,4H), 3.0(m,6H), 2.7(m,4H), 1.9(m,4H).

Example 13U: m.p. 264-266 °C; MS: 609(M+H,100%); NMR (CDCl₃,300MHz): δ 8.6(d,2H), 7.95(d,2H), 7.8(d,2H), 7.6(d,2H), 7.3(m,14H), 5.1(d,2H), 3.7(m,4H), 3.2(m,6H).

Example 13V: MS: 521(M+H,100%); NMR (CDCl₃,300MHz) : δ 8.8(s,2H), 7.2(m,12H), 4.15(s,2H), 3.8(t,2H), 3.6(m,4H), 2.9(m,4H).

Example 13W: m.p. 182-184 °C; MS: 687(M+H,100%); NMR (CDCl₃,300MHz): δ 7.2(m,10H), 6.95(d,2H), 6.2(d,2H), 4.9(d,2H), 4.15(d,2H), 3.9(bs,2H), 3.7(d,2H), 3.0(m,6H), 1.45(s,18H).

Example 13X: MS: 547(M+H,100%); NMR (CDCl₃, 300MHz) : δ 7.2(m,10H), 6.2(d,2H), 6.0(d,2H), 4.8(d,2H), 4.5(bs,2H), 3.8(s,2H), 3.5(d,2H), 3.1(m,6H).

Example 13Y: MS: 507 (M+1, 100%); NMR (CDCl₃,300MHz) : δ 7.0-7.4 (m, 20H), 4.7-4.8 (d, 2H), 4.5 (m, 2H), 3.45 (m, 4H), 3.1 (m, 4H), 2.9 (m, 2H).

Example 13Z: MS: 577((M+H)⁺, 100%); 594((M + NH₄)⁺, 80%). NMR (CDCl₃): δ 8.08(s 1H), 7.48(dd, 1H, J = 8.4, 1.9 Hz), 7.25-7.38(m, 3H); 7.19(d, 1H, J = 8.4 Hz), 7.00-7.03(m, 2H), 3.96(ABq, 2H), 3.73(d, 1H, J = 1.1 Hz), 3.50(dd, 1H, J = 11.4, 1.1Hz), 3.23(br.s., 1H), 2.98(ABx, 2H).

Example 14A: NMR (CDCl₃): δ 8.27(dd, 1H, J = 4.9, 2.0 Hz), 7.66(dd, 1H, J = 7.5, 2.0 Hz), 7.14-7.27(m, 4H), 6.68(dd, 2H, J = 7.5, 3.6 Hz), 4.16(d, 1H, J = 0.8 Hz), 3.90(ABq, 2H), 3.72(dd, 1H, J = 11.3, 1.1Hz), 3.14(br.s., 1H), 2.87(ABx, 2H).

Example 14B: MS: 685((M+H)⁺, 100%). NMR (CDCl₃) : δ 8.34(dd, 1H, J = 4.8, 1.9 Hz), 7.16-7.37(m, 4H), 6.93(dd, 1H, J = 7.6, 4.8 Hz), 6.87(dd, 2H, J = 7.5, 1.7 Hz), 4.14(s, 1H), 3.90(ABq, 2H), 3.68(td, 1H, J = 6.3, 0.7 Hz), 2.99(d, 2H, J = 6.3 Hz), 2.43(br.s., 1H).

Example 14C: MS: 541((M+H)⁺, 100%). NMR (CD₃OD) : δ 7.53(dd, 1H, J = 9.5, 2.6 Hz), 7.15-7.26(m, 3H), 7.06(d, 1H, J = 2.6 Hz), 6.96-6.99(m, 2H), 6.42(d, 1H, J = 9.5 Hz), 3.80(s, 1H), 3.71(ABq, 2H), 3.62(dd, 1H, J = 11.7, 0.7 Hz), 2.92(ABx, 2H).

Example 14D: MS: 458(M+H⁺, 100%).

Example 14E: MS: 543(M+H⁺, 100%) NMR (CDCl₃): δ 7.4-7.1(m, 5H), 4.42(d, J = 17.5 Hz, 1H), 4.10(s, 1H), 3.83(d, J = 10.6 Hz), 3.2-3.0(m, 2H), 2.82(d, J = 17.6 Hz, 1H), 2.36(s, 1H), 2.14(m, 2H), 1.5-1.2(m, 6H), 0.85(t, J = 7.0 Hz, 3H).

Example 14F: MS: 739.5(M+H⁺) NMR (CDCl₃): δ 7.4-7.0(m, 15H), 3.90(s, 1H), 3.3-3.1(m, 2H), 3.04(m, 2H), 2.43(dd, J = 14.7, 5.7 Hz, 1H), 2.17(s, 1H), 1.824(m, 1H), 1.26(t, J = 6.0 Hz, 1H), 1.02(dd, J = 9.0, 5.1 Hz, 1H)

Example 14G: MS: 479(M+H⁺, 100%) NMR (CDCl₃): δ 7.24(bs, 5H), 7.15-6.9(m, 3H), 6.72(d, J = 7.5 Hz, 2H), 4.15(s, 1H), 4.06(d, J = 10.5 Hz, 1H), 3.5-3.2(m, 2H), 2.98(s, 1H).

Example 14H: MS: 587(M+H⁺, 100%) NMR (CDCl₃): δ 7.4-6.9(m, 10H), 3.92(s, 1H), 3.8-3.6(m, 2H), 3.03(m, 2H), 2.50(bs, 1H), 2.19(dd, J = 14.3, 7.0 Hz, 1H), 1.57(m, 1H), 1.0-0.7(m, 2H).

Example 14I: MS: 457(M+H⁺, 100%), 474(M + NH₄⁺, 15%) NMR (CDCl₃): δ 7.4-6.9(m, 15H), 4.69(d, J = 6.2 Hz, 1H), 3.95(m, 2H), 3.77(m, 2H), 3.37(m, 1H), 3.25-3.0(m, 3H), 2.15(b, 1H), 1.61(m, 2H), 1.24(m, 1H), 1.0-0.7(m, 2H).

Example 14J: NMR (CDCl₃): δ 7.42(d, 2H, J = 7.3 Hz), 7.22-7.38(m, 6H), 7.11(d, 2H, J = 6.6 Hz), 6.54(s, 1H), 6.35(s, 1H), 5.31(s, 2H), 3.84(ABq, 2H), 3.72(s, 1H), 3.49(d, 1H, J = 10.4 Hz), 2.90-3.01(m, 2H), 2.39(s, 3H).

Example 14K: NMR ((CD₃)₂SO): δ 7.00-7.33(m, 7H), 3.61(s, 1H), 3.55(ABq, 2H), 3.45(d, 1H, J = 11.0 Hz), 2.95(ABₓ, 2H), 2.09(s, 3H,).

The structures of the Examples below are shown in Table 2e.

### Ketal formation: Preparation of Triacetonide (XXVIa):

Lithium borohydride (1.2 gr, 56.2 mmol) was added in four portions to a suspension of L-mannonic-g-lactone (5 gr, 28.1 mmol) in methanol (250 mL) at 0°C over 10 min. Ice bath was removed and reaction stirred at room temperature for 30 min. Reaction was quenched at 0°C with 2N HCl. Solvent was evaporated and residue taken up in acetone (75 mL) to which 2,2-dimethoxypropane (20 mL, 168.6 mmol) and camphorsulphonic acid (20 gr, 84.3 mmol) were added in four portions. Reaction becomes clear for a few minutes and then a precipitate forms. Reaction stirred at room temperature for 14 h. Solvent volume then reduced by 2/3 at reduced pressure and then poured into EtOAc, washed with saturated NaHCO₃, dried (MgSO₄) and concentrated. Solid residue taken up in hexane and filtered thru a pad of silica gel. Filtrate concentrated to give triacetonide (XXVIa) as a yellowish solid (7.1 gr, 80%). m.p. 72-74 °C; MS: 303 (M+H,100%); NMR (CDCl₃,300MHz): δ 4.25 (m, 2H), 4.15 (m, 2H), 4.05 (m, 4H), 1.5 (s, 6H), 1.45 (s, 6H), 1.4 (s, 6H).

### Selective Acetonide Deprotection: Preparation of Tetraol (XXVIb) :

Compound (XXVIa) (14 gr) in 70% AcOH (200 mL) was stirred at 45 °C for 2 h. Solvent removed at reduced pressure with a bath temperature of 45 °C. Residue recrystalized from ether. Mother liquors concentrated and chromatographed (silica, 10% methanol in methylene chloride) to give the desired product as a white solid (8.2 gr, 80%). m.p. 91-93 °C; [a]_{D}= -26.40 (c=3, H20); MS: 240 (M+NH₄,100%); NMR (CDCl₃,300MHz): δ 3.95 (m, 6H), 3.75 (m, 4H), 2.5 (bs, 2H), 1.4 (s,6H).

### Epoxide Formation: Preparation of Diepoxide (XXVIc):

A solution of Compound (XXVIb) (1 g, 4.5 mmol) in pyridine (5 mL) was cooled to -20 °C and treated with p-toluenesulfonyl chloride (1.89 g, 10 mmol). Stirring continued at -20 °C for 20 min, 0 °C for 20 min, and 23 °C for 20 min. The reaction was then diluted with methylene chloride and washed with 2N HCl and NaHCO₃. The organic extract was dried over MgSO₄ and concentrated. The crude product was then taken up in methanol (14 mL) and cooled to 0°C. Next, K₂CO₃ (3.11 g, 22 mmol) was added and the reaction stirred at room temperature for 30 min. The methanol was then stripped off (do not evaporate to dryness, epoxide is volatile) and the crude was washed with water, extracted with ether, dried over MgSO₄, filtered, and concentrated. The compound was purified on SiO₂ and eluted with 30-60% ether/petroleum ether to afford the diepoxide (0.63 g, 75%) as an oil. NMR (CDCl₃,300MHz): δ 3.81 (m, 2H), 3.10 (m, 2H), 2.80 (t, 2H), 2.68 (m, 2H), 1.40 (s, 6H, CH₃).

### Opening of Epoxide: Prepepartion of Diol (XXVId):

To a suspension of cuprous bromide-dimethyl sulfide complex (1.8 g, 8.7 mmol) in anhydrous THF (5 mL) at -20 °C was added 8.5 ml benzylmagnesium chloride (2M in THF, 17 mmol). Reaction stirred at -20 °C for 30 min and at 0 °C for 1 h. Next, Compound (XXVIc) (0.54 g, 3 mmol) in THF (5 mL) was added, and the reaction stirred at 0 °C for 1 h. The excess reagent was quenched with saturated NH₄Cl solution and allowed to warm to room temperature. The contents were then washed with water and brine, extracted with ether, dried over MgSO₄, filtered, and concentrated. Crude material was then purified by flash chromatography (30-60% ether/petroleum ether) to yield 0.84 g (78%) of an oil. MS: 371 (M+H,66%); NMR (CDCl₃,300MHz): δ 7.2-7.4 (m, 10H), 4.65 (s, 2H), 3.6-3.8 (m, 4H), 2.6-3.0 (m, 4H), 1.8-2.2 (m, 4H), 1.4 (s, 6H).

### Hydroxyl Displacement: Preparation of Diazide (XXVIe):

To a solution of Compound (XXVId) (0.48 g, 1.3 mmol) and triphenyl phosphine (1.0 g, 3.9 mmol) in THF (5 mL) at 0 °C was added diethylazodicarboxylate (0.61 mL, 3.9 mmol) and dipheylphosphorylazide (0.84 mL, 3.9 mmol). Contents were allowed to warm to room temperature in the ice bath for 1 h. The excess reagents were quenched by the addition of methanol (0.2 mL, 5 mmol) at 0 °C. The mixture was then stirred at room temperature for 30 min and then concentrated to a small volume. Crude was then purified on SiO₂ using 1:40 ethyl acetate/hexane giving 0.245 g (45%) of an oil. MS: 438 (M+NH₄,8%); NMR (CDCl₃,300MHz): δ 7.2-7.4 (m, 10H), 4.18 (s, 2H), 2.7-3.0 (m, 6H), 2.0-2.3 (m, 4H), 1.58 (s, 6H).

### Reduction of Diazide (XXVIe) :

To Compound (XXVIe) (0.245 g, 0.58 mmol) in ethanol (6 mL) under N₂ was added 10% Pd/C (73.5 mg, 30%/weight). Reaction stirred under H₂ atmosphere at room temperature overnite. Crude was then filtered through celite and concentrated. 0.21 g (98%) of the diamine was collected as an oil and taken onto next step without further purification. MS: 369(M+H,100%); NMR (CDCl₃,300MHz); δ 7.05-7.3 (m, 10H), 3.9 (bs, 2H), 3.05 (bs. 4H), 2.8 (m, 2H), 2.6 (m, 4H), 1.7 (bs, 4H), 1.35 (s, 6H).

### Cyclization of the Diamine: Formation of Cyclic Urea (XXVIf):

The diamine (0.21 g, 0.57 mmol) was dissolved in methylene chloride (50 mL) and carbonyldiimidazole (0.102 g, 0.63 mmol) was added and the reaction stirred at 23 °C overnite. The solution was then concentrated and purified on SiO₂ using 75% ethyl acetate/hexane as elutent which gave 85mg (38%) of (XXVIf) as a foam. MS: 395 (M+H,100%); NMR (CDCl₃,300MHz): δ 7.0-7.2 (m, 10H), 3.6-4.0 (m, 4H), 3.6-2.7 (m, 4H), 1.8-1.9 (m, 4H), 1.3 (s, 6H).

### Alkylation of the Cyclic Urea (XXVIf) :

To Compound (XXVIf) (85mg, 0.22 mmol) in dry DMF (3 mL) was added 60% NaH (0.07 g, 1.7 mmol). The solution was stirred for 5 min at room temperature. Next, benzyl bromide (0.1 mL, 0.86 mmol) was added and the reaction stirred at 23 °C overnite. Reaction was then quenched with methanol (several drops), washed with H₂O, extracted with ether, dried (MgSO₄), and concentrated. Crude was then purified on silica gel using 1:1 hexane/ethyl acetate affording 0.03 g (25%) of the bis-alkylated urea as a foam. MS: 575 (M+H,100%); NMR (CDCl₃, 300MHz): δ 7.1-7.4 (m, 20H), 5.1 (d, 2H), 4.0 (d, 2H), 3.75 (bs, 2H), 3.6 (m, 2H), 2.7 (m, 2H), 2.6 (m, 2H), 1.9-2.0 (m, 4H), 1.25 (s, 6H).

### Deprotection of Acetonide: Preparation of Example 16A:

To above prepared bis-alkylated cyclic urea (0.03 g, 0.05 mmol) in THF (2 mL) at room temperature was added several drops of concentrated HCl. Reaction stirred at room temperature for 2 h. Reaction was then washed with 1 N NaOH, extracted with ethyl acetate, dried (MgSO₄), and concentrated. Chromatography (silica, 1-5% methanol in methylenechloride) gave 0.024 g (85%) of example 16A as a foam. MS: 535 (M+H,100%); NMR (CDCl₃,300MHz): δ 7.1-7.3 (m, 20H), 5.15 (d, 2H), 3.9 (d, 2H), 3.5 (bs, 2H), 3.3-3.4 (m, 2H), 2.7-2.8 (m, 2H), 2.5-2.6 (m, 2H), 2.0-2.1 (m, 6H).

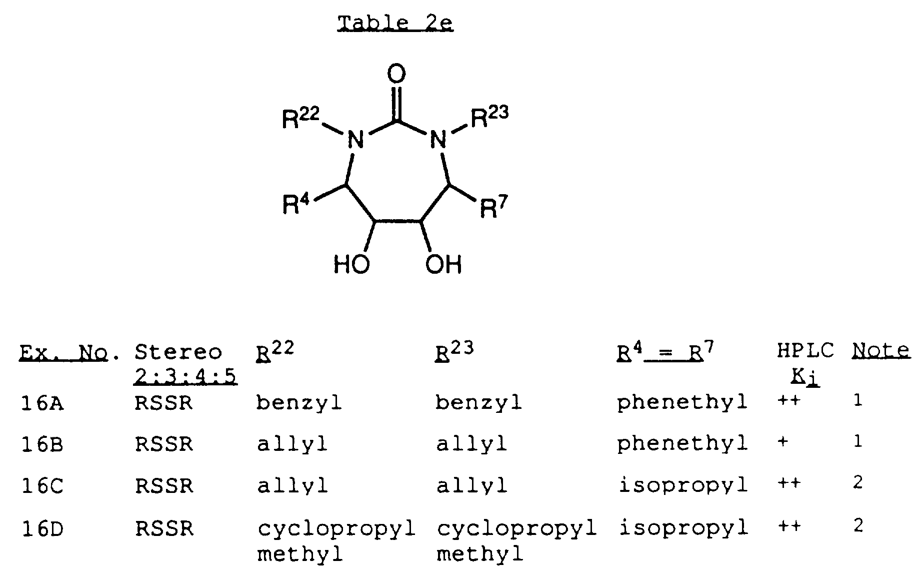

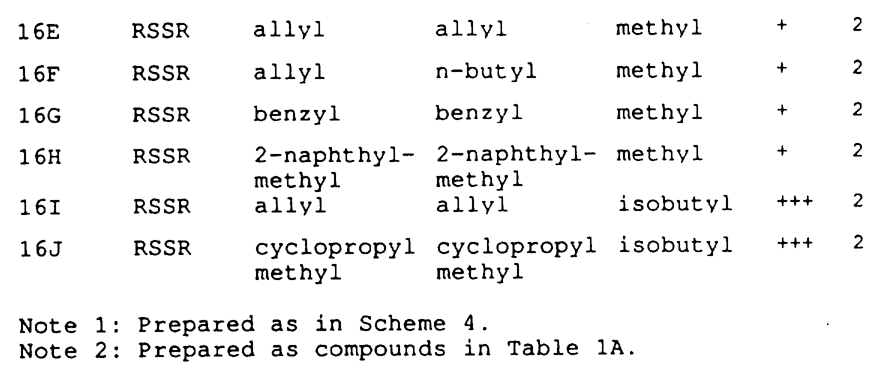

Listed below are physical data for representative compounds of the invention.

Example 16A: MS: 535 (M+1, 100%); NMR (CDCl₃,300MHz): δ 7.1-7.3 (m, 20H), 5.15 (d, 2H), 3.9 (d, 2H), 3.5 (bs, 2H), 3.3-3.4 (m, 2H), 2.7-2.8 (m, 2H), 2.5-2.6 (m, 2H), 2.0-2.1 (m, 6H).

Example 16B: MS: 435 (M+1, 100%); NMR (CDCl₃,300MHz): δ 7.1-7.3 ( m, 10H), 5.8 (m, 2H), 5.15 (s, 2H), 5.1 (d, 2H), 4.5-4.6 (m, 2H), 3.8 (s, 2H), 3.3-3.5 (m, 4H), 2.5-2.9 (m, 4H), 2.2 (m, 2H), 2.0 (m, 4H).

Example 16C: MS: 312 (22, M + 2), 311 (100, M + 1), 267 (1). HRMS: Calc. 311.2334. Found: 311.2330. NMR (CDCl₃): δ 5.81(m, 2H), 5.2(m, 4H), 4.4(m, 2H), 4.0(br s, 2H), 3.4 (m, 2H), 3.1(br s, 2H), 3.0(m, 2H), 2.4(m, 2H), 1.2(d, 6H), 0.9(d, 6H).

Example 16D: MS: 341(4), 340 (25), 339 (100, M + 1), 321 (1), 295 (2), 256 (2). HRMS: Calc. 339.2647. Found: 339.2652. NMR (CDCl₃): δ 4.2(brs, 2H), 3.65(m, 2H), 3.20(m, 4H), 2.6(m, 4H), 1.2(d, 6H), 1.0(m, 2H), 0.9(d, 6H), 0.5(m, 4H), 0.2(m, 4H).

Example 16E: MS: 256 (15, M + 2), 255 (100, M + 1). HRMS: Calc. 255.1706. Found: 255.1708.NMR (CDCl₃) δ 5.8(m, 2H), 5.2(m, 4H), 4.0(m, 2H), 3.8(br s, 2H), 3.65(m, 2H), 3.4(m, 2H), 2.8(m, 2H), 1.2(d, 6H).

Example 16F: MS: 272 (16, M + 2), 271 (100, M + 1). HRMS: Calc. 271.2021. Found: 271.2036. NMR (CDCl₃): δ 5.8(m, 1H), 5.2(m, 2H), 4.0(m, 2H), 3.8(br s, 2H), 3.6-3.3(3H), 3.0(m, 2H), 2.45(m, 2H), 1.35(m, 2H), 1.2(d, 6H), 0.9(m, 4H).

Example 16G: MS: 356 (23, M + 2), 355 (100, M + 1). HRMS: Calc. 355.2021. Found: 355.2012. NMR (CDCl₃): δ 7.4-7.2(m, 10H), 4.9(d, 2H), 4.2(d, 2H), 3.6(br s, 2H), 3.3(m, 2H), 2.2(m, 2H), 1.2(d, 6H).

Example 16H: mp 236-238°C. MS: 456 (29, M + 2), 455 (100, M + 1), 315 (10), 158 (5). HRMS: 455.2334. Found: 455.2333. NMR (CDCl₃): δ 7.9-7.8(m, 8H), 7.6-7.45(m, 6H), 5.0(d, 2H), 4.4(m, 2H), 3.6(br s, 2H), 3.4(m, 2H), 1.9(m, 2H), 1.2(m, 2H).

Example 16I: MS 339 (100, M+1), 311 (4). HRMS 339.265377 (calc. mass = 339.264768). NMR (CDCl₃): δ 5.91-5.79 (m), 5.29-5.18 (m, 4H), 4.53-4.46 (m, 2H), 3.76 (s, 2H), 3.41-3.33 (m, 4H), 2.13 (s, 2H), 1.81-1.71 (m, 2H), 1.71-1.62 (m, 2H), 1.45-1.35 (m, 2H), 0.90 (t, 12H).

Example 16J: m.p. 137-139 °C. MS 367 (100, M+1). HRMS 367.295357 (calc. mass = 367.296068). NMR (CDCl₃): δ 3.94 (s, 2H), 3.82 (d, 2H), 3.77 (d, 2H), 3.48 (d, 2H), 2.91 (bs, 2H), 2.63 (d, 2H), 2.61 (d, 2H), 1.99 (m, 2H), 1.70-1.61 (m, 2H), 1.43 (m, 2H), 1.26 (s, 2H), 1.07-0.98 (m, 2H), 0.91 (t, 12H), 0.52 (m, 2H), 0.22 (m, 2H).

The structures of the Examples below are shown in Table 2f-h.

### Synthesis of Thiourea (XXVIIa) :

Diaminodimem Compound (XXIb) (22.45g, 47.1 mmol) was dissolved in 200 mL of tetrahydrofuran and to this solution was added 9.23g (51.8 mmol) of thiocarbonyl diimidazole. After stirring the mixture for 18 hours at room temperature TLC (10:1:10 ethyl acetate: ethanol: hexane) indicated complete reaction. The reaction mixture was taken to dryness and the solid residue purified by flash chrmatography (silica gel, 250g, 1:1 ethyl acetate: hexane) to provide solid which was triturated with hexane to provide 17.8g (73% yield) of XXVIIa as a white solid.

### Synthesis of Compound (XXVIIb) :

Compound (XXVIIa) (3.108g, 6mmol) was dissolved in 15ml acetonitrile and to this solution was added methyl iodide 1.5ml (24mmol) via syringe and stirred at room temperature for one hour. The contents were then taken to dryness. The residue was dissolved in 30ml dimethylformamide and to this solution, cooled in a 0°C ice bath, was added NaH (60% in oil) 720mg (18mmol) slowly (EVOLUTION!). The contents were stirred at room temperature for 30 minutes. The mixture was cooled in a 0°C ice bath and benzyl bromide (2.052g, 12mmol) was added via syringe and stirred at room temperature for 18 hours. TLC (2:3 EtOAc:Hexane R_{f}=0.25) indicated a complete reaction. The reaction was worked up by diluting with water (300ml) and extracting with diethyl ether (3x50ml). The organic layer was dried over magnesium sulfate and the filtrate taken to dryness. The residue was purified on SiO₂ gel (200g; 2:3 EtOAc:Hexane) to provide 2.923g (78.2% yield) of XXVIIb as a colorless oil.

### Synthesis of Compounds (XXVIIc) and (XXVIId) :

Compound (XXVIIb) (2.900g, 4.65mmol) was dissolved in 25ml pyridine and to this solution was added 742mg (4.65mmol) benzylhydroxylamine hydrochloride. The contents were refluxed in a 125°C oil bath for 18 hours. (Caution: Methyl mercaptan is a by-product and the reaction should be vented to a Clorox scrubber). TLC indicated a complete reaction. The reaction was diluted with 150ml dichloromethane. The organic layer was washed with 1N HCl (2x300ml) followed by sat. sodium bicarbonate solution (100ml). It was separated and dried over magnesium sulfate and the filtrate taken to dryness. The residue was purified on SiO₂ gel(130g; using 1:3 EtOAc:Hexane) to provide 584mg (18.0% yield) of Compound (XXVIIc) as a colorless oil. 1:2 EtOAc:Hexane was used to provide 2.113g of a side product thiourea (XXVIId).

### Synthesis of Oxime (XXVIIe):

Compound (XXVIIc) (584mg, 0.84mmmol) was dissolved in 5ml dimethylformamide and to this solution, cooled in a 0°C ice bath, was added NaH( 60% in oil) 80mg (2mmol) slowly (EVOLUTION!). The contents were stirred at room temperature for 30 minutes. The mixture was cooled in a 0°C ice bath and benzyl bromide (0.24ml, 2mmol) was added via syringe and stirred at room temperature for 18 hours. TLC (1:3 EtOAc:Hexane R_{f}=0.2 6) indicated a complete reaction. The reaction was worked up by diluting with water (50ml) and extracting with diethyl ether (2x25ml). The organic layer was dried over magnesium sulfate and the filtrate taken to dryness. The residue was purified on SiO₂ gel (33g; 1:3 EtOAc:Hexane) to provide 491mg (74.2% yield) of a colorless oil.

### Example 18A

Compound (XXVIId) (450mg, 0.57mmol) was placed in a 25ml R.B. Flask and cooled in a 0°C ice bath. To this flask was added 4M HCl in dioxane (5ml, 20mmol) and the mixture stirred at room temperature for 18 hours. TLC (2:3 EtOAc:Hexane Rf=0.29) indicated a complete reaction. The mixture was worked up by quenching in sat.sodium bicarbonate solution (50ml) and extracting with dichloromethane (2x50ml). The organic extracts were dried over magnesium sulfate and the filtrate taken to dryness. The residue was purified on SiO₂ gel(33g; 2:3 EtOAc:Hexane) to provide 246mg (70.5% yield) of Example 18A as a waxy solid.

### Example 17A

Example 18A (160mg, 0.26mmol) was dissolved in 5ml ethanol. To this mixture was added 50mg of 10% palladium hydroxide on Carbon and the suspension stirred for 18 hours under hydrogen(1 atm). TLC (10:1:10 EtOAc:EtOH: Hexane R_{f}=0.3) indicated a complete reaction. The suspension was filtered through a celite pad and the filtrate taken to dryness. The residue was purified on SiO₂ gel (33g; 10:1:10 EtOAc:EtOH:Hexane) to provide 97mg of Example 17A (69.5% yield) as a white solid.

### Example 19A

Compound (XXVIId) (500mg, 0.82mmol) was placed in a 25ml R.B. Flask and cooled in a 0°C ice bath. To this flask was added 4M HCl in dioxane (7.5ml, 30mmol) and the mixture stirred at room temperature for 18 hours. TLC (1:2 EtOAc:Hexane R_{f}=0.29) indicated a complete reaction. The mixture was worked up by quenching in sat.sodium bicarbonate solution (50ml) and extracting with dichloromethane (2x50ml). The organic extracts were dried over magnesium sulfate and the filtrate taken to dryness. The residue was purified on SiO₂ gel (33g; 1:2 EtOAc:Hexane) to provide 181mg (51.1% yield) of Example 19A as a white solid.

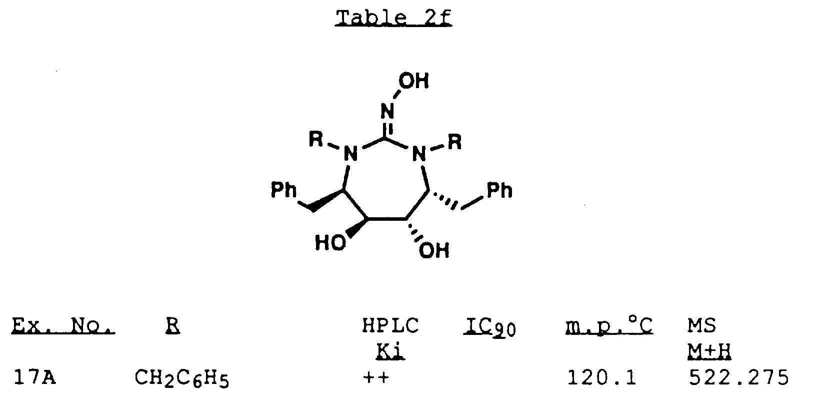

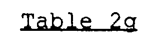

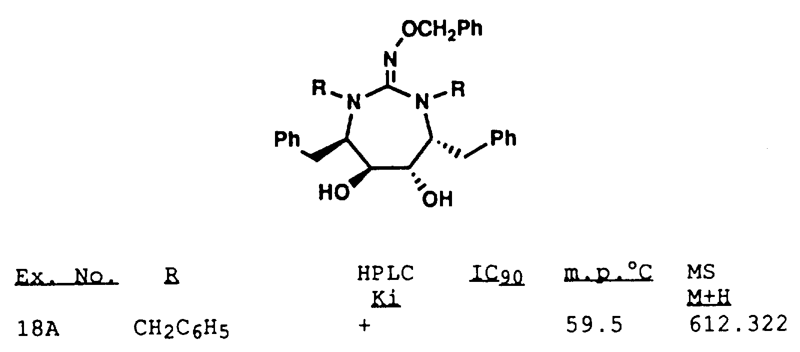

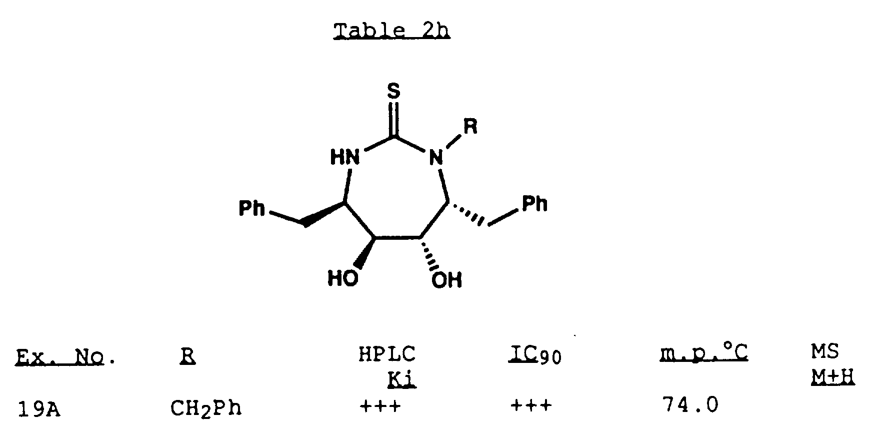

The structures of the Examples below are shown in Table 2i.

### Acetylation of Diol: Compound (XXVIIIa):

Example 1X (3.517g, 7.58mmol) was dissolved in 25ml pyridine and to this solution, cooled in a 0°C ice bath, was added 350mg 4-Dimethylaminopyridine and 7.16ml (75.85mmol) acetic anhydride. The contents were stirred at room temperature for 18 hours. TLC (1:4 EtOAc:Hexane R_{f}=0.3) indicated a complete reaction. The reaction was diluted with 250ml dichloromethane. The organic layer was washed with 1N HCl (2x300ml) followed by sat. sodium bicarbonate solution (100ml). It was separated and dried over magnesium sulfate and the filtrate taken to dryness. The residue was purified on SiO₂ gel (200g; 1:5 EtOAc:Hexane) to provide 2.632g (67.0%) of XXVIIIa as a white solid.

### Nitration of Benzyl Group: Compound (XXVIIIb) and (XXVIIIc):

Compound (XXVIIIa) (518mg, lmmol) was dissolved in 4ml acetonitrile and to this solution, cooled in a -40°C dry ice-acetone bath, was added 4.4ml (2.2mmol) 0.5M Nitronium tetrafluoroborate in sulfolane. The contents were stored in a -40°C freezer for 18 hours. TLC indicated a complete reaction. The reaction was diluted with 100ml ether and washed with water (2x50ml). The organic layer was dried over magnesium sulfate and the filtrate taken to dryness. The residue was purified on SiO₂ gel (75g; 1:3 EtOAC:Hexane for XXVIIIb, 1:2 EtOAc:Hexane for XXVIIIc) to provide 106mg (17.4% yield) of XXVIIIb as a white solid and 159mg(26.2% yield) of XXVIIIc as a white solid.

### Example 20A

Compound (XXVIIIb) (106mg, 0.174mmol) was dissolved in 5ml methanol and to this solution was added 0.5ml 0.5M sodium methoxide in methanol via syringe. The contents were stirred at room temperature for 30 minutes. TLC indicated a complete reaction. The mixture was quenched by adding 500mg of AG50W-X8 acid resin and stirring the suspension at room temperature for 5 minutes. The filtrate was taken to dryness and the residue purified on SiO₂ gel (33g; 1:2 EtOAc:Hexane) to provide 43mg (47.1% yield) of Example 20A as a white solid.

### Example 20B

Compound (XXVIIIc) (159mg, 0.261mmol) was dissolved in 5ml methanol and to this solution was added 0.5ml 0.5M sodium methoxide in methanol via syringe. The contents were stirred at room temperature for 30 minutes. A white precipitate started forming after 15 minutes. TLC indicated a complete reaction. The mixture was quenched by adding 500mg of AG50W-X8 acid resin and stirring the suspension at room temperature for 5 minutes. 10ml dichloromethane was then added to solubilize the solid. The filtrate was taken to dryness and the residue provided lllmg (81.1% yield) of Example 20B as a white solid.

### Example 20E

Example 20A (100mg, 0.191mmol) was dissolved in 5ml ethanol. To this mixture was added 50mg of 5% palladium on Carbon and the suspension stirred for 18 hours under hydrogen(1 atm). TLC indicated a complete reaction. The suspension was filtered through a celite pad and the filtrate taken to dryness. The residue provided 47mg (53.0% yield) of Example 20E as a white solid.

### Example 20F

Example 20B (100mg, 0.191mmol) was suspended in 5ml ethanol. To this mixture was added 50mg of 5% palladium on Carbon and the suspension stirred for 18 hours under hydrogen(1 atm). The starting material went into solution as the reaction progressed. TLC indicated a complete reaction. The suspension was filtered through a celite pad and the filtrate taken to dryness. The residue provided 49mg (55.2% yield) of example 20F as a white solid.

### Example 20G

A. Synthesis of 4-Fluorobenzyl Cyclic Urea (XXXI):

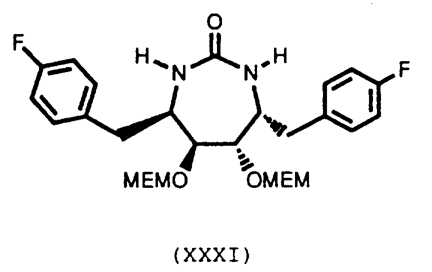

The synthesis of 4-fluorobenzyl cyclic urea is outlined in Scheme 7. N-acetyl-D-4-fluorophenylalanine methyl ester (23.9 g, 0.1 mol), obtained using the procedure of M.J. Burk (*J. Am. Chem. Soc.* **1991***, 113*, 8518), was dissolved in 40 mL of acetic acid and treated with 100 mL of concentrated HCl, 40 mL of water and heated to reflux for 5 hrs. The solution was cooled to room temperature and then made basic (pH = 10) with 50% NaOH while cooling in an ice bath. Benzyl chloroformate (25 mL, 29 g, 0.17 mol) and NaOH are added in four portions and the solution is maintained alkaline by the addition of NaOH. The mixture is then stirred at rt for 30 min. The alkaline solution is extracted with ether (2 X 500 mL) and the solution acidified with conc HCl to pH 1. The precipitate is extracted into methylene chloride and dried over MgSO₄. The solution is filtered and concentrated to give 20 g of the N-Cbz-D-4-fluorophenylalanine as a white solid that is used without further purification.

A solution of N,O-dimethylhydroxylamine hydrochloride (8.0 g, 0.082 mol) in DMF is prepared by gentle warming. The solution is allowed to cool slightly and treated with N-methylmorpholine (8.2 g, 0.082 mol) and diluted with THF to facilitate transfering of the resulting thick suspension.

A solution of N-Cbz-D-4-fluorophenylalanine (20 g, 0.063 mol) in THF is treated with N-methylmorpholine (9.0 g 0.09 mol) and cooled to 0°C in an ice bath. To the stirred cold solution is added isobutyl chloroformate (8.6 g, 0.063 mol) in small portions over a period of 10 mins. Then the solution of N,O-dimethylhydroxylamine in DMF prepared above is added and the reaction mixture is stirred for 20 mins. Most of the solvent is removed on a rotorary evaporator and the residue is partitioned between water and methylene chloride. The organic layer is washed successively with 1 N HCl, 1 N NaOH, water, brine and then dried over MgSO₄. The solution is then filtered and concentrated and the residue chromatographed on silica gel (50% EtOAc/Hex) to give 16 g of the amide.

Using the procedure of by J-A. Fehrentz and B. Castro *(Synthesis,* **1983,** 676) 11 g (0.031 mol) of N-Cbz-D-4-fluorophenyl-alanine N,O-dimethylhydroxylamide was converted to 9.0 g of N-Cbz-D-4-fluorophenylalaninal obtained as a thick oil that was used without further purification.

N-Cbz-D-4-fluorophenylalaninal (9.0 g , 0.031 mol) was converted, using procedure 1, to (2R,3S,4S,5R)-2.5-bis(N-Cbz-amino)-3,4-dihydroxy-1,6-di(4-fluorophenyl)hexane (4 g) obtained as a white solid. MS: (CI, NH₃) (M+H)⁺ = 605.

The (2R,3S,4S,5R)-2.5-bis(N-Cbz-amino)-3,4-dihydroxy-1,6-di(4-fluorophenyl)hexane (4.0 g, 0.0066 mol) was converted, as described in procedure 4, to 1.3 g of the 4-fluorobenzyl cyclic urea (XXXI) obtained as a white solid. MS: (CI, NH₃) (M+H)⁺ = 539.3

B. The 4-fluorobenzyl cyclic urea (XXXI) (270 mg, 0.5 mmol) was alkylated with 3-benzoxybenzyl chloride (350 mg, 1.5 mmol) according to general procedure 5. The resulting intermediate was dissolved in THF and hydrogenated for 12 hours (200 mg 10% Pd/C, 55 psi) to remove the benzyl protecting groups. The MEM group was then removed, according to general procedure 5, to give, after chromatography on HPLC (silica gel, 10% MeOH/CHCl₃), 140 mg of Example 20G as a white foam. MS: (CI, NH₃) (M+H)⁺ = 575.2 (100%).

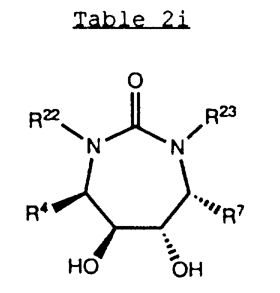

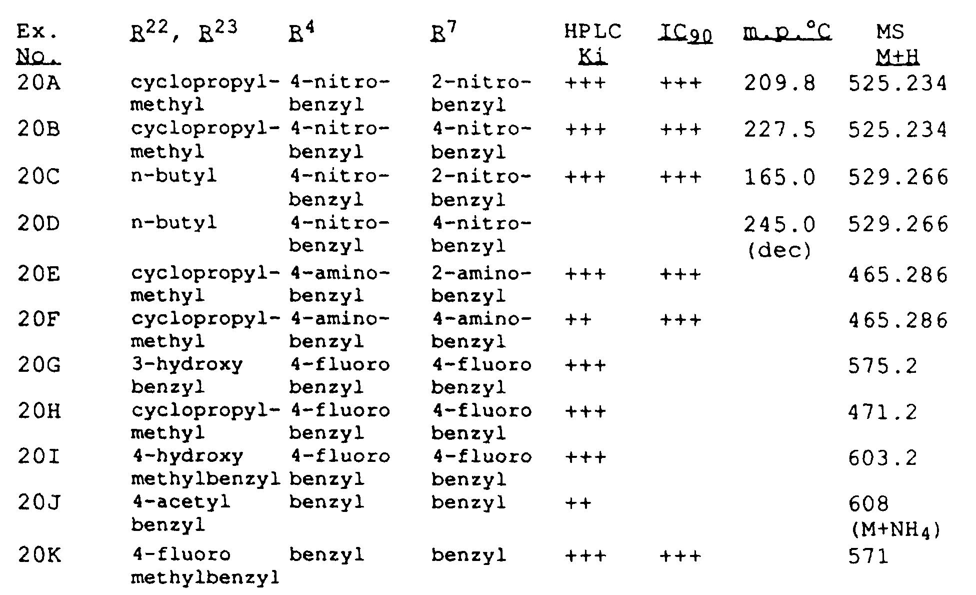

The structures of the Examples below are shown in Table 2j.

### Example 21A

### A. Synthesis of Aziridine Urea (XXXIIa) :

A solution of Example 1A (5.3 g, 0.016 mol) in pyridine was treated with acetic anhydride (3.3 g, 0.033 mol) and strirred at room temperature for 3 hrs. 10 mL of MeOH was added and the mixture was evaporated to dryness. The residue was extracted into methylene chloride and washed sequentially with water, 1 N HCl, brine, and dried over MgSO₄. The solution was filtered, concentrated and the residue chromatographed on silica gel (5%MeOH/CHCl₃) to give 2.0 g of the corresponding monoacetate product as a white solid. The solid obtained was dissolved in methylene chloride and cooled in an ice bath under nitrogen. To this was added DAST (0.875 g, 0.005 mol) via syringe and the solution stirred for 10 mins. The mixture was quenched with sat'd NaHCO₃ and the organic layer washed with water and brine. The solution is dried over MgSO₄ then filtered and concentrated to give 1.9 g of the acetate aziridine (XXXIIa) which is used without further purification.

B. The acetate aziridine (XXXIIa) (100 mg. 0.29 mmol) is dissolved in MeOH (2 mL) and treated with 1 N NaOH (0.5 ml) and stirred at rt for 30 min. The mixture is diluted with water (20 mL) and extracted into CH₂Cl₂. The extract is washed with water and brine, dried over MgSO₄ then filtered and concentrated to give 30 mg of Example 21A as a white solid. MS: (CI, NH₃) (M+H)⁺ = 309.0

### Example 21B and 21C

The acetate aziridine (XXXIIa) (200 mg, 0.57 mmol) is alkylated with benzyl bromide (120 mg, 0.69 mmol) according to Procedure 5 to give a mixture of products. This was HPLC chromatographed on silica gel (50% EtOAc/Hex) to give first 50 mg of Example 21B as a white solid. MS: (CI, NH₃) (M+H)⁺ = 309.0. This was followed by 30 mg of Example 21C, obtained as a colorless oil. MS: (CI, NH₃) (M+H)⁺ = 489.2.

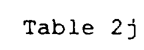

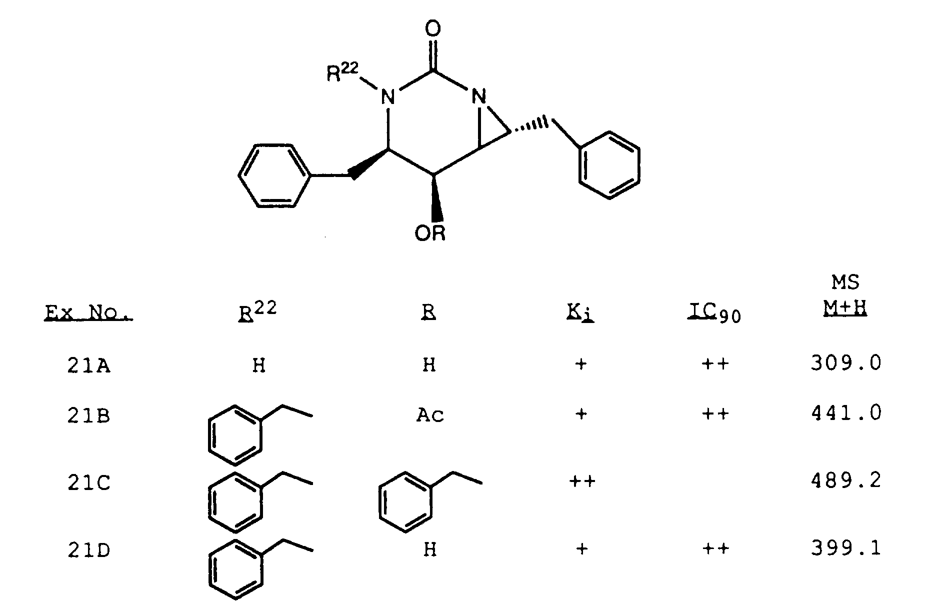

The structures of the Examples below are shown in Table 2k.

### Preparation of the Cyclic Urea (XXXIIIa):

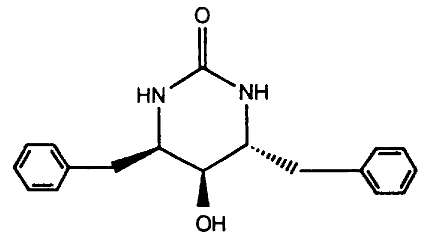

### A. Preparation of 4-Amino-2-(t-butoxycarbonylamino)-1,5-diphenyl-3-(2-methoxyethoxymethyl)pentane.

A mixture of 595 mg (1.50 mmole) of 4-azido-2-(t-butoxycarbonylamino-1,5-diphenyl-3-hydroxypentane (EP 0 402 646 Al), 10 ml of dioxane, 0.2 ml (1.75 mmole) of MEM chloride, and 0.32 ml (1.83 mmole) of diisopropylethylamine was heated at 80°C for 16 hrs. Evaporated the solvent and purified the residue by flash chromatography on silica gel with 85:15 hexane-ethyl acetate to give 0.64 g (88%) of an oil. Mass spec (M+H)⁺ = 485.2. This was reduced to the title compound with hydrogen using 100 mg of 10 % Pd on carbon in 60 ml of ethyl acetate and 0.6 ml of acetic acid in 49% yield.

### B. Preparation of 2,4-diamino-1,5-diphenyl-3-hydroxypentane.

The product from Part A (218 mg) was dissolved in 2 ml of ice cold 1:1 trifluoroacetic acid-dichloromethane. After 1 hr the solution was poured into a mixture of sodium bicarbonate solution and ethyl acetate. The ethyl acetate extract yielded 163 mg of the desired diamino compound.

### C. Cyclization of the Diamine

The product from Part B (146 mg), 75 mg of carbonyl diimidazole, and 0.15 ml of diisopropylethylamine were dissolved in 2.5 ml of anydrous THF and stirred at room temperature for 16 hrs. The solvent was evaporated. The residue was purified by preparative TLC on silica gel with 90:10 dichloromethane - methanol to give 108 mg (69 %) of the cyclic urea. Mass spec (M+H)⁺ = 385.1.

### N-Alkylation of the Cyclic Urea (XXXIIIa):

D. The product from Part C (93 mg) was dissolved in 2.5 ml of anhydrous DMF, and 100 mg of 60% NaH in mineral oil was added. The mixture was stirred for one hr. m-Benzyloxbenzyl chloride (350 mg) was added, and the mixture was stirred for 16 hrs at room temperature. Water and ethyl acetate were added. The ethyl acetate extract was washed with water, dried and evaporated . The residue was purified by prep TLC on silica gel with 60:40 hexane - ethyl acetate to give 105 mg (54%) of the desired bis-alkylated product. Mass spec (M+H)⁺ = 777.5

### Deprotection of Protecting Groups (Example 22A):

The product from part D (103 mg) was dissolved in 4N HCl/dioxane for 16 hrs. The solution was evaporated and purified by prep TLC on silica gel with 60:40 hexane - ethyl acetate. Mass spec (M+H)⁺ = 689.4. The purified material was hydrogenated for 16 hrs in the presence of 3 ml ethanol. 0.2 ml of acetic acid, and 35 mg of 10% Pd on carbon to give Example 22A. Mass spec (M+H)⁺ = 509.25; calculated, 509.24.

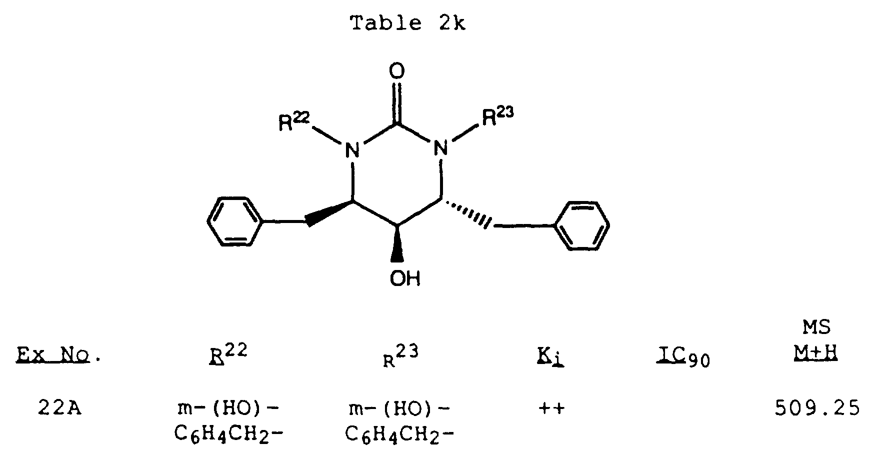

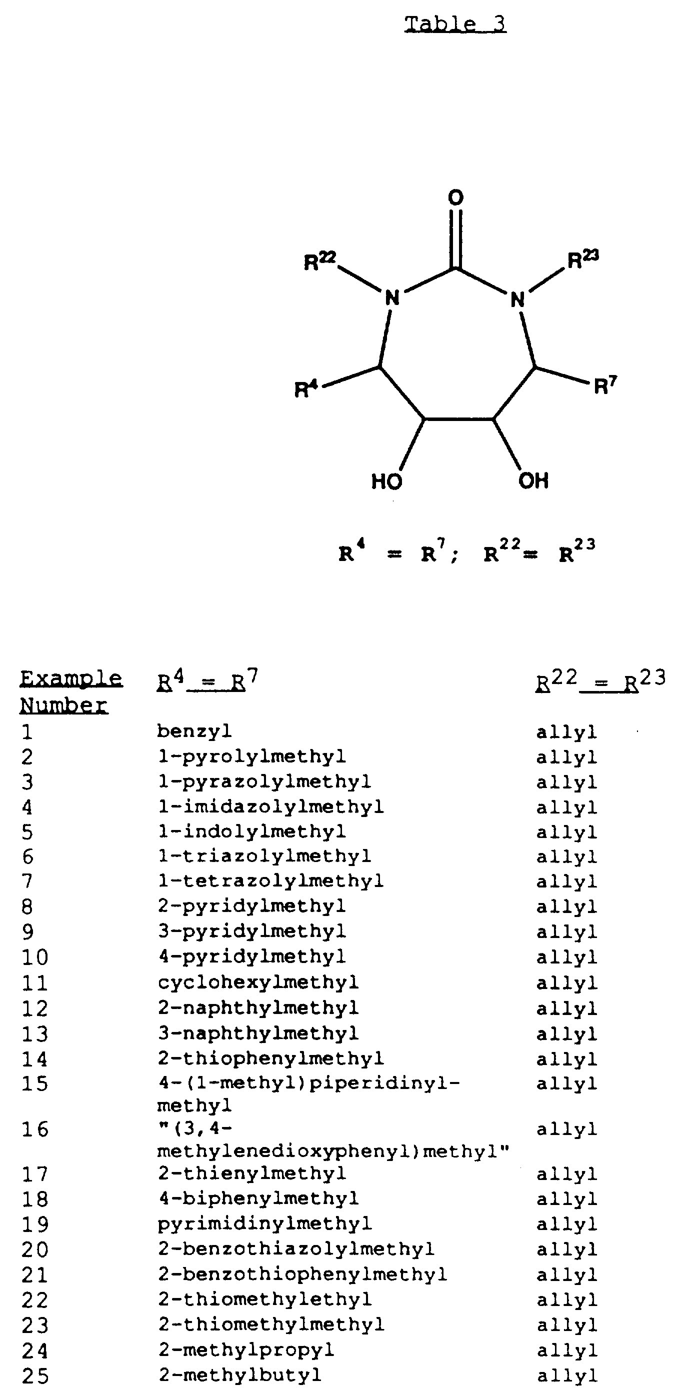

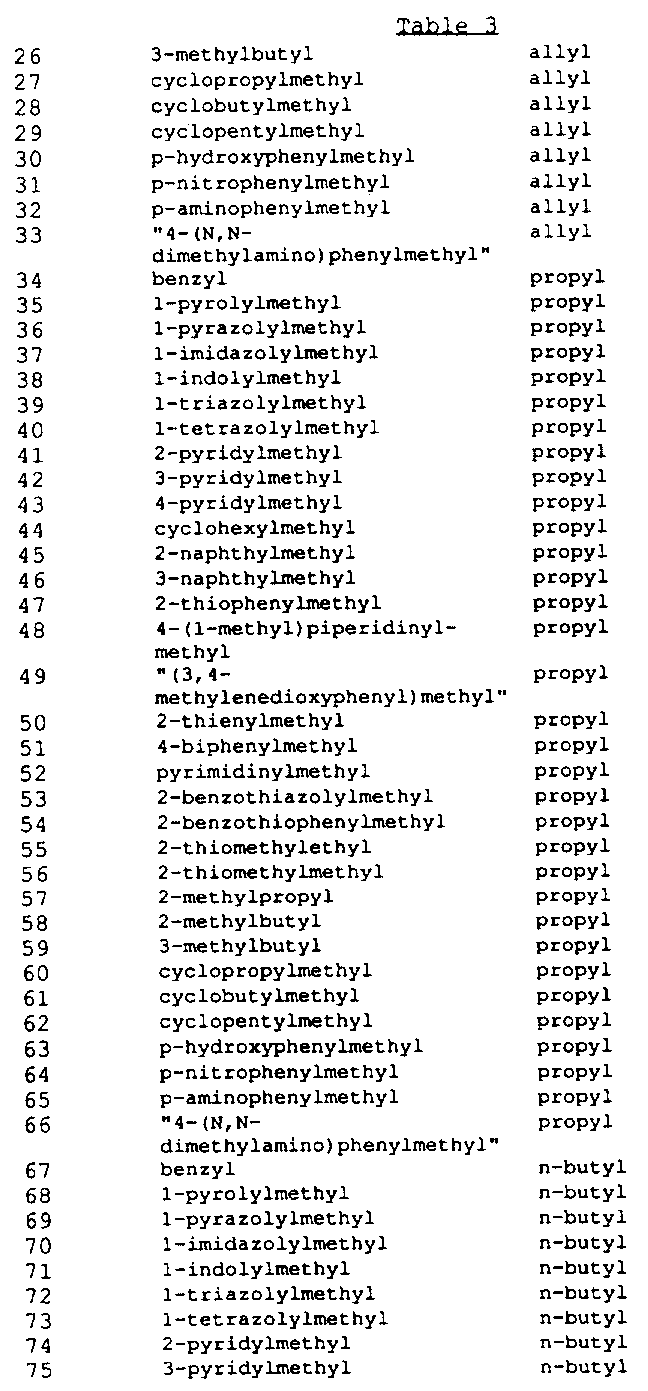

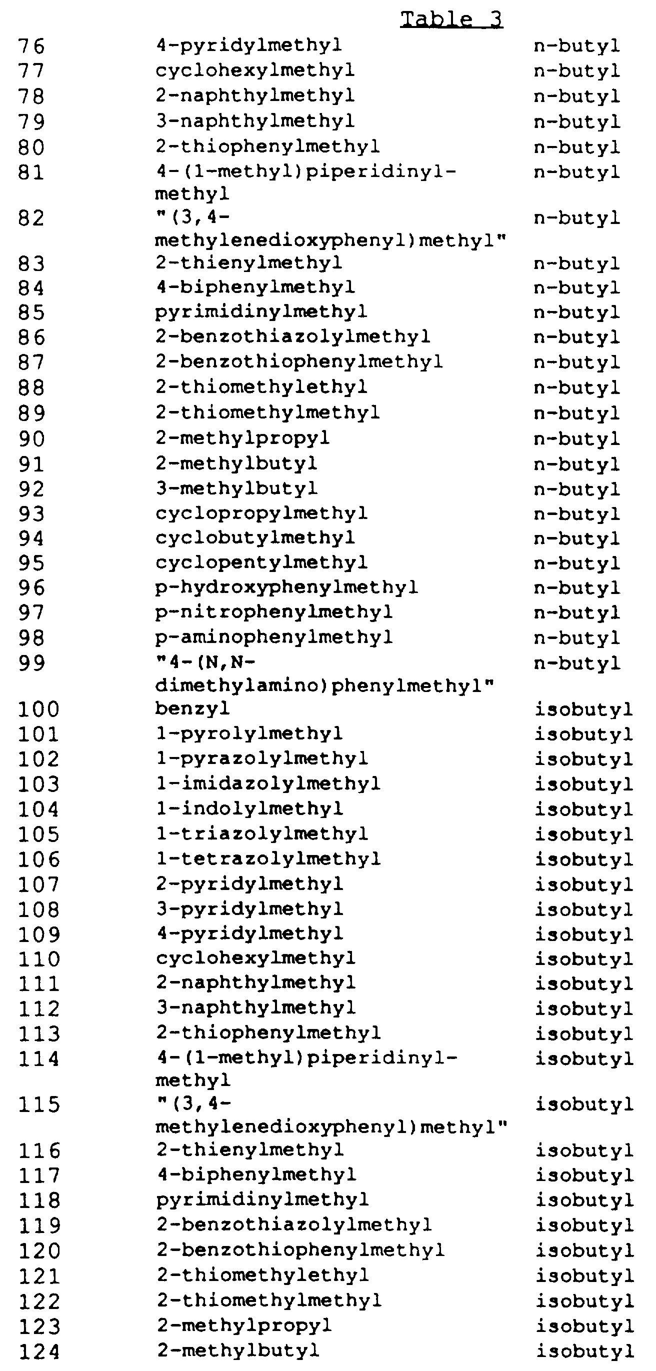

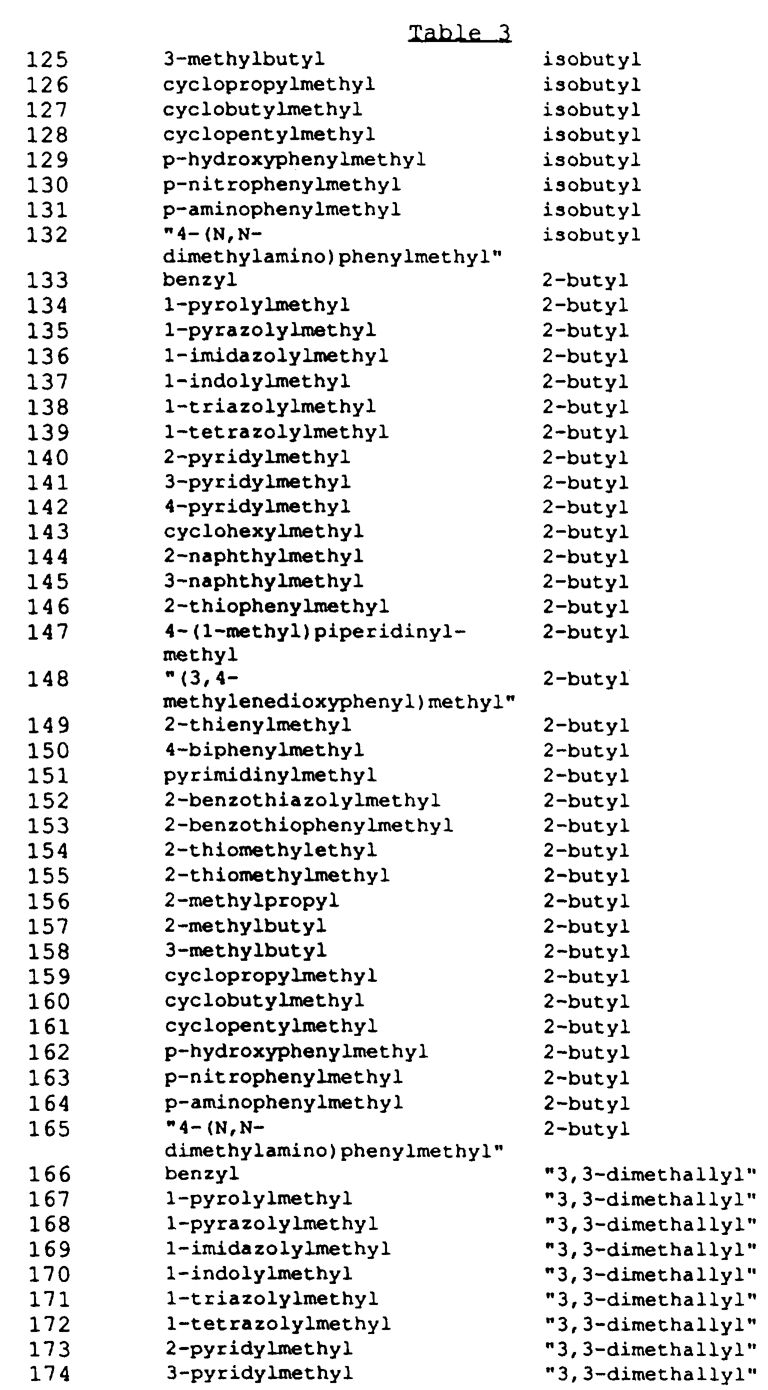

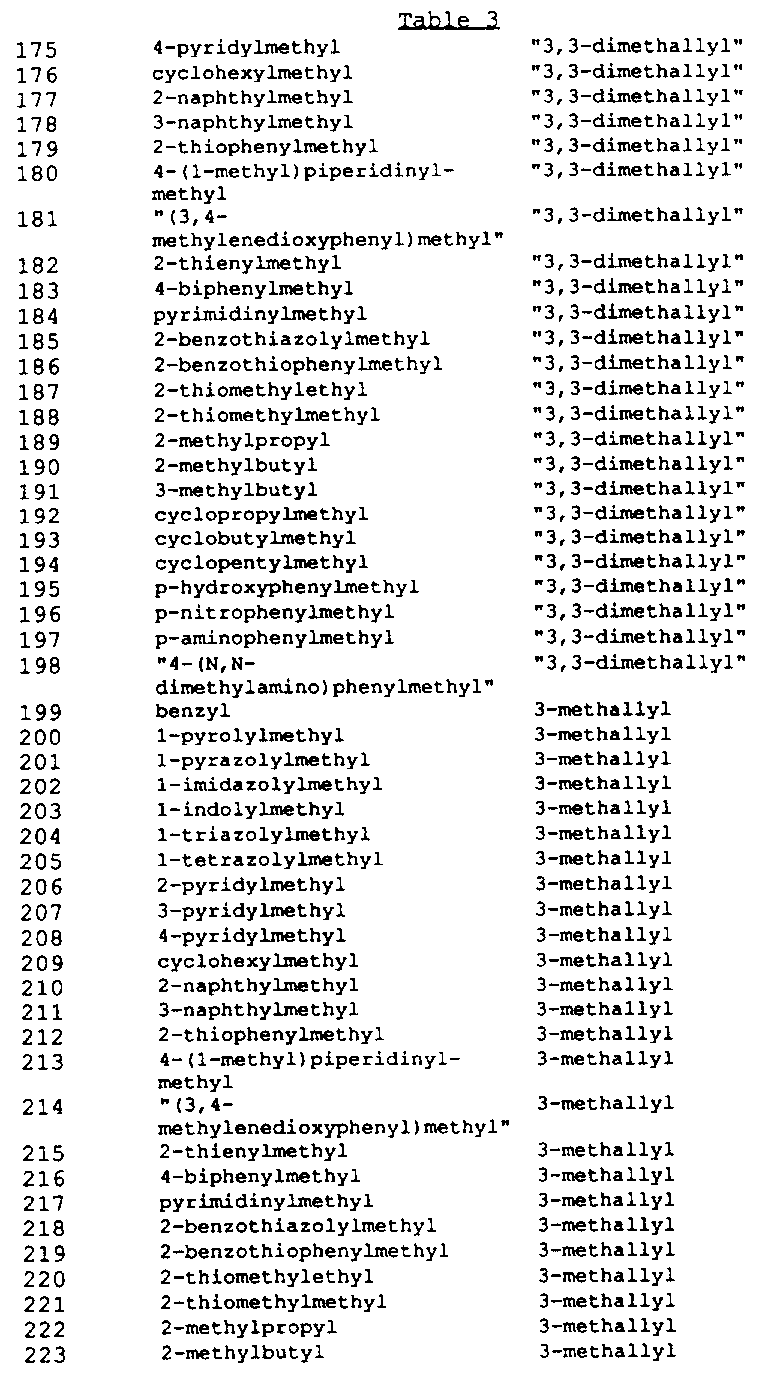

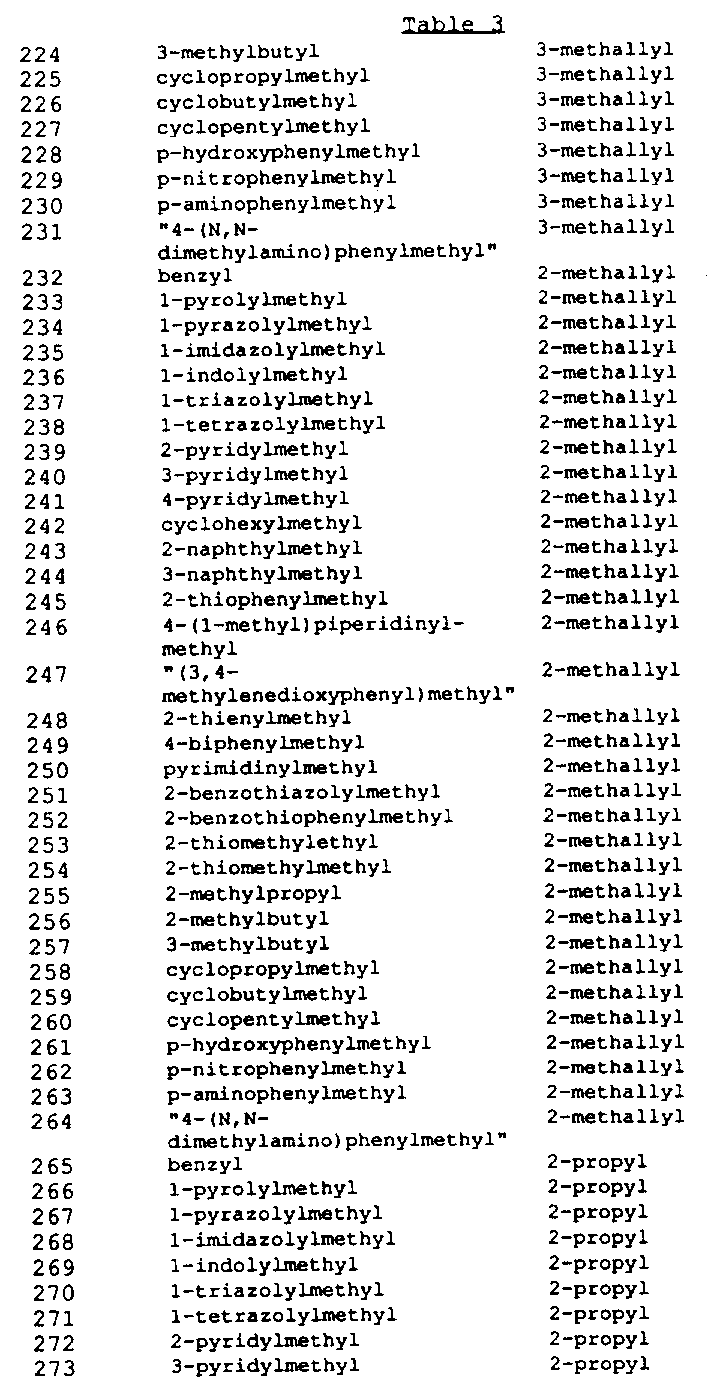

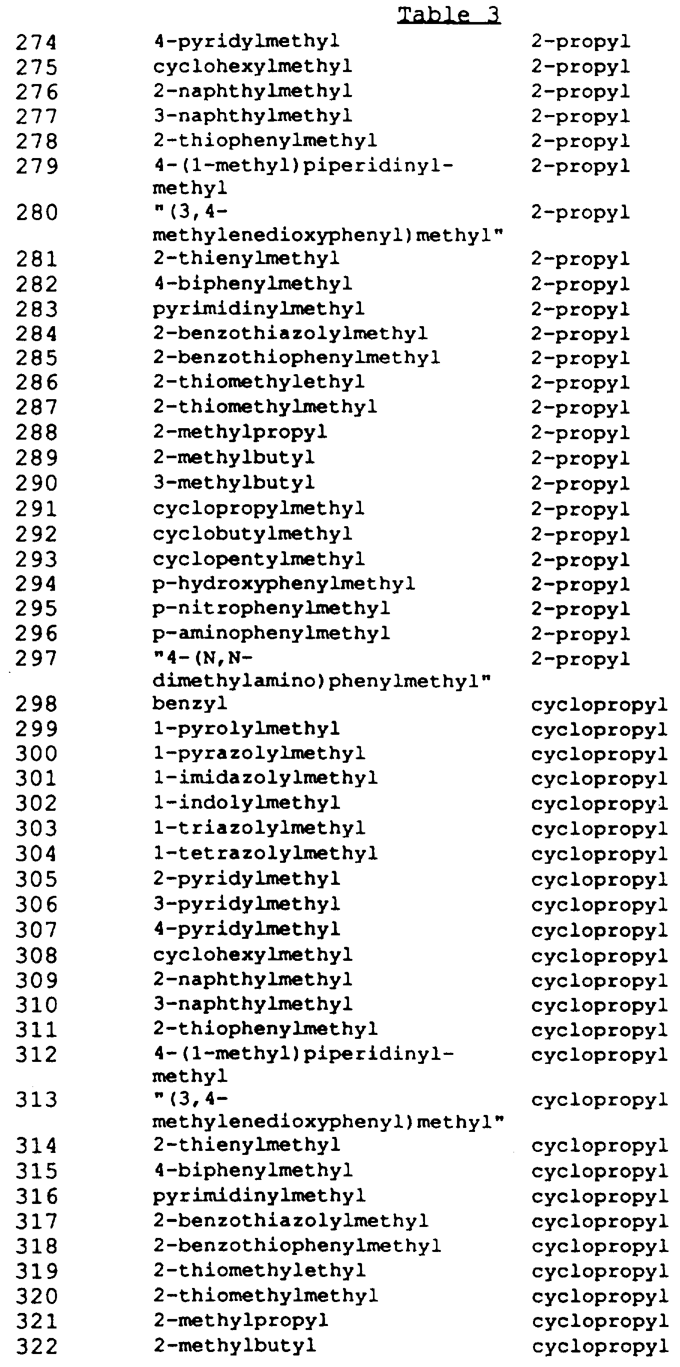

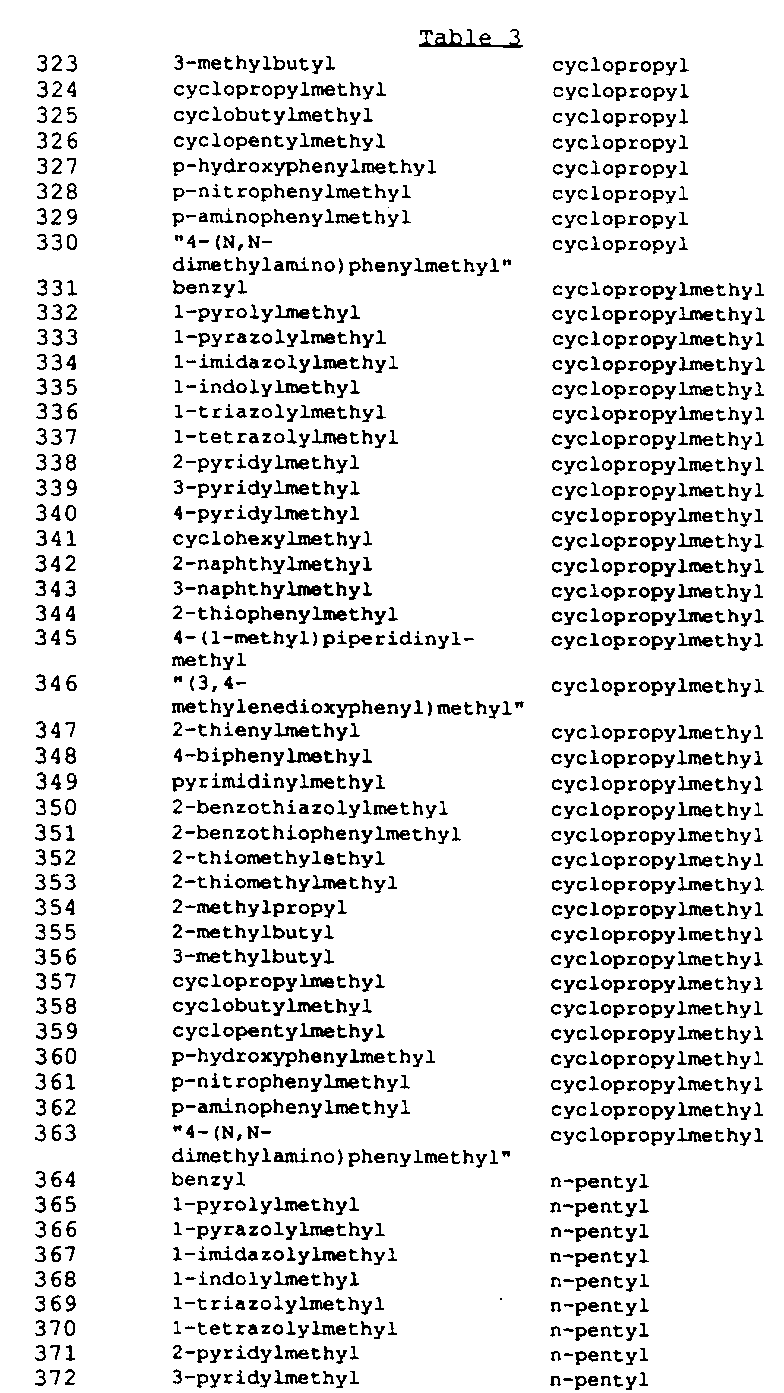

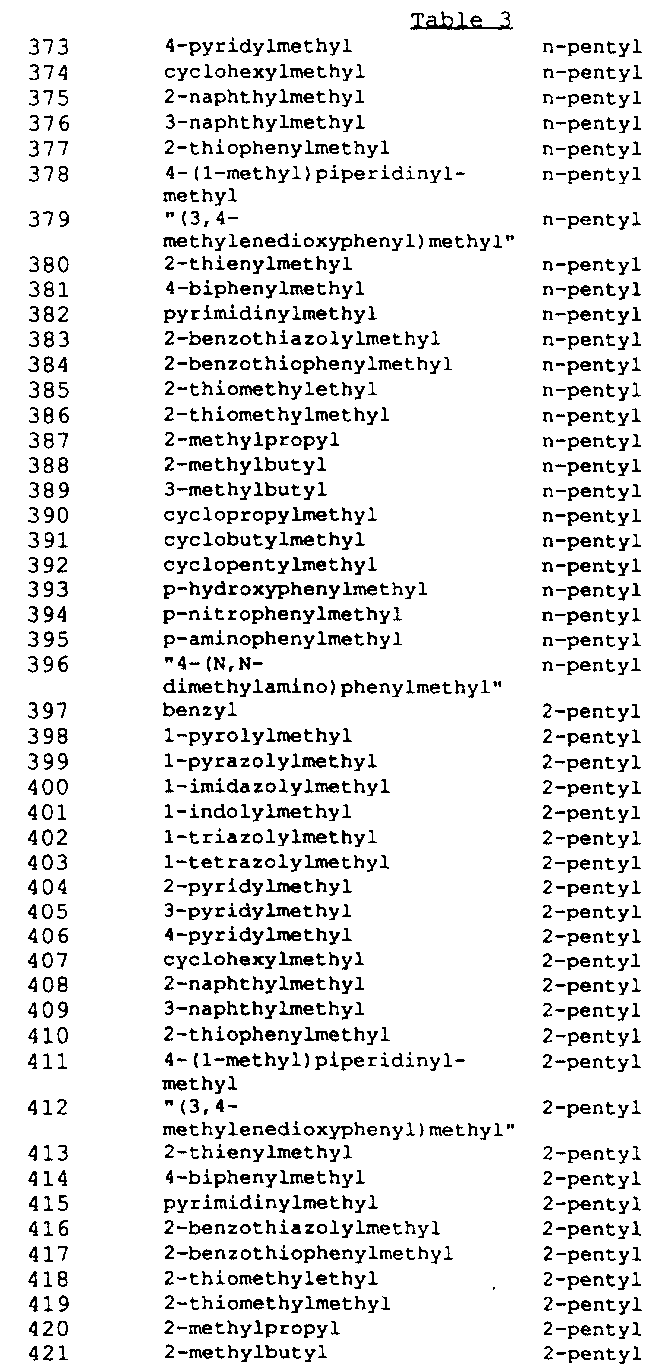

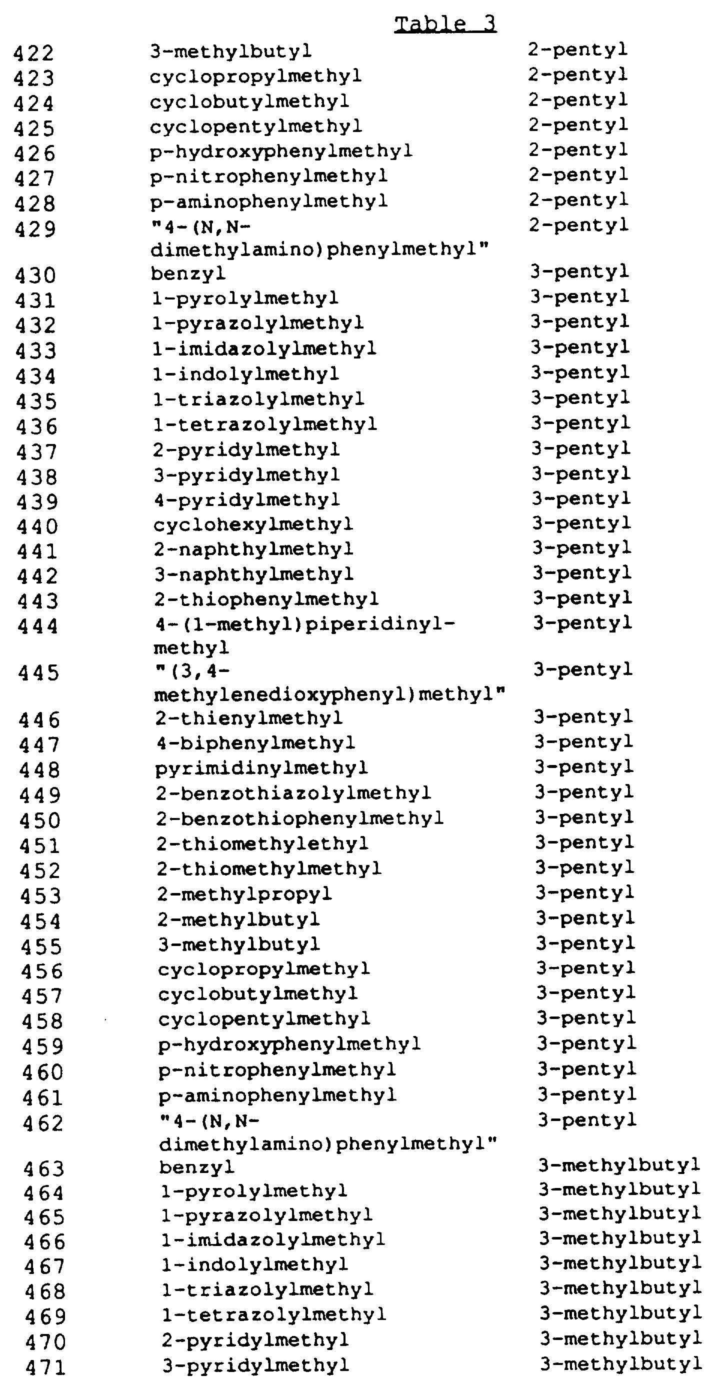

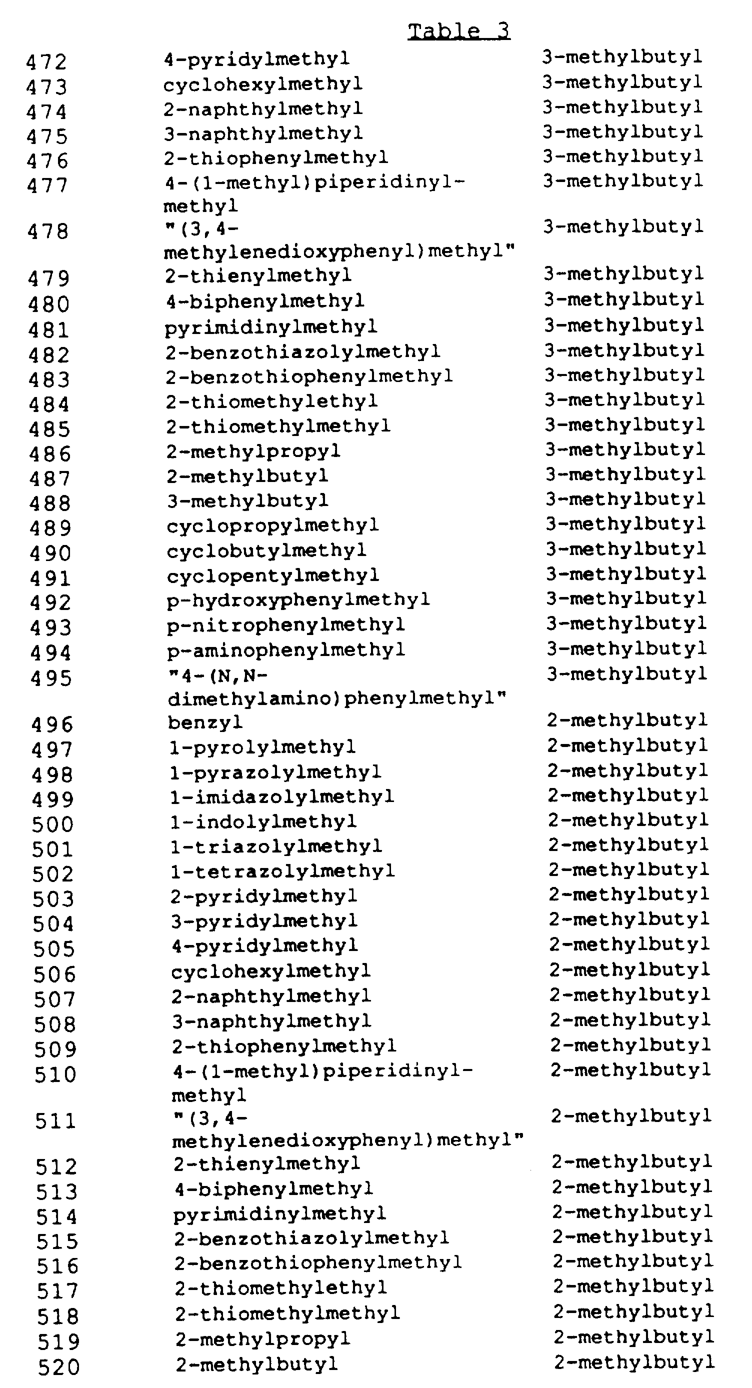

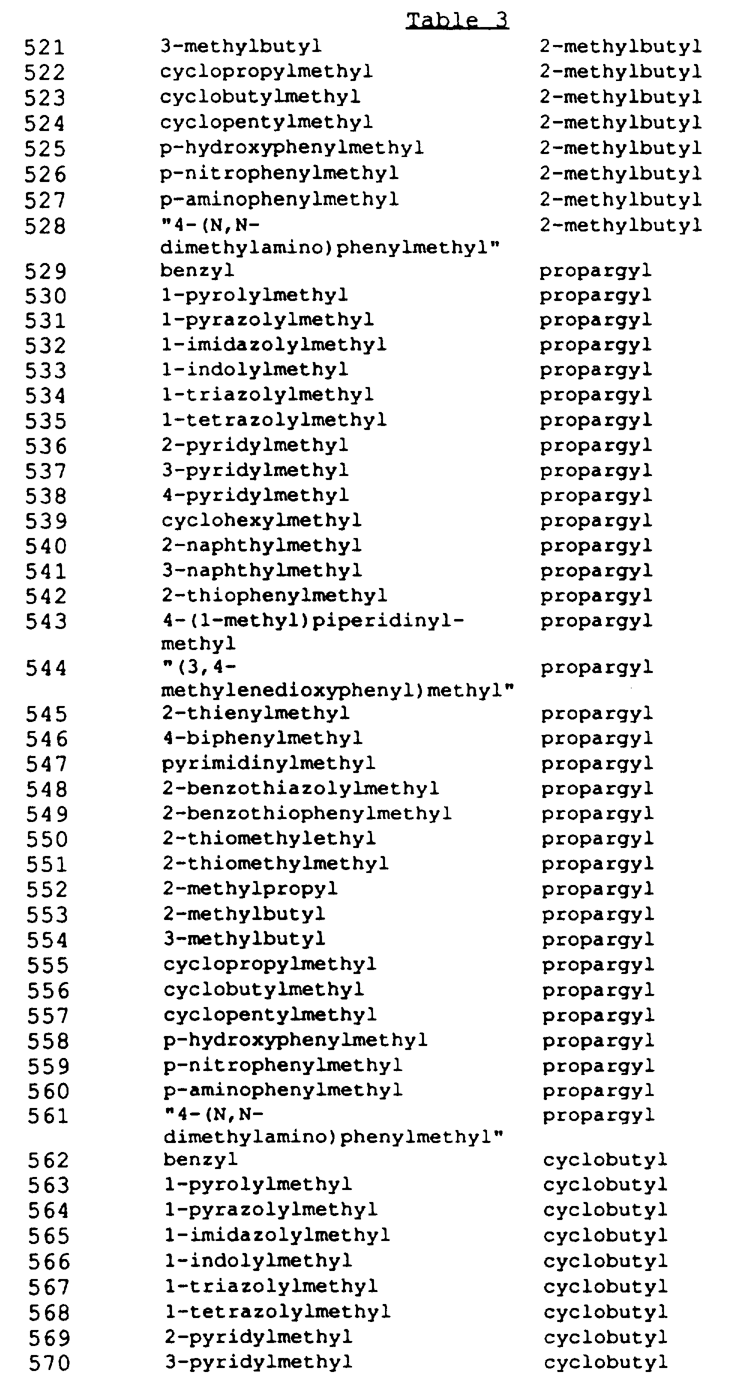

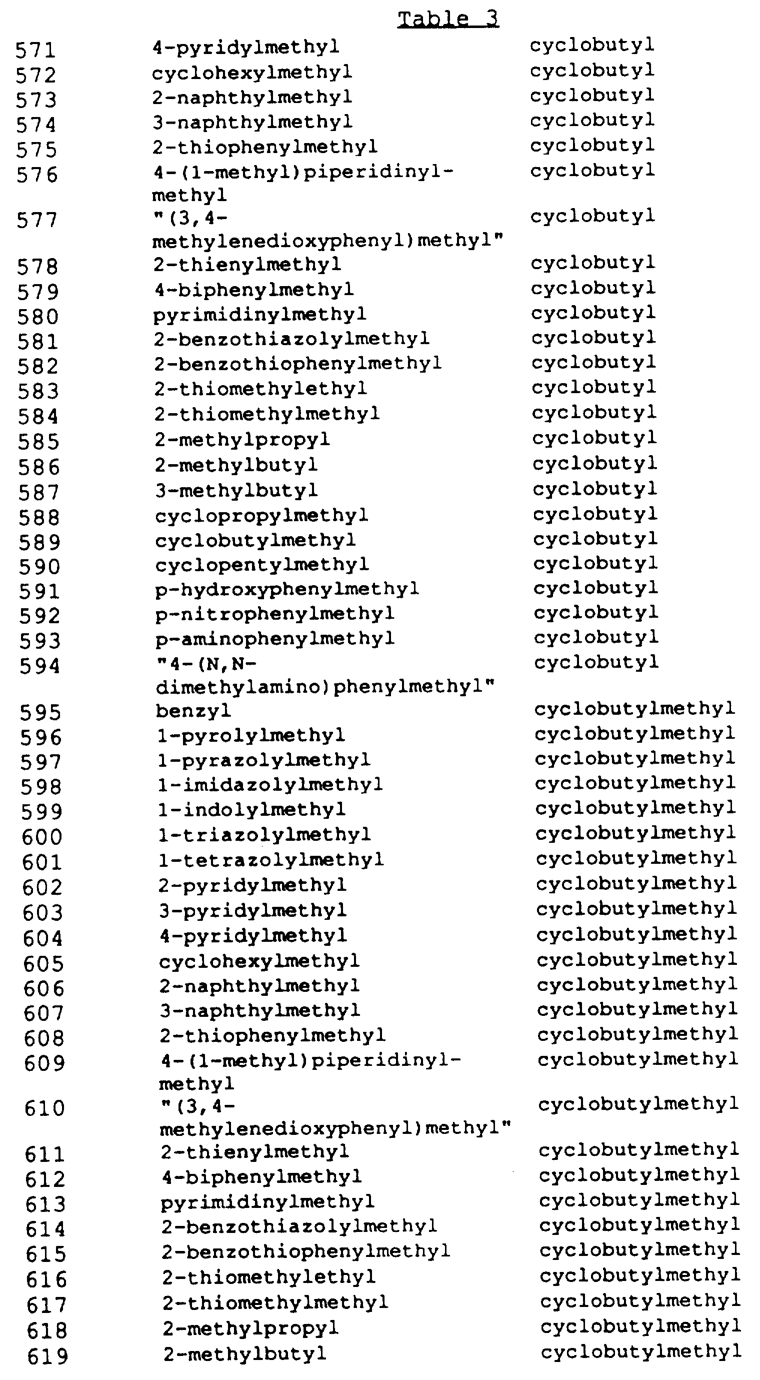

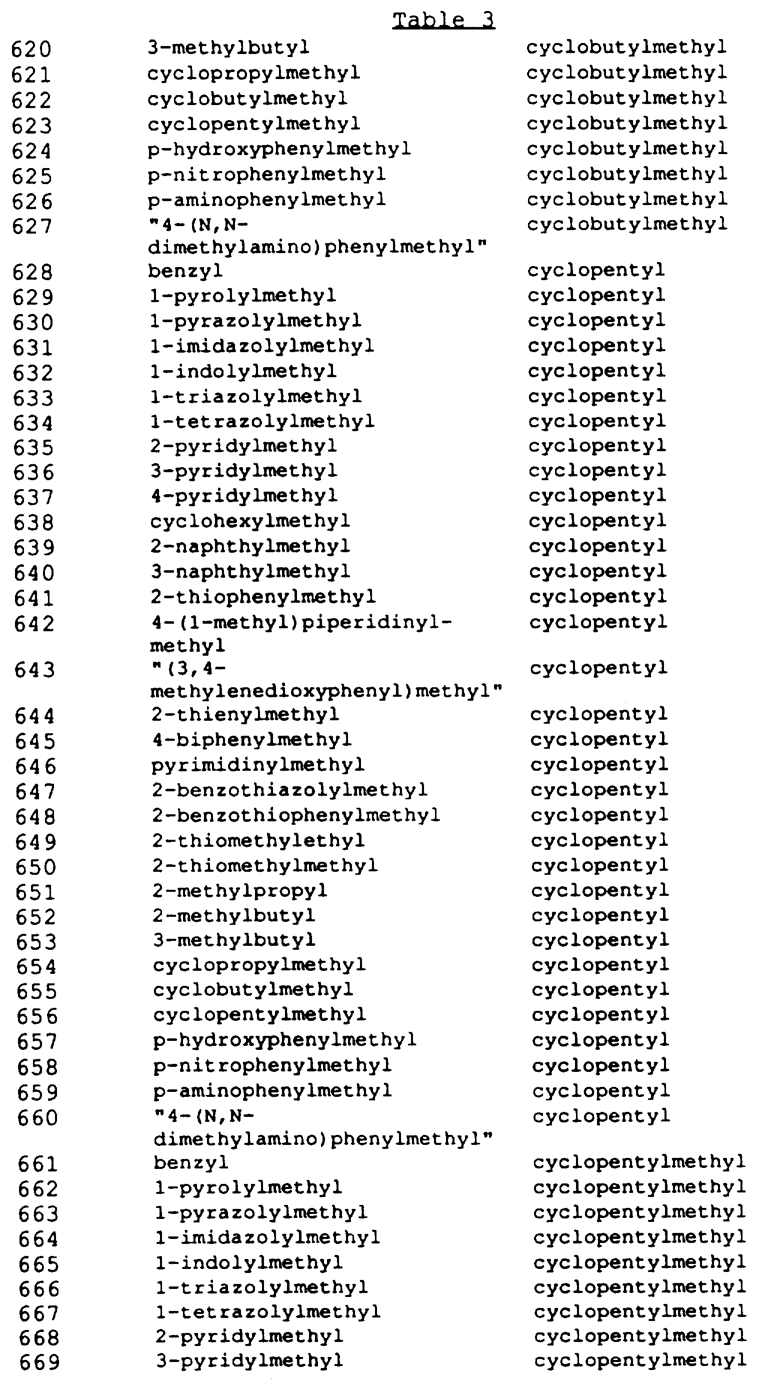

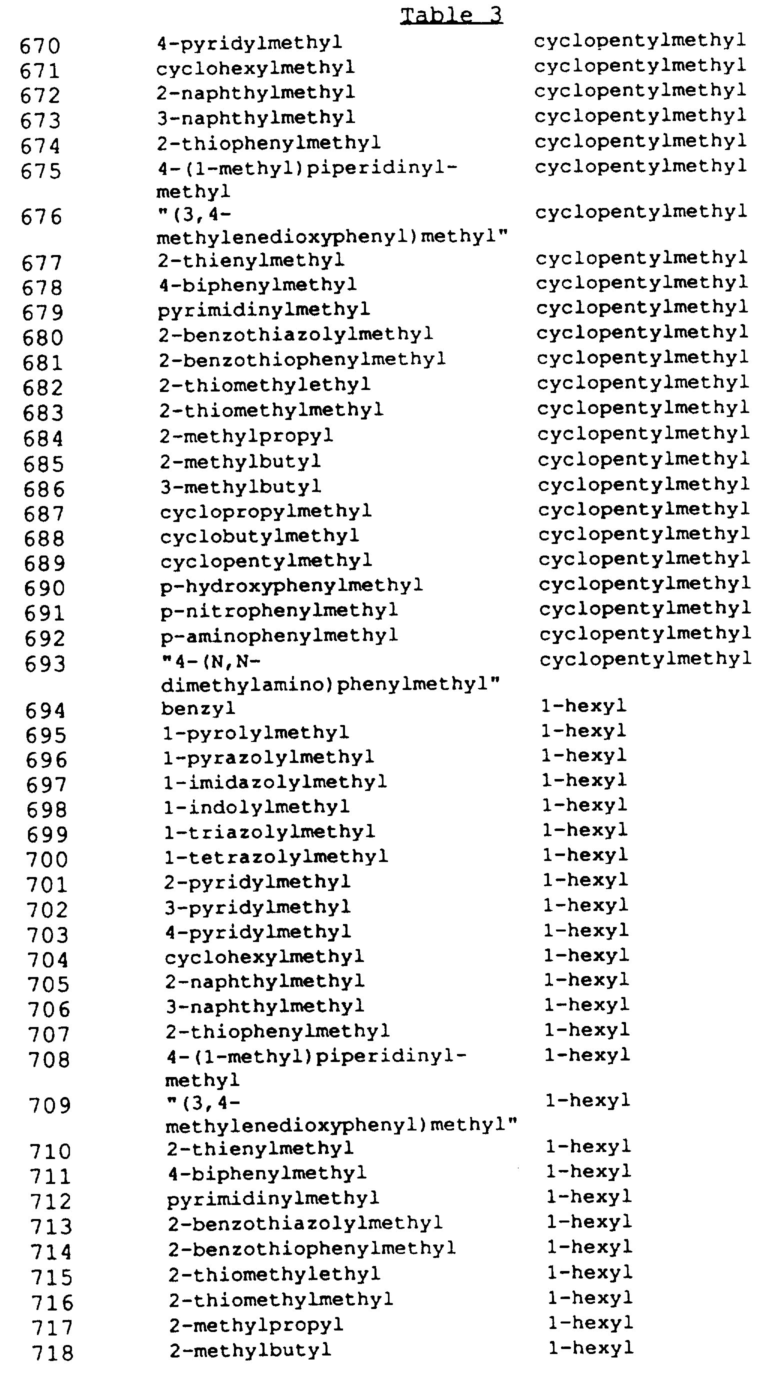

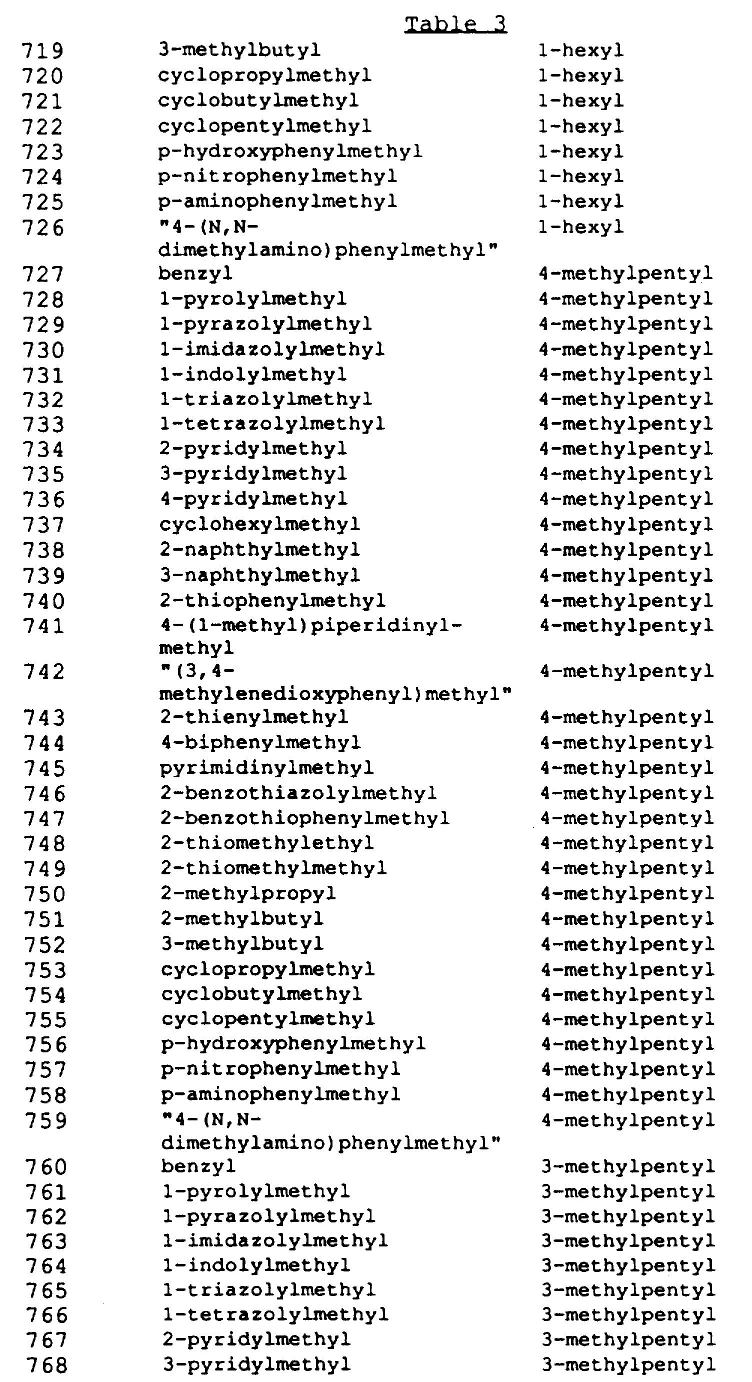

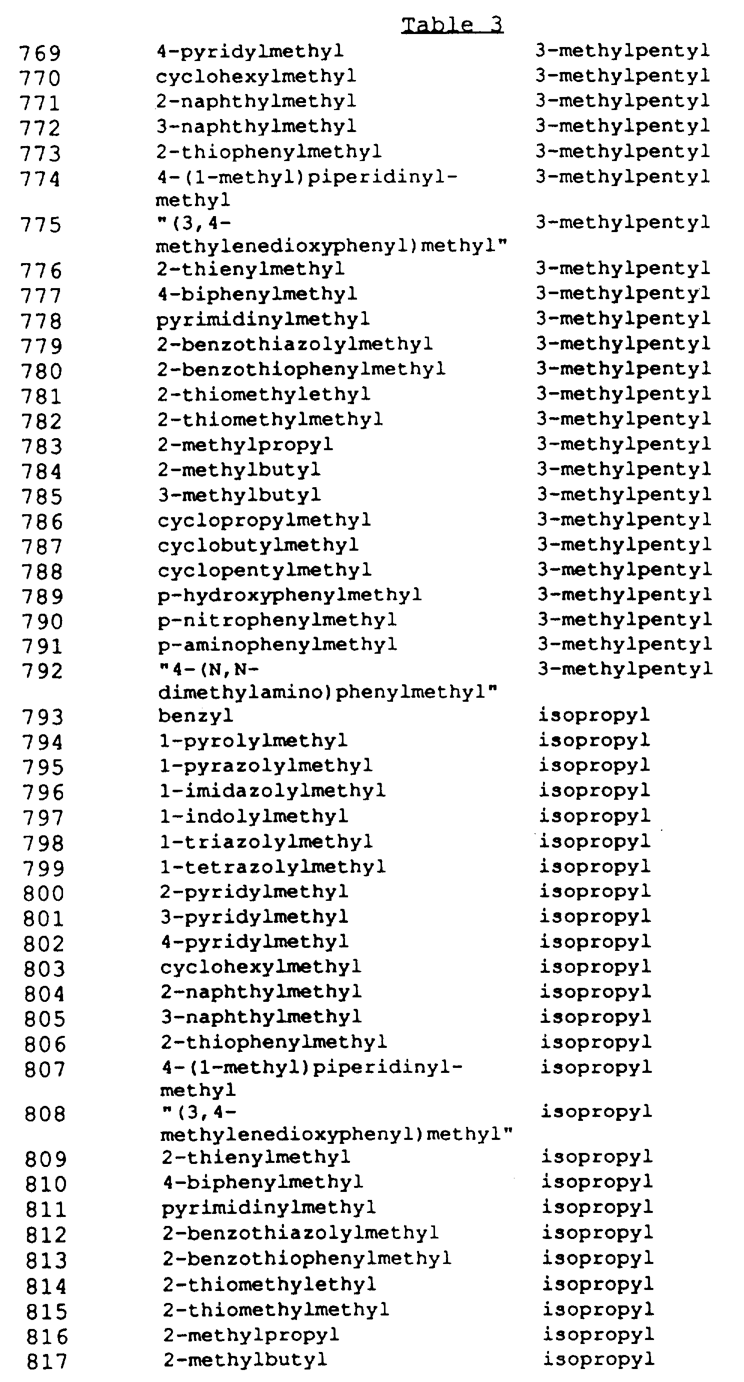

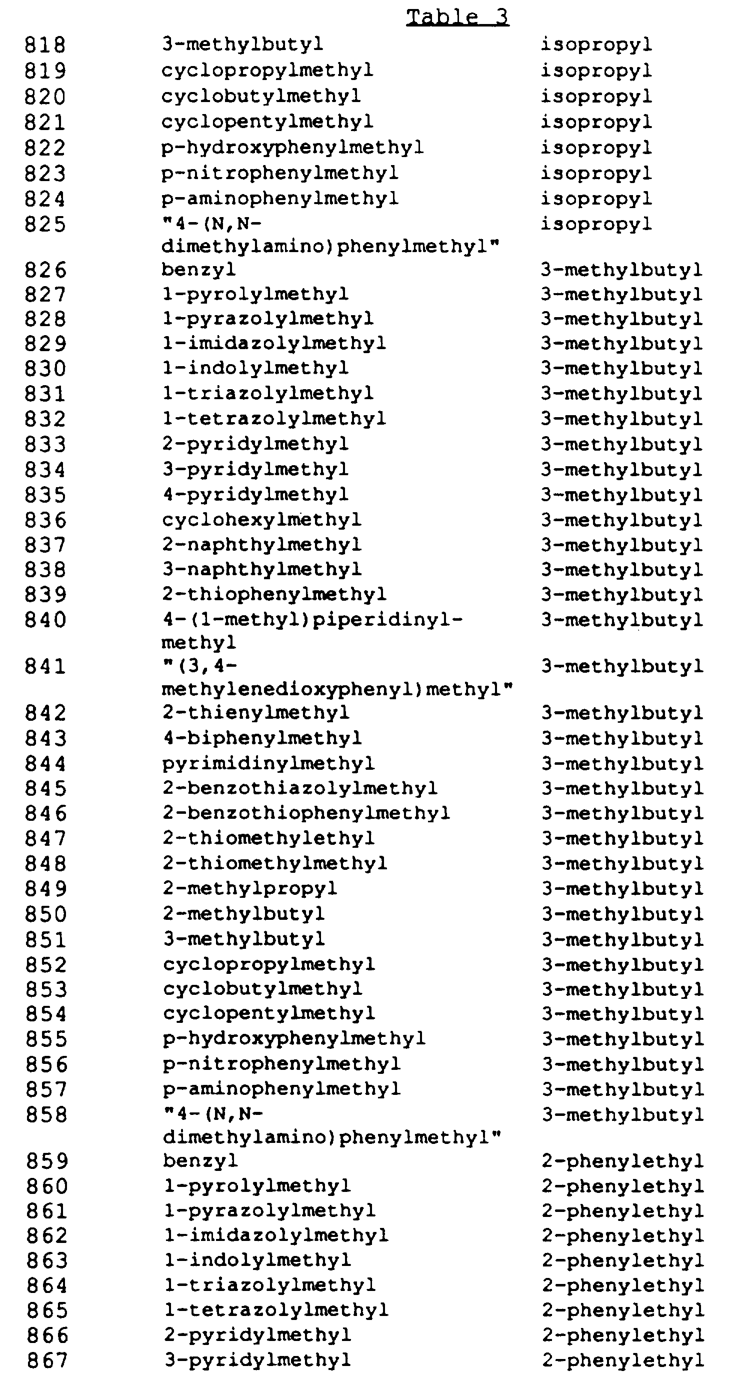

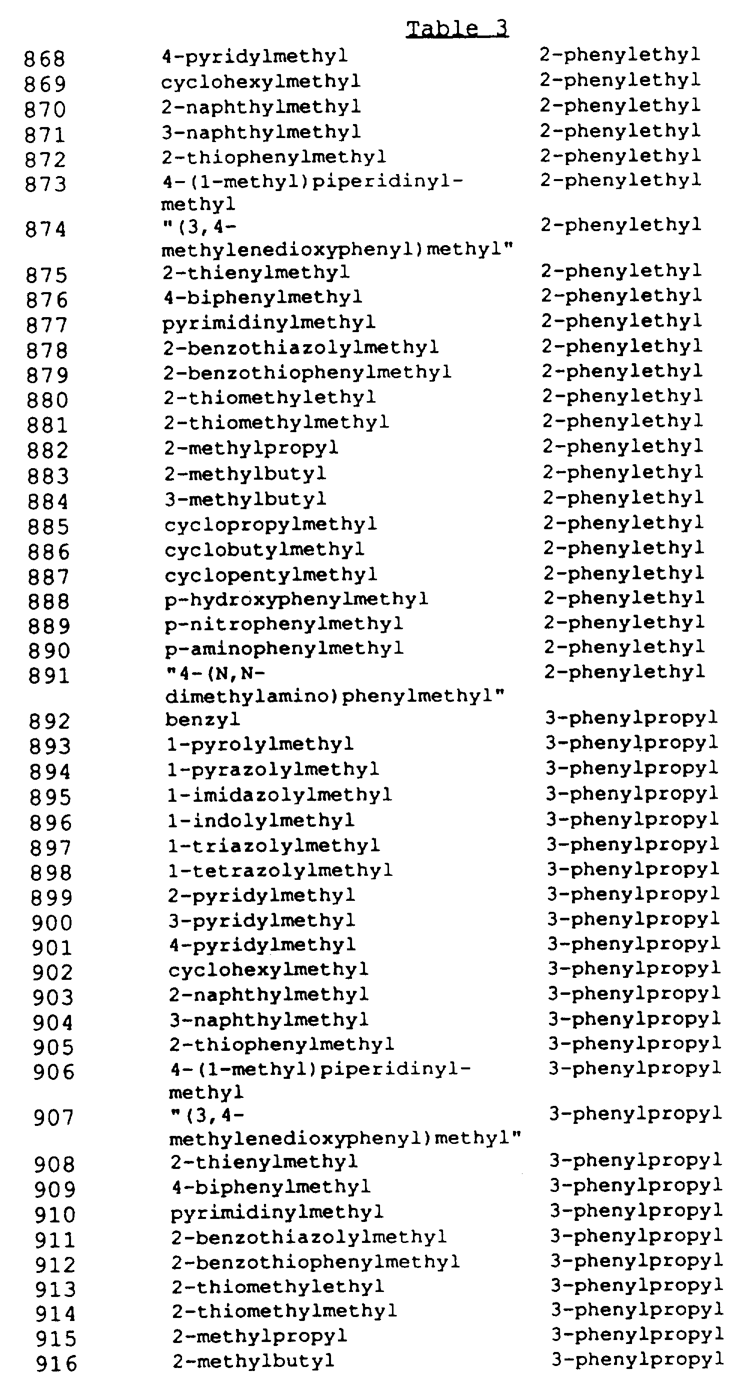

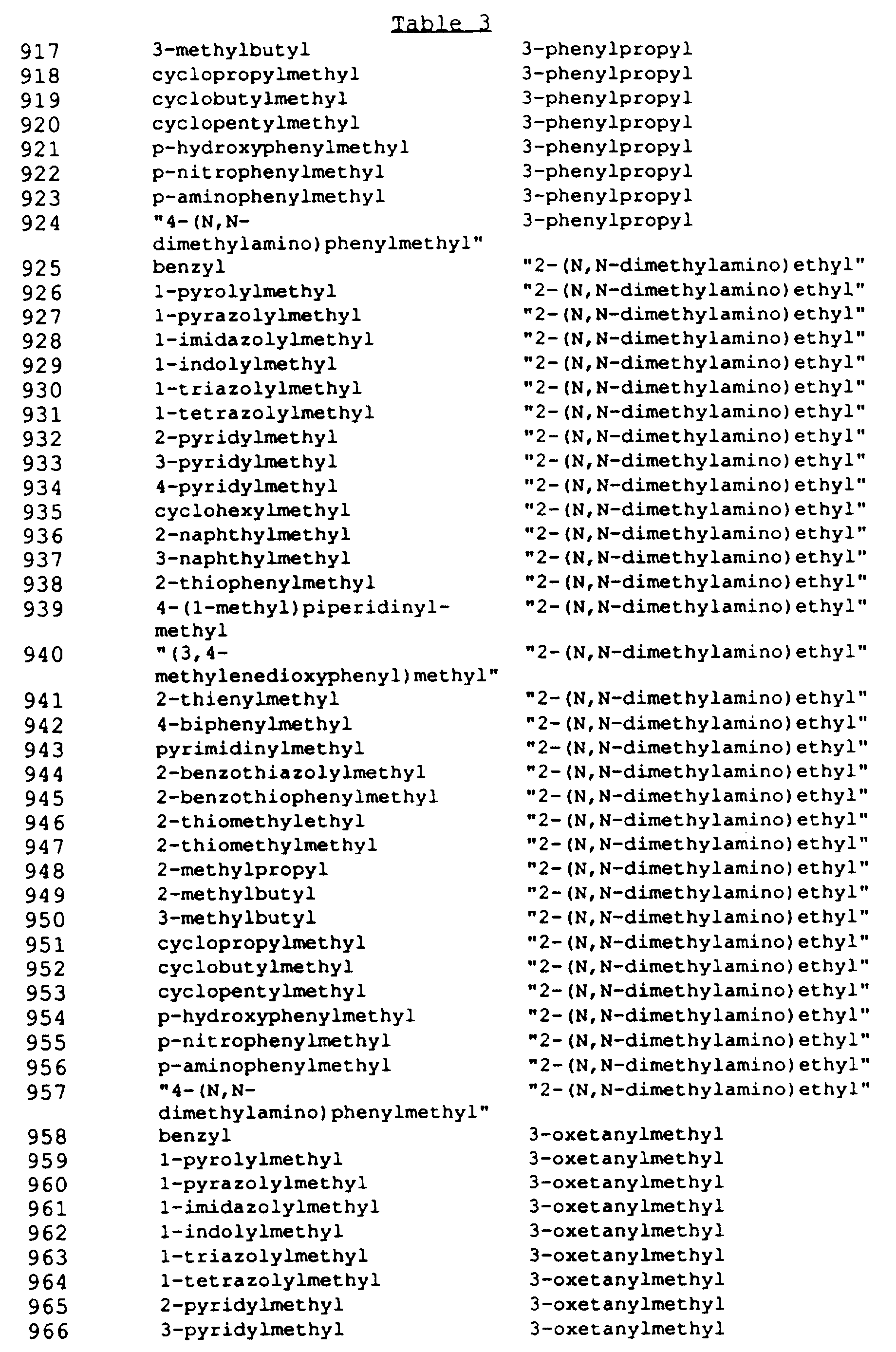

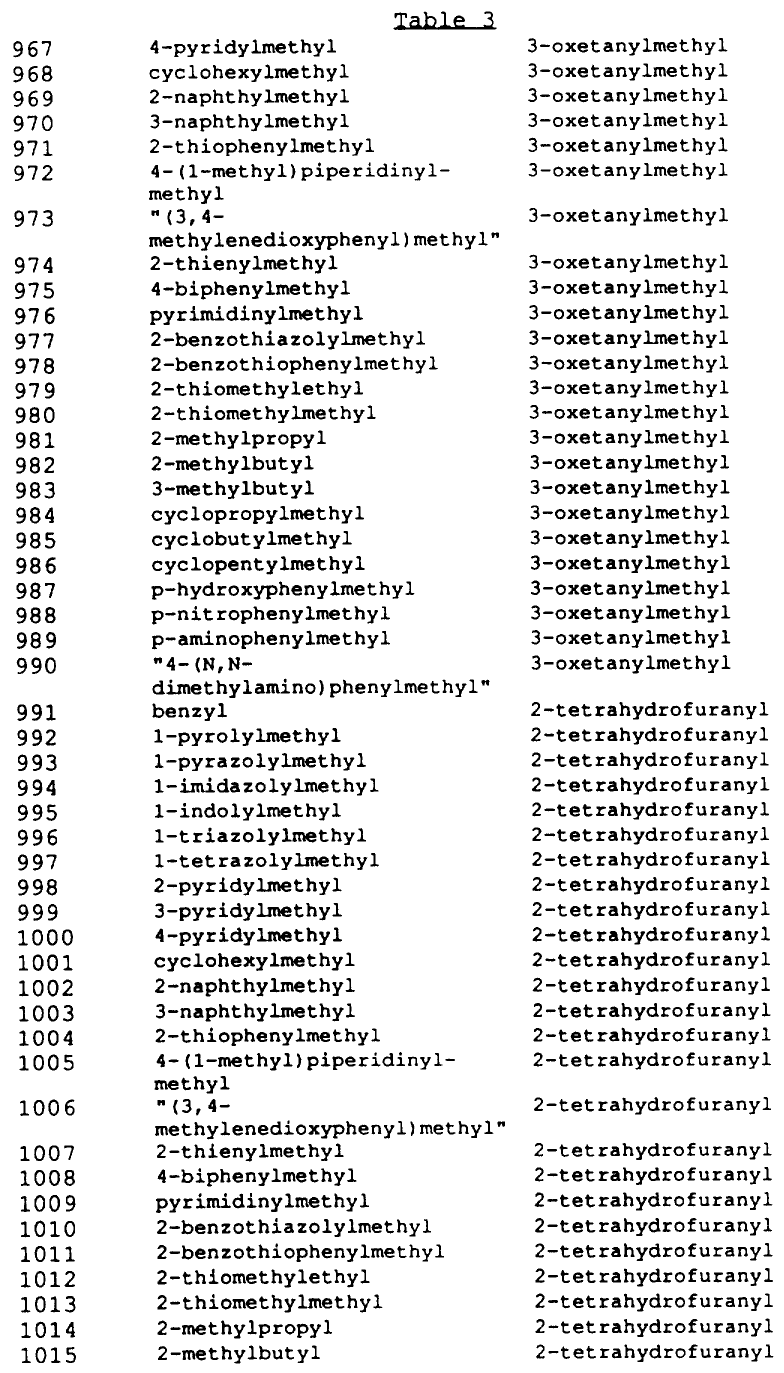

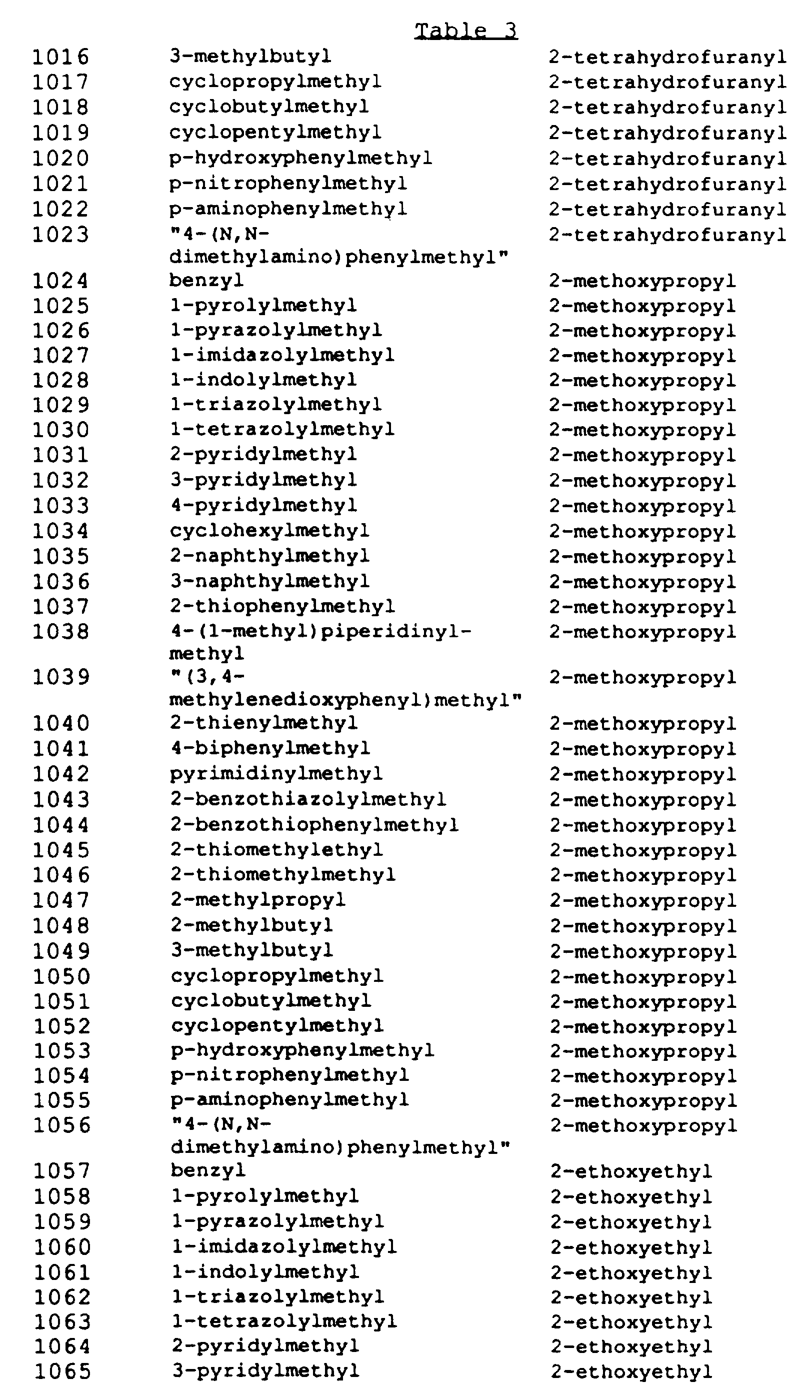

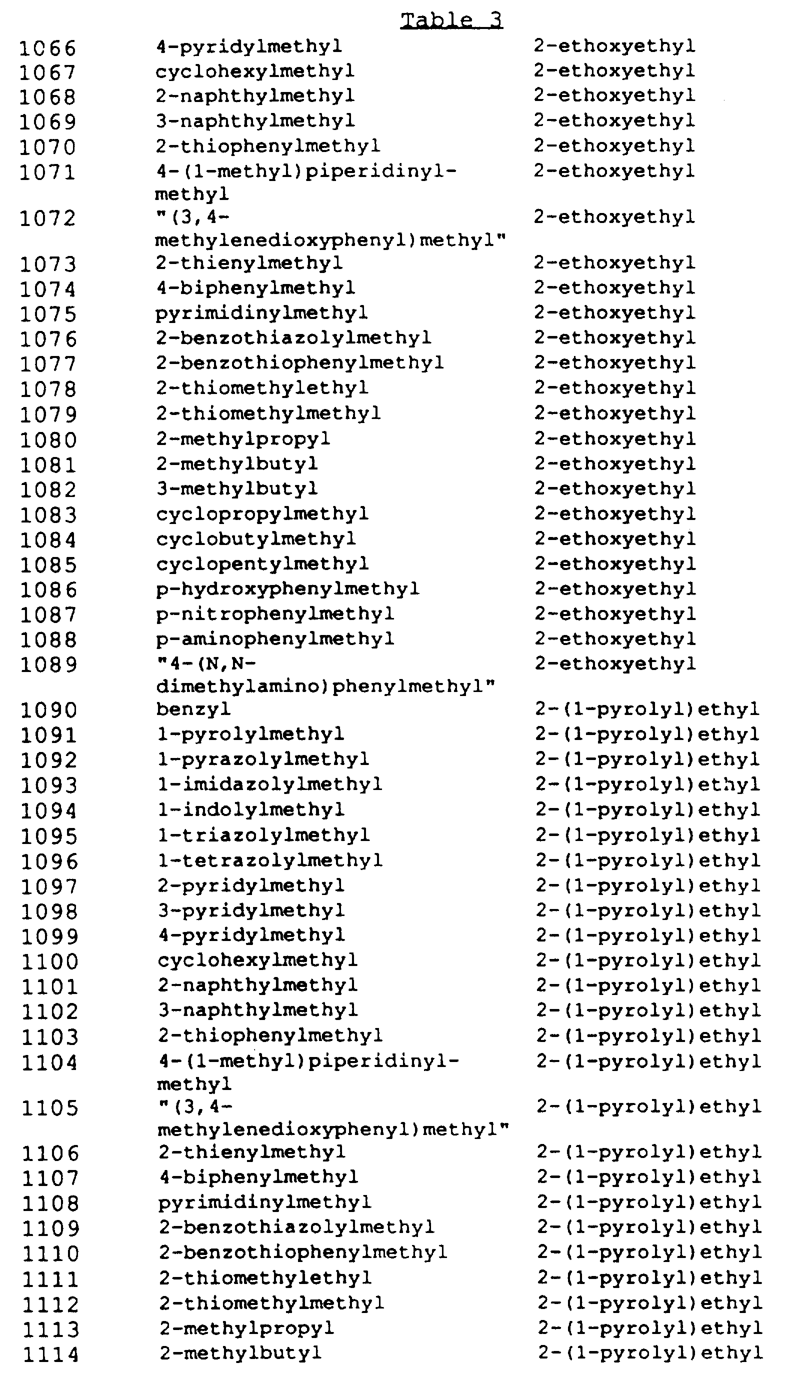

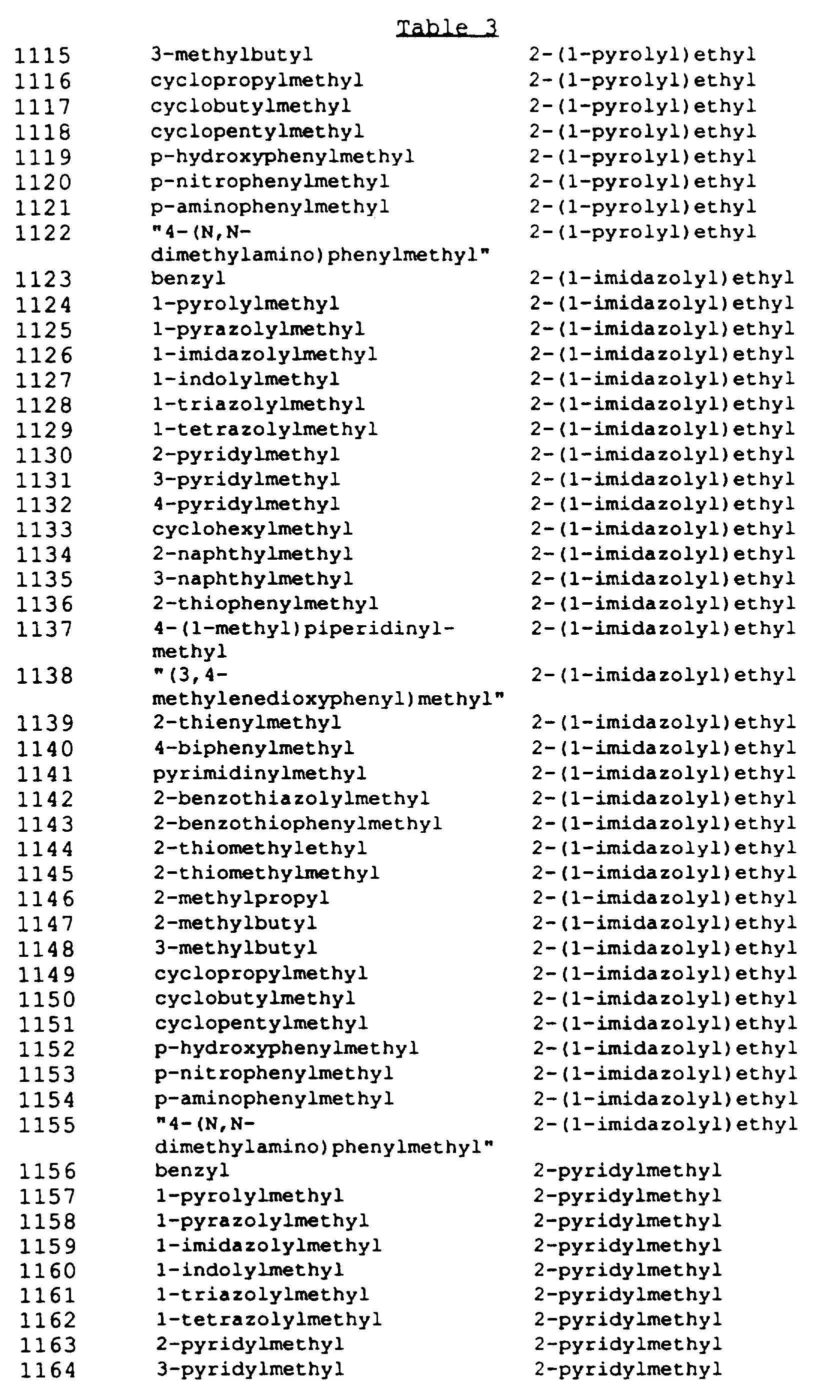

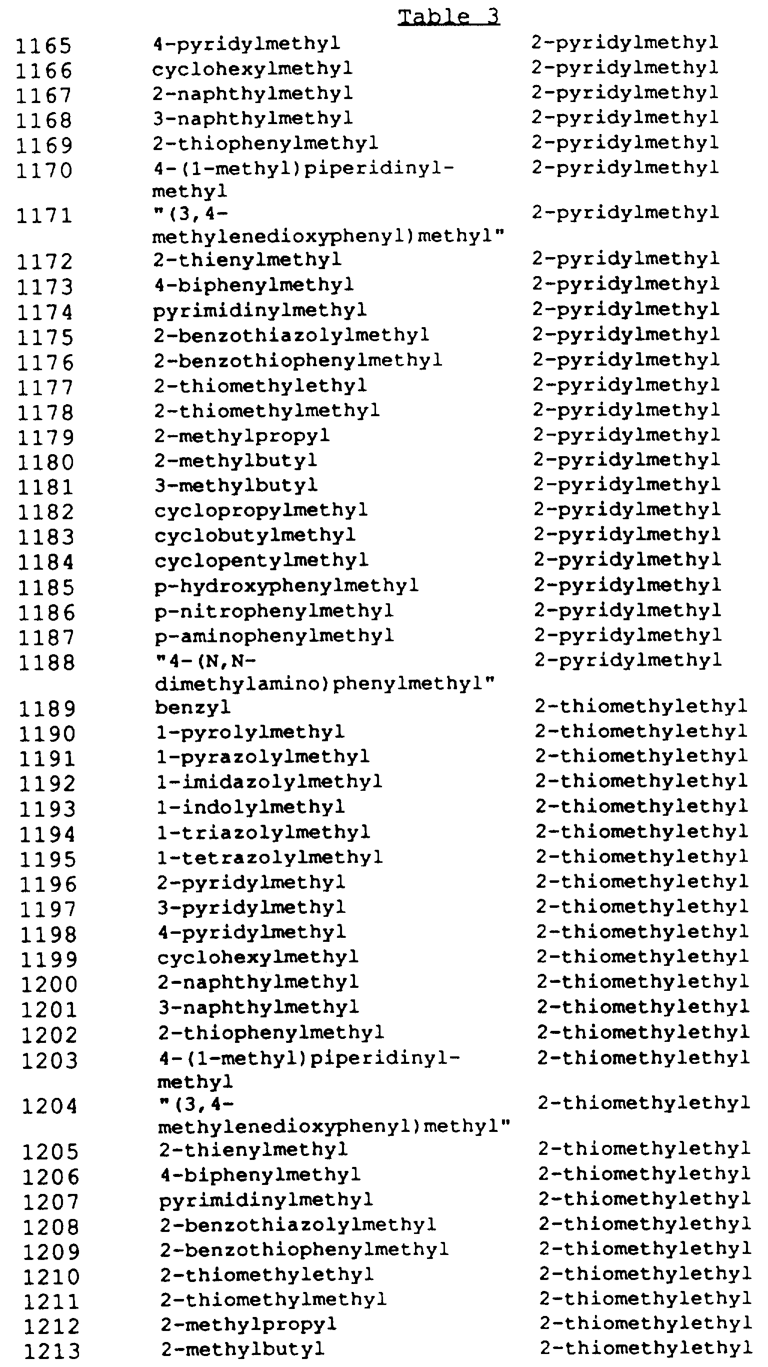

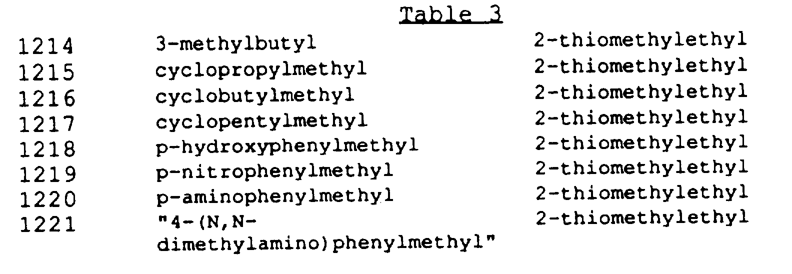

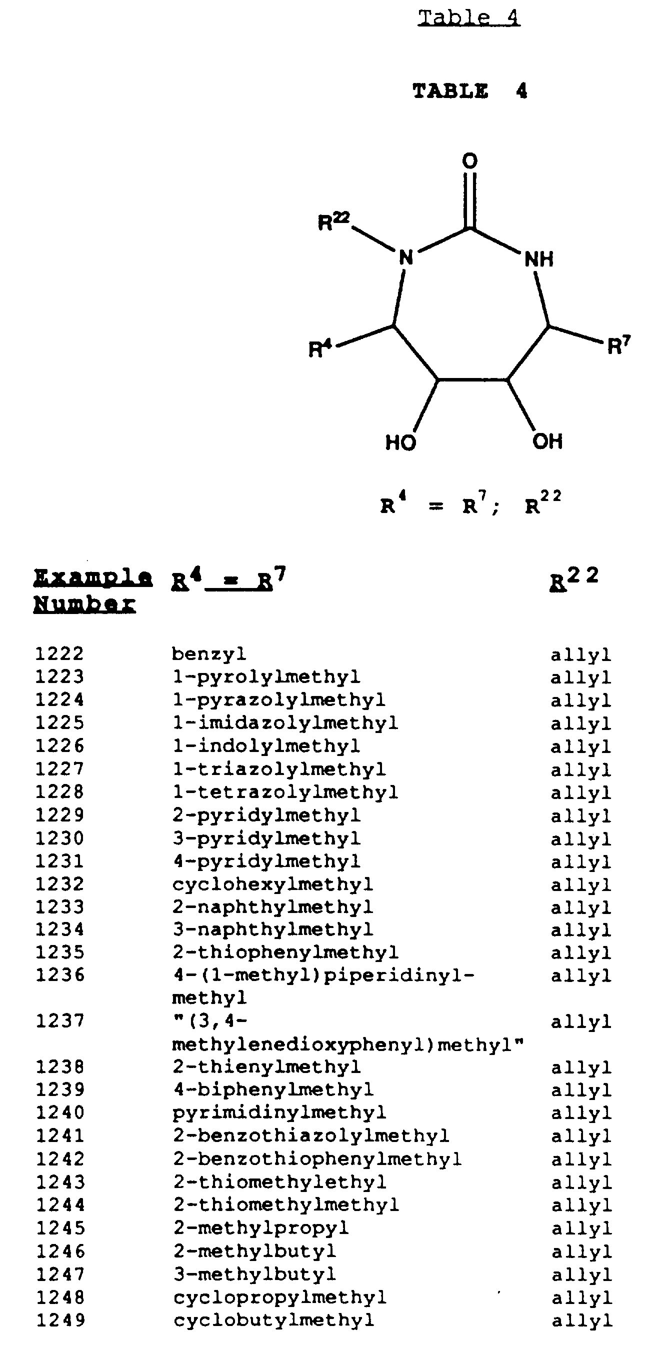

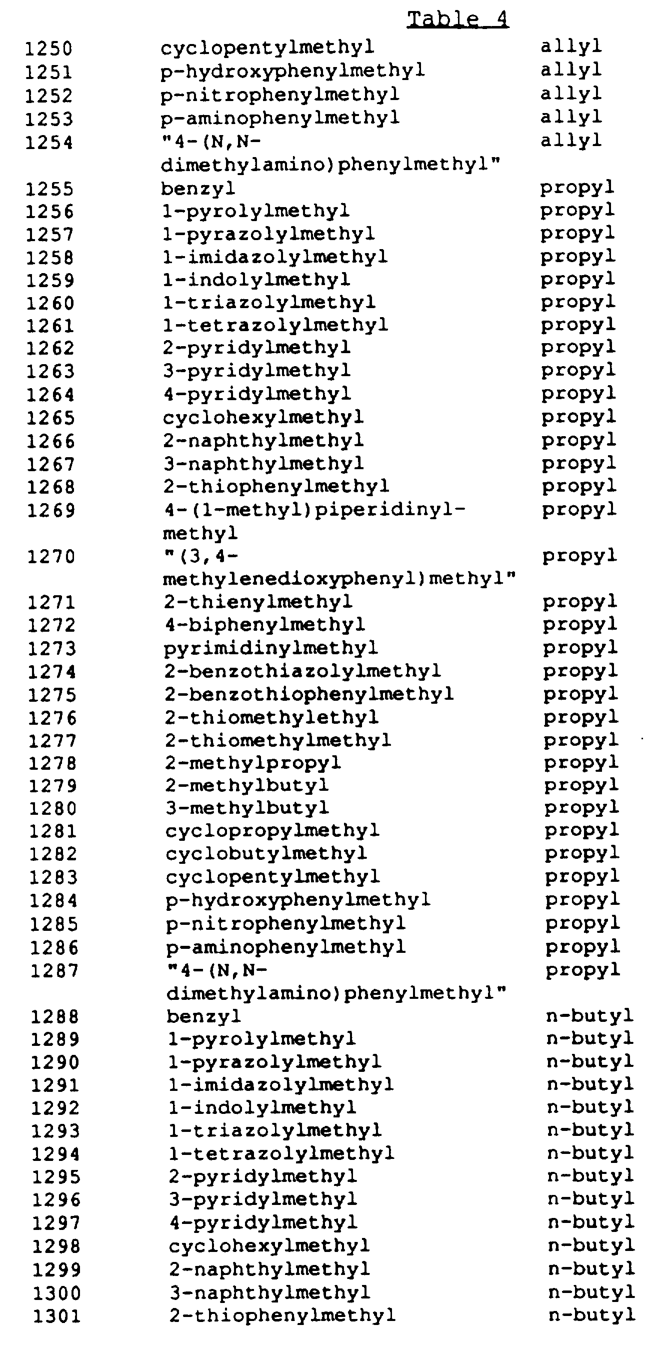

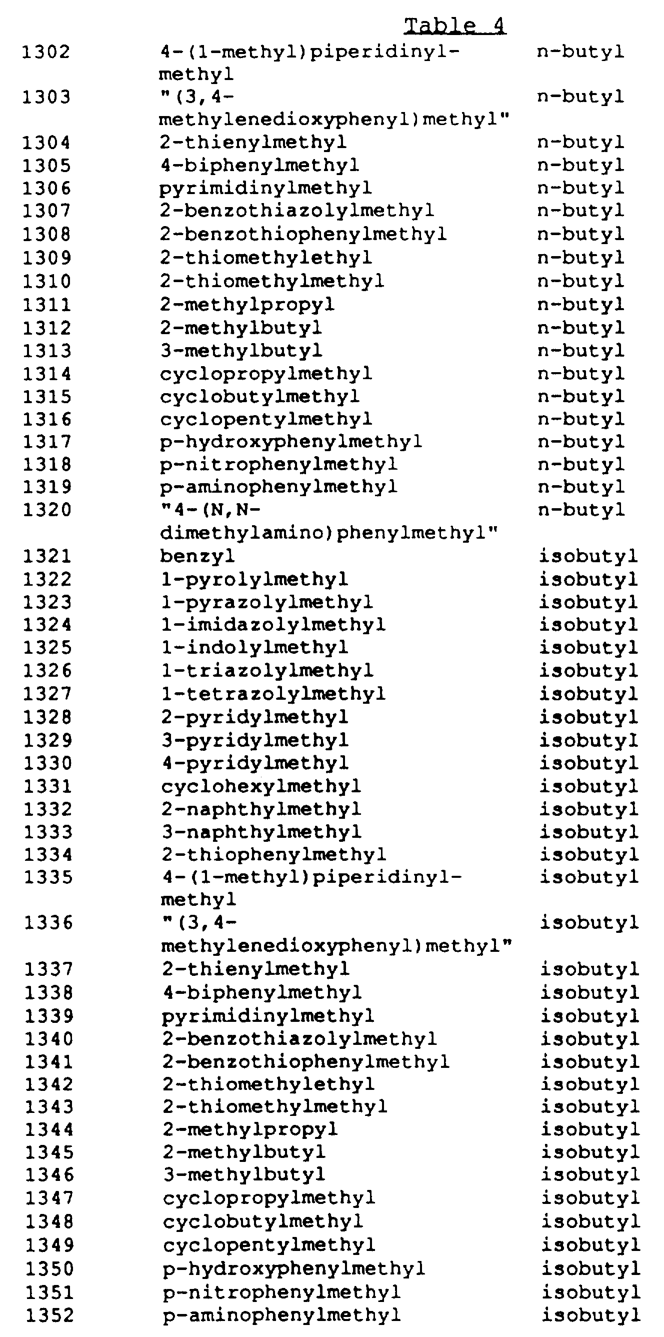

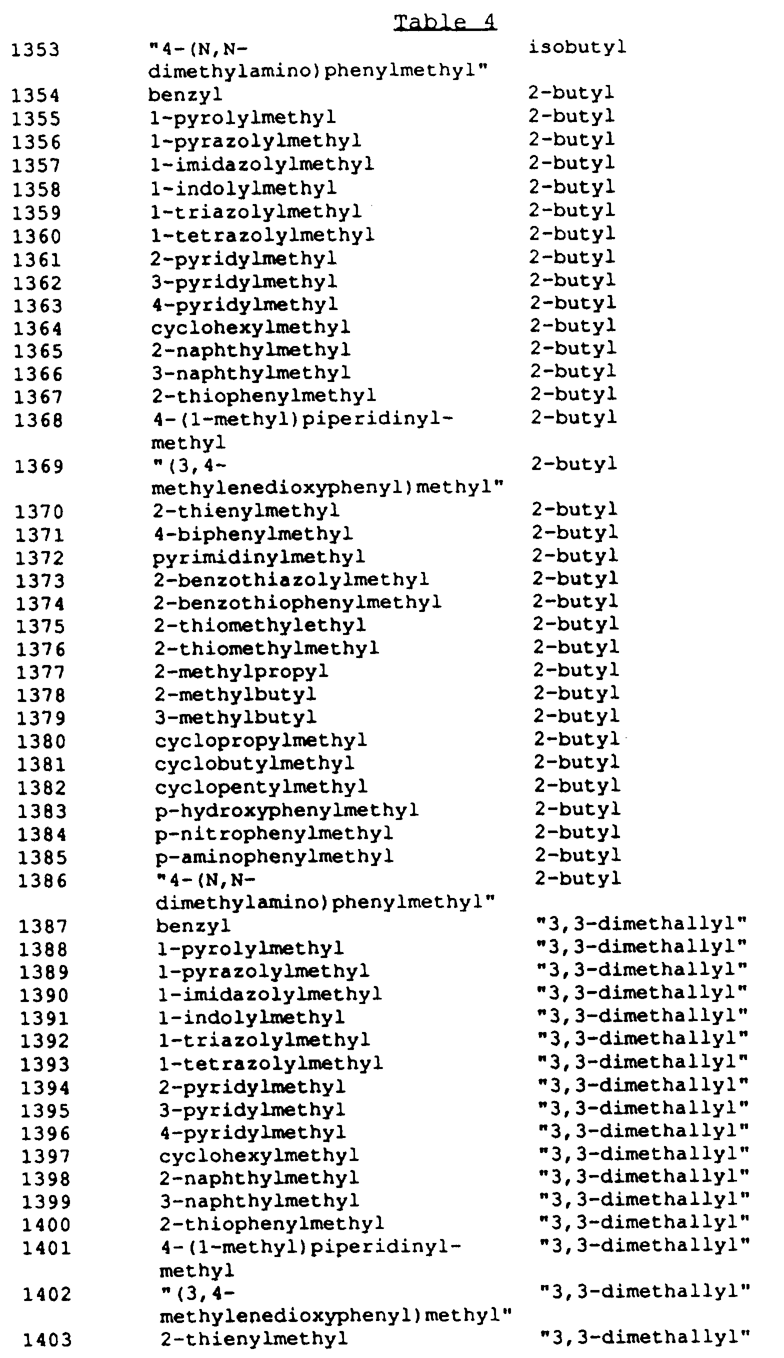

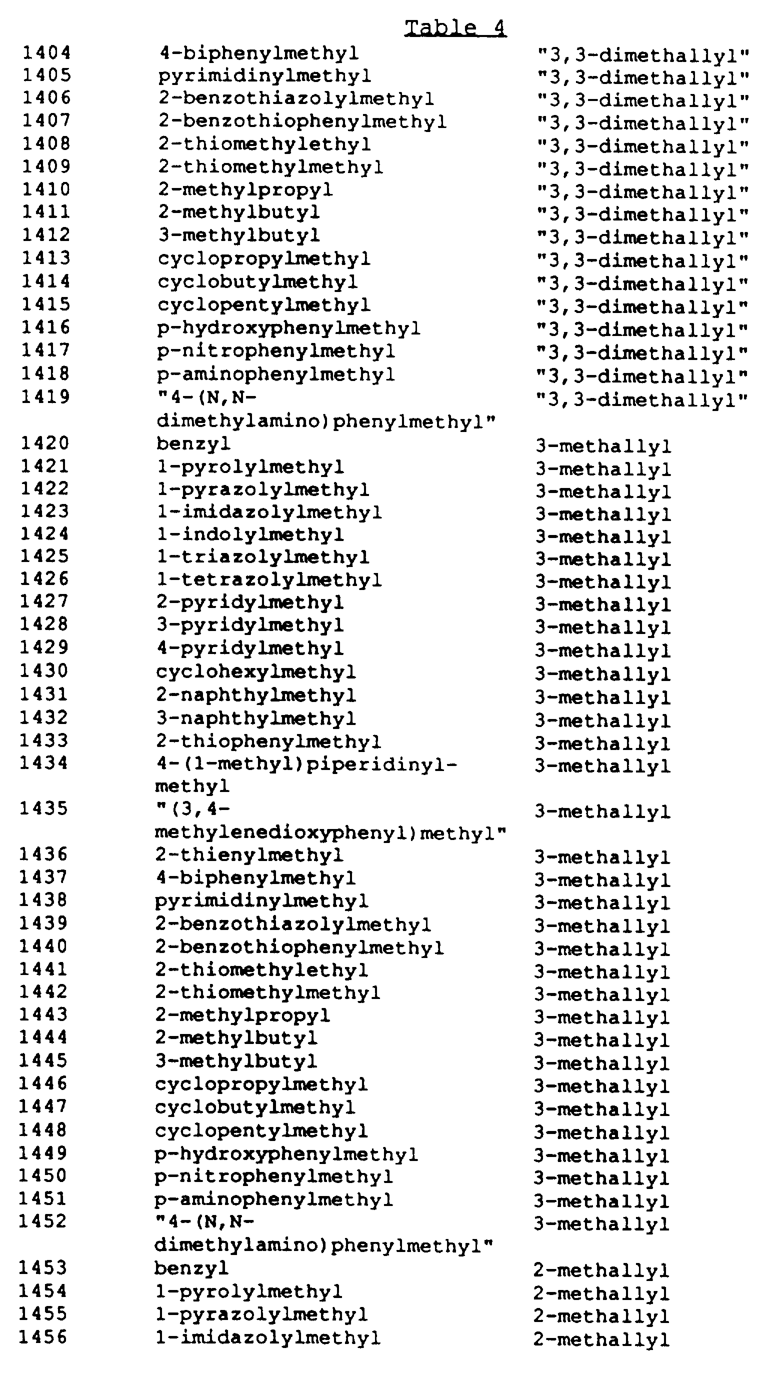

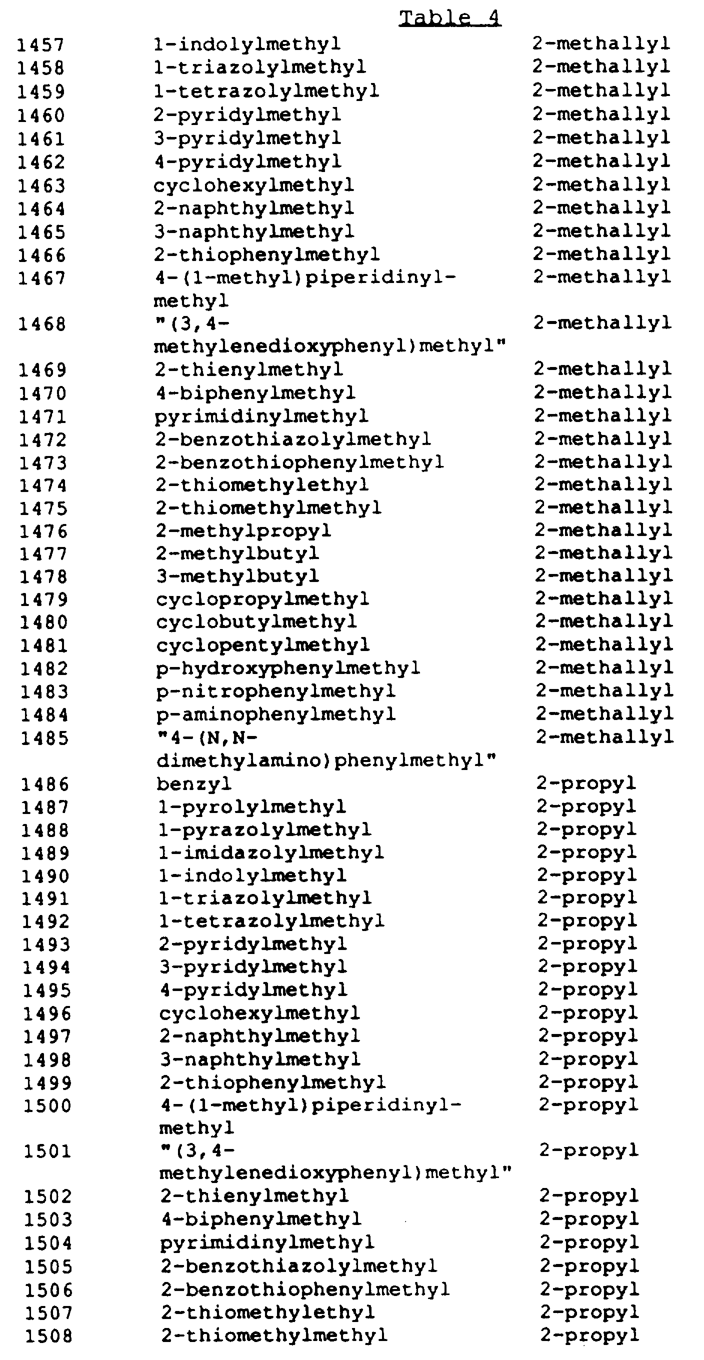

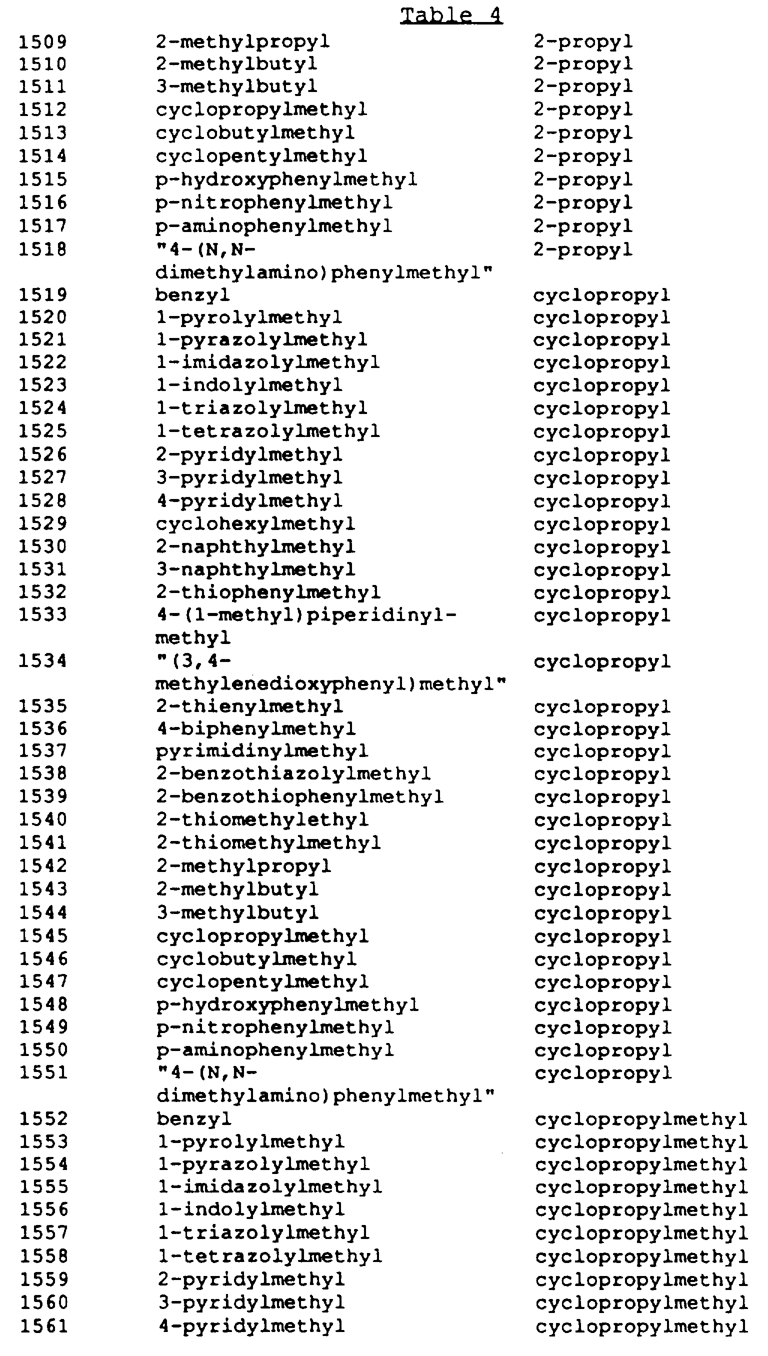

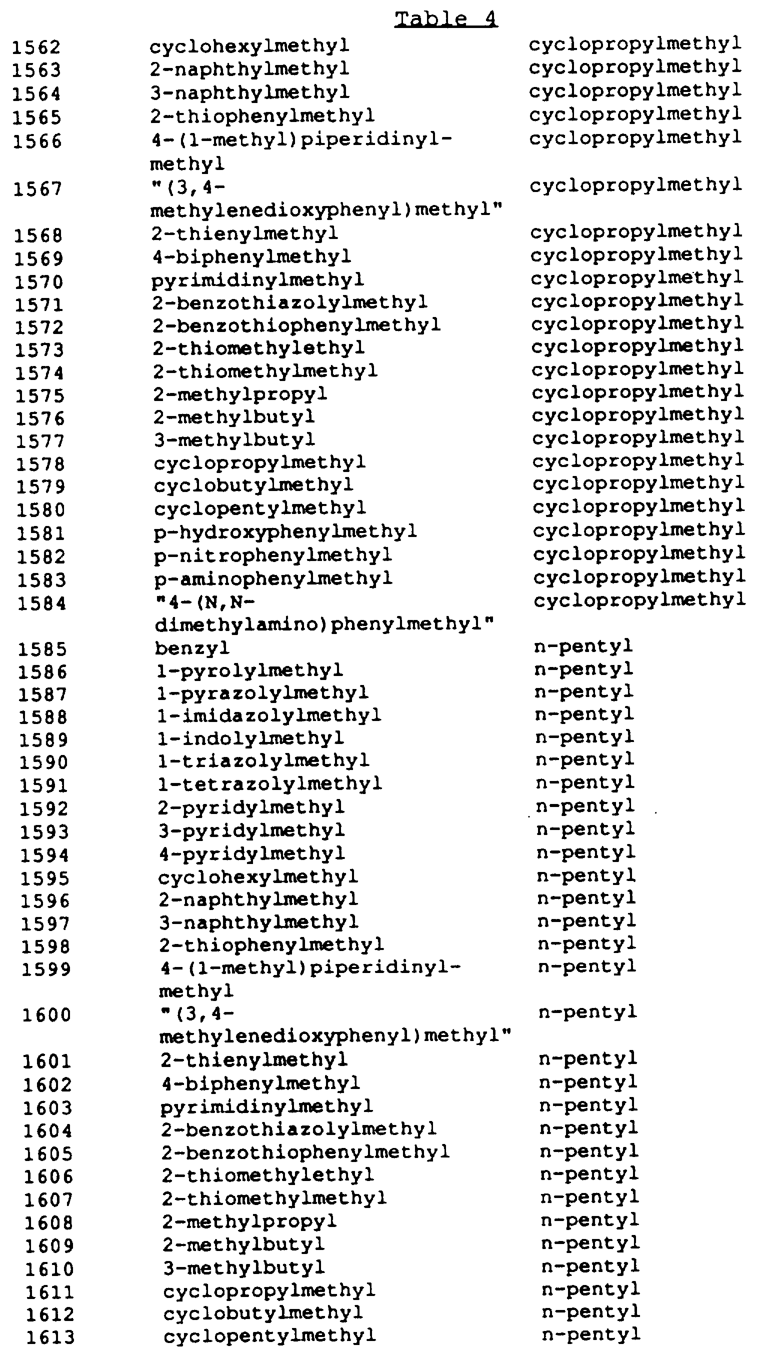

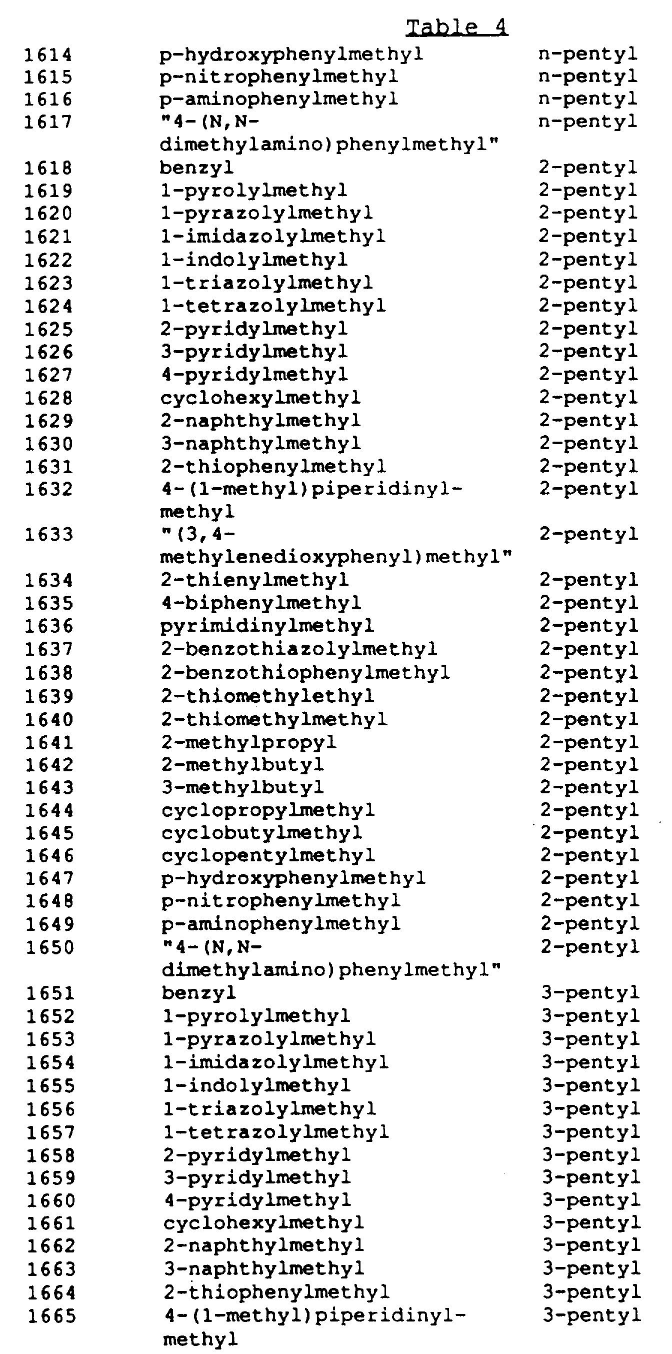

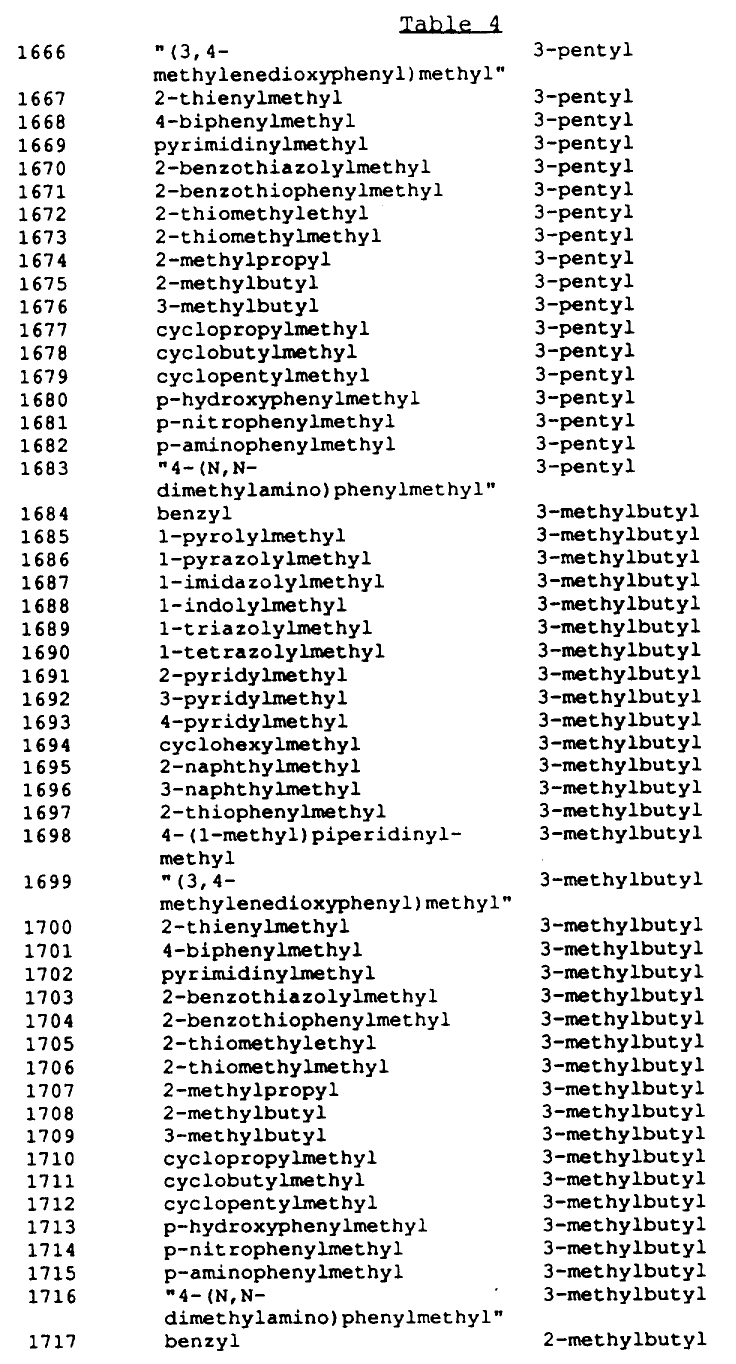

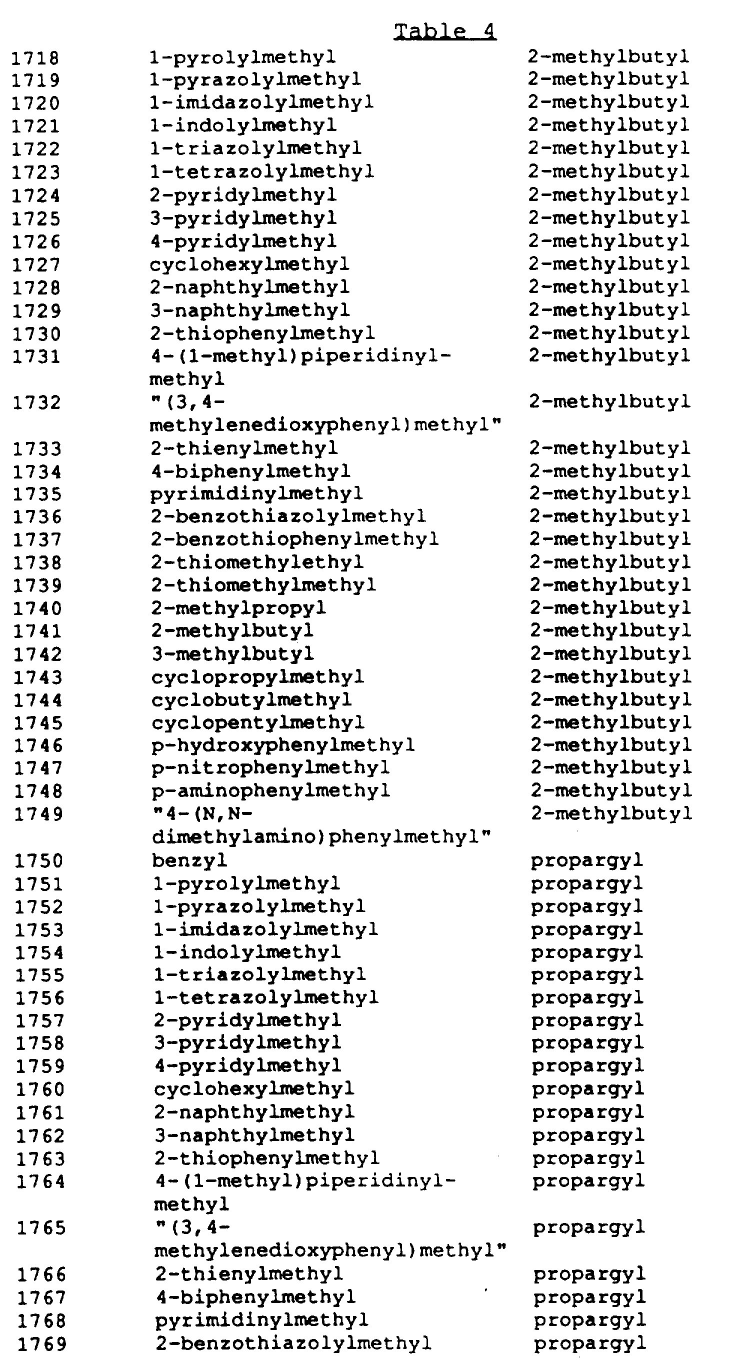

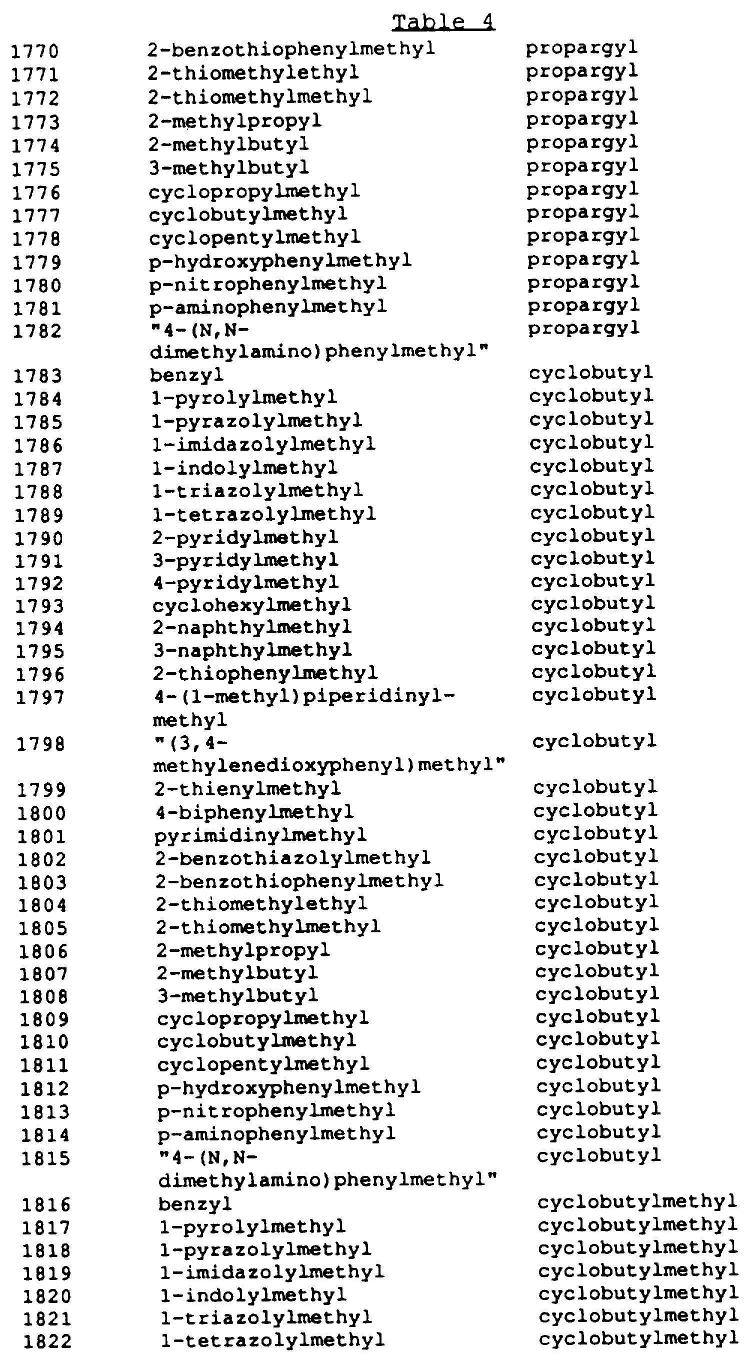

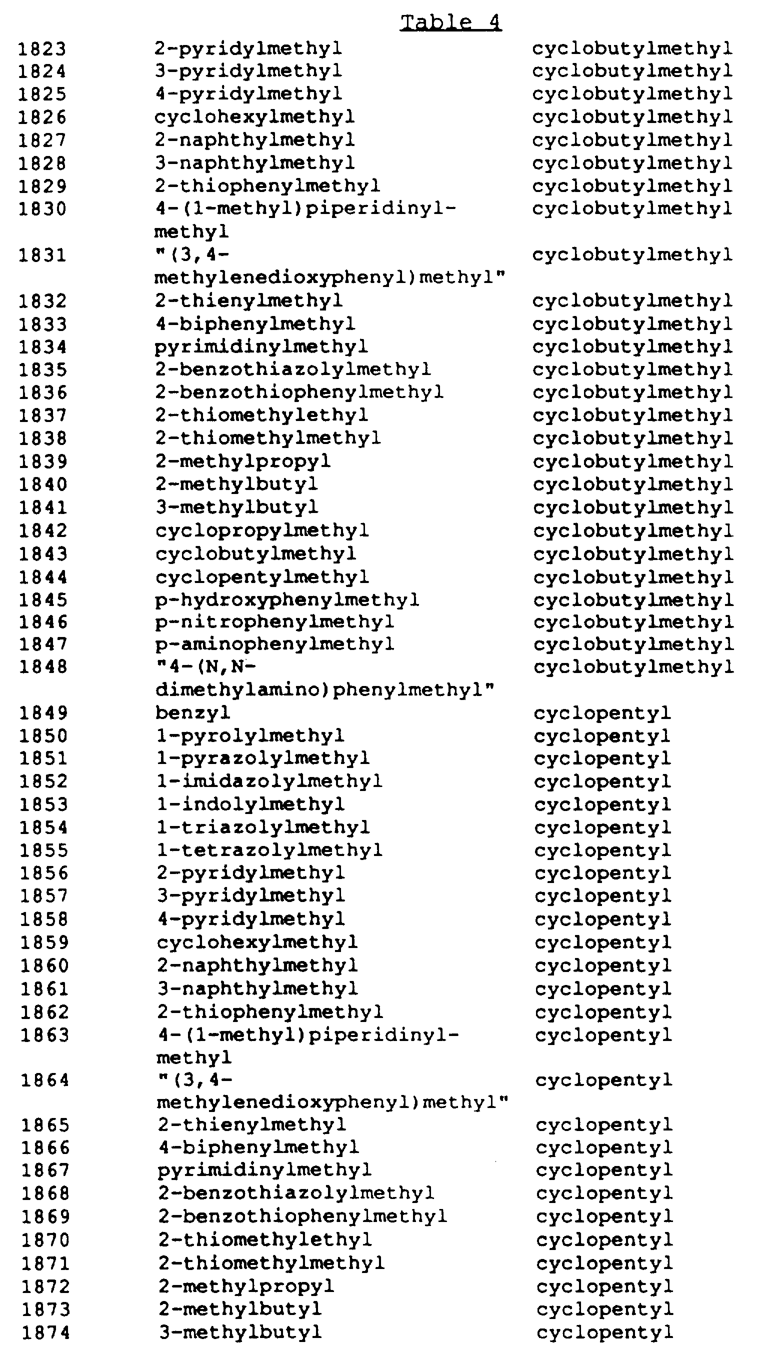

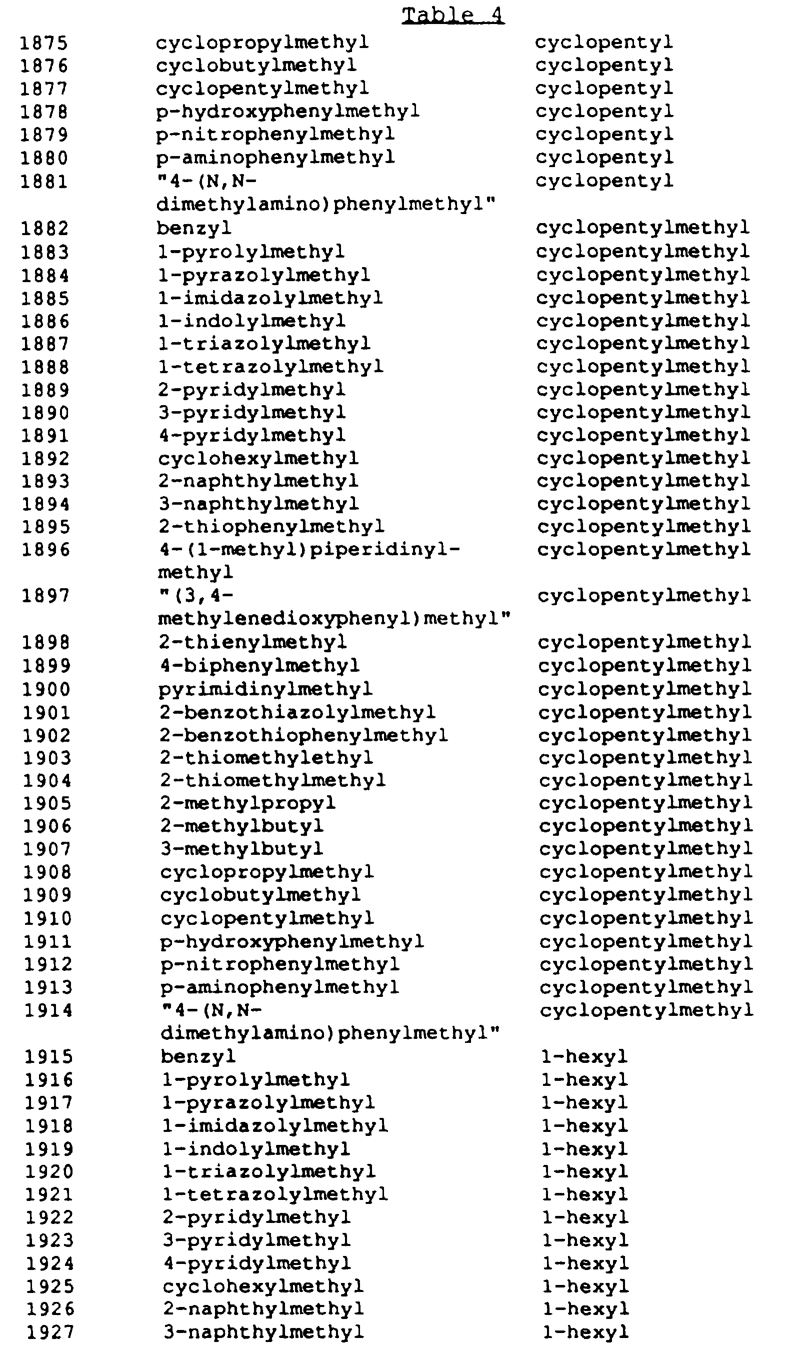

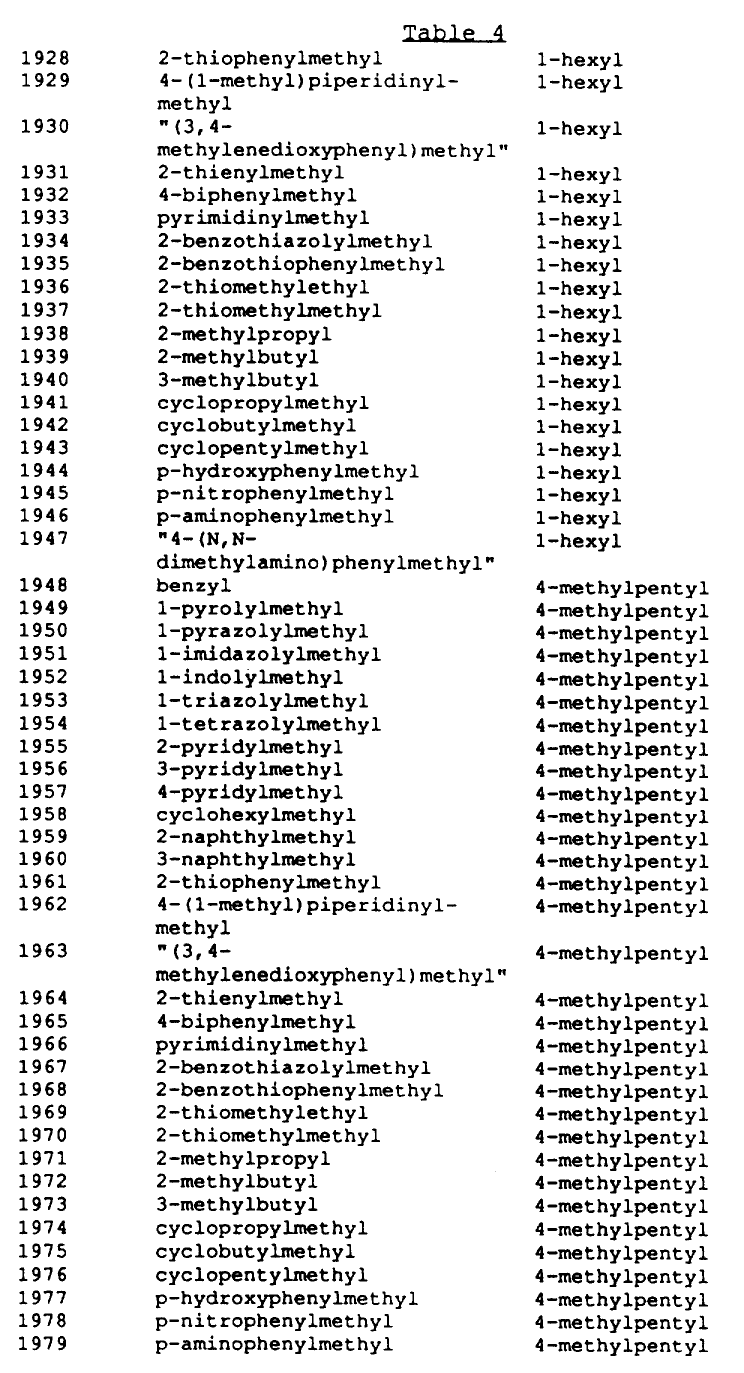

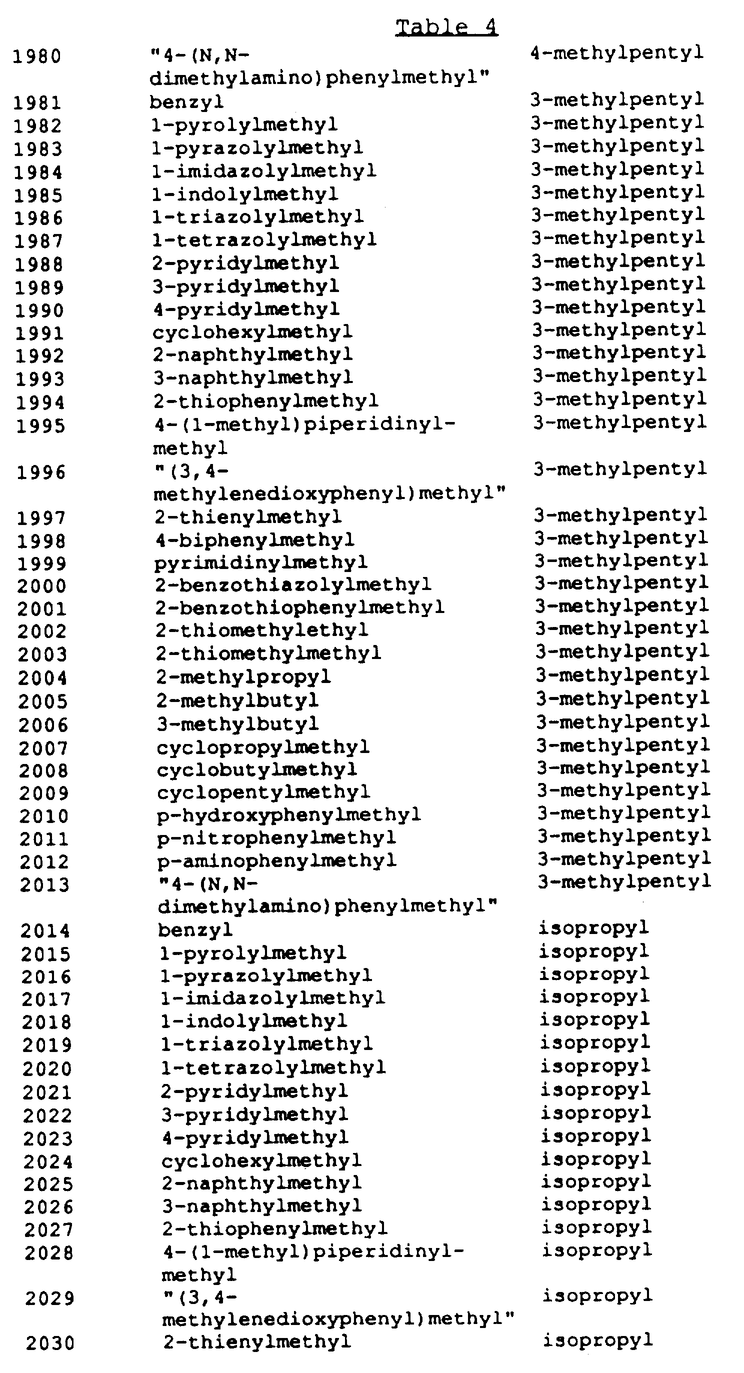

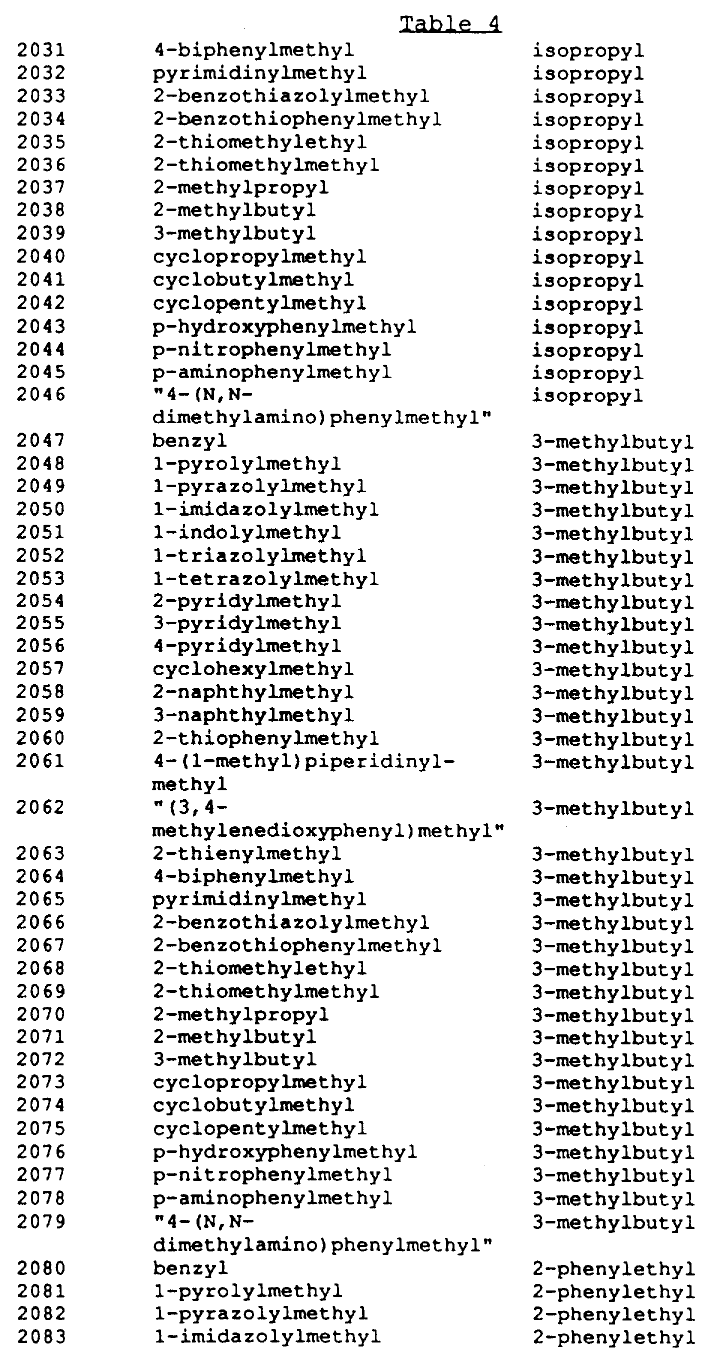

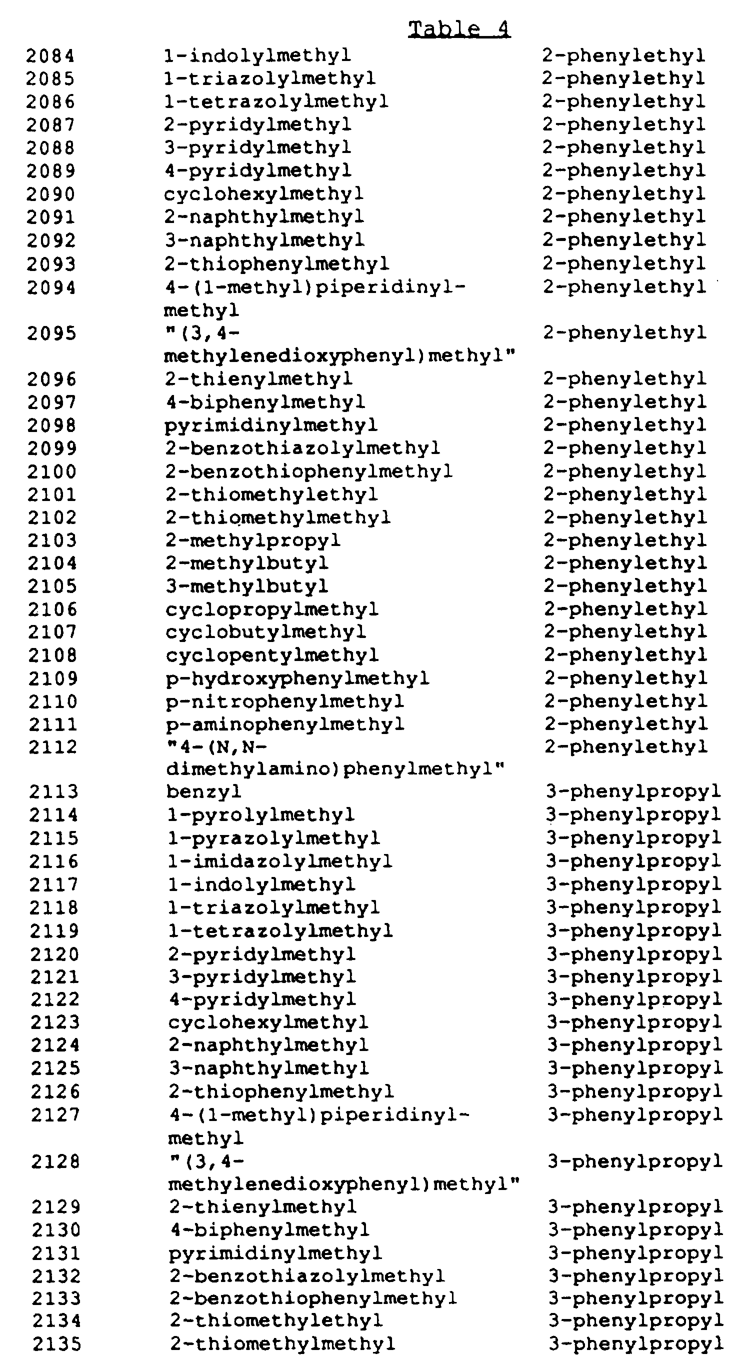

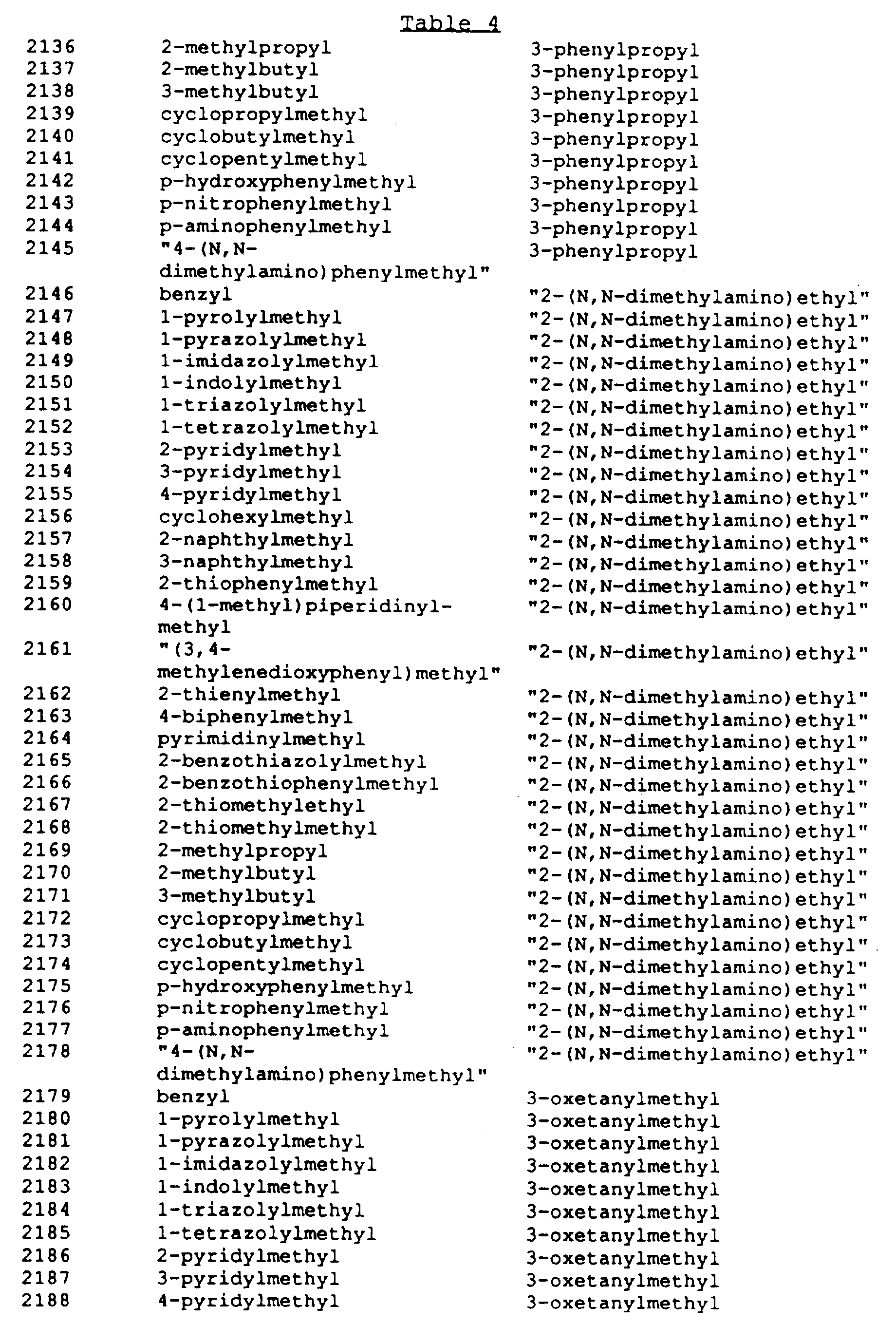

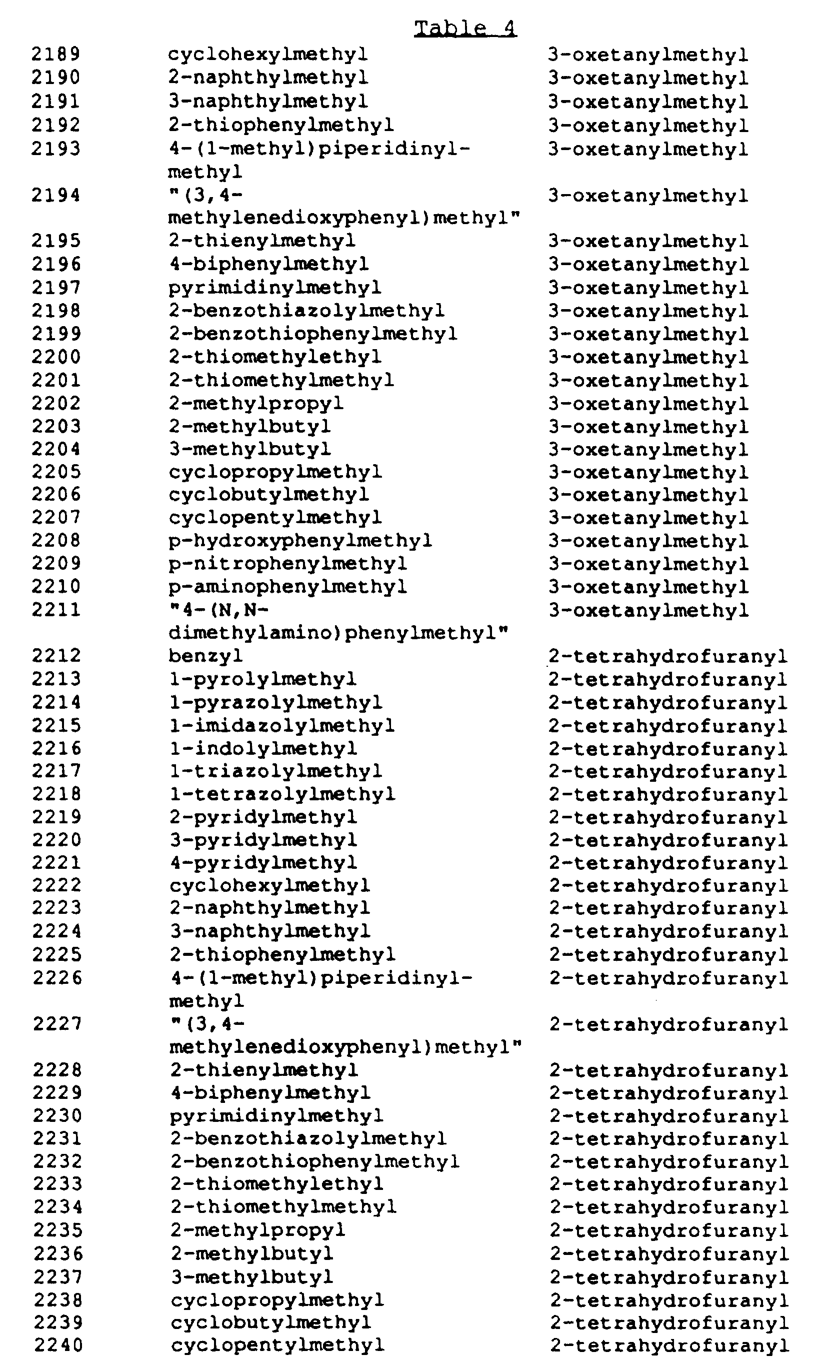

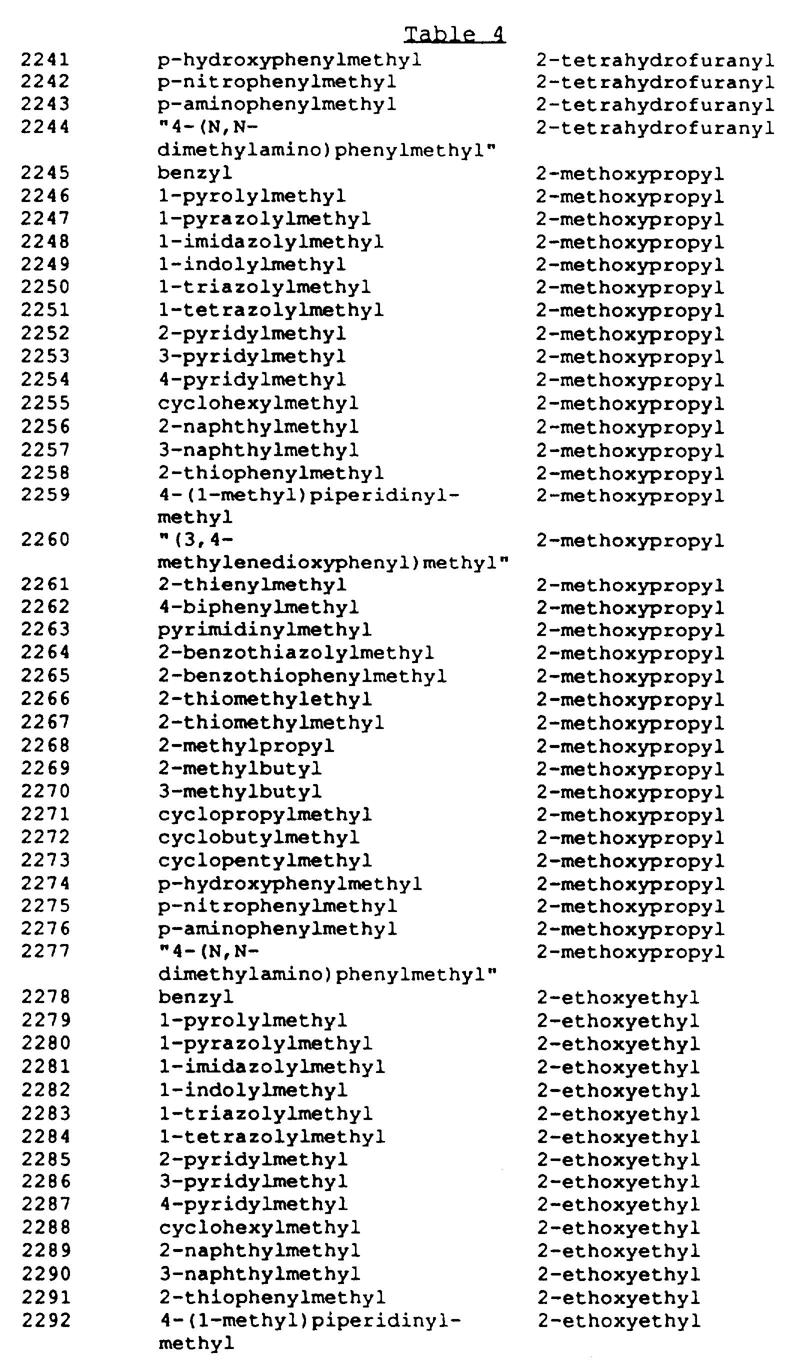

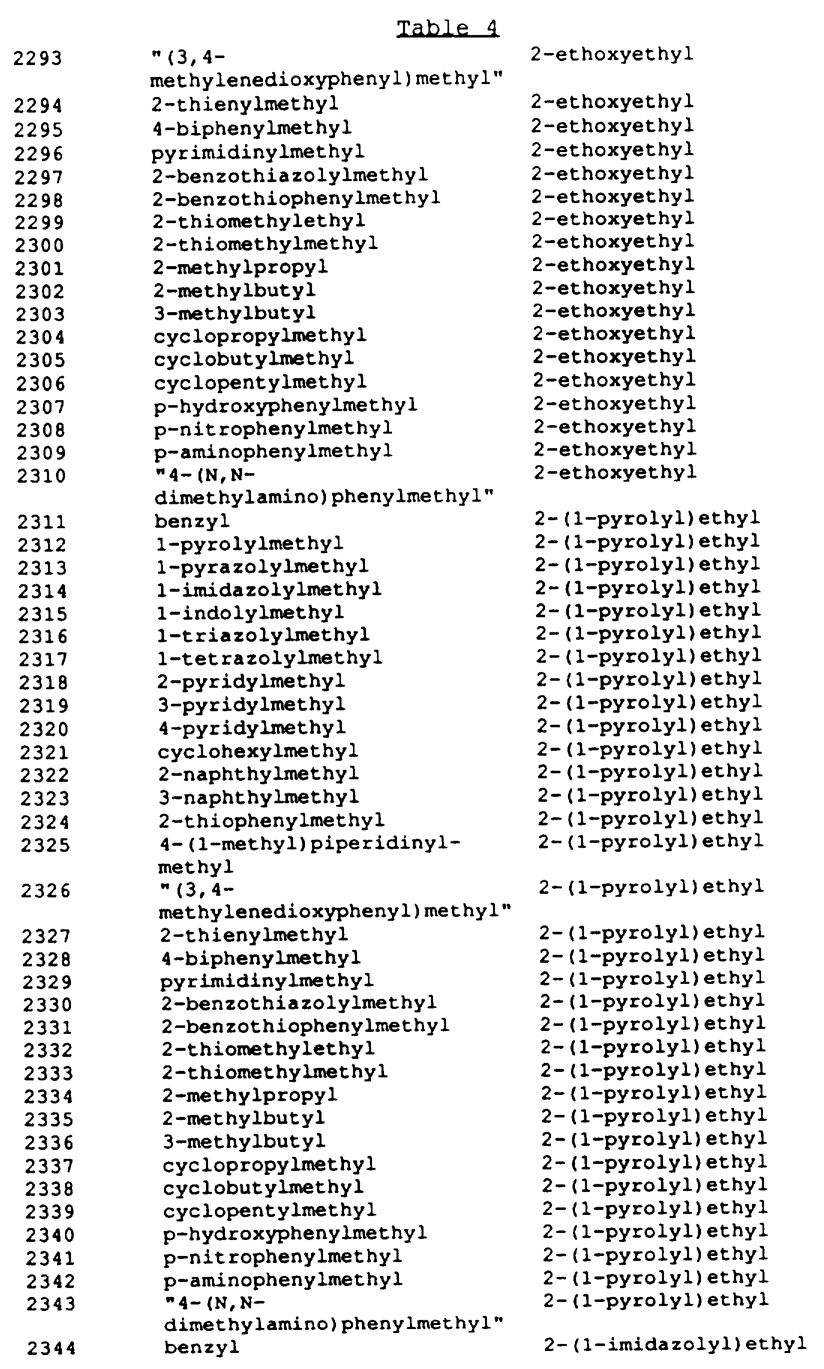

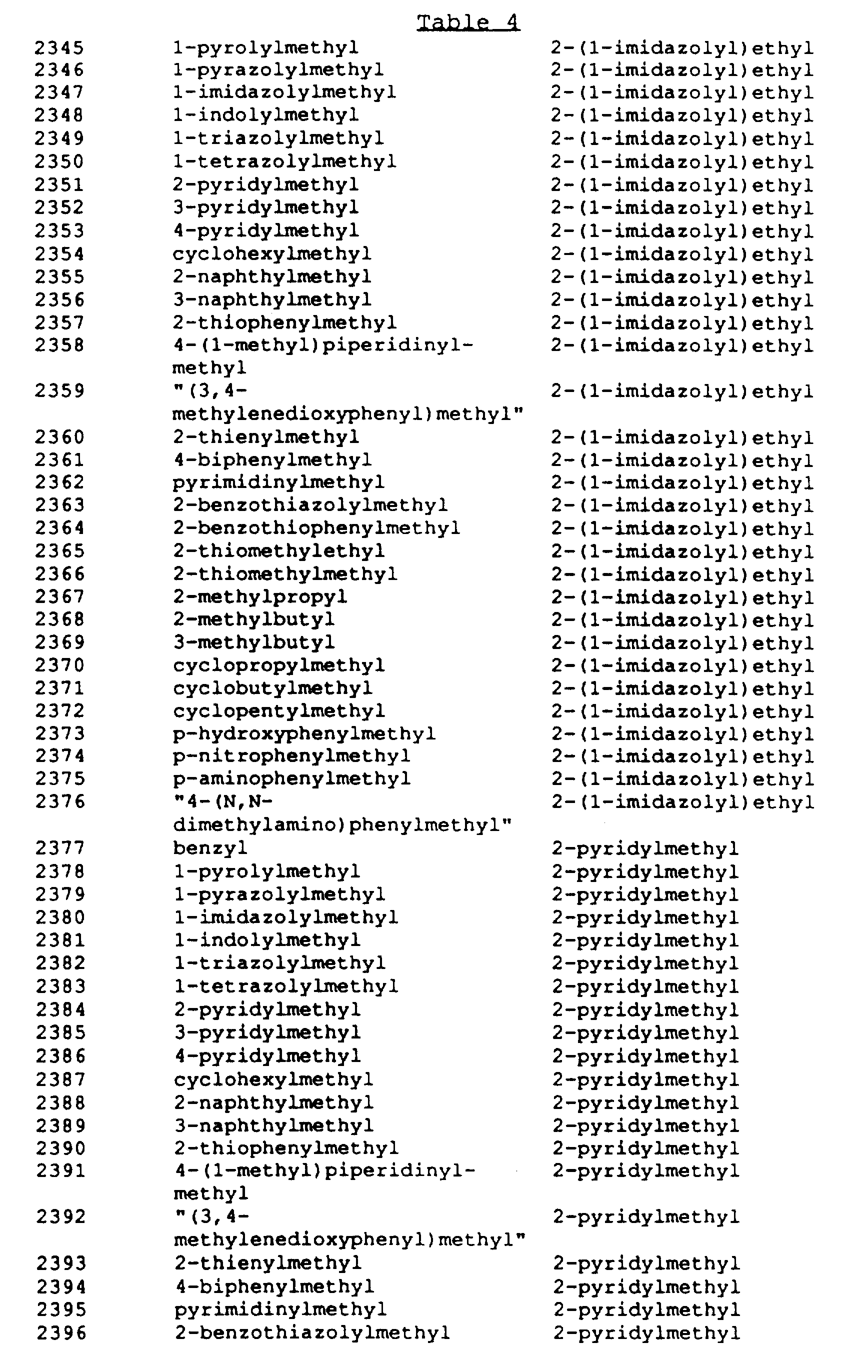

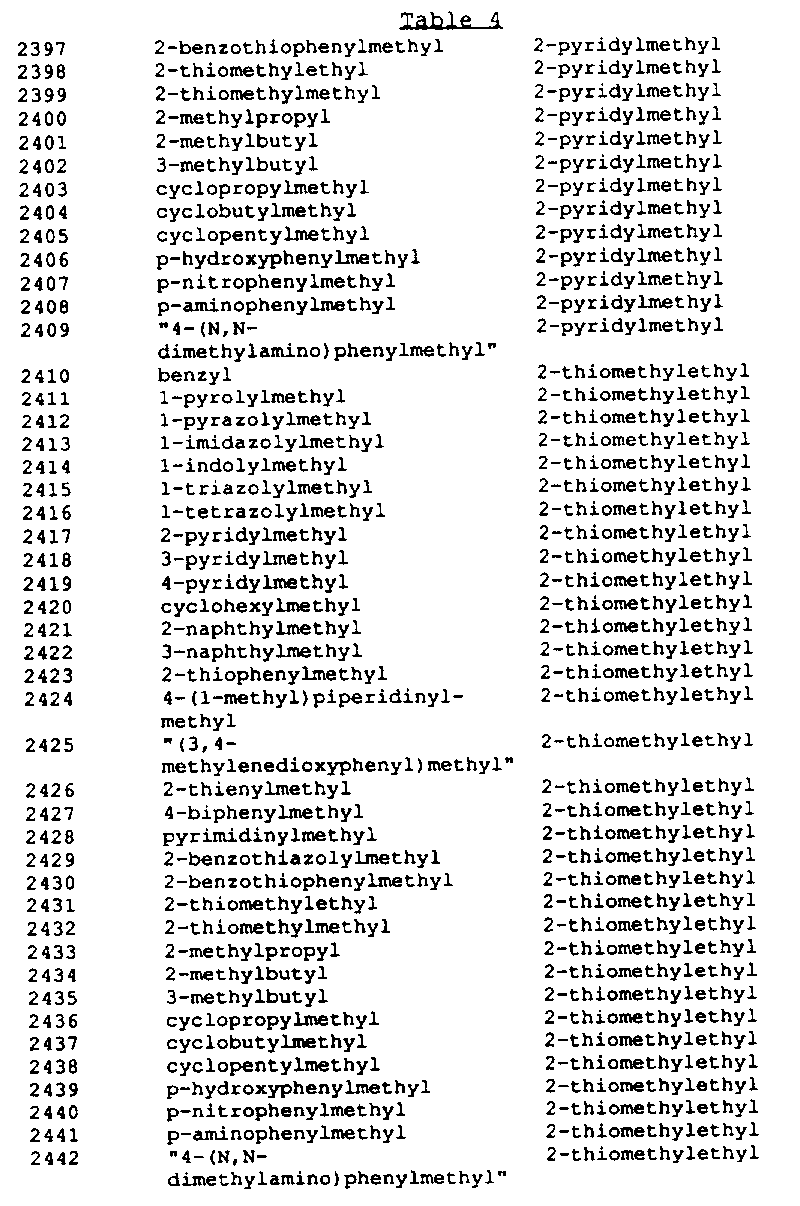

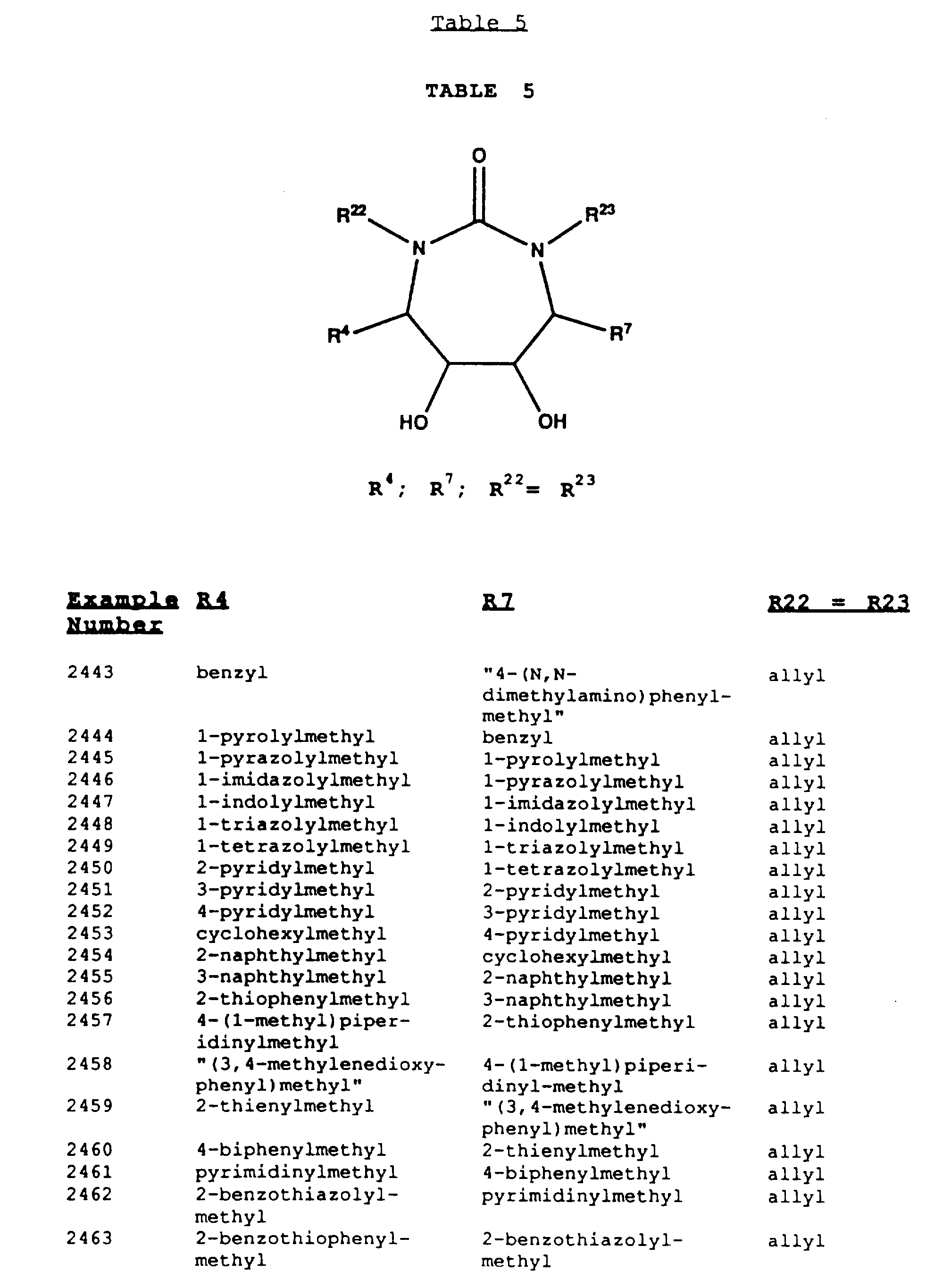

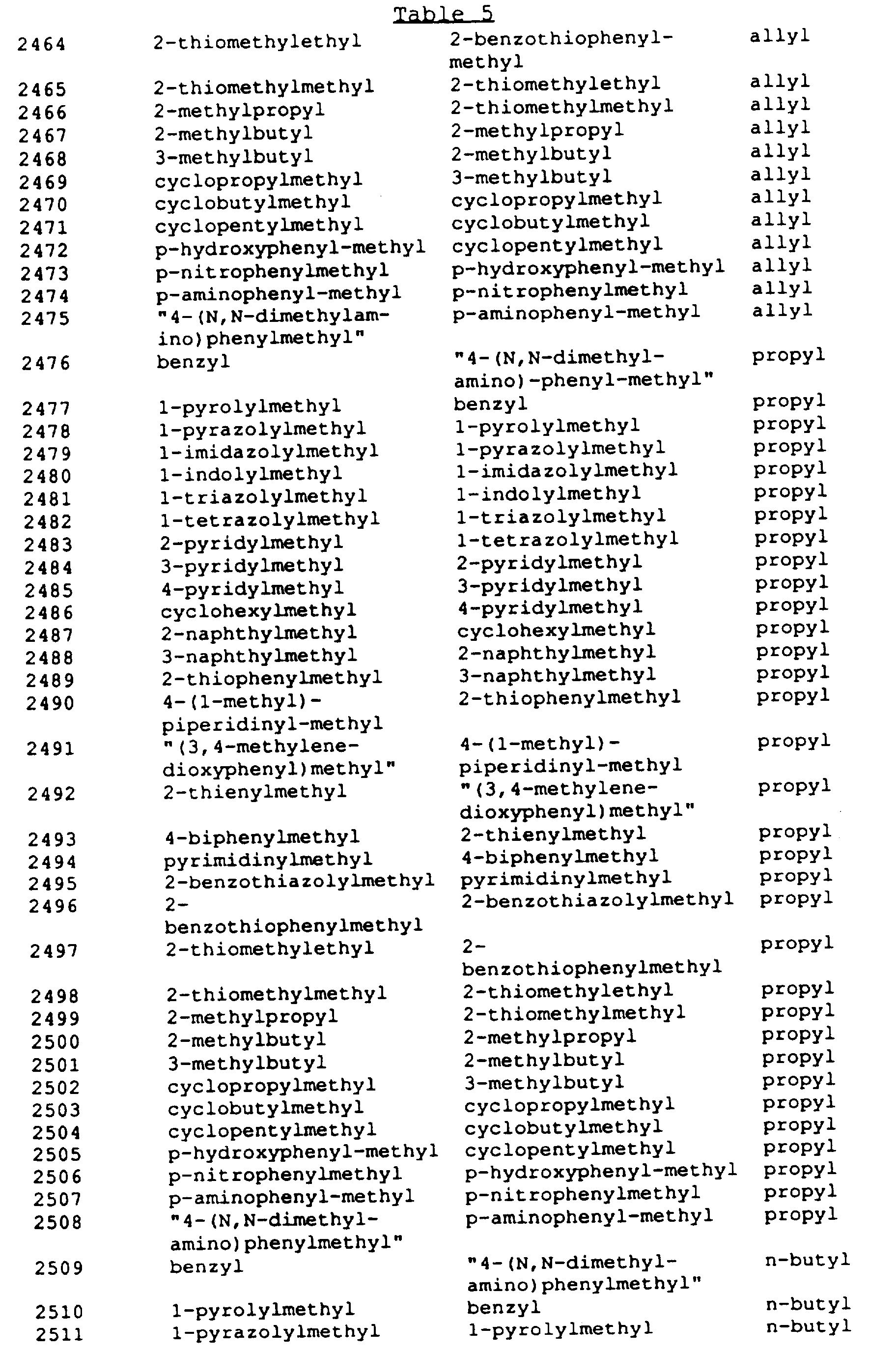

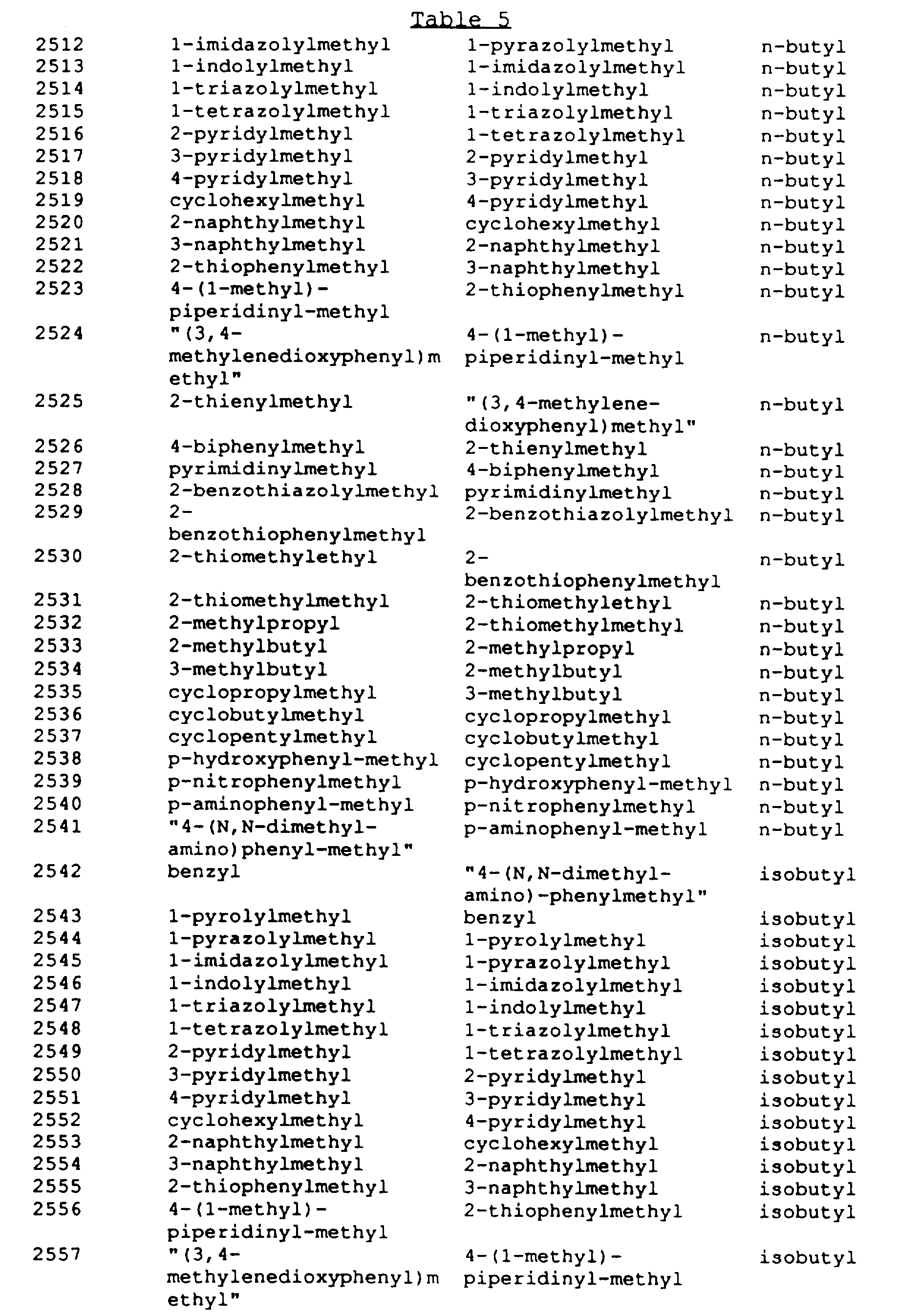

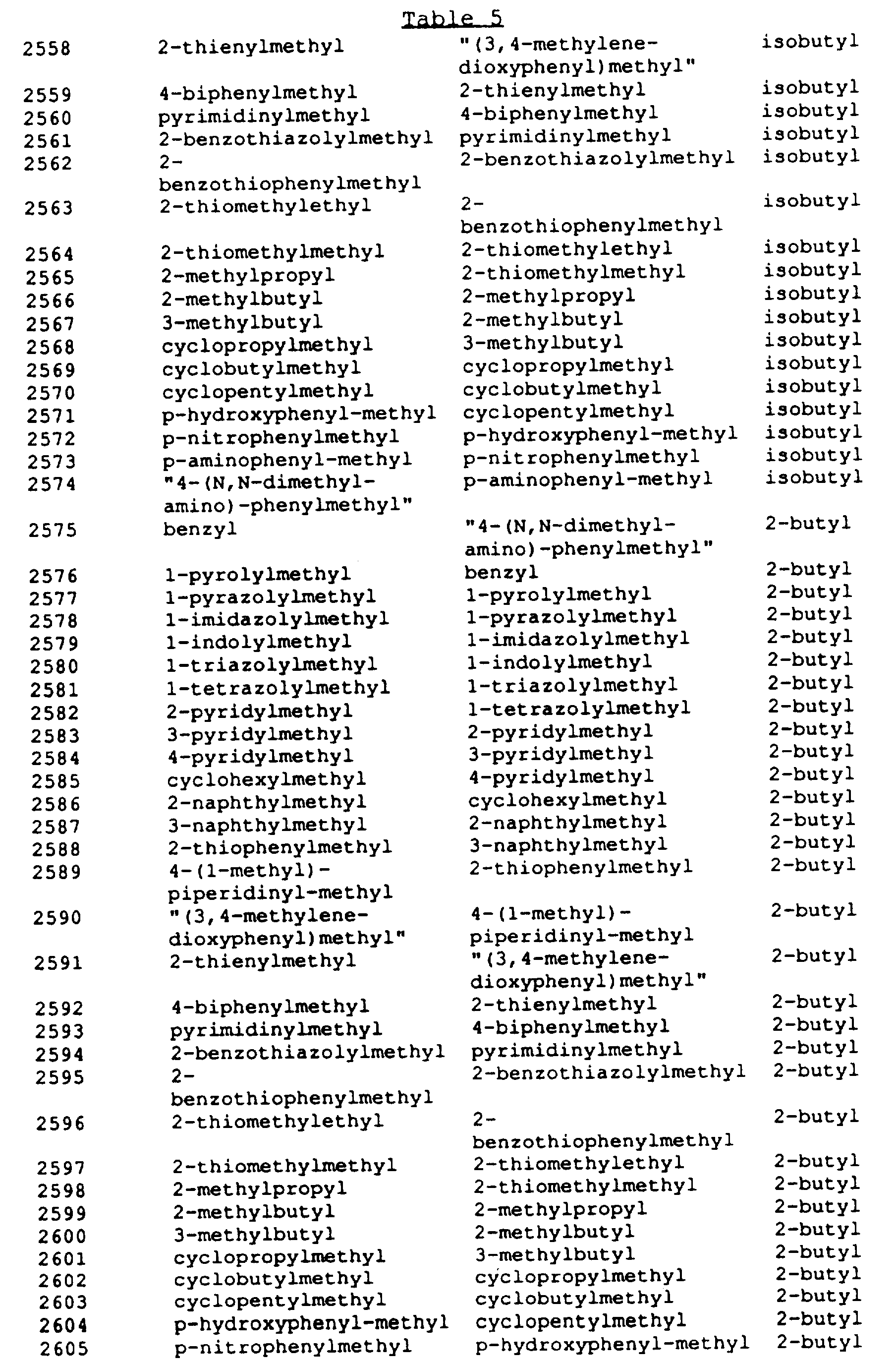

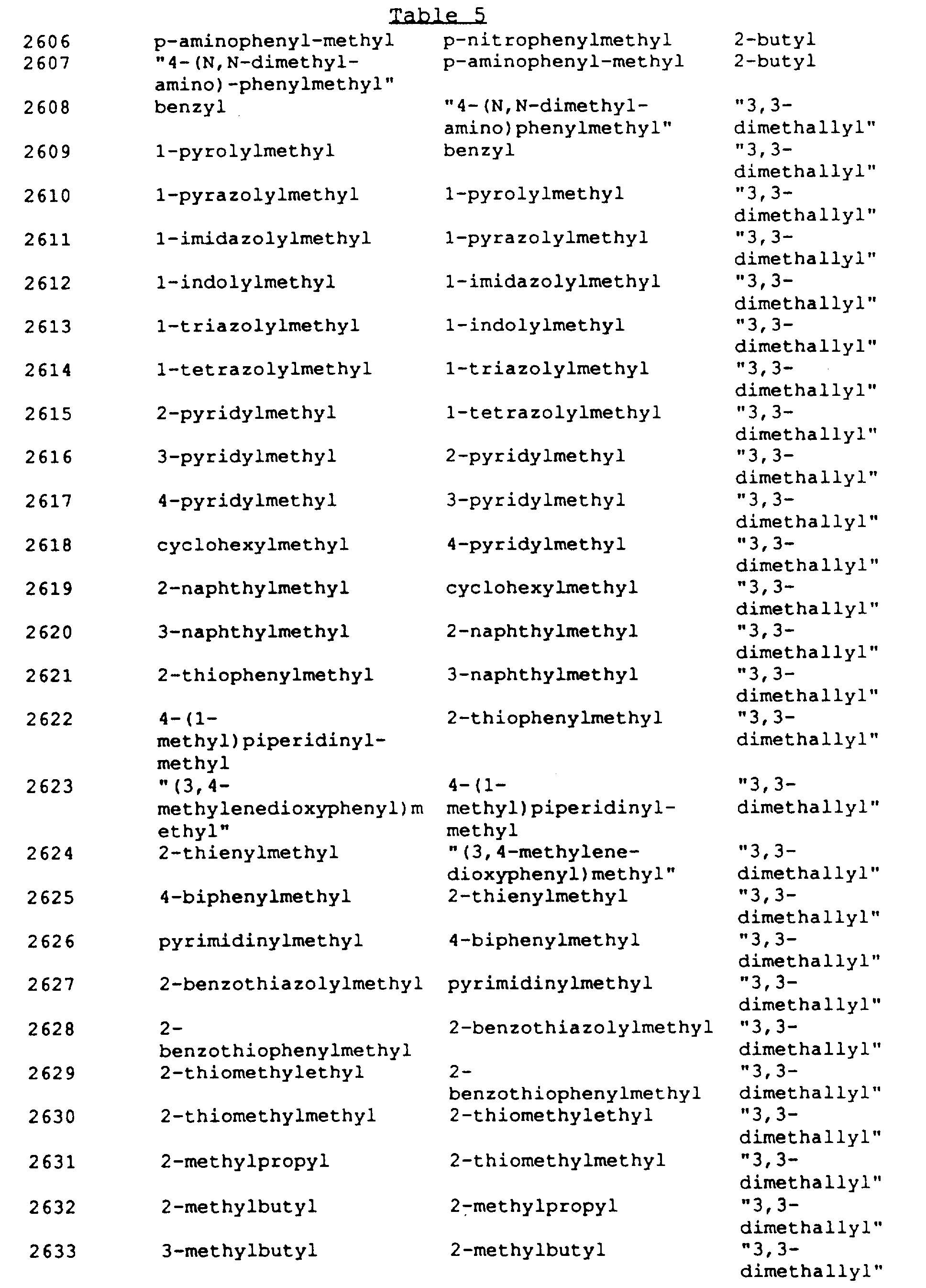

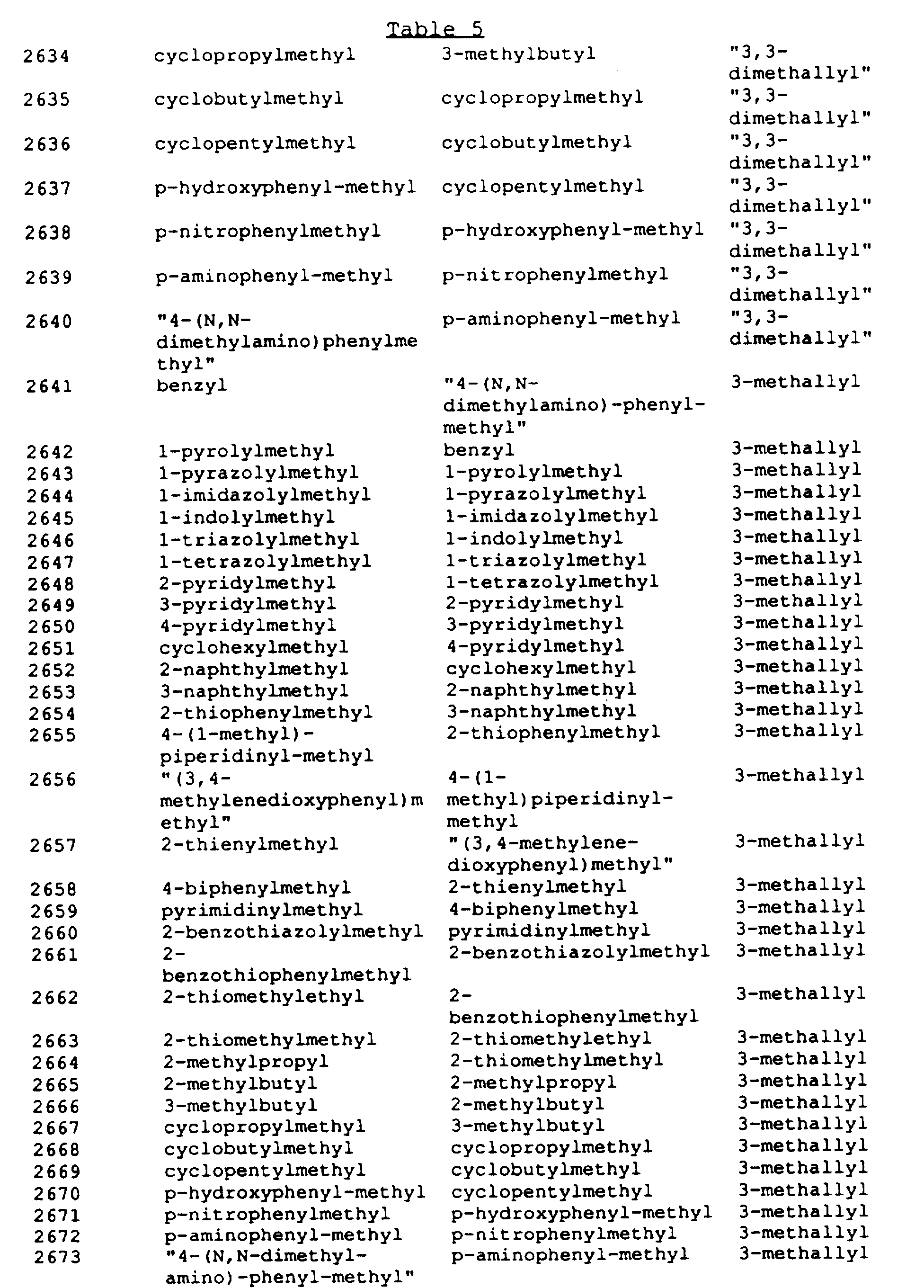

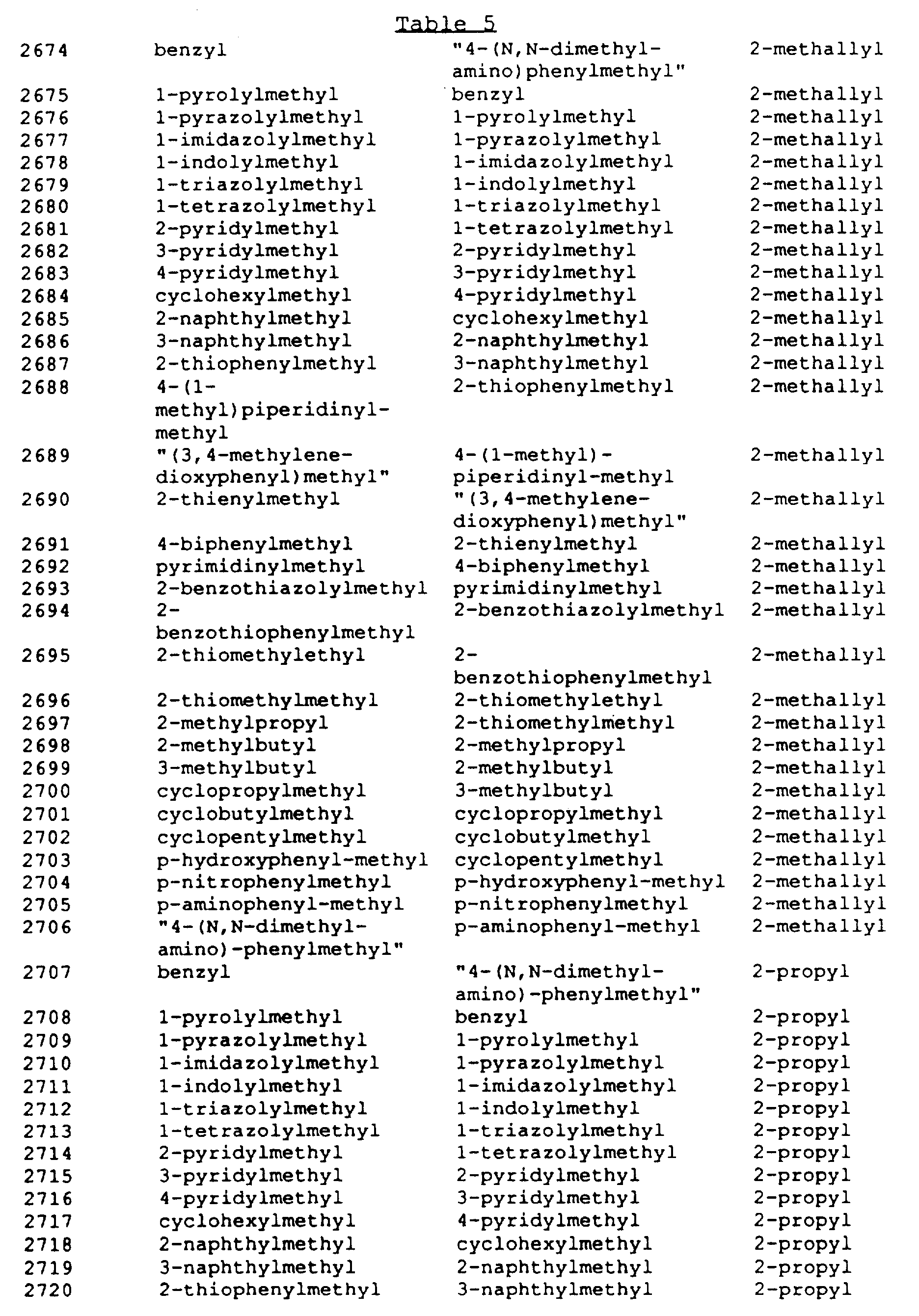

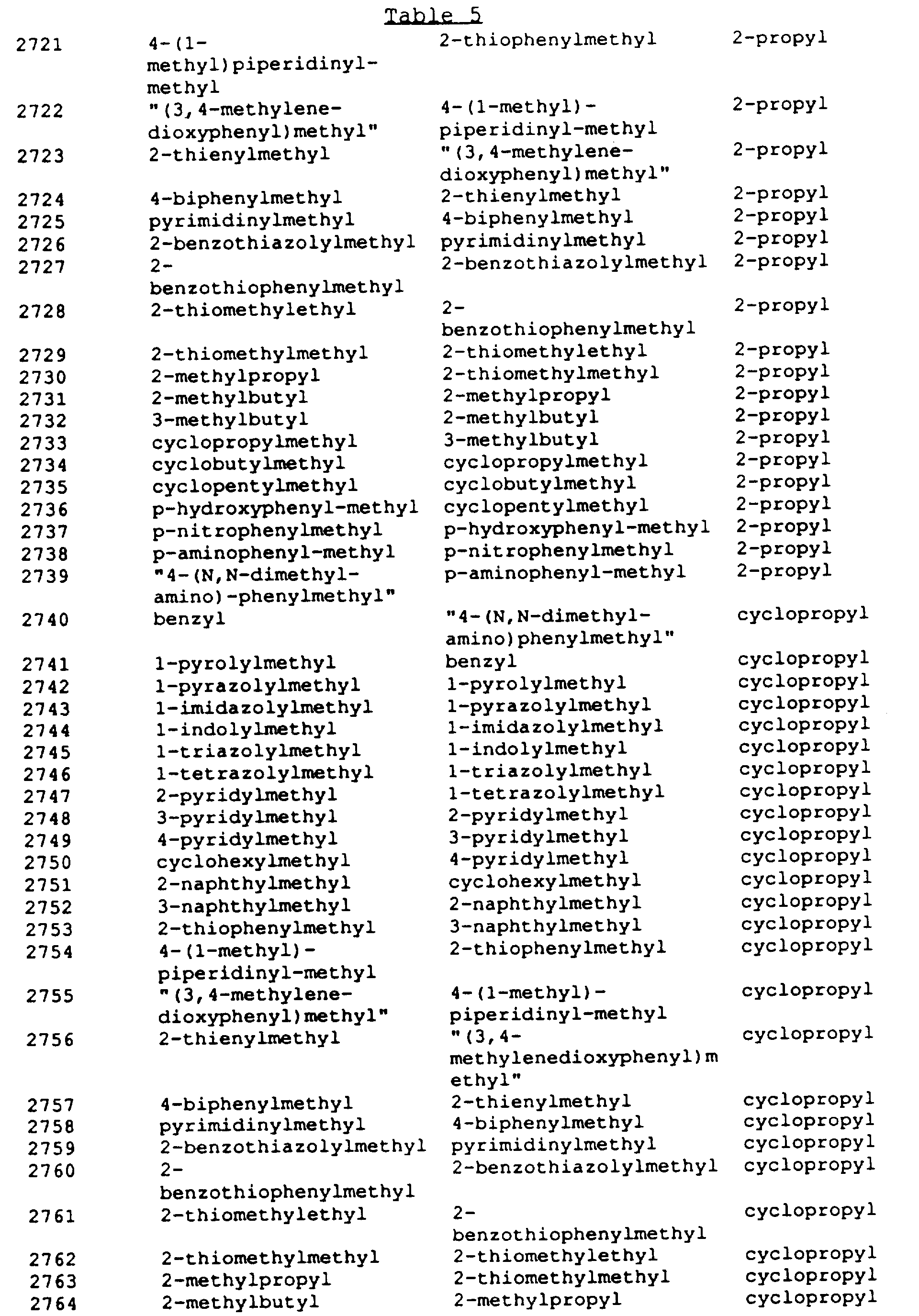

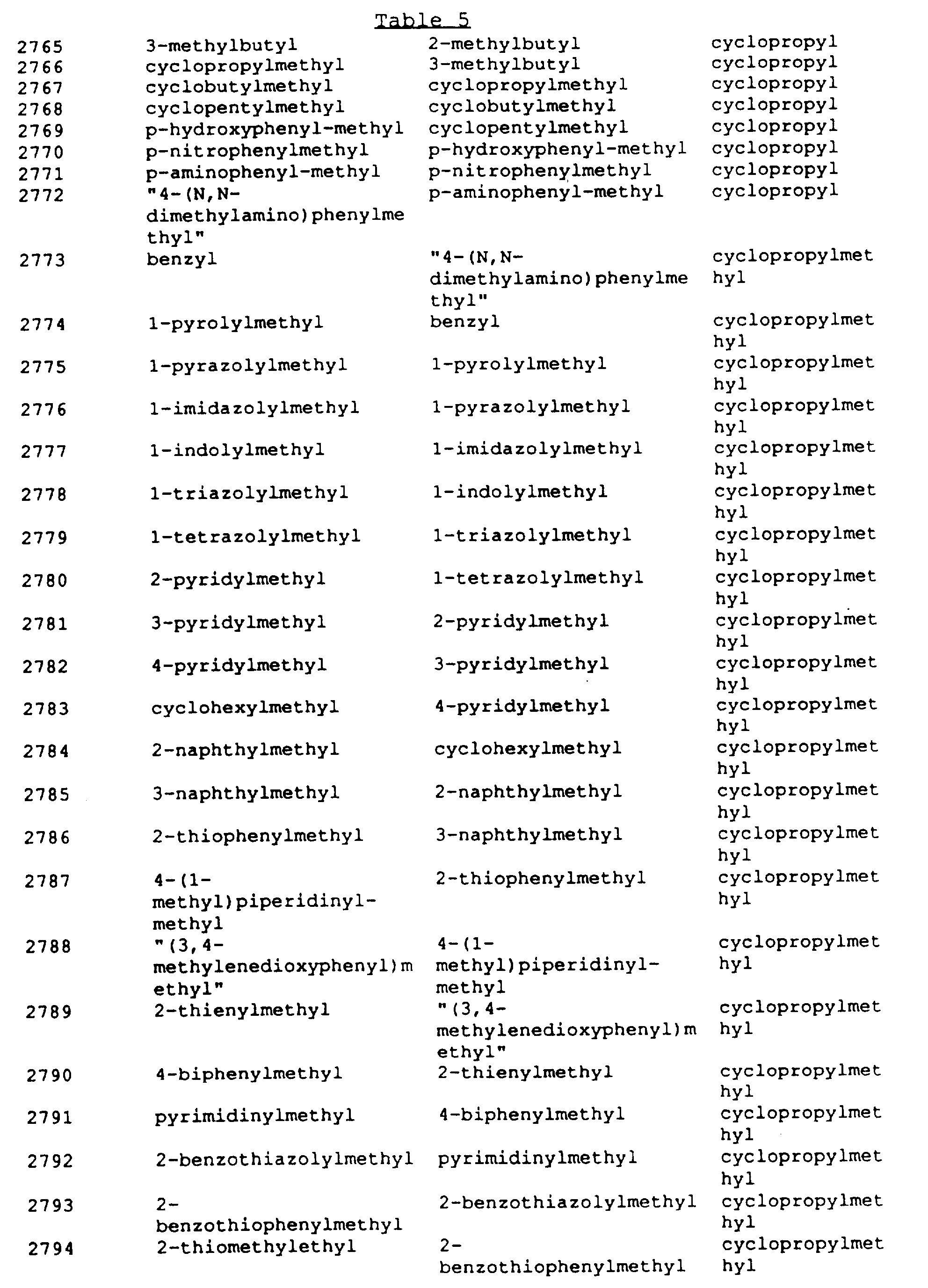

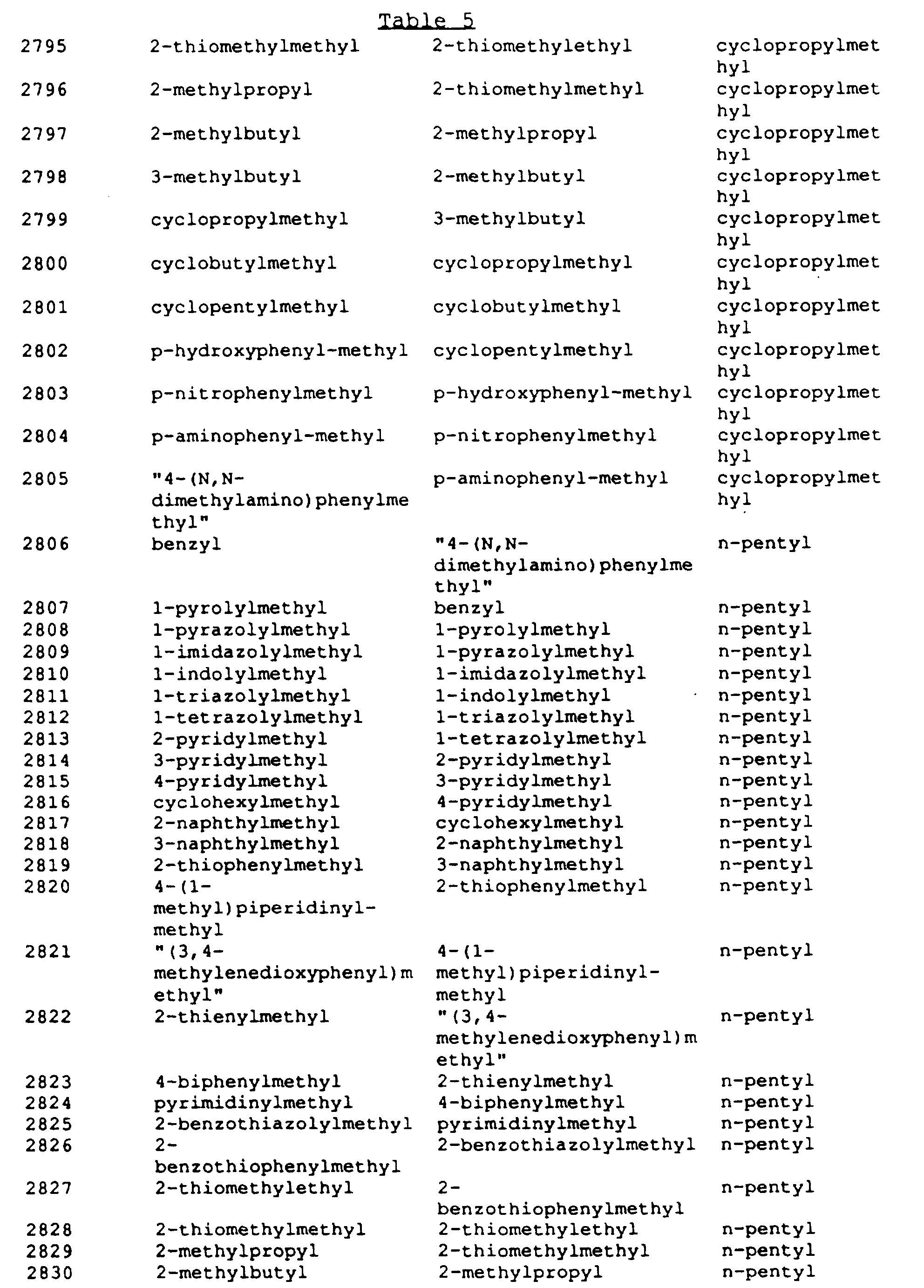

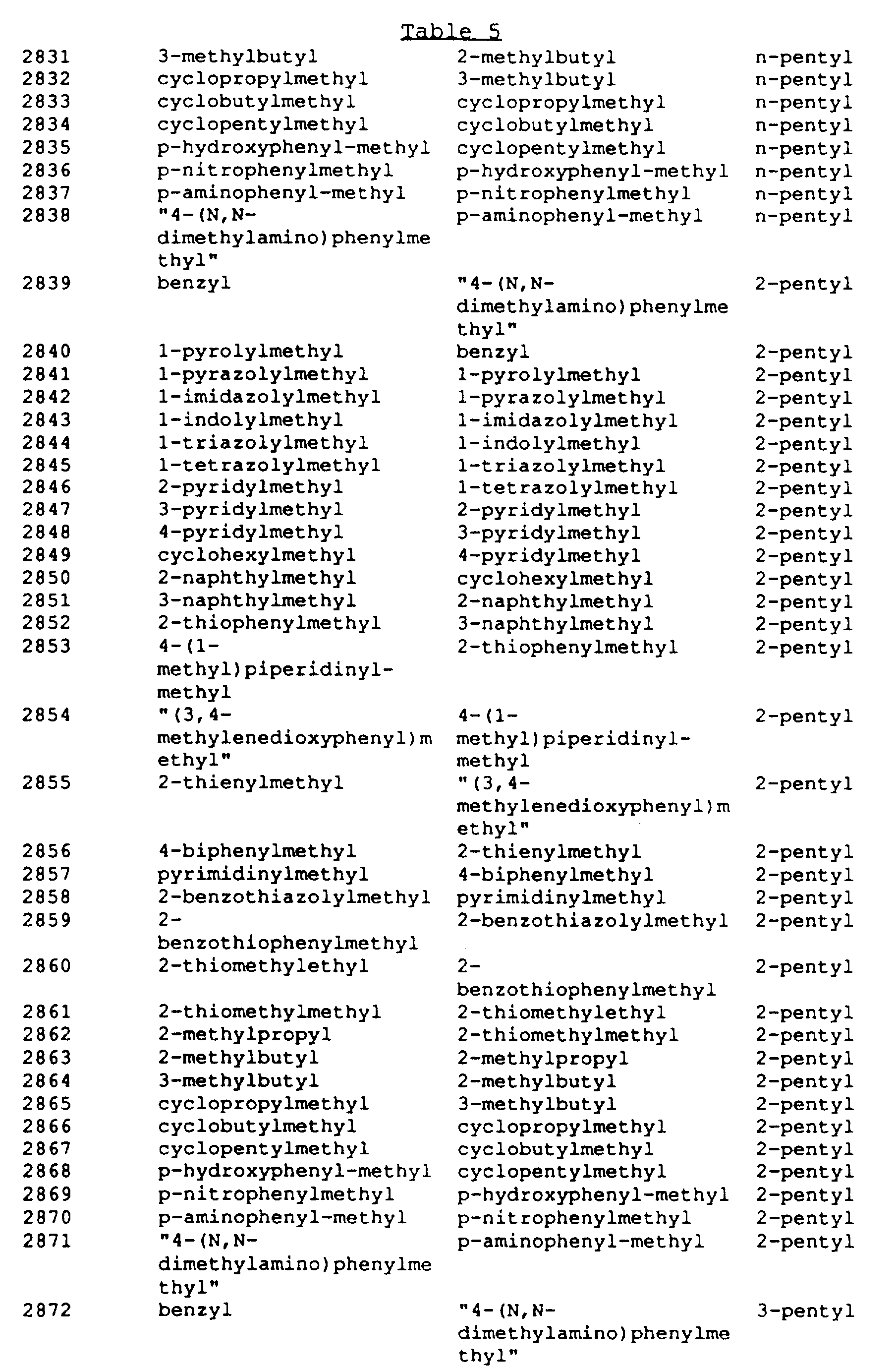

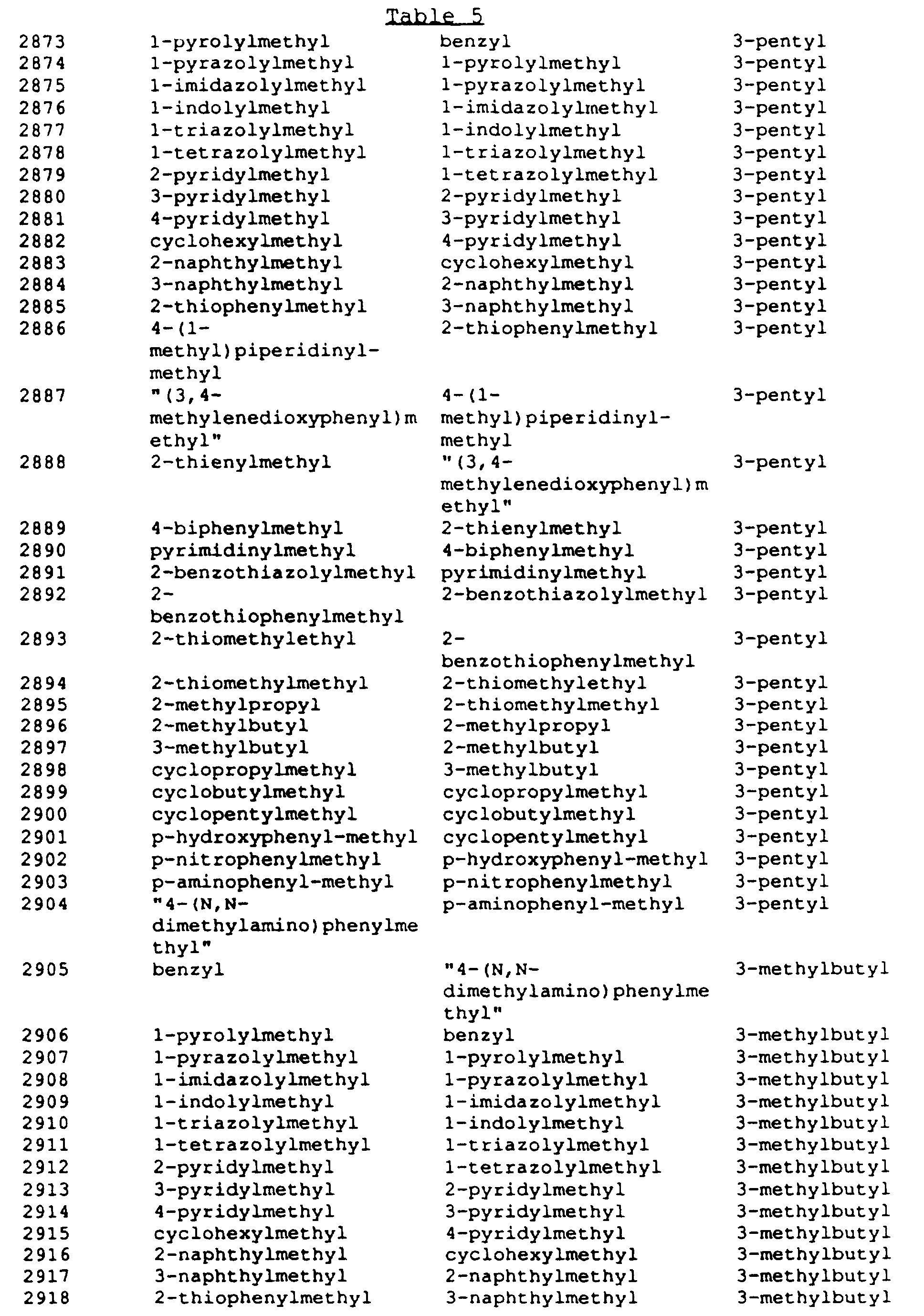

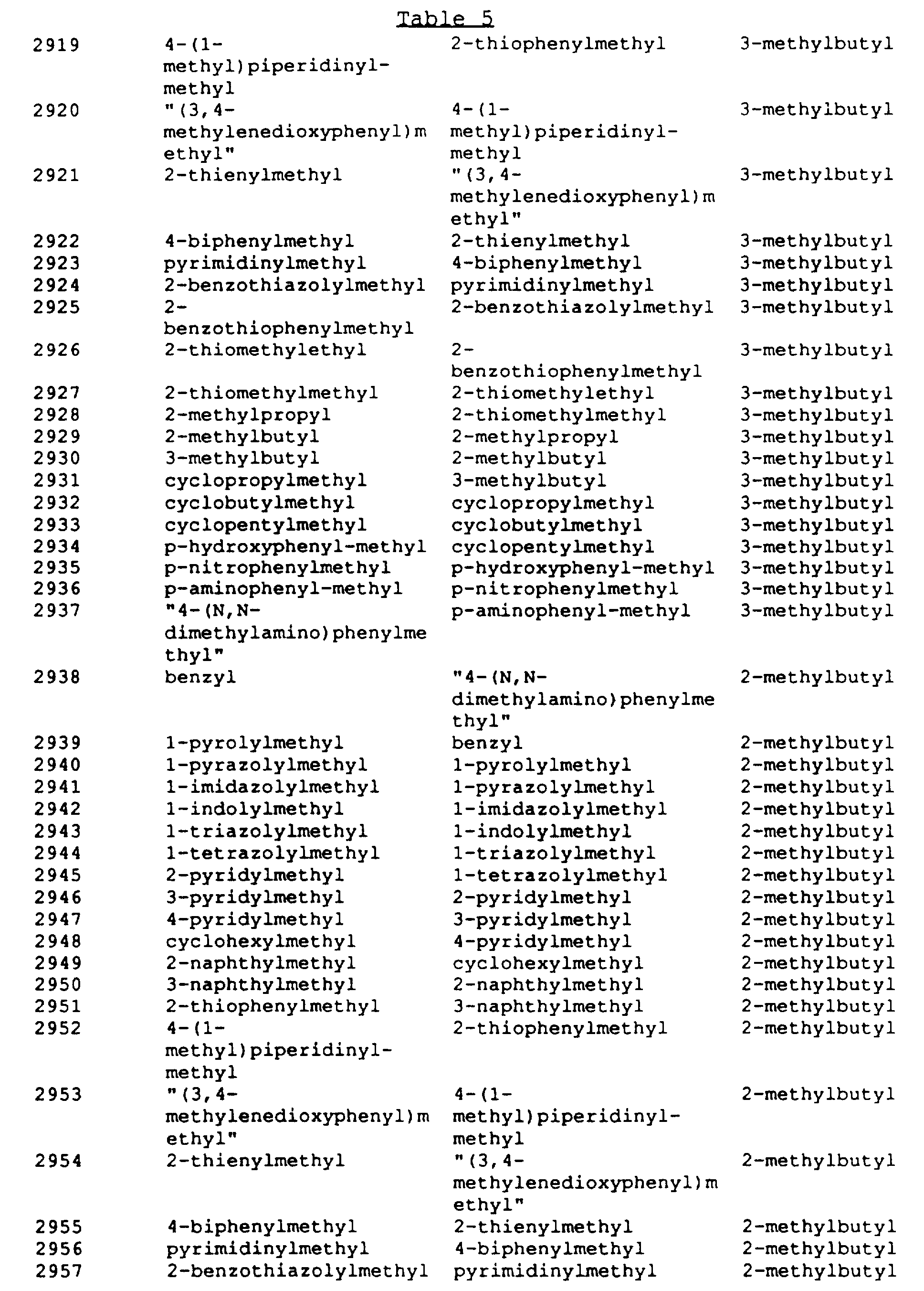

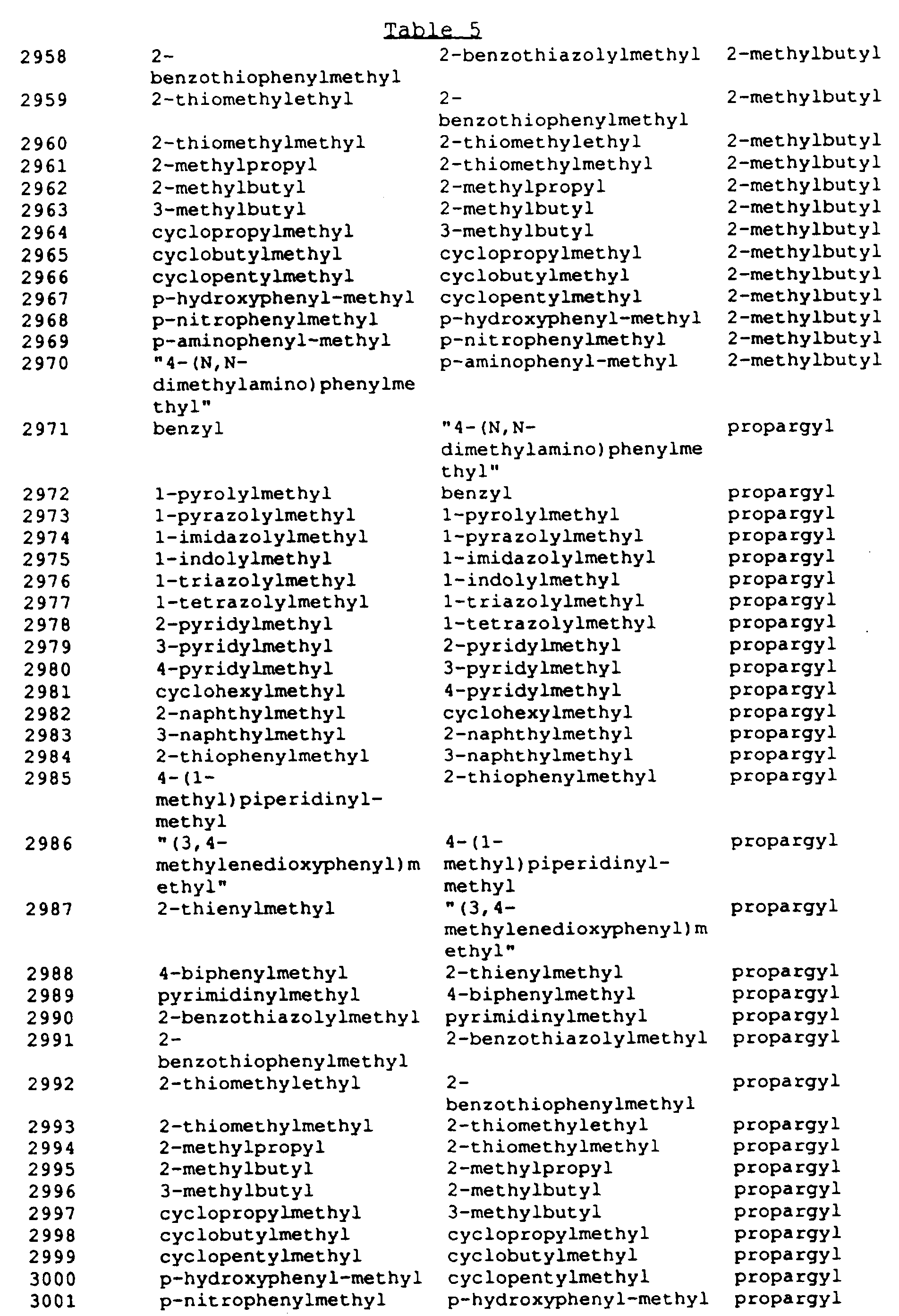

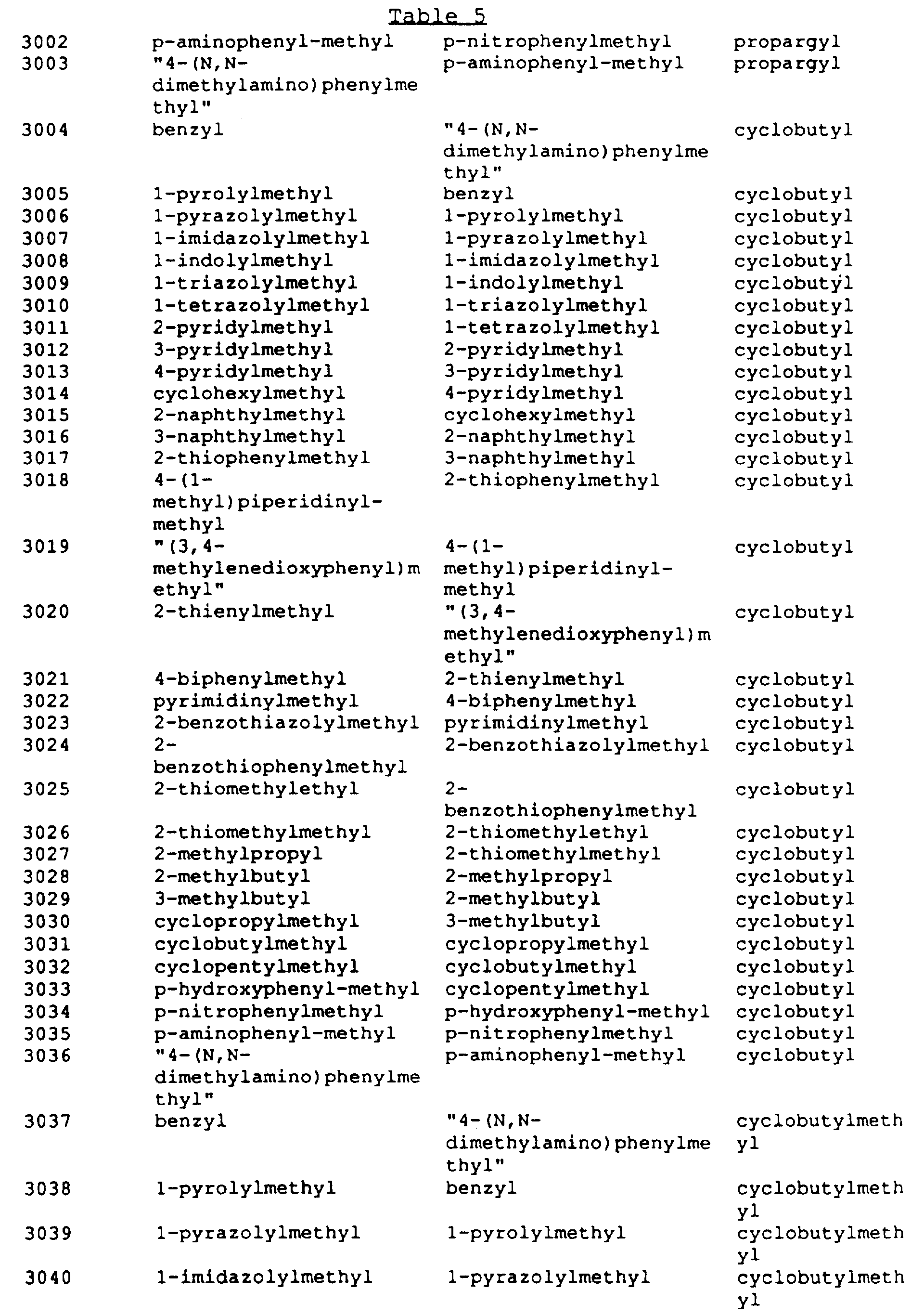

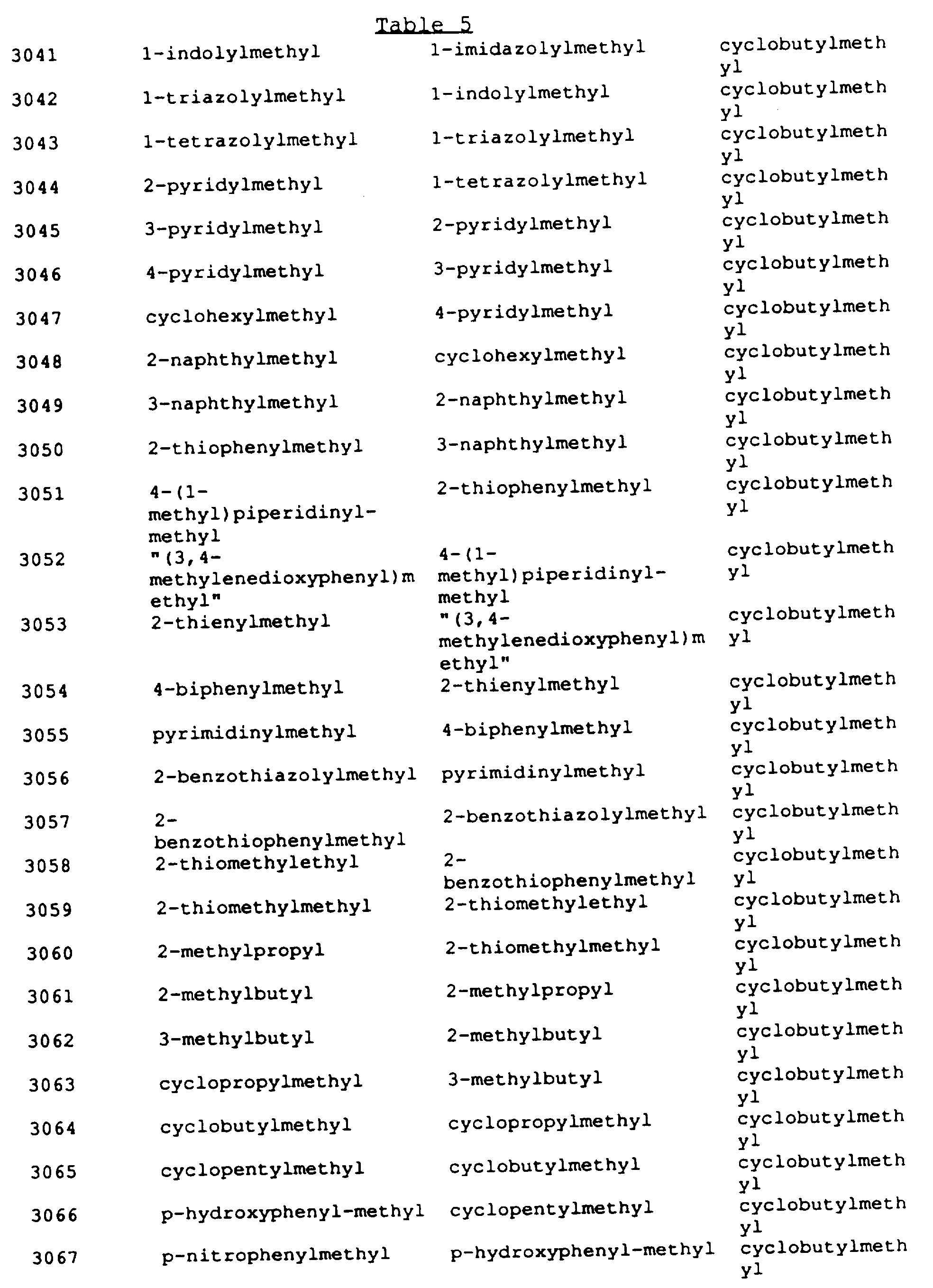

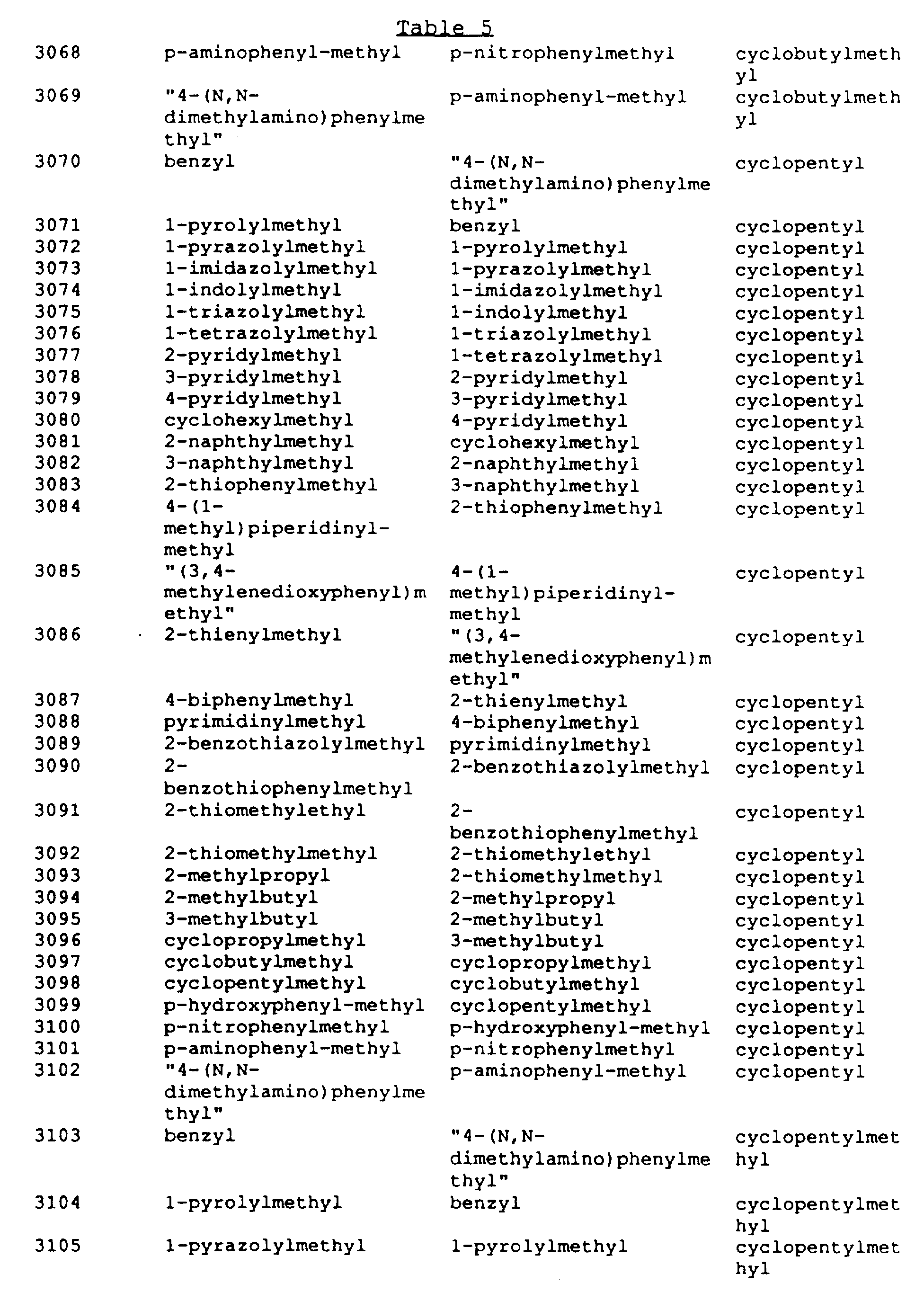

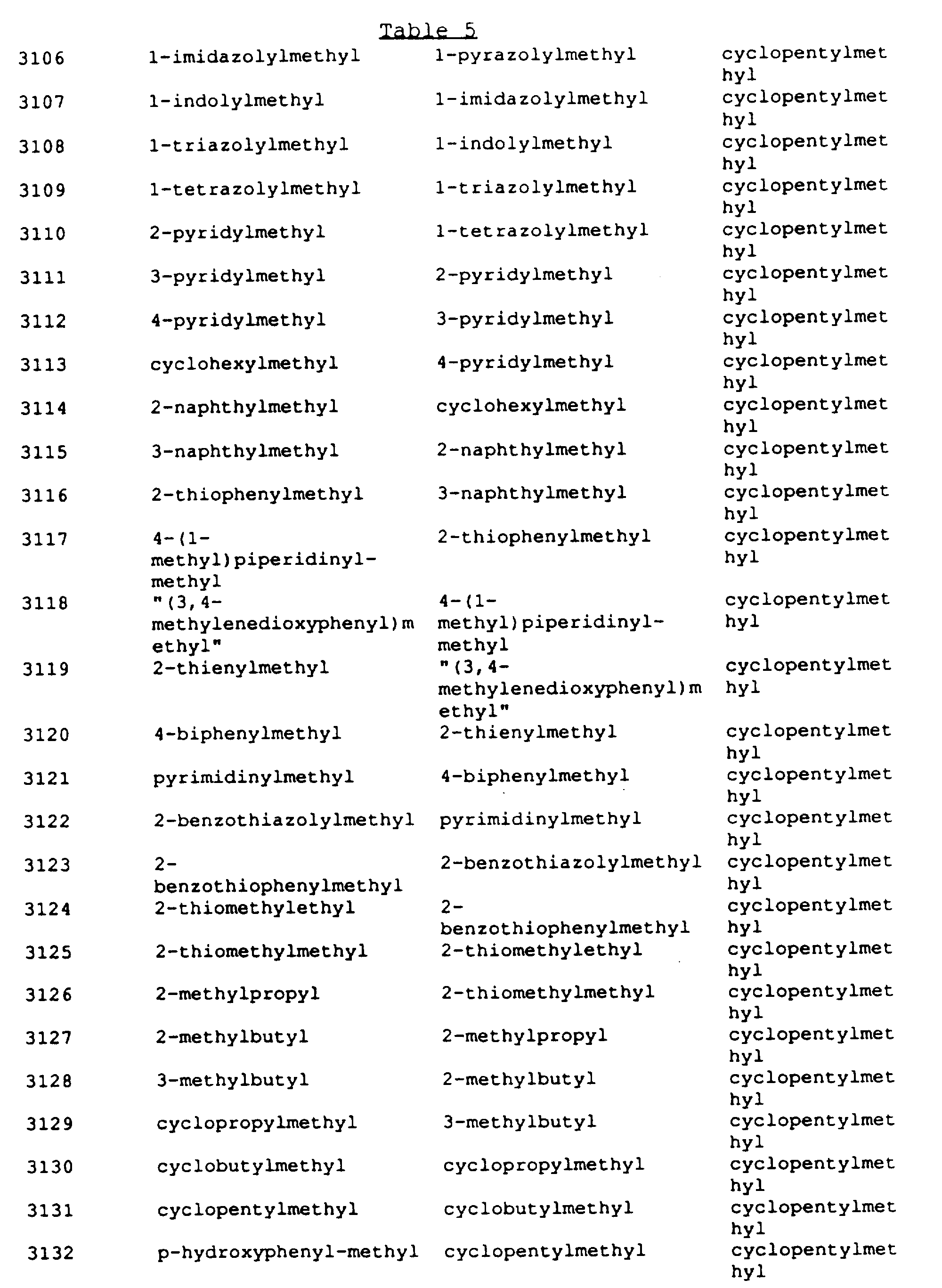

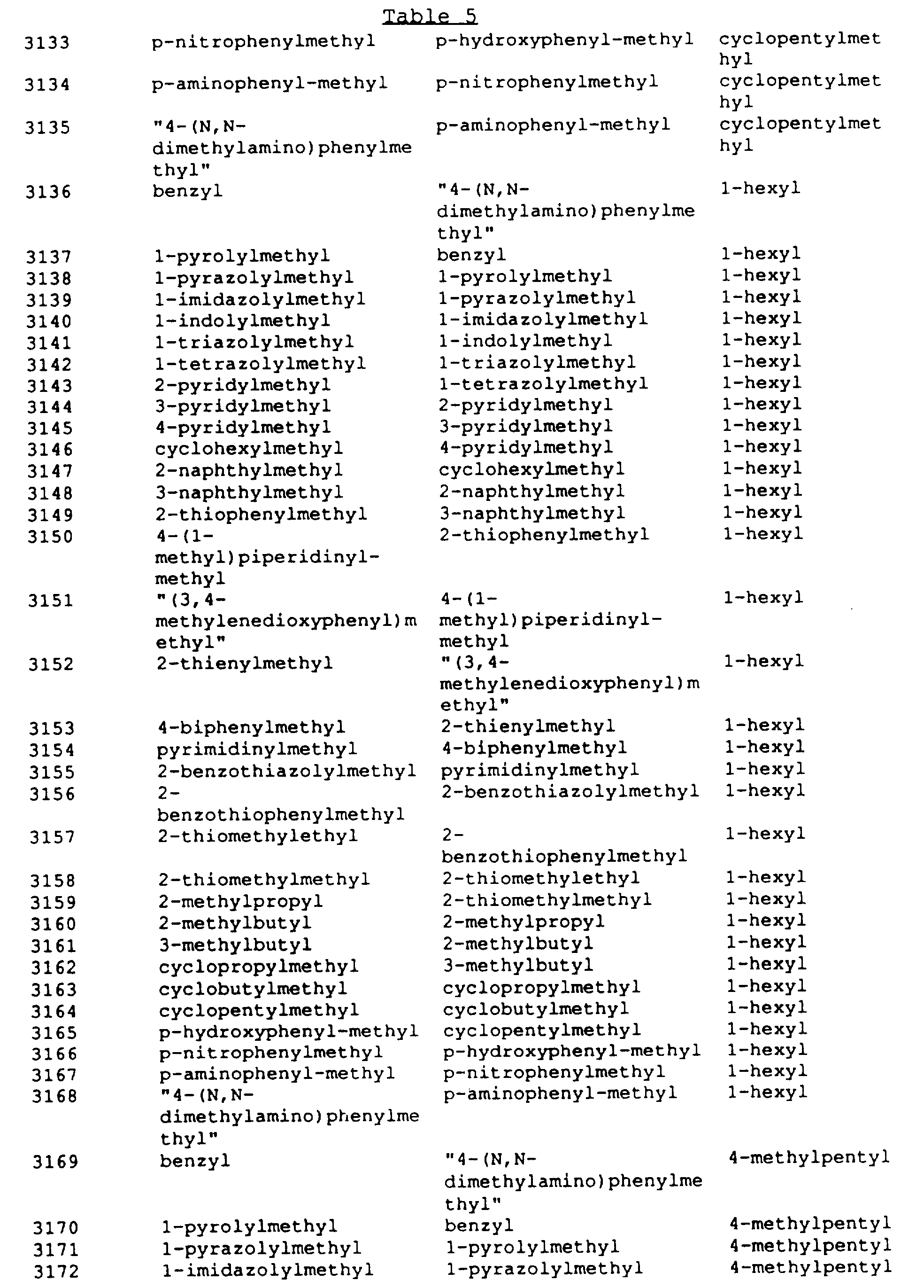

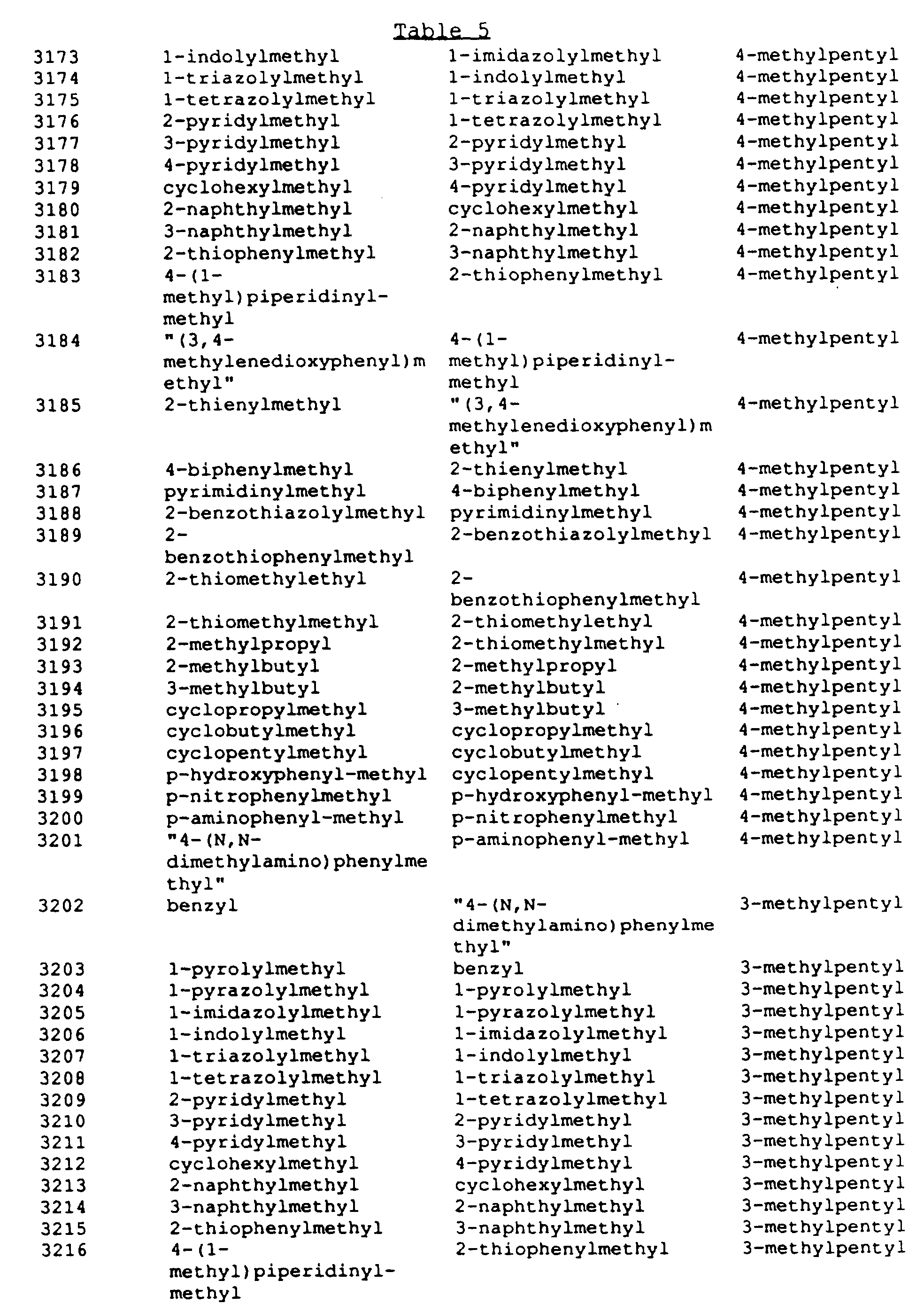

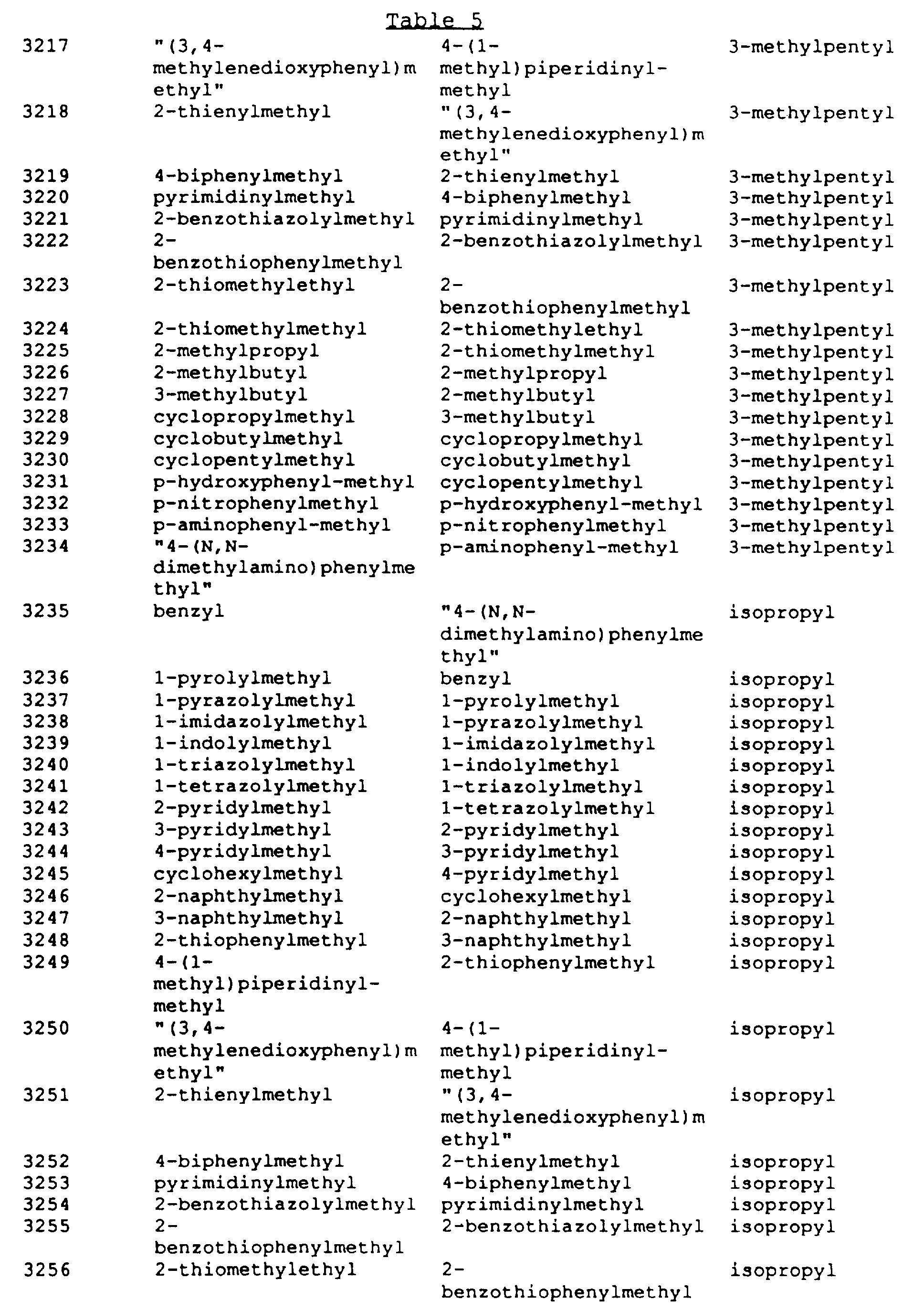

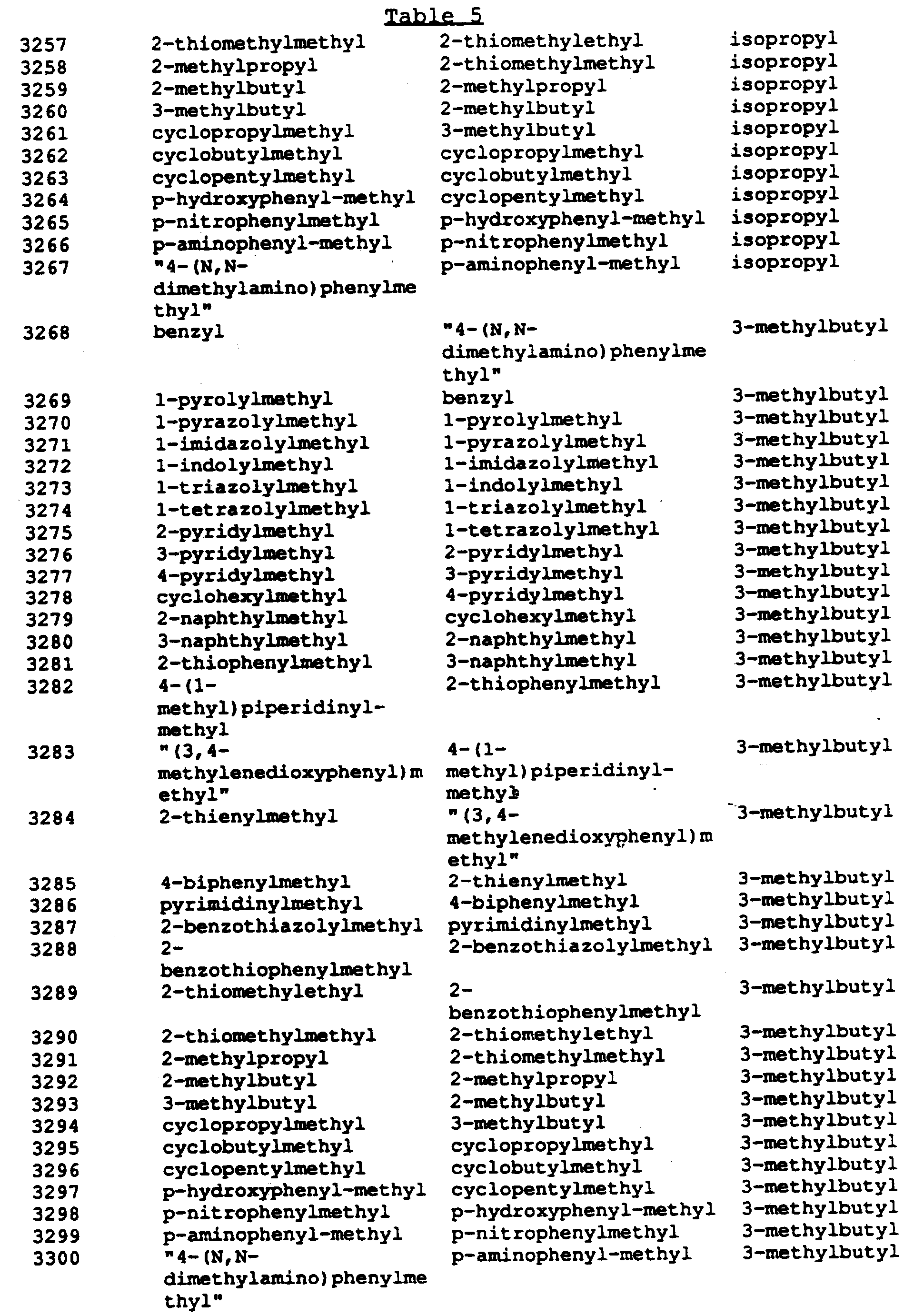

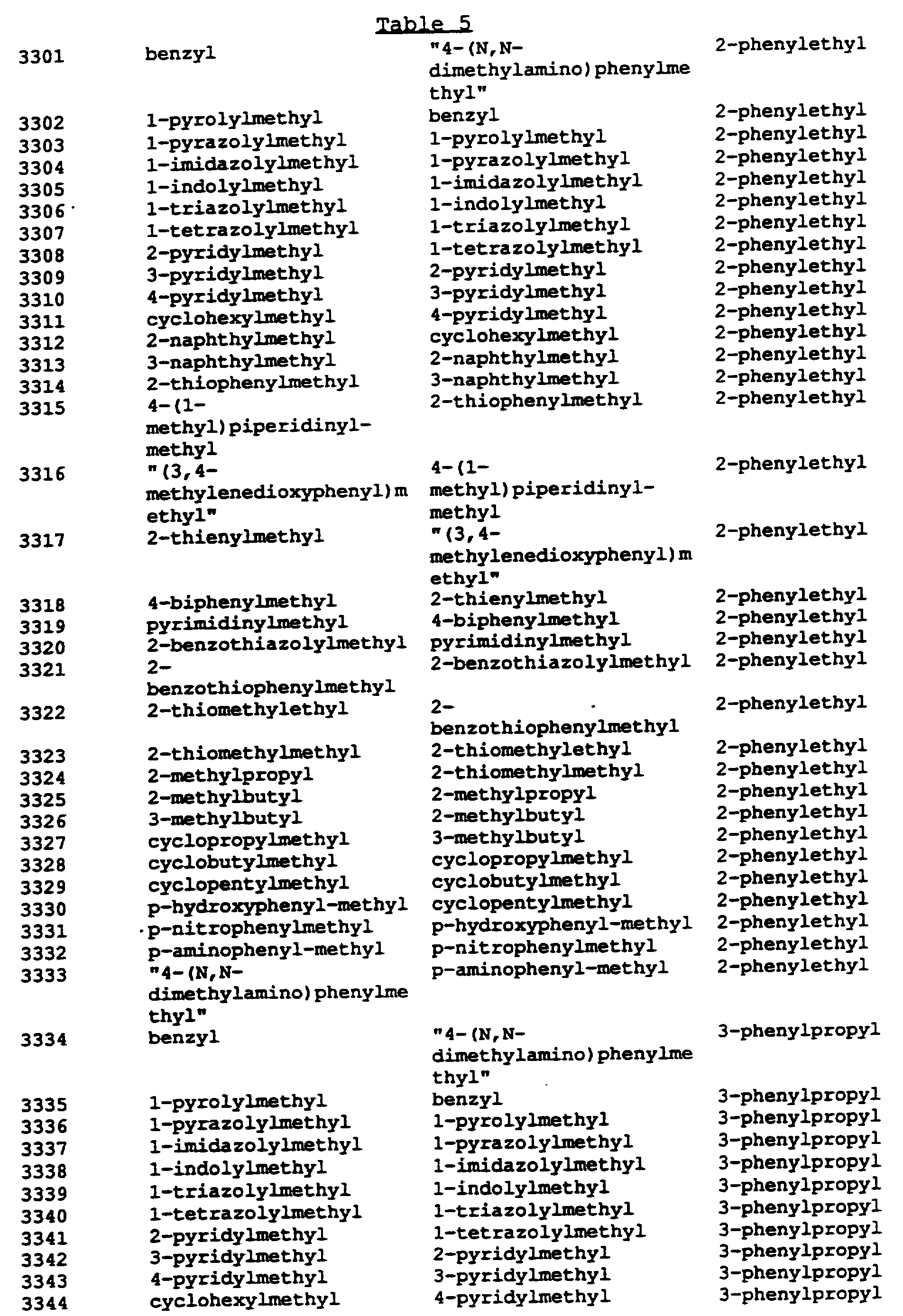

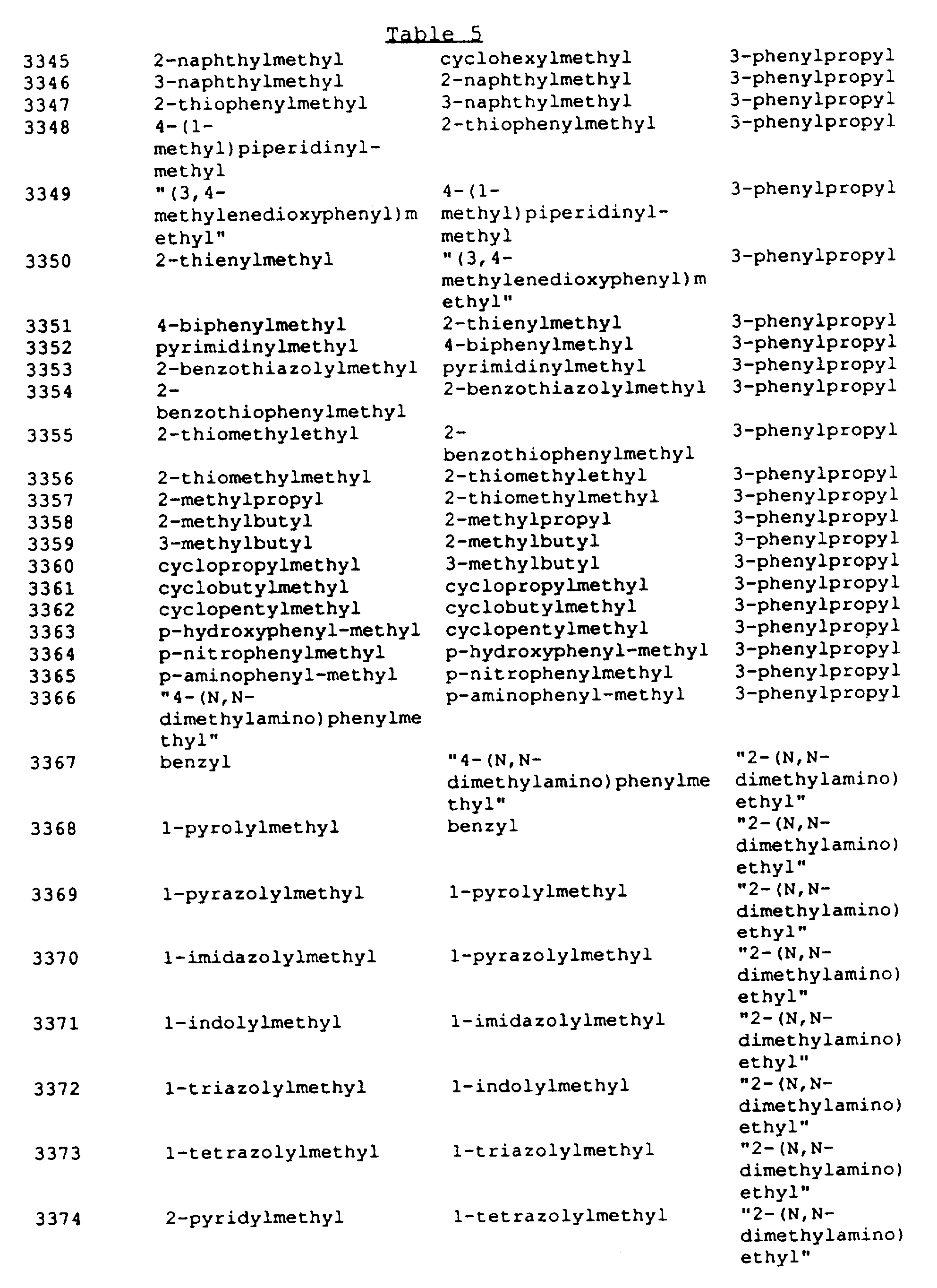

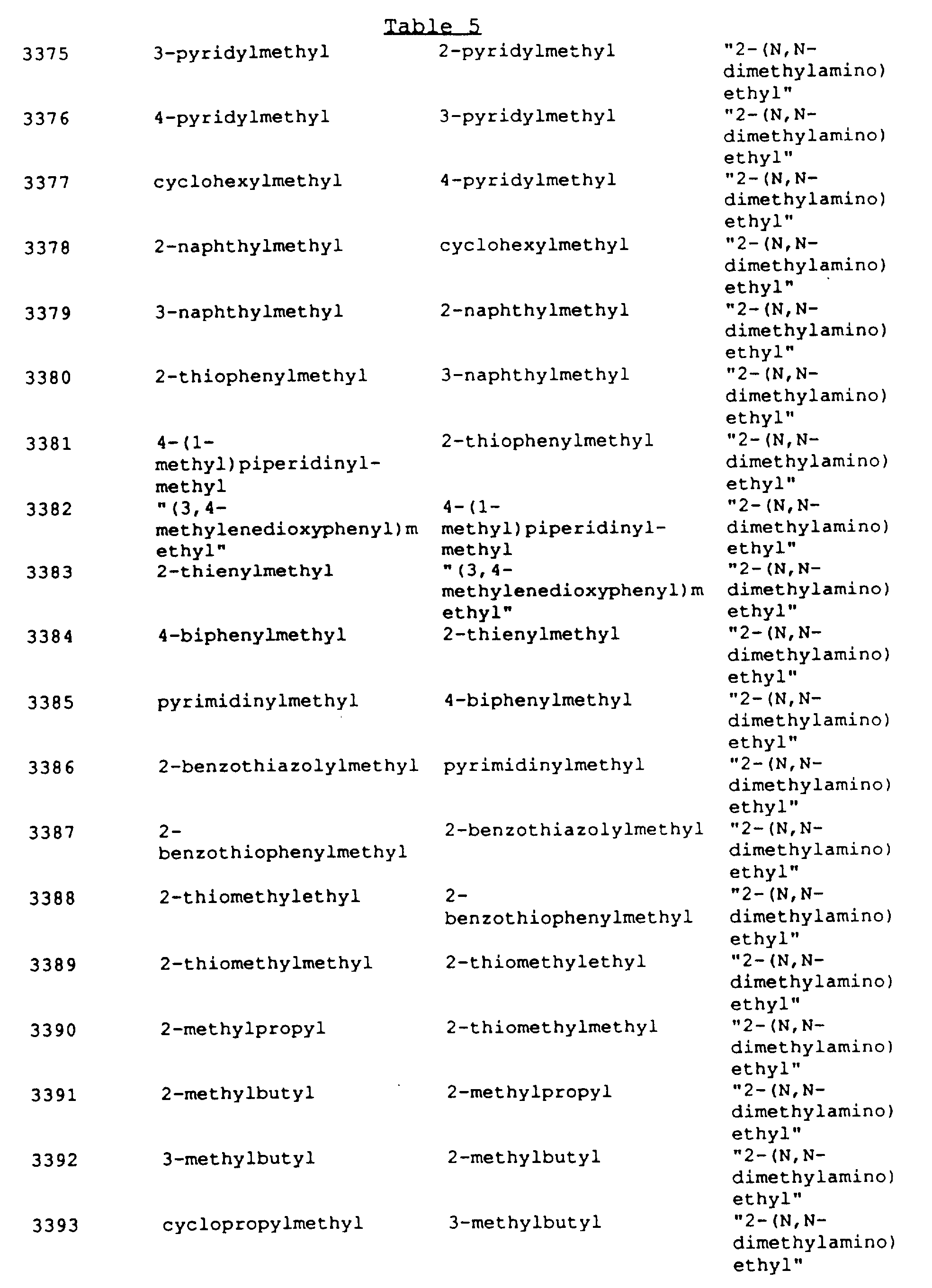

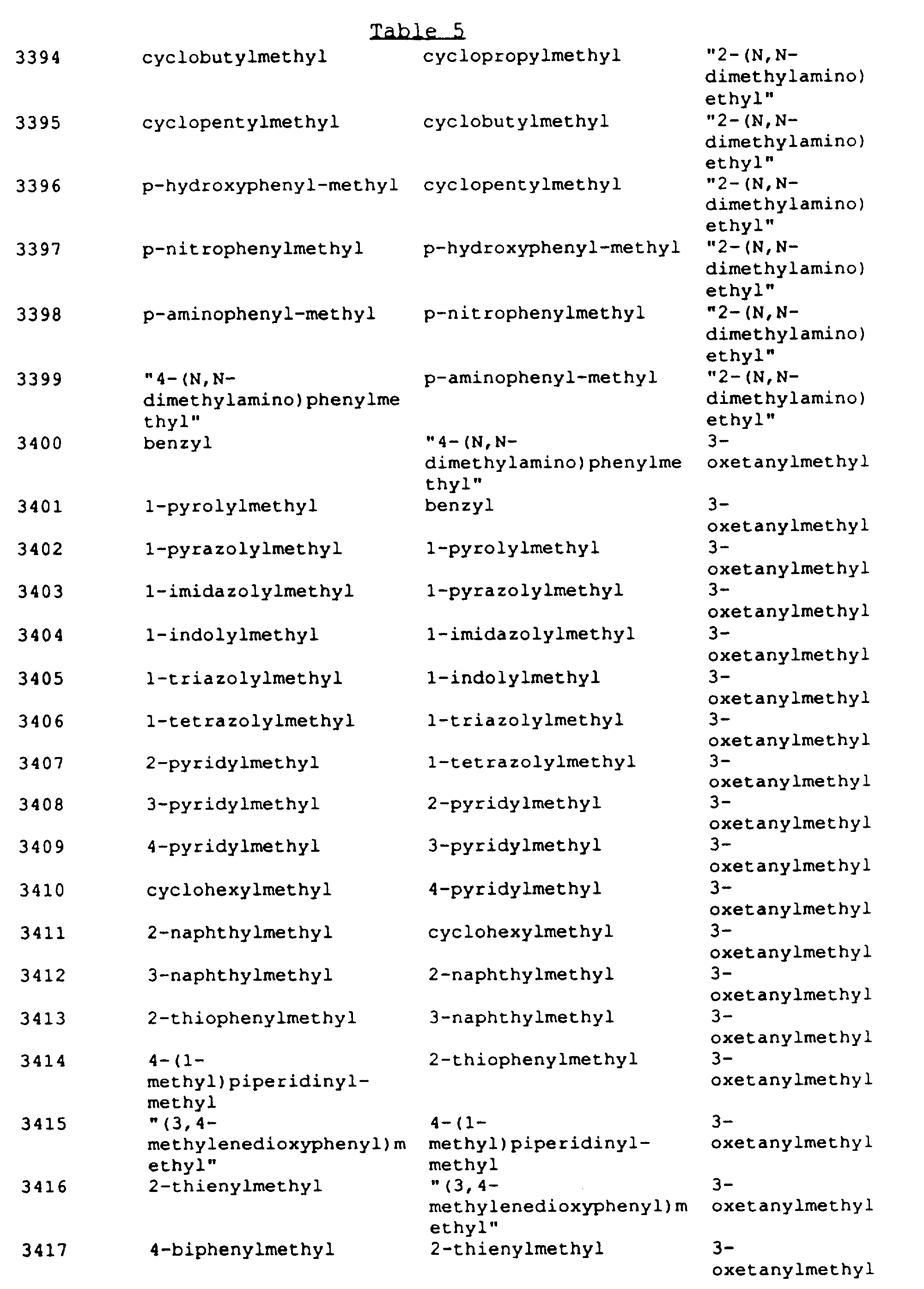

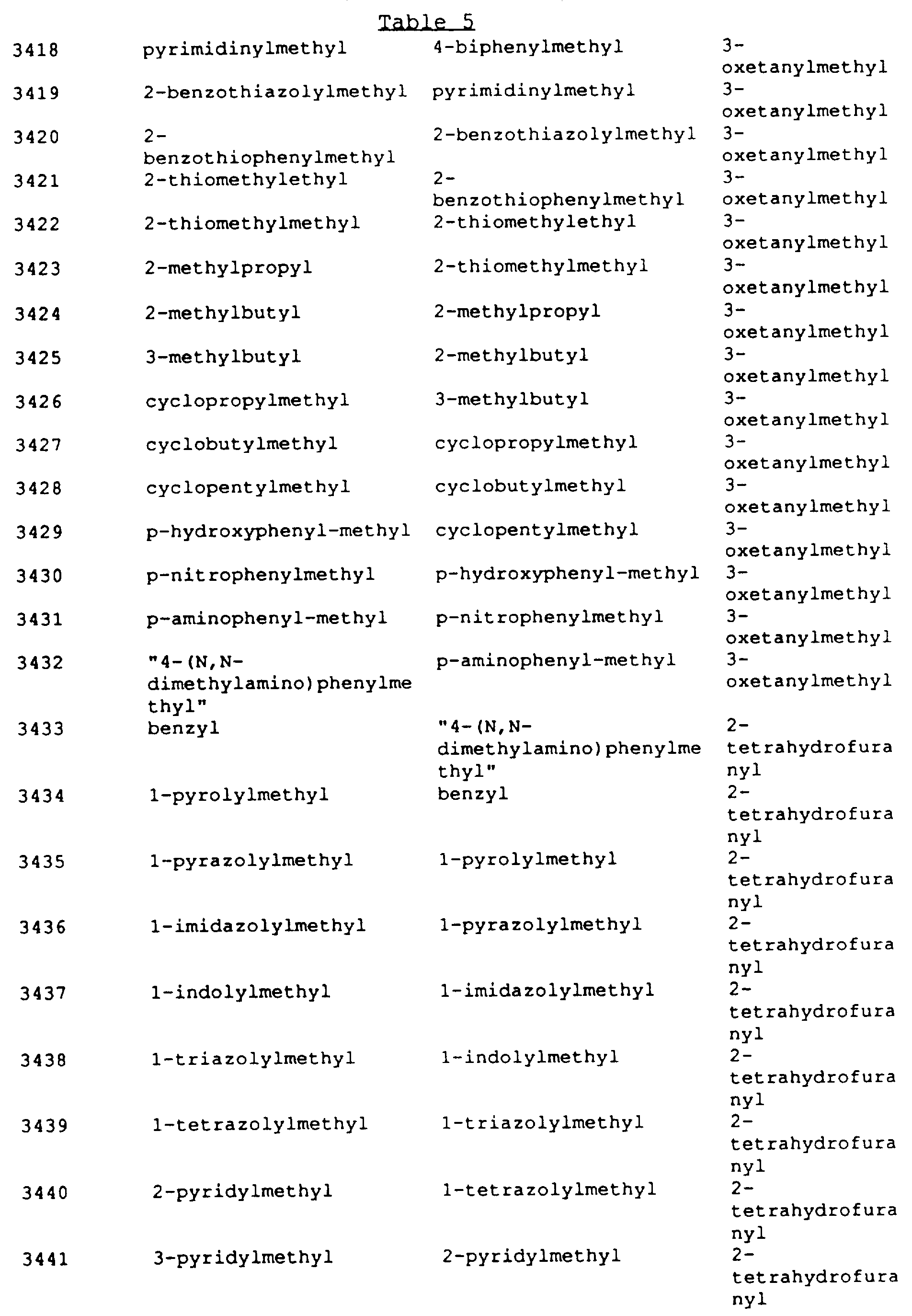

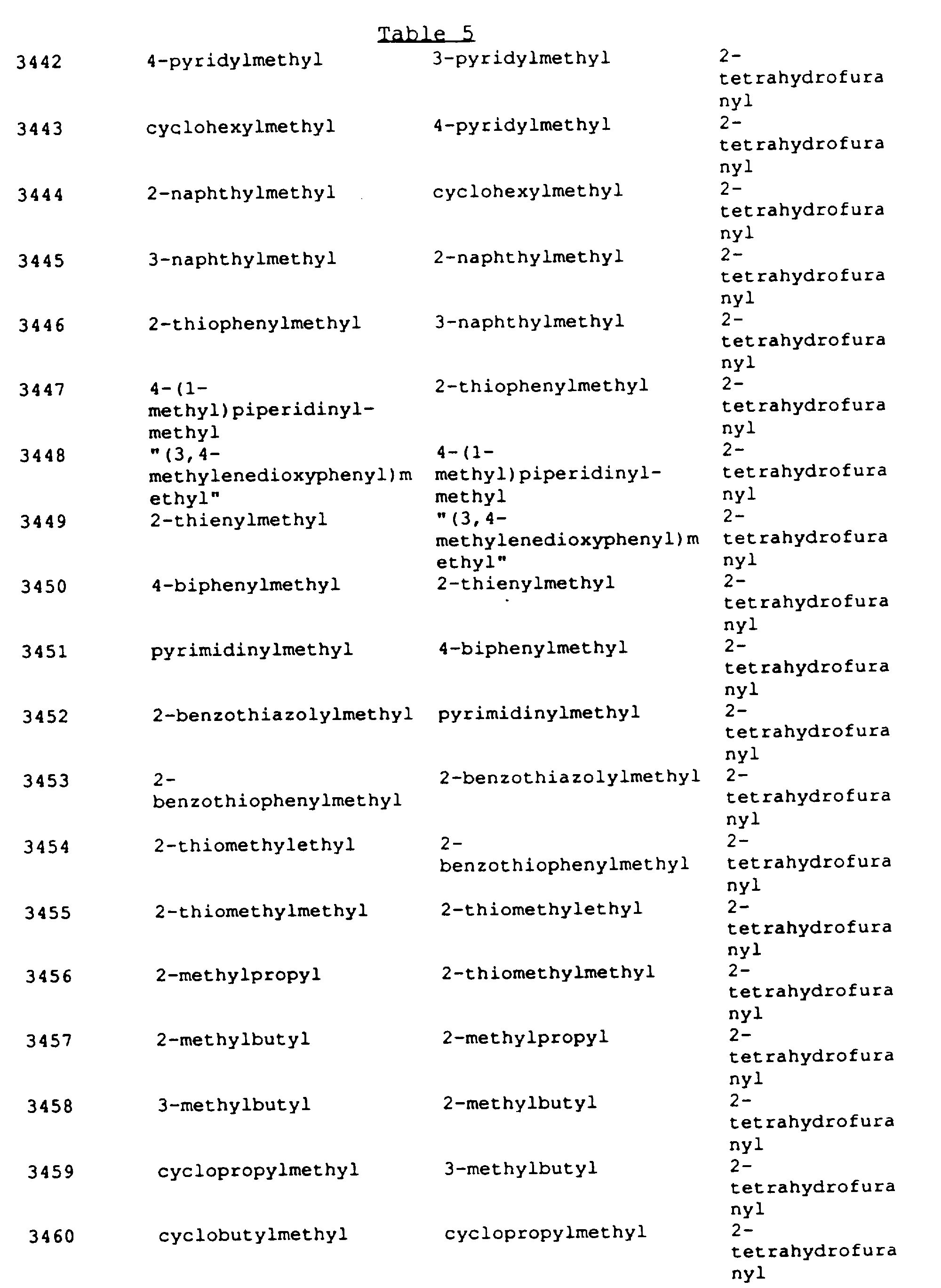

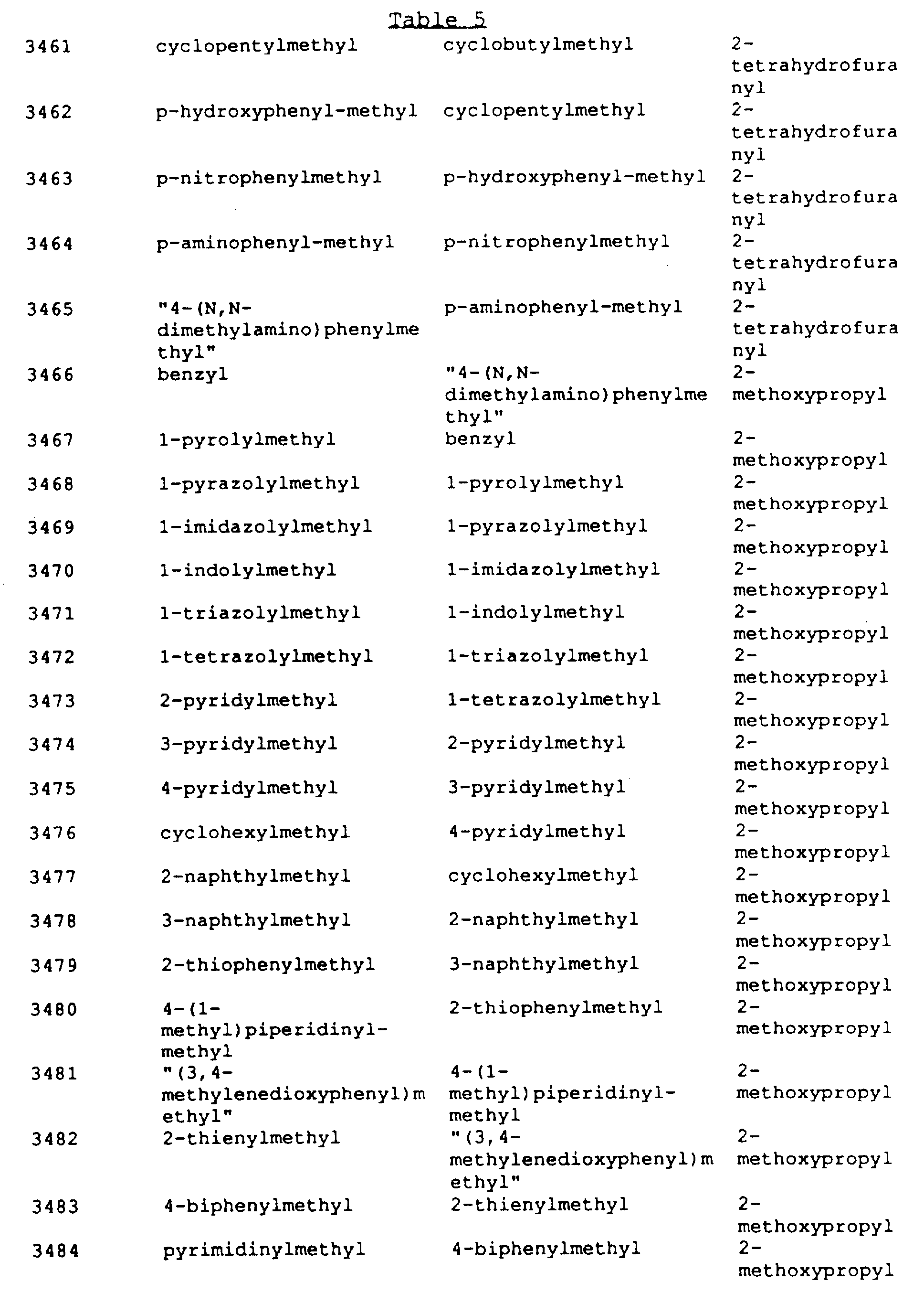

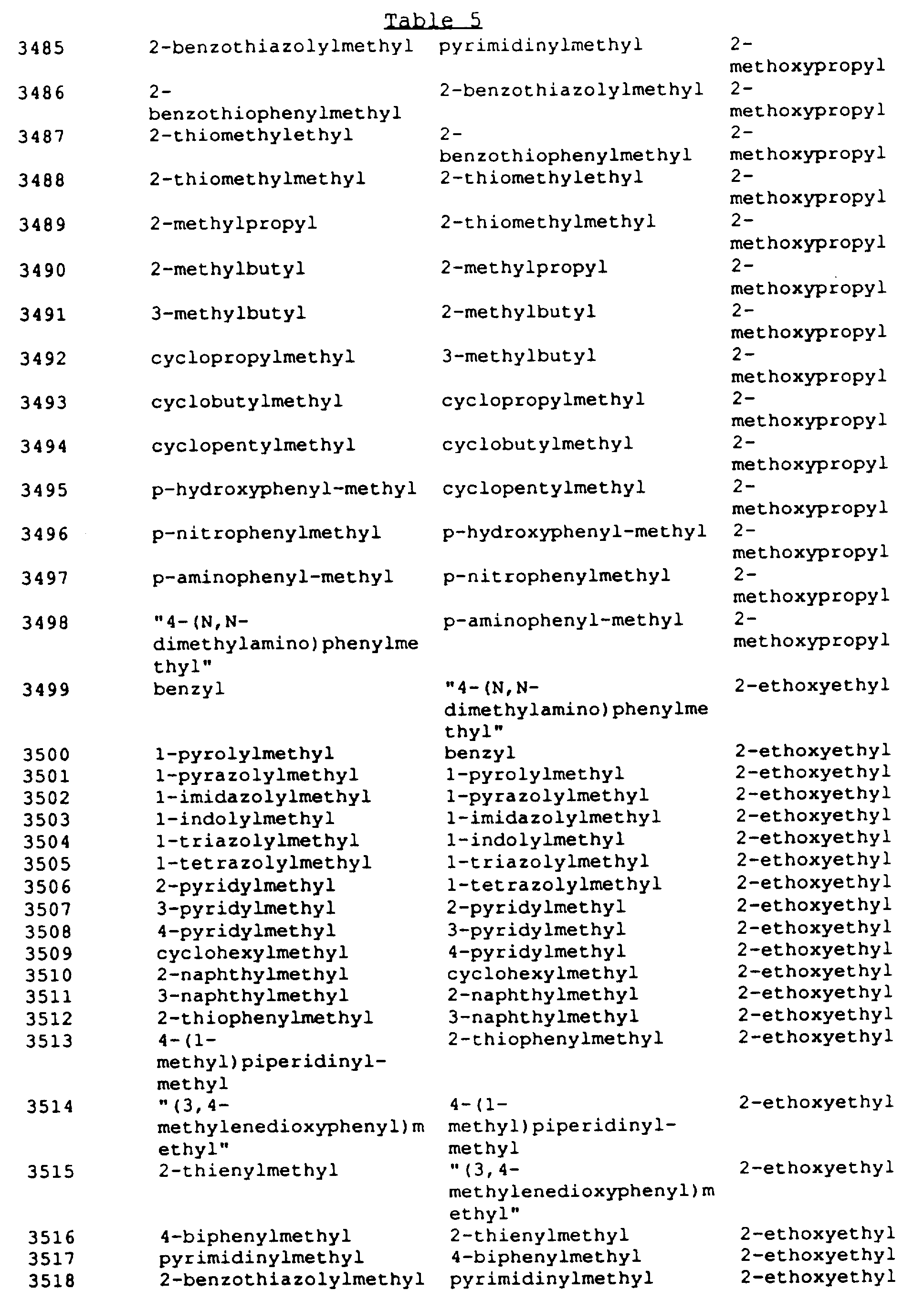

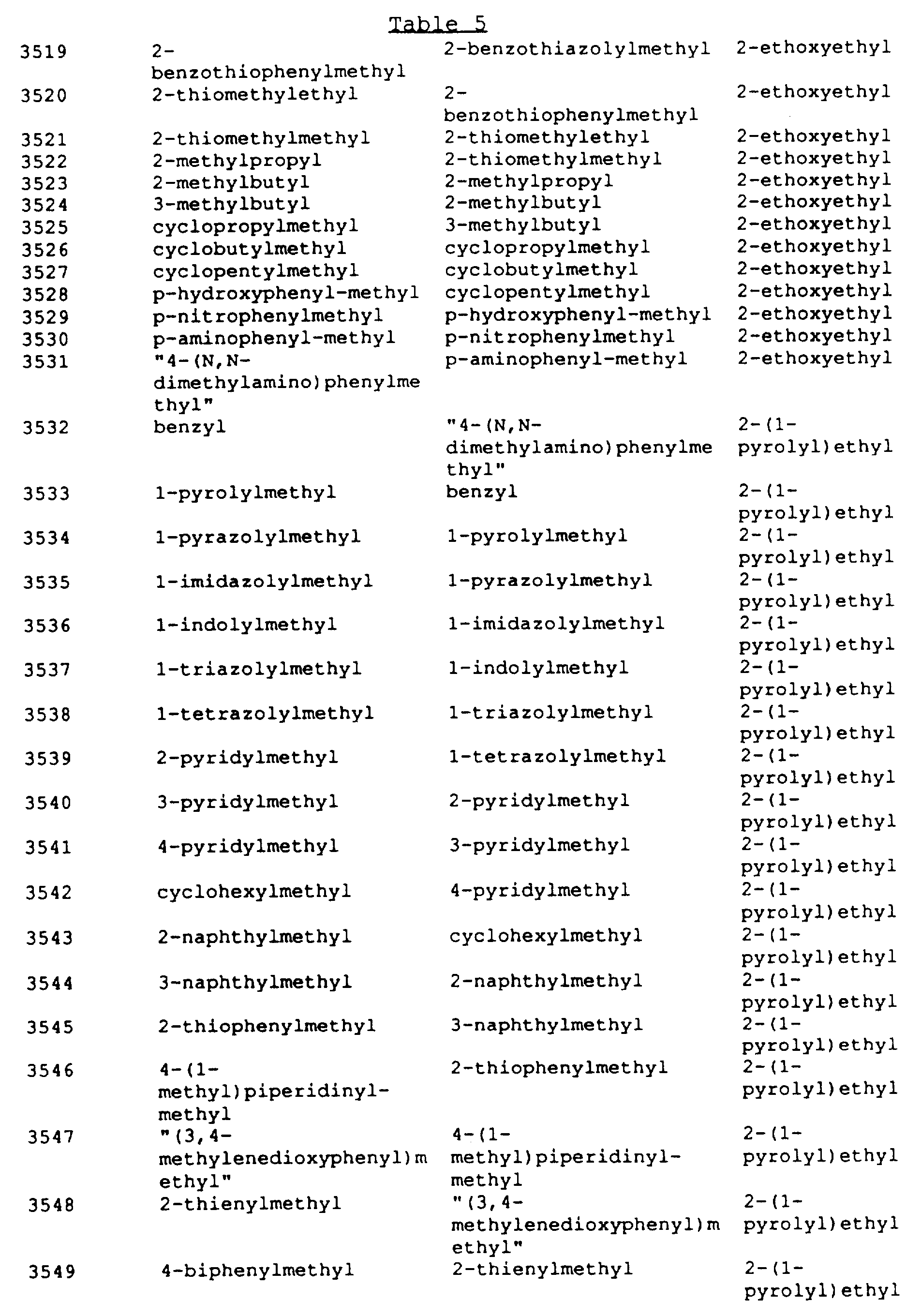

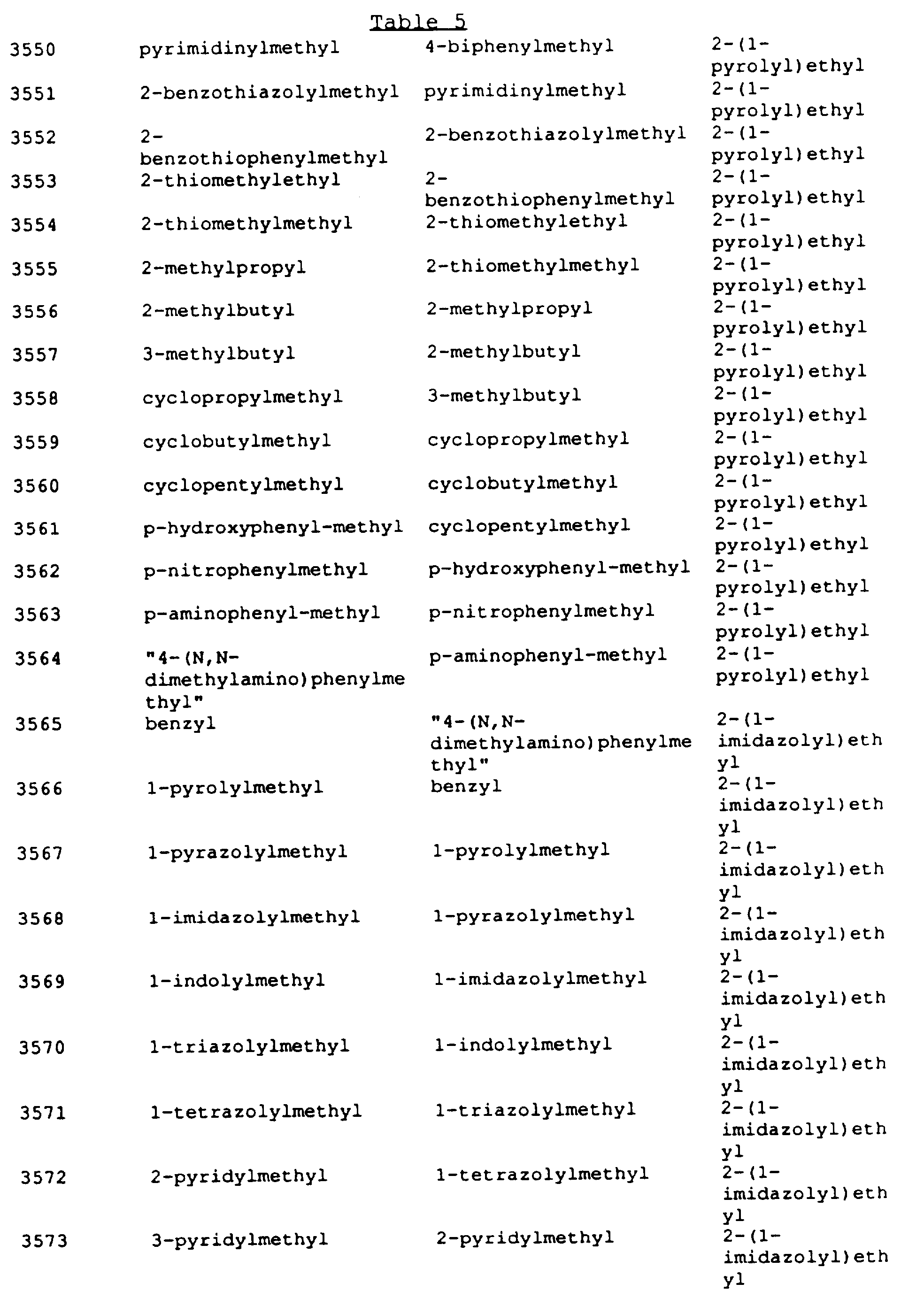

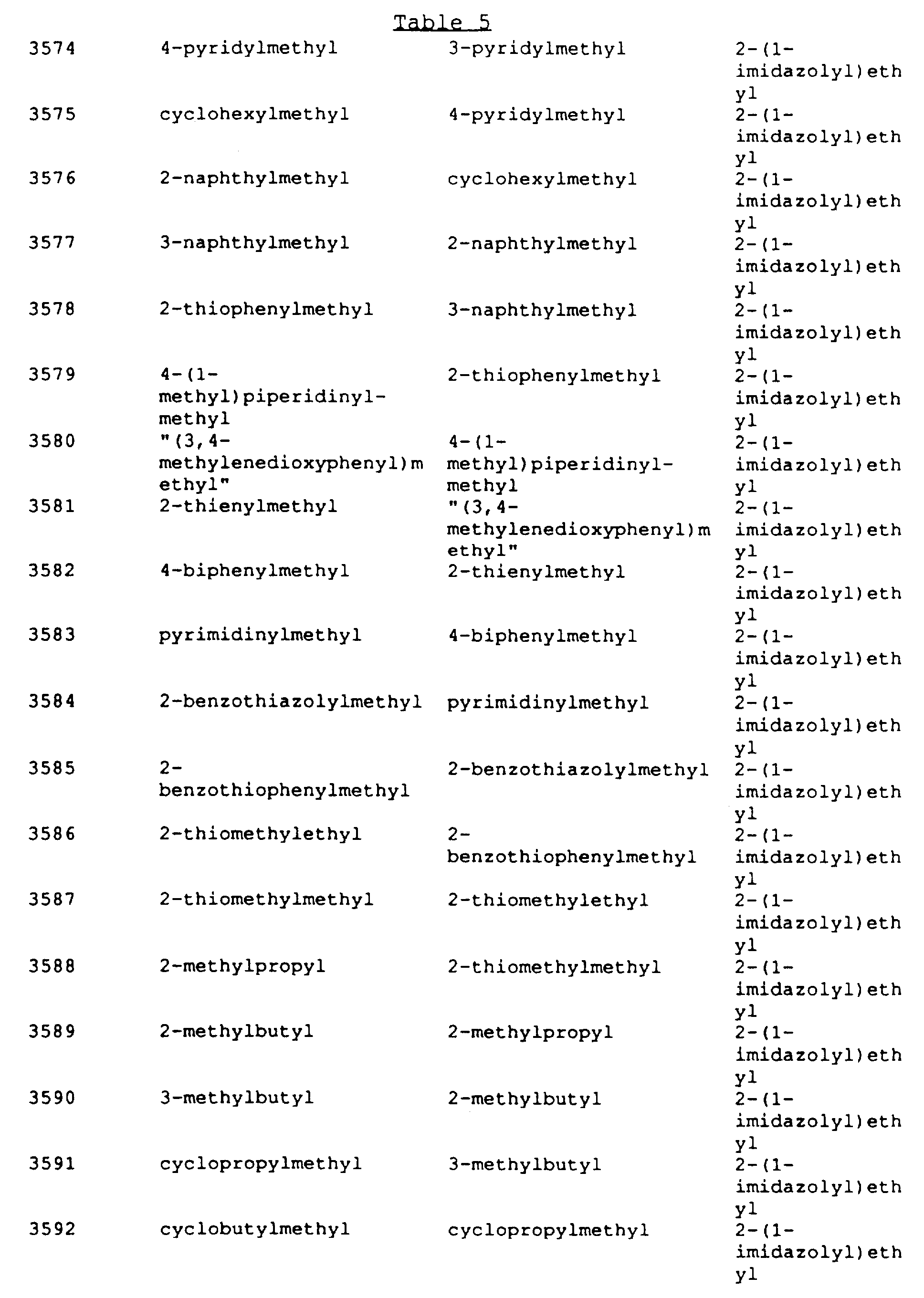

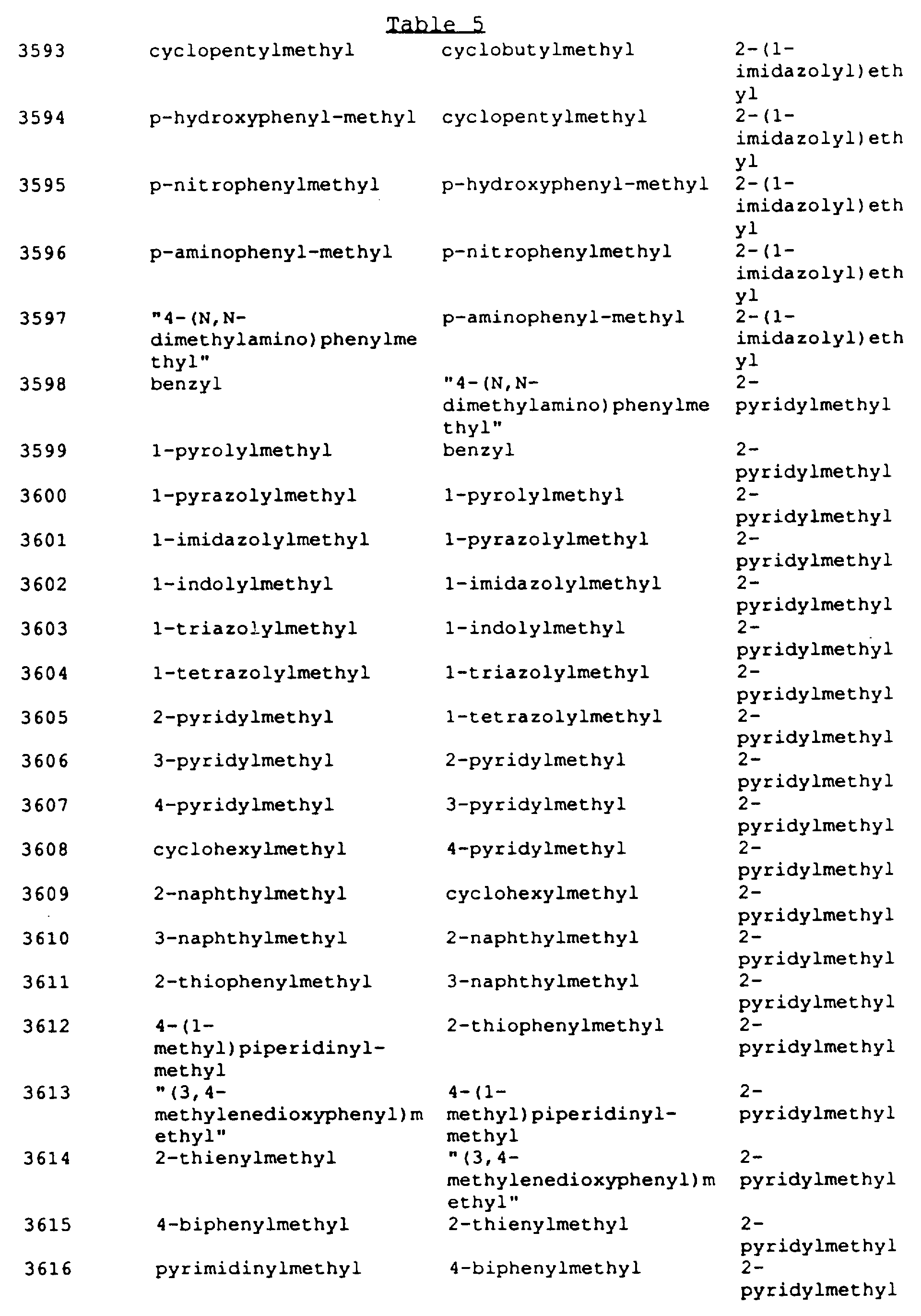

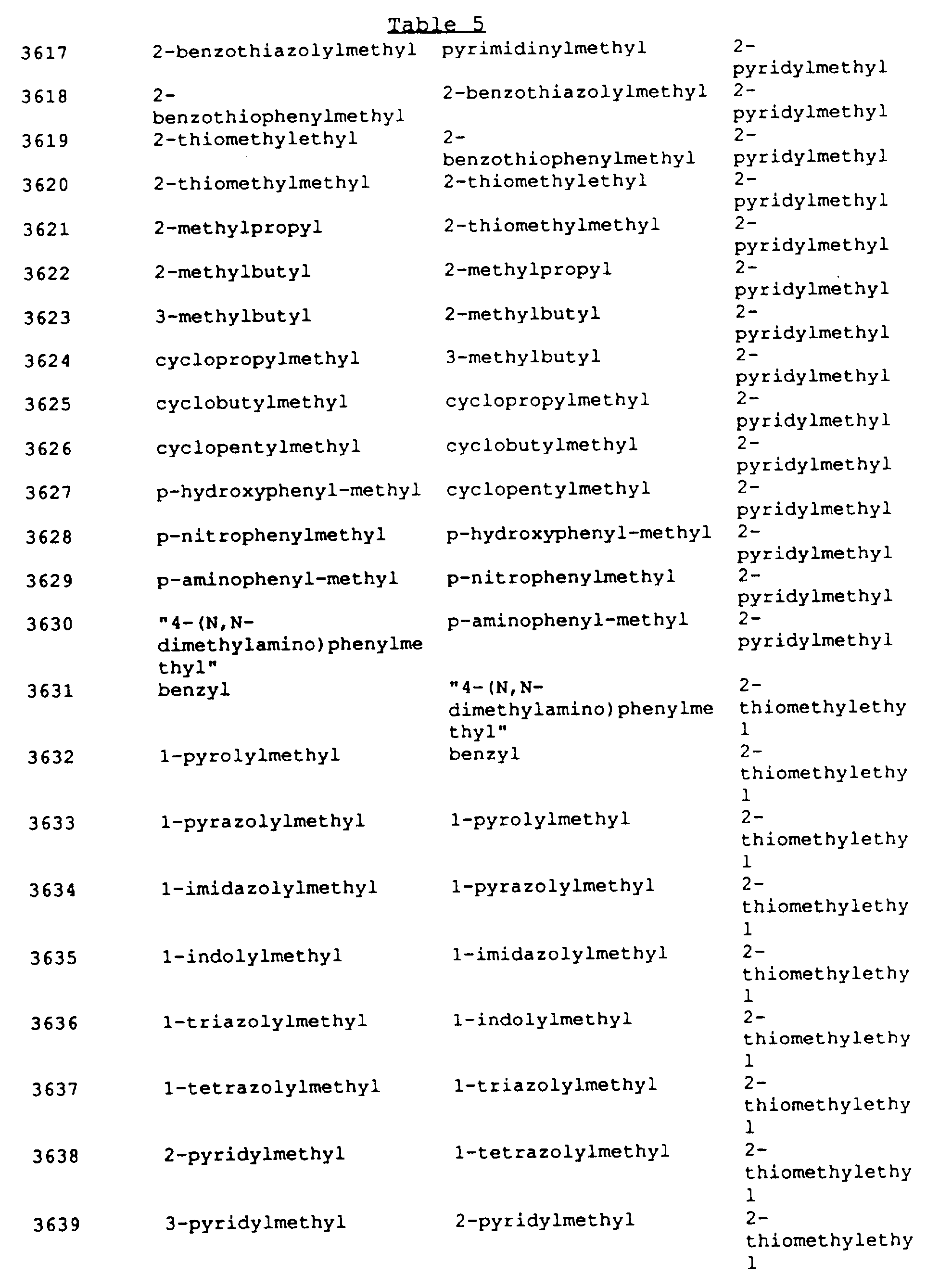

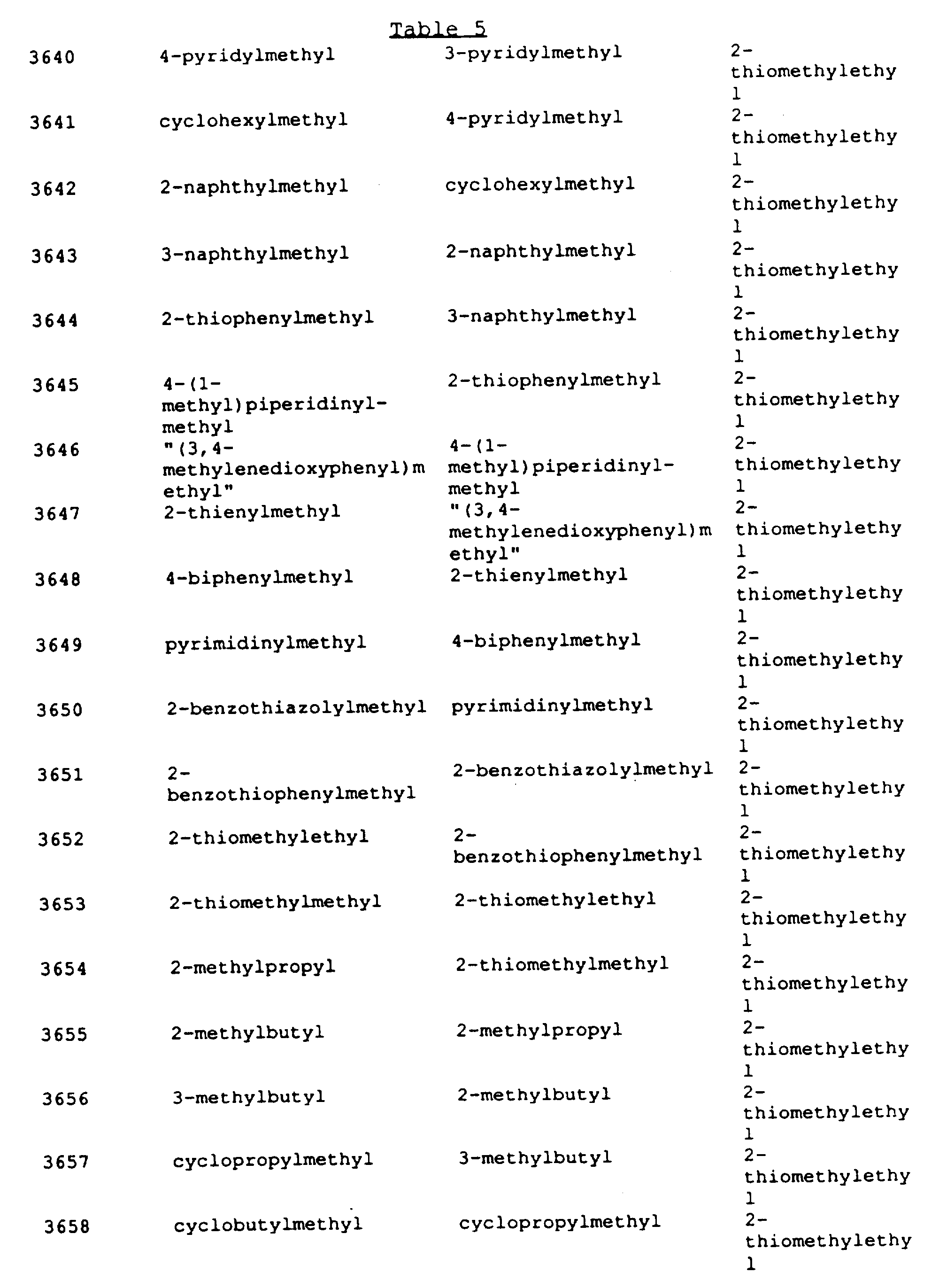

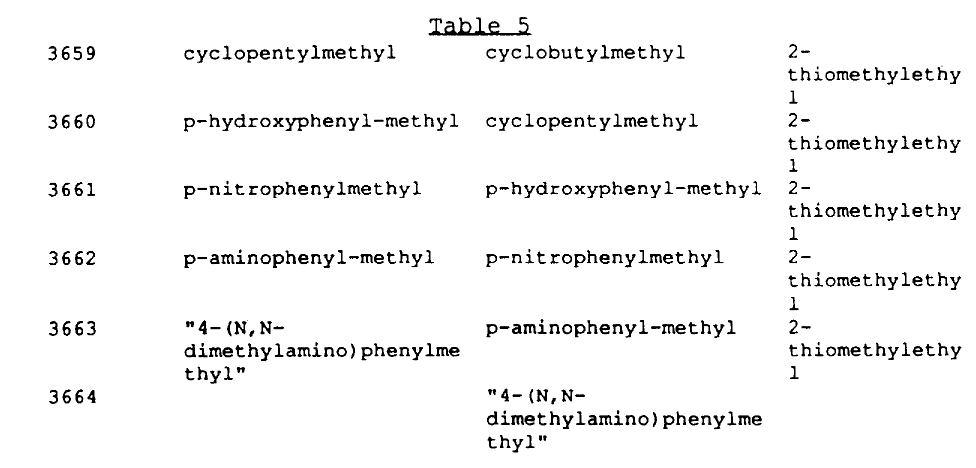

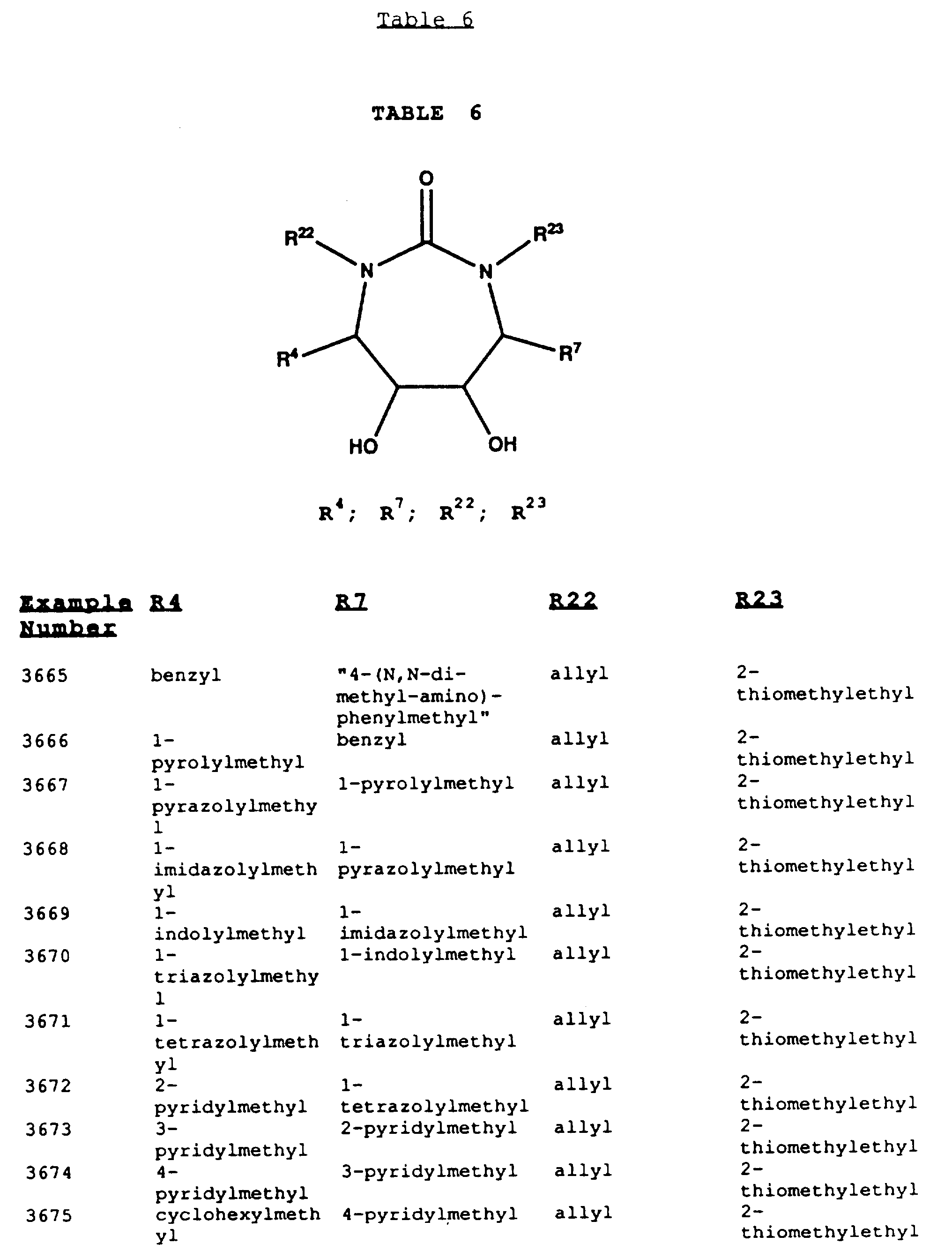

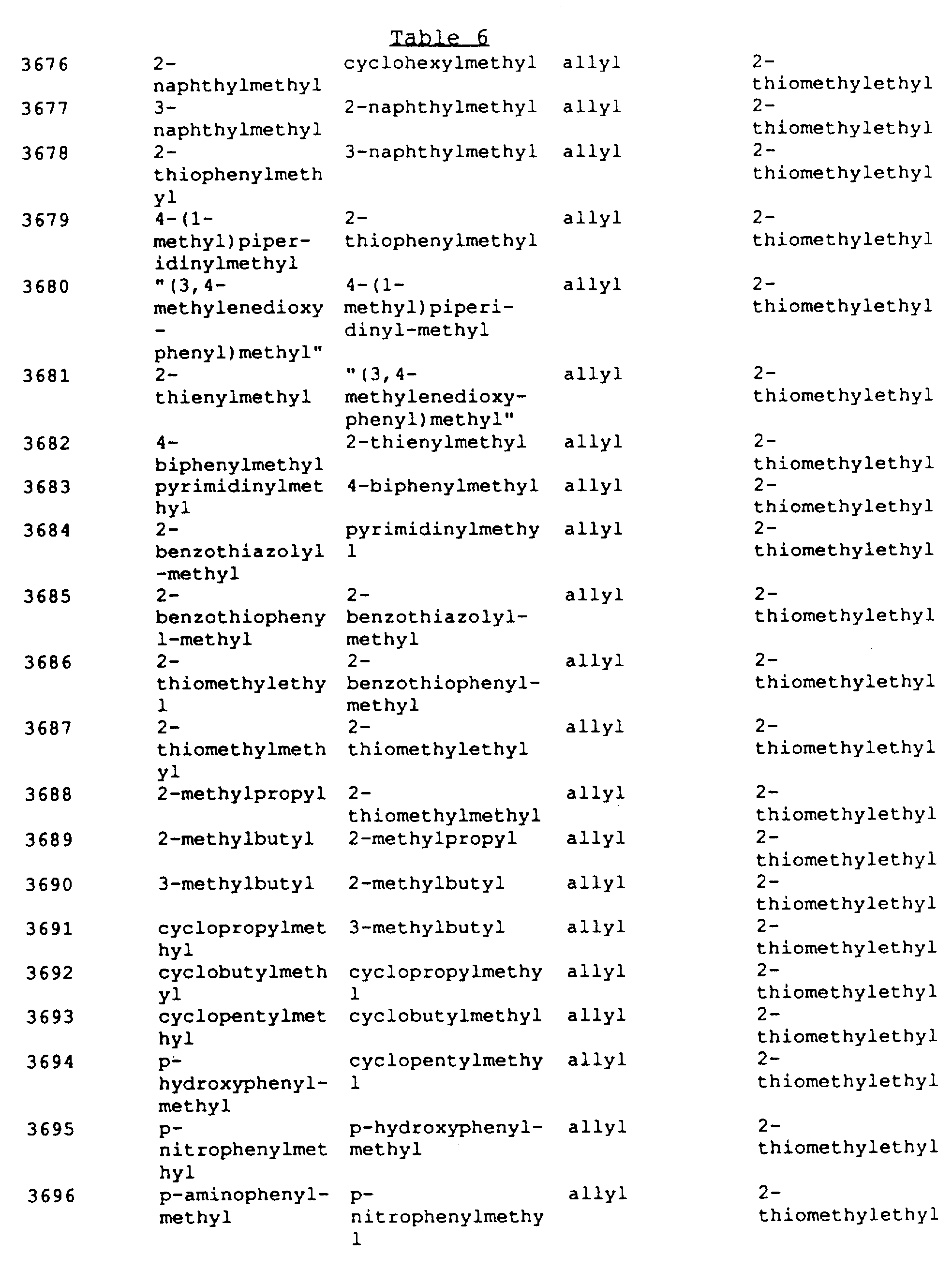

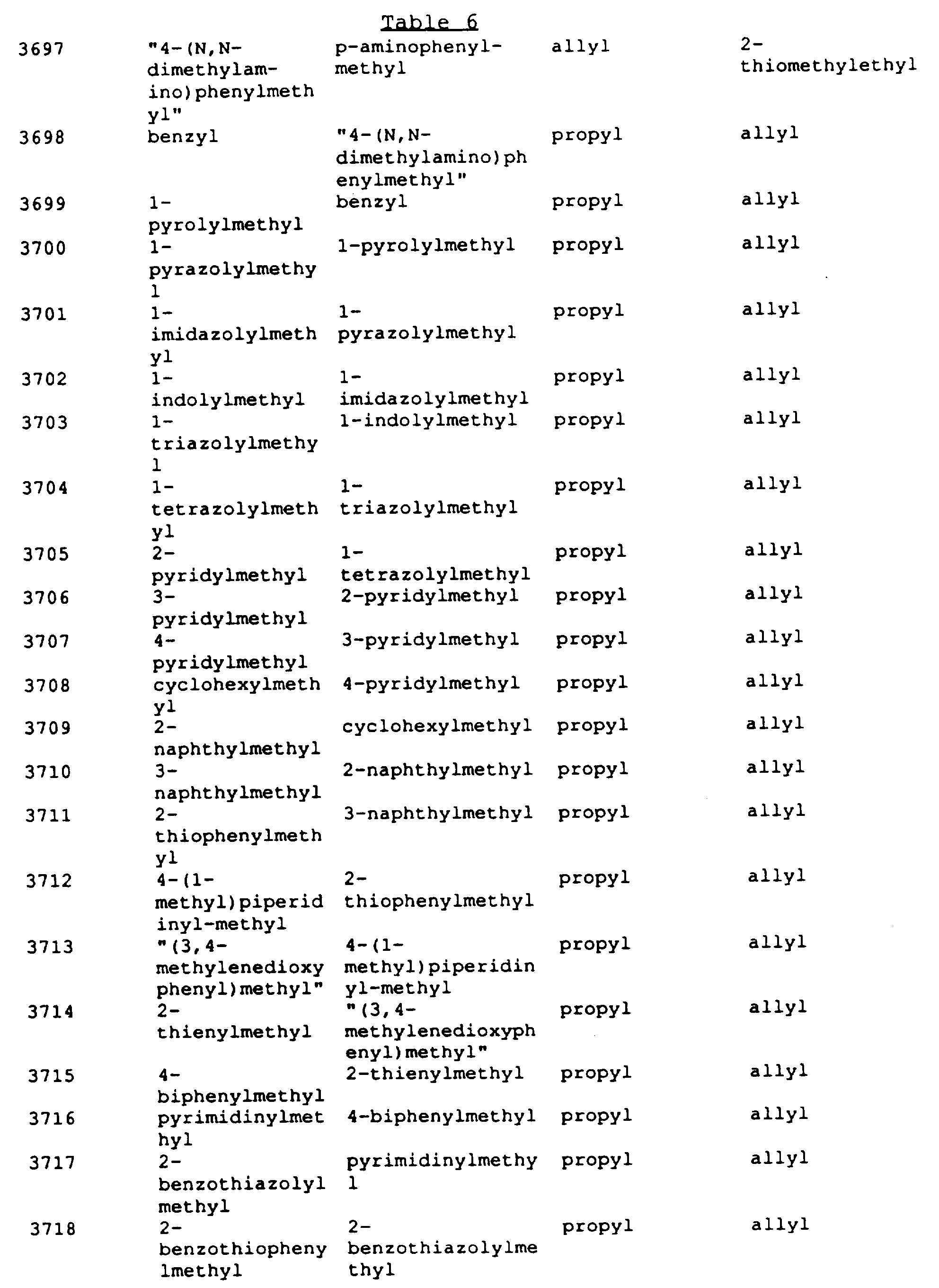

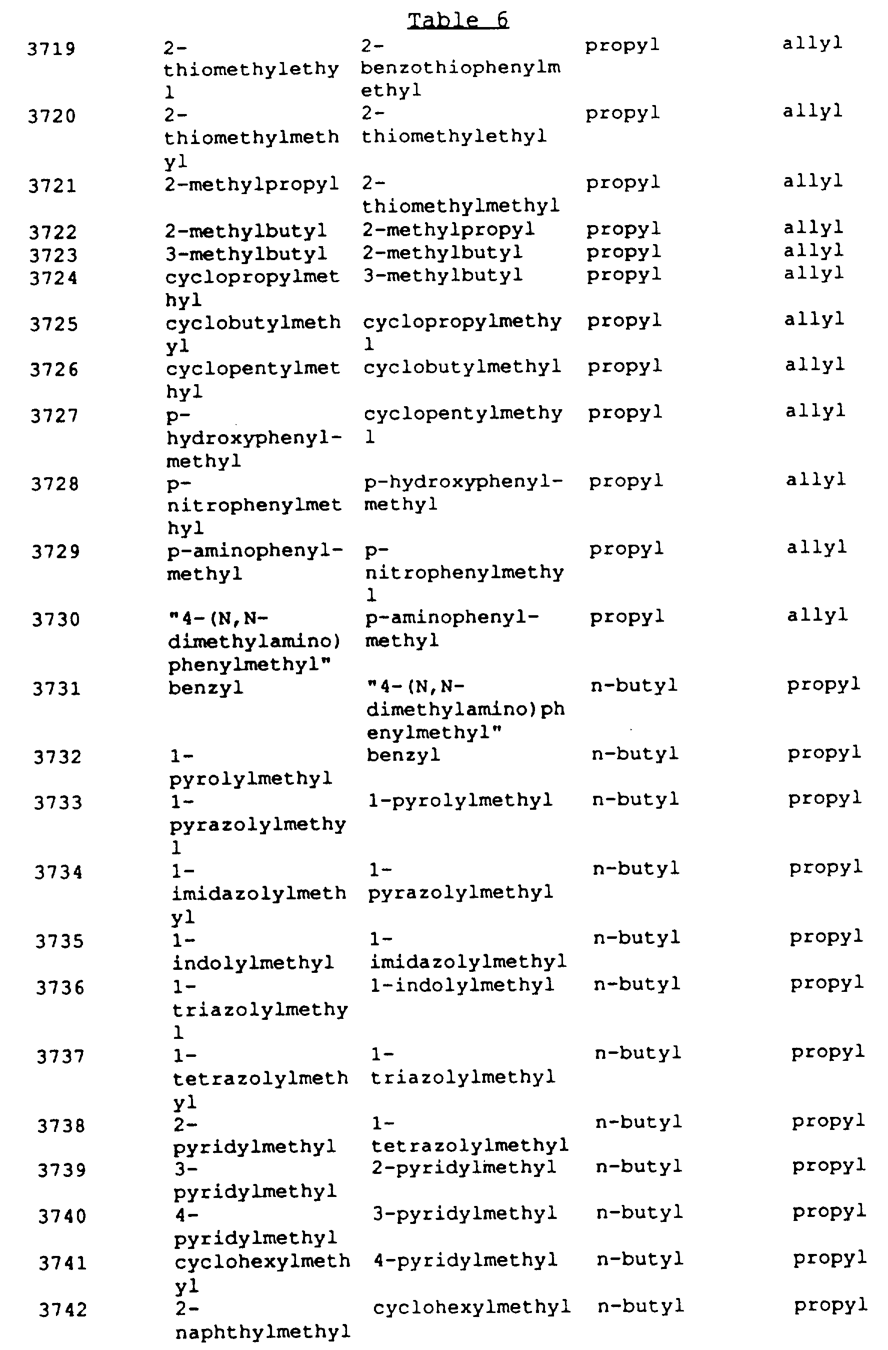

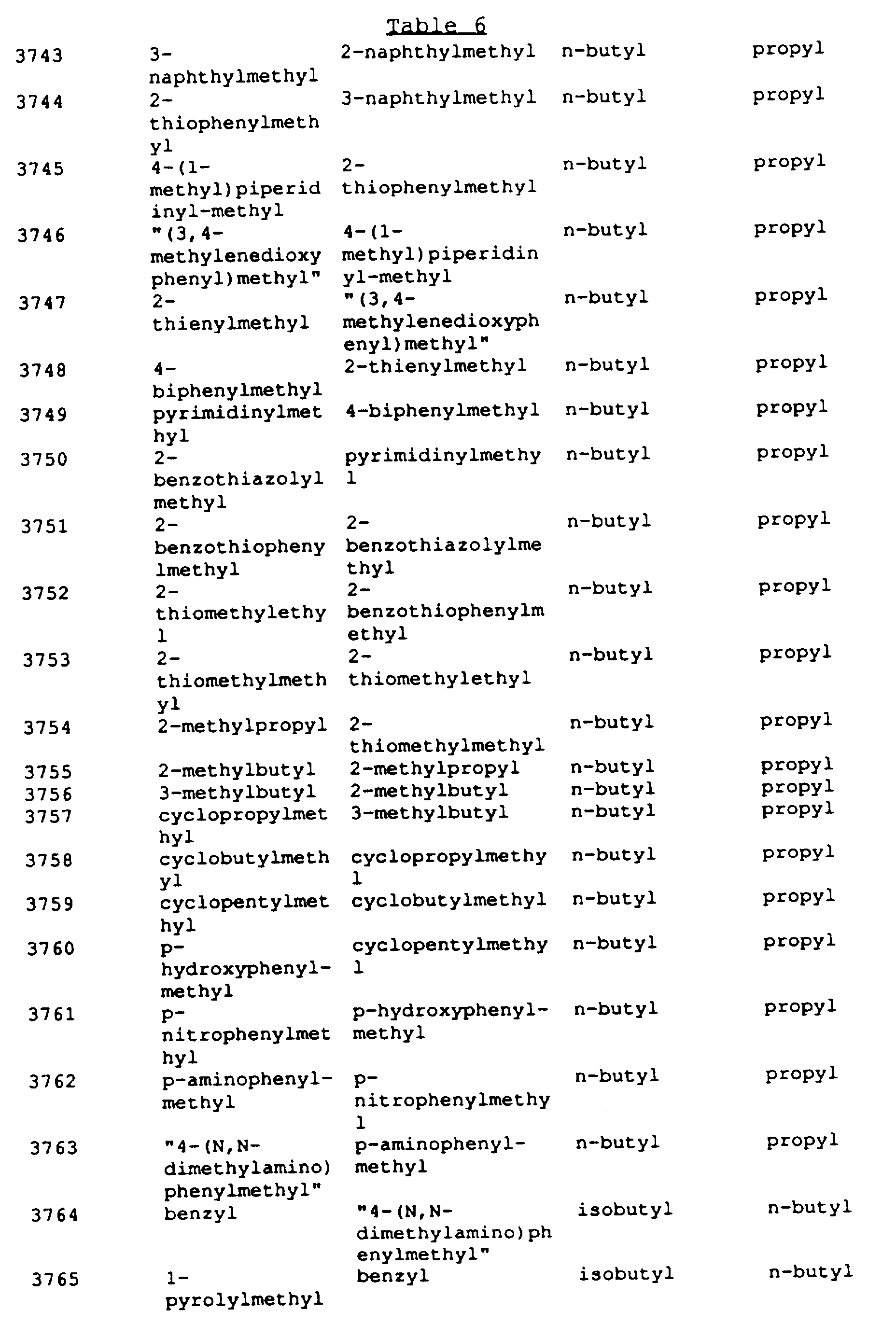

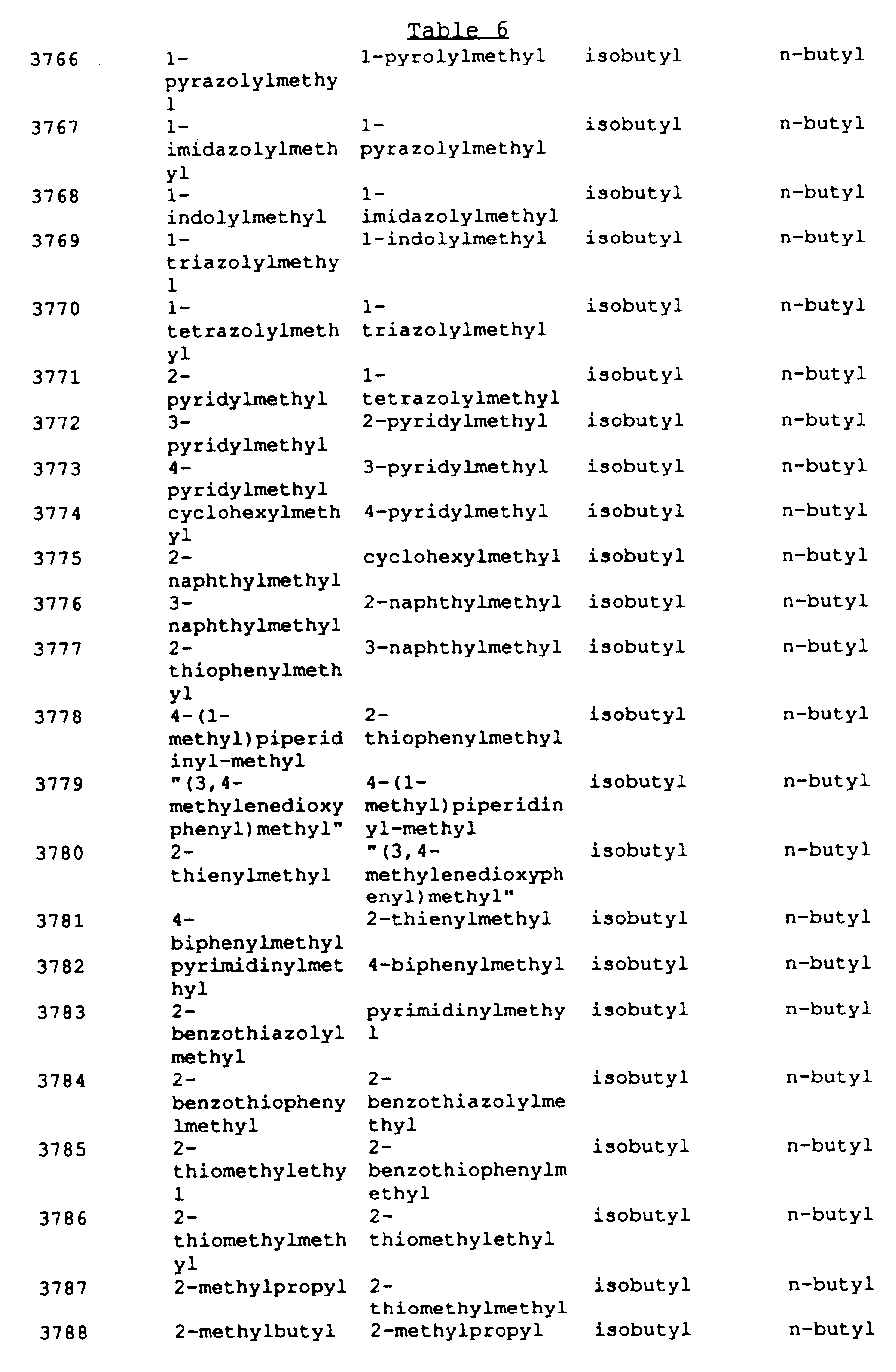

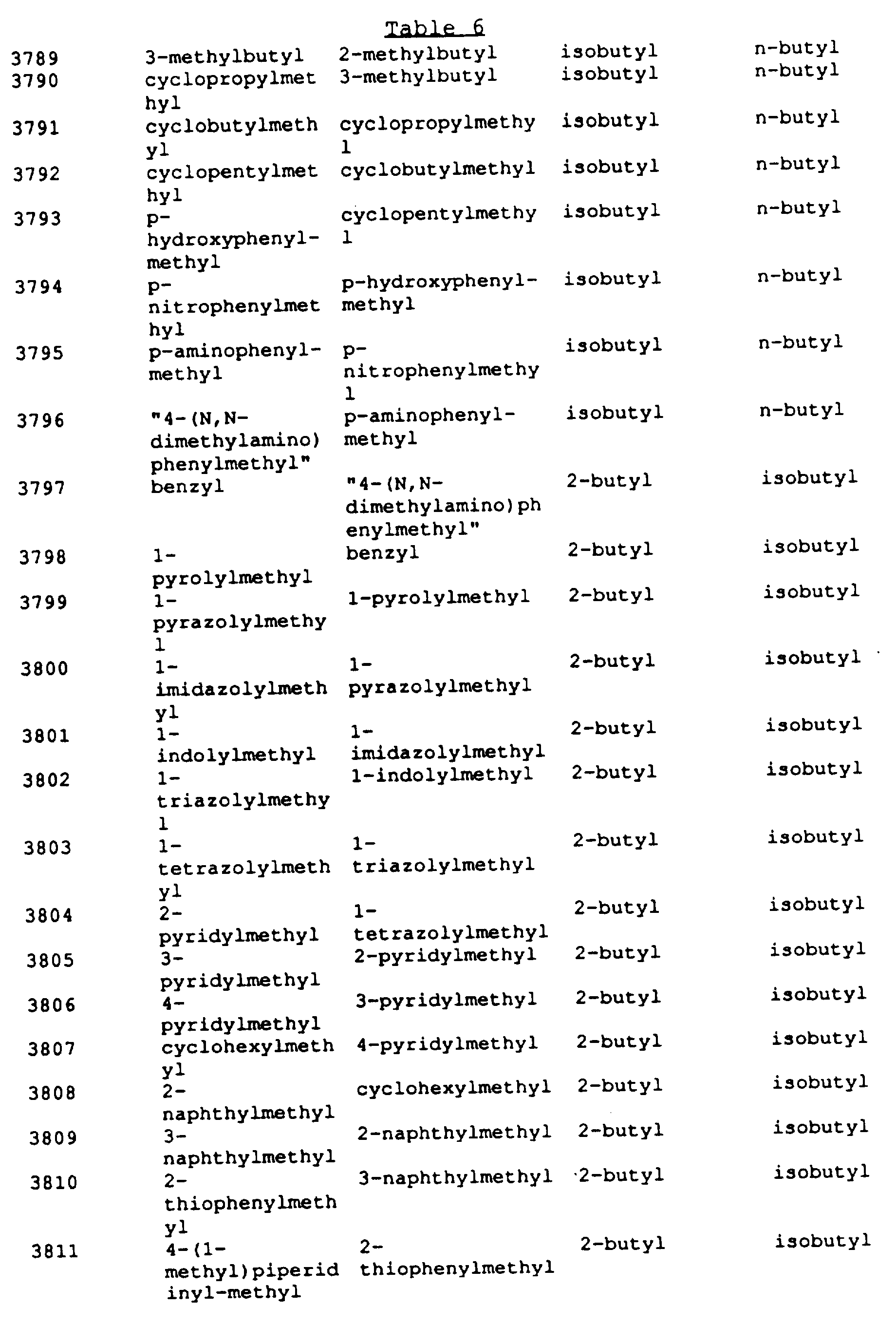

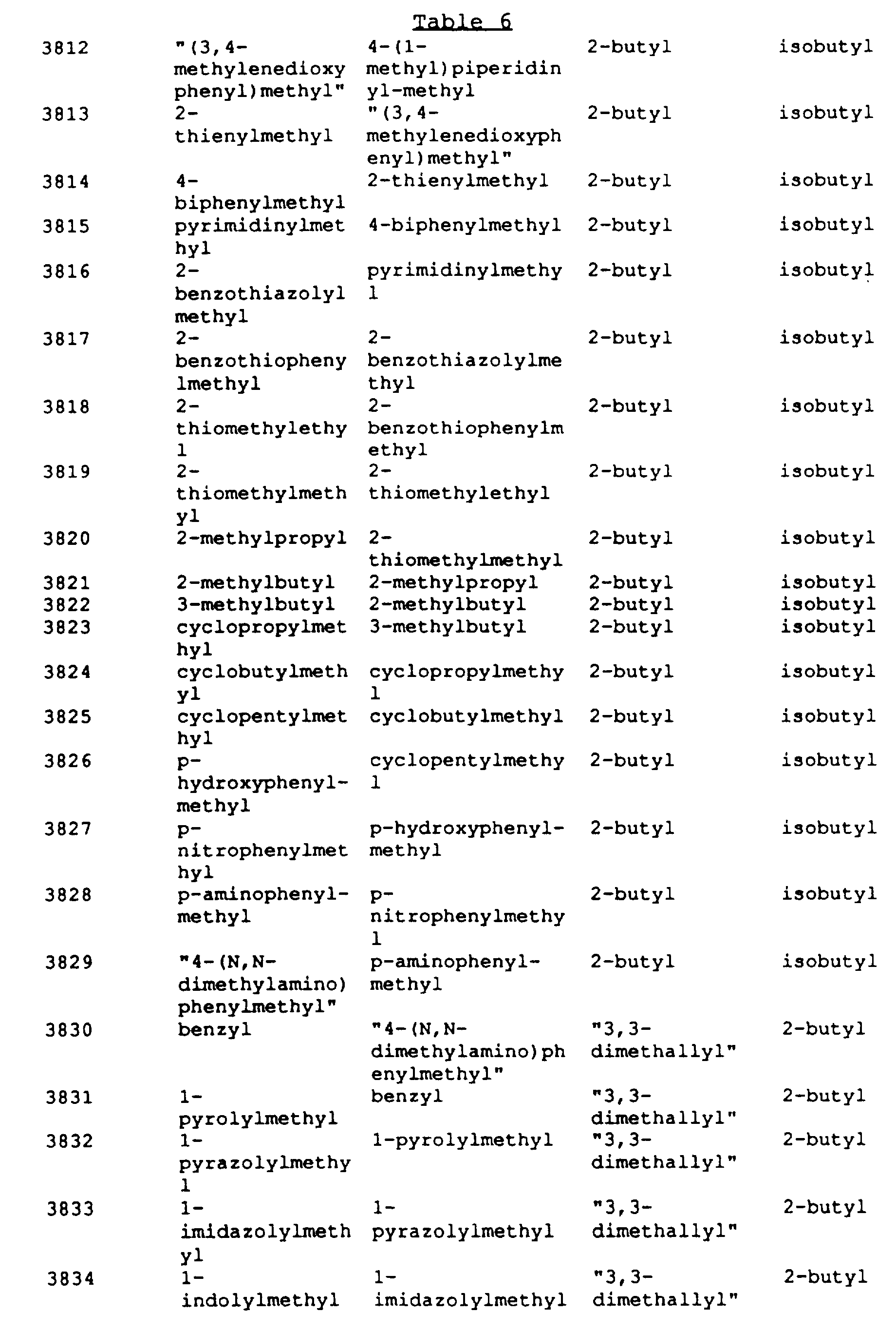

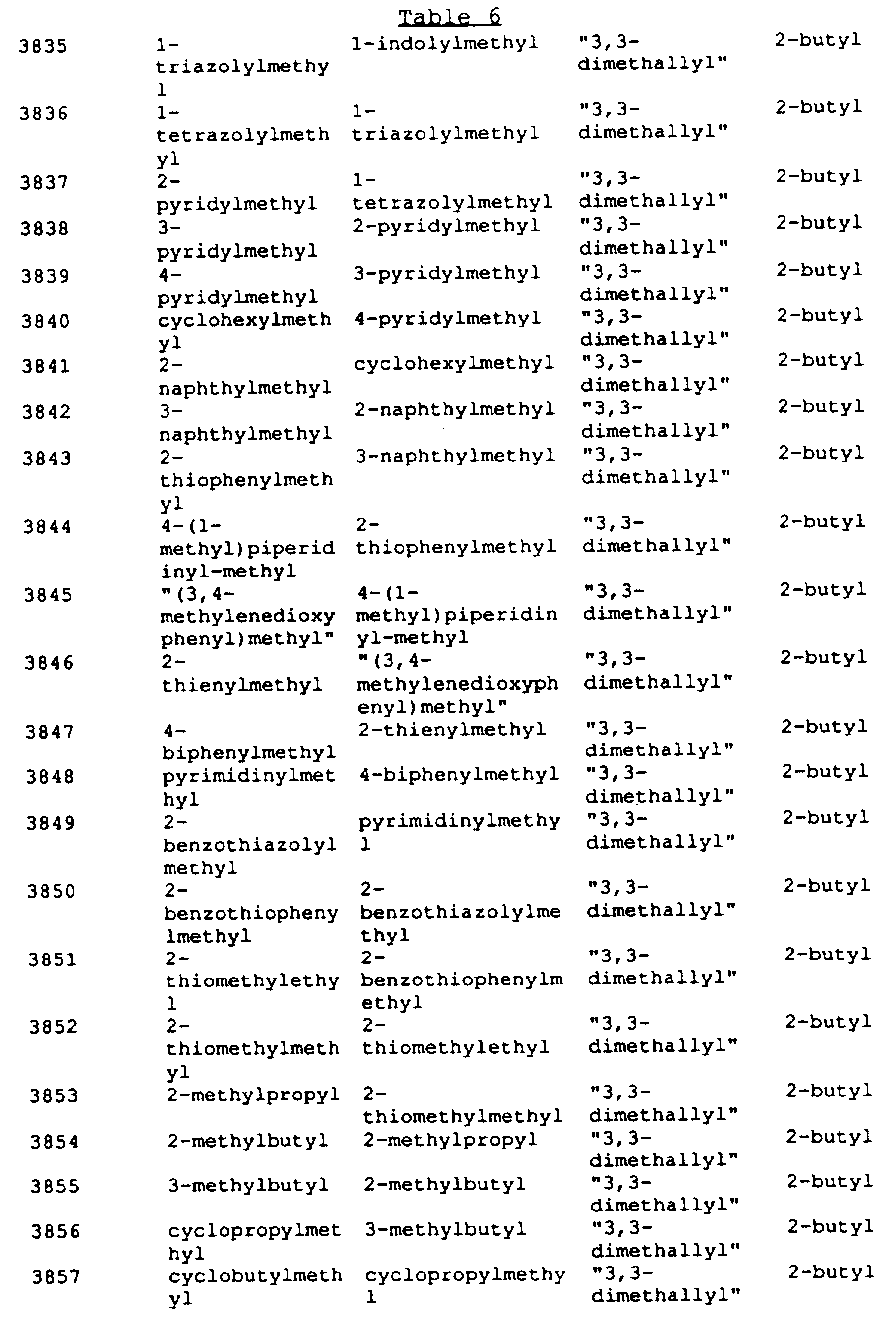

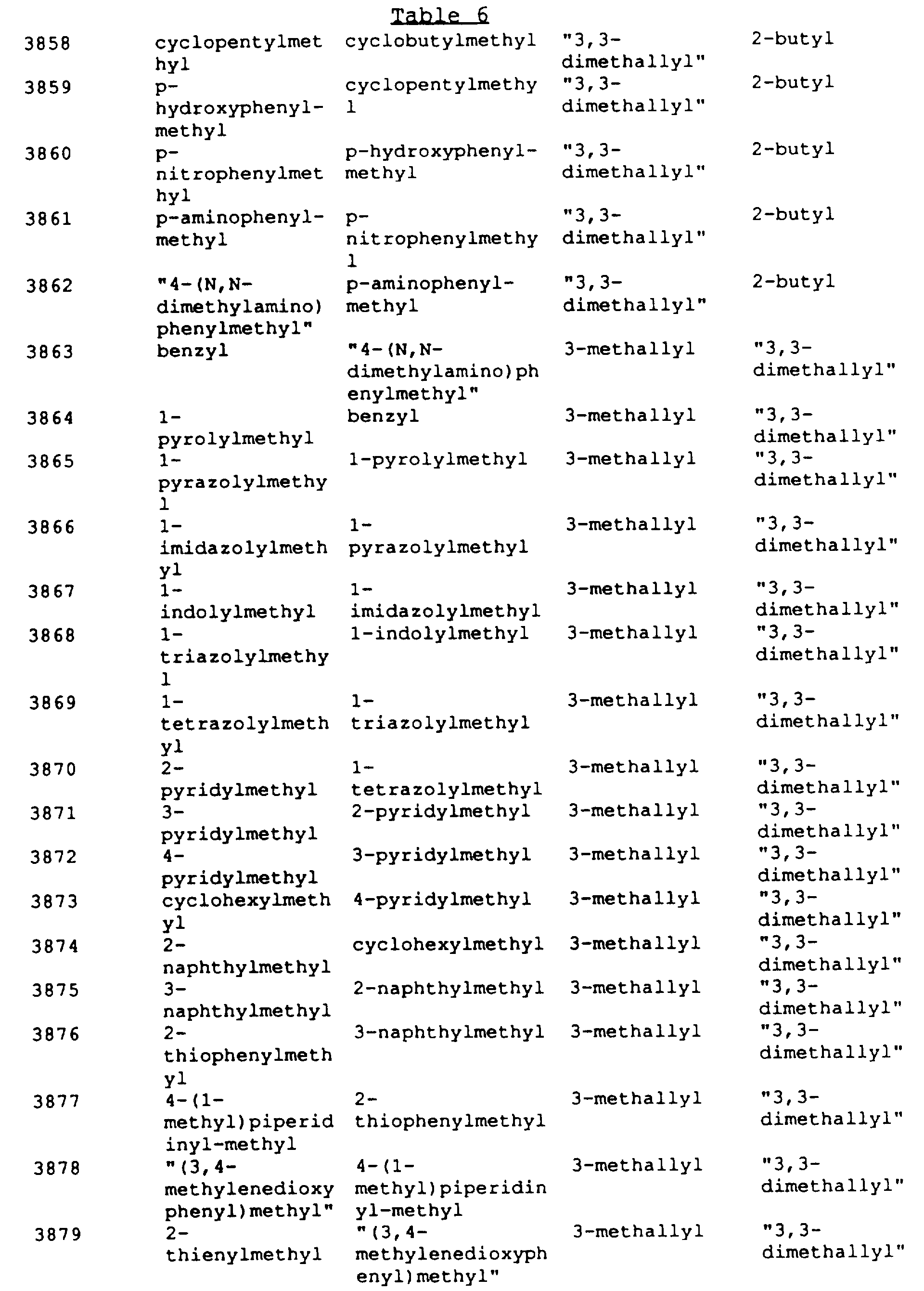

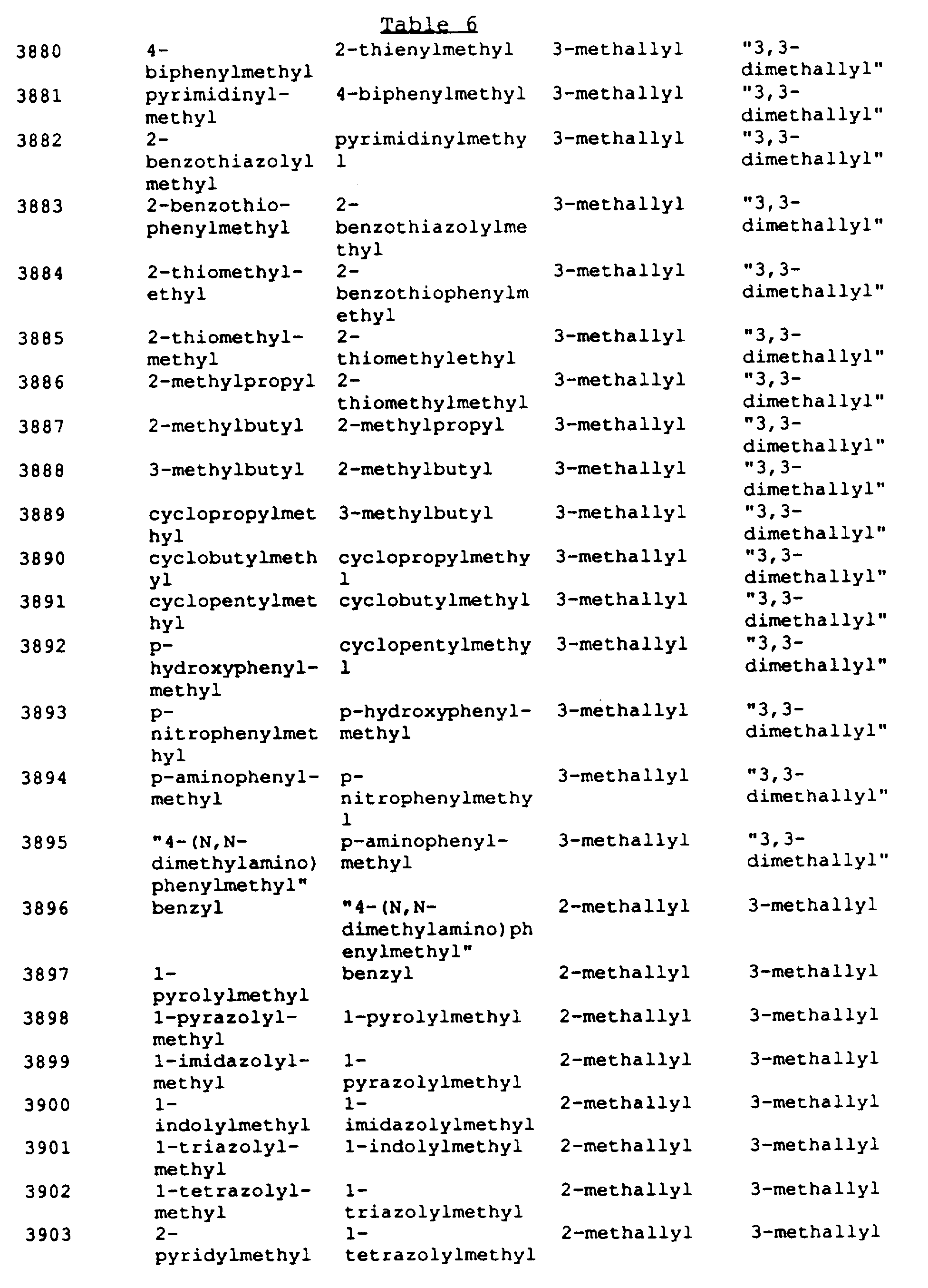

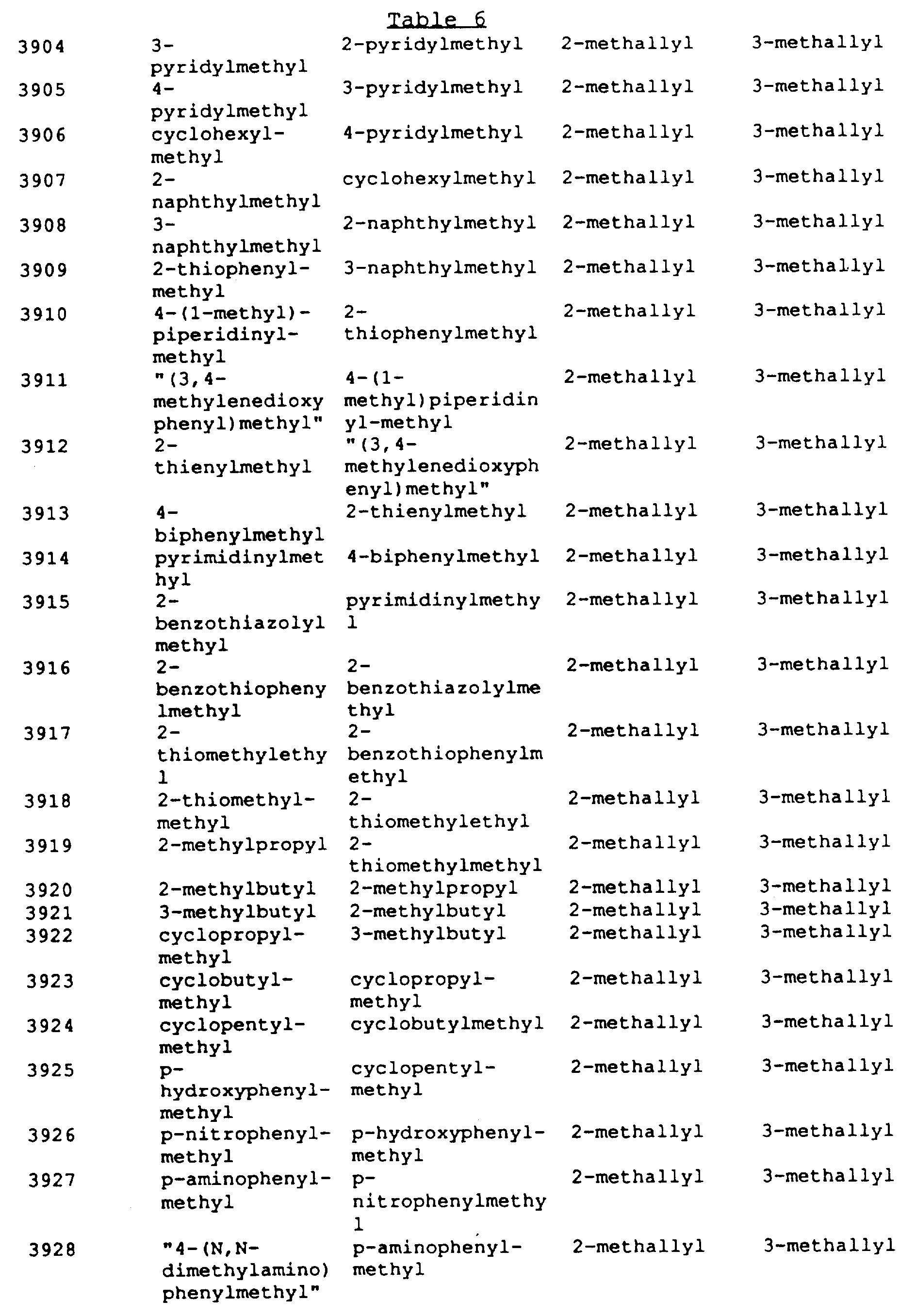

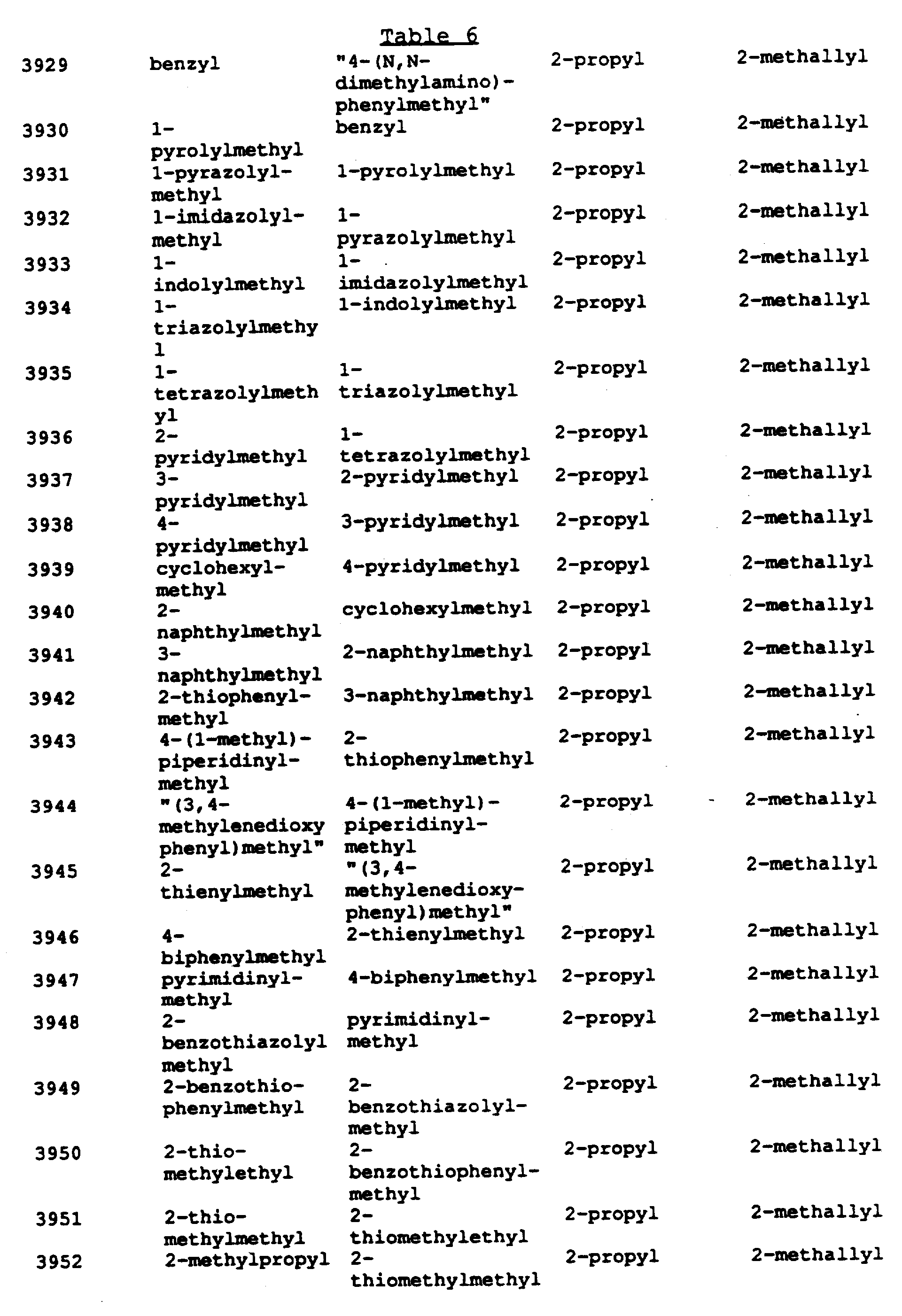

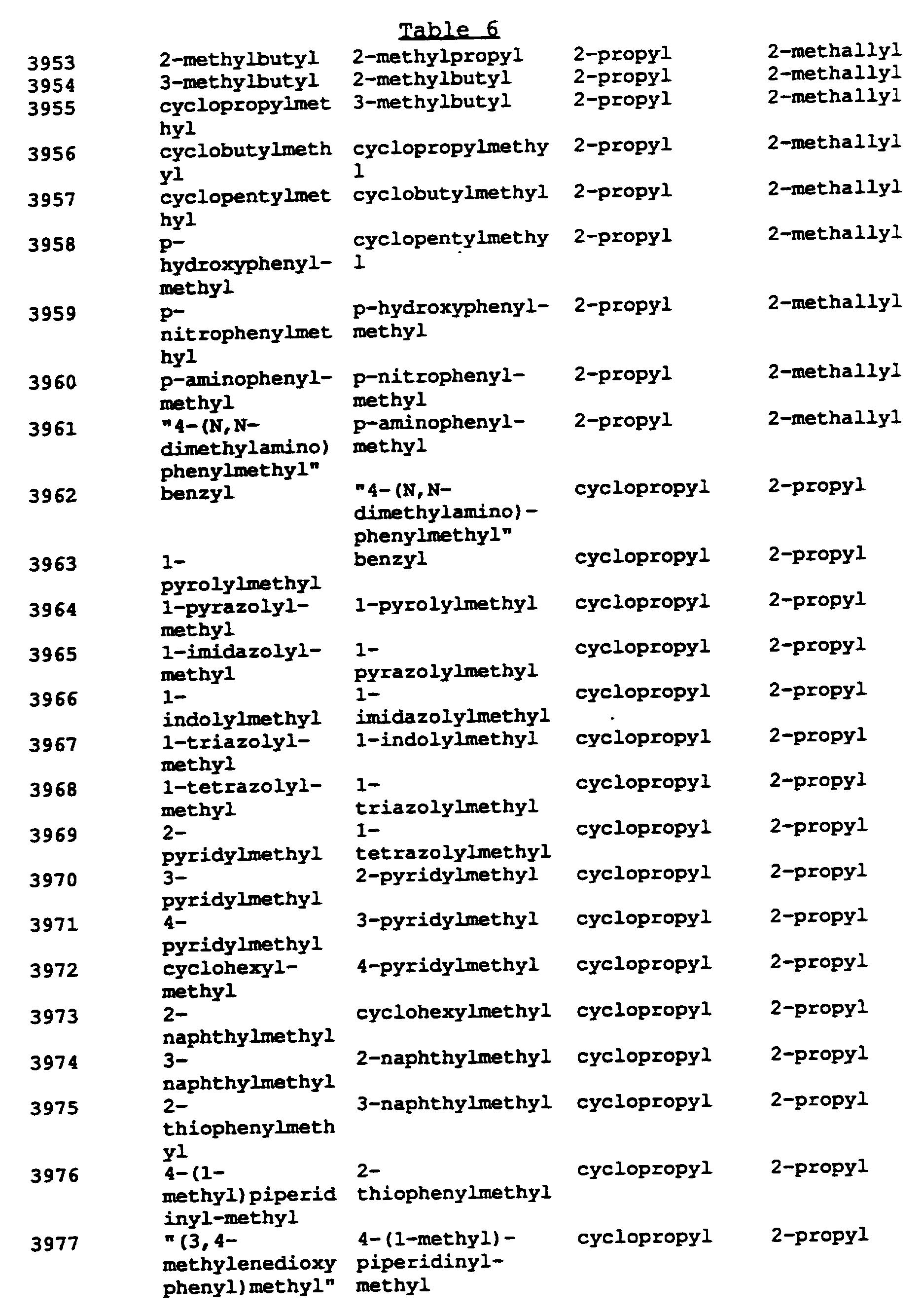

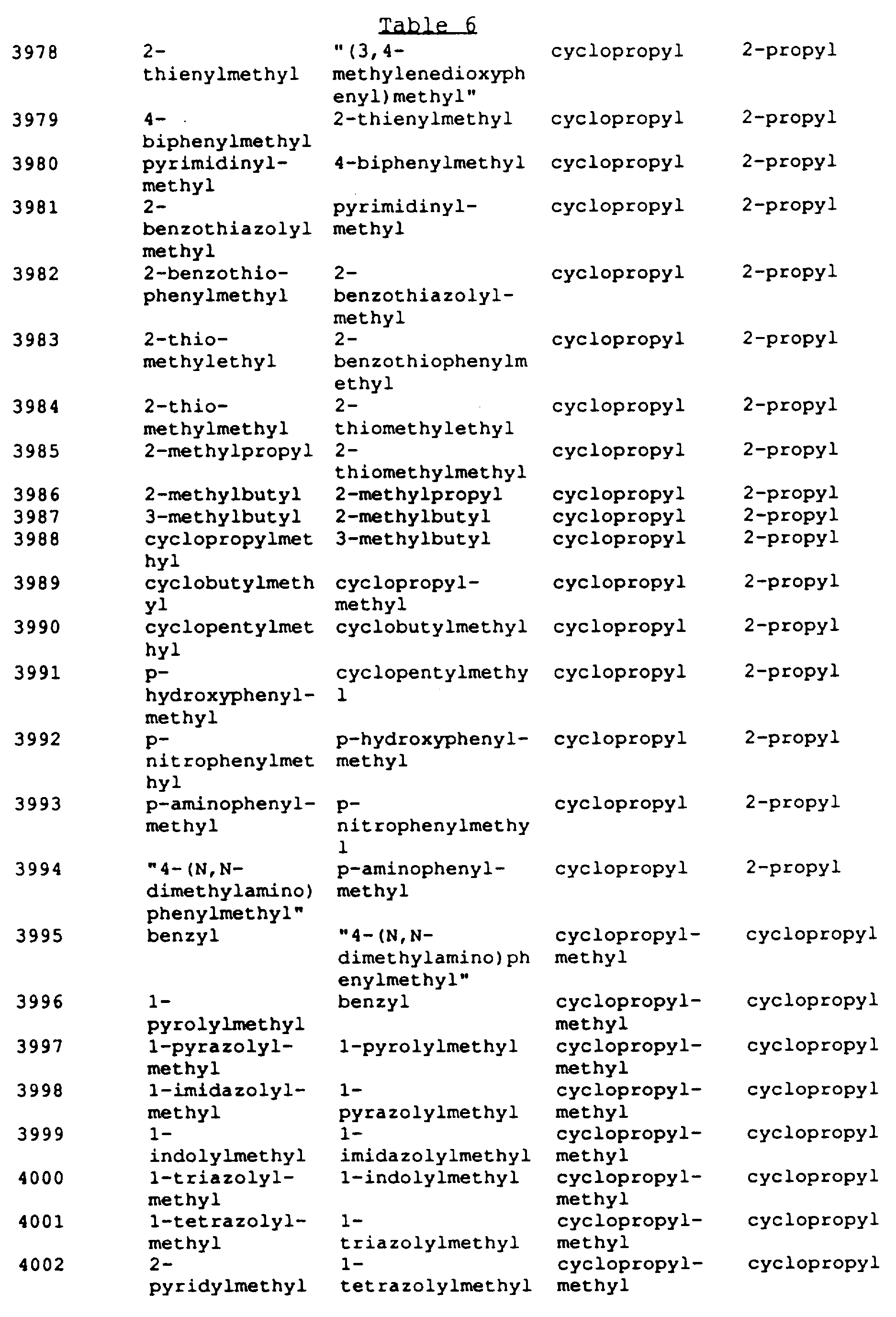

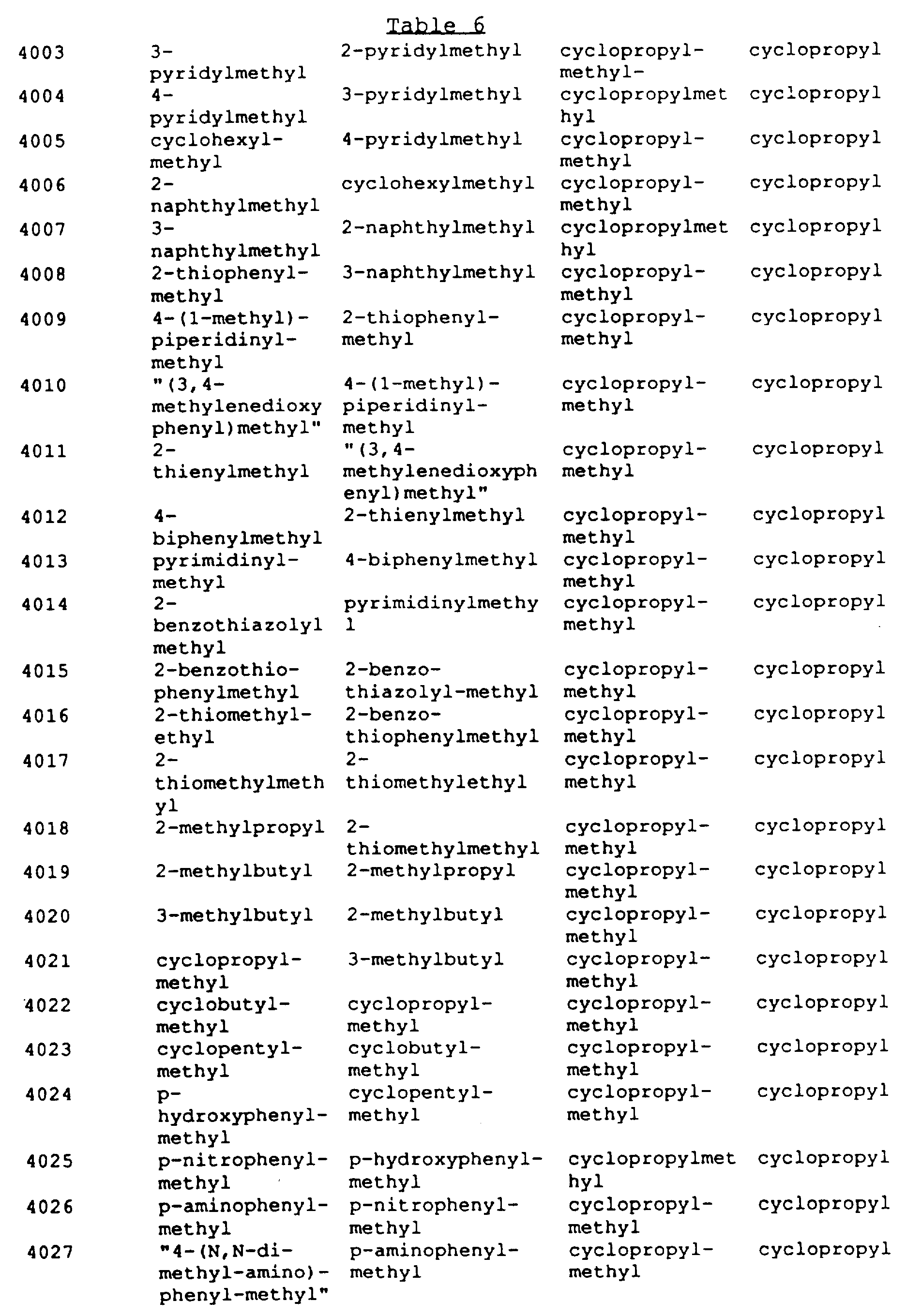

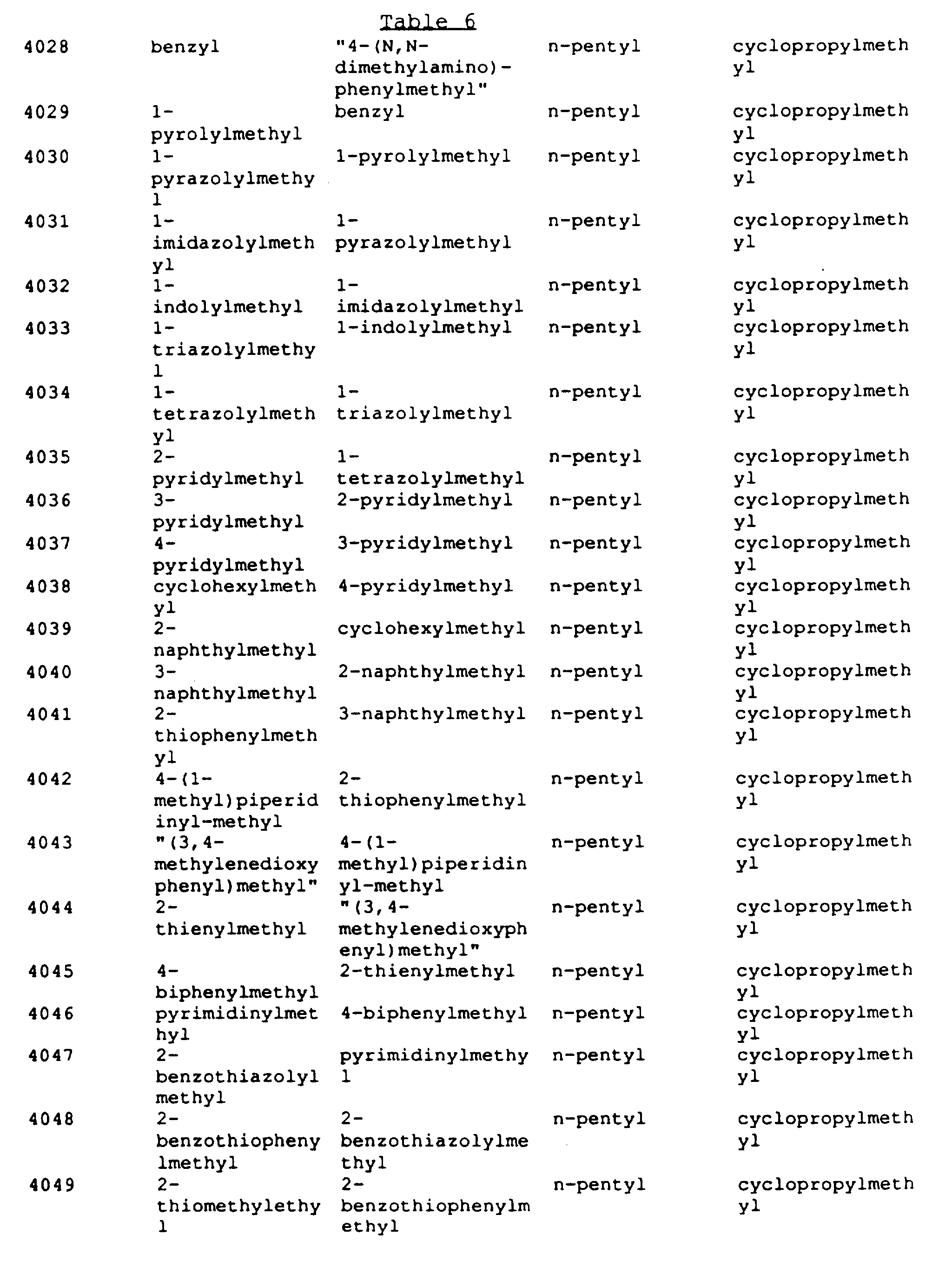

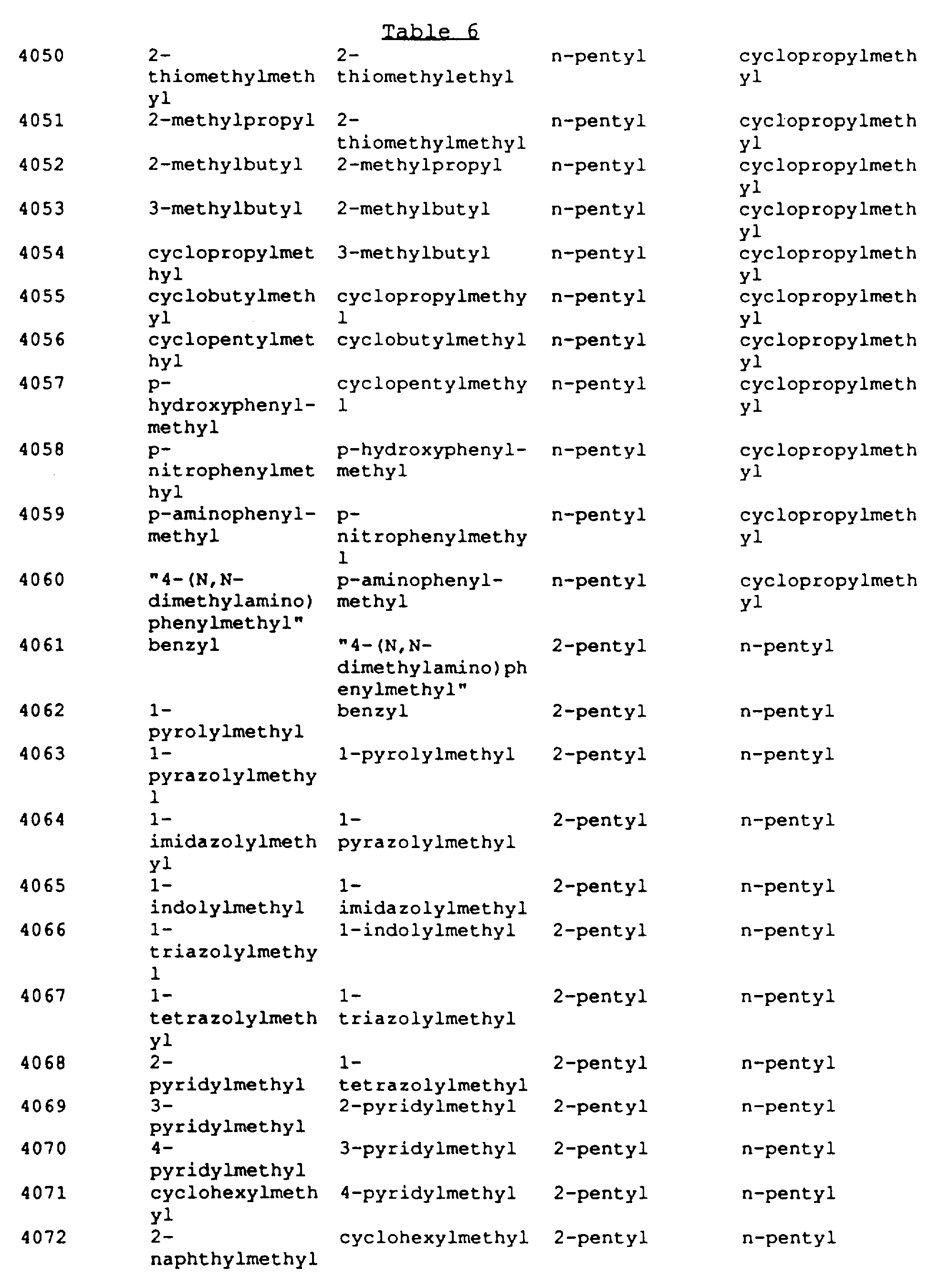

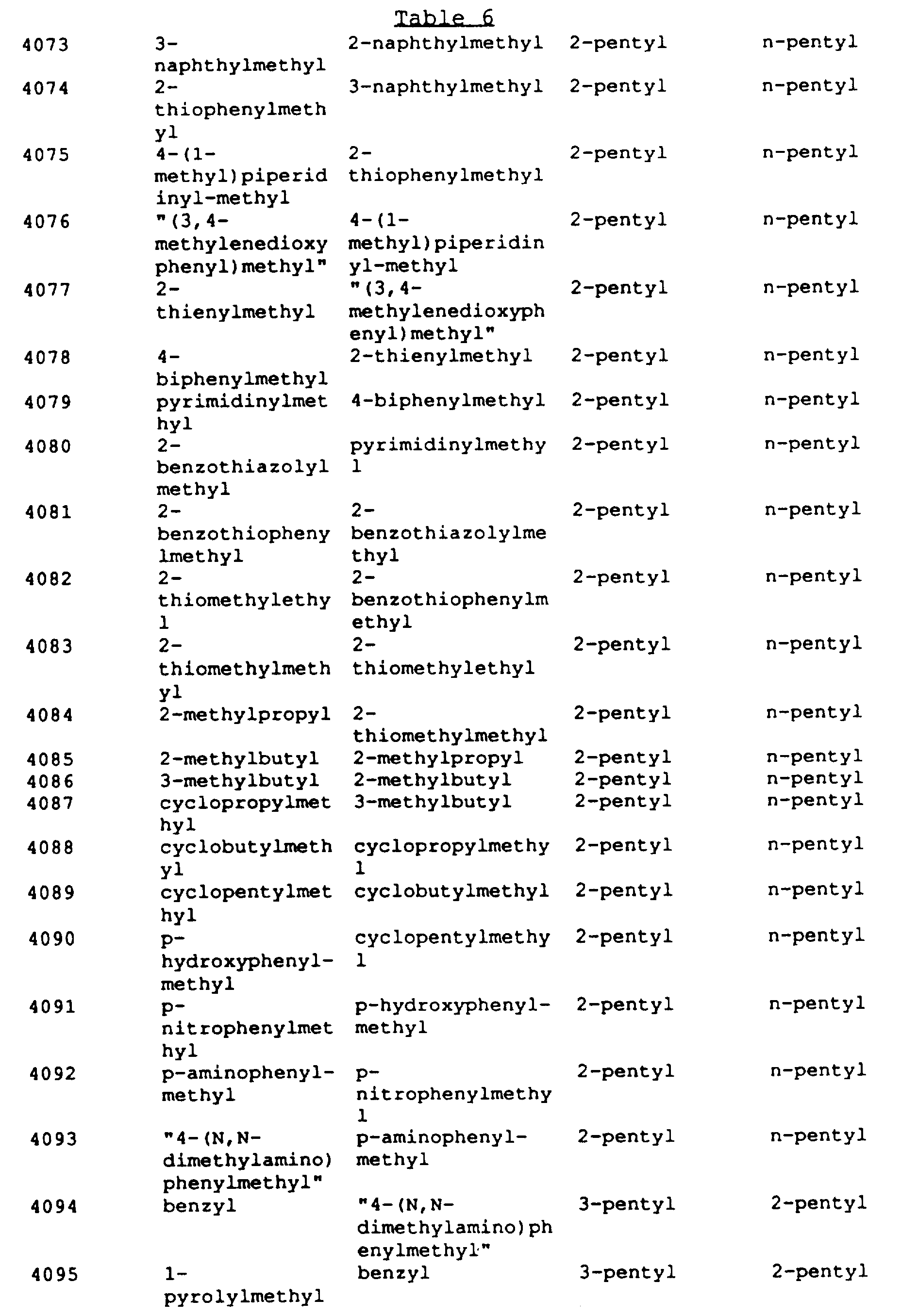

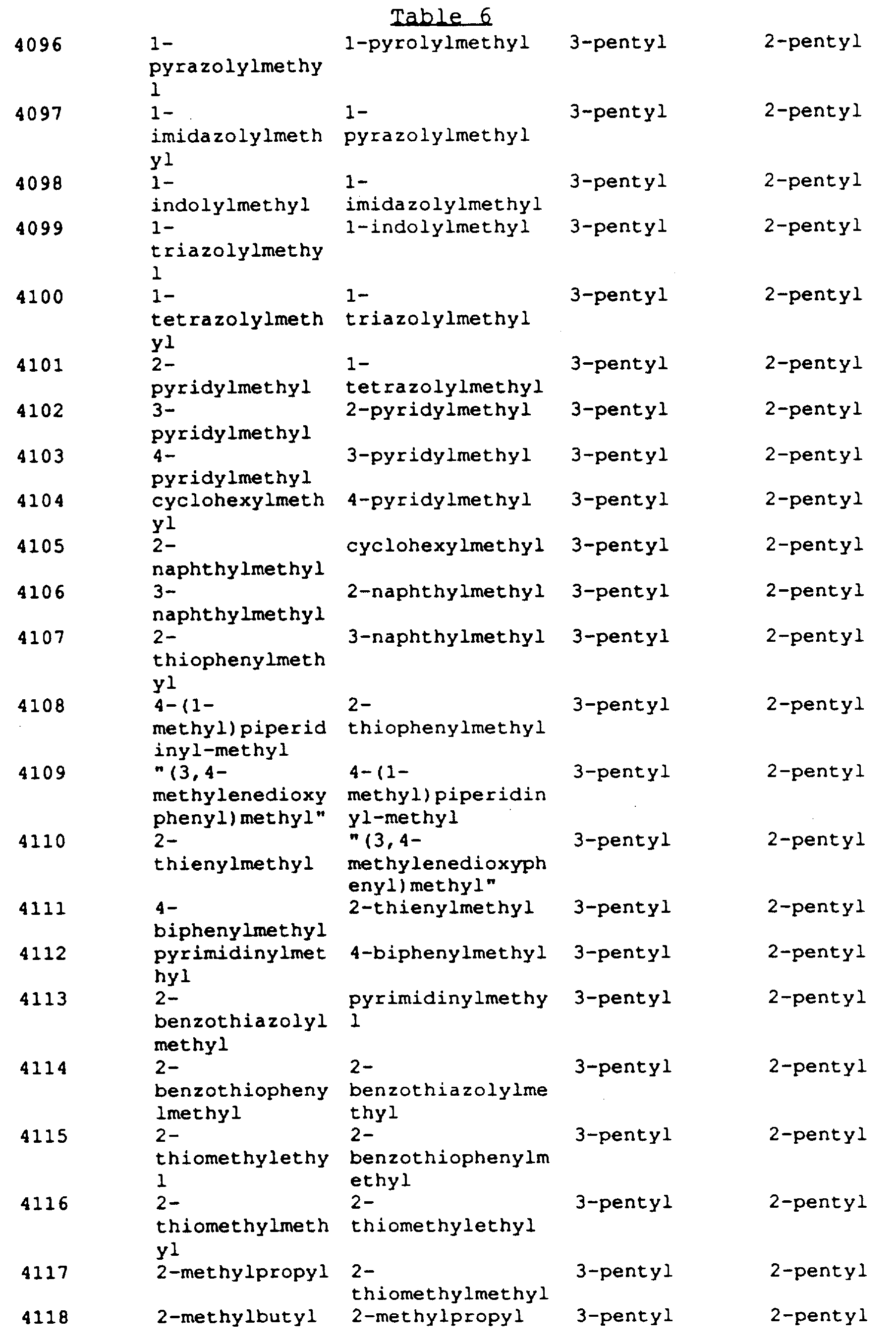

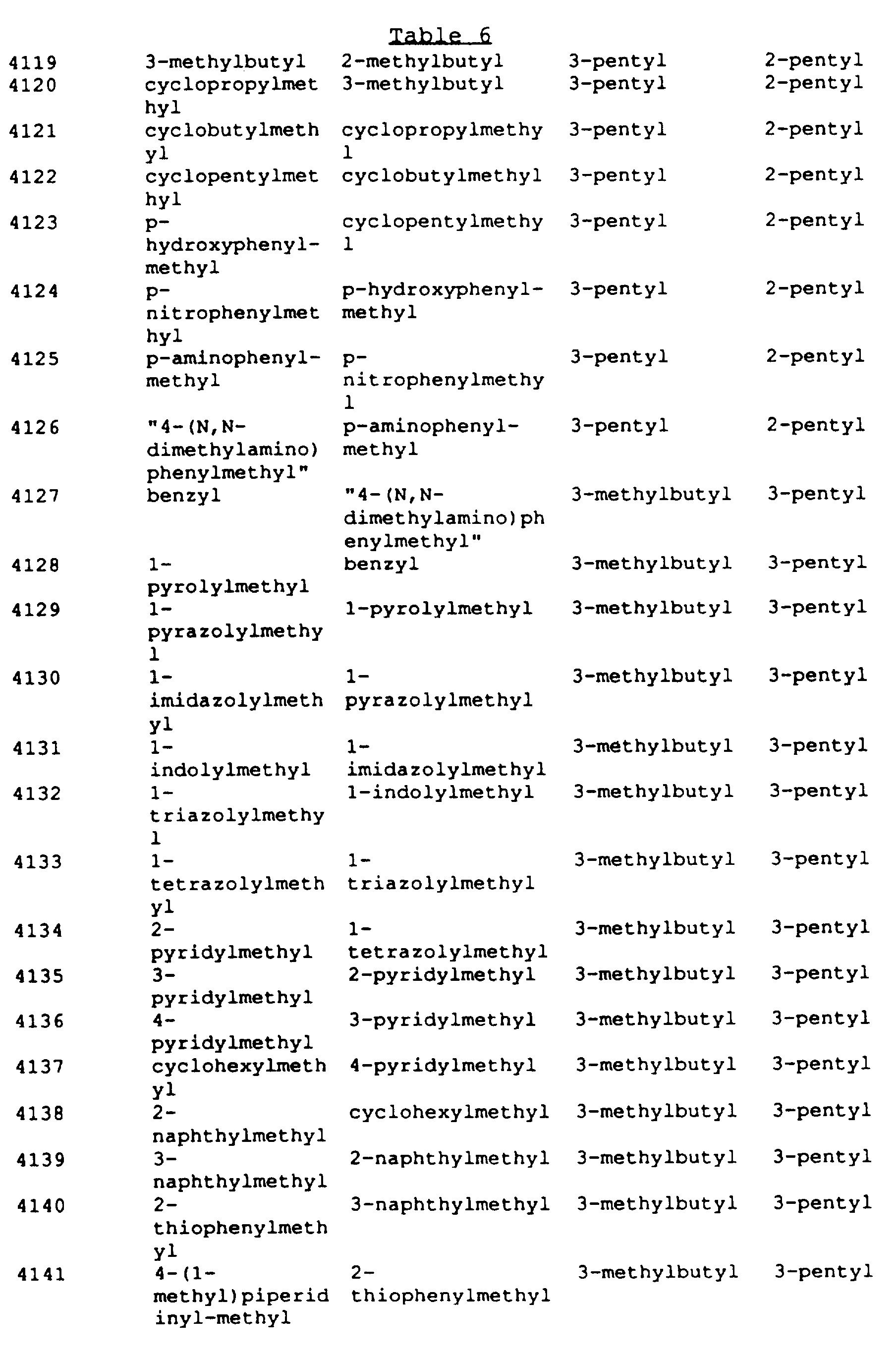

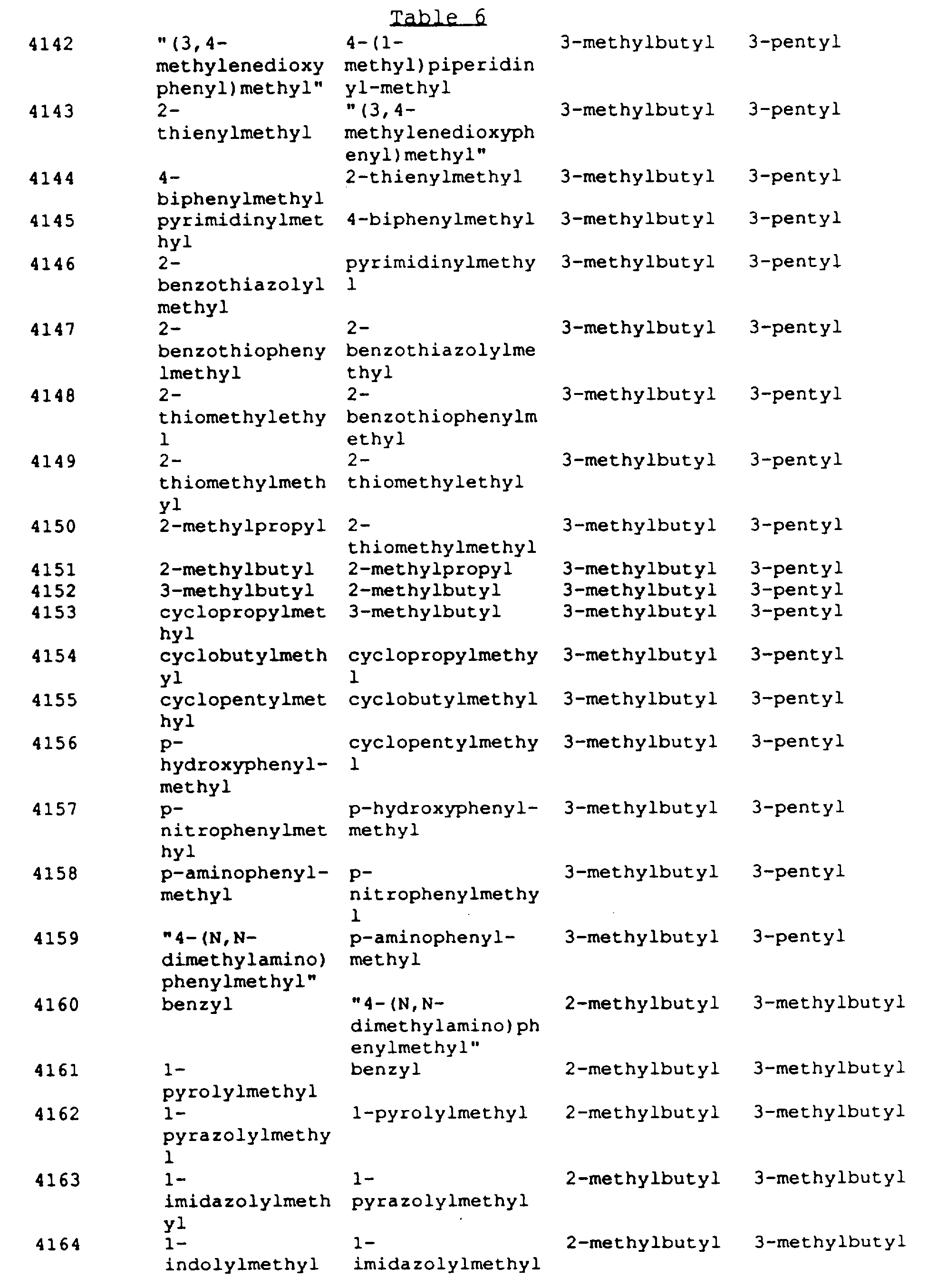

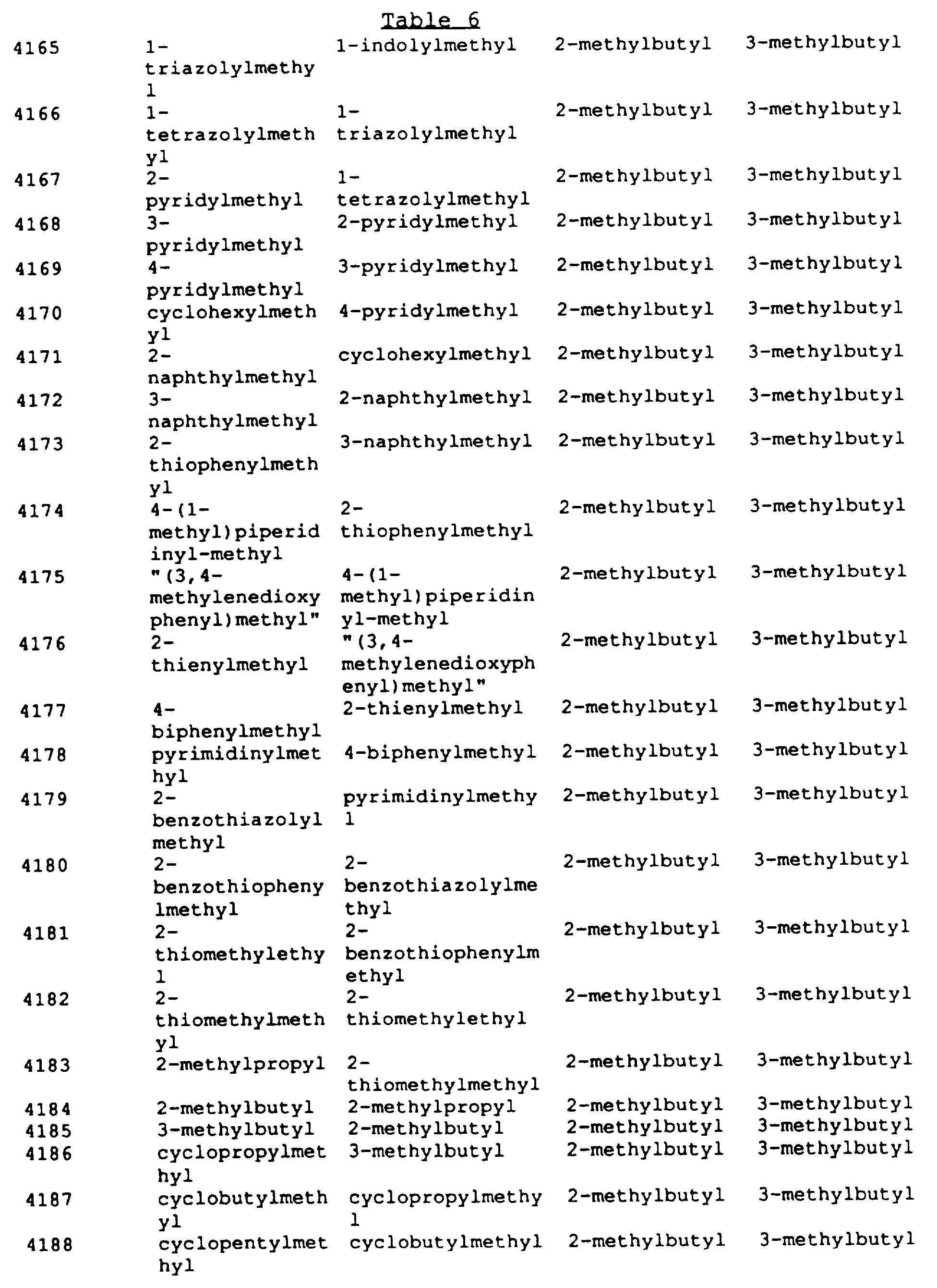

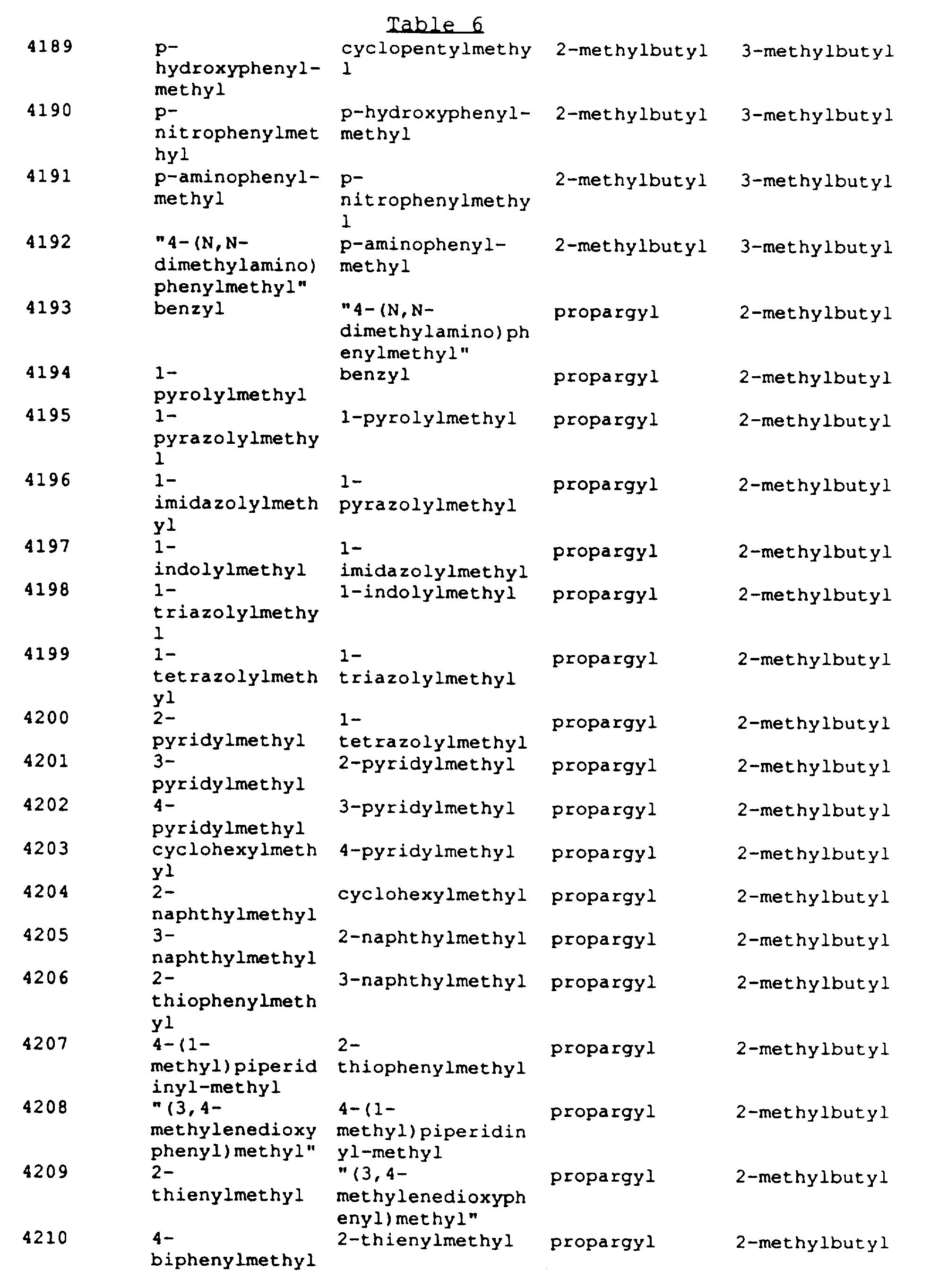

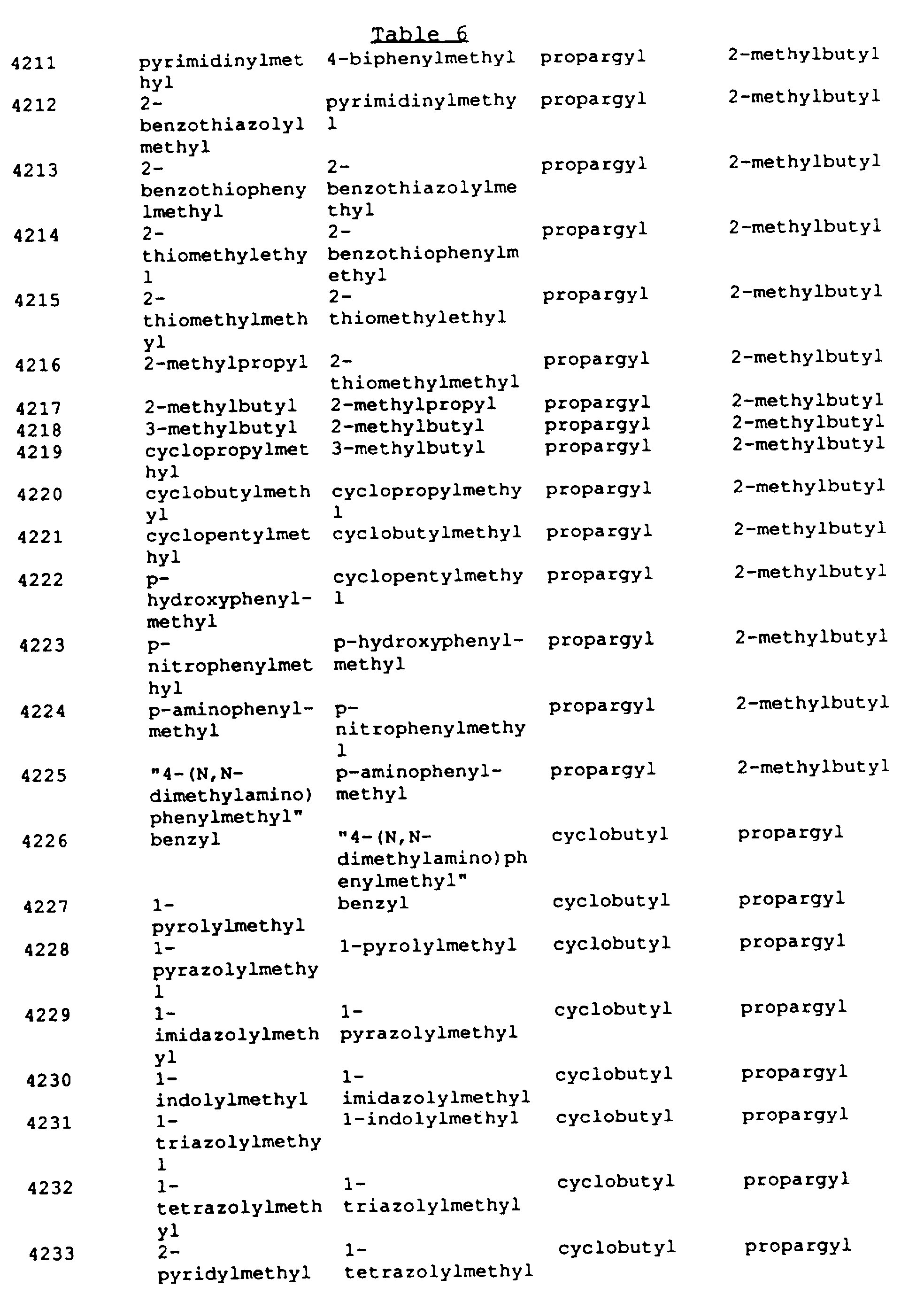

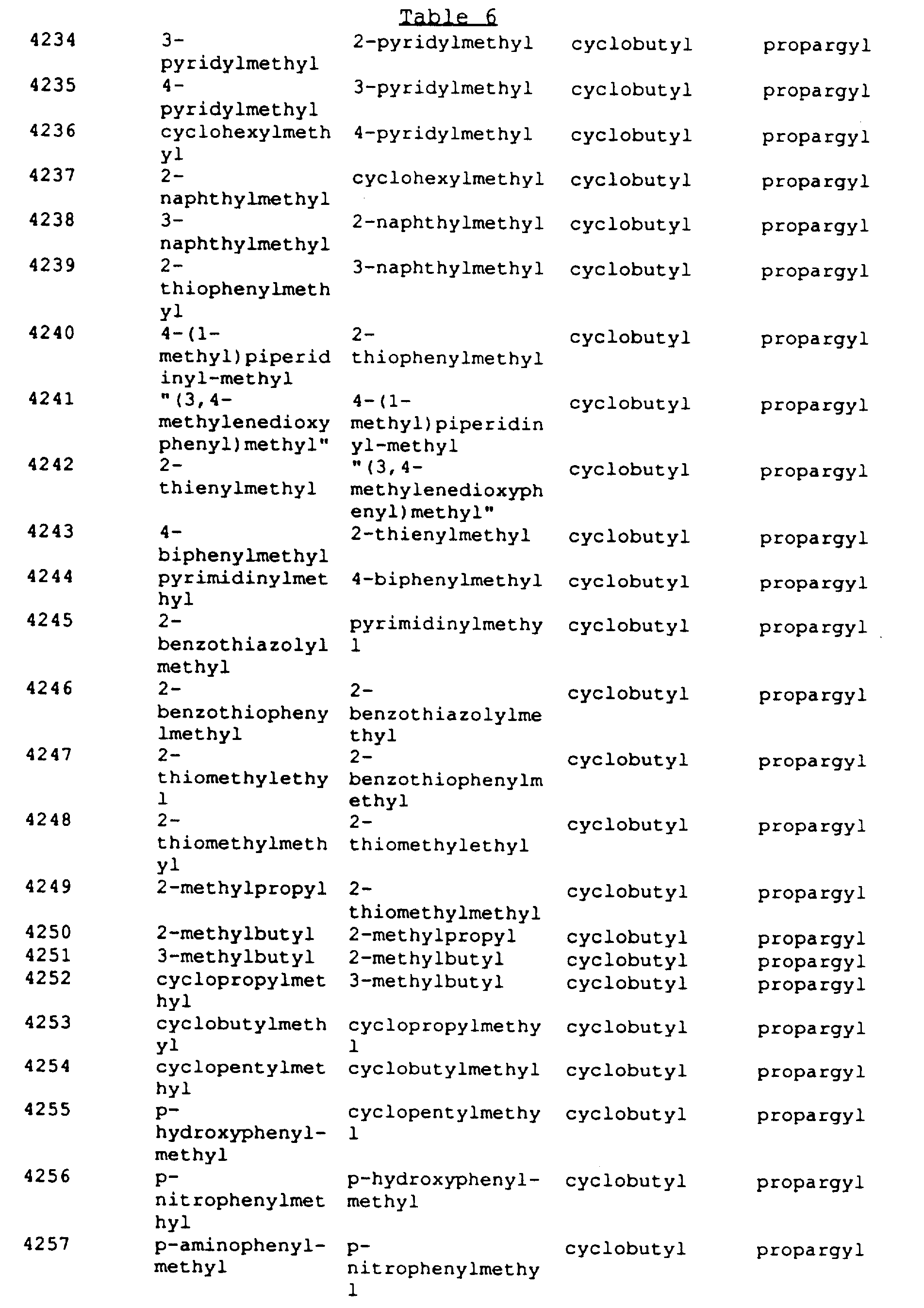

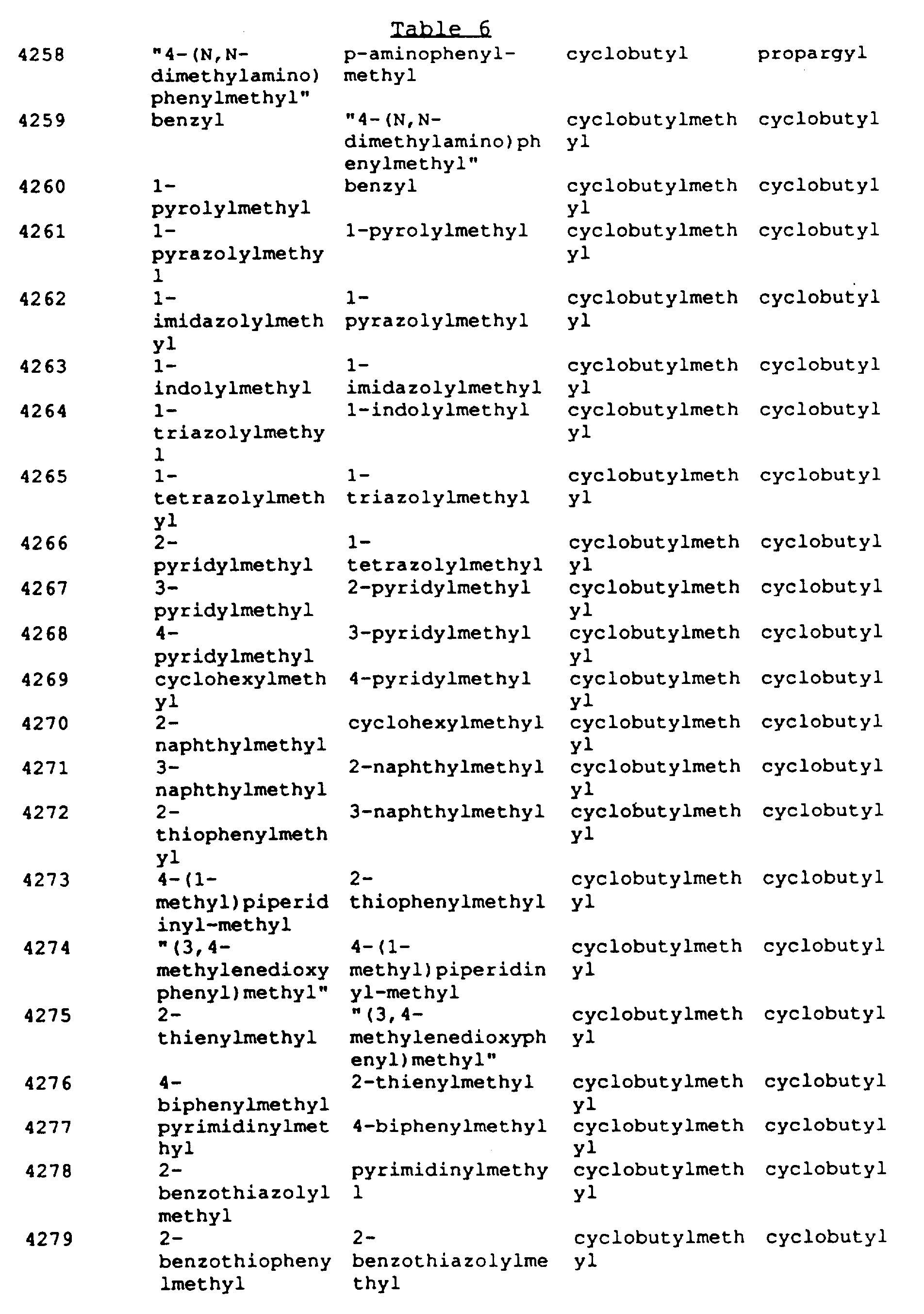

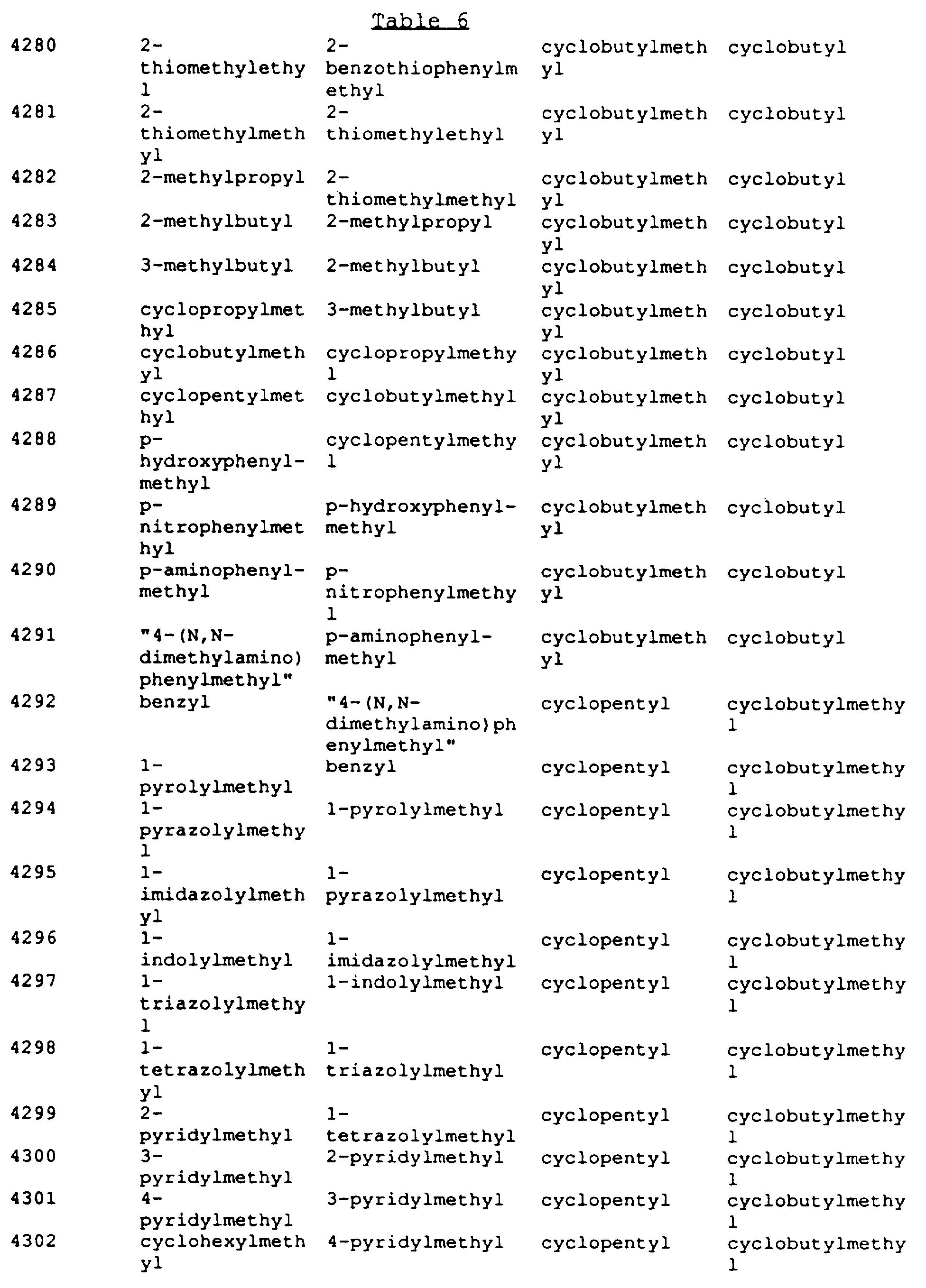

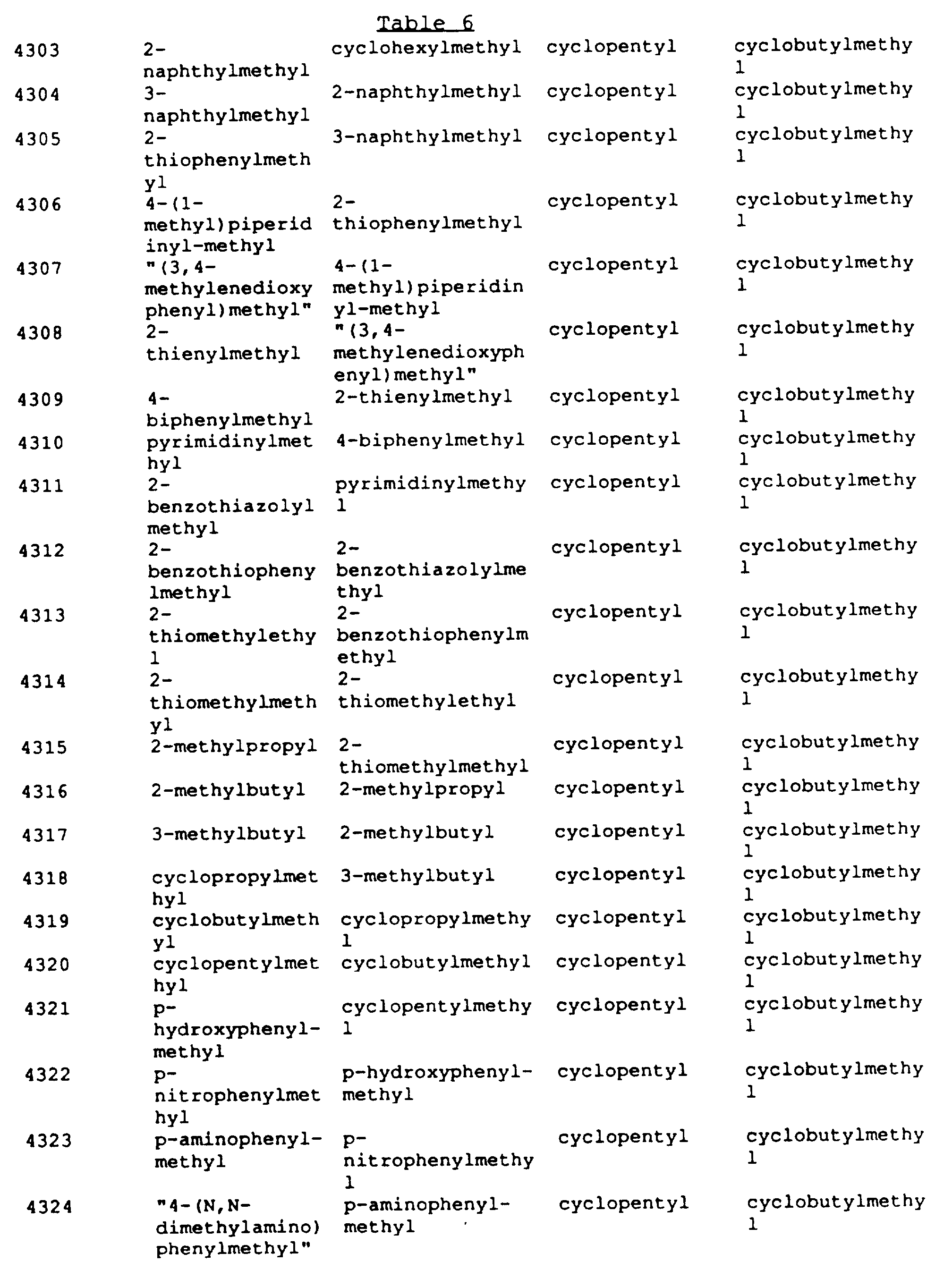

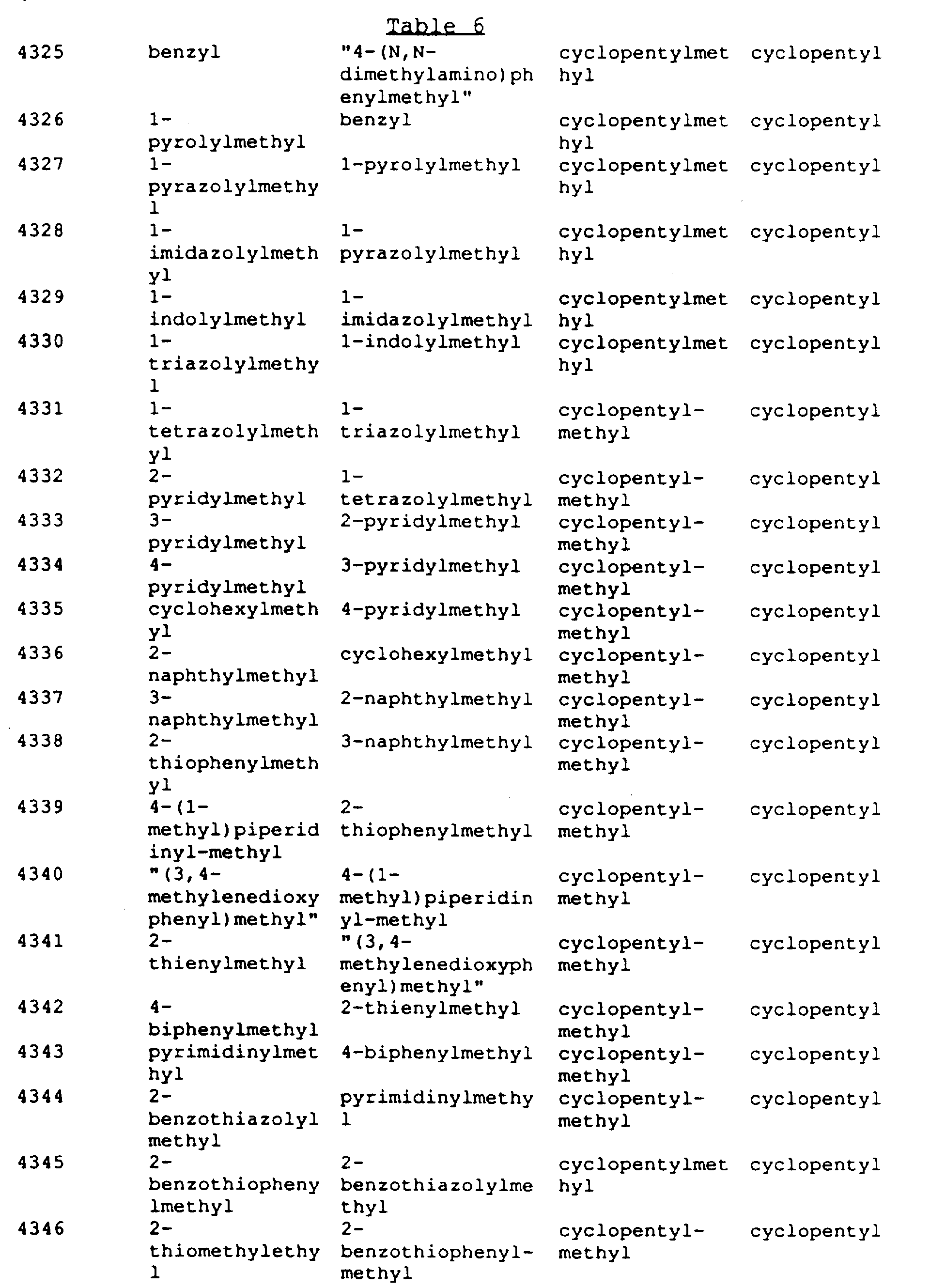

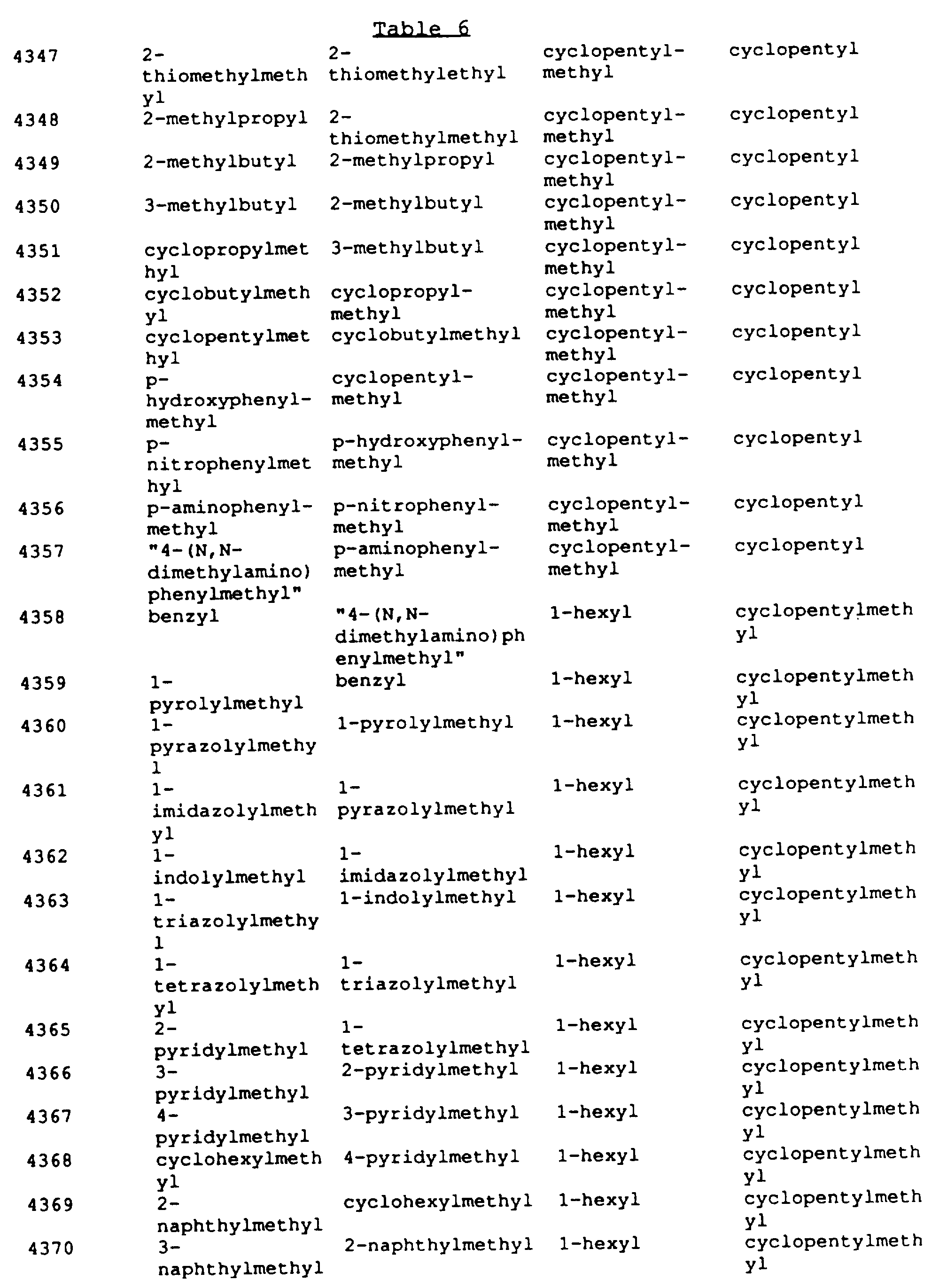

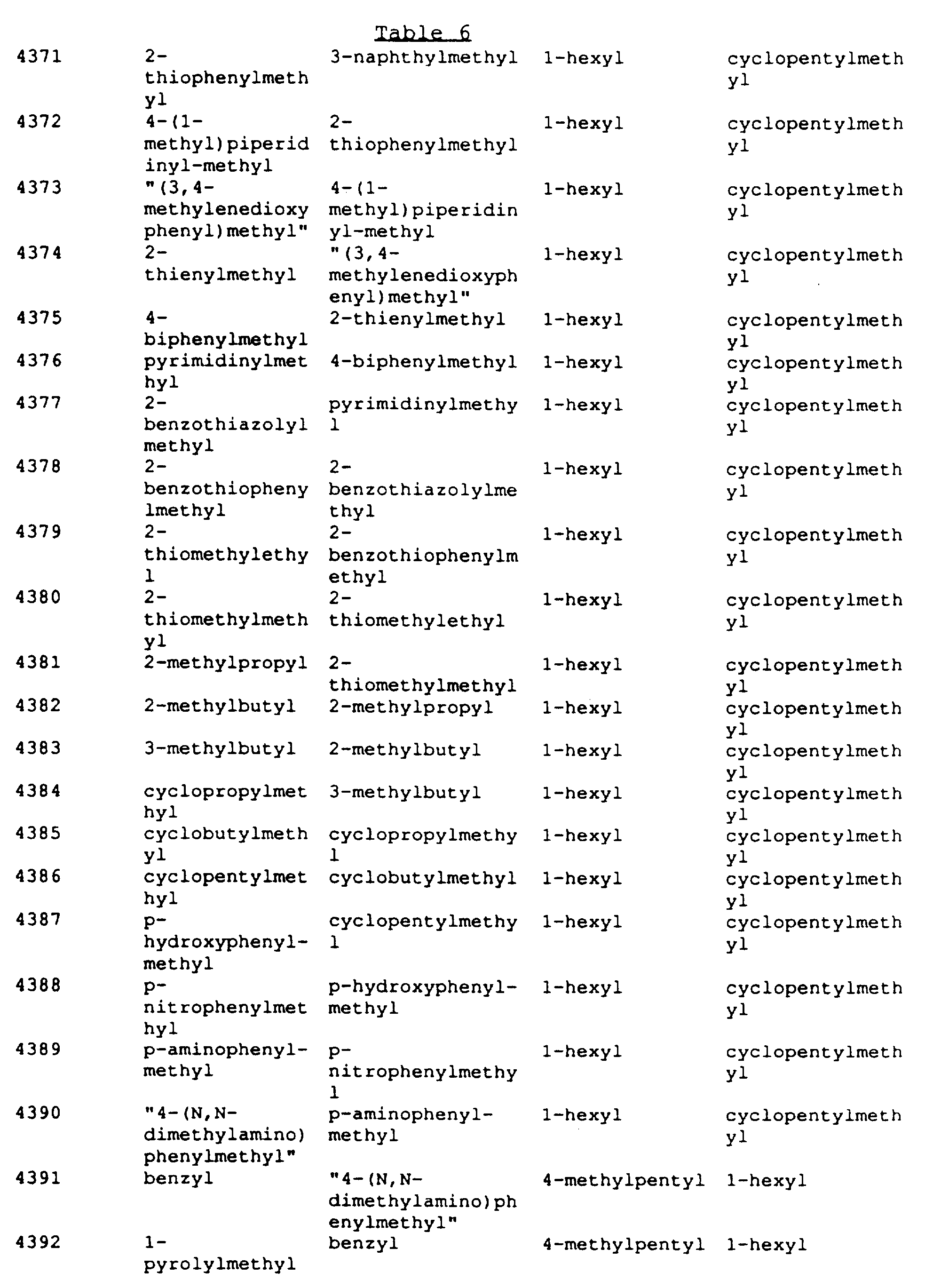

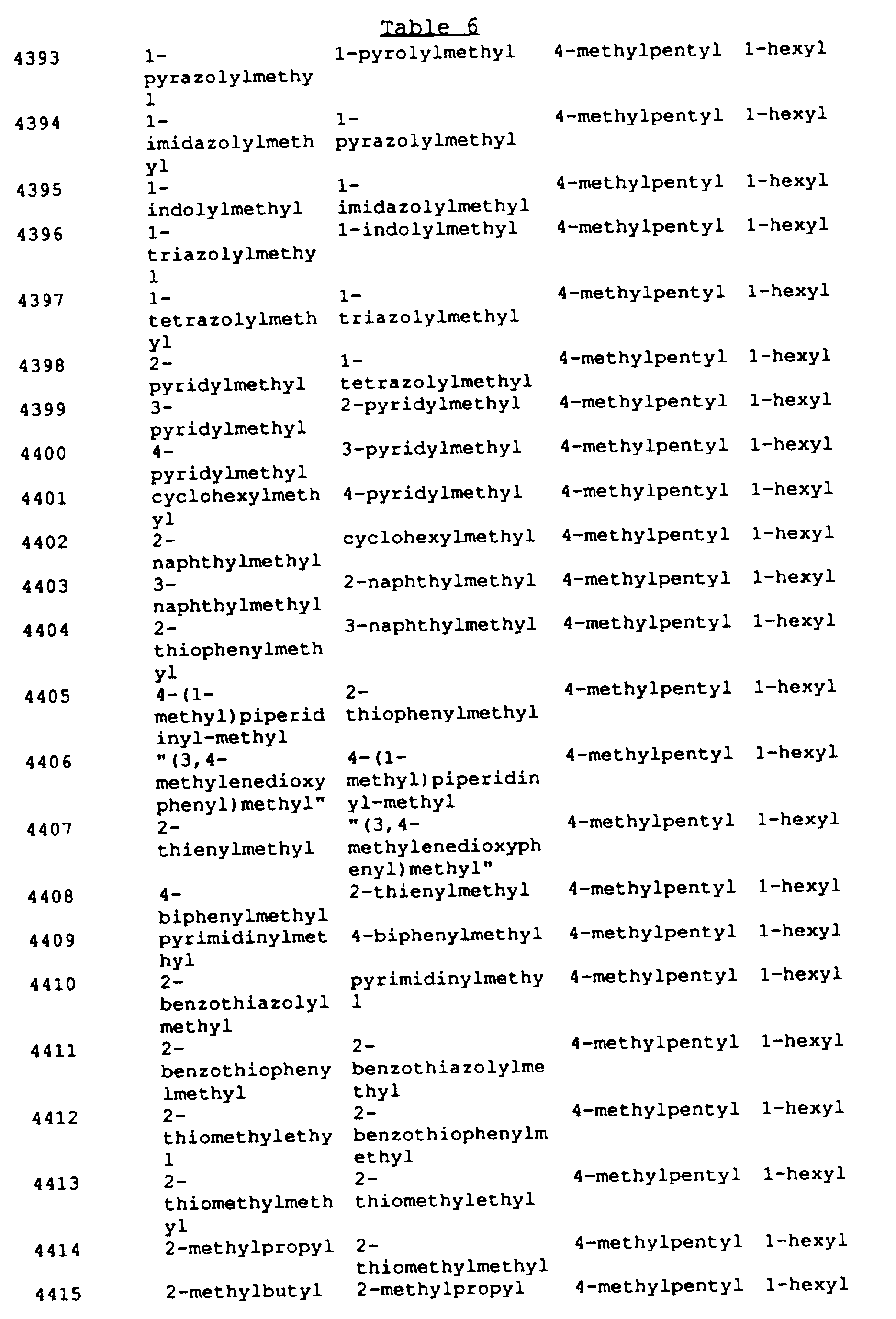

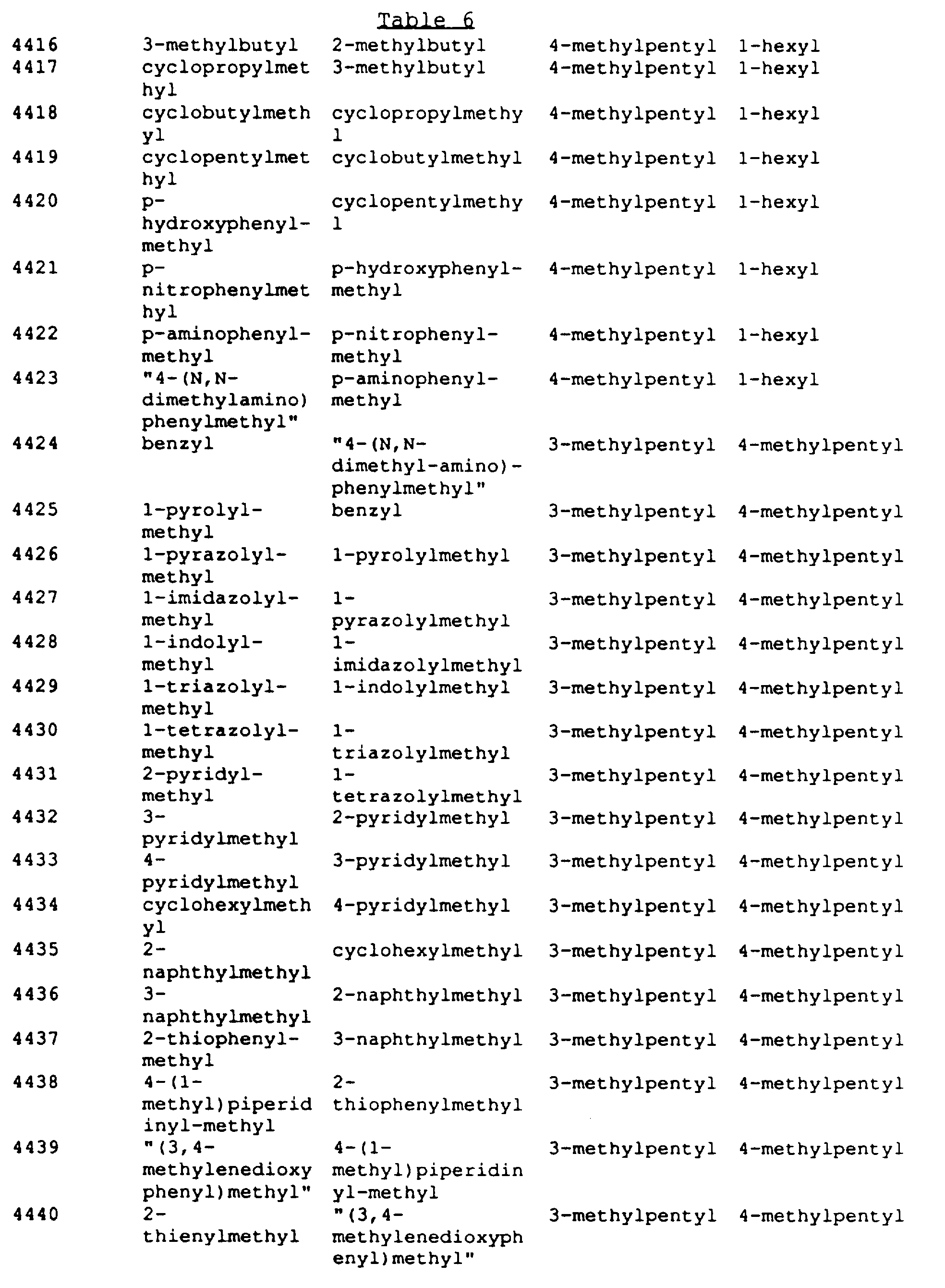

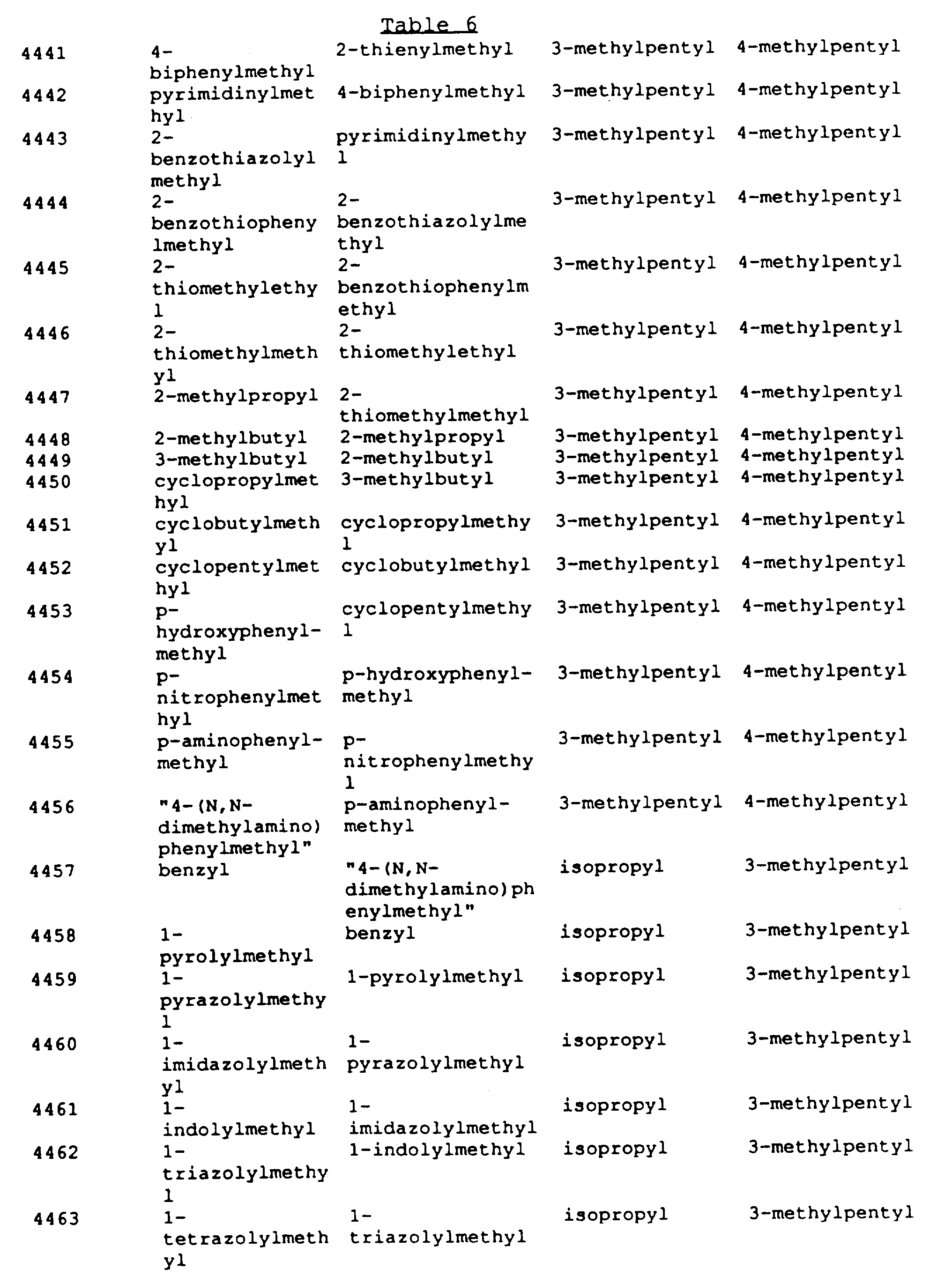

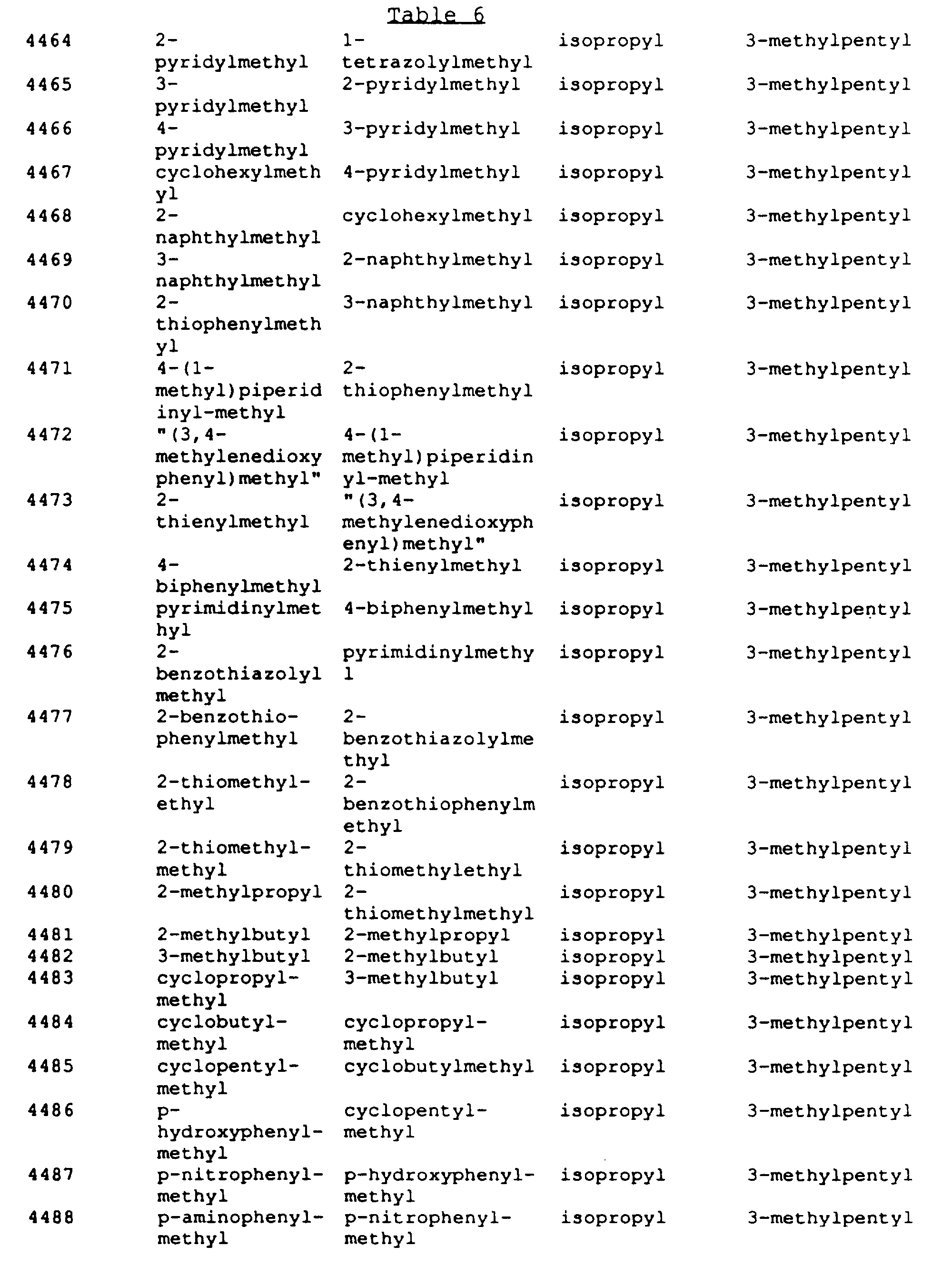

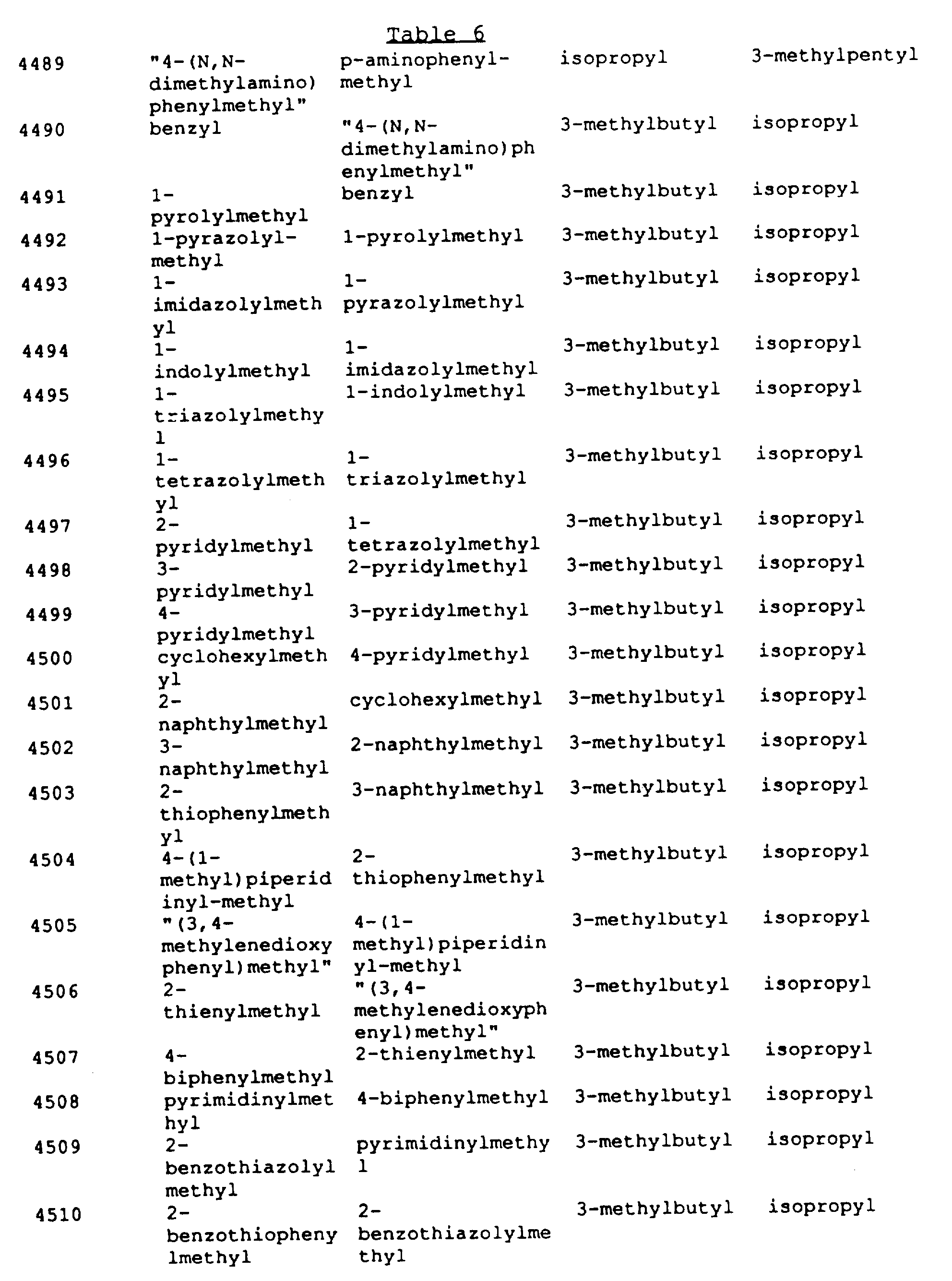

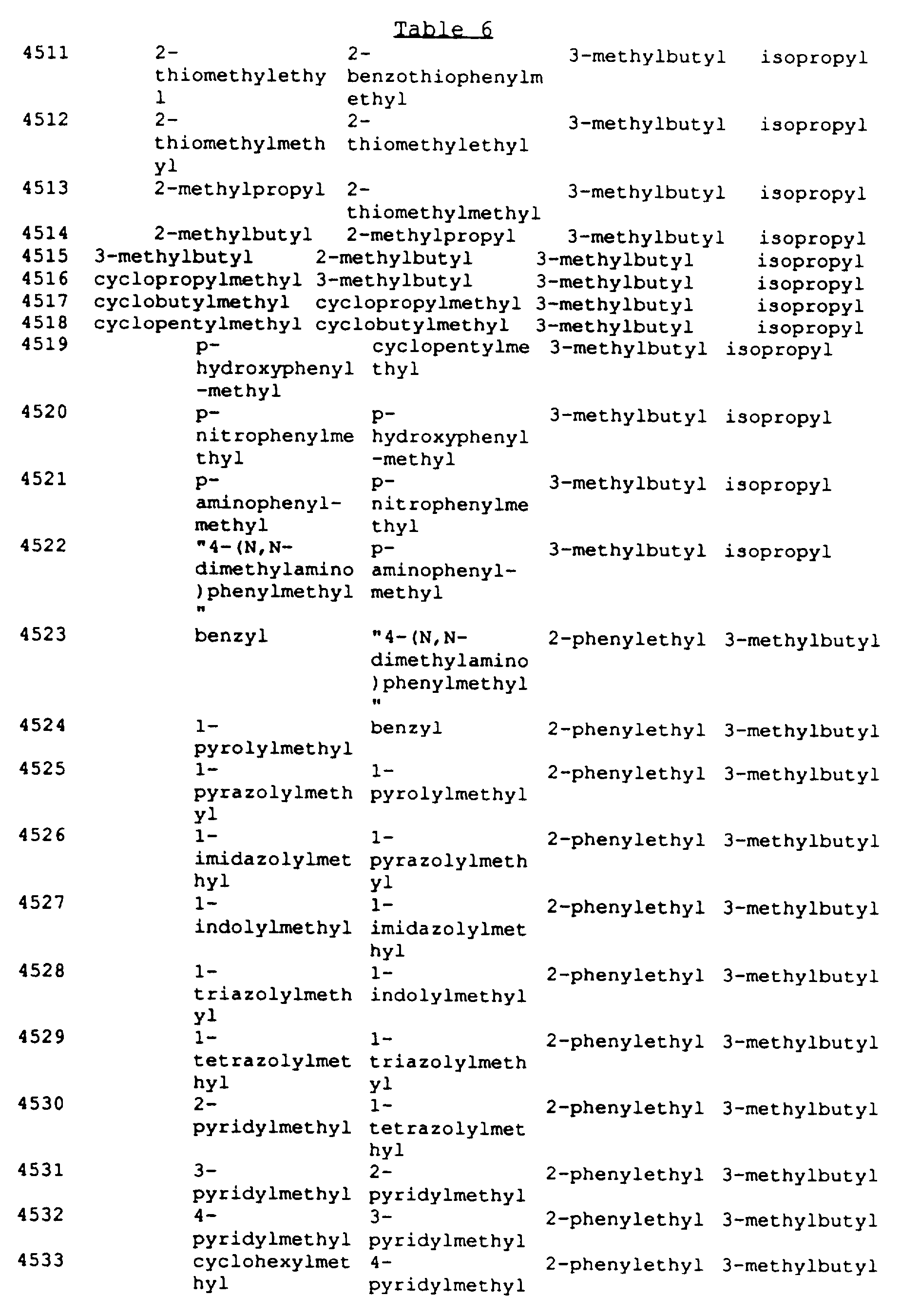

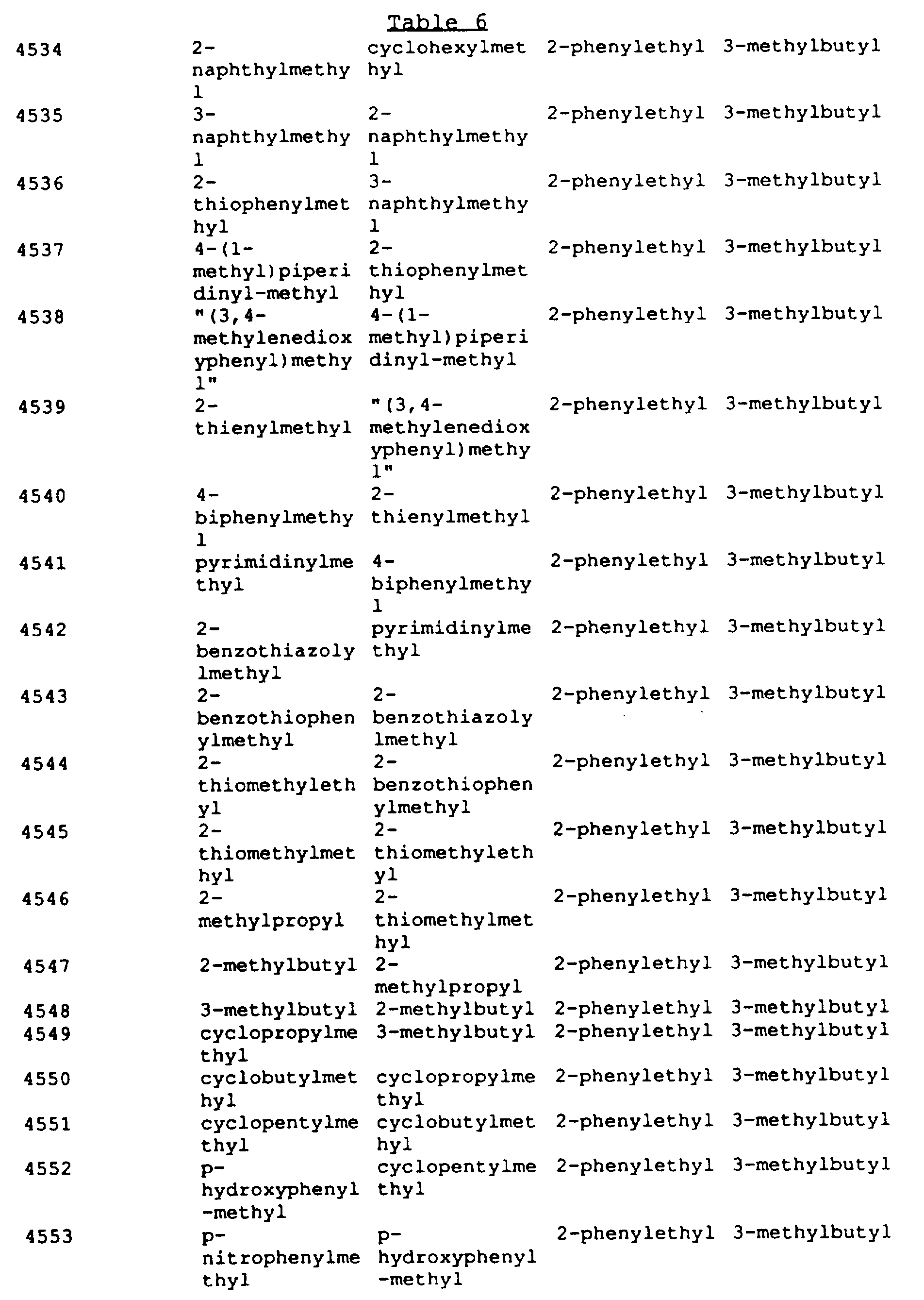

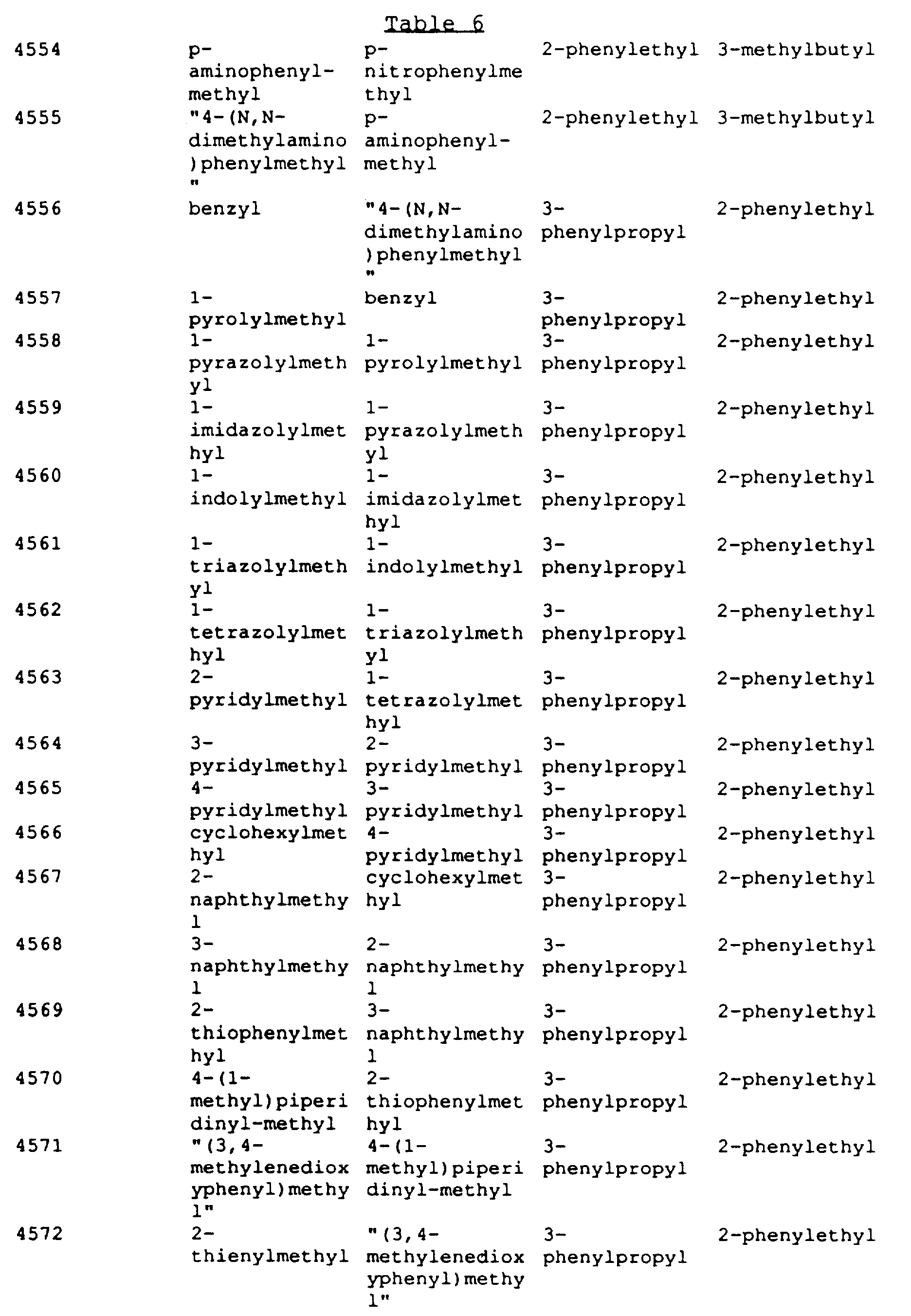

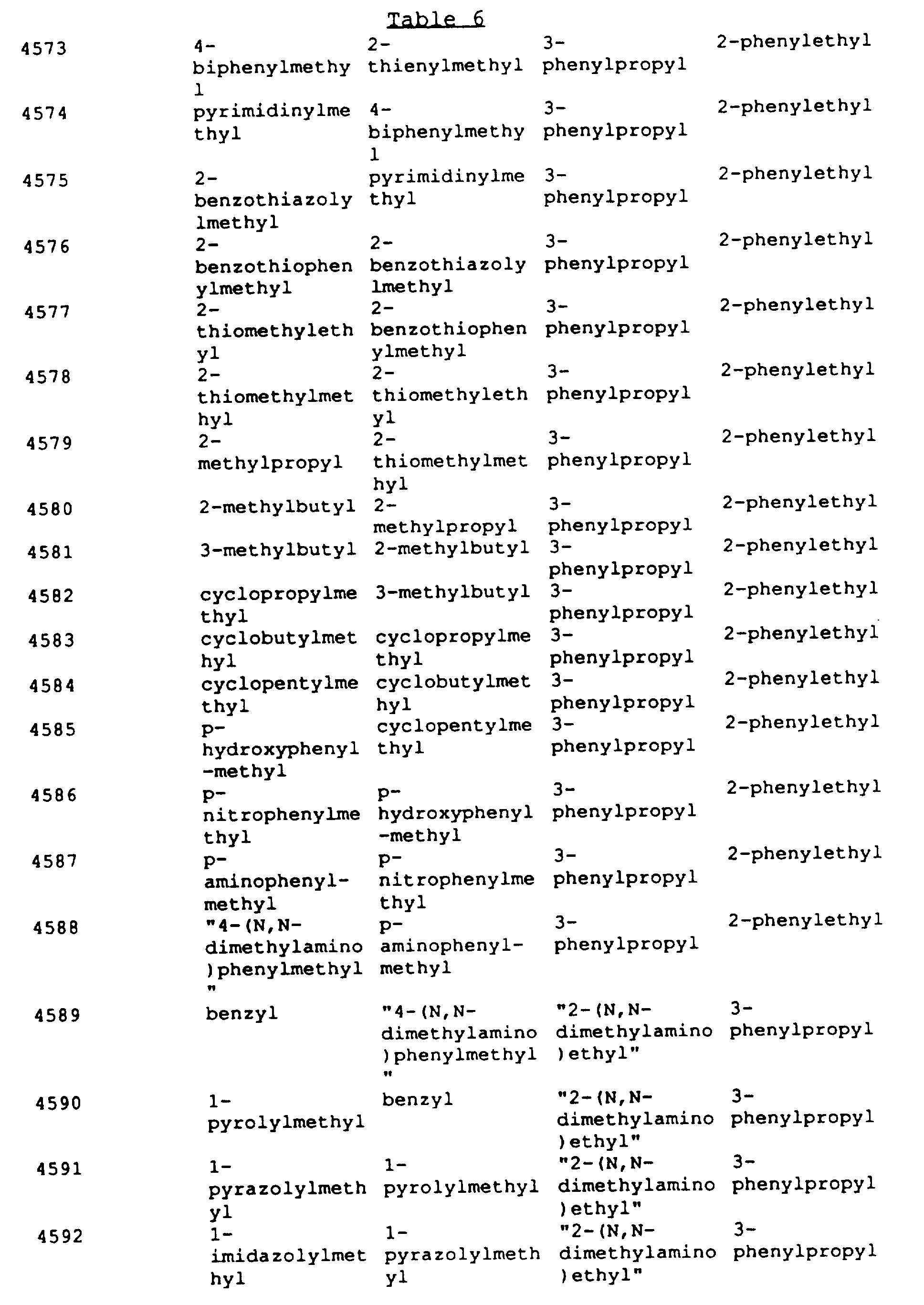

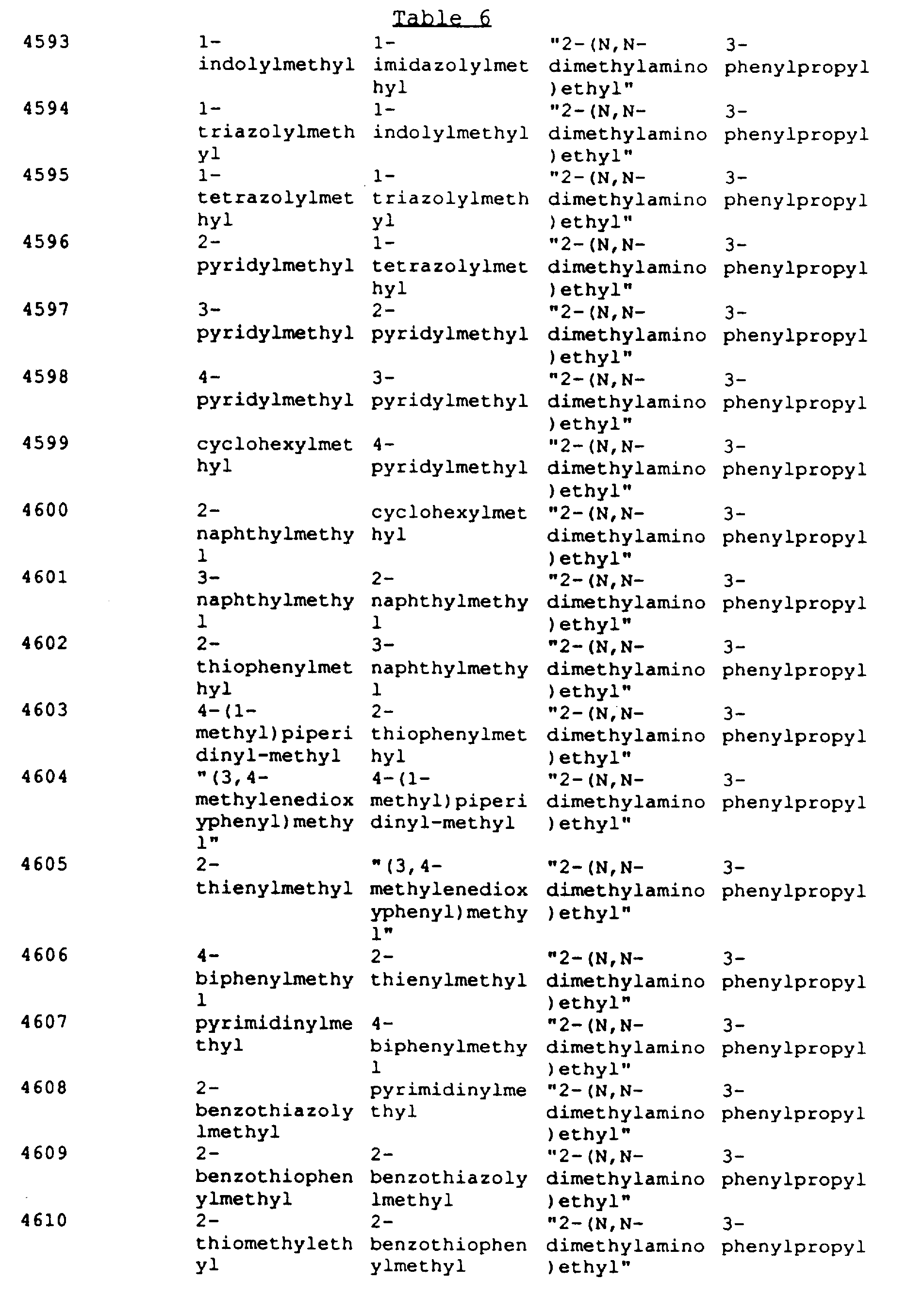

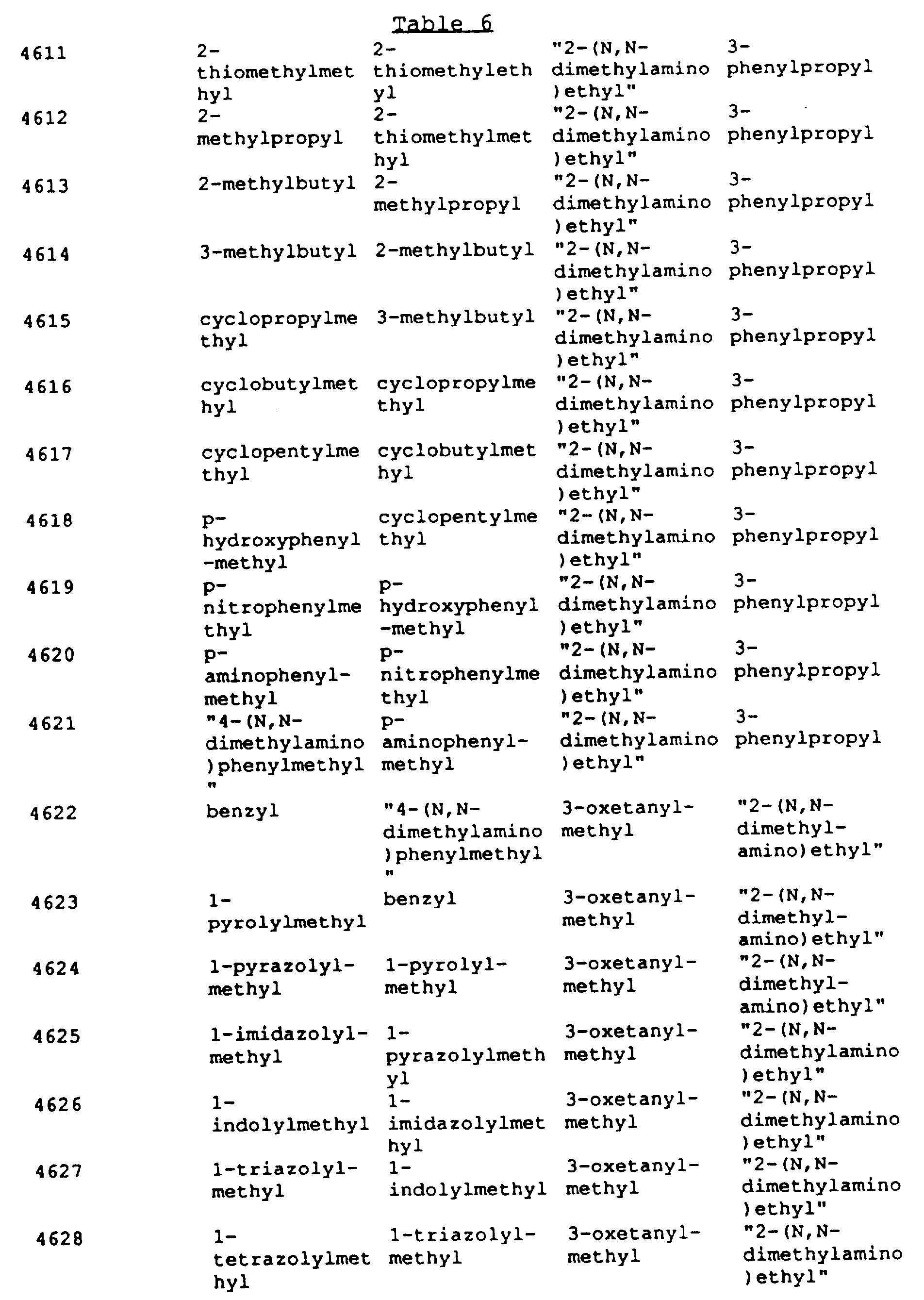

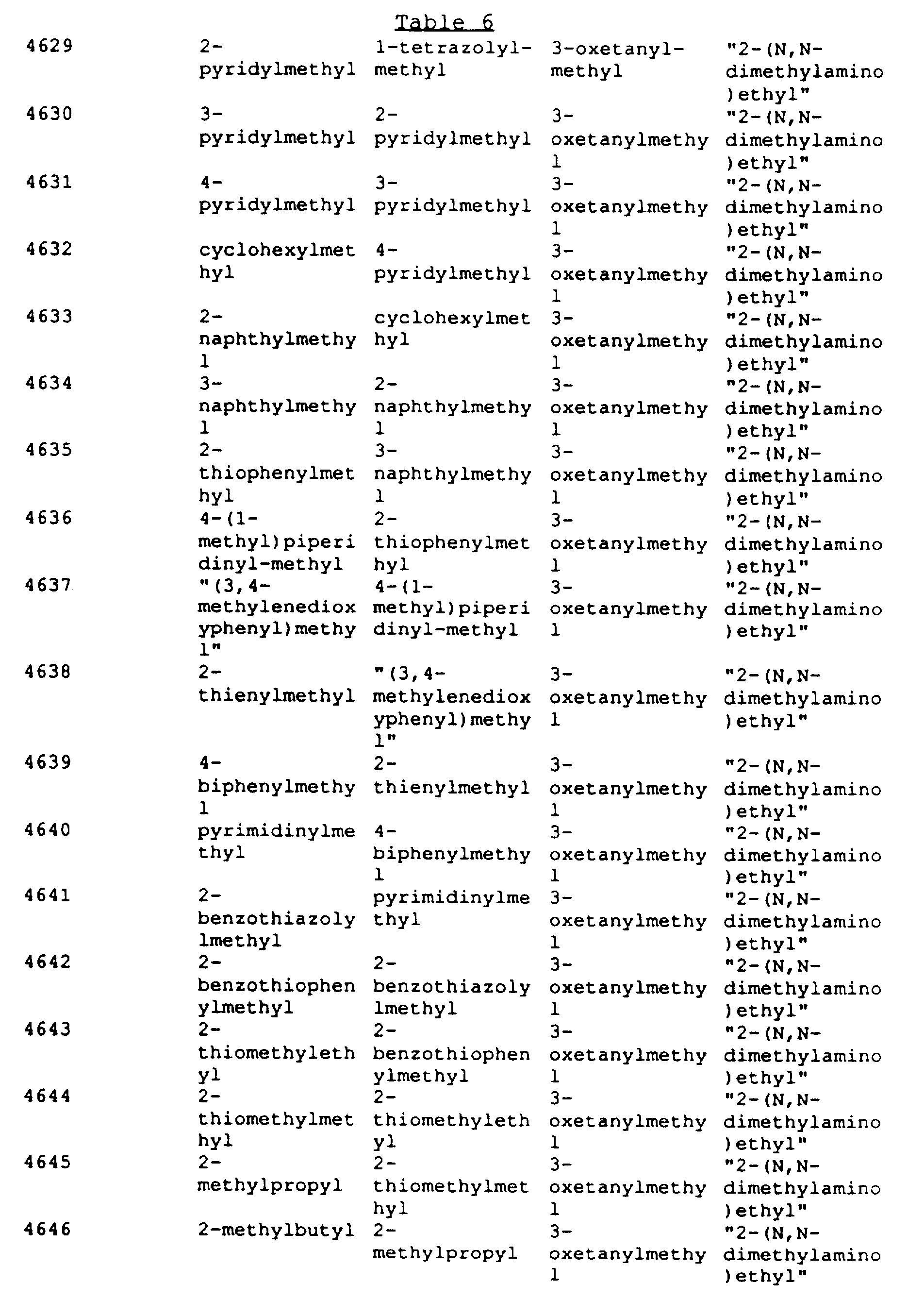

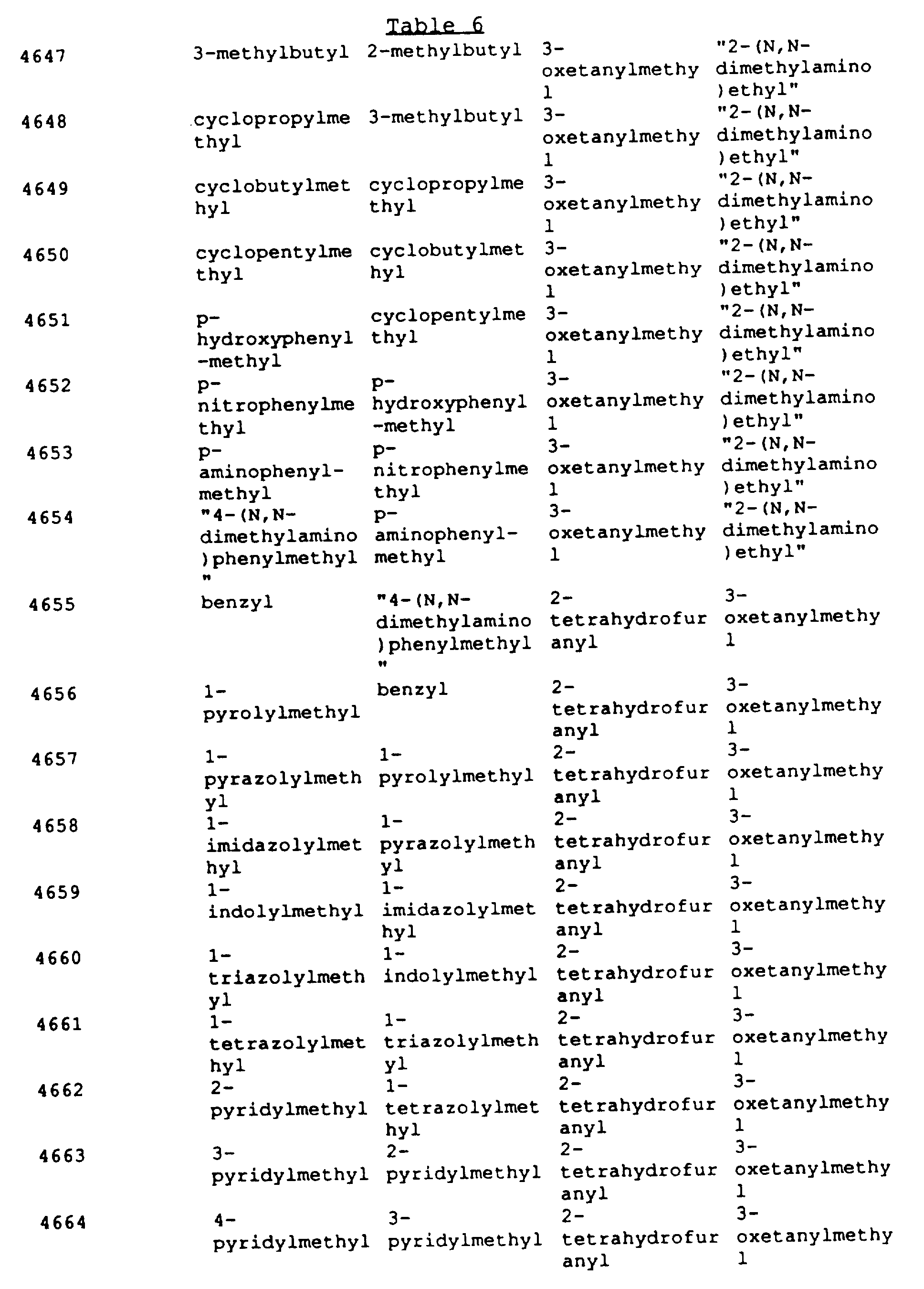

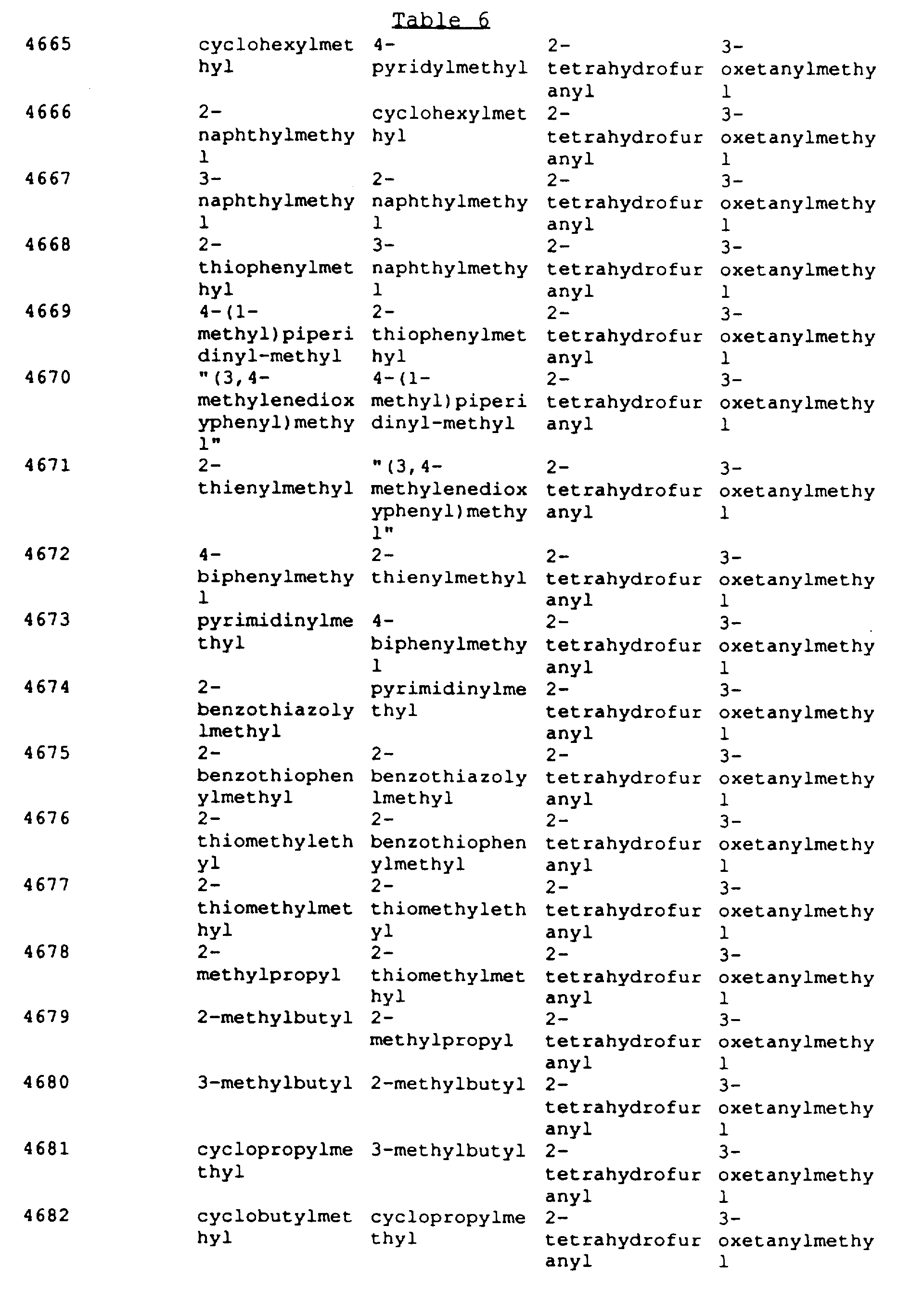

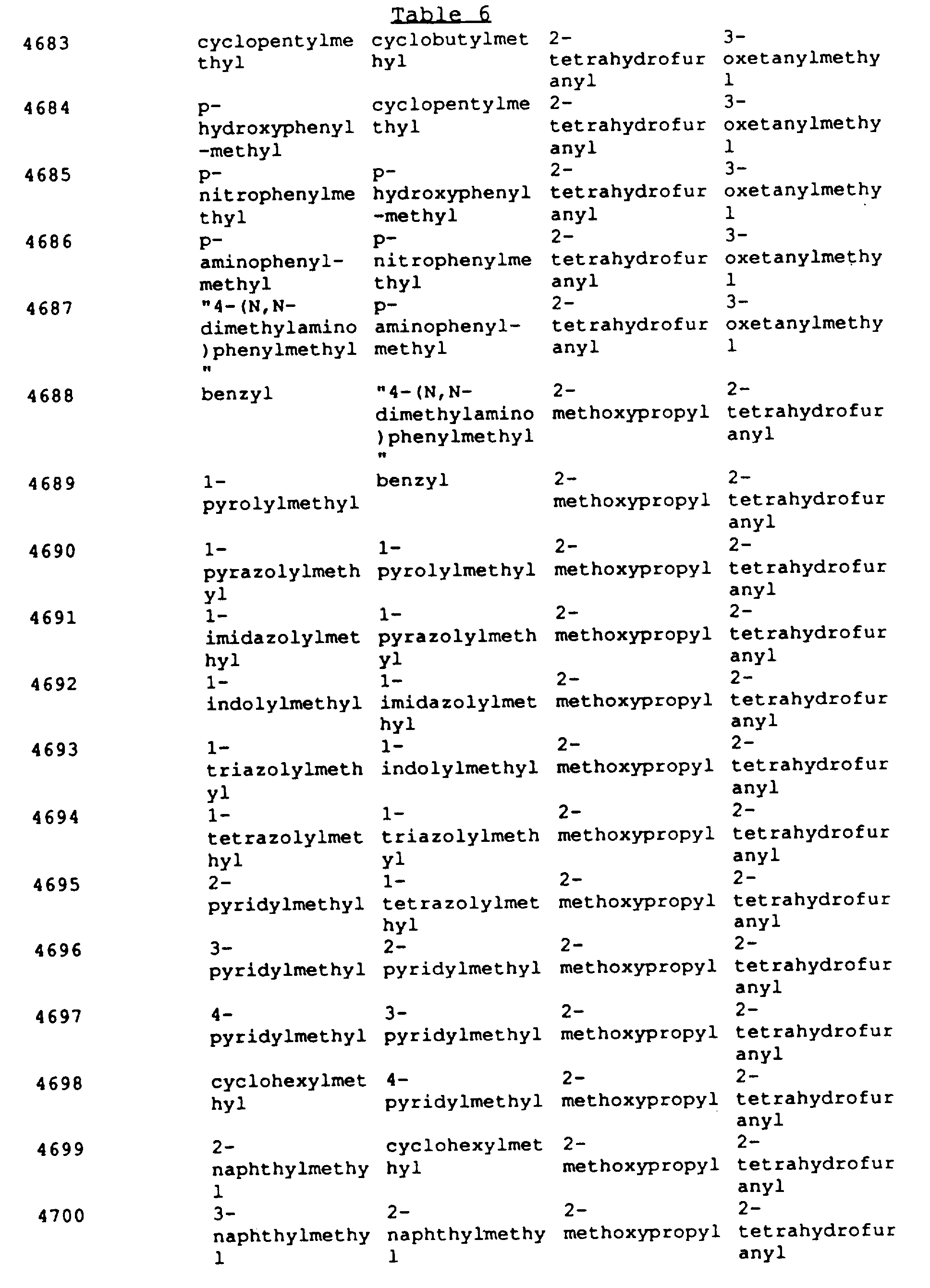

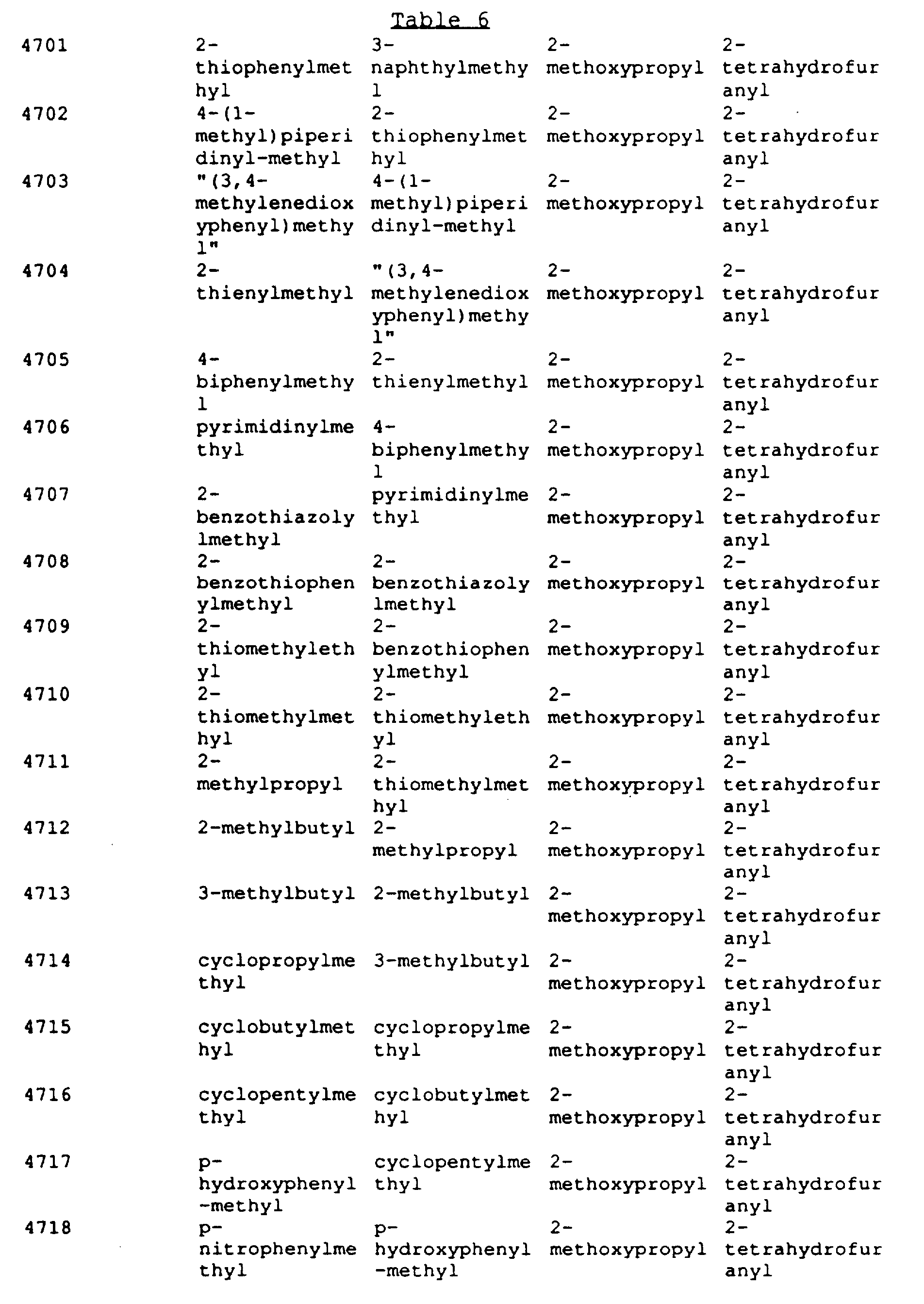

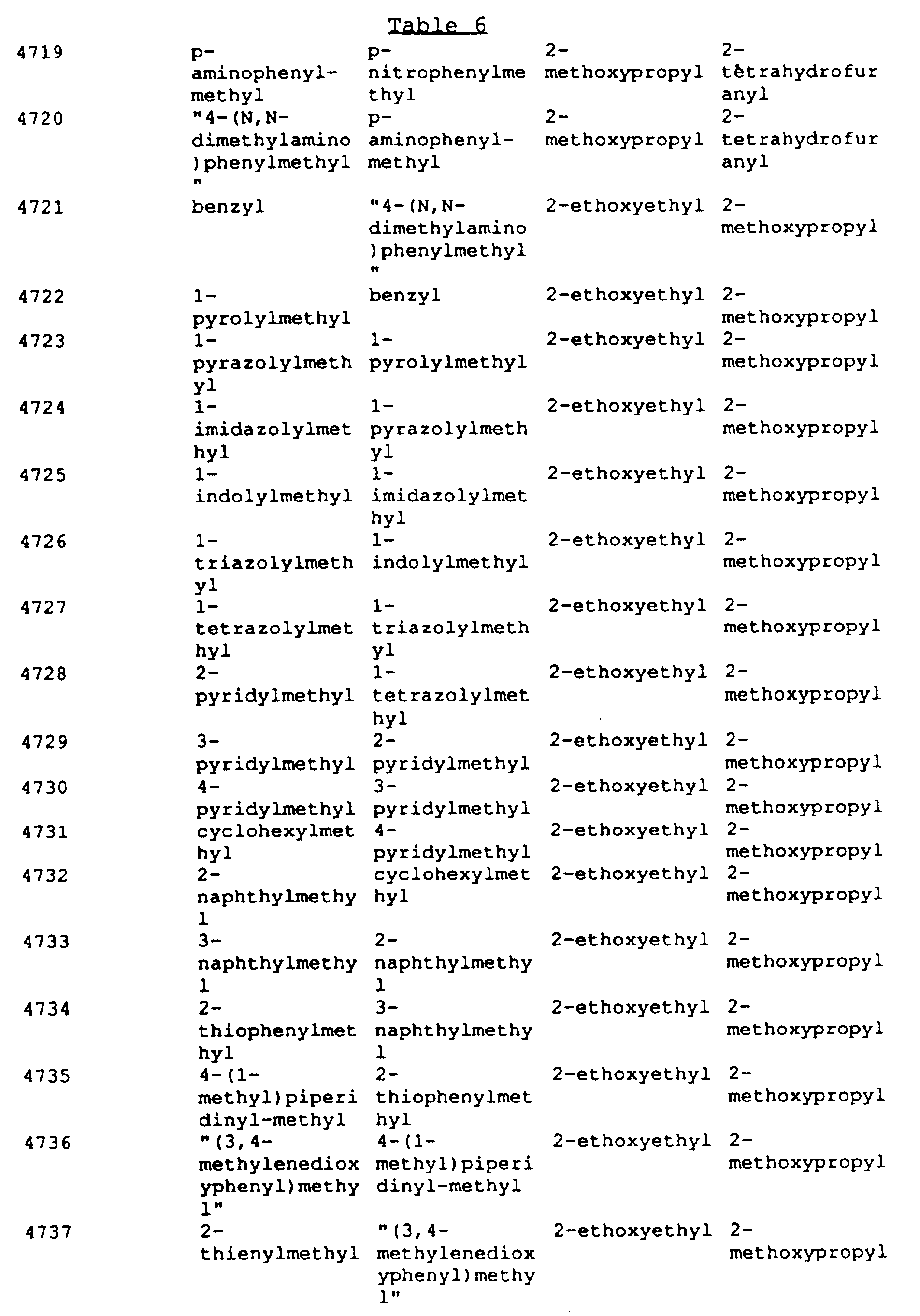

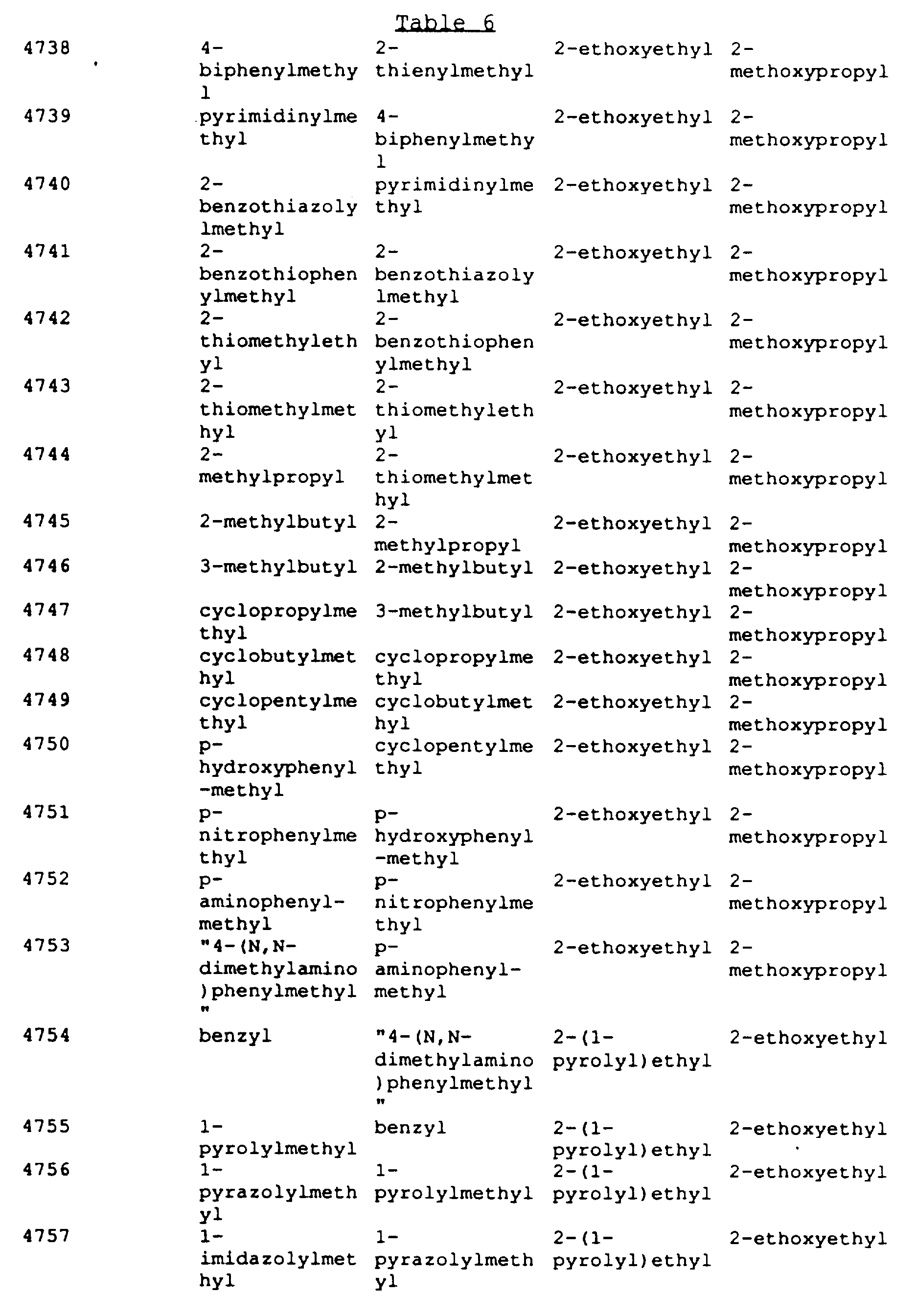

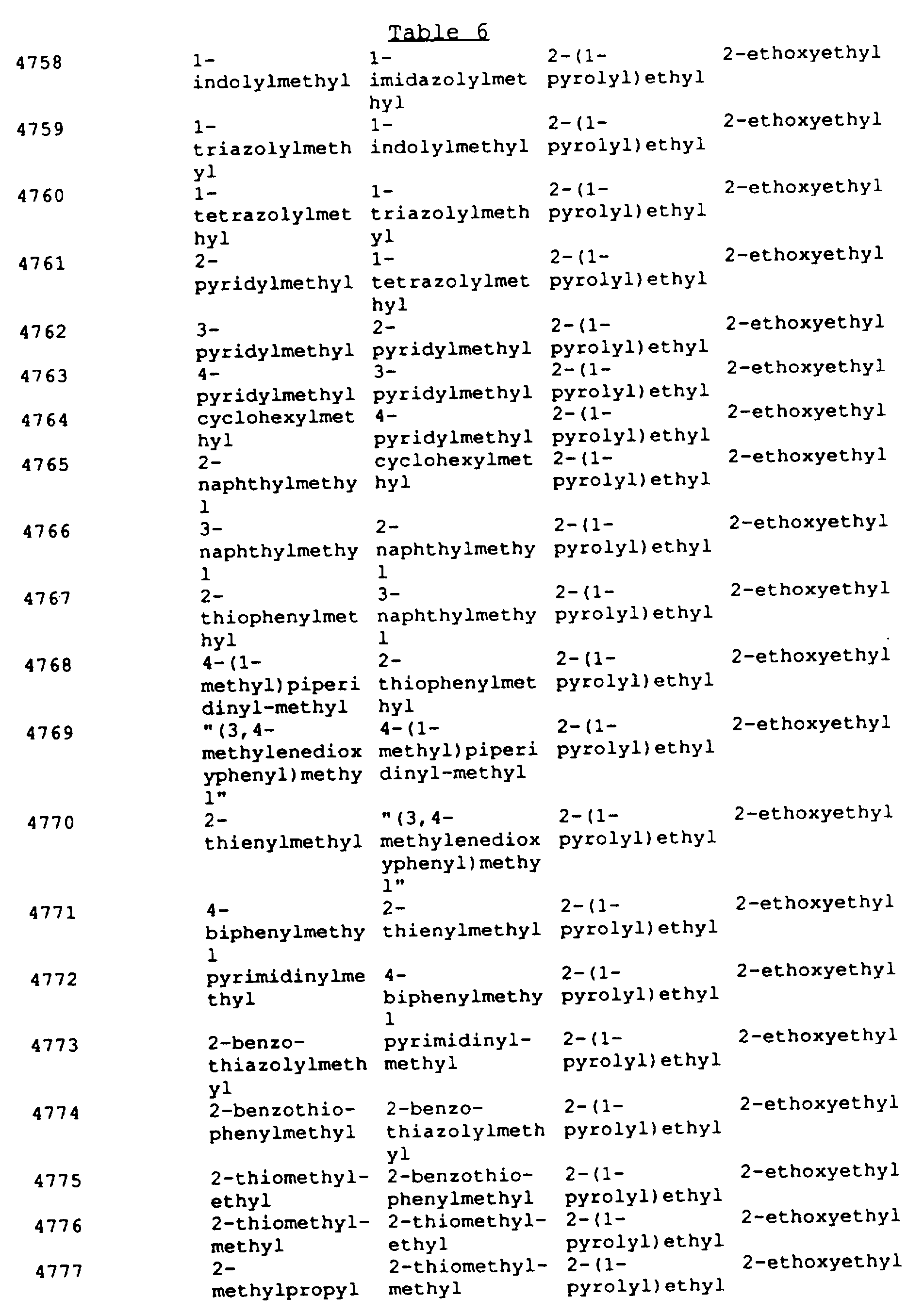

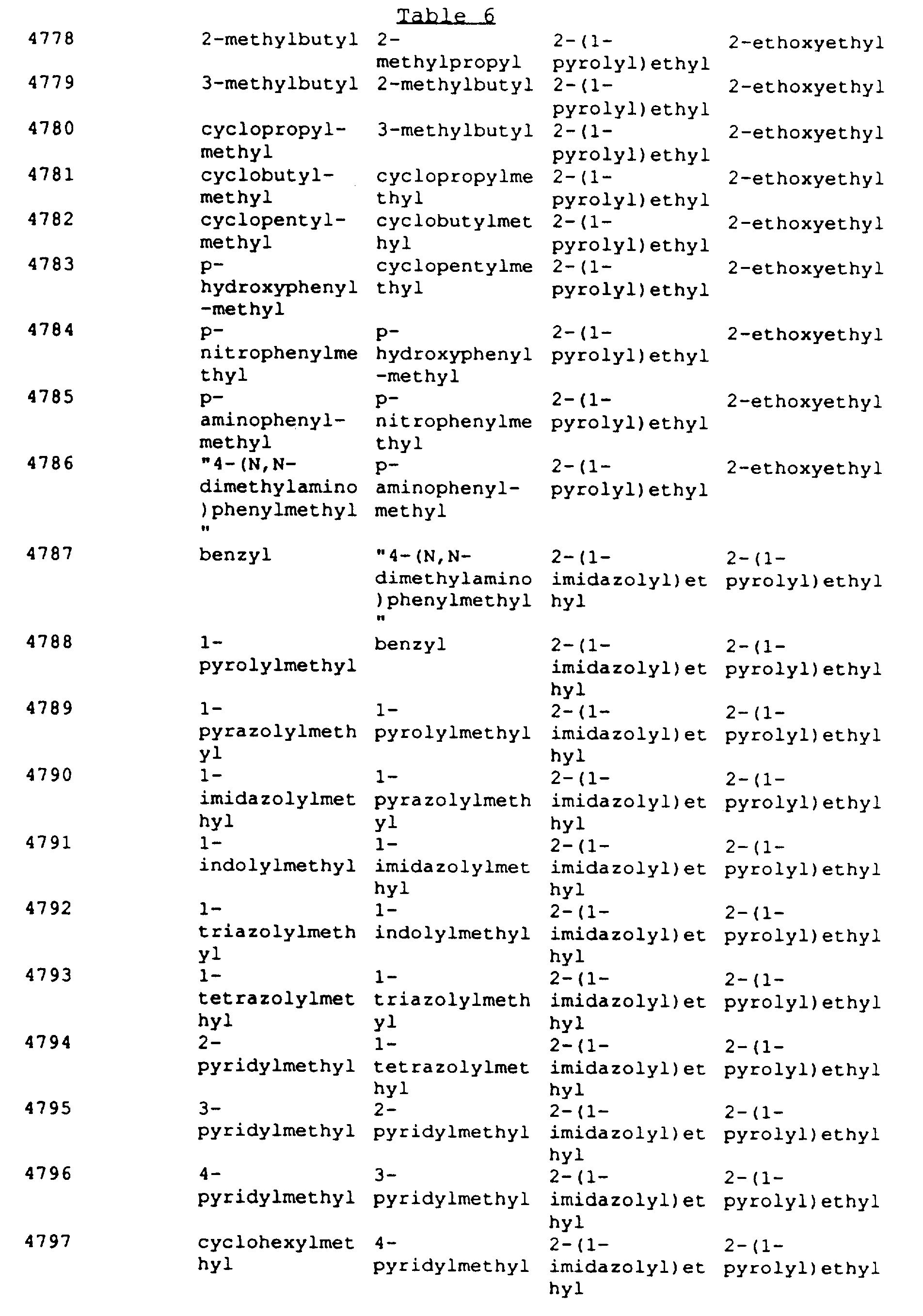

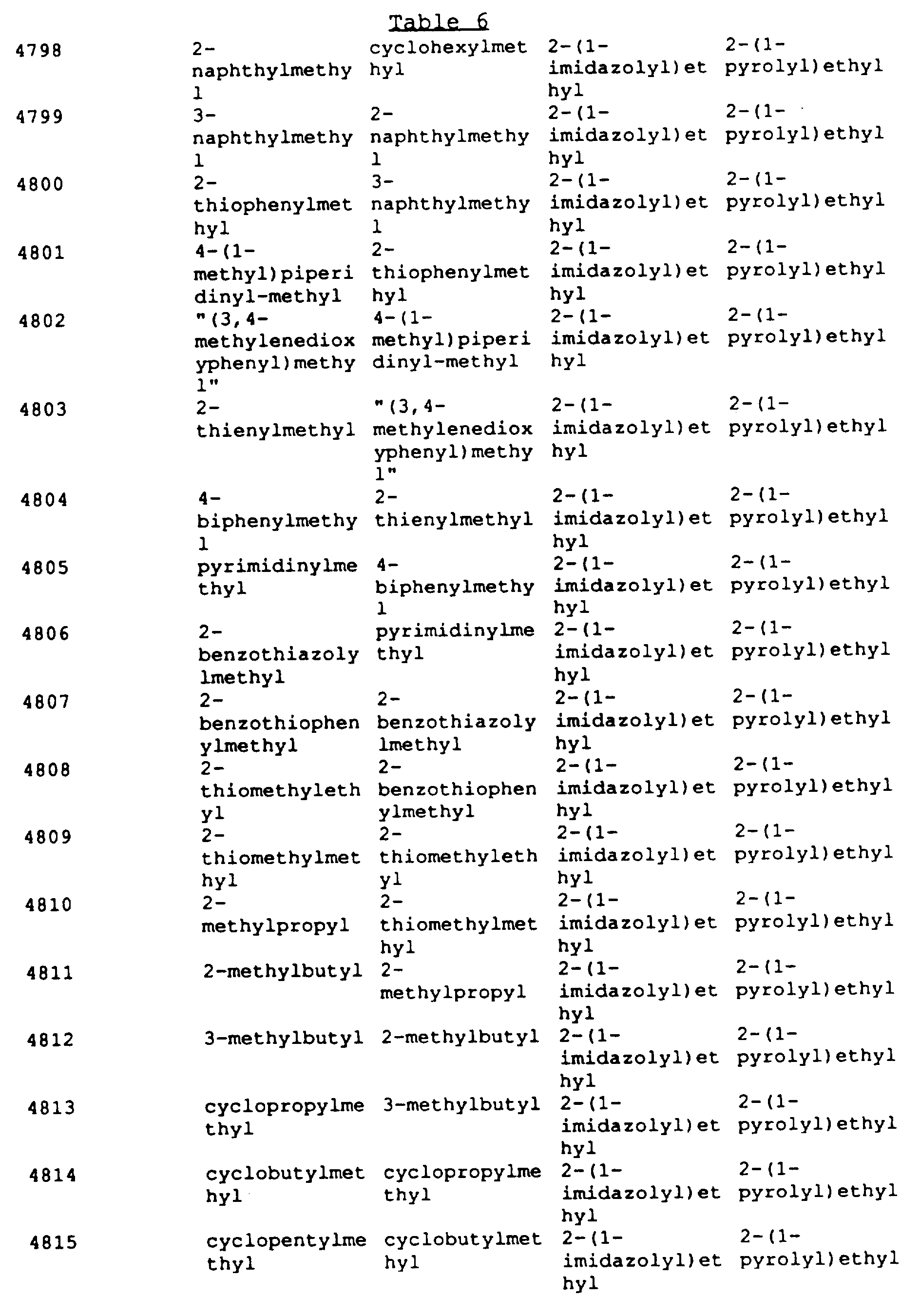

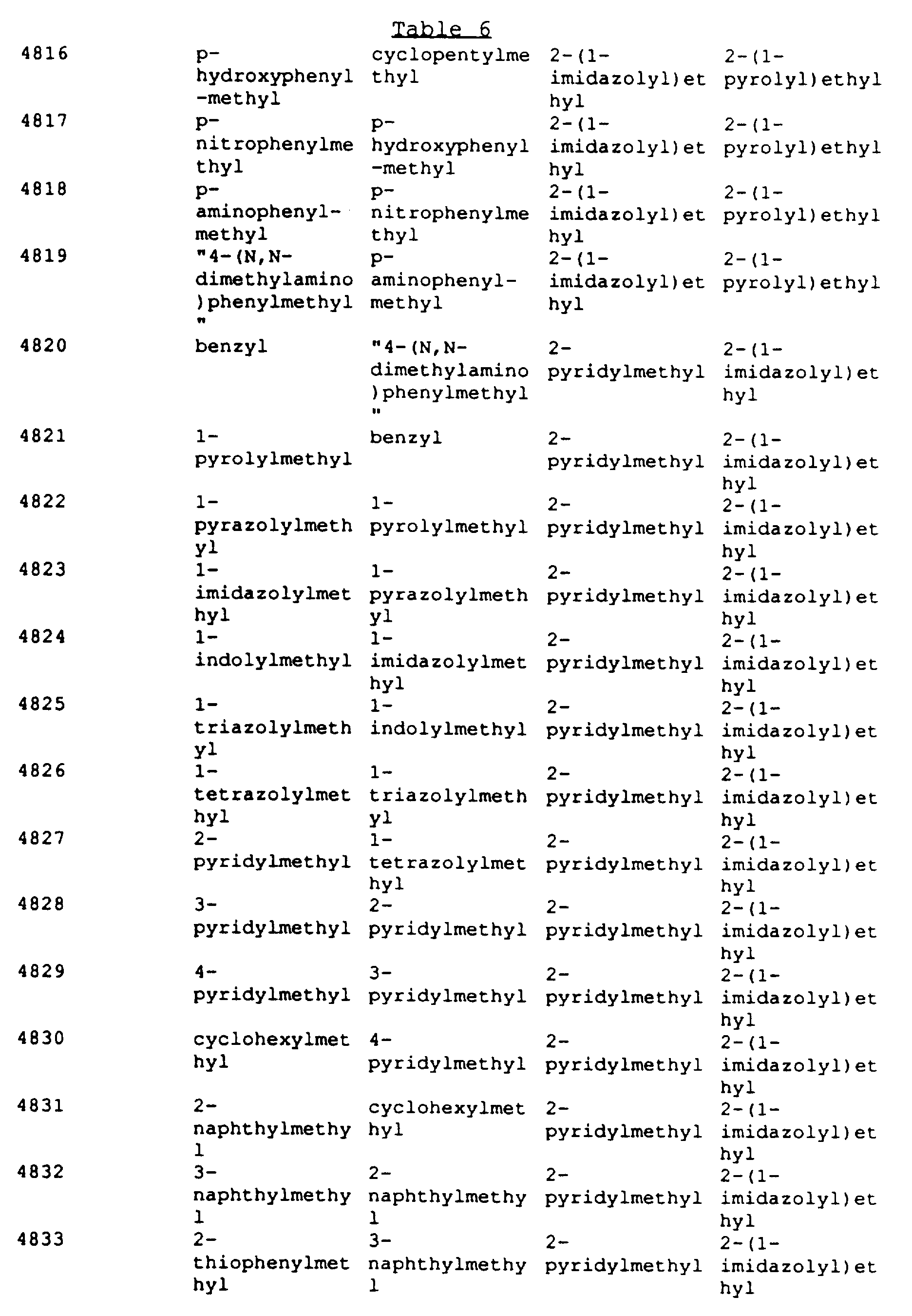

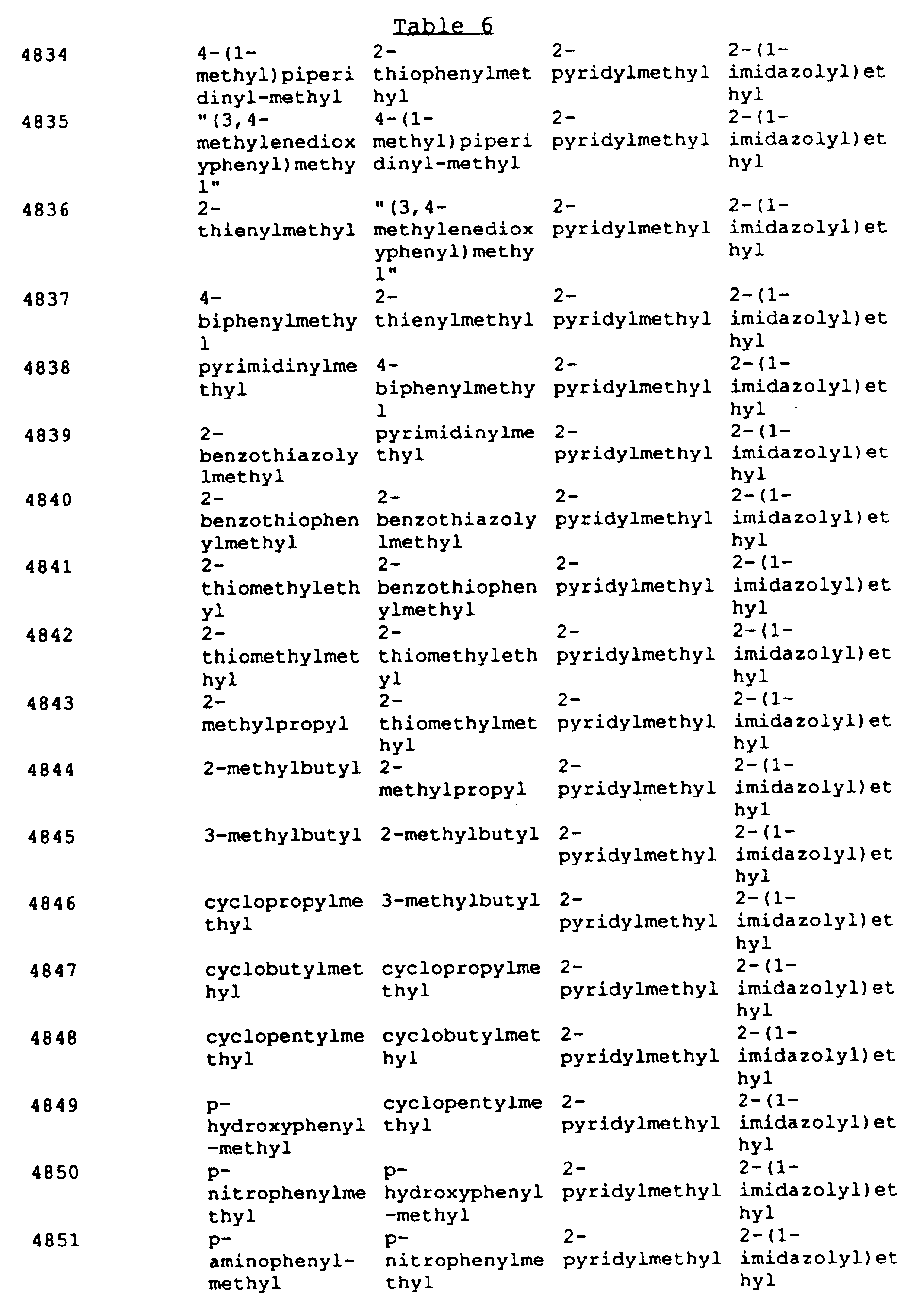

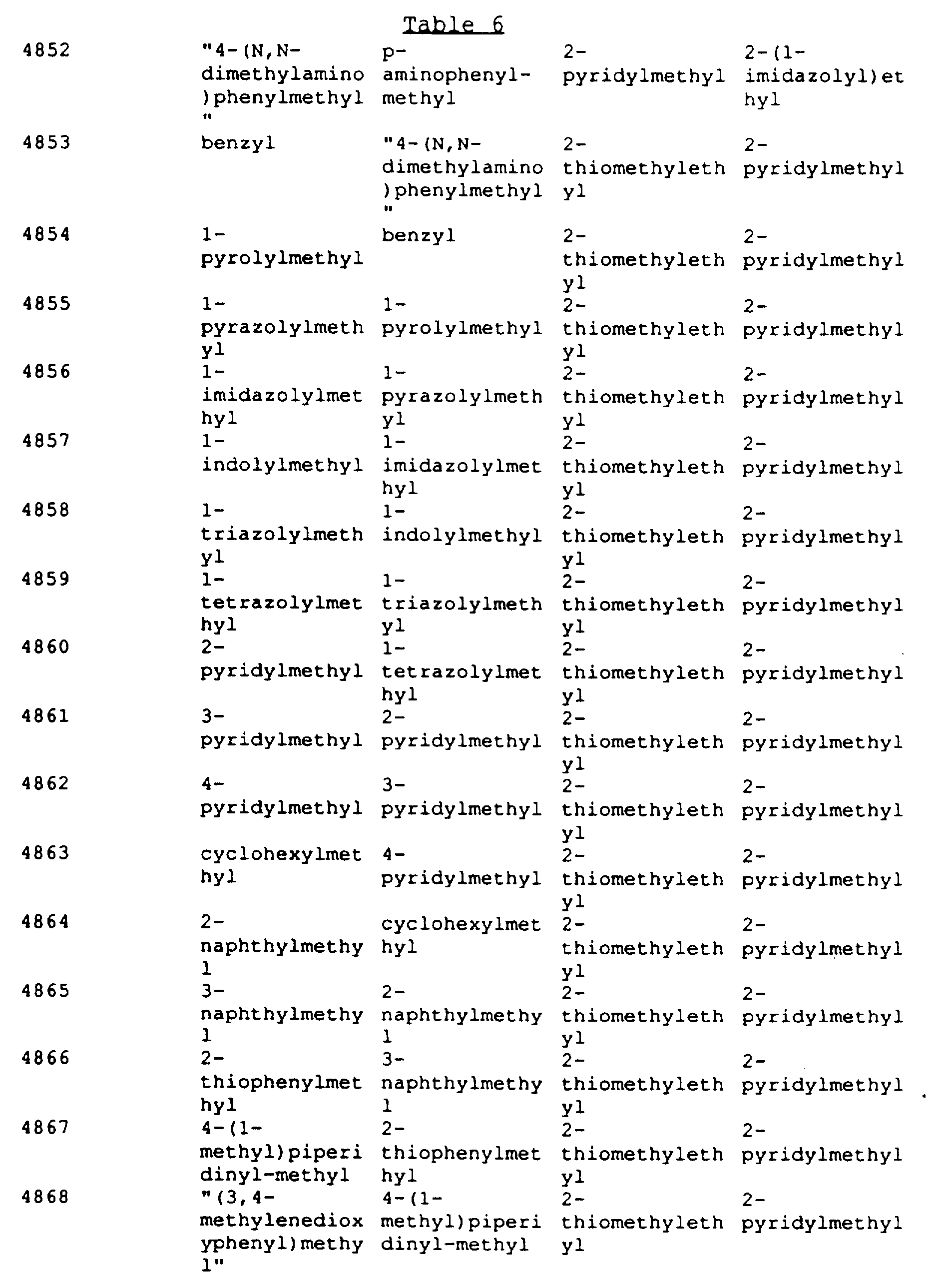

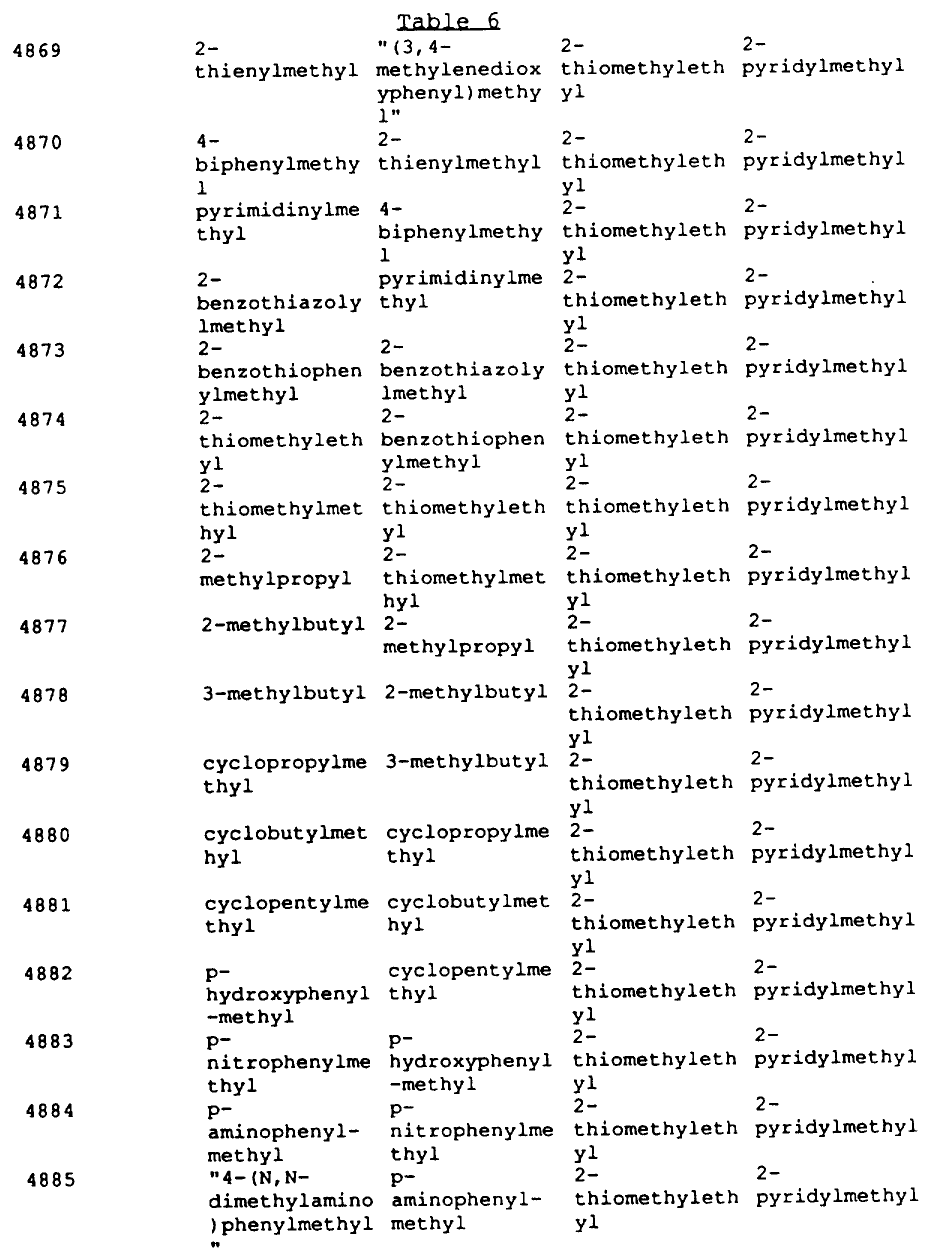

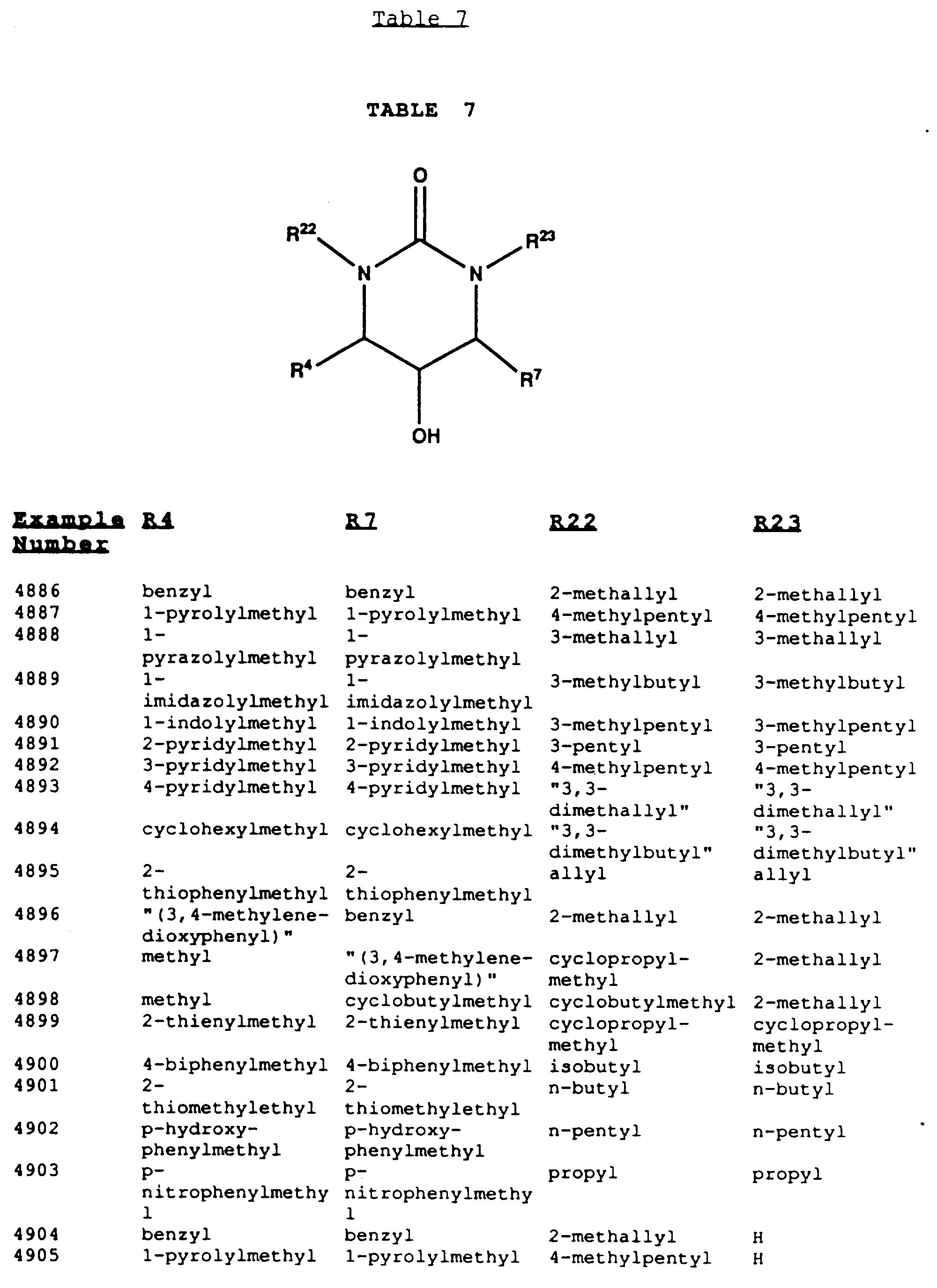

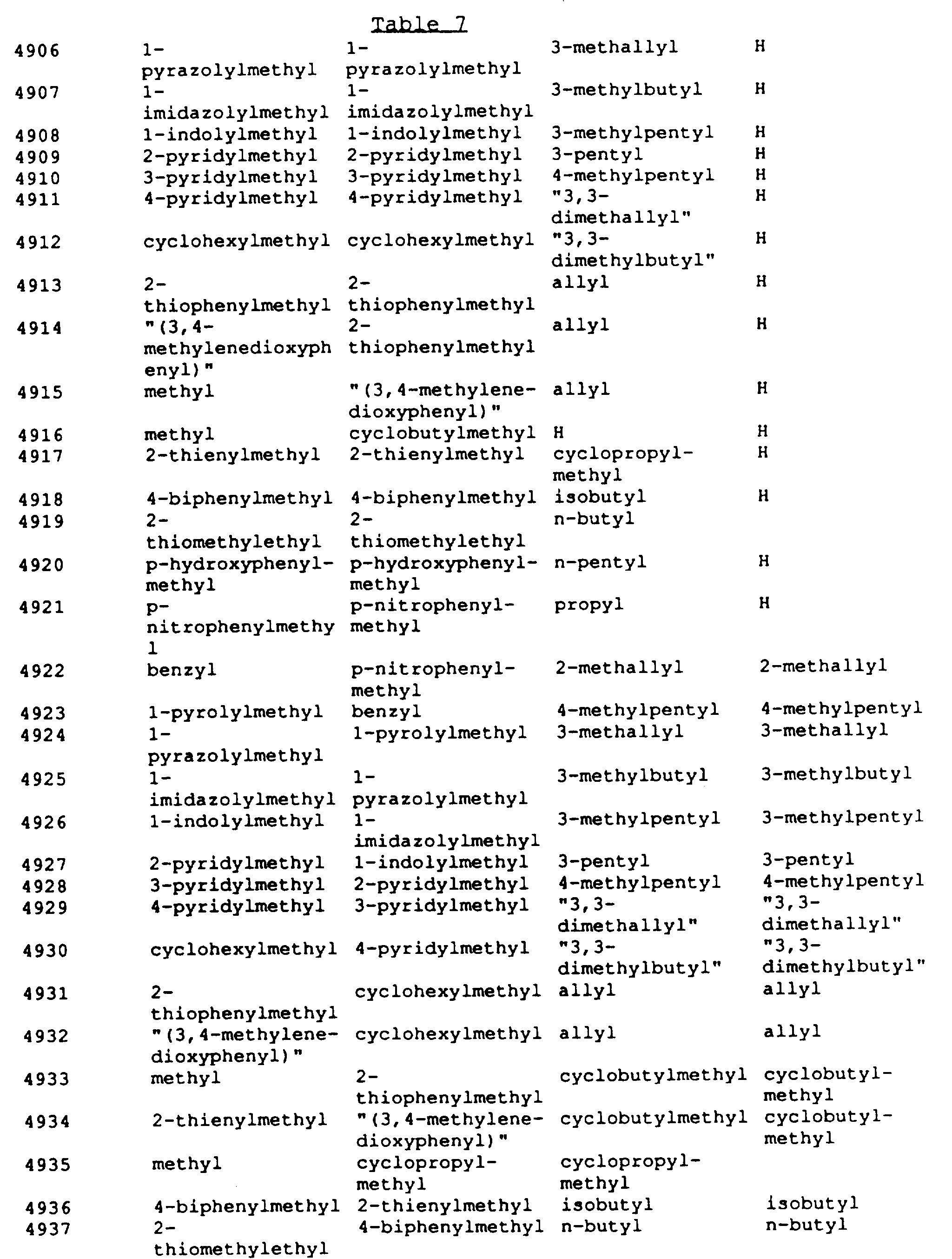

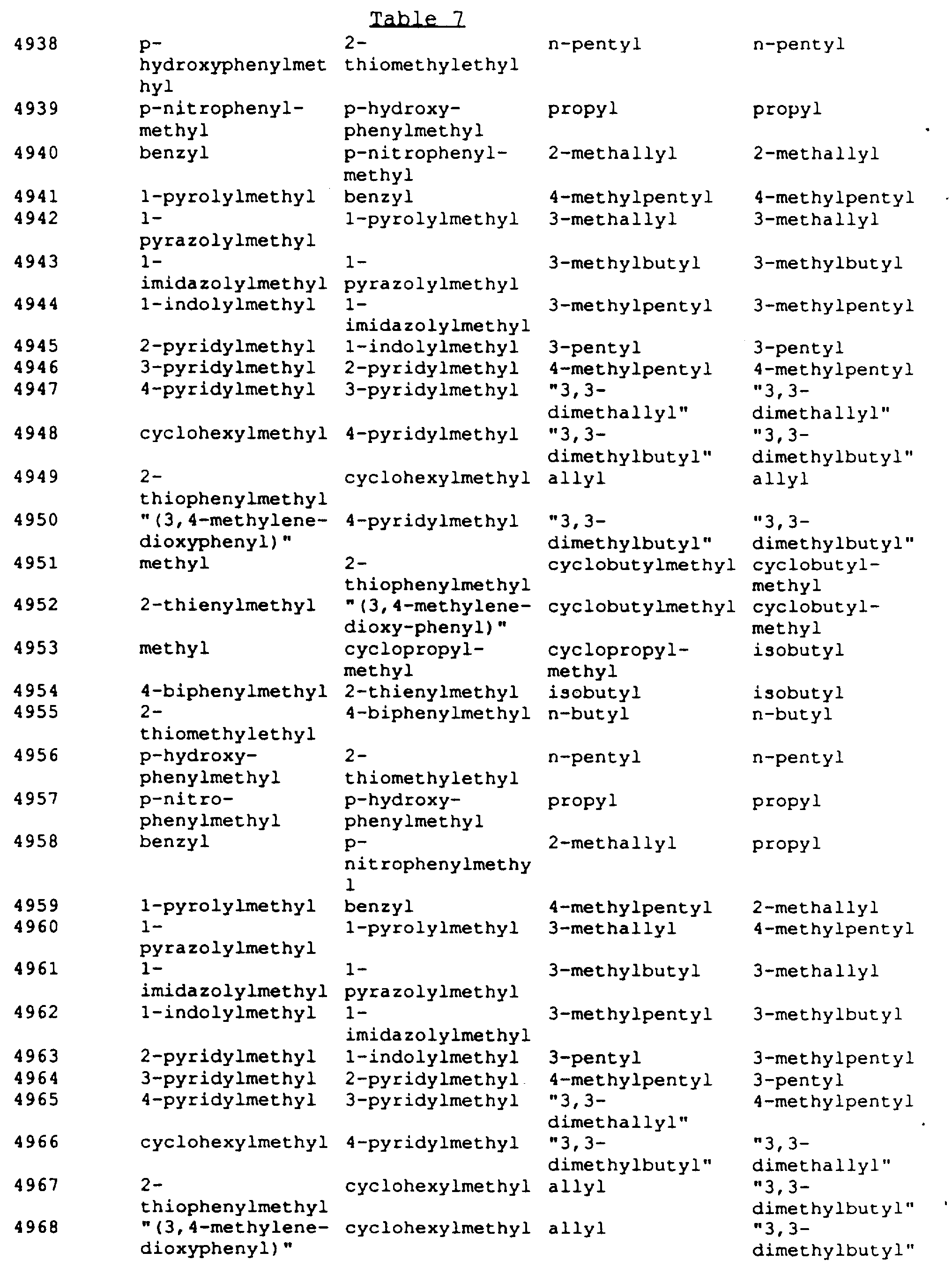

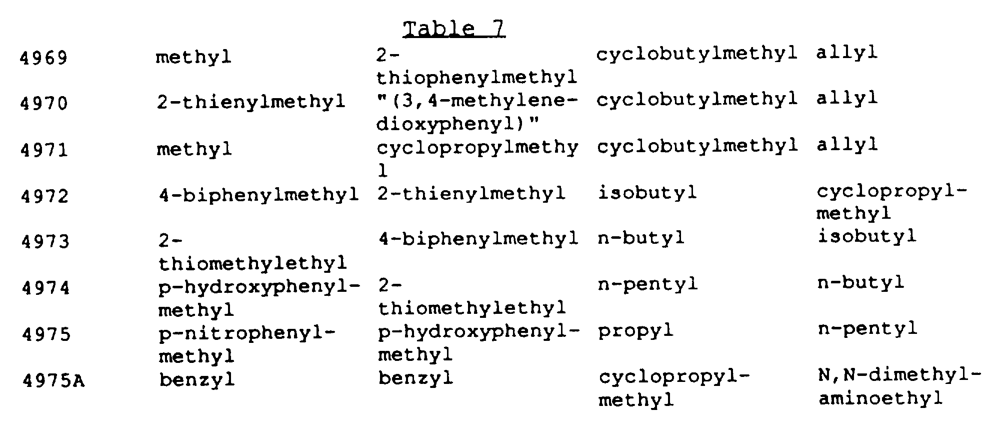

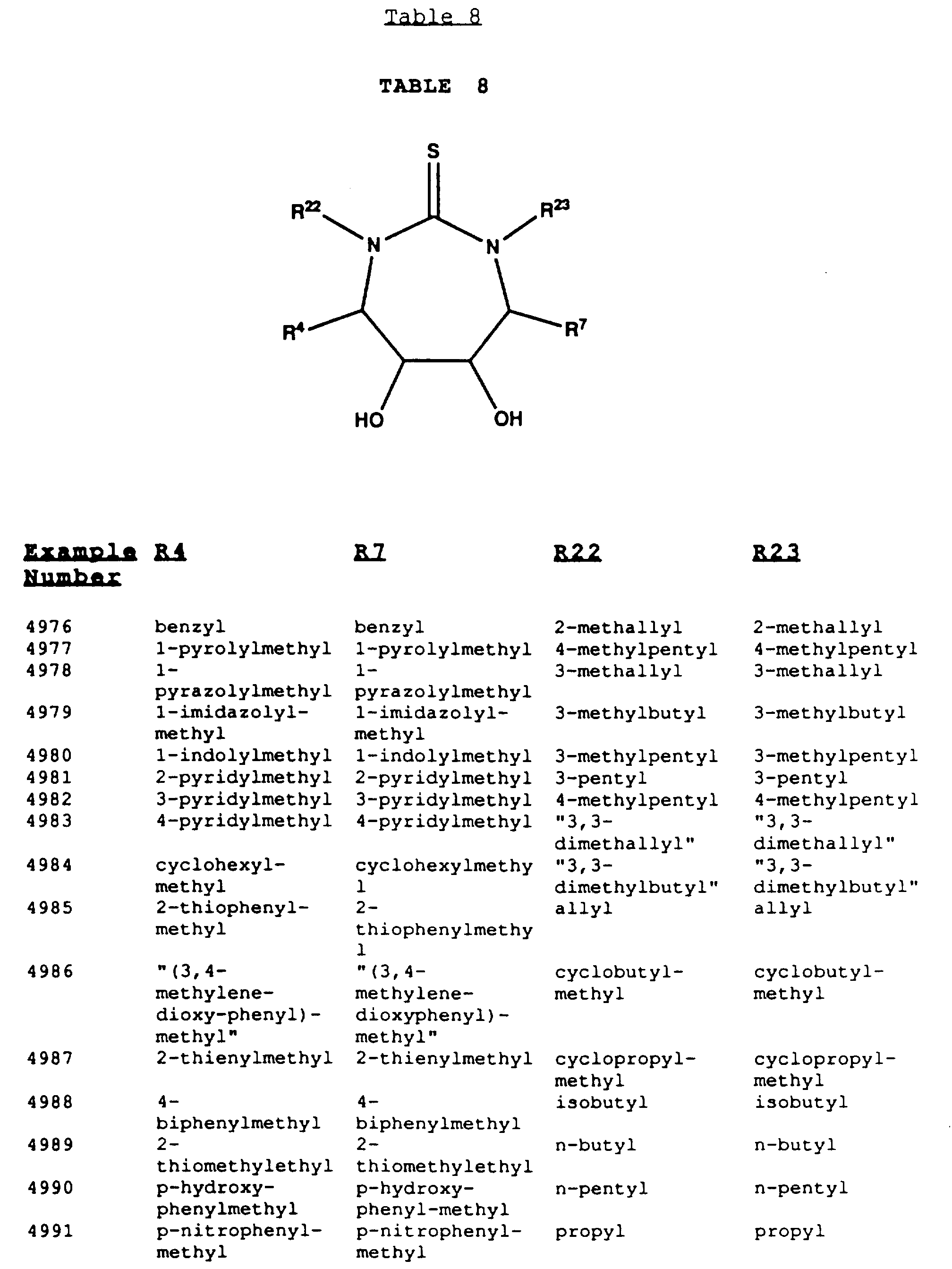

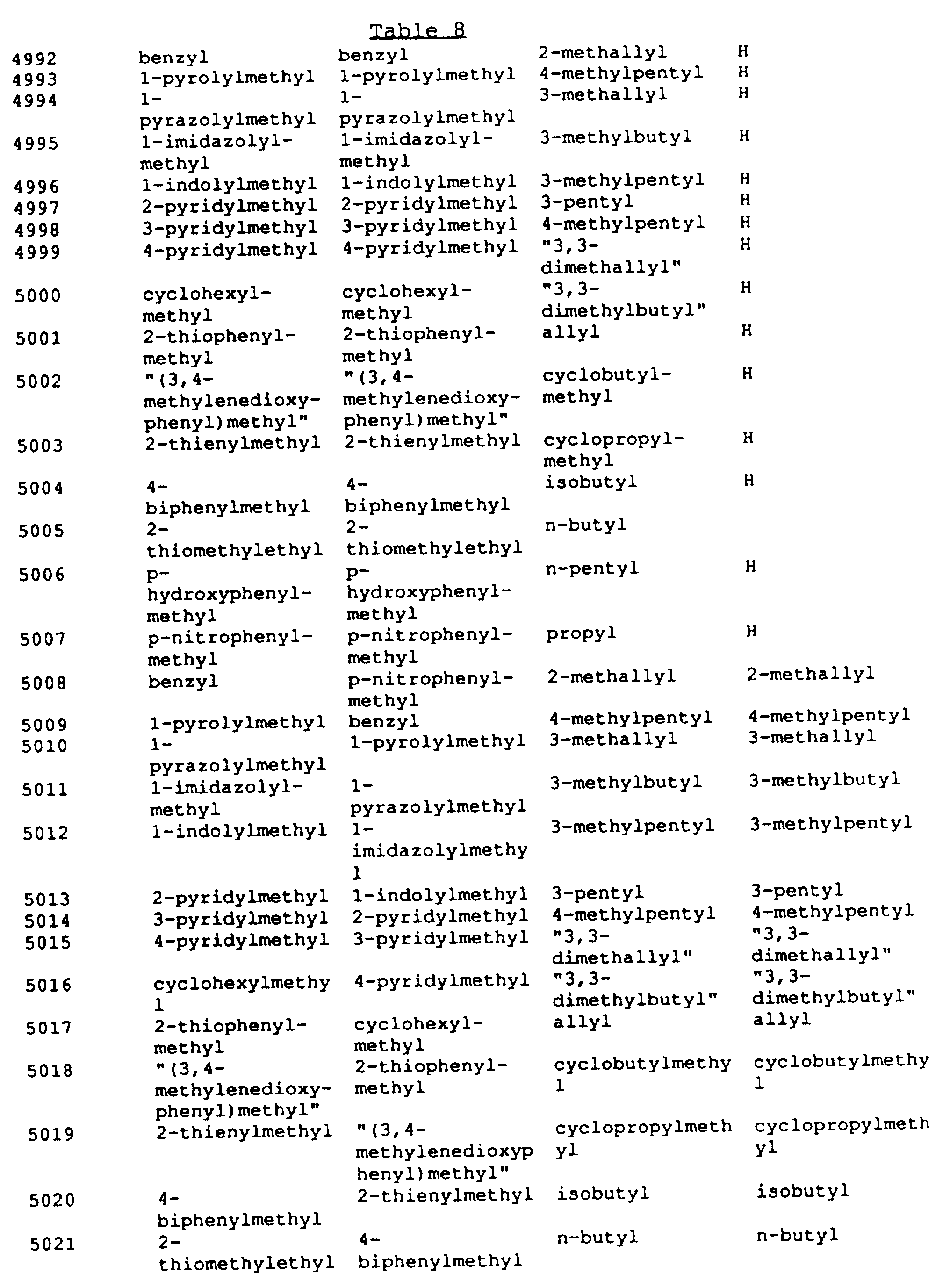

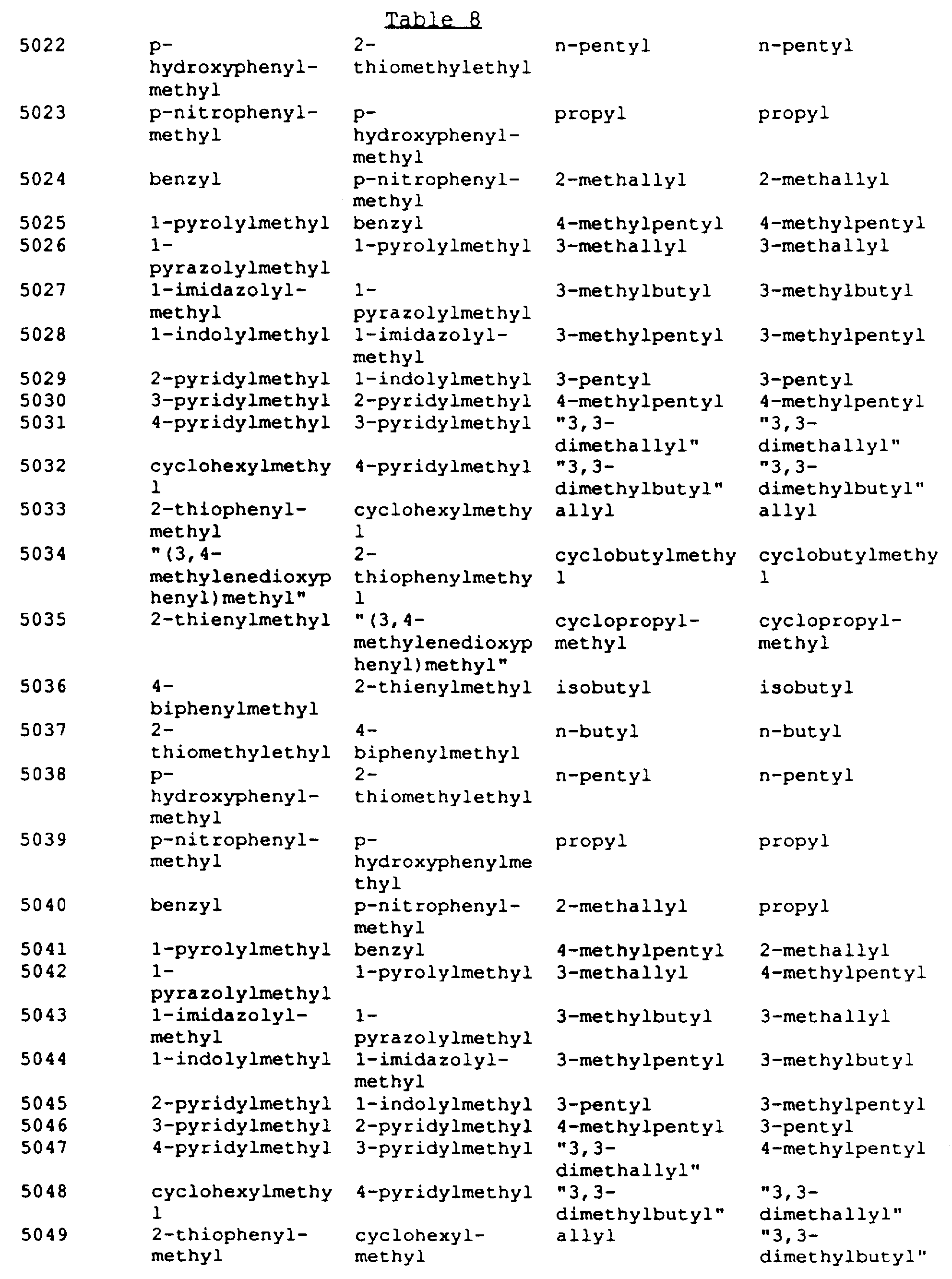

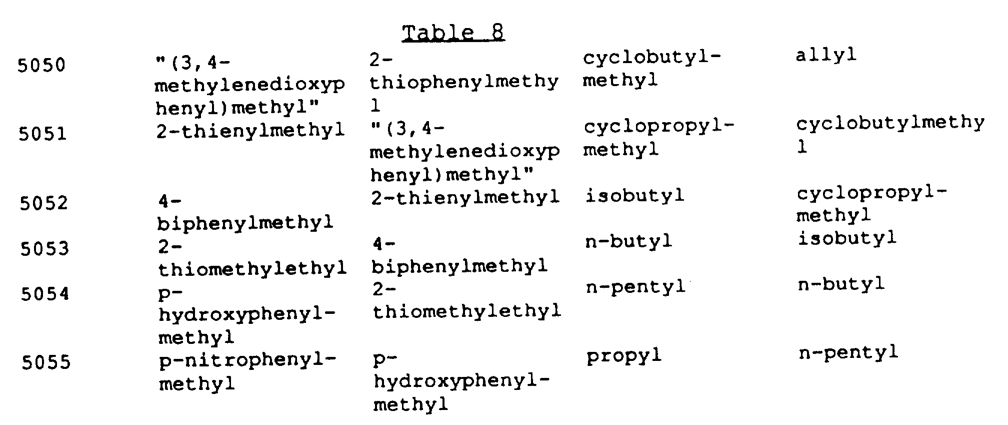

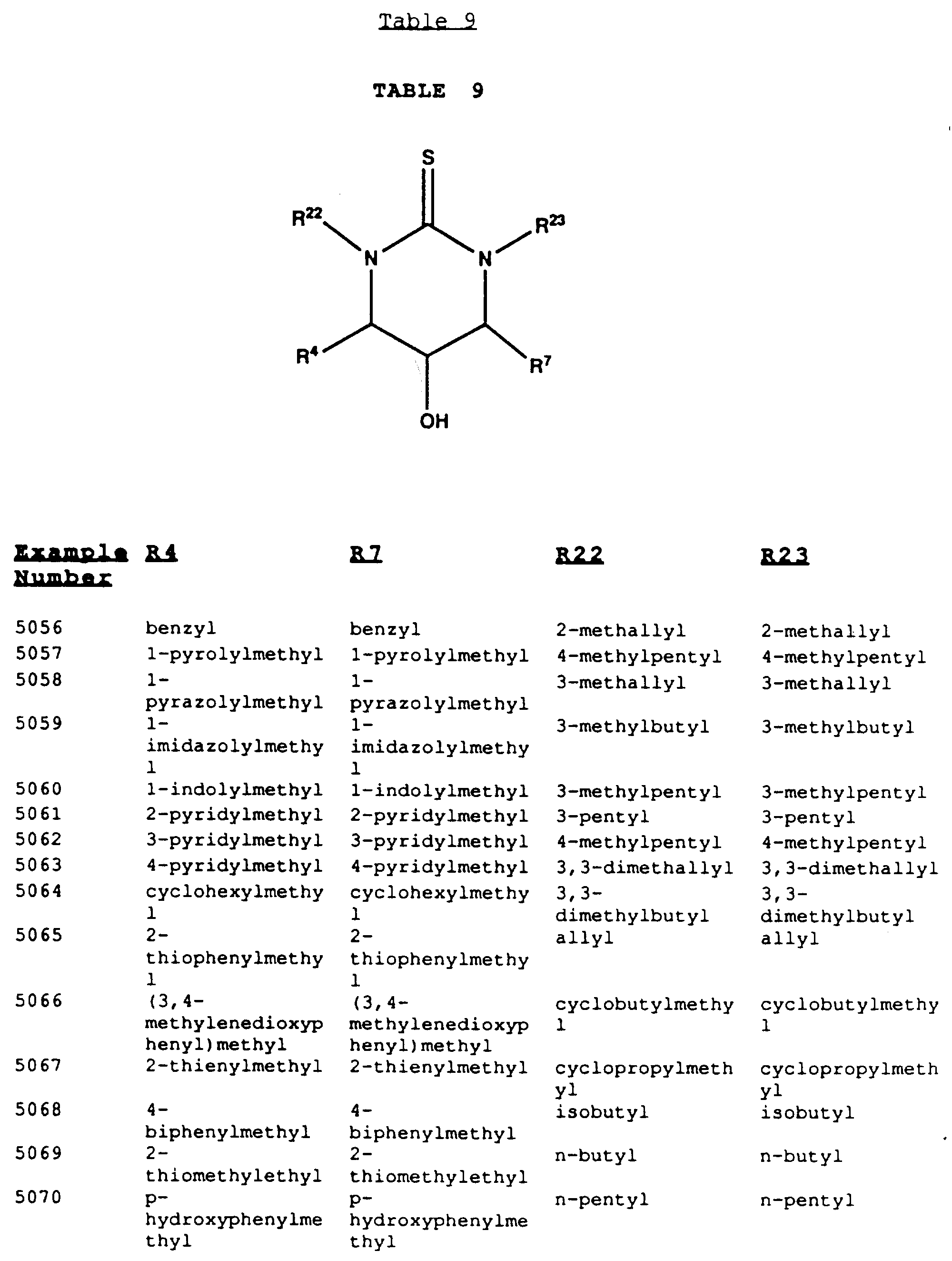

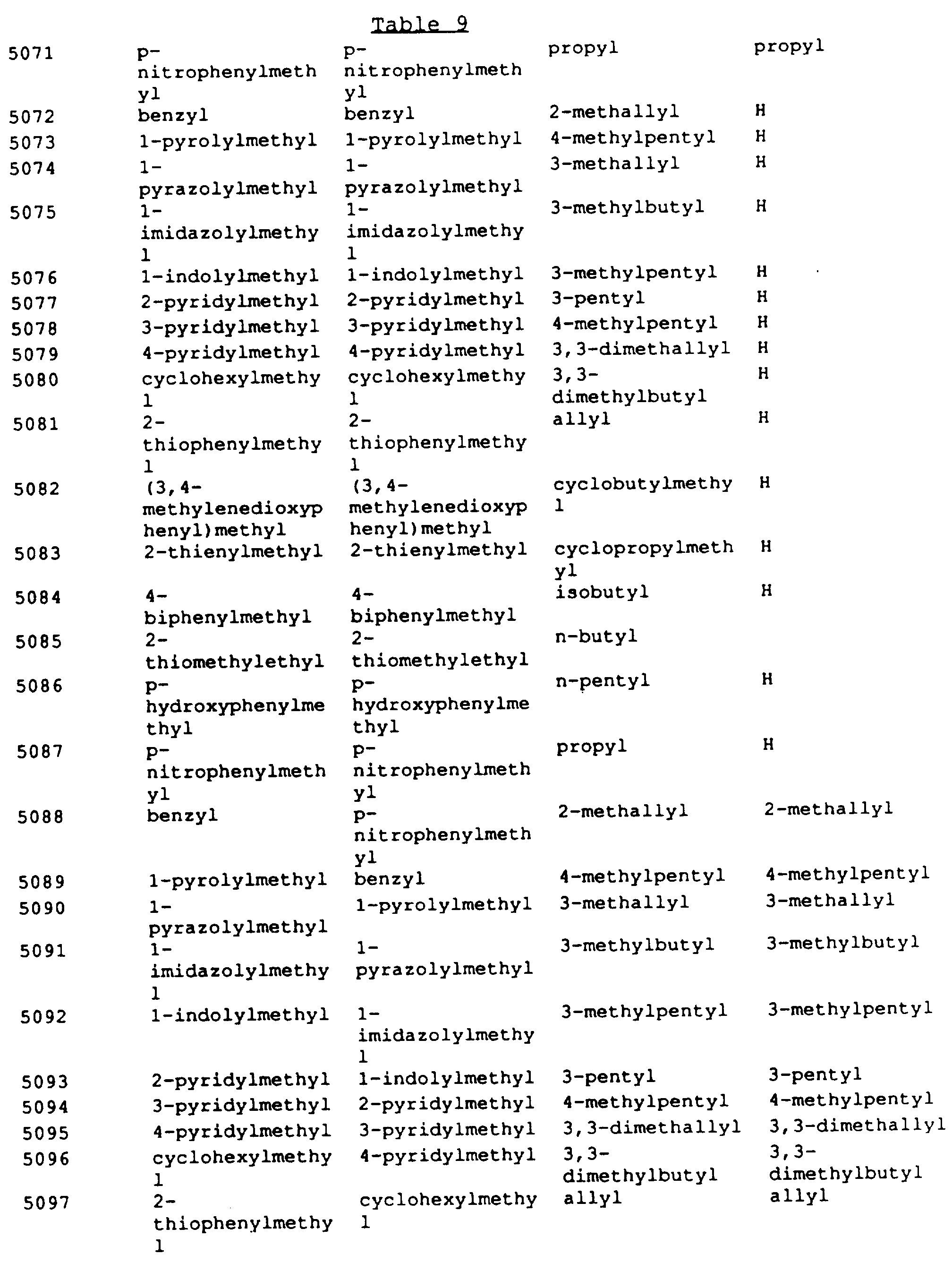

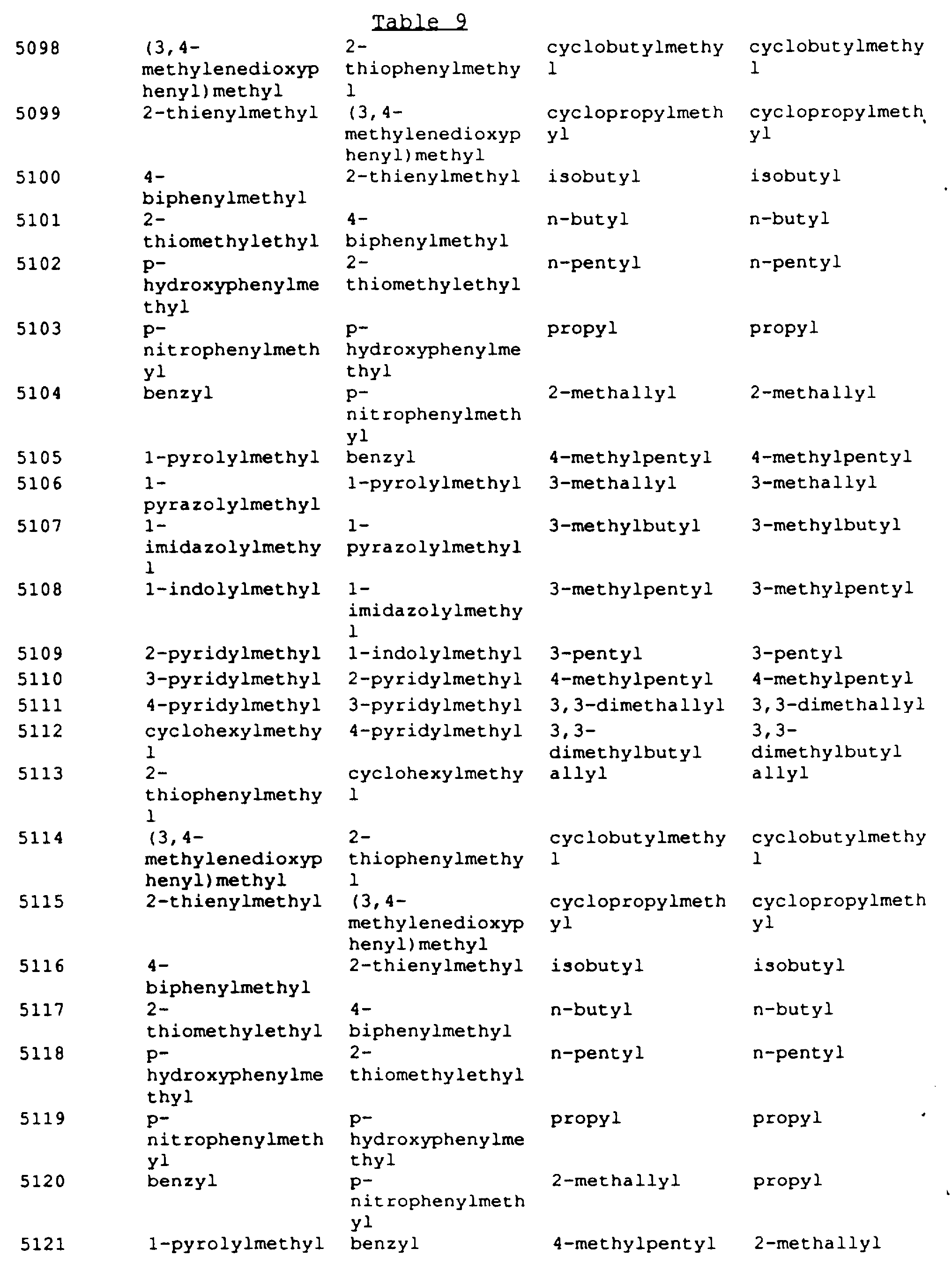

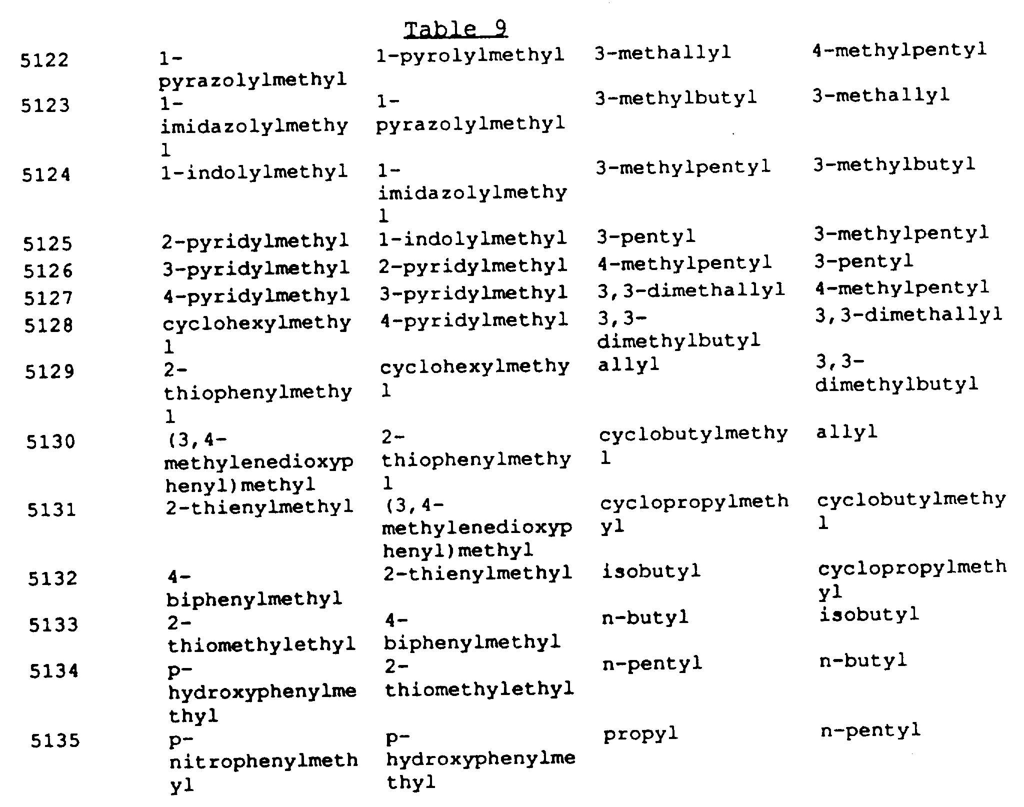

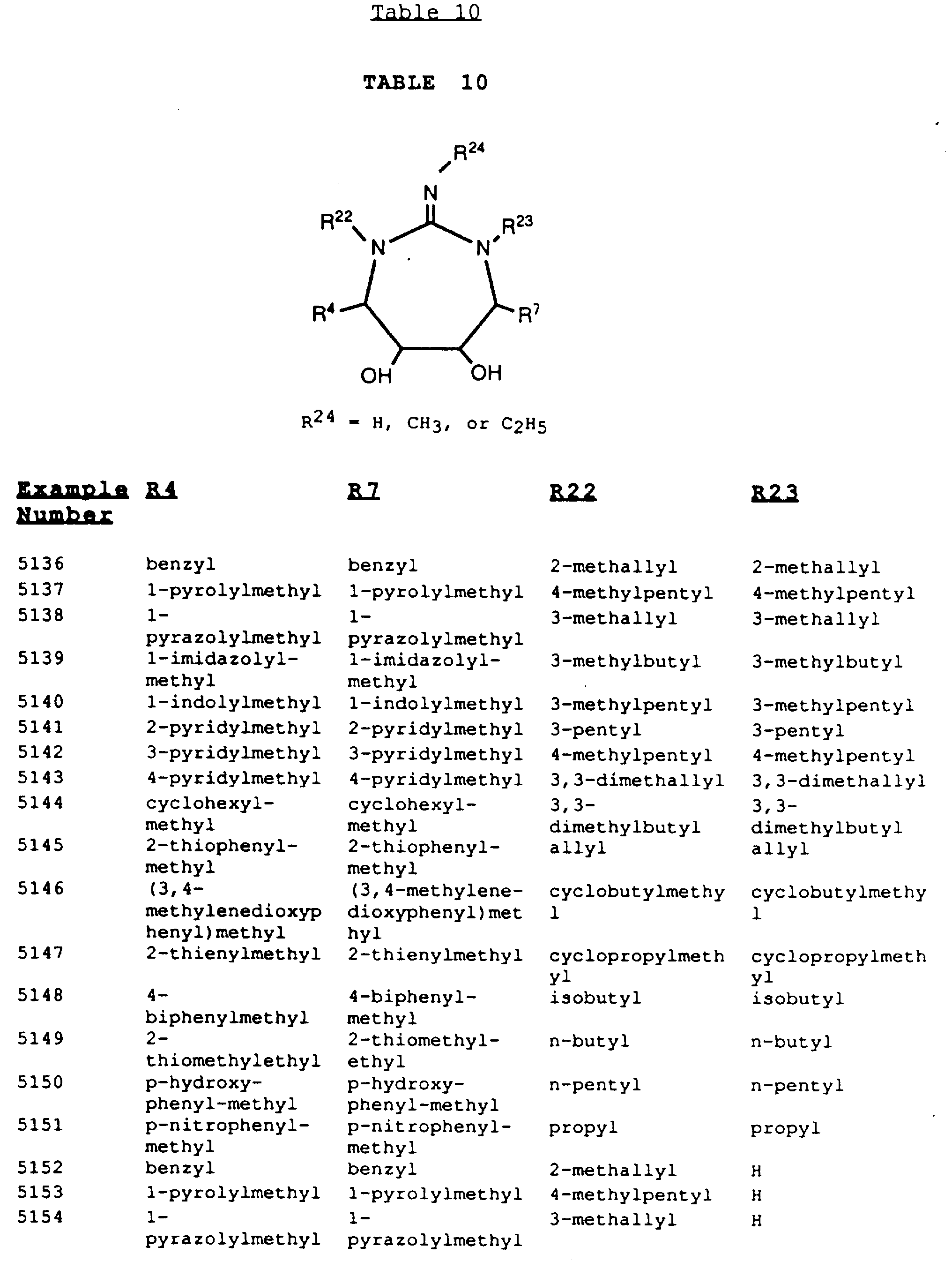

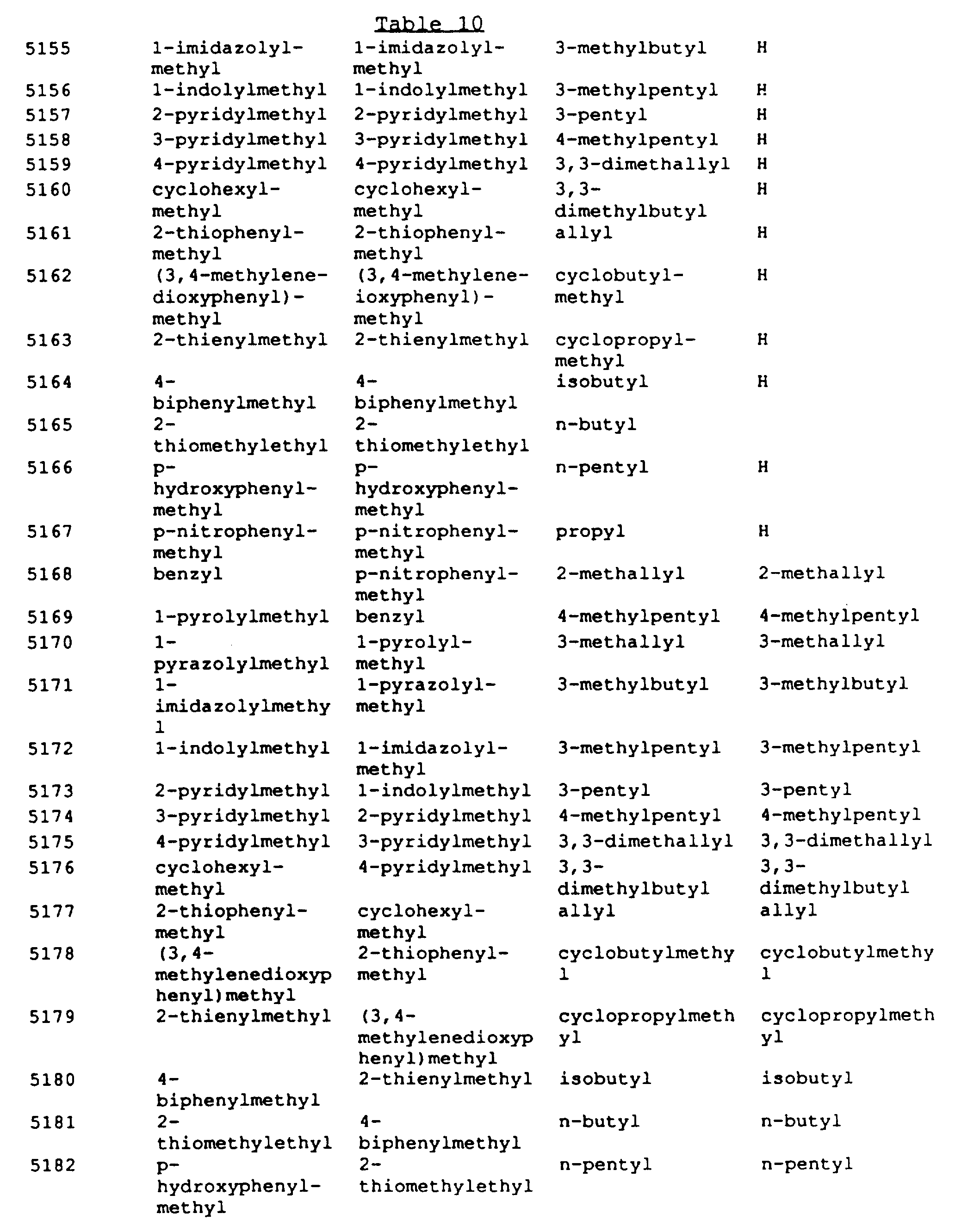

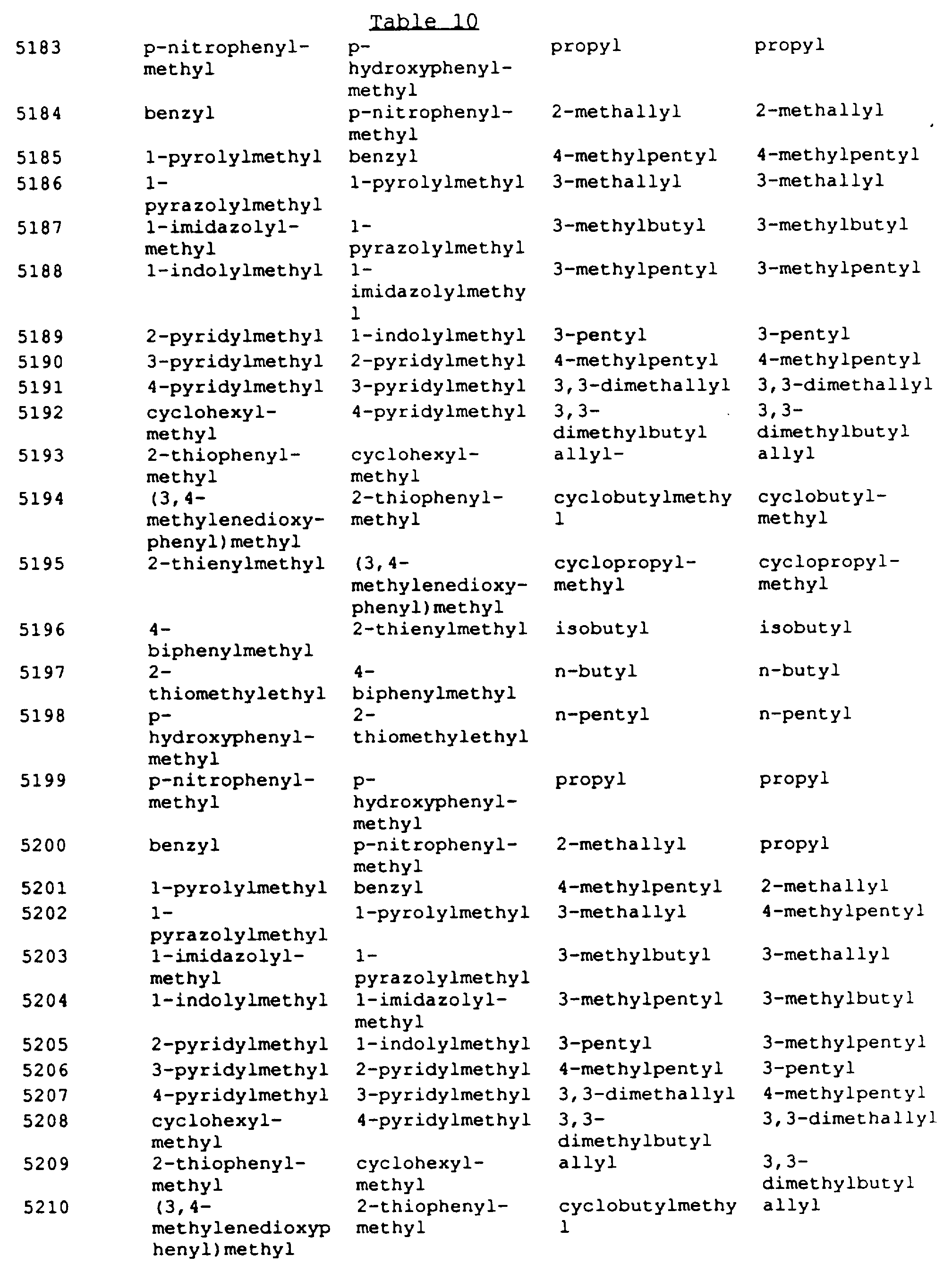

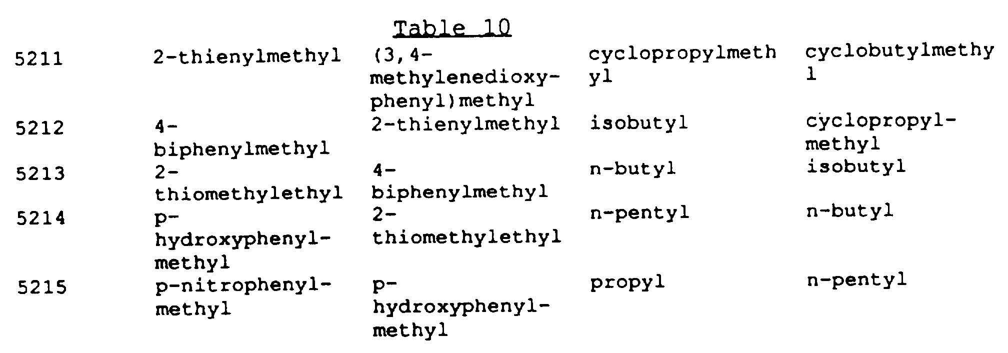

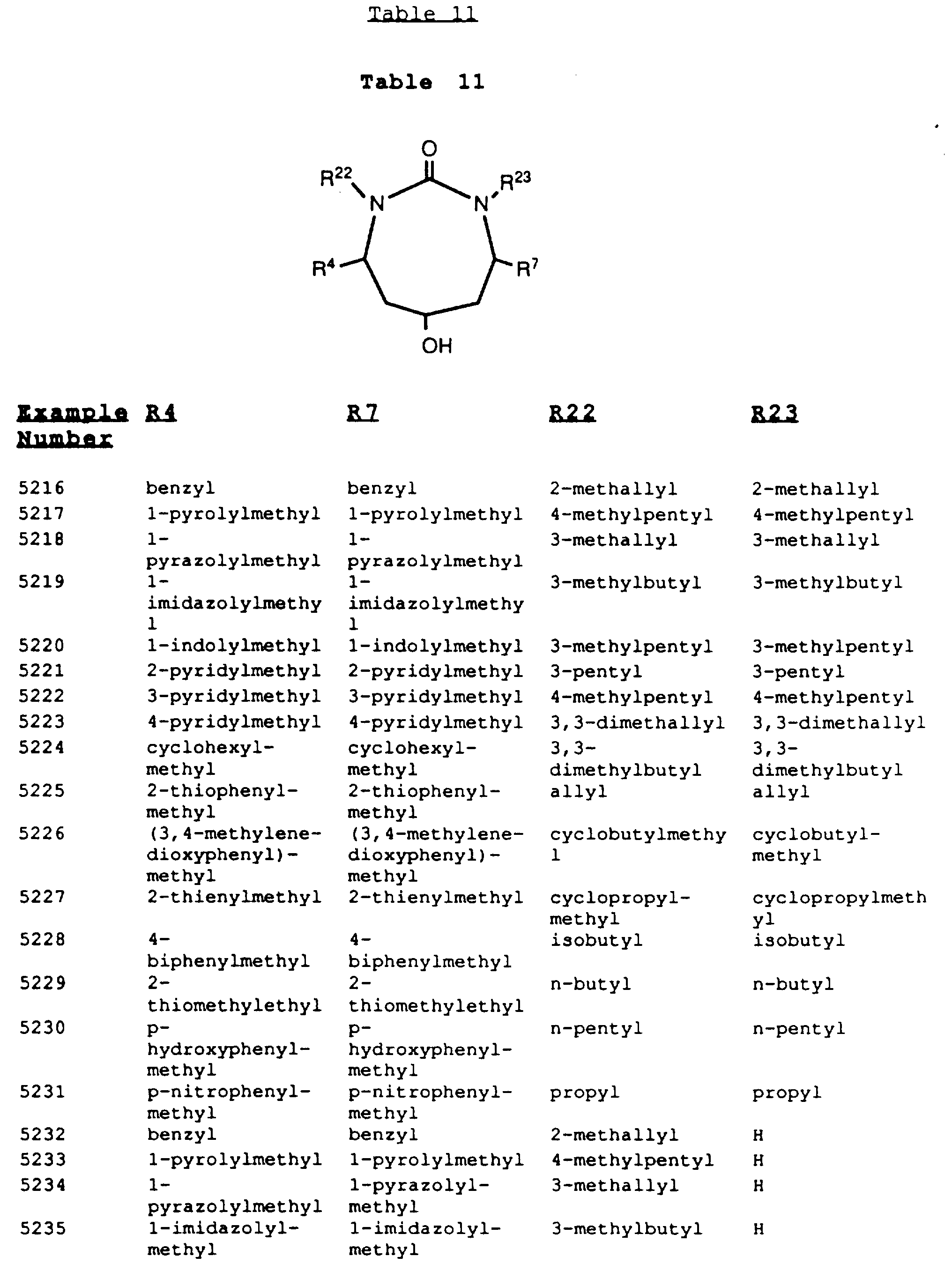

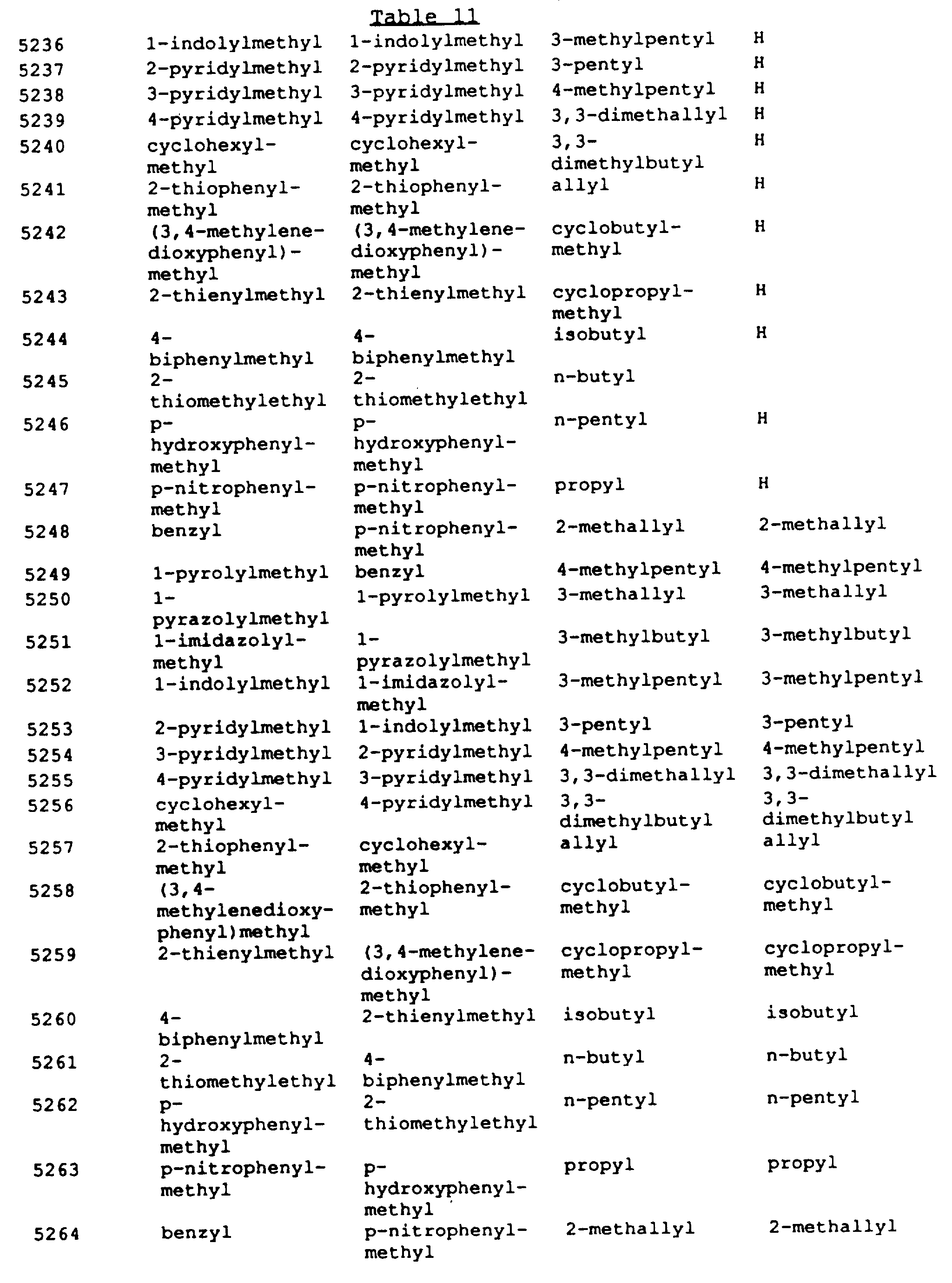

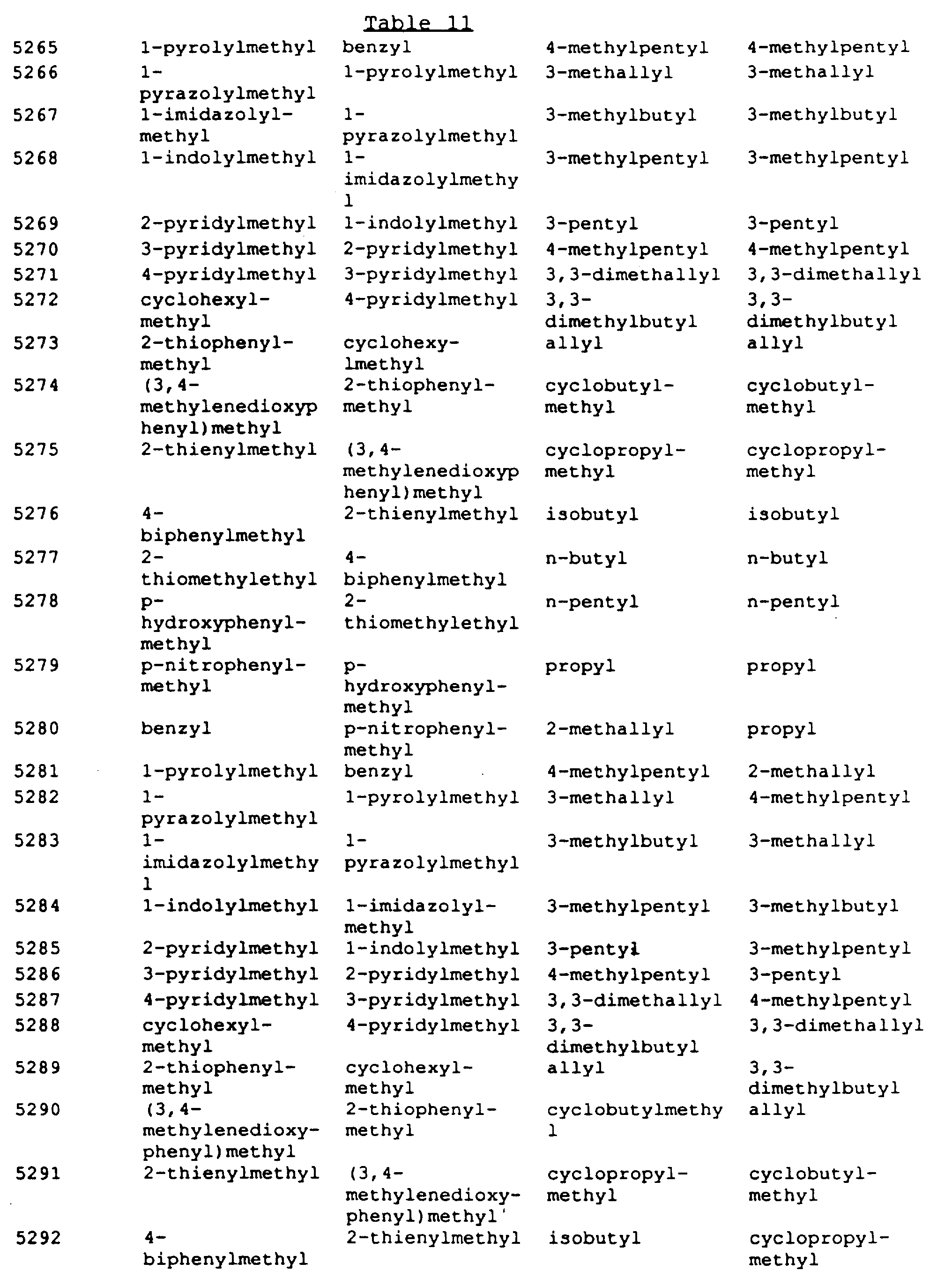

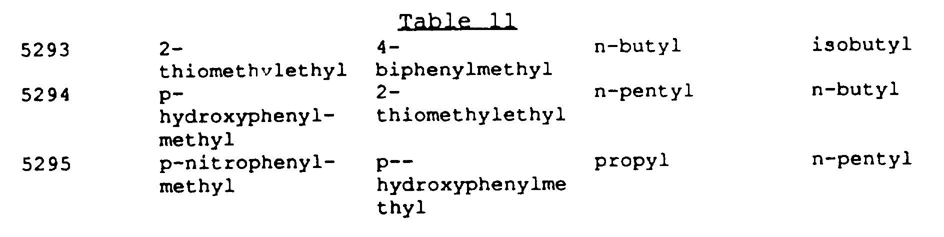

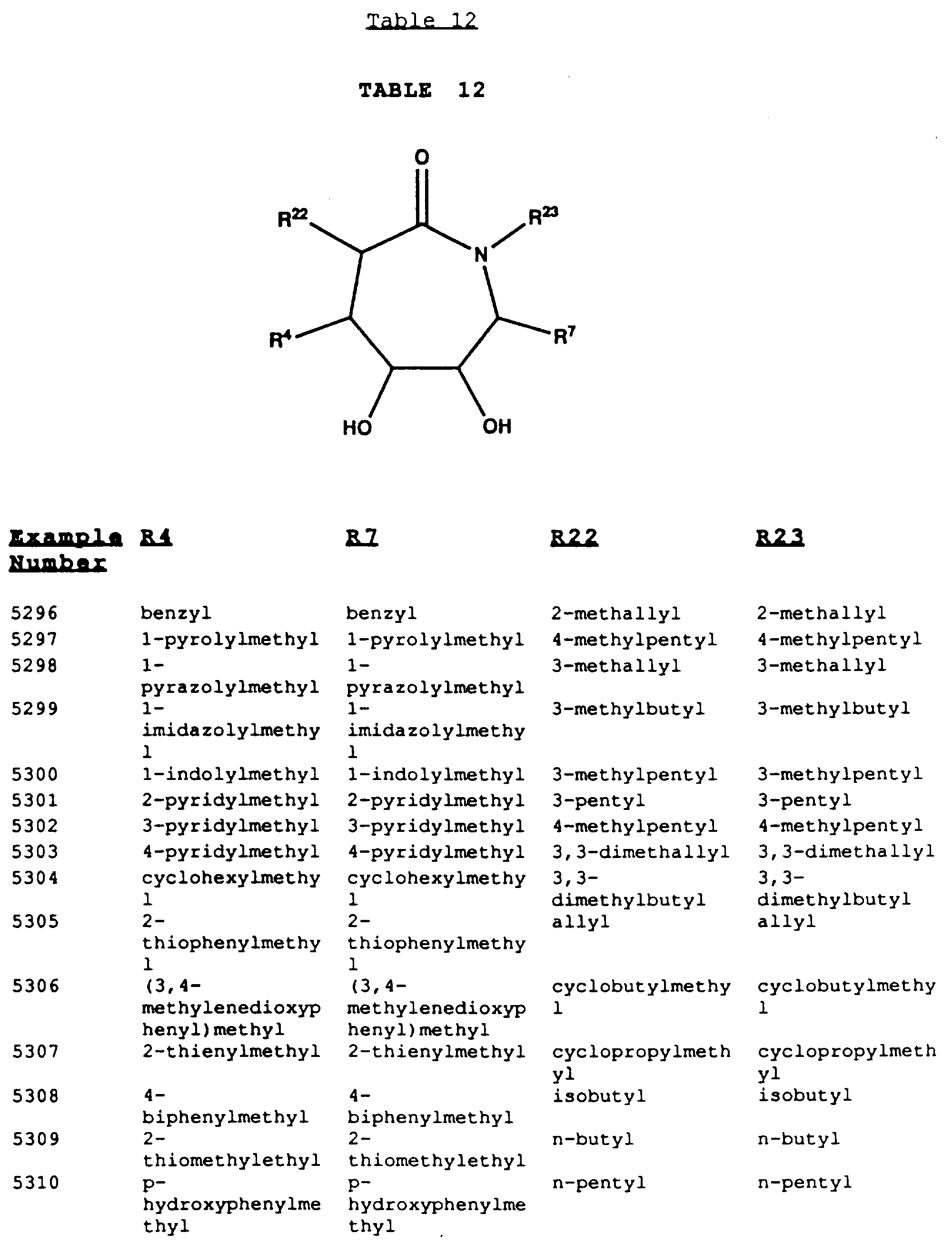

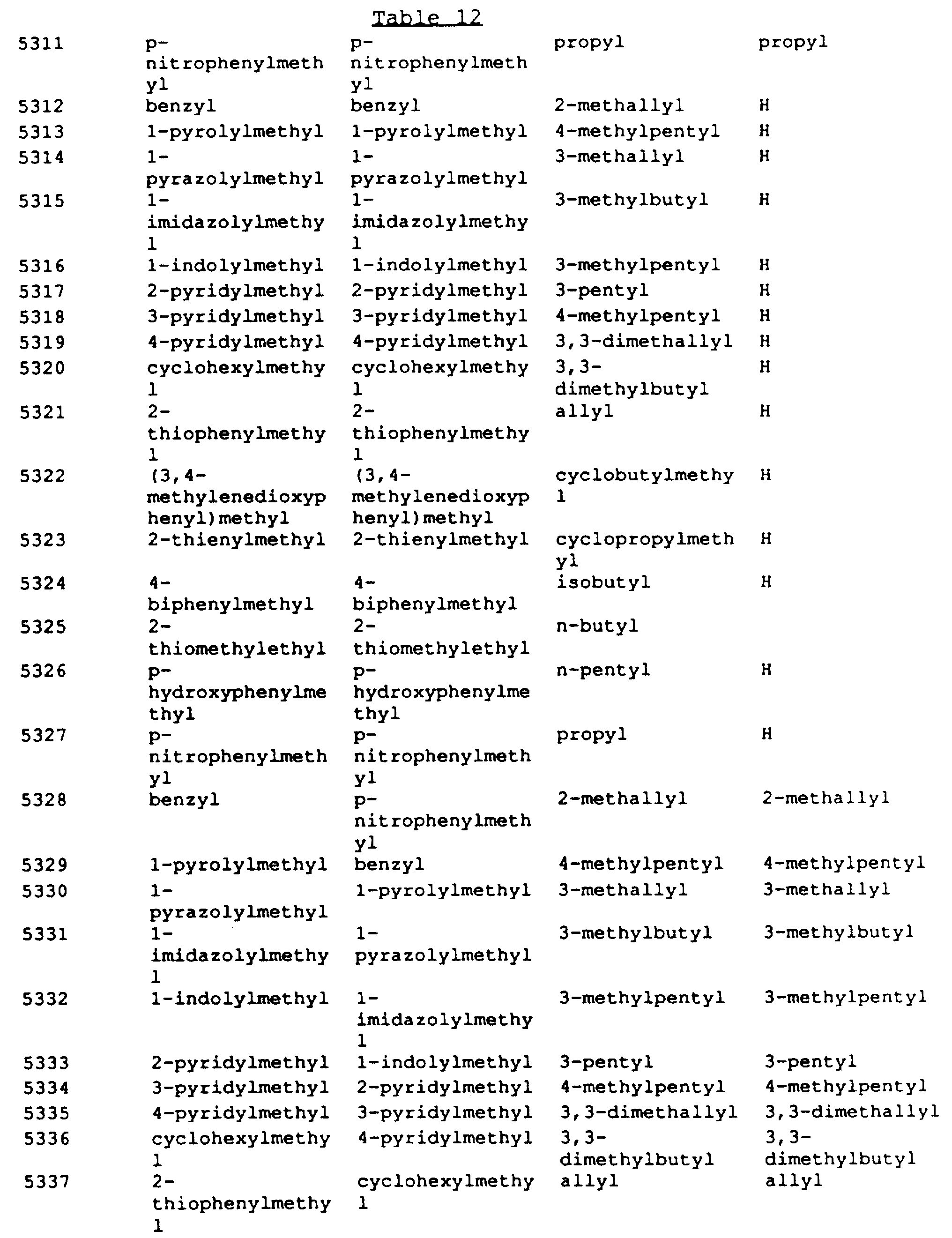

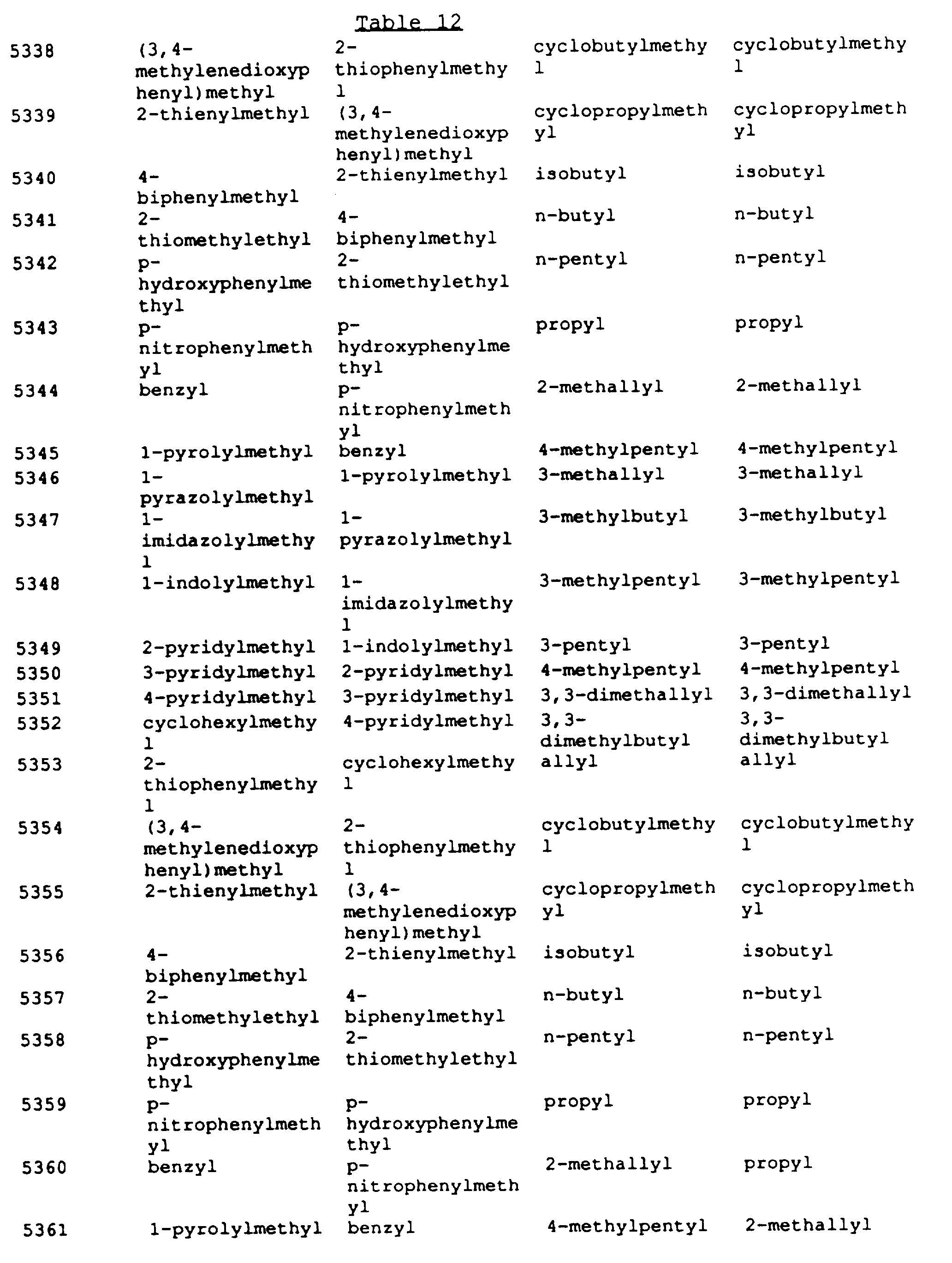

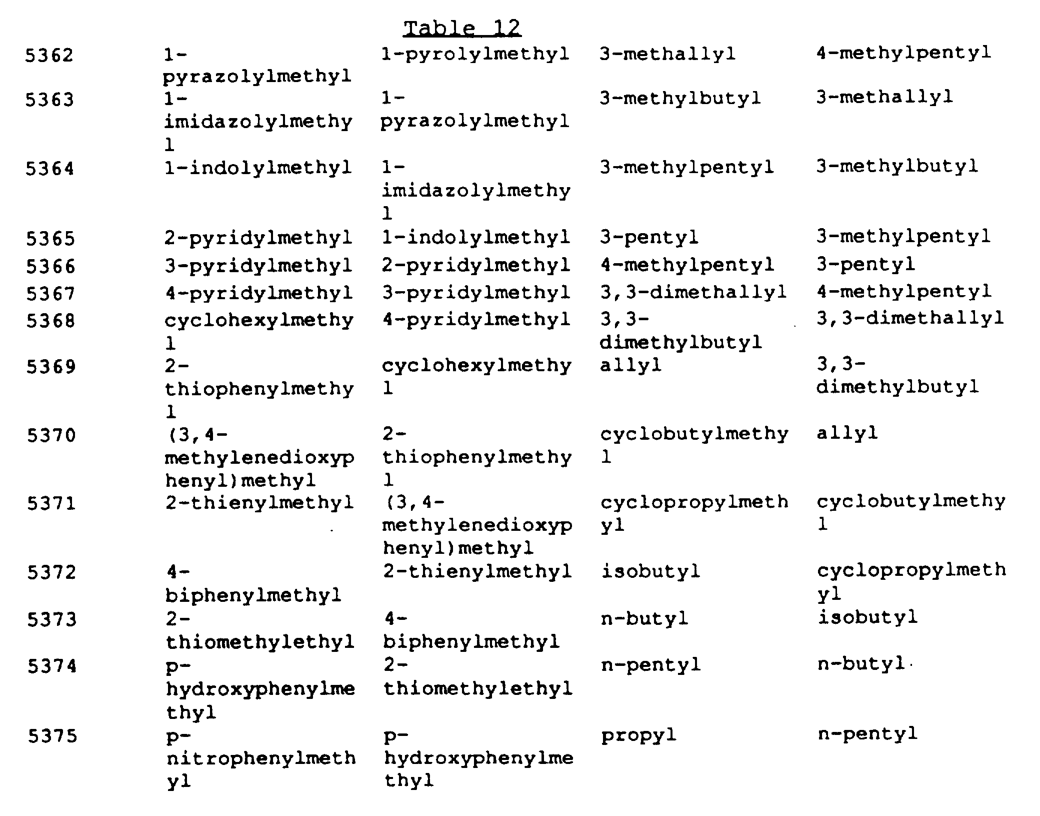

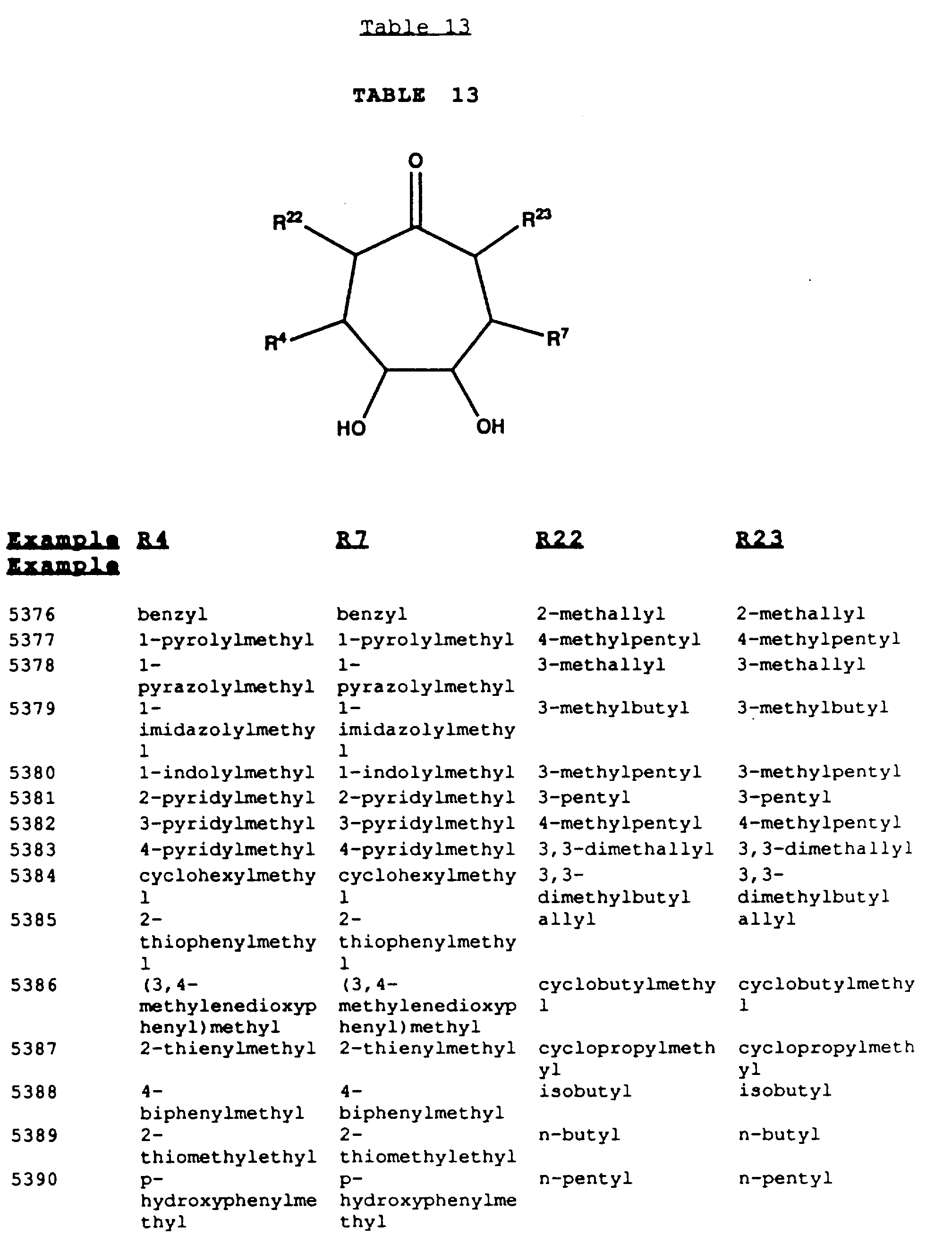

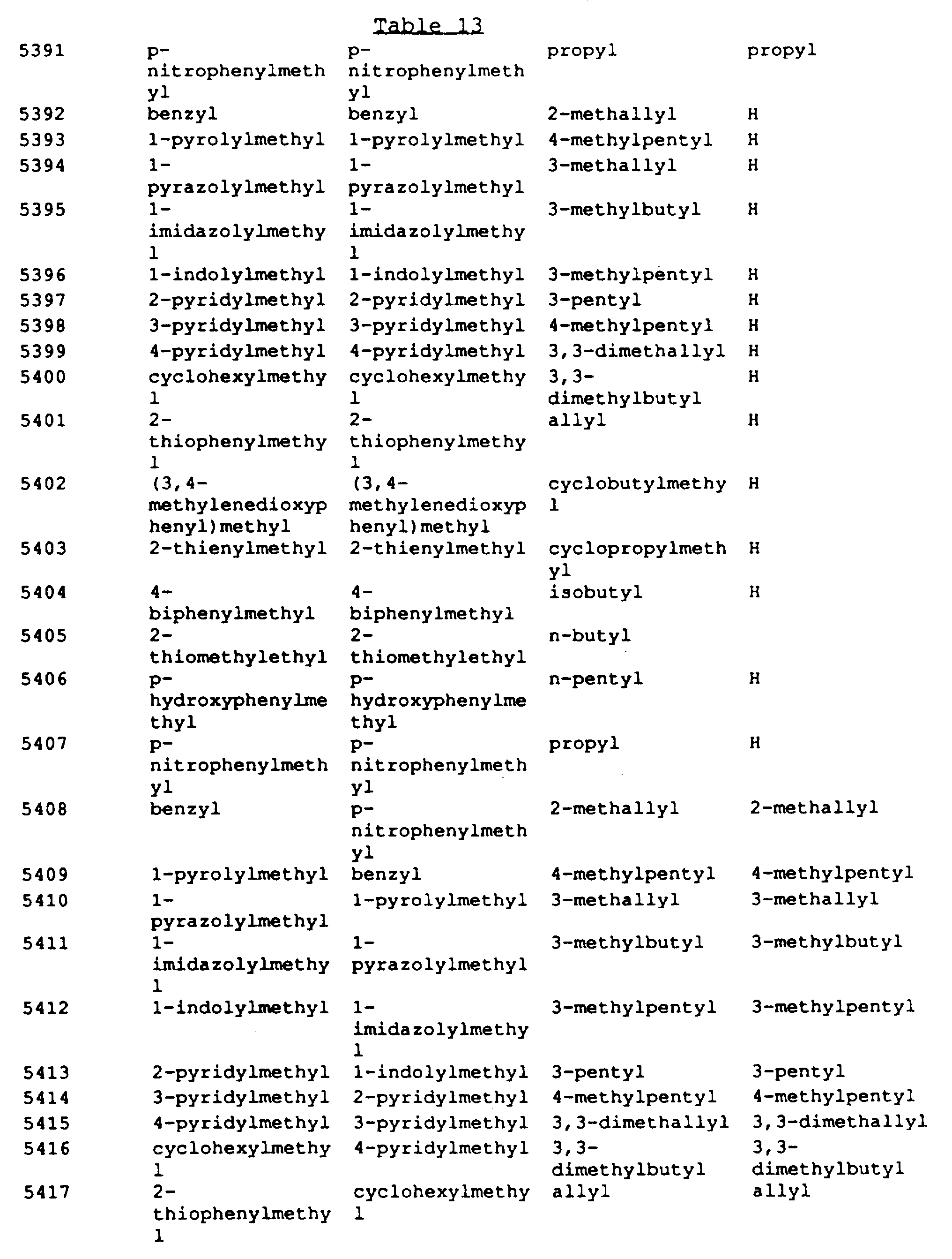

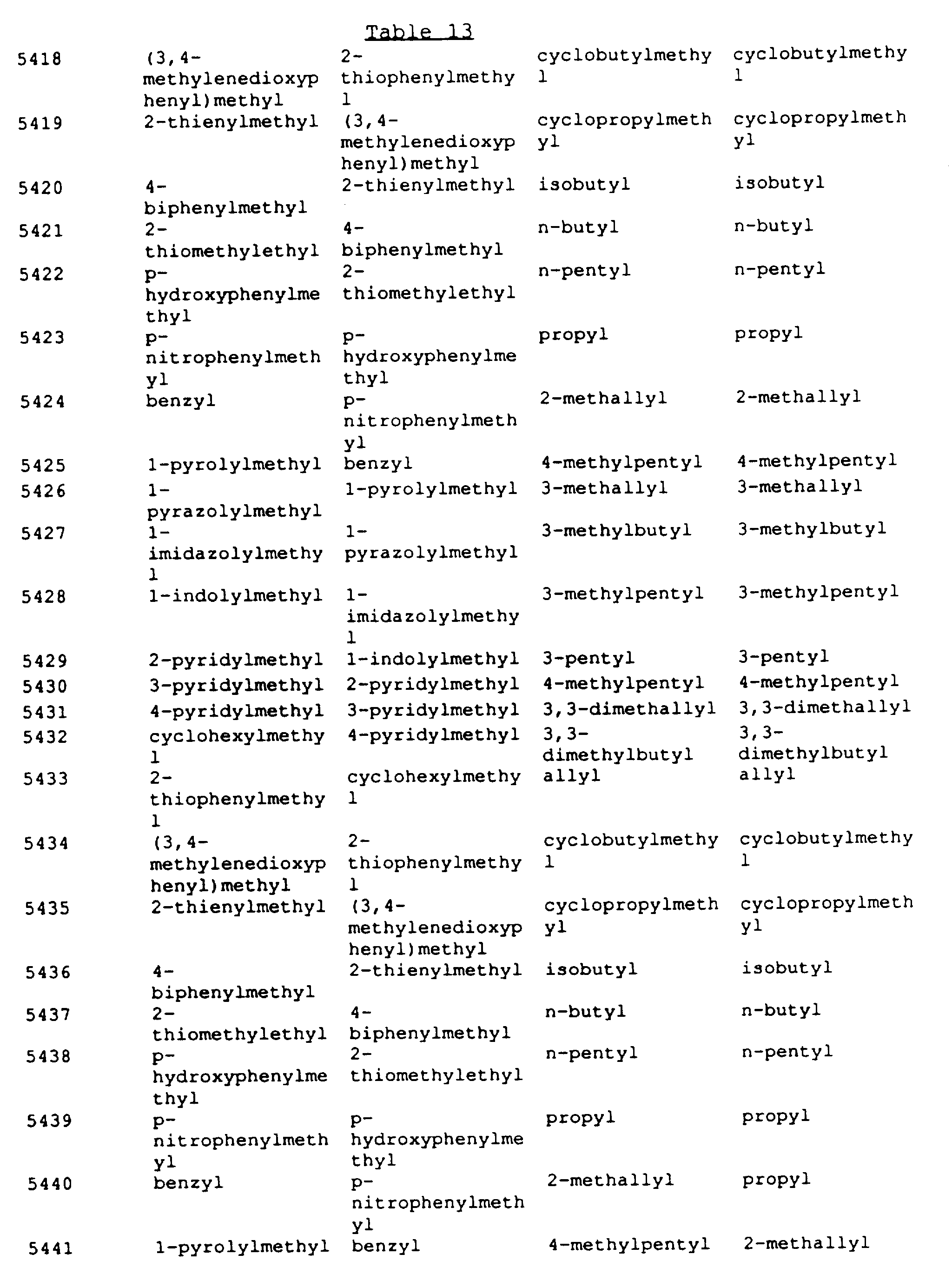

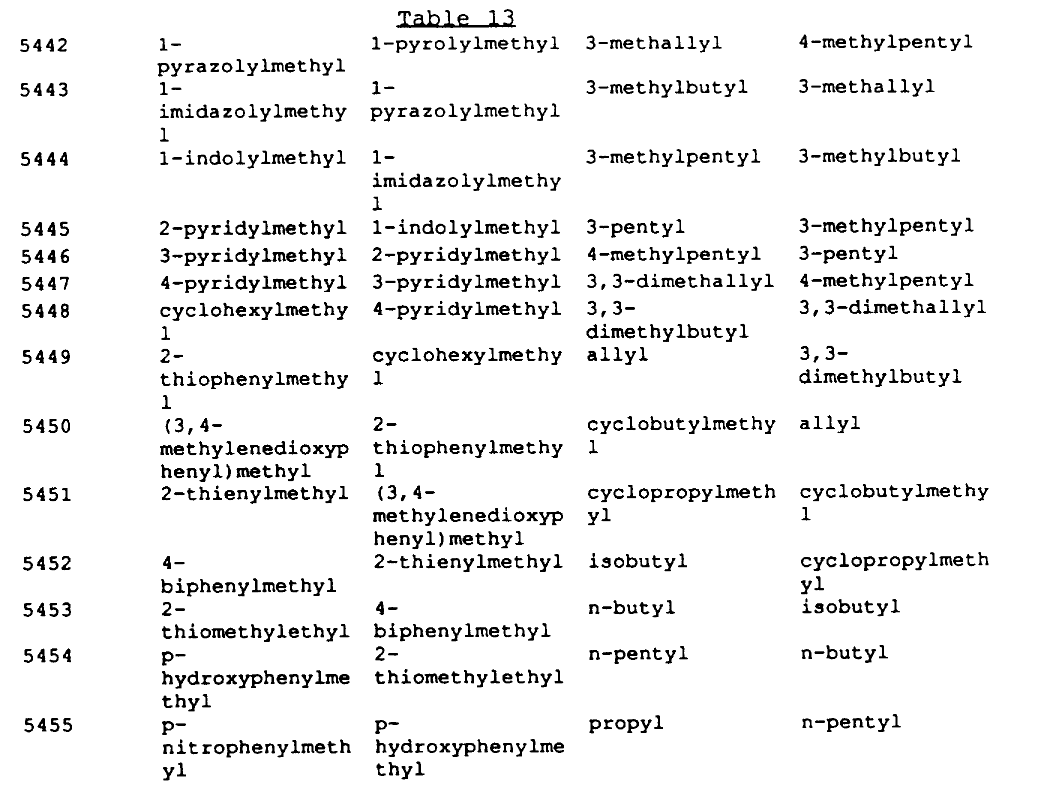

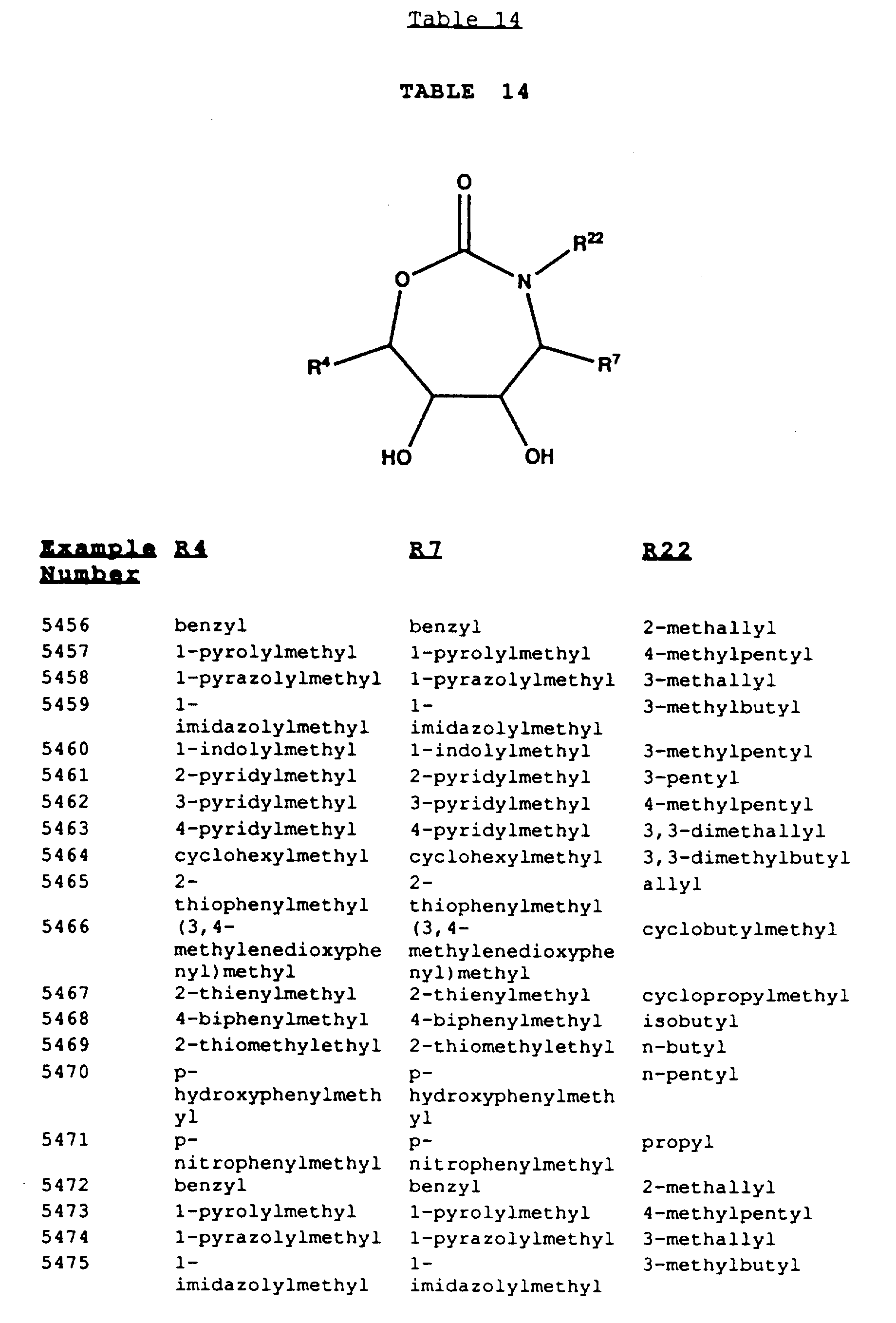

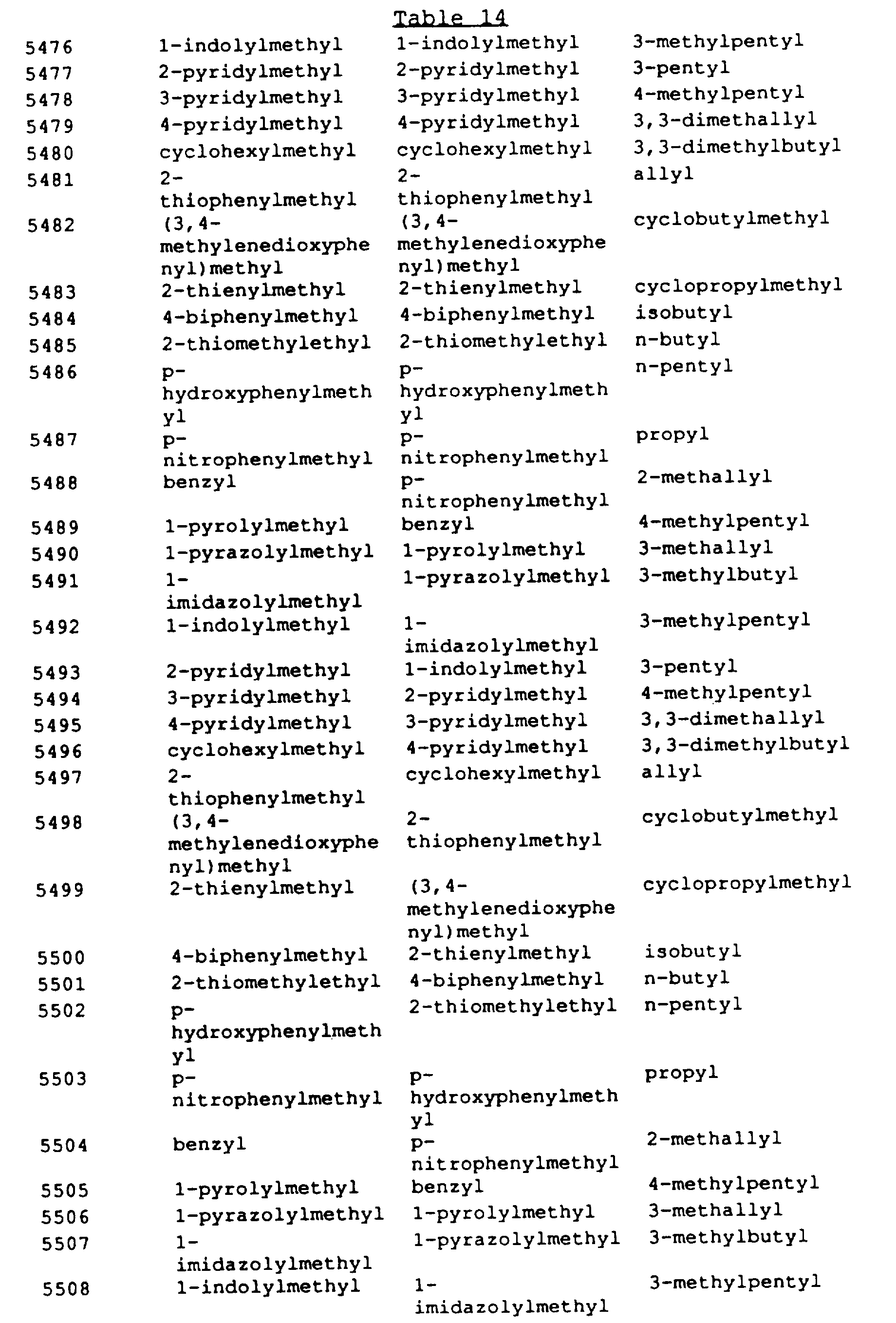

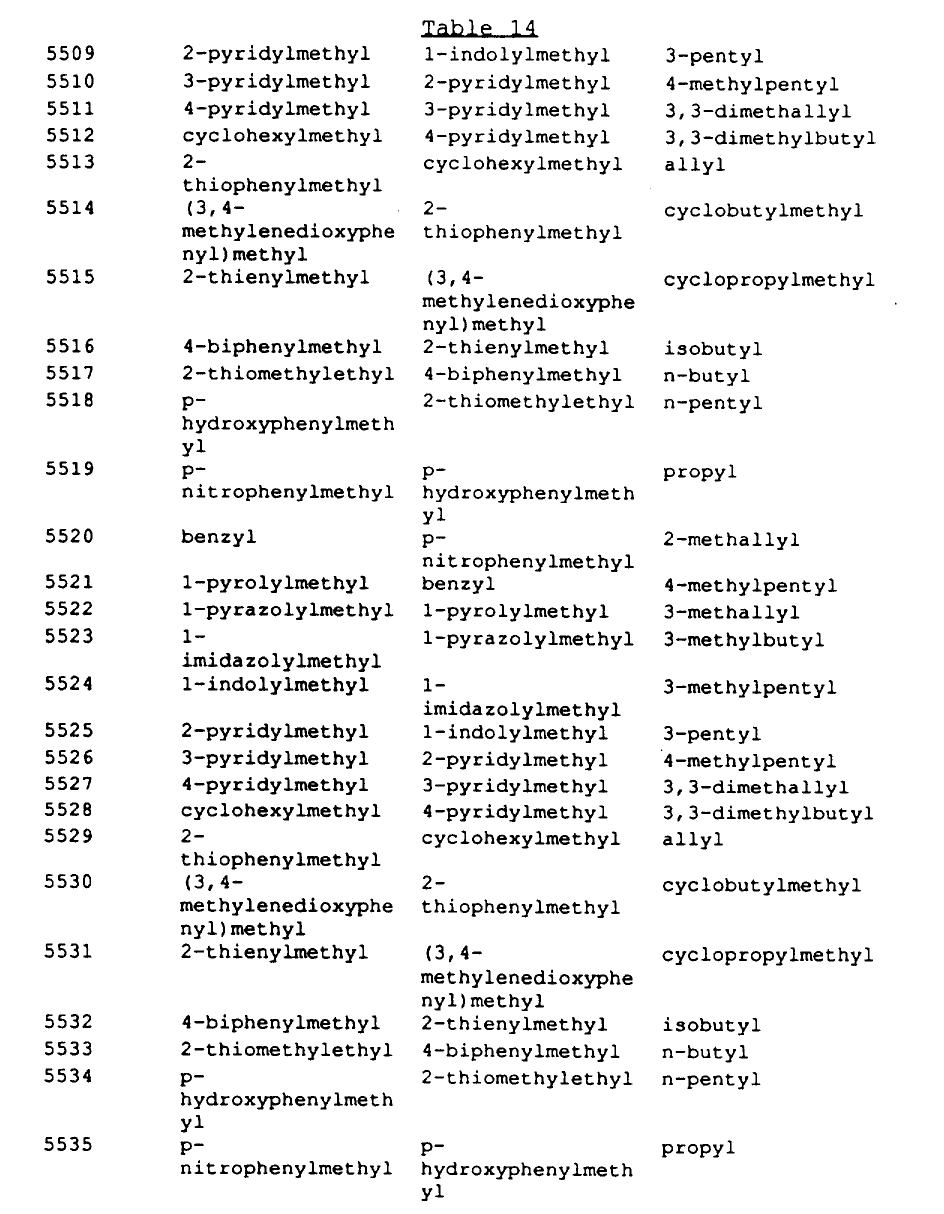

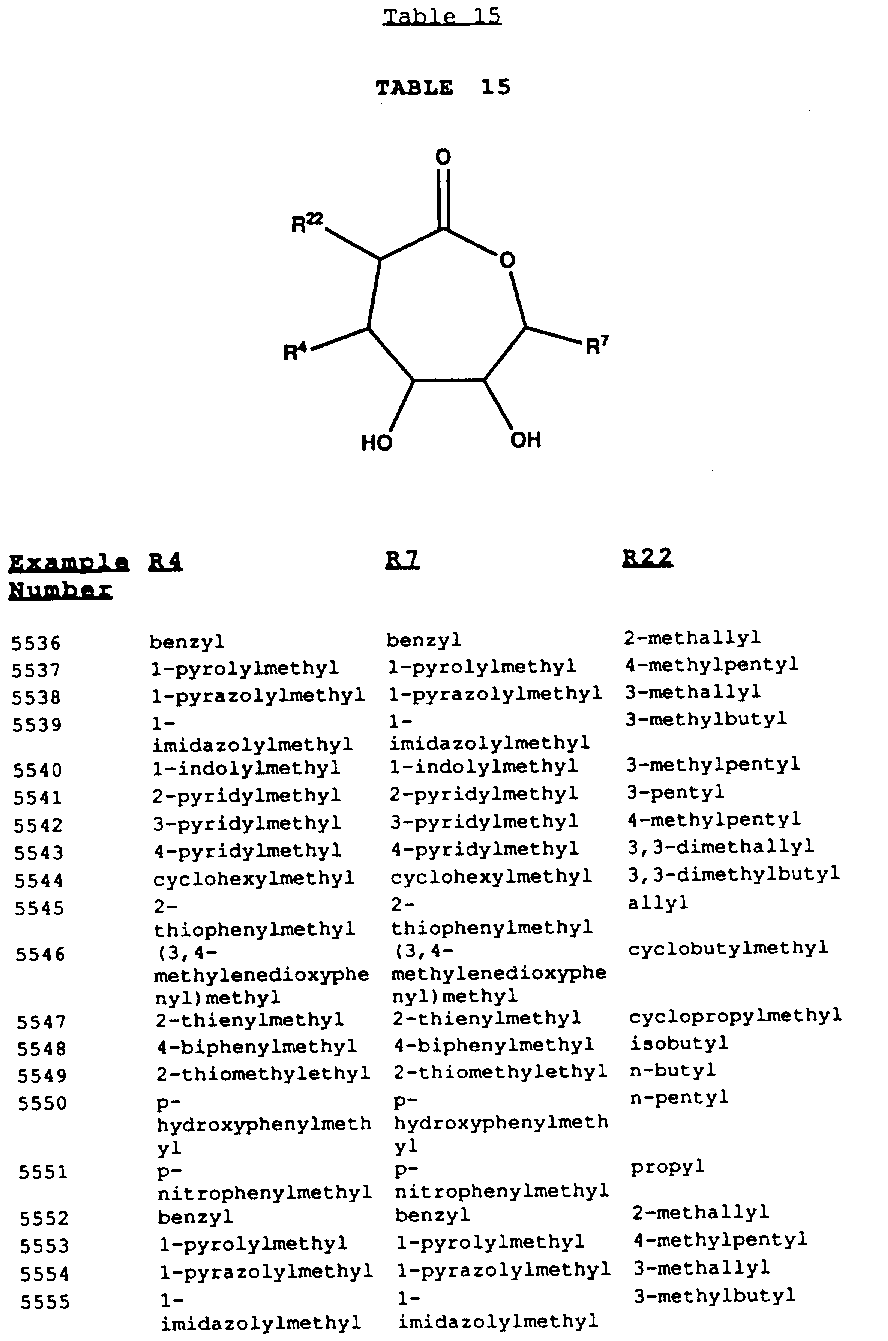

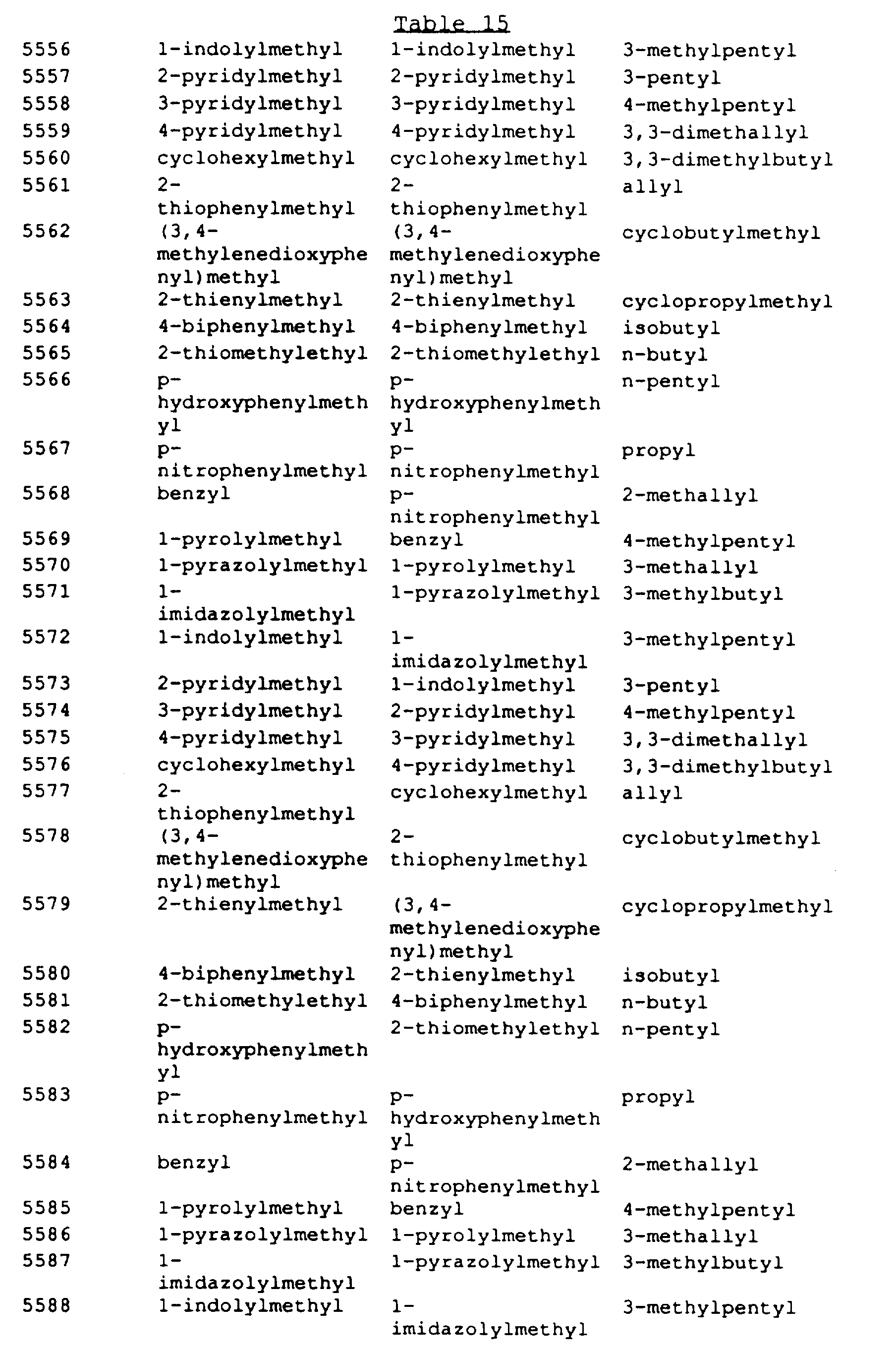

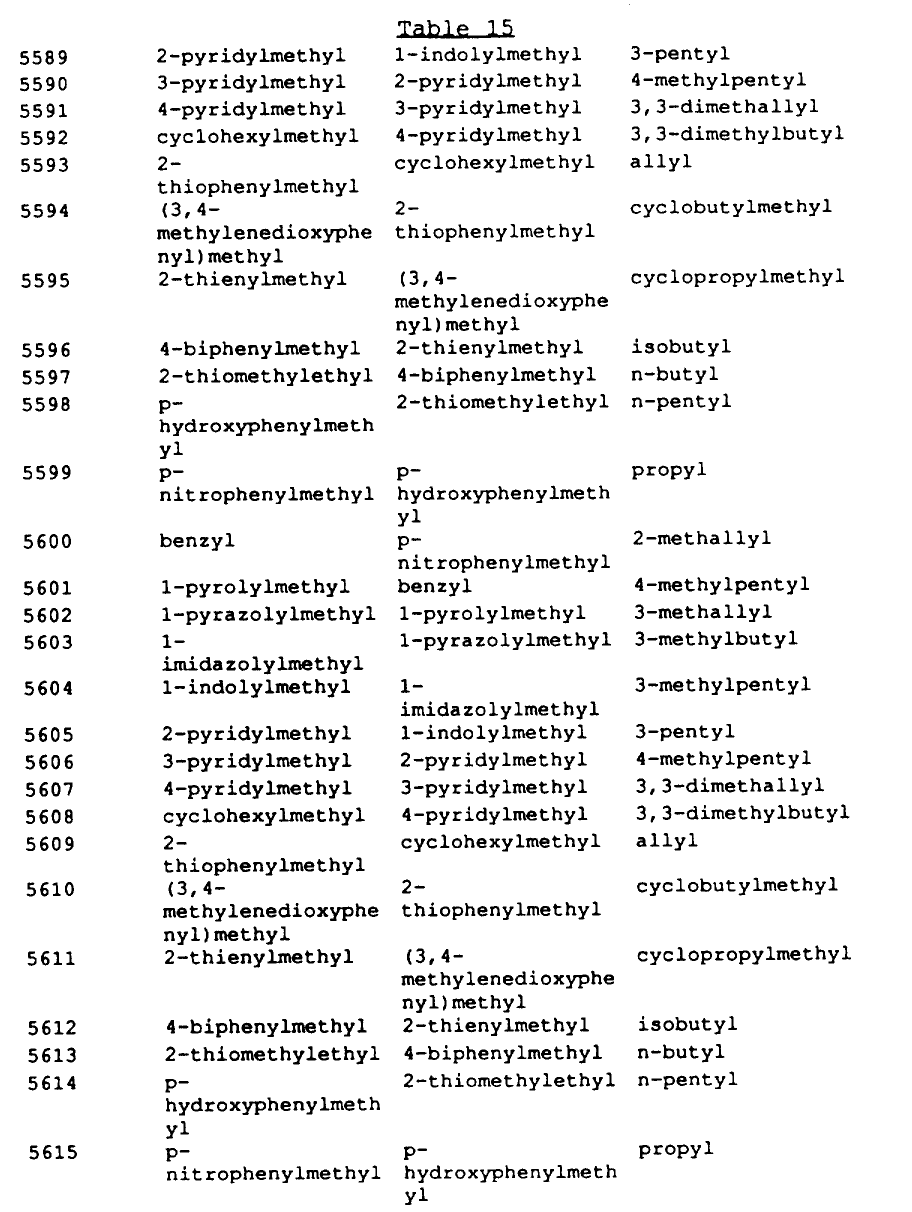

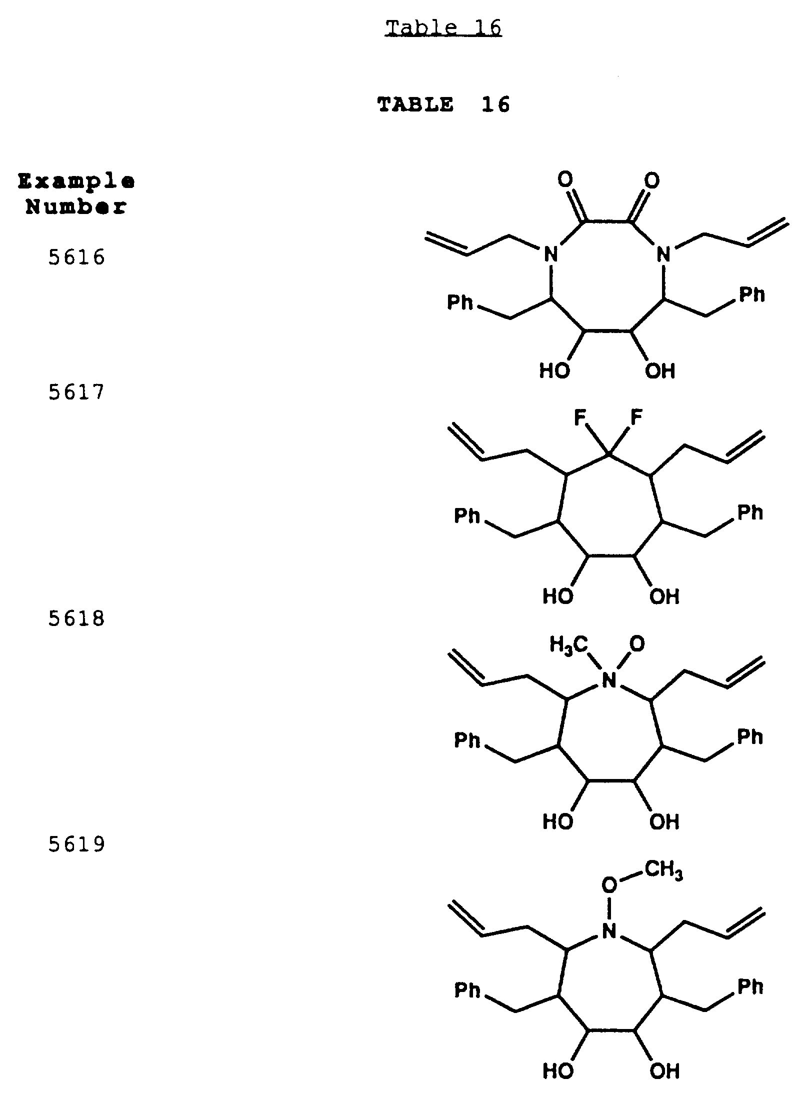

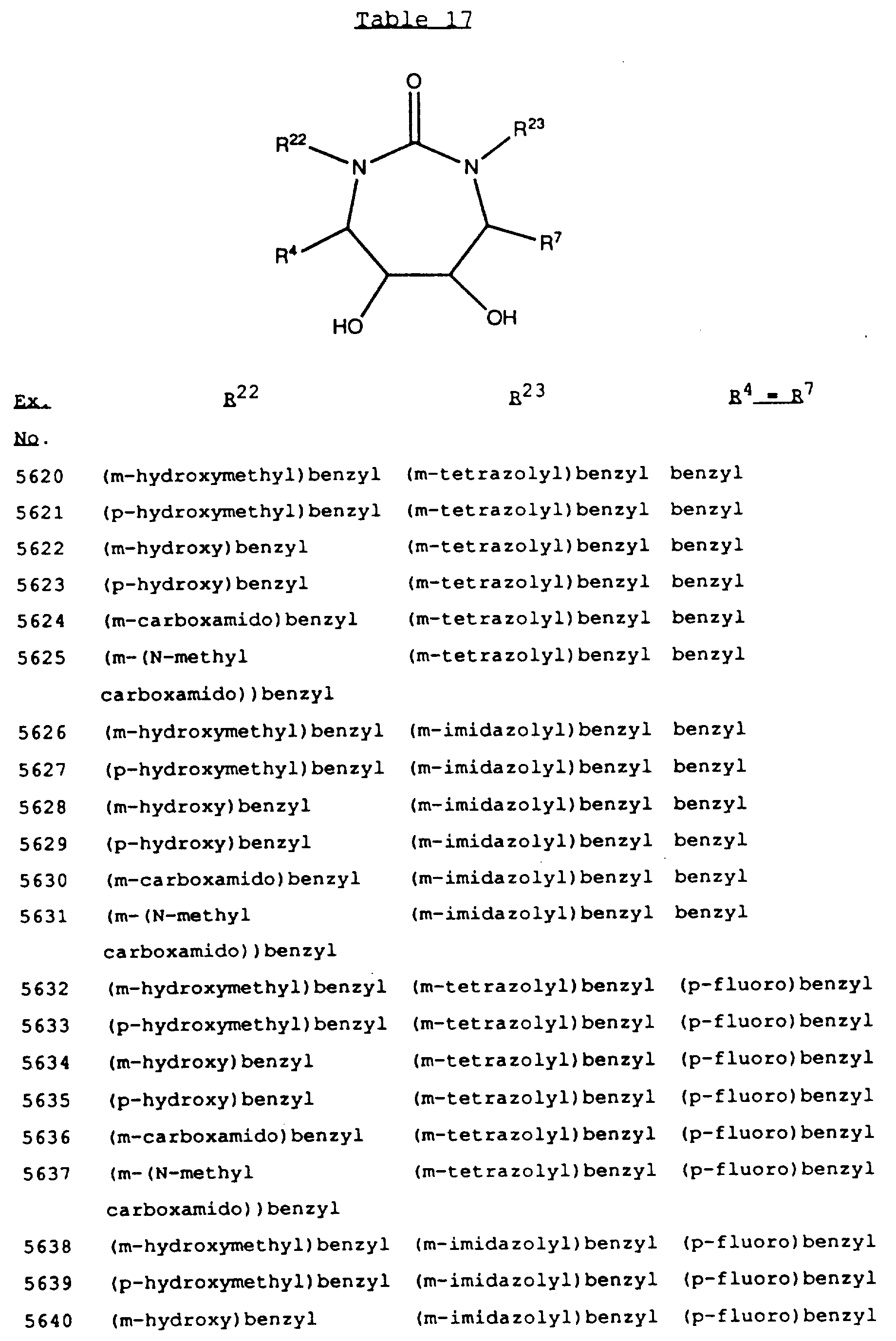

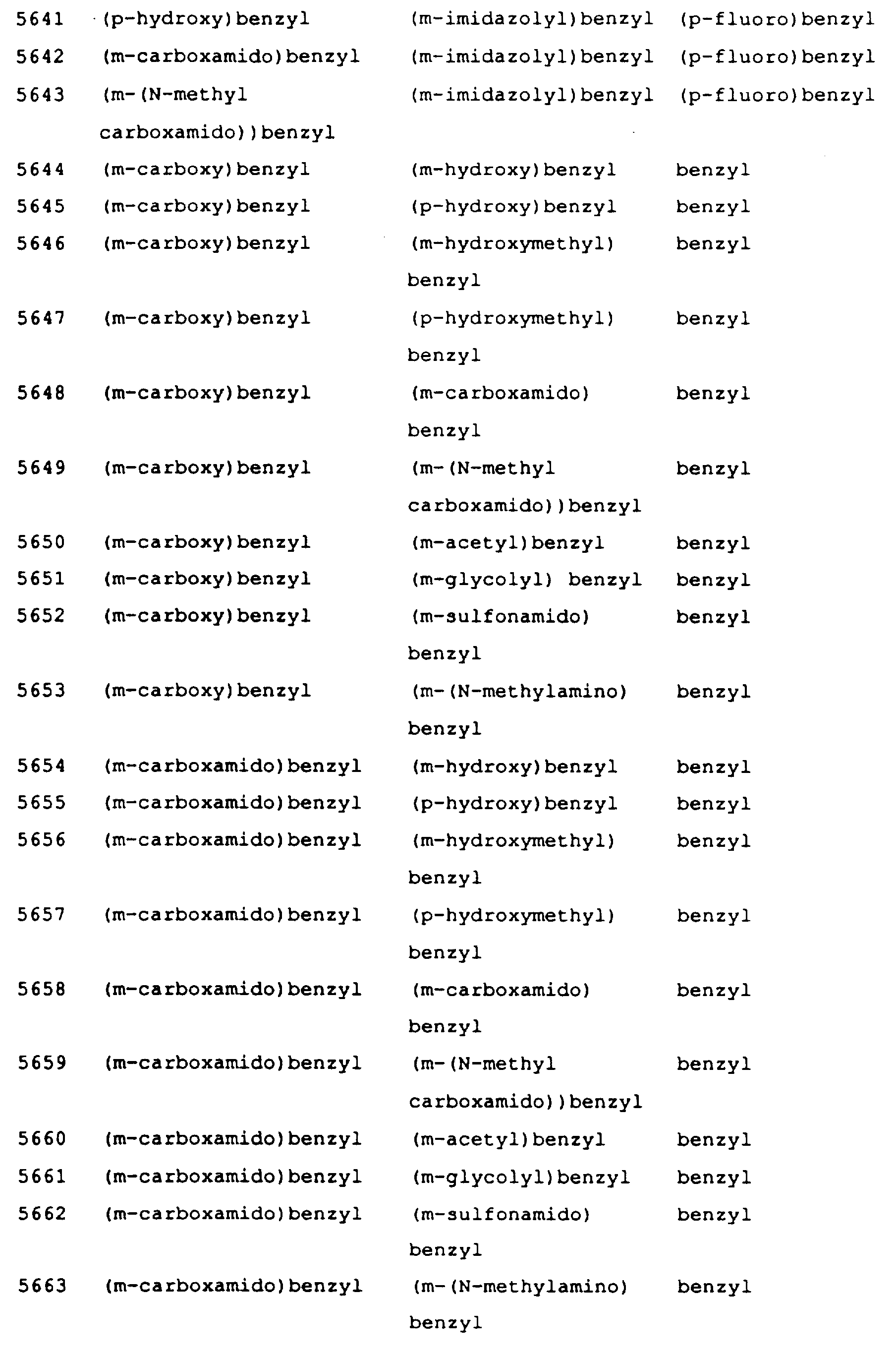

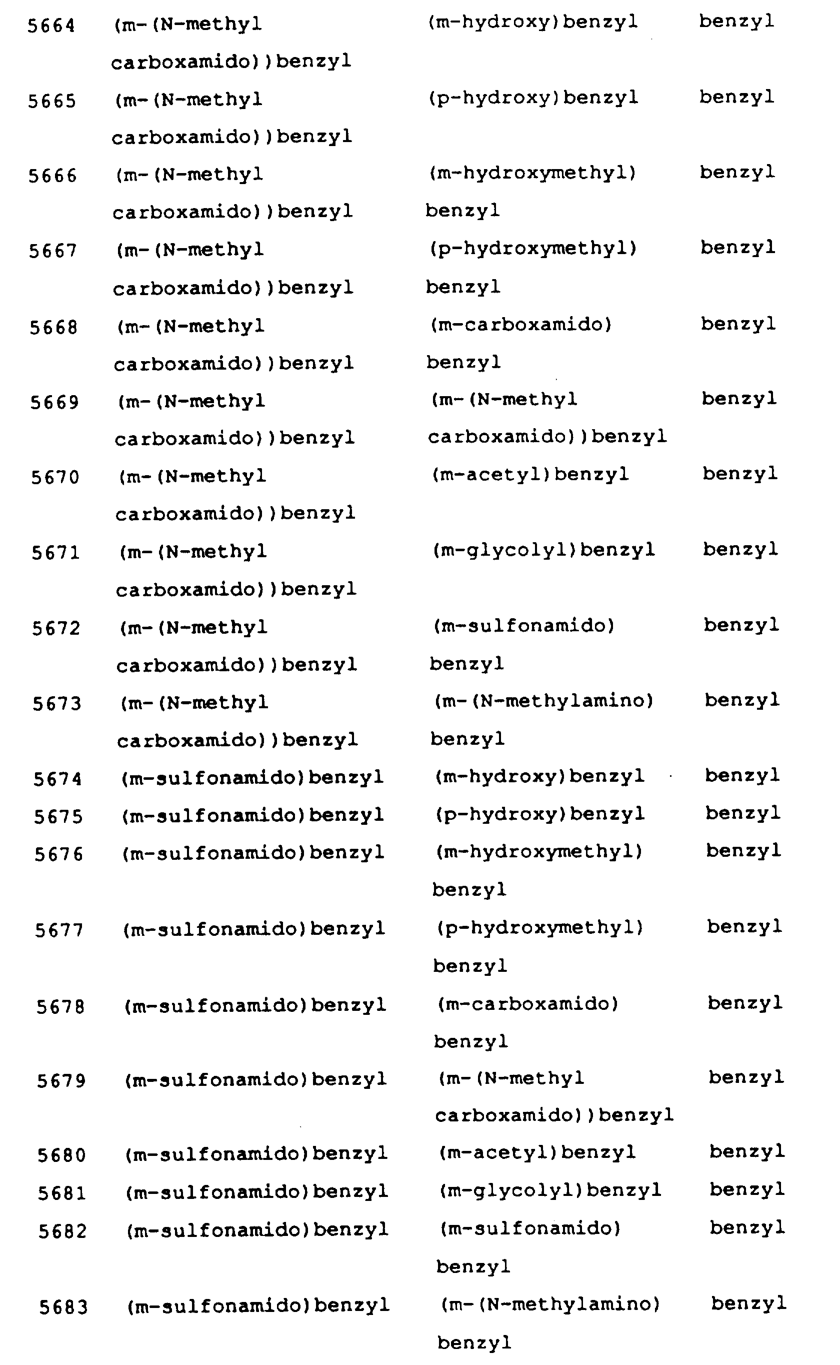

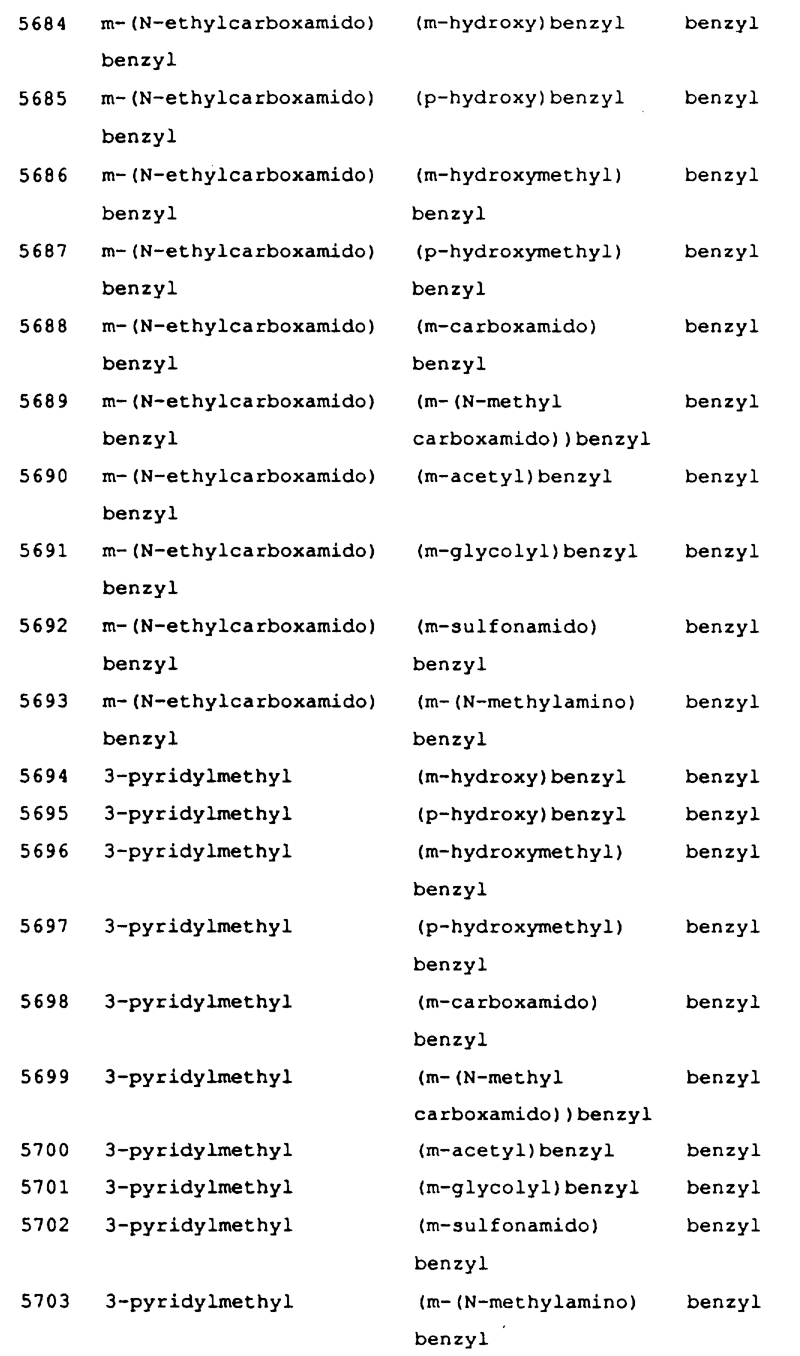

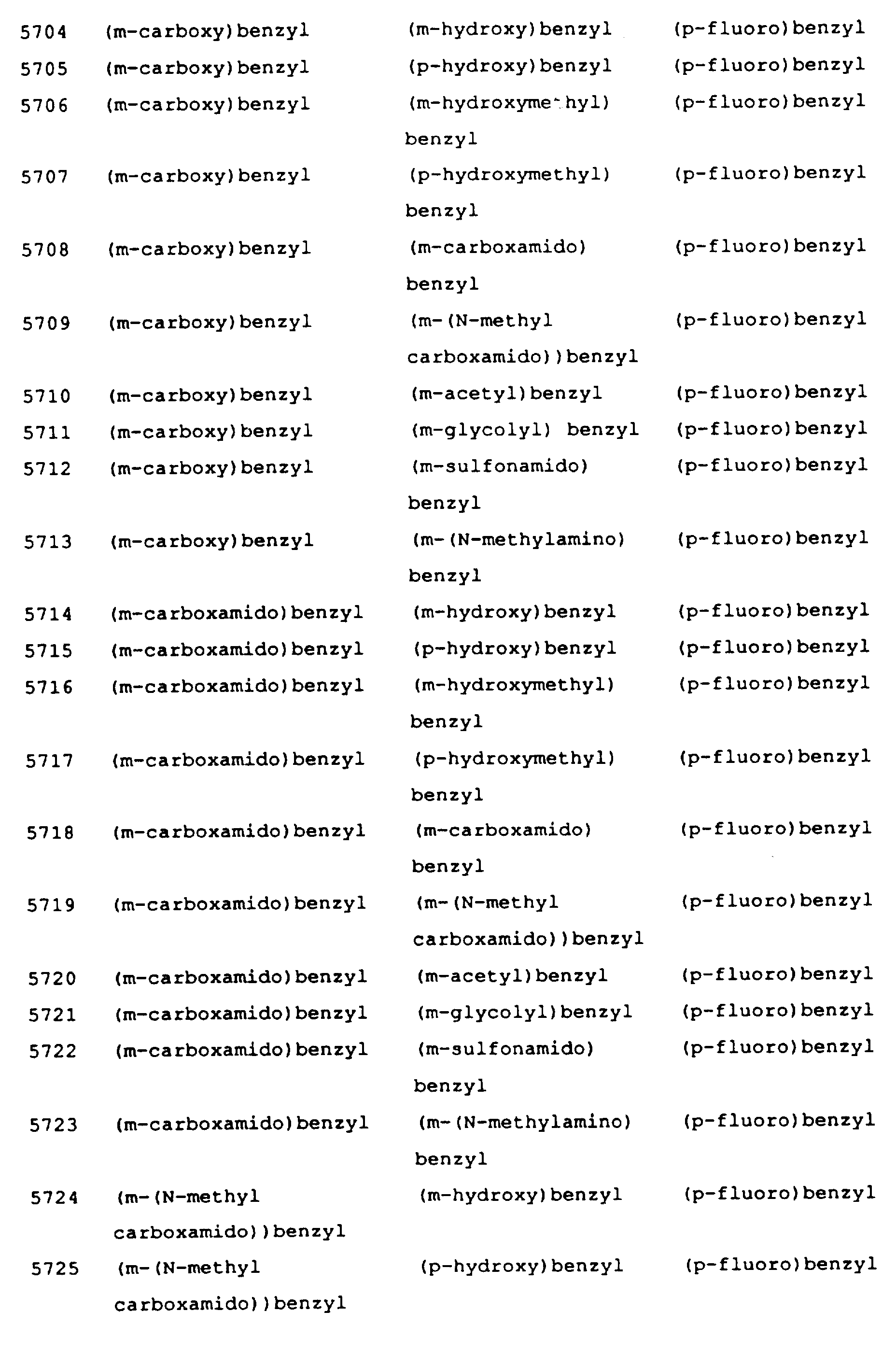

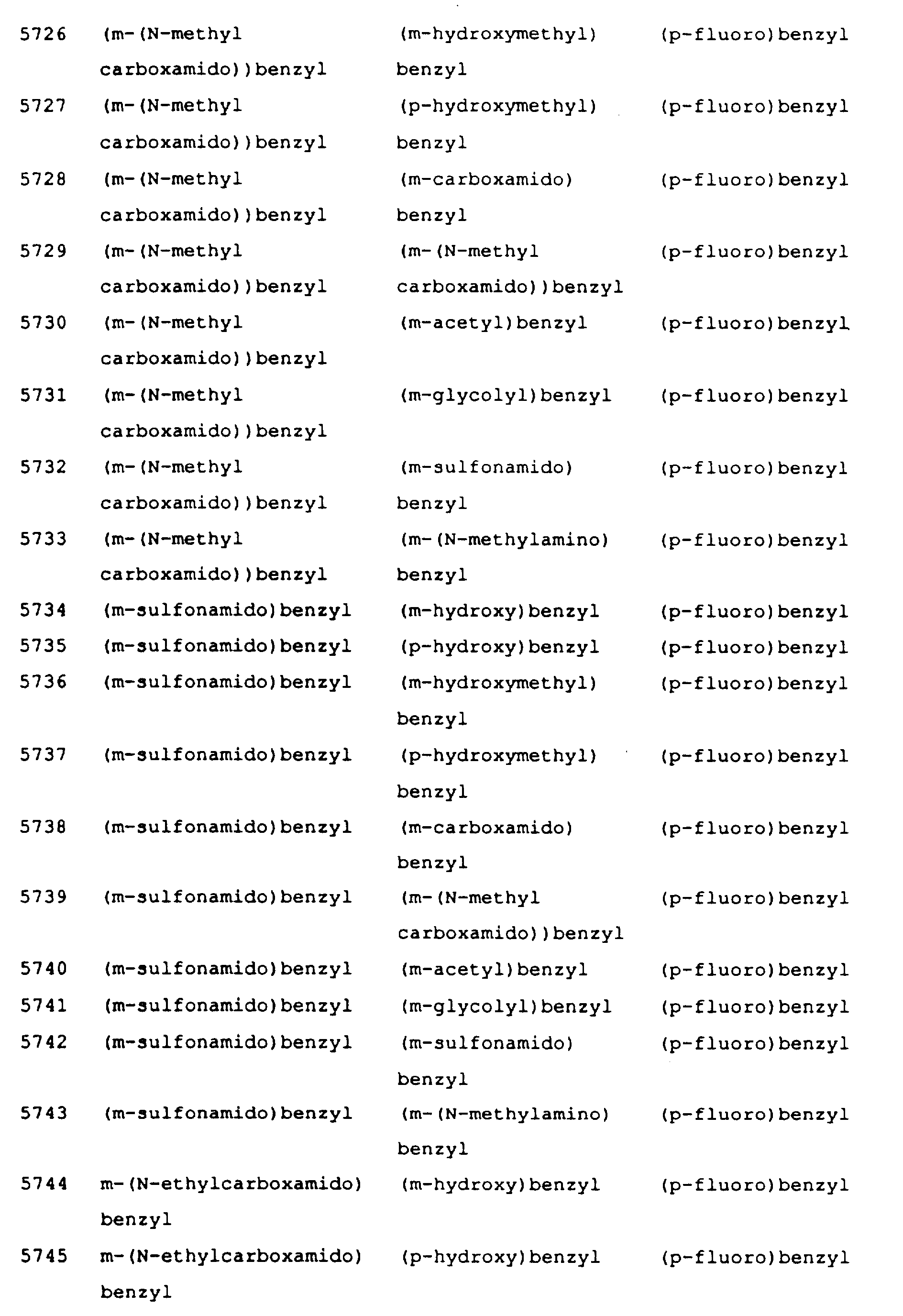

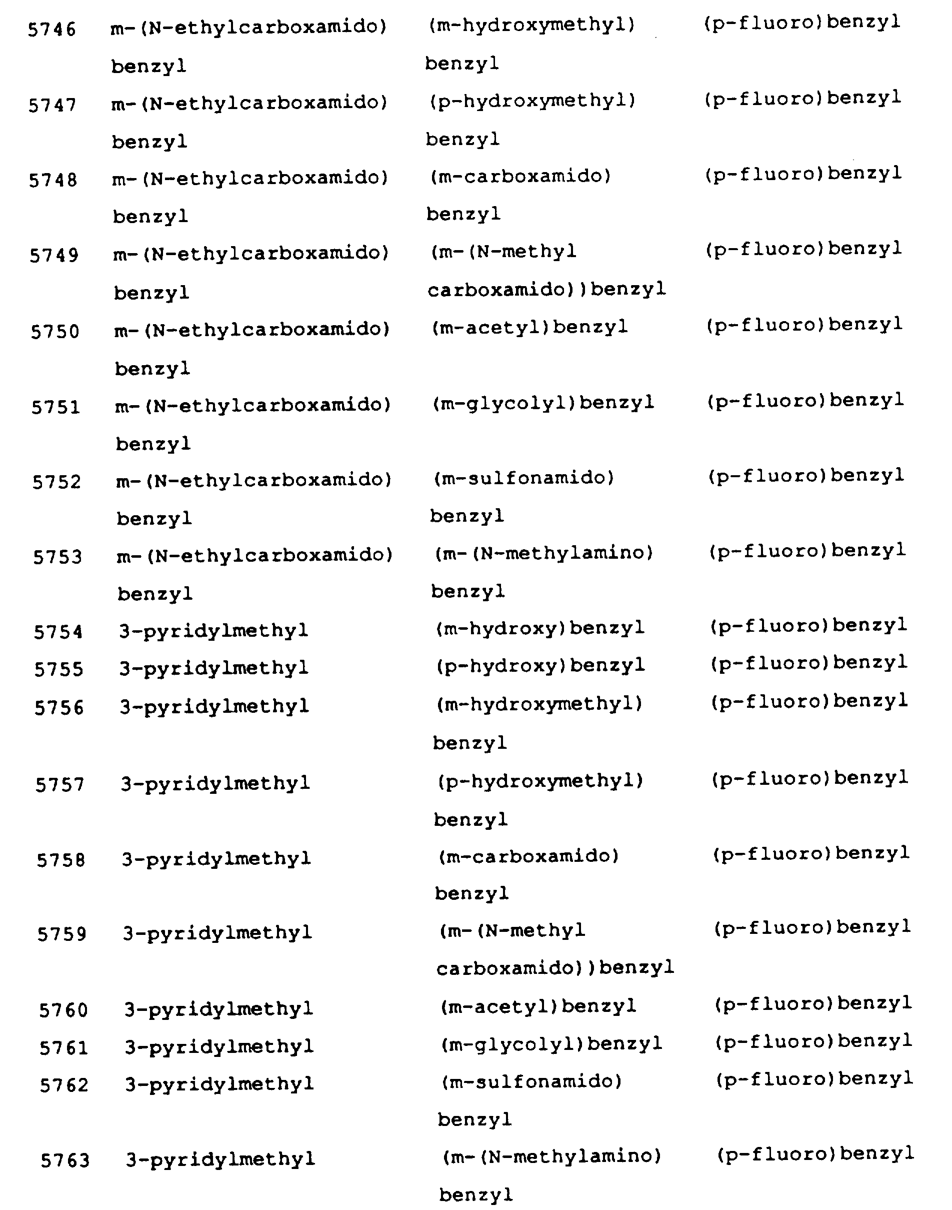

## Claims

1. A compound of the formula (I): 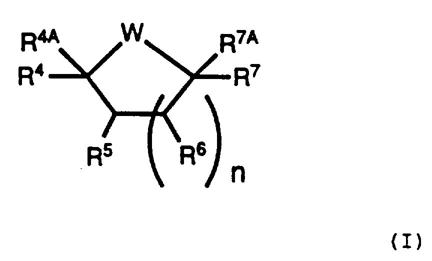 or a pharmaceutically acceptable salt or prodrug form thereof wherein:
R⁴ and R⁷ are independently selected from the following groups:
hydrogen;
C₁-C₈ alkyl substituted with 0-3 R¹¹;
C₂-C₈ alkenyl substituted with 0-3 R¹¹;
C₂-C₈ alkynyl substituted with 0-3 R¹¹;
C₃-C₈ cycloalkyl substituted with 0-3 R¹¹;
C₆-C₁₀ bicycloalkyl substituted with 0-3 R¹¹;
aryl substituted with 0-3 R¹²;
a C₆-C₁₄ carbocyclic residue substituted with 0-3 R¹²;
a heterocyclic ring system substituted with 0-2 R¹², composed of 5 to 10 atoms including at least one nitrogen, oxygen or sulfur atom;
R^{4A} and R^{7A} are independently selected from the following groups:
hydrogen;
C₁-C₄ alkyl substituted with halogen or C₁-C₂ alkoxy;
benzyl substituted with halogen or C₁-C₂ alkoxy;
R⁴ and R^{4A} can alternatively join to form a 5-7 membered carbocyclic ring substituted with 0-2 R¹²;
R⁷ and R^{7A} can alternatively join to form a 5-7 membered carbocyclic ring substituted with 0-2 R¹²;
n is 1;
R⁵ is selected from fluoro, difluoro, =O, C₁-C₃ alkyl or -OR²⁰;
R⁶ is selected from: hydrogen, =O, fluoro, difluoro, C₁-C₃ alkyl or -OR²¹;
R⁵ and R⁶ can alternatively join to form an epoxide ring; -OCH₂SCH₂O-; -OS(=O)O-; -OC(=O)O-; -OCH₂O-; -OC(=S)O-; -OC(=O)C(=O)O-; -OC(CH₃)₂O-; -OC(OCH₃)(CH₂CH₂CH₃)O-; or any hydrolysable group that, when administered to a mammalian subject, is metabolically cleaved to form the original diol wherein R⁵ and R⁶ are -OH;
R²⁰ and R²¹ are independently selected from:
hydrogen;
C₁-C₆ alkyl substituted with 0-3 R¹¹;
C₃-C₆ alkoxyalkyl substituted with 0-3 R¹¹;
C1-C6 alkylcarbonyl substituted with 0-3 R¹¹;
C₁-C₆ alkoxycarbonyl substituted with 0-3 R¹¹;
benzoyl substituted with 0-3 R¹²;
phenoxycarbonyl substituted with 0-3 R¹²;
phenylaminocarbonyl substituted with 0-3 R¹²; or
any hydrolysable group that, when administered to a mammalian subject, is metabolically cleaved to form the original diol wherein R⁵ and R⁶ are -OH;
R¹¹ is selected from one or more of the following:
keto, halogen, cyano, -CH₂NR¹³R¹⁴, -NR¹³R¹⁴, -CO₂R¹³, -OC(=O)R¹³, -OR¹³, C₂-C₆ alkoxyalkyl, -S(O)ₘR¹³, -NHC(-NH)NHR¹³, -C(=NH)NHR¹³, -C(-O)NR¹³R¹⁴, -NR¹⁴C(-O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, C₁-C₄ alkyl, C₂-C₄ alkenyl, C₃-C₆ cycloalkyl, C₃-C₆ cycloalkylmethyl;
1-3 amino acids, linked together via amide bonds and linked to R⁴ or R⁷ via the amine or carboxylate terminus;
a C₅-C₁₄ carbocyclic residue substituted with 0-3 R¹²;
aryl substituted with 0-3 R¹²; or
a heterocyclic ring system substituted with 0-2 R¹², composed of 5 to 10 atoms including at least one nitrogen, oxygen or sulfur atom;
R¹², when a substituent on carbon, is selected from one or more of the following:
phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -OCH₂CO₂H, 2-(1-morpholino)ethoxy; or
a 5- or 6-membered heterocyclic ring containing from 1 to 4 heteroatoms selected from oxygen, nitrogen or sulfur;
or R¹² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R¹² is attached to a saturated carbon atom, it may be carbonyl or thiocarbonyl;
R¹², when a substituent on nitrogen, is selected from one or more of the following:
phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, -CO₂H, C₁-C₄ alkylcarbonyloxy, C₁-C₄ alkylcarbonyl;
R¹³ is H, phenyl, benzyl, C₁-C₆ alkyl, or C₃-C₆ alkoxyalkyl;
R¹⁴ is OH, H, C₁-C₄ alkyl, or benzyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
R¹⁵ is H or CH₃;
m is 0, 1 or 2;
W is selected from:
-N(R²²)C(=Z)N(R²³)-;
-N(R²²)S(=O)N(R²³)-;
wherein:
Z is O, S, or NR²⁴;
R²² and R²³ are independently selected from the following:
hydrogen;
C₁-C₈ alkyl substituted with 0-3 R³¹;
C₂-C₈ alkenyl substituted with 0-3 R³¹;
C₂-C₈ alkynyl substituted with 0-3 R³¹;
C₃-C₈ cycloalkyl substituted with 0-3 R³¹;
C₆-C₁₀ bicycloalkyl substituted with 0-3 R³¹;
aryl substituted with 0-3 R³²;
a C₆-C₁₄ carbocyclic residue substituted with 0-3 R³²;
a heterocyclic ring system substituted with 0-2 R³², composed of 5 to 10 atoms including at least one nitrogen, oxygen or sulfur atom;
R²⁴ is selected from: hydroxy; amino; C₁-C₄ alkyl; C₁-C₄ alkoxy; C₁-C₄ aminoalkyl; cyano; nitro; benzyloxy;
alternatively, R²² can join with R⁴ or R^{4A} to form a five- or six-membered fused heterocyclic or carbocyclic ring substituted with 0-2 R¹²; and
alternatively, R²³ can join with R⁷ or R^{7A} to form a five- or six-membered fused heterocyclic or carbocyclic ring substituted with 0-2 R¹²; and
alternatively, W can join with R⁵ or R⁶ to form a three-to seven-membered fused heterocyclic or carbocyclic ring substituted with 0-2 R¹²;
R³¹ is selected from one or more of the following:
keto, halogen, cyano, -CH₂NR¹³R¹⁴, -NR¹³R¹⁴,
-CO₂R¹³,
-OC(=O)R¹³, -OR¹³, C₂-C₆ alkoxyalkyl, -S(O)ₘR¹³,
-NHC(=NH)NHR¹³, -C(=NH)NHR¹³, -C(=O)NR¹³R¹⁴,
-NR¹⁴C(=O)R¹³, =NOR¹⁴, -NR¹⁴C(-O)OR¹⁴,
-OC(=O)NR¹³R¹⁴, -NR¹³C(=O)NR¹³R¹⁴, -NR¹⁴SO₂NR¹³R¹⁴,
-NR¹⁴SO₂R¹³, -SO₂NR¹³R¹⁴, C₁-C₄ alkyl, C₂-C₄ alkenyl, C₃-C₆ cycloalkyl, C₃-C₆ cycloalkylmethyl;
1-3 amino acids, linked together via amide bonds and linked to R⁴ or R⁷ via the amine or carboxylate terminus;
a C₅-C₁₄ carbocyclic residue substituted-with 0-3 R³²;
aryl substituted with 0-3 R³²; or
a heterocyclic ring system substituted with 0-2 R³², composed of 5 to 10 atoms including at least one nitrogen, oxygen or sulfur atom;
R³², when a substituent on carbon, is selected from one or more of the following:
phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -OCH₂CO₂H, 2-(1-morpholino)ethoxy, -C(R¹⁴)-N(OR¹⁴); or
a 5- or 6-membered heterocyclic ring containing from 1 to 4 heteroatoms selected from oxygen, nitrogen or sulfur;
or R³² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R³² is attached to a saturated carbon atom, it may be carbonyl or thiocarbonyl;
R³², when a substituent on nitrogen, is selected from one or more of the following:
phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, -CO₂H, C₁-C₄ alkylcarbonyloxy, C₁-C₄ alkylcarbonyl, -C(R¹⁴)=N(OR¹⁴);
provided that:
R⁴, R^{4A}, R⁷, and, R^{7A} are not all hydrogen;
when R⁴ and R^{4A} are both hydrogen, at least one of the following is not hydrogen: R²², R²³.

2. A compound of claim 1 of formula (I): 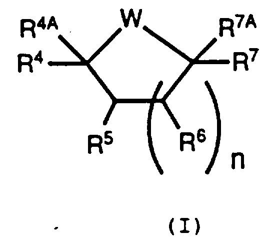 or a pharmaceutically acceptable salt or prodrug form thereof wherein:
R⁴ and R⁷ are independently selected from the following groups:
hydrogen;
C₁-C₄ alkyl substituted with 0-3 R¹¹;
C₃-C₄ alkenyl substituted with 0-3 R¹¹;
C₃-C₄ alkynyl substituted with 0-3 R¹¹;
R^{4A} and R^{7A} are hydrogen;
n is 1;
R⁵ is selected from fluoro, difluoro, =O, or -OR²⁰;
R⁶ is selected from: hydrogen, =O, fluoro, difluoro, or -OR²¹;
R⁵ and R⁶ can alternatively join to form an epoxide ring; -OCH₂SCH₂O-; -OS(=O)O-; -OC(=O)O-; -OCH₂O-; -OC(=S)O-; -OC(=O)C(=O)O-; -OC(CH₃)₂O-; -OC(OCH₃)(CH₂CH₂CH₃)O-; or any hydrolysed group that, when administered to a mammalian subject, is metabolically cleaved to form the original diol wherein R⁵ and R⁶ are -OH;
R²⁰ and R²¹ are independently selected from:
hydrogen;
C₁-C₆ alkylcarbonyl;
C₁-C₆ alkoxycarbonyl;
benzoyl; or
any hydrolysable group that, when administered to a mammalian subject, is metabolically cleaved to form the original diol wherein R⁵ and R⁶ are -OH;
R¹¹ is selected from one or more of the following:
keto, halogen, cyano, -CH₂NR¹³R¹⁴, -NR¹³R¹⁴, -CO₂R¹³,
-OC(=O)R¹³, -OR¹³, C₂-C₄ alkoxyalkyl, -S(O)ₘR¹³, C₁-C₄ alkyl, C₂-C₄ alkenyl, C₃-C₆ cycloalkyl;
a C₅-C₁₄ carbocyclic residue substituted with 0-3 R¹²;
aryl substituted with 0-3 R¹²; or
a heterocyclic ring system substituted with 0-2 R¹², composed of 5 to 10 atoms including at least one nitrogen, oxygen or sulfur atom;
R¹², when a substituent on carbon, is selected from one or more of the following:
phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴; or
a 5- or 6-membered heterocyclic ring containing from 1 to 4 heteroatoms selected from oxygen, nitrogen or sulfur;
or R¹² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R¹² is attached to a saturated carbon atom, it may be carbonyl or thiocarbonyl;
R¹², when a substituent on nitrogen, is selected from one or more of the following:
phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, C₁-C₄ alkylcarbonyloxy, C₁-C₄ alkylcarbonyl, -CO₂H;
R¹³ is H, C₁-C₆ alkyl, or C₃-C₆ alkoxyalkyl;
R¹⁴ is OH, H, C₁-C₄ alkyl, or benzyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
R¹⁵ is H or CH₃;
m is 0, 1 or 2;
W is
-N(R²²)C(=Z)N(R²³)-;
wherein:
Z is O, S, N-CN, N-OH, N-OCH₃;
R²² and R²³ are independently selected from the following:
hydrogen;
C₁-C₈ alkyl substituted with 0-3 R³¹;
C₃-C₈ alkenyl substituted with 0-3 R³¹;
C₃-C₈ alkynyl substituted with 0-3 R³¹;
C₃-C₆ cycloalkyl substituted with 0-3 R³¹;
R³¹ is selected from one or more of the following:
keto, halogen, cyano, -CH₂NR¹³R¹⁴, -NR¹³R¹⁴, -CO₂R¹³, -OC(=O)R¹³, -OR¹³, C₂-C₄ alkoxyalkyl, -S(O)ₘR¹³, C₁-C₄ alkyl, C₂-C₄ alkenyl, C₃-C₆ cycloalkyl;
a C₅-C₁₄ carbocyclic residue substituted with 0-3 R¹²;
aryl substituted with 0-3 R³²; or
a heterocyclic ring system substituted with 0-2 R³², composed of 5 to 10 atoms including at least one nitrogen, oxygen or sulfur atom;
R³², when a substituent on carbon, is selected from one or more of the following:
phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -C(R¹⁴)=N(OR¹⁴); or
a 5- or 6-membered heterocyclic ring containing from 1 to 4 heteroatoms selected from oxygen, nitrogen or sulfur;
or R³² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R³² is attached to a saturated carbon atom, it may be carbonyl or thiocarbonyl;
R³², when a substituent on nitrogen, is selected from one or more of the following:
phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, C₁-C₄ alkylcarbonyloxy, C₁-C₄ alkylcarbonyl, -CO₂H, -C(R¹⁴)=N(OR¹⁴);
provided that:
R⁴, R^{4A}, R⁷, and R^{7A} are not all hydrogen;
when R⁴ and R^{4A} are both hydrogen, at least one of the following is not hydrogen: R²², R²³.

3. A compound of Claim 1 of formula (II): 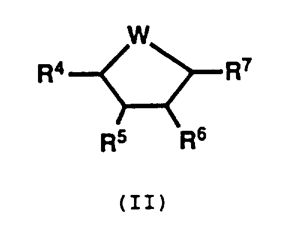 or a pharmaceutically acceptable salt or prodrug form thereof wherein:
R⁴ and R⁷ are independently selected from the following groups:
hydrogen;
C₁-C₄ alkyl substituted with 0-3 R¹¹;
C₃-C₄ alkenyl substituted with 0-3 R¹¹;
R⁵ is -OR²⁰;
R⁶ is hydrogen or -OR²¹;
R²⁰ and R²¹ are independently hydrogen or any hydrolysable group that, when administered to a mammalian subject, is metabolically cleaved to form the original diol wherein R⁵ and R⁶ are -OH;
R¹¹ is selected from one or more of the following:
keto, halogen, -CH₂NR¹³R¹⁴, -NR¹³R¹⁴, -OR¹³, C₂-C₄ alkoxyalkyl, C₁-C₄ alkyl, C₂-C₄ alkenyl, C₃-C₆ cycloalkyl;
aryl substituted with 0-3 R¹²; or
a heterocyclic ring system substituted with 0-2 R¹², composed of 5 to 10 atoms including at least one nitrogen, oxygen or sulfur atom;
R¹², when a substituent on carbon, is selected from one or more of the following:
phenyl, benzyl, phenethyl, phenoxy, benzyloxy, halogen, C₁-C₄ alkyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, methylenedioxy, C₁-C₄ haloalkyl, C₁-C₄ alkylcarbonyl, C₁-C₄ alkylcarbonylamino, hydroxy, hydroxymethyl; or
a 5- or 6-membered heterocyclic ring containing from 1 to 4 heteroatoms selected from oxygen, nitrogen or sulfur;
R¹², when a substituent on nitrogen, is selected from benzyl or methyl;
R¹³ is H, C₁-C₂ alkyl, or C₃-C₆ alkoxyalkyl;
R¹⁴ is OH, H or C₁-C₂ alkyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
W is
-N(R²²)C(=Z)N(R²³)-;
wherein:
Z is O, S, or N-CN;
R²² and R²³ are independently selected from the following:
hydrogen;
C₁-C₄ alkyl substituted with 0-3 R³¹;
C₃-C₄ alkenyl substituted with 0-3 R³¹;
R³¹ is selected from one or more of the following:
keto, halogen, -CH₂NR¹³R¹⁴, -NR¹³R¹⁴, -OR¹³, C₂-C₄ alkoxyalkyl, C₁-C₄ alkyl, C₂-C₄ alkenyl, C₃-C₆ cycloalkyl;
aryl substituted with 0-3 R³²; or
a heterocyclic ring system substituted with 0-2 R³², composed of 5 to 10 atoms including at least one nitrogen, oxygen or sulfur atom;
R³², when a substituent on carbon, is selected from one or more of the following:
phenyl, benzyl, phenethyl, phenoxy, benzyloxy, halogen, C₁-C₄ alkyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, methylenedioxy, C₁-C₄ haloalkyl, C₁-C₄ alkylcarbonyl, C₁-C₄ alkylcarbonylamino, hydroxy, hydroxymethyl, -C(R¹⁴)=N(OR¹⁴); or
a 5- or 6-membered heterocyclic ring containing from 1 to 4 heteroatoms selected from oxygen, nitrogen or sulfur;
R³², when a substituent on nitrogen, is selected from benzyl or methyl;
provided that:
when R⁴ is hydrogen, R⁷ is not hydrogen;
when R⁴ is hydrogen, at least one of the following is not hydrogen: R²², R²³.

4. A compound of Claim 3 wherein:
R⁴ and R⁷ are independently selected from the following groups:
hydrogen;
C₁-C₃ alkyl substituted with 0-1 R¹¹;
R⁵ is -OR²⁰;
R⁶ is hydrogen or -OR²¹;
R²⁰ and R²¹ are independently hydrogen or any hydrolysable group that, when administered to a mammalian subject, is metabolically cleaved to form the original diol wherein R⁵ and R⁶ are -OH;
R¹¹ is selected from one or more of the following:
halogen, -OR¹³, C₁-C₄ alkyl, C₃-C₅ cycloalkyl;
aryl substituted with 0-2 R¹²; or
a heterocyclic ring system chosen from pyridyl, pyrimidinyl, triazinyl, furanyl, thienyl, pyrrolyl, pyrazolyl, imidazolyl, tetrazolyl, benzofuranyl, indolyl, quinolinyl, isoquinolinyl;
R¹², when a substituent on carbon, is selected from one or more of the following:
benzyloxy, halogen, methyl, C₁-C₄ alkoxy, CF₃, 2-(1-morpholino)ethoxy, -CO₂H, hydroxamic acid, hydrazide, oxime, cyano, boronic acid, sulfonamide, formyl, C₃-C₆ cycloalkoxy, C₁-C₄ alkyl substituted with -NR¹³R¹⁴, -NR¹³R¹⁴, hydroxy, hydroxymethyl; or
R¹², when a substituent on nitrogen, is methyl;
R¹³ is H or methyl;
R¹⁴ is OH, H or methyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
W is -N(R²²)C(=O)N(R²³)- or -N(R²²)C(=N-CN)N(R²³)-;
R²² and R²³ are independently selected from the following:
hydrogen;
C₁-C₄ alkyl substituted with 0-1 R³¹;
C₃-C₄ alkenyl substituted with 0-1 R³¹;
R³¹ is selected from one or more of the following:
halogen, -OR¹³, C₁-C₄ alkyl, C₃-C₅ cycloalkyl;
aryl substituted with 0-2 R³²; or
a heterocyclic ring system chosen from pyridyl, pyrimidinyl, triazinyl, furanyl, thienyl, pyrrolyl, pyrazolyl, imidazolyl, tetrazolyl, benzofuranyl, indolyl, quinolinyl, isoquinolinyl;
R³², when a substituent on carbon, is selected from one or more of the following:
benzyloxy, halogen, methyl, C₁-C₄ alkoxy, CF₃, 2-(1-morpholino)ethoxy, -CO₂H, hydroxamic acid, hydrazide, oxime, cyano, boronic acid, sulfonamide, formyl, C₃-C₆ cycloalkoxy, C₁-C₄ alkyl substituted with -NR¹³R¹⁴, -NR¹³R¹⁴, hydroxy, hydroxymethyl, -C(R¹⁴)=N(OR¹⁴); or
R³², when a substituent on nitrogen, is methyl;
provided that:
when R⁴ is hydrogen, R⁷ is not hydrogen;
when R⁴ is hydrogen, at least one of the following is not hydrogen: R²² and R²³.

5. A compound of Claim 3 wherein:
R⁴ and R⁷ are benzyl;
R⁵ is -OH;
R⁶ is hydrogen or -OH;
R¹³ is H or methyl;
R¹⁴ is H or methyl;
W is -N(R²²)C(=O)N(R²³)- or -N(R²²)C(=N-CN)N(R²³)-;
R²² and R²³ are independently selected from the following:
hydrogen;
C₁-C₄ alkyl substituted with 0-1 R³¹;
R³¹ is selected from one or more of the following:
C₃-C₅ cycloalkyl;
aryl substituted with 0-2 R³²; or
a heterocyclic ring system chosen from pyridyl, thienyl, quinolinyl, or isoquinolinyl;
R³², when a substituent on carbon, is selected from one or more of the following:
-CONH₂, -CO₂H, -CHO, -CH₂NHOH, -CH₂NR¹³R¹⁴, -NR¹³R¹⁴, hydroxy, hydroxymethyl, -C (R¹⁴)=N (OR¹⁴); or
R³², when a substituent on nitrogen, is methyl.

6. A compound of Claim 1 of formula: 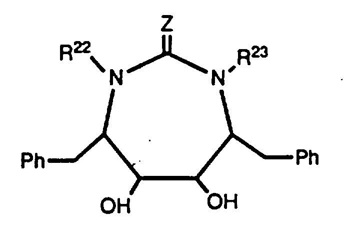 wherein Z is O, S, or N-CN
wherein R²² and R²³ are independently selected from the group consisting of:
hydrogen, allyl, propyl, cyclopropylmethyl, n-butyl, i-butyl, pyridylmethyl, methallyl, n-pentyl, i-pentyl, hexyl, benzyl, pyridylmethyl, isoprenyl, propargyl, picolinyl, methoxyethyl, cyclohexylmethyl, dimethyl-butyl, ethoxyethyl, methyl-oxazolinylmethyl, naphthylmethyl, methyloxazolinylmethyl, vinyloxyethyl, pentafluorobenzyl, quinolinylmethyl, carboxybenzyl, chloro-thienyl, picolinyl, benzyloxybenzyl, phenylbenzyl, adamantylethyl, cyclopropylmethoxybenzyl, ethoxybenzyl, hydroxybenzyl, hydroxymethylbenzyl, aminobenzyl, formylbenzyl, cyanobenzyl, cinnamyl, allyloxybenzyl, fluorobenzyl, cyclobutylmethyl, formaldoximebenzyl, cyclopentylmethyl, nitrobenzyl, nitrilobenzyl, carboxamidobenzyl, carbomethoxybenzyl, tetrazolylbenzyl and dimethylallyl.

7. A compound of Claim 1 of formula (IIa): 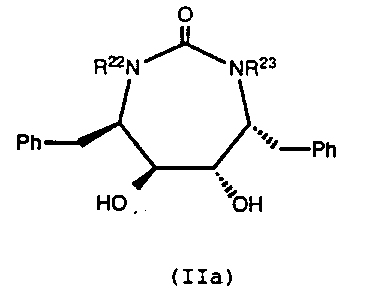 wherein R²² and R²³ are independently selected from the group consisting of:
hydrogen, allyl, propyl, cyclopropylmethyl, n-butyl, i-butyl, pyridylmethyl, methallyl, n-pentyl, i-pentyl, hexyl, benzyl, pyridylmethyl, isoprenyl, propargyl, picolinyl, methoxyethyl, cyclohexylmethyl, dimethyl-butyl, ethoxyethyl, methyl-oxazolinylmethyl, naphthylmethyl, methyloxazolinylmethyl, vinyloxyethyl, pentafluorobenzyl, quinolinylmethyl, carboxybenzyl, chloro-thienyl, picolinyl, benzyloxybenzyl, phenylbenzyl, adamantylethyl, cyclopropylmethoxybenzyl, ethoxybenzyl, hydroxybenzyl, hydroxymethylbenzyl, aminobenzyl, formylbenzyl, cyanobenzyl, cinnamyl, allyloxybenzyl, fluorobenzyl, cyclobutylmethyl, formaldoximebenzyl, cyclopentylmethyl, nitrobenzyl, nitrilobenzyl, carboxamidobenzyl, carbomethoxybenzyl, tetrazolylbenzyl, dimethylallyl, amidinobenzyl, and (boronic acid)benzyl.

8. A compound of Claim 1 of formula (IIa): 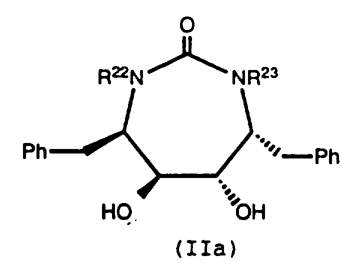 which is selected from the group consisting of:
the compound of formula (IIa) wherein R²² is allyl and R²³ is allyl;
the compound of formula (IIa) wherein R²² is propyl and R²³ is propyl;
the compound of formula (IIa) wherein R²² is cyclopropylmethyl and R²³ is cyclopropylmethyl;
the compound of formula (IIa) wherein R²² is n-butyl and R²³ is n-butyl;
the compound of formula (IIa) wherein R²² is i-pentyl and R²³ is i-pentyl;
the compound of formula (IIa) wherein R²² is 4-pyridylmethyl and R²³ is 4-pyridylmethyl;
the compound of formula (IIa) wherein R²² is 2-methallyl and R²³ is 2-methallyl;
the compound of formula (IIa) wherein R²² is n-pentyl and R²³ is n-pentyl;
the compound of formula (IIa) wherein R²² is i-butyl and R²³ is i-butyl;
the compound of formula (IIa) wherein R²² is benzyl and R²³ is benzyl;
the compound of formula (IIa) wherein R²² is 3-pyridylmethyl and R²³ is 3-pyridylmethyl;
the compound of formula (IIa) wherein R²² is allyl and R²³ is isoprenyl;
the compound of formula (IIa) wherein R²² is 3-propargyl and R²³ is 3-propargyl;
the compound of formula (IIa) wherein R²² is 2-picolinyl and R²³ is 2-picolinyl;
the compound of formula (IIa) wherein R²² is 2-methoxyethyl and R²³ is 2-methoxyethyl;
the compound of formula (IIa) wherein R²² is cyclohexylmethyl and R²³ is cyclohexylmethyl;
the compound of formula (IIa) wherein R²² is 3,3-dimethyl-1-butyl and R²³ is 3,3-dimethyl-1-butyl;
the compound of formula (IIa) wherein R²² is 2-ethoxyethyl and R²³ is 2-ethoxyethyl;
the compound of formula (IIa) wherein R²² is 3-methyl-5-oxazolinylmethyl and R²³ is hydrogen;
the compound of formula (IIa) wherein R²² is 1-naphthylmethyl and R²³ is 1-naphthylmethyl;
the compound of formula (IIa) wherein R²² is 3-methyloxazolinylmethyl and R²³ is 3-methyloxazolinylmethyl;
the compound of formula (IIa) wherein R²² is 2-vinyloxyethyl and R²³ is 2-vinyloxyethyl;
the compound of formula (IIa) wherein R²² is 2,3,4,5,6-pentafluorobenzyl and R²³ is 2,3,4,5,6-pentafluorobenzyl;
the compound of formula (IIa) wherein R²² is benzyl and R²³ is 2-quinolinylmethyl;
the compound of formula (IIa) wherein R²² is 4-carboxybenzyl and R²³ is 4-carboxybenzyl;
the compound of formula (IIa) wherein R²² is 5-chloro-2-thienyl and R²³ is 5-chloro-2-thienyl;
the compound of formula (IIa) wherein R²² is 2-quinolinylmethyl and R²³ is 2-quinolinylmethyl;
the compound of formula (IIa) wherein R²² is 2-propyl and R²³ is 2-picolinyl;
the compound of formula (IIa) wherein R²² is 3-benzyloxybenzyl and R²³ is 3-benzyloxybenzyl;
the compound of formula (IIa) wherein R²² is 4-phenylbenzyl and R²³ is phenylbenzyl;
the compound of formula (IIa) wherein R²² is 2-adamantylethyl and R²³ is 2-adamantylethyl;
the compound of formula (IIa) wherein R²² is hydrogen and R²³ is cyclopropylmethyl;
the compound of formula (IIa) wherein R²² is 2-picolinyl and R²³ is 2-naphthylmethyl;
the compound of formula (IIa) wherein R²² is 3-allyl and R²³ is hydrogen;
the compound of formula (IIa) wherein R²² is 3-allyl and R²³ is 2-picolinyl;
the compound of formula (IIa) wherein R²² is 3-allyl and R²³ is 4-picolinyl;
the compound of formula (IIa) wherein R²² is 3-benzyloxybenzyl and R²³ is 3-benzyloxybenzyl;
the compound of formula (IIa) wherein R²² is 3-cyclopropylmethoxybenzyl and R²³ is 3-cyclopropylmethoxybenzyl;
the compound of formula (IIa) wherein R²² is 3-ethoxybenzyl and R²³ is 3-ethoxybenzyl;
the compound of formula (IIa) wherein R²² is 4-benzyloxybenzyl and R²³ is 4-benzyloxybenzyl.
the compound of formula (IIa) wherein R²² is 3-hydroxybenzyl and R²³ is 3-hydroxybenzyl;
the compound of formula (IIa) wherein R²² is 4-hydroxybenzyl and R²³ is 4-hydroxybenzyl;
the compound of formula (IIa) wherein R²² is 3-hydroxymethylbenzyl and R²³ is 3-hydroxymethylbenzyl;
the compound of formula (IIa) wherein R²² is 4-hydroxymethylbenzyl and R²³ is 4-hydroxymethylbenzyl;
the compound of formula (IIa) wherein R²² is 3-aminobenzyl and R²³ is 3-aminobenzyl;
the compound of formula (IIa) wherein R²² is 3-carboxylbenzyl and R²³ is 3-carboxylbenzyl;
the compound of formula (IIa) wherein R²² is 3-formylbenzyl and R²³ is 3-formylbenzyl;
the compound of formula (IIa) wherein R²² is 3-cyanobenzyl and R²³ is 3-cyanobenzyl;
the compound of formula (IIa) wherein R²² is 2-naphthylmethyl and R²³ is 2-naphthylmethyl;
the compound of formula (IIa) wherein R²² is n-butyl and R²³ is benzyl;
the compound of formula (IIa) wherein R²² is allyl and R²³ is cyclopropylmethyl;
the compound of formula (IIa) wherein R²² is n-butyl and R²³ is cyclopropylmethyl
the compound of formula (IIa) wherein R²² is 3-methallyl and R²³ is benzyl;
the compound of formula (IIa) wherein R²² is benzyl and R²³ is ethyl;
the compound of formula (IIa) wherein R²² is benzyl and R²³ is 4-picolinyl;
the compound of formula (IIa) wherein R²² is cyclopropylmethyl and R²³ is 4-picolinyl;
the compound of formula (IIa) wherein R²² is benzyl and R²³ is cyclopentylmethyl;
the compound of formula (IIa) wherein R²² is cyclopropylmethyl and R²³ is cyclopentylmethyl;
the compound of formula (IIa) wherein R²² is benzyl and R²³ is n-propyl;
the compound of formula (IIa) wherein R²² is cyclopropylmethyl and R²³ is cinnamyl;
the compound of formula (IIa) wherein R²² is cyclopropylmethyl and R²³ is 2-naphthylmethyl;
the compound of formula (IIa) wherein R²² is cyclopentylmethyl and R²³ is 2-naphthylmethyl;
- the compound of formula (IIa) wherein R²² is benzyl and R²³ is 2-naphthylmethyl;
the compound of formula (IIa) wherein R²² is cyclopropylmethyl and R²³ is 2-picolinyl;
the compound of formula (IIa) wherein R²² is 3-cyanobenzyl and R²³ is 3-cyanobenzyl;
the compound of formula (IIa) wherein R²² is 3-allyl and R²³ is 2-naphthylmethyl;
the compound of formula (IIa) wherein R²² is n-propyl and R²³ is 2-naphthylmethyl;
the compound of formula (IIa) wherein R²² is n-butyl and R²³ is 2-naphthylmethyl;
the compound of formula (IIa) wherein R²² is H and R²³ is 2-naphthylmethyl;
the compound of formula (IIa) wherein R²² is 4-picolinyl and R²³ is 2-naphthylmethyl;
the compound of formula (IIa) wherein R²² is 3-allyl and R²³ is cyclopentylmethyl;
the compound of formula (IIa) wherein R²² is 3-allyl and R²³ is 2-quinolinylmethyl;
the compound of formula (IIa) wherein R²² is 3-picolinyl and R²³ is cyclopropylmethyl;
the compound of formula (IIa) wherein R²² is 3-picolinyl and R²³ is 2-naphthylmethyl;
the compound of formula (IIa) wherein R²² is 3-allyloxybenzyl and R²³ is 3-allyloxybenzyl;
the compound of formula (IIa) wherein R²² is 3-allyloxybenzyl and R²³ is 3-hydroxybenzyl;
the compound of formula (IIa) wherein R²² is 3-picolinyl and R²³ is 3-picolinyl;
the compound of formula (IIa) wherein R²² is 2-naphthylmethyl and R²³ is 4-fluorobenzyl;
the compound of formula (IIa) wherein R²² is 3-carbomethoxybenzyl and R²³ is 3-carbomethyoxybenzyl;
the compound of formula (IIa) wherein R²² is 4-formylbenzyl and R²³ is 4-formylbenzyl;
the compound of formula (IIa) wherein R²² is 4-cyanobenzyl and R²³ is 4-cyanobenzyl;
the compound of formula (IIa) wherein R²² is 4-hydroxybenzyl and R²³ is n-propyl;
the compound of formula (IIa) wherein R²² is 3-hydroxybenzyl and R²³ is n-propyl;
the compound of formula (IIa) wherein R²² is 3-carboxybenzyl and R²³ is 3-carboxybenzyl;
the compound of formula (IIa) wherein R²² is cyclobutylmethyl and R²³ is cyclobutylmethyl;
the compound of formula (IIa) wherein R²² is cyclopentylmethyl and R²³ is cyclopentylmethyl;
the compound of formula (IIa) wherein R²² is n-butyl and R²³ is 3-methallyl;
the compound of formula (IIa) wherein R²² is n-butyl and R²³ is cyclopentylmethyl;
the compound of formula (IIa) wherein R²² is 3-formaldoximebenzyl and R²³ is 3-formaldoximebenzyl;
the compound of formula (IIa) wherein R²² is cyclopropylmethyl and R²³ is 3-hydroxybenzyl;
the compound of formula (IIa) wherein R²² is cyclopropylmethyl and R²³ is 4-hydroxybenzyl;
the compound of formula (IIa) wherein R²² is 3-(N-methylamino)benzyl and R²³ is 3-(N-methylamino)benzyl;
the compound of formula (IIa) wherein R²² is 3-acetylbenzyl and R²³ is 3-acetylbenzyl;
the compound of formula (IIa) wherein R²² is 3-hydroxylaminobenzyl and R²³ is 3-hydroxylaminobenzyl;
the compound of formula (IIa) wherein R²² is 2-naphthylmethyl and R²³ is 3-hydroxybenzyl;
the compound of formula (IIa) wherein R²² is 4-hydroxymethylbenzyl and R²³ is 3-hydroxybenzyl;
the compound of formula (IIa) wherein R²² is N-methyl-(3-amido)benzyl and R²³ is N-methyl-(3-amido)benzyl;
the compound of formula (IIa) wherein R²² is N-methyl-(3-amido)benzyl and R²³ is 3-(amidino)benzyl;
the compound of formula (IIa) wherein R²² is 3-(5-tetrazolyl)benzyl and R²³ is cyclopropylmethyl;
the compound of formula (IIa) wherein R²² is 3-(5-tetrazolyl)benzyl and R²³ is 3-(5-tetrazolyl) benzyl;
the compound of formula (IIa) wherein R²² is phenylmethyl-3-boronic acid and R²³ is phenylmethyl-3-boronic acid.

9. A compound of Claim 1 of formula (IIa): 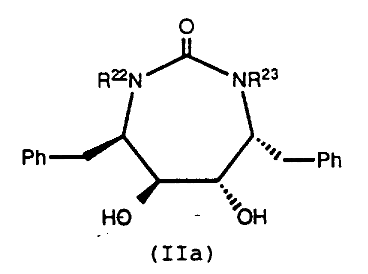 which is selected from the group consisting of:
the compound of formula (IIa) wherein R²² is allyl and R²³ is allyl;
the compound of formula (IIa) wherein R²² is propyl and R²³ is propyl;
the compound of formula (IIa) wherein R²² is cyclopropylmethyl and R²³ is cyclopropylmethyl;
the compound of formula (IIa) wherein R²² is n-butyl and R²³ is n-butyl;
the compound of formula (IIa) wherein R²² is i-pentyl and R²³ is i-pentyl;
the compound of formula (IIa) wherein R²² is 2-methallyl and R²³ is 2-methallyl;
the compound of formula (IIa) wherein R²² is n-pentyl and R²³ is n-pentyl;
the compound of formula (IIa) wherein R²² is benzyl and R²³ is benzyl;
the compound of formula (IIa) wherein R²² is allyl and R²³ is isoprenyl;
the compound of formula (IIa) wherein R²² is 3-hydroxybenzyl and R²³ is 3-hydroxybenzyl;
the compound of formula (IIa) wherein R²² is 4-hydroxybenzyl and R²³ is 4-hydroxybenzyl;
the compound of formula (IIa) wherein R²² is 3-hydroxymethylbenzyl and R²³ is 3-hydroxymethylbenzyl;
the compound of formula (IIa) wherein R²² is 4-hydroxymethylbenzyl and R²³ is 4-hydroxymethylbenzyl;
the compound of formula (IIa) wherein R²² is 3-aminobenzyl and R²³ is 3-aminobenzyl;
the compound of formula (IIa) wherein R²² is 3-carboxylbenzyl and R²³ is 3-carboxylbenzyl;
the compound of formula (IIa) wherein R²² is 3-formylbenzyl and R²³ is 3-formylbenzyl;
the compound of formula (IIa) wherein R²² is 3-cyanobenzyl and R²³ is 3-cyanobenzyl;
the compound of formula (IIa) wherein R²² is 2-naphthylmethyl and R²³ is 2-naphthylmethyl;
the compound of formula (IIa) wherein R²² is n-butyl and R²³ is benzyl;
the compound of formula (IIa) wherein R²² is allyl and R²³ is cyclopropylmethyl;
the compound of formula (IIa) wherein R²² is n-butyl and R²³ is cyclopropylmethyl
the compound of formula (IIa) wherein R²² is 3-methallyl and R²³ is benzyl;
the compound of formula (IIa) wherein R²² is benzyl and R²³ is ethyl;
the compound of formula (IIa) wherein R²² is benzyl and R²³ is 4-picolinyl;
the compound of formula (IIa) wherein R²² is cyclopropylmethyl and R²³ is 4-picolinyl;
the compound of formula (IIa) wherein R²² is benzyl and R²³ is cyclopentylmethyl;
the compound of formula (IIa) wherein R²² is cyclopropylmethyl and R²³ is cyclopentylmethyl;
the compound of formula (IIa) wherein R²² is benzyl and R²³ is n-propyl;
the compound of formula (IIa) wherein R²² is cyclopropylmethyl and R²³ is cinnamyl;
the compound of formula (IIa) wherein R²² is cyclopropylmethyl and R²³ is 2-naphthylmethyl;
the compound of formula (IIa) wherein R²² is cyclopentylmethyl and R²³ is 2-naphthylmethyl;
the compound of formula (IIa) wherein R²² is benzyl and R²³ is 2-naphthylmethyl;
the compound of formula (IIa) wherein R²² is cyclopropylmethyl and R²³ is 2-picolinyl;
the compound of formula (IIa) wherein R²² is 3-cyanobenzyl and R²³ is 3-cyanobenzyl;
the compound of formula (IIa) wherein R²² is 3-allyl and R²³ is 2-naphthylmethyl;
the compound of formula (IIa) wherein R²² is n-propyl and R²³ is 2-naphthylmethyl;
the compound of formula (IIa) wherein R²² is n-butyl and R²³ is 2-naphthylmethyl;
the compound of formula (IIa) wherein R²² is H and R²³ is 2-naphthylmethyl;
the compound of formula (IIa) wherein R²² is 4-picolinyl and R²³ is 2-naphthylmethyl;
the compound of formula (IIa) wherein R²² is 3-allyl and R²³ is cyclopentylmethyl;
the compound of formula (IIa) wherein R²² is 3-allyl and R²³ is 2-quinolinylmethyl;
the compound of formula (IIa) wherein R²² is 3-picolinyl and R²³ is cyclopropylmethyl;
the compound of formula (IIa) wherein R²² is 3-picolinyl and R²³ is 2-naphthylmethyl;
the compound of formula (IIa) wherein R²² is 3-allyloxybenzyl and R²³ is 3-allyloxybenzyl;
the compound of formula (IIa) wherein R²² is 3-allyloxybenzyl and R²³ is 3-hydroxybenzyl;
the compound of formula (IIa) wherein R²² is 3-picolinyl and R²³ is 3-picolinyl;
the compound of formula (IIa) wherein R²² is 2-naphthylmethyl and R²³ is 4-fluorobenzyl;
the compound of formula (IIa) wherein R²² is 3-carbomethoxybenzyl and R²³ is 3-carbomethyoxybenzyl;
the compound of formula (IIa) wherein R²² is 4-formylbenzyl and R²³ is 4-formylbenzyl;
the compound of formula (IIa) wherein R²² is 4-cyanobenzyl and R²³ is 4-cyanobenzyl;
the compound of formula (IIa) wherein R²² is 4-hydroxybenzyl and R²³ is n-propyl;
the compound of formula (IIa) wherein R²² is 3-hydroxybenzyl and R²³ is n-propyl;
the compound of formula (IIa) wherein R²² is 3-carboxybenzyl and R²³ is 3-carboxybenzyl;
the compound of formula (IIa) wherein R²² is cyclobutylmethyl and R²³ is cyclobutylmethyl;
the compound of formula (IIa) wherein R²² is cyclopentylmethyl and R²³ is cyclopentylmethyl;
the compound of formula (IIa) wherein R²² is n-butyl and R²³ is 3-methallyl;
the compound of formula (IIa) wherein R²² is n-butyl and R²³ is cyclopentylmethyl;
the compound of formula (IIa) wherein R²² is 3-formaldoximebenzyl and R²³ is 3-formaldoximebenzyl;
the compound of formula (IIa) wherein R²² is cyclopropylmethyl and R²³ is 3-hydroxybenzyl;
the compound of formula (IIa) wherein R²² is cyclopropylmethyl and R²³ is 4-hydroxybenzyl;
the compound of formula (IIa) wherein R²² is 3-(N-methylamino)benzyl and R²³ is 3-(N-methylamino)benzyl;
the compound of formula (IIa) wherein R²² is 3-acetylbenzyl and R²³ is 3-acetylbenzyl;
the compound of formula (IIa) wherein R²² is 3-hydroxylaminobenzyl and R²³ is 3-hydroxylaminobenzyl;
the compound of formula (IIa) wherein R²² is 2-naphthylmethyl and R²³ is 3-hydroxybenzyl;
the compound of formula (IIa) wherein R²² is 4-hydroxymethylbenzyl and R²³ is 3-hydroxybenzyl;
the compound of formula (IIa) wherein R²² is N-methyl-(3-amido)benzyl and R²³ is N-methyl-(3-amido)benzyl;
the compound of formula (IIa) wherein R²² is N-methyl-(3-amido)benzyl and R²³ is 3-(amidino)benzyl;
the compound of formula (IIa) wherein R²² is 3-(5-tetrazolyl)benzyl and R²³ is cyclopropylmethyl;
the compound of formula (IIa) wherein R²² is 3-(5-tetrazolyl)benzyl and R²³ is 3-(5-tetrazolyl)benzyl;
the compound of formula (IIa) wherein R²² is phenylmethyl-3-boronic acid and R²³ is phenylmethyl-3-boronic acid.

10. The compound of Claim 1 of formula (IIa): 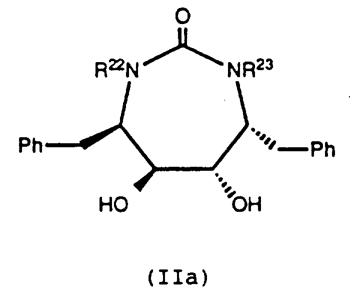 which is selected from the group consisting of:
the compound of formula (IIa) wherein R²² is allyl and R²³ is allyl;
the compound of formula (IIa) wherein R²² is cyclopropylmethyl and R²³ is cyclopropylmethyl;
the compound of formula (IIa) wherein R²² is n-butyl and R²³ is n-butyl;
the compound of formula (IIa) wherein R²² is propyl and R²³ is propyl;
the compound of formula (IIa) wherein R²² is i-pentyl and R²³ is i-pentyl;
the compound of formula (IIa) wherein R²² is benzyl and R²³ is benzyl;
the compound of formula (IIa) wherein R²² is 3-hydroxybenzyl and R²³ is 3-hydroxybenzyl;
the compound of formula (IIa) wherein R²² is 4-hydroxybenzyl and R²³ is 4-hydroxybenzyl;
the compound of formula (IIa) wherein R²² is 3-hydroxymethylbenzyl and R²³ is 3-hydroxymethylbenzyl;
the compound of formula (IIa) wherein R²² is 4-hydroxymethylbenzyl and R²³ is 4-hydroxymethylbenzyl;
the compound of formula (IIa) wherein R²² is 3-aminobenzyl and R²³ is 3-aminobenzyl;
the compound of formula (IIa) wherein R²² is 3-carboxylbenzyl and R²³ is 3-carboxylbenzyl;
the compound of formula (IIa) wherein R²² is 3-formylbenzyl and R²³ is 3-formylbenzyl
the compound of formula (IIa) wherein R²² is 3-formaldoximebenzyl and R²³ is 3-formaldoximebenzyl;
the compound of formula (IIa) wherein R²² is 3-(N-methylamino)benzyl and R²³ is 3-(N-methylamino)benzyl;
the compound of formula (IIa) wherein R²² is 3-acetylbenzyl and R²³ is 3-acetylbenzyl;
the compound of formula (IIa) wherein R²² is 3-hydroxylaminobenzyl and R²³ is 3-hydroxylaminobenzyl;
the compound of formula (IIa) wherein R²² is 2-naphthylmethyl and R²³ is 3-hydroxybenzyl;
the compound of formula (IIa) wherein R²² is 4-hydroxymethylbenzyl and R²³ is 3-hydroxybenzyl;
the compound of formula (IIa) wherein R²² is N-methyl-(3-amido)benzyl and R²³ is N-methyl-(3-amido)benzyl;
the compound of formula (IIa) wherein R²² is N-methyl-(3-amido)benzyl and R²³ is 3-(amidino)benzyl;
the compound of formula (IIa) wherein R²² is 3-(5-tetrazolyl)benzyl and R²³ is cyclopropylmethyl;
the compound of formula (IIa) wherein R²² is 3-(5-tetrazolyl)benzyl and R²³ is 3-(5-tetrazolyl)benzyl;
the compound of formula (IIa) wherein R²² is phenylmethyl-3-boronic acid and R²³ is phenylmethyl-3-boronic acid.

11. A compound of Claim 1 of formula (IIb): 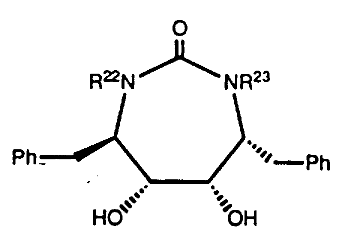 or a pharmaceutically acceptable salt or prodrug form thereof wherein:
R²² and R²³ are independently selected from the group consisting of: hydrogen, cyclopropylmethyl, CH₂(C₆H₄)-p-OCH₂C₆H₅, CH₂(C₆H₄)-p-OH, cyclopentylmethyl, allyl, n-butyl, beta-napthylmethyl, benzyl, CH₂(C₆H₄)-m-OCH₂C₆H₅, p-nitrobenzyl, m-nitrobenzyl, CH₂(C₆H₄)-m-OH, CH₂(C₆H₄)-m-(CH₂OH), p-aminobenzyl, m-aminobenzyl p-nitrilobenzyl, m-nitrilobenzyl, dimethylallyl, cyclohexylmethyl, cyclobutylmethyl, propyl, 3-methyl-1-butyl, carboxamidobenzyl, and formaldoximebenzyl.

12. A compound of Claim 1 of formula (Ib): 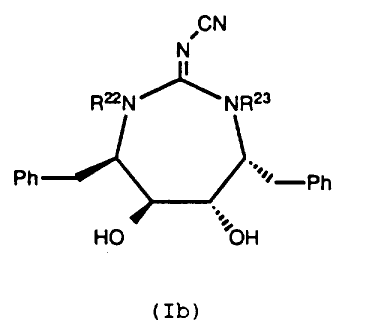 or a pharmaceutically acceptable salt or prodrug form thereof wherein:
R²² and R²³ are independently selected from the group consisting of: hydrogen, cyclopropylmethyl, CH₂(C₆H₄)-p-OCH₂C₆H₅, CH₂(C₆H₄)-p-OH, cyclopentylmethyl, allyl, n-butyl, beta-napthylmethyl, benzyl, CH₂(C₆H₄)-m-OCH₂C₆H₅, p-nitrobenzyl, m-nitrobenzyl, CH₂(C₆H₄)-m-OH, p-aminobenzyl, m-aminobenzyl, p-nitrilobenzyl, m-nitrilobenzyl, dimethylallyl, cyclohexylmethyl, cyclobutylmethyl, propyl, 3-methyl-1-butyl, carboxamidobenzyl, and formaldoximebenzyl.

13. Use of a compound according to anyone of claims 1-12 for the preparation of a medicament for the treatment of viral infections.

14. A pharmaceutical composition comprising a pharmaceutical acceptable carrier and at least one compound according to anyone of claims 1-12.

## Patentansprüche

1. Verbindung der Formel (I): 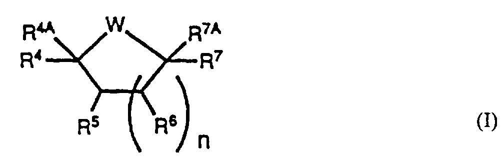 oder ein pharmazeutisch annehmbares Salz oder eine Prodrug-Form derselben, worin:
R⁴ und R⁷ unabhängig aus den folgenden Gruppen ausgewählt sind:
Wasserstoff,
mit 0-3 R¹¹ substituiertes C₁-C₈-Alkyl,
mit 0-3 R¹¹ substituiertes C₂-C₈-Alkenyl,
mit 0-3 R¹¹ substituiertes C₂-C₈-Alkinyl,
mit 0-3 R¹¹ substituiertes C₃-C₈-Cycloalkyl,
mit 0-3 R¹¹ substituiertes C₆-C₁₀-Bicycloalkyl,
mit 0-3 R¹² substituiertes Aryl,
ein mit 0-3 R¹² substituierter carbocyclischer C₆-C₁₄-Rest,
ein mit 0-2 R¹² substituiertes heterocyclisches Ringsystem, das sich aus 5 bis 10 Atomen einschließlich wenigstens eines Stickstoff-, Sauerstoff- oder Schwefelatoms zusammensetzt,
R^{4A} und R^{7A} unabhängig aus den folgenden Gruppen ausgewählt sind:
Wasserstoff,
mit Halogen oder C₁-C₂-Alkoxy substituiertes C₁-C₄-Alkyl,
mit Halogen oder C₁-C₂-Alkoxy substituiertes Benzyl,
R⁴ und R^{4A} sich wahlweise unter Bilden eines 5-7-gliedrigen carbocyclischen, mit 0-2 R¹² substituierten Ringes verbinden können,
R⁷ und R^{7A} sich wahlweise unter Bilden eines 5-7-gliedrigen carbocyclischen, mit 0-2 R¹² substituierten Ringes verbinden können,
n 1 ist,
R⁵ aus Fluor, Difluor, =O, C₁-C₃-Alkyl oder -OR²⁰ ausgewählt ist,
R⁶ aus Wasserstoff, =O, Fluor, Difluor, C₁-C₃-Alkyl oder -OR²¹ ausgewählt ist,
R⁵ und R⁶ sich wahlweise unter Bilden eines Epoxidringes, -OCH₂SCH₂O-, -OS(=O)O-, -OC(=O)O-, -OCH₂O-, -OC(=S)O-, -OC(=O)C(=O)O-, -OC(CH₃)₂O-, -OC(OCH₃)(CH₂CH₂CH₃)O- oder einer hydrolysierbaren Gruppe verbinden können, welche beim Verabreichen an einen Säuger unter Bilden des ursprünglichen Diols, bei dem R⁵ und R⁶ -OH sind, metabolisch gespalten wird,
R²⁰ und R²¹ unabhängig aus
Wasserstoff,
mit 0-3 R¹¹ substituiertes C₁-C₆-Alkyl,
mit 0-3 R¹¹ substituiertes C₃-C₆-Alkoxyalkyl,
mit 0-3 R¹¹ substituiertes C₁-C₆-Alkylcarbonyl,
mit 0-3 R¹¹ substituiertes C₁-C₆-Alkoxycarbonyl,
mit 0-3 R¹² substituiertes Benzoyl,
mit 0-3 R¹² substituiertes Phenoxycarbonyl,
mit 0-3 R¹² substituiertes Phenylaminocarbonyl oder
einer hydrolysierbaren Gruppe ausgewählt ist, die beim Verabreichen an einen Säuger unter Bilden des ursprünglichen Diols, bei dem R⁵ und R⁶ -OH sind, metabolisch gespalten wird,
R¹¹ aus einem oder mehreren der Folgenden ausgewählt ist:
Keto, Halogen, Cyan, -CH₂NR¹³R¹⁴, -NR¹³R¹⁴, -CO₂R¹³, -OC(=O)R¹³, -OR¹³, C₂-C₆-Alkoxyalkyl, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, C₁-C₄-Alkyl, C₂-C₄-Alkenyl, C₃-C₆-Cycloalkyl, C₃-C₆-Cycloalkylmethyl,
1-3 Aminosäuren, die miteinander über Amidbindungen verbunden sind und an R⁴ oder R⁷ über den Amin- oder Carboxylatteminus gebunden sind,
ein mit 0-3 R¹² substituierter carbocylischer C₅-C₁₄-Rest,
mit 0-3 R¹² substituiertes Aryl oder
ein mit 0-2 R¹² substituiertes heterocyclisches Ringsystem, das sich aus 5 bis 10 Atomen einschließlich wenigstens eines Stickstoff-, Sauerstoff- oder Schwefelatoms zusammensetzt,
R¹², wenn es ein Substituent an Kohlenstoff ist, aus einem oder mehreren der Folgenden ausgewählt ist: Phenyl, Benzyl, Phenethyl, Phenoxy, Benzyloxy, Halogen, Hydroxy, Nitro, Cyano, C₁-C₄-Alkyl, C₃-C₆-Cycloalkyl, C₃-C₆-Cycloalkylmethyl, C₇-C₁₀-Arylalkyl, C₁-C₄-Alkoxy, -CO₂H, Hydroxamsäure, Hydrazid, Oxim, Organyldihydroxyboran (Boronsäure), Sulfonamid, Formyl, C₃-C₆-Cycloalkoxy, -OR¹³, mit -NR¹³R¹⁴ substituiertesC₁-C₄-Alkyl, -NR¹³R¹⁴, C₂-C₆-Alkoxyalkyl, C₁-C₄-Hydroxyalkyl, Methylendioxy,Ethylendioxy, C₁-C₄-Halogenalkyl, C₁-C₄-Halogenalkoxy, C₁-C₄-Alkoxycarbonyl, C₁-C₄-Alkylcarbonyloxy, C₁-C₄-Alkylcarbonyl, C₁-C₄-Alkylcarbonylamino, -S(O)ₘR¹³, -SO₂NR¹³R¹⁴, -NHSO₂R¹⁴, -OCH₂CO₂H, 2-(1-Morpholino)ethoxy oder ein 5- oder 6-gliedriger heterocyclischer Ring, der 1 bis 4 aus Sauerstoff, Stickstoff oder Schwefel ausgewählte Heteroatome enthält,
oder R¹² eine 3- oder 4-Kohlenstoffkette sein kann, die an benachbarte Kohlenstoffe des Ringes unter Bilden eines kondensierten 5- oder 6-gliedrigen Ringes gebunden ist, wobei der 5- oder 6-gliedrige Ring gegebenenfalls an den aliphatischen Kohlenstoffen mit Halogen, C₁-C₄-Alkyl, C₁-C₄-Alkoxy, Hydroxy oder -NR¹³R¹⁴ substituiert ist oder, wenn R¹² an ein gesättigtes Kohlenstoffatom gebunden ist, Carbonyl oder Thiocarbonyl sein kann,
R¹², wenn es ein Substituent an Stickstoff ist, aus einem oder mehreren der Folgenden ausgewählt ist: Phenyl, Benzyl, Phenethyl, Hydroxy, C₁-C₄-Hydroxyalkyl, C₁-C₄-Alkoxy, C₁-C₄-Alkyl, C₃-C₆-Cycloalkyl, C₃-C₆-Cycloalkylmethyl, -CH₂NR¹³R¹⁴, -NR¹³R¹⁴, C₂-C₆-Alkoxyalkyl, C₁-C₄-Halogenalkyl, C₁-C₄-Alkoxycarbonyl, -CO₂H, C₁-C₄-Alkylcarbonyloxy, C₁-C₄-Alkylcarbonyl,
R¹³ H, Phenyl, Benzyl, C₁-C₆-Alkyl oder C₃-C₆-Alkoxyalkyl ist,
R¹⁴ OH, H, C₁-C₄-Alkyl oder Benzyl ist,
R¹³ und R¹⁴ sich wahlweise unter Bilden von -(CH₂)₄-, -(CH₂)₅-, -CH₂CH₂N(R¹⁵)CH₂CH₂- oder -CH₂CH₂OCH₂CH₂- verbinden können,
R¹⁵ H oder CH₃ ist,
m 0, 1 oder 2 ist,
W aus -N(R²²)C(=Z)N(R²³)-, -N(R²²)S(=O)N(R²³)- ausgewählt ist, worin
Z O, S oder NR²⁴ ist,
R²² und R²³ unabhängig aus den Folgenden ausgewählt sind:
Wasserstoff,
mit 0-3 R³¹ substituiertes C₁-C₈-Alkyl,
mit 0-3 R³¹ substituiertes C₂-C₈-Alkenyl,
mit 0-3 R³¹ substituiertes C₂-C₈-Alkinyl,
mit 0-3 R³¹ substituiertes C₃-C₈-Cycloalkyl,
mit 0-3 R³¹ substituiertes C₆-C₁₀-Bicycloalkyl,
mit 0-3 R³² substituiertes Aryl,
ein mit 0-3 R³² substituierter carbocyclischer C₆-C₁₄-Rest,
ein mit 0-2 R³² substituiertes heterocyclisches Ringsystem, das sich aus 5 bis 10 Atomen einschließlich wenigstens eines Stickstoff-, Sauerstoff- oder Schwefelatoms zusammensetzt,
R²⁴ aus Hydroxy, Amino, C₁-C₄-Alkyl, C₁-C₄-Alkoxy, C₁-C₄-Aminoalkyl, Cyano, Nitro, Benzyloxy ausgewählt ist,
R²² sich wahlweise mit R⁴ oder R^{4A} unter Bilden eines fünf- oder sechsgliedrigen, kondensierten, heterocyclischen oder carbocylischen, mit 0-2 R¹² substituierten Ringes verbinden kann und
R²³ sich wahlweise mit R⁷ oder R^{7A} unter Bilden eines fünf- oder sechsgliedrigen, kondensierten, heterocyclischen oder carbocylischen, mit 0-2 R¹² substituierten Ringes verbinden kann und
W sich wahlweise mit R⁵ oder R⁶ unter Bilden eines drei- bis siebengliedrigen, kondensierten, heterocyclischen oder carbocylischen, mit 0-2 R¹² substituierten Ringes verbinden kann,
R³¹ aus einem oder mehreren der Folgenden ausgewählt ist:
Keto, Halogen, Cyan, -CH₂NR¹³R¹⁴, -NR¹³R¹⁴, -CO₂R¹³, -OC(=O)R¹³, -OR¹³, C₂-C₆-Alkoxyalkyl, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³-R¹⁴, -NR¹³C(=O)NR¹³R¹⁴, -NR¹⁴SO₂NR¹³R¹⁴, -NR¹⁴SO₂R¹³, -SO₂R¹³R¹⁴, C₁-C₄-Alkyl, C₂-C₄-Alkenyl, C₃-C₆-Cycloalkyl, C₃-C₆-Cycloalkylmethyl,
1-3 Aminosäuren, die miteinander über Amidbindungen und mit R⁴ oder R⁷ über den Amin- oder Carboxylatterminus verbunden sind,
ein mit 0-3 R³² substituierter carbocyclischer C₅-C₁₄-Rest,
mit 0-3 R³² substituiertes Aryl oder
ein mit 0-2 R³² substituiertes heterocyclisches Ringsystem, das sich aus 5 bis 10 Atomen einschließlich wenigstens eines Stickstoff-, Sauerstoff- oder Schwefelatoms zusammensetzt,
R³², wenn es ein Substituent an Kohlenstoff ist, aus einem oder mehreren der Folgenden ausgewählt ist:
Phenyl, Benzyl, Phenethyl, Phenoxy, Benzyloxy, Halogen, Hydroxy, Nitro, Cyano, C₁-C₄-Alkyl, C₃-C₆-Cycloalkyl, C₃-C₆-Cycloalkylmethyl, C₇-C₁₀-Arylalkyl, C₁-C₄-Alkoxy, -CO₂H, Hydroxamsäure, Hydrazid, Oxim, Organyldihydroxyboran (Boronsäure), Sulfonamid, Formyl, C₃-C₆-Cycloalkoxy, -OR¹³, mit -NR¹³R¹⁴ substituiertesC₁-C₄-Alkyl, -NR¹³R¹⁴, C₂-C₆-Alkoxyalkyl, C₁-C₄-Hydroxyalkyl, Methylendioxy, Ethylendioxy, C₁-C₄-Halogenalkyl, C₁-C₄-Halogenalkoxy, C₁-C₄-Alkoxycarbonyl, C₁-C₄-Alkylcarbonyloxy, C₁-C₄-Alkylcarbonyl, C₁-C₄-Alkylcarbonylamino, -S(O)ₘR¹³, -SO₂NR¹³R¹⁴, -NHSO₂R¹⁴, -OCH₂CO₂H, 2-(1-Morpholino)ethoxy, -C(R¹⁴)=N-(OR¹⁴) oder
ein 5- oder 6-gliedriger heterocyclischerRing, der 1 bis 4 aus Sauerstoff, Stickstoff oder Schwefel ausgewählte Heteroatome enthält,
oder R³² eine 3- oder 4-Kohlenstoffkette sein kann, die an benachbarte Kohlenstoffe des Ringes unter Bilden eines kondensierten 5- oder 6-gliedrigen Ringes gebunden ist und gegebenenfalls an den aliphatischen Kohlenstoffen mit Halogen, C₁-C₄-Alkyl, C₁-C₄-Alkoxy, Hydroxy oder -NR¹³R¹⁴ substituiert ist oder, wenn R³² an ein gesättigtes Kohlenstoffatom gebunden ist, Carbonyl oder Thiocarbonyl sein kann,
R³², wenn es ein Substituent an Stickstoff ist, aus einem oder mehreren der Folgenden ausgewählt ist: Phenyl, Benzyl, Phenethyl, Hydroxy, C₁-C₄-Hydroxyalkyl, C₁-C₄-Alkoxy, C₁-C₄-Alkyl, C₃-C₆-Cycloalkyl, C₃-C₆-Cycloalkylmethyl, -CH₂NR¹³R¹⁴, -NR¹³R¹⁴, C₂-C₆-Alkoxyalkyl, C₁-C₄-Halogenalkyl, C₁-C₄-Alkoxycarbonyl, -CO₂H, C₁-C₄-Alkylcarbonyloxy, C₁-C₄-Alkylcarbonyl, -C(R¹⁴)=N(OR¹⁴),
vorausgesetzt, daß R⁴, R^{4A}, R⁷ und R^{7A} nicht alle Wasserstoff sind,
wenn R⁴ und R^{4A} beide Wasserstoff sind, wenigstens eines der Folgenden nicht Wasserstoff ist: R²², R²³.

2. Verbindung von Anspruch 1 der Formel (I): 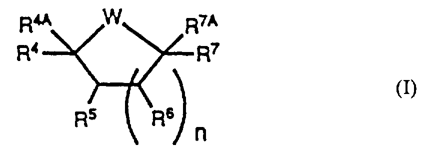 oder ein pharmazeutisch annehmbares Salz oder eine Prodrug-Form derselben, worin:
R⁴ und R⁷ unabhängig aus den folgenden Gruppen ausgewählt sind:
Wasserstoff,
mit 0-3 R¹¹ substituiertes C₁-C₄-Alkyl,
mit 0-3 R¹¹ substituiertes C₃-C₄-Alkenyl,
mit 0-3 R¹¹ substituiertes C₃-C₄-Alkinyl,
R^{4A} und R^{7A} Wasserstoff sind,
n 1 ist,
R⁵ aus Fluor, Difluor, =O oder -OR²⁰ ausgewählt ist,
R⁶ aus Wasserstoff, =O, Fluor, Difluor oder -OR²¹ ausgewählt ist;
R⁵ und R⁶ sich wahlweise unter Bilden eines Epoxidringes, -OCH₂SCH₂O-, -OS(=O)O-, -OC(=O)O-, -OCH₂O-, -OC(=S)O-, -OC(=O)C(=O)O-, -OC(CH₃)₂O-, -OC(OCH₃)(CH₂CH₂CH₃)O- oder einer hydrolysierbaren Gruppe verbinden können, welche beim Verabreichen an einen Säuger unter Bilden des ursprünglichen Diols, bei dem R⁵ und R⁶ -OH sind, metabolisch gespalten wird,
R²⁰ und R²¹ unabhängig aus
Wasserstoff,
C₁-C₆-Alkylcarbonyl,
C₁-C₆-Alkoxycarbonyl,
Benzoyl oder einer hydrolysierbaren Gruppe ausgewählt sind, die beim Verabreichen an einen Säuger unter Bilden des ursprünglichen Diols, bei dem R⁵ und R⁶ -OH sind, metabolisch gespalten wird,
R¹¹ aus einer oder mehreren der Folgenden ausgewählt ist:
Keto, Halogen, Cyan, -CH₂NR¹³R¹⁴, -NR¹³R¹⁴, -CO₂R¹³, -OC(=O)R¹³, -OR¹³, C₂-C₄-Alkoxyalkyl, -S(O)ₘR¹³, C₁-C₄-Alkyl, C₂-C₄-Alkenyl, C₃-C₆-Cycloalkyl, ein mit 0-3 R¹² substituierter carbocylischer C₅-C₁₄-Rest,
mit 0-3 R¹² substituiertes Aryl oder
ein mit 0-2 R¹² substituiertes heterocyclisches Ringsystem, das sich aus 5 bis 10 Atomen einschließlich wenigstens eines Stickstoff-, Sauerstoff- oder Schwefelatoms zusammensetzt,
R¹², wenn es ein Substituent an Kohlenstoff ist, aus einem oder mehreren der Folgenden ausgewählt ist: Phenyl, Benzyl, Phenethyl, Phenoxy, Benzyloxy, Halogen, Hydroxy, Nitro, Cyano, C₁-C₄-Alkyl, C₃-C₆-Cycloalkyl, C₃-C₆-Cycloalkylmethyl, C₇-C₁₀-Arylalkyl, C₁-C₄-Alkoxy, -CO₂H, Hydroxamsäure, Hydrazid, Oxim, Organyldihydroxyboran (Boronsäure), Sulfonamid, Formyl, C₃-C₆-Cycloalkoxy, -OR¹³, mit -NR¹³R¹⁴ substituiertesC₁-C₄-Alkyl, -NR¹³R¹⁴, C₂-C₆-Alkoxyalkyl, C₁-C₄-Hydroxyalkyl, Methylendioxy, Ethylendioxy, C₁-C₄-Halogenalkyl, C₁-C₄-Halogenalkoxy, C₁-C₄-Alkoxycarbonyl, C₁-C₄-Alkylcarbonyloxy, C₁-C₄-Alkylcarbonyl,C₁-C₄-Alkylcarbonylamino, -S(O)ₘR¹³, -SO₂NR¹³R¹⁴, -NHSO₂R¹⁴ oder ein 5- oder 6-gliedriger heterocyclischer Ring, der 1 bis 4 aus Sauerstoff, Stickstoff oder Schwefel ausgewählte Heteroatome enthält,
oder R¹² eine 3- oder 4-Kohlenstoffkette sein kann, die an benachbarte Kohlenstoffe des Ringes unter Bilden eines kondensierten 5- oder 6-gliedrigen Ringes gebunden ist, wobei der 5- oder 6-gliedrige Ring gegebenenfalls an den aliphatischen Kohlenstoffen mit Halogen, C₁-C₄-Alkyl, C₁-C₄-Alkoxy, Hydroxy oder -NR¹³R¹⁴ substituiert ist oder, wenn R¹² an ein gesättigtes Kohlenstoffatom gebunden ist, Carbonyl oder Thiocarbonyl sein kann,
R¹², wenn es ein Substituent an Stickstoff ist, aus einem oder mehreren der Folgenden ausgewählt ist: Phenyl, Benzyl, Phenethyl, Hydroxy, C₁-C₄-Hydroxyalkyl, C₁-C₄-Alkoxy, C₁-C₄-Alkyl, C₃-C₆-Cycloalkyl, C₃-C₆-Cycloalkylmethyl, -CH₂NR¹³R¹⁴, -NR¹³R¹⁴, C₂-C₆-Alkoxyalkyl, C₁-C₄-Halogenalkyl, C₁-C₄-Alkoxycarbonyl, C₁-C₄-Alkylcarbonyloxy, C₁-C₄-Alkylcarbonyl, -CO₂H,
R¹³ H, C₁-C₆-Alkyl oder C₃-C₆-Alkoxyalkyl ist,
R¹⁴ OH, H, C₁-C₄-Alkyl oder Benzyl ist,
R¹³ und R¹⁴ sich wahlweise unter Bilden von -(CH₂)₄-, -(CH₂)₅-, -CH₂CH₂N(R¹⁵)CH₂CH₂- oder -CH₂CH₂OCH₂CH₂- verbinden können,
R¹⁵ H oder CH₃ ist,
m 0, 1 oder 2 ist,
W -N(R²²)C(=Z)N(R²³)- ist, worin
Z O, S, N-CN, N-OH, N-OCH₃ ist,
R²² und R²³ unabhängig aus den Folgenden ausgegwählt sind:
Wasserstoff,
mit 0-3 R³¹ substituiertes C₁-C₈-Alkyl,
mit 0-3 R³¹ substituiertes C₃-C₈-Alkenyl,
mit 0-3 R³¹ substituiertes C₃-C₈-Alkinyl,
mit 0-3 R³¹ substituiertes C₃-C₆-Cycloalkyl,
R³¹ aus einem oder mehreren der Folgenden ausgewählt ist:
Keto, Halogen, Cyan, -CH₂NR¹³R¹⁴, -NR¹³R¹⁴, -CO₂R¹³, -OC(=O)R¹³, -OR¹³, C₂-C₄-Alkoxyalkyl, -S(O)ₘR¹³, C₁-C₄-Alkyl, C₂-C₄-Alkenyl, C₃-C₆-Cycloalkyl,
ein mit 0-3 R¹² substituierter carbocyclischer C₅-C₁₄-Rest,
mit 0-3 R³² substituiertes Aryl oder
ein mit 0-2 R³² substituiertes heterocyclisches Ringsystem, das sich aus 5 bis 10 Atomen einschließlich wenigstens eines Stickstoff-, Sauerstoff- oder Schwefelatoms zusammensetzt,
R³², wenn es ein Substituent an Kohlenstoff ist, aus einem oder mehreren der Folgenden ausgewählt ist:
Phenyl, Benzyl, Phenethyl, Phenoxy, Benzyloxy, Halogen, Hydroxy, Nitro, Cyano, C₁-C₄-Alkyl, C₃-C₆-Cycloalkyl, C₃-C₆-Cycloalkylmethyl, C₇-C₁₀-Arylalkyl, C₁-C₄-Alkoxy, -CO₂H, Hydroxamsäure, Hydrazid, Oxim, Organyldihydroxyboran (Boronsäure), Sulfonamid, Formyl, C₃-C₆-Cycloalkoxy, -OR¹³, mit -NR¹³R¹⁴ substituiertesC₁-C₄-Alkyl, -NR¹³R¹⁴, C₂-C₆-Alkoxyalkyl, C₁-C₄-Hydroxyalkyl, Methylendioxy, Ethylendioxy, C₁-C₄-Halogenalkyl, C₁-C₄-Halogenalkoxy, C₁-C₄-Alkoxycarbonyl, C₁-C₄-Alkylcarbonyloxy, C₁-C₄-Alkylcarbonyl,C₁-C₄-Alkylcarbonylamino, -S(O)ₘR¹³, -SO₂NR¹³R¹⁴, -NHSO₂R¹⁴, -C(R¹⁴)=N(OR¹⁴) oder ein 5- oder 6-gliedriger heterocyclischer Ring, der 1 bis 4 aus Sauerstoff, Stickstoff oder Schwefel ausgewählte Heteroatome enthält,
oder R³² eine 3- oder 4-Kohlenstoffkette sein kann, die an benachbarte Kohlenstoffe des Ringes unter Bilden eines kondensierten 5- oder 6-gliedrigen Ringes gebunden ist, wobei der 5- oder 6-gliedrige Ring gegebenenfalls an den aliphatischen Kohlenstoffen mit Halogen, C₁-C₄-Alkyl, C₁-C₄-Alkoxy, Hydroxy oder -NR¹³R¹⁴ substituiert ist oder, wenn R³² an ein gesättigtes Kohlenstoffatom gebunden ist, Carbonyl oder Thiocarbonyl sein kann,
R³², wenn es ein Substituent an Stickstoff ist, aus einem oder mehreren der Folgenden ausgewählt ist: Phenyl, Benzyl, Phenethyl, Hydroxy, C₁-C₄-Hydroxyalkyl, C₁-C₄-Alkoxy, C₁-C₄-Alkyl, C₃-C₆-Cycloalkyl, C₃-C₆-Cycloalkylmethyl, -CH₂NR¹³R¹⁴, -NR¹³R¹⁴, C₂-C₆-Alkoxyalkyl, C₁-C₄-Halogenalkyl, C₁-C₄-Alkoxycarbonyl, C₁-C₄-Alkylcarbonyloxy, C₁-C₄-Alkylcarbonyl, -CO₂H, -C(R¹⁴)=N(OR¹⁴),
vorausgesetzt, daß R⁴, R^{4A}, R⁷ und R^{7A} nicht alle Wasserstoff sind,
wenn R⁴ und R^{4A} beide Wasserstoff sind, wenigstens eines der Folgenden nicht Wasserstoff ist: R²², R²³.

3. Verbindung von Anspruch 1 der Formel (II): 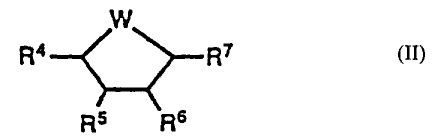 oder ein pharmazeutisch annehmbares Salz oder eine Prodrug-Form derselben, worin:
R⁴ und R⁷ unabhängig aus den folgenden Gruppen ausgewählt sind:
Wasserstoff,
mit 0-3 R¹¹ substituiertes C₁-C₄-Alkyl,
mit 0-3 R¹¹ substituiertes C₃-C₄-Alkenyl,
R⁵ -OR²⁰ ist,
R⁶ Wasserstoff oder -OR²¹ ist,
R²⁰ und R²¹ unabhängig Wasserstoff oder eine hydrolysierbare Gruppe sind, die beim Verabreichen an einen Säuger unter Bilden des ursprünglichen Diols, bei dem R⁵ und R⁶ -OH sind, metabolisch gespalten wird,
R¹¹ aus einem oder mehreren der Folgenden ausgewählt ist:
Keto, Halogen, -CH₂NR¹³R¹⁴, -NR¹³R¹⁴, -OR¹³, C₂-C₄-Alkoxyalkyl, C₁-C₄-Alkyl, C₂-C₄-Alkenyl, C₃-C₆-Cycloalkyl, mit 0-3 R¹² substituiertes Aryl oder ein mit 0-2 R¹² substituiertes heterocyclisches Ringsystem, das sich aus 5 bis 10 Atomen einschließlich wenigstens eines Stickstoff-, Sauerstoff- oder Schwefelatoms zusammensetzt,
R¹², wenn es ein Substituent an Kohlenstoff ist, aus einem oder mehreren der Folgenden ausgewählt ist:
Phenyl, Benzyl, Phenethyl, Phenoxy, Benzyloxy, Halogen, C₁-C₄-Alkyl, C₇-C₁₀-Arylalkyl, C₁-C₄-Alkoxy,-CO₂H, Hydroxamsäure, Hydrazid, Oxim, Organyldihydroxylboran (Boronsäure), Sulfonamid, Formyl, C₃-C₆-Cycloalkoxy, -OR¹³, mit -NR¹³R¹⁴ substituiertes C₁-C₄-Alkyl, -NR¹³R¹⁴, Methylendioxy, C₁-C₄-Halogenalkyl, C₁-C₄-Alkylcarbonyl, C₁-C₄-Alkylcarbonylamino, Hydroxy, Hydroxymethyl oder ein 5- oder 6-gliedriger heterocyclischer Ring, der 1 bis 4 aus Sauerstoff, Stickstoff oder Schwefel ausgewählte Heteroatome enthält,
R¹², wenn es ein Substituent an Stickstoff ist, aus Benzyl oder Methyl ausgewählt ist,
R¹³ H, C₁-C₂-Alkyl oder C₃-C₆-Alkoxyalkyl ist,
R¹⁴ OH, H oder C₁-C₂-Alkyl ist,
R¹³ und R¹⁴ sich wahlweise unter Bilden von -(CH₂)₄-, -(CH₂)₅-, -CH₂CH₂N(R¹⁵)CH₂CH₂- oder -CH₂CH₂OCH₂CH₂- verbinden können,
W -N(R²²)C(=Z)N(R²³)- ist, worin
Z O, S oder N-CN ist,
R²² und R²³ unabhängig aus den Folgenden ausgewählt sind:
Wasserstoff,
mit 0-3 R³¹ substituiertes C₁-C₄-Alkyl,
mit 0-3 R³¹ substituiertes C₃-C₄-Alkenyl,
R³¹ aus einem oder mehreren der Folgenden ausgewählt ist:
Keto, Halogen, -CH₂NR¹³R¹⁴, -NR¹³R¹⁴, -OR¹³, C₂-C₄-Alkoxyalkyl, C₁-C₄-Alkyl, C₂-C₄-Alkenyl, C₃-C₆-Cycloalkyl,
mit 0-3 R³² substituiertes Aryl,
ein mit 0-2 R³² substituiertes heterocyclisches Ringsystem, das sich aus 5 bis 10 Atomen einschließlich wenigstens eines Stickstoff-, Sauerstoff- oder Schwefelatoms zusammensetzt,
R³², wenn es ein Substituent an Kohlenstoff ist, aus einem oder mehreren der Folgenden ausgewählt ist:
Phenyl, Benzyl, Phenethyl, Phenoxy, Benzyloxy, Halogen, C₁-C₄-Alkyl, C₇-C₁₀-Arylalkyl, C₁-C₄-Alkoxy, -CO₂H, Hydroxamsäure, Hydrazid, Oxim, Organyldihydroxylboron (Boronsäure), Sulfonamid, Formyl, C₃-C₆-Cycloalkoxy, -OR¹³, mit -NR¹³R¹⁴ substituiertes C₁-C₄-Alkyl, -NR¹³R¹⁴, Methylendioxy, C₁-C₄-Halogenalkyl, C₁-C₄-Alkylcarbonyl, C₁-C₄-Alkylcarbonylamino, Hydroxy, Hydroxymethyl, -C(R¹⁴)=N(OR¹⁴) oder ein 5- oder 6-gliedriger heterocyclischer Ring, der 1 bis 4 aus Sauerstoff, Stickstoff oder Schwefel ausgewählte Heteroatome enthält,
R³², wenn es ein Substituent an Stickstoff ist, aus Benzyl oder Methyl ausgewählt ist,
vorausgesetzt, daß wenn R⁴ Wasserstoff ist, R⁷ nicht Wasserstoff ist,
wenn R⁴ Wasserstoff ist, wenigstens eines der Folgenden nicht Wasserstoff ist: R²², R²³.

4. Verbindung von Anspruch 3, wobei
R⁴ und R⁷ unabhängig aus den folgenden Gruppen ausgewählt sind:
Wasserstoff,
mit 0-1 R¹¹ substituiertes C₁-C₃-Alkyl,
R⁵ -OR²⁰ ist,
R⁶ Wasserstoff oder -OR²¹ ist,
R²⁰ und R²¹ unabhängig Wasserstoff oder eine hydrolysierbare Gruppe sind, die beim Verabreichen an einen Säuger unter Bilden des ursprünglichen Diols, bei dem R⁵ und R⁶ -OH sind, metabolisch gespalten wird,
R¹¹ aus einem oder mehreren der Folgenden ausgewählt ist:
Halogen, -OR¹³, C₁-C₄-Alkyl, C₃-C₅-Cycloalkyl, mit 0-2 R¹² substituiertes Aryl oder ein heterocyclisches Ringsystem, das aus Pyridyl, Pyrimidinyl, Triazinyl, Furanyl, Thienyl, Pyrrolyl, Pyrazolyl, Imidazolyl, Tetrazolyl, Benzofuranyl, Indolyl, Chinolinyl, Isochinolinyl ausgewählt ist,
R¹², wenn es ein Substituent an Kohlenstoff ist, aus einem oder mehreren der Folgenden ausgewählt ist:
Benzyloxy, Halogen, Methyl, C₁-C₄-Alkoxy, CF₃, 2-(1-Morpholino)ethoxy, -CO₂H, Hydroxamsäure, Hydrazid, Oxim, Cyano, Organyldihydroxylboron (Boronsäure), Sulfonamid, Formyl, C₃-C₆-Cycloalkoxy, mit -NR¹³R¹⁴ substituiertes C₁-C₄-Alkyl, -NR¹³R¹⁴, Hydroxy, Hydroxymethyl oder
R¹², wenn es ein Substituent an Stickstoff ist, Methyl ist,
R¹³ H oder Methyl ist,
R¹³ OH, H oder Methyl ist,
R¹³ und R¹⁴ sich wahlweise unter Bilden von -(CH₂)₄, -(CH₂)₅-, -CH₂CH₂N(R¹⁵)CH₂CH₂- oder -CH₂CH₂OCH₂CH₂- verbinden können,
W -N(R²²)C(=O)N(R²³)- oder -N(R²²)C(=N-CN)N(R²³)- ist,
R²² und R²³ unabhängig aus den Folgenden ausgewählt sind:
Wasserstoff,
mit 0-1 R³¹ substituiertes C₁-C₄-Alkyl,
mit 0-1 R³¹ substituiertes C₃-C₄-Alkenyl,
R³¹ aus einem oder mehreren der Folgenden ausgewählt ist:
Halogen, -OR¹³, C₁-C₄-Alkyl, C₃-C₅-Cycloalkyl, mit 0-2 R³² substituiertes Aryl oder ein heterocyclisches Ringsystem, das aus Pyridyl, Pyrimidinyl, Triazinyl, Furanyl, Thienyl, Pyrrolyl, Pyrazolyl, Imidazolyl, Tetrazolyl, Benzofuranyl, Indolyl, Chinolinyl, Isochinolinyl ausgewählt ist,
R³², wenn es ein Substituent an Kohlenstoff ist, aus einem oder mehreren der Folgenden ausgewählt ist:
Benzyloxy, Halogen, Methyl, C₁-C₄-Alkoxy, CF₃, 2-(1-Morpholino)ethoxy, -CO₂H, Hydroxamsäure, Hydrazid, Oxim, Cyano, Organyldihydroxylboron (Boronsäure), Sulfonamid, Formyl, C₃-C₆-Cycloalkoxy, mit -NR¹³R¹⁴ substituiertes C₁-C₄-Alkyl, -NR¹³R¹⁴, Hydroxy, Hydroxymethyl, -C(R¹⁴)=N(OR¹⁴⁾ oder
R³², wenn es ein Substituent an Stickstoff ist, Methyl ist,
vorausgesetzt, daß:
wenn R⁴ Wasserstoff ist, R⁷ nicht Wasserstoff ist,
wenn R⁴ Wasserstoff ist, wenigstens eines der Folgenden nicht Wasserstoff ist: R²² und R²³.

5. Verbindung von Anspruch 3, wobei:
R⁴ und R⁷ Benzyl sind,
R⁵ -OH ist,
R⁶ Wasserstoff oder -OH ist,
R¹³ H oder Methyl ist,
R¹⁴ H oder Methyl ist,
W -N(R²²)C(=O)N(R²³)- oder -N(R²²)C(=N-CN)N(R²³)- ist,
R²² und R²³ unabhängig aus den Folgenden ausgewählt sind:
Wasserstoff,
mit 0-1 R³¹ substituiertes C₁-C₄-Alkyl,
R³¹ aus einem oder mehreren der Folgenden ausgewählt ist:
C₃-C₅-Cycloalkyl,
mit 0-2 R³² substituiertes Aryl oder
ein heterocyclisches Ringsystem, das aus Pyridyl, Thienyl, Chinolinyl oder Isochinolinyl ausgewählt ist,
R³², wenn es ein Substituent an Kohlenstoff ist, aus einem oder mehreren der Folgenden ausgewählt ist: -CONH₂, -CO₂H, -CHO, -CH₂NHOH, -CH₂NR¹³R¹⁴, -NR¹³R¹⁴, Hydroxy, Hydroxymethyl, -C(R¹⁴)=N(OR¹⁴) oder
R³², wenn es ein Substituent an Stickstoff ist, Methyl ist.

6. Verbindung von Anspruch 1 der Formel: 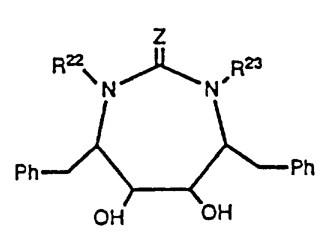
worin Z O, S oder N-CN ist,
worin R²² und R²³ unabhängig aus der Gruppe ausgewählt sind, die aus:
Wasserstoff, Allyl, Propyl, Cyclopropylmethyl, n-Butyl, Isobutyl, Pyridylmethyl, Methallyl, n-Pentyl, Isopentyl, Hexyl, Benzyl, Pyridylmethyl, Isoprenyl, Propargyl, Picolinyl, Methoxyethyl, Cyclohexylmethyl, Dimethylbutyl, Ethoxyethyl, Methyloxazolinylmethyl,Naphthylmethyl, Methyloxazolinylmethyl, Vinyloxyethyl, Pentafluorbenzyl, Chinolinylmethyl, Carboxybenzyl,Chlorthienyl, Picolinyl, Benzyloxybenzyl,Phenylbenzyl,Adamantylethyl, Cyclopropylmethoxybenzyl,Ethoxybenzyl,Hydroxybenzyl,Hydroxymethylbenzyl,Aminobenzyl,Formylbenzyl, Cyanbenzyl, Cinnamyl, Allyloxybenzyl, Fluorbenzyl, Cyclobutylmethyl, Formaldoximbenzyl, Cyclopentylmethyl, Nitrobenzyl, Nitrilobenzyl, Carboxamidobenzyl, Carbomethoxybenzyl, Tetrazolylbenzyl und Dimethylallyl besteht.

7. Verbindung von Anspruch 1 der Formel (IIa): 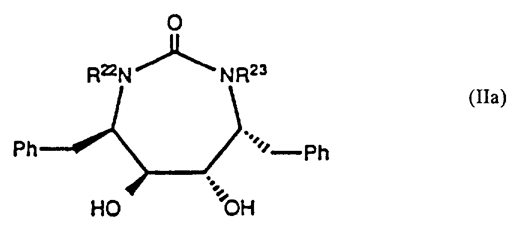 worin R²² und R²³ unabhängig aus der Gruppe ausgewählt sind, die aus:
Wasserstoff, Allyl, Propyl, Cyclopropylmethyl, n-Butyl, Isobutyl, Pyridylmethyl, Methallyl, n-Pentyl, Isopentyl, Hexyl, Benzyl, Pyridylmethyl, Isoprenyl, Propargyl, Picolinyl, Methoxyethyl, Cyclohexylmethyl, Dimethylbutyl, Ethoxyethyl, Methyloxazolinylmethyl,Naphthylmethyl,Methyloxazolinylmethyl,Vinyloxyethyl, Pentafluorbenzyl, Chinolinylmethyl, Carboxybenzyl,Chlorthienyl, Picolinyl, Benzyloxybenzyl,Phenylbenzyl,Adamantylethyl, Cyclopropylmethoxybenzyl,Ethoxybenzyl,Hydroxybenzyl,Hydroxymethylbenzyl,Aminobenzyl,Formylbenzyl, Cyanbenzyl, Cinnamyl, Allyloxybenzyl, Fluorbenzyl, Cyclobutylmethyl, Formaldoximbenzyl, Cyclopentylmethyl, Nitrobenzyl, Nitrilobenzyl, Carboxamidobenzyl, Carbomethoxybenzyl, Tetrazolylbenzyl, Dimethylallyl, Amidinobenzyl und Benzyldihydroxyboran besteht.

8. Verbindung von Anspruch 1 der Formel (IIa): 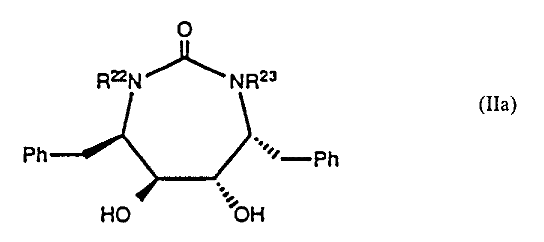 welche aus der Gruppe ausgewählt ist, die aus
der Verbindung der Formel (IIa), bei der R²² Allyl ist und R²³ Allyl ist,
der Verbindung der Formel (IIa), bei der R²² Propyl ist und R²³ Propyl ist,
der Verbindung der Formel (IIa), bei der R²² Cyclopropylmethyl ist und R²³ Cyclopropylmethyl ist,
der Verbindung der Formel (IIa), bei der R²² n-Butyl ist und R²³ n-Butyl ist,
der Verbindung der Formel (IIa), bei der R²² Isopentyl ist und R²³ Isopentyl ist,
der Verbindung der Formel (IIa), bei der R²² 4-Pyridylmethyl ist und R²³ 4-Pyridylmethyl ist,
der Verbindung der Formel (IIa), bei der R²² 2-Methallyl ist und R²³ 2-Methallyl ist,
der Verbindung der Formel (IIa), bei der R²² n-Pentyl ist und R²³ n-Pentyl ist,
der Verbindung der Formel (IIa), bei der R²² Isobutyl ist und R²³ Isobutyl ist,
der Verbindung der Formel (IIa), bei der R²² Benzyl ist und R²³ Benzyl ist,
der Verbindung der Formel (IIa), bei der R²² 3-Pyridylmethyl ist und R²³ 3-Pyridylmethyl ist,
der Verbindung der Formel (IIa), bei der R²² Allyl ist und R²³ Isoprenyl ist,
der Verbindung der Formel (IIa), bei der R²² 3-Propargyl ist und R²³ 3-Propargyl ist,
der Verbindung der Formel (IIa), bei der R²² 2-Picolinyl ist und R²³ 2-Picolinyl ist,
der Verbindung der Formel (IIa), bei der R²² 2-Methoxyethyl ist und R²³ 2-Methoxyethyl ist,
der Verbindung der Formel (IIa), bei der R²² Cyclohexylmethyl ist und R²³ Cyclohexylmethyl ist,
der Verbindung der Formel (IIa), bei der R²² 3,3-Dimethyl-1-butyl ist und R²³ 3,3-Dimethyl-1-butyl ist,
der Verbindung der Formel (IIa), bei der R²² 2-Ethoxyethyl ist und R²³ 2-Ethoxyethyl ist,
der Verbindung der Formel (IIa), bei der R²² 3-Methyl-5-oxazolinylmethyl ist und R²³ Wasserstoff ist,
der Verbindung der Formel (IIa), bei der R²² 1-Naphthylmethyl ist und R²³ 1-Naphthylmethyl ist,
der Verbindung der Formel (IIa), bei der R²² 3-Methyloxazolinylmethyl ist und R²³ 3-Methyloxazolinylmethyl ist,
der Verbindung der Formel (IIa), bei der R²² 2-Vinyloxyethyl ist und R²³ 2-Vinyloxyethyl ist,
der Verbindung der Formel (IIa), bei der R²² 2,3,4,5,6-Pentafluorbenzyl ist und R²³ 2,3,4,5,6-Pentafluorbenzyl ist,
der Verbindung der Formel (IIa), bei der R²² Benzyl ist und R²³ 2-Chinolinylmethyl ist,
der Verbindung der Formel (IIa), bei der R²² 4-Carboxybenzyl ist und R²³ 4-Carboxybenzyl ist,
der Verbindung der Formel (IIa), bei der R²² 5-Chlor-2-thienyl ist und R²³ 5-Chlor-2-thienyl ist,
der Verbindung der Formel (IIa), bei der R²² 2-Chinolinylmethyl ist und R²³ 2-Chinolinylmethyl ist,
der Verbindung der Formel (IIa), bei der R²² 2-Propyl ist und R²³ 2-Picolinyl ist,
der Verbindung der Formel (IIa), bei der R²² 3-Benzyloxybenzyl ist und R²³ 3-Benzyloxybenzyl ist,
der Verbindung der Formel (IIa), bei der R²² 4-Phenylbenzyl ist und R²³ Phenylbenzyl ist,
der Verbindung der Formel (IIa), bei der R²² 2-Adamantylethyl ist und R²³ 2-Adamantylethyl ist,
der Verbindung der Formel (IIa), bei der R²² Wasserstoff ist und R²³ Cyclopropylmethyl ist,
der Verbindung der Formel (IIa), bei der R²² 2-Picolinyl ist und R²³ 2-Naphthylmethyl ist,
der Verbindung der Formel (IIa), bei der R²² 3-Allyl ist und R²³ Wasserstoff ist,
der Verbindung der Formel (IIa), bei der R²² 3-Allyl ist und R²³ 2-Picolinyl ist,
der Verbindung der Formel (IIa), bei der R²² 3-Allyl ist und R²³ 4-Picolinyl ist,
der Verbindung der Formel (IIa), bei der R²² 3-Benzyloxybenzyl ist und R²³ 3-Benzyloxybenzyl ist,
der Verbindung der Formel (IIa), bei der R²² 3-Cyclopropylmethoxybenzyl ist und R²³ 3-Cyclopropylmethoxybenzyl ist,
der Verbindung der Formel (IIa), bei der R²² 3-Ethoxybenzyl ist und R²³ 3-Ethoxybenzyl ist,
der Verbindung der Formel (IIa), bei der R²² 4-Benzyloxybenzyl ist und R²³ 4-Benzyloxybenzyl ist,
der Verbindung der Formel (IIa), bei der R²² 3-Hydroxybenzyl ist und R²³ 3-Hydroxybenzyl ist,
der Verbindung der Formel (IIa), bei der R²² 4-Hydroxybenzyl ist und R²³ 4-Hydroxybenzyl ist,
der Verbindung der Formel (IIa), bei der R²² 3-Hydroxymethylbenzyl ist und R²³ 3-Hydroxymethylbenzyl ist,
der Verbindung der Formel (IIa), bei der R²² 4-Hydroxymethylbenzyl ist und R²³ 4-Hydroxymethylbenzyl ist,
der Verbindung der Formel (IIa), bei der R²² 3-Aminobenzyl ist und R²³ 3-Aminobenzyl ist,
der Verbindung der Formel (IIa), bei der R²² 3-Carboxylbenzyl ist und R²³ 3-Carboxylbenzyl ist,
der Verbindung der Formel (IIa), bei der R²² 3-Formylbenzyl ist und R²³ 3-Formylbenzyl ist,
der Verbindung der Formel (IIa), bei der R²² 3-Cyanbenzyl ist und R²³ 3-Cyanbenzyl ist,
der Verbindung der Formel (IIa), bei der R²² 2-Naphthylmethyl ist und R²³ 2-Naphthylmethyl ist,
der Verbindung der Formel (IIa), bei der R²² n-Butyl ist und R²³ Benzyl ist,
der Verbindung der Formel (IIa), bei der R²² Allyl ist und R²³ Cyclopropylmethyl ist,
der Verbindung der Formel (IIa), bei der R²² n-Butyl ist und R²³ Cyclopropylmethyl ist,
der Verbindung der Formel (IIa), bei der R²² 3-Methallyl ist und R²³ Benzyl ist,
der Verbindung der Formel (IIa), bei der R²² Benzyl ist und R²³ Ethyl ist,
der Verbindung der Formel (IIa), bei der R²² Benzyl ist und R²³ 4-Picolinyl ist,
der Verbindung der Formel (IIa), bei der R²² Cyclopropylmethyl ist und R²³ 4-Picolinyl ist,
der Verbindung der Formel (IIa), bei der R²² Benzyl ist und R²³ Cyclopentylmethyl ist,
der Verbindung der Formel (IIa), bei der R²² Cyclopropylmethyl ist und R²³ Cyclopentylmethyl ist,
der Verbindung der Formel (IIa), bei der R²² Benzyl ist und R²³ n-Propyl ist,
der Verbindung der Formel (IIa), bei der R²² Cyclopropylmethyl ist und R²³ Cinnamyl ist,
der Verbindung der Formel (IIa), bei der R²² Cyclopropylmethyl ist und R²³ 2-Naphthylmethyl ist,
der Verbindung der Formel (IIa), bei der R²² Cyclopentylmethyl ist und R²³ 2-Naphthylmethyl ist,
der Verbindung der Formel (IIa), bei der R²² Benzyl ist und R²³ 2-Naphthylmethyl ist,
der Verbindung der Formel (IIa), bei der R²² Cyclopropylmethyl ist und R²³ 2-Picolinyl ist,
der Verbindung der Formel (IIa), bei der R²² 3-Cyanbenzyl ist und R²³ 3-Cyanbenzyl ist,
der Verbindung der Formel (IIa), bei der R²² 3-Allyl ist und R²³ 2-Naphthylmethyl ist,
der Verbindung der Formel (IIa), bei der R²² n-Propyl ist und R²³ 2-Naphthylmethyl ist,
der Verbindung der Formel (IIa), bei der R²² n-Butyl ist und R²³ 2-Naphthylmethyl ist,
der Verbindung der Formel (IIa), bei der R²² H ist und R²³ 2-Naphthylmethyl ist,
der Verbindung der Formel (IIa), bei der R²² 4-Picolinyl ist und R²³ 2-Naphthylmethyl ist,
der Verbindung der Formel (IIa), bei der R²² 3-Allyl ist und R²³ Cyclopentylmethyl ist,
der Verbindung der Formel (IIa), bei der R²² 3-Allyl ist und R²³ 2-Chinolinylmethyl ist,
der Verbindung der Formel (IIa), bei der R²² 3-Picolinyl ist und R²³ Cyclopropylmethyl ist,
der Verbindung der Formel (IIa), bei der R²² 3-Picolinyl ist und R²³ 2-Naphthylmethyl ist,
der Verbindung der Formel (IIa), bei der R²² 3-Allyloxybenzyl ist und R²³ 3-Allyloxybenzyl ist,
der Verbindung der Formel (IIa), bei der R²² 3-Allyloxybenzyl ist und R²³ 3-Hydroxybenzyl ist,
der Verbindung der Formel (IIa), bei der R²² 3-Picolinyl ist und R²³ 3-Picolinyl ist,
der Verbindung der Formel (IIa), bei der R²² 2-Naphthylmethyl ist und R²³ 4-Fluorbenzyl ist,
der Verbindung der Formel (IIa), bei der R²² 3-Carbomethoxybenzyl ist und R²³ 3-Carboxymethoxybenzyl ist,
der Verbindung der Formel (IIa), bei der R²² 4-Formylbenzyl ist und R²³ 4-Formylbenzyl ist,
der Verbindung der Formel (IIa), bei der R²² 4-Cyanbenzyl ist und R²³ 4-Cyanbenzyl ist,
der Verbindung der Formel (IIa), bei der R²² 4-Hydroxybenzyl ist und R²³ n-Propyl ist,
der Verbindung der Formel (IIa), bei der R²² 3-Hydroxybenzyl ist und R²³ n-Propyl ist,
der Verbindung der Formel (IIa), bei der R²² 3-Carboxybenzyl ist und R²³ 3-Carboxybenzyl ist,
der Verbindung der Formel (IIa), bei der R²² Cyclobutylmethyl ist und R²³ Cyclobutylmethyl ist,
der Verbindung der Formel (IIa), bei der R²² Cyclopentylmethyl ist und R²³ Cyclopentylmethyl ist,
der Verbindung der Formel (IIa), bei der R²² n-Butyl ist und R²³ 3-Methallyl ist,
der Verbindung der Formel (IIa), bei der R²² n-Butyl ist und R²³ Cyclopentylmethyl ist,
der Verbindung der Formel (IIa), bei der R²² 3-Formaldoximbenzyl ist und R²³ 3-Formaldoximbenzyl ist,
der Verbindung der Formel (IIa), bei der R²² Cyclopropylmethyl ist und R²³ 3-Hydroxybenzyl ist,
der Verbindung der Formel (IIa), bei der R²² Cyclopropylmethyl ist und R²³ 4-Hydroxybenzyl ist,
der Verbindung der Formel (IIa), bei der R²² 3-(N-Methylamino)benzyl ist und R²³ 3-(N-Methylamino)benzyl ist,
der Verbindung der Formel (IIa), bei der R²² 3-Acetylbenzyl ist und R²³ 3-Acetylbenzyl ist,
der Verbindung der Formel (IIa), bei der R²² 3-Hydroxylaminobenzyl ist und R²³ 3-Hydroxylaminobenzyl ist,
der Verbindung der Formel (IIa), bei der R²² 2-Naphthylmethyl ist und R²³ 3-Hydroxybenzyl ist,
der Verbindung der Formel (IIa), bei der R²² 4-Hydroxymethylbenzyl ist und R²³ 3-Hydroxybenzyl ist,
der Verbindung der Formel (IIa), bei der R²² N-Methyl-(3-amido)benzyl ist und R²³ N-Methyl-(3-amido)benzyl ist,
der Verbindung der Formel (IIa), bei der R²² N-Methyl-(3-amido)benzyl ist und R²³ 3-(Amidino)benzyl ist,
der Verbindung der Formel (IIa), bei der R²² 3-(5-Tetrazolyl)benzyl ist und R²³ Cyclopropylmethyl ist,
der Verbindung der Formel (IIa), bei der R²² 3-(5-Tetrazolyl)benzyl ist und R²³ 3-(5-Tetrazolyl)benzyl ist,
der Verbindung der Formel (IIa), bei der R²² 3-Phenylmethyldihydroxyboran ist und R²³ Phenylmethyl-3-boronsäure ist, besteht.

9. Verbindung von Anspruch 1 der Formel (IIa): 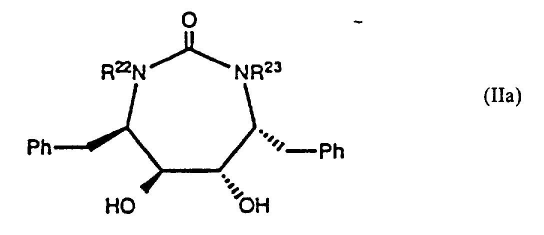 die aus der Gruppe ausgewählt ist, die aus
der Verbindung der Formel (IIa), bei der R²² Allyl ist und R²³ Allyl ist,
der Verbindung der Formel (IIa), bei der R²² Propyl ist und R²³ Propyl ist,
der Verbindung der Formel (IIa), bei der R²² Cyclopropylmethyl ist und R²³ Cyclopropylmethyl ist,
der Verbindung der Formel (IIa), bei der R²² n-Butyl ist und R²³ n-Butyl ist,
der Verbindung der Formel (IIa), bei der R²² Isopentyl ist und R²³ Isopentyl ist,
der Verbindung der Formel (IIa), bei der R²² 2-Methallyl ist und R²³ 2-Methallyl ist,
der Verbindung der Formel (IIa), bei der R²² n-Pentyl ist und R²³ n-Pentyl ist,
der Verbindung der Formel (IIa), bei der R²² Benzyl ist und R²³ Benzyl ist,
der Verbindung der Formel (IIa), bei der R²² Allyl ist und R²³ Isoprenyl ist,
der Verbindung der Formel (IIa), bei der R²² 3-Hydroxybenzyl ist und R²³ 3-Hydroxybenzyl ist,
der Verbindung der Formel (IIa), bei der R²² 4-Hydroxybenzyl ist und R²³ 4-Hydroxybenzyl ist,
der Verbindung der Formel (IIa), bei der R²² 3-Hydroxymethylbenzyl ist und R²³ 3-Hydroxymethylbenzyl ist,
der Verbindung der Formel (IIa), bei der R²² 4-Hydroxymethylbenzyl ist und R²³ 4-Hydroxymethylbenzyl ist,
der Verbindung der Formel (IIa), bei der R²² 3-Aminobenzyl ist und R²³ 3-Aminobenzyl ist,
der Verbindung der Formel (IIa), bei der R²² 3-Carboxylbenzyl ist und R²³ 3-Carboxylbenzyl ist,
der Verbindung der Formel (IIa), bei der R²² 3-Formylbenzyl ist und R²³ 3-Formylbenzyl ist,
der Verbindung der Formel (IIa), bei der R²² 3-Cyanbenzyl ist und R²³ 3-Cyanbenzyl ist,
der Verbindung der Formel (IIa), bei der R²² 2-Naphthylmethyl ist und R²³ 2-Naphthylmethyl ist,
der Verbindung der Formel (IIa), bei der R²² n-Butyl ist und R²³ Benzyl ist,
der Verbindung der Formel (IIa), bei der R²² Allyl ist und R²³ Cyclopropylmethyl ist,
der Verbindung der Formel (IIa), bei der R²² n-Butyl ist und R²³ Cyclopropylmethyl ist,
der Verbindung der Formel (IIa), bei der R²² 3-Methallyl ist und R²³ Benzyl ist,
der Verbindung der Formel (IIa), bei der R²² Benzyl ist und R²³ Ethyl ist,
der Verbindung der Formel (IIa), bei der R²² Benzyl ist und R²³ 4-Picolinyl ist,
der Verbindung der Formel (IIa), bei der R²² Cyclopropylmethyl ist und R²³ 4-Picolinyl ist,
der Verbindung der Formel (IIa), bei der R²² Benzyl ist und R²³ Cyclopentylmethyl ist,
der Verbindung der Formel (IIa), bei der R²² Cyclopropylmethyl ist und R²³ Cyclopentylmethyl ist,
der Verbindung der Formel (IIa), bei der R²² Benzyl ist und R²³ n-Propyl ist,
der Verbindung der Formel (IIa), bei der R²² Cyclopropylmethyl ist und R²³ Cinnamyl ist,
der Verbindung der Formel (IIa), bei der R²² Cyclopropylmethyl ist und R²³ 2-Naphthylmethyl ist,
der Verbindung der Formel (IIa), bei der R²² Cyclopentylmethyl ist und R²³ 2-Naphthylmethyl ist,
der Verbindung der Formel (IIa), bei der R²² Benzyl ist und R²³ 2-Naphthylmethyl ist,
der Verbindung der Formel (IIa), bei der R²² Cyclopropylmethyl ist und R²³ 2-Picolinyl ist,
der Verbindung der Formel (IIa), bei der R²² 3-Cyanbenzyl ist und R²³ 3-Cyanbenzyl ist,
der Verbindung der Formel (IIa), bei der R²² 3-Allyl ist und R²³ 2-Naphthylmethyl ist,
der Verbindung der Formel (IIa), bei der R²² n-Propyl ist und R²³ 2-Naphthylmethyl ist,
der Verbindung der Formel (IIa), bei der R²² n-Butyl ist und R²³ 2-Naphthylmethyl ist,
der Verbindung der Formel (IIa), bei der R²² H ist und R²³ 2-Naphthylmethyl ist,
der Verbindung der Formel (IIa), bei der R²² 4-Picolinyl ist und R²³ 2-Naphthylmethyl ist,
der Verbindung der Formel (IIa), bei der R²² 3-Allyl ist und R²³ Cyclopentylmethyl ist,
der Verbindung der Formel (IIa), bei der R²² 3-Allyl ist und R²³ 2-Chinolinylmethyl ist,
der Verbindung der Formel (IIa), bei der R²² 3-Picolinyl ist und R²³ Cyclopropylmethyl ist,
der Verbindung der Formel (IIa), bei der R²² 3-Picolinyl ist und R²³ 2-Naphthylmethyl ist,
der Verbindung der Formel (IIa), bei der R²² 3-Allyloxybenzyl ist und R²³ 3-Allyloxybenzyl ist,
der Verbindung der Formel (IIa), bei der R²² 3-Allyloxybenzyl ist und R²³ 3-Hydroxybenzyl ist,
der Verbindung der Formel (IIa), bei der R²² 3-Picolinyl ist und R²³ 3-Picolinyl ist,
der Verbindung der Formel (IIa), bei der R²² 2-Naphthylmethyl ist und R²³ 4-Fluorbenzyl ist,
der Verbindung der Formel (IIa), bei der R²² 3-Carbomethoxybenzyl ist und R²³ 3-Carbomethoxybenzyl ist,
der Verbindung der Formel (IIa), bei der R²² 4-Formylbenzyl ist und R²³ 4-Formylbenzyl ist,
der Verbindung der Formel (IIa), bei der R²² 4-Cyanbenzyl ist und R²³ 4-Cyanbenzyl ist,
der Verbindung der Formel (IIa), bei der R²² 4-Hydroxybenzyl ist und R²³ n-Propyl ist,
der Verbindung der Formel (IIa), bei der R²² 3-Hydroxybenzyl ist und R²³ n-Propyl ist,
der Verbindung der Formel (IIa), bei der R²² 3-Carboxybenzyl ist und R²³ 3-Carboxybenzyl ist,
der Verbindung der Formel (IIa), bei der R²² Cyclobutylmethyl ist und R²³ Cyclobutylmethyl ist,
der Verbindung der Formel (IIa), bei der R²² Cyclopentylmethyl ist und R²³ Cyclopentylmethyl ist,
der Verbindung der Formel (IIa), bei der R²² n-Butyl ist und R²³ 3-Methallyl ist,
der Verbindung der Formel (IIa), bei der R²² n-Butyl ist und R²³ Cyclopentylmethyl ist,
der Verbindung der Formel (IIa), bei der R²² 3-Formaldoximbenzyl ist und R²³ 3-Formaldoximbenzyl ist,
der Verbindung der Formel (IIa), bei der R²² Cyclopropylmethyl ist und R²³ 3-Hydroxybenzyl ist,
der Verbindung der Formel (IIa), bei der R²² Cyclopropylmethyl ist und R²³ 4-Hydroxybenzyl ist,
der Verbindung der Formel (IIa), bei der R²² 3-(N-Methylamino)benzyl ist und R²³ 3-(N-Methylamino)benzyl ist,
der Verbindung der Formel (IIa), bei der R²² 3-Acetylbenzyl ist und R²³ 3-Acetylbenzyl ist,
der Verbindung der Formel (IIa), bei der R²² 3-Hydroxylaminobenzyl ist und R²³ 3-Hydroxylaminobenzyl ist,
der Verbindung der Formel (IIa), bei der R²² 2-Naphthylmethyl ist und R²³ 3-Hydroxybenzyl ist,
der Verbindung der Formel (IIa), bei der R²² 4-Hydroxymethylbenzyl ist und R²³ 3-Hydroxybenzyl ist,
der Verbindung der Formel (IIa), bei der R²² N-Methyl-(3-amido)benzyl ist und R²³ N-Methyl-(3-amido)benzyl ist,
der Verbindung der Formel (IIa), bei der R²² N-Methyl-(3-amido)benzyl ist und R²³ 3-(Amidino)benzyl ist,
der Verbindung der Formel (IIa), bei der R²² 3-(5-Tetrazolyl)benzyl ist und R²³ Cyclopropylmethyl ist,
der Verbindung der Formel (IIa), bei der R²² 3-(5-Tetrazolyl)benzyl ist und R²³ 3-(5-Tetrazolyl)benzyl ist,
der Verbindung der Formel (IIa), bei der R²² 3-Phenylmethyldihydroxyboran ist und R²³ Phenylmethyl-3-boronsäure ist, besteht.

10. Verbindung von Anspruch 1 der Formel (IIa): 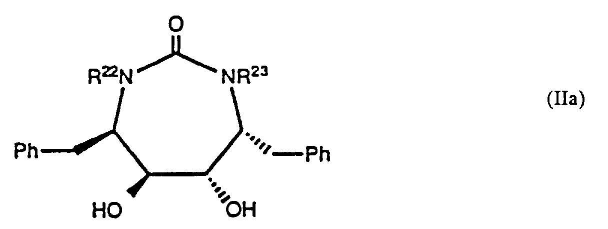 die aus der Gruppe ausgewählt ist, die aus
der Verbindung der Formel (IIa), bei der R²² Allyl ist und R²³ Allyl ist,
der Verbindung der Formel (IIa), bei der R²² Cyclopropylmethyl ist und R²³ Cyclopropylmethyl ist,
der Verbindung der Formel (IIa), bei der R²² n-Butyl ist und R²³ n-Butyl ist,
der Verbindung der Formel (IIa), bei der R²² Propyl ist und R²³ Propyl ist,
der Verbindung der Formel (IIa), bei der R²² Isopentyl ist und R²³ Isopentyl ist,
der Verbindung der Formel (IIa), bei der R²² Benzyl ist und R²³ Benzyl ist,
der Verbindung der Formel (IIa), bei der R²² 3-Hydroxybenzyl ist und R²³ 3-Hydroxybenzyl ist,
der Verbindung der Formel (IIa), bei der R²² 4-Hydroxybenzyl ist und R²³ 4-Hydroxybenzyl ist,
der Verbindung der Formel (IIa), bei der R²² 3-Hydroxymethylbenzyl ist und R²³ 3-Hydroxymethylbenzyl ist,
der Verbindung der Formel (IIa), bei der R²² 4-Hydroxymethylbenzyl ist und R²³ 4-Hydroxymethylbenzyl ist,
der Verbindung der Formel (IIa), bei der R²² 3-Aminobenzyl ist und R²³ 3-Aminobenzyl ist,
der Verbindung der Formel (IIa), bei der R²² 3-Carboxylbenzyl ist und R²³ 3-Carboxylbenzyl ist,
der Verbindung der Formel (IIa), bei der R²² 3-Formylbenzyl ist und R²³ 3-Formylbenzyl ist,
der Verbindung der Formel (IIa), bei der R²² 3-Formaldoximbenzyl ist und R²³ 3-Formaldoximbenzyl ist,
der Verbindung der Formel (IIa), bei der R²² 3-(N-Methylamino)benzyl ist und R²³ 3-(N-Methylamino)benzyl ist,
der Verbindung der Formel (IIa), bei der R²² 3-Acetylbenzyl ist und R²³ 3-Acetylbenzyl ist,
der Verbindung der Formel (IIa), bei der R²² 3-Hydroxylaminobenzyl ist und R²³ 3-Hydroxylaminobenzyl ist,
der Verbindung der Formel (IIa), bei der R²² 2-Naphthylmethyl ist und R²³ 3-Hydroxybenzyl ist,
der Verbindung der Formel (IIa), bei der R²² 4-Hydroxymethylbenzyl ist und R²³ 3-Hydroxybenzyl ist,
der Verbindung der Formel (IIa), bei der R²² N-Methyl-(3-amido)benzyl ist und R²³ N-Methyl-(3-amido)benzyl ist,
der Verbindung der Formel (IIa), bei der R²² N-Methyl-(3-amido)benzyl ist und R²³ 3-(Amidino)benzyl ist,
der Verbindung der Formel (IIa), bei der R²² 3-(5-Tetrazolyl)benzyl ist und R²³ Cyclopropylmethyl ist,
der Verbindung der Formel (IIa), bei der R²² 3-(5-Tetrazolyl)benzyl ist und R²³ 3-(5-Tetrazolyl)benzyl ist,
der Verbindung der Formel (IIa), bei der 3-Phenylmethyldihydroxyboran ist und R23 Phenylmethyl-3-boronsäure ist, besteht.

11. Verbindung von Anspruch 1 der Formel (IIb): 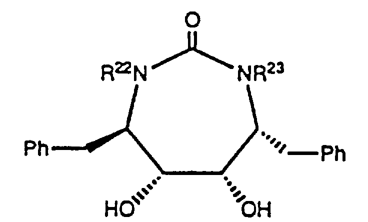 oder ein pharmazeutisch annehmbares Salz oder eine Prodrug-Form derselben, wobei
R²² und R²³ unabhängig aus der Gruppe ausgewählt sind, die aus
Wasserstoff, Cyclopropylmethyl, CH₂(C₆H₄)-p-OCH₂C₆H₅, CH₂(C₆H₄)-p-OH, Cyclopentylmethyl,Allyl, n-Butyl, beta-Naphthylmethyl, Benzyl, CH₂(C₆H₄)-m-OCH₂C₆H₅, p-Nitrobenzyl, m-Nitrobenzyl, CH₂(C₆H₄)-m-OH, CH₂(C₆H₄)-m-(CH₂OH), p-Aminobenzyl, m-Aminobenzyl, p-Nitrilobenzyl, m-Nitrilobenzyl, Dimethylallyl, Cyclohexylmethyl, Cyclobutylmethyl, Propyl, 3-Methyl-1-butyl, Carboxamidobenzyl und Formaldoximbenzyl besteht.

12. Verbindung von Anspruch 1 der Formel (Ib): 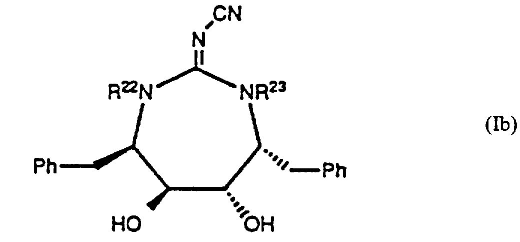 oder ein pharmazeutisch annehmbares Salz oder eine Prodrug-Form derselben, wobei
R²² und R²³ unabhängig aus der Gruppe ausgewählt sind, die aus
Wasserstoff, Cyclopropylmethyl, CH₂(C₆H₄)-p-OCH₂C₆H₅, CH₂(C₆H₄)-p-OH, Cyclopentylmethyl, Allyl, n-Butyl, beta-Naphthylmethyl, Benzyl, CH₂(C₆H₄)-m-OCH₂C₆H₅, p-Nitrobenzyl, m-Nitrobenzyl, CH₂(C₆H₄)-m-OH, p-Aminobenzyl, m-Aminobenzyl, p-Nitrilobenzyl, m-Nitrilobenzyl, Dimethylallyl, Cyclohexylmethyl, Cyclobutylmethyl, Propyl, 3-Methyl-1-butyl, Carboxamidobenzyl und Formaldoximbenzyl besteht.

13. Verwendung einer Verbindung gemäß einem der Ansprüche 1-12 zur Herstellung eines Arzneimittels zur Behandlung von Virusinfektionen.

14. Pharmazeutische Zusammensetzung, die einen pharmazeutisch annehmbaren Träger und wenigstens eine Verbindung gemäß einem der Ansprüche 1-12 umfaßt.

## Revendications

1. Un dérivé de formule (I): 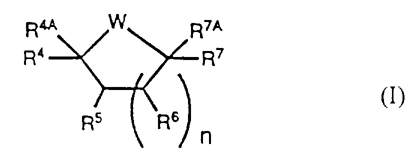 ou un de ses sels ou de ses précurseurs de médicament pharmaceutiquement acceptables dans laquelle:
R⁴ et R⁷ sont indépendamment choisis parmi les groupes suivants:
hydrogène;
alkyle en C₁ à C₈ substitué par 0 à 3 R¹¹;
alkényle en C₂ à C₈ substitué par 0 à 3 R¹₁;
alkynyle en C₁ à C₈ substitué par 0 à 3 R¹¹;
cycloalkyle en C₃ à C₈ substitué par 0 à 3 R¹¹;
bicycloalkyle en C₆ à C₁₀ substitué par 0 à 3 R¹¹;
aryle substitué par 0 à 3 R¹² ;
un résidu carbocyclique en C₆ à C₁₄ substitué par 0 à 3 R¹² ;
un système cyclique hétérocyclique substitué par 0 à 2 R¹², composé de 5 à 10 atomes y compris au moins un atome d'azote, d'oxygène ou de soufre;
R⁴ et R^{7A} sont indépendamment choisis parmi les groupes suivants:
hydrogène;
alkyle en C₁ à C₄ substitué par un halogène ou un alkoxy en C₁ à C₂ ;
benzyle substitué par un halogène ou un alkoxy en C₁ à C₂ ;
R⁴ et R^{4A} peuvent aussi se réunir pour former un cycle carbocyclique à 5 à 7 éléments substitués par 0 à 2 R¹² ;
R⁷ et R^{7A} peuvent aussi se réunir pour former un cycle carbocyclique à 5 à 7 éléments substitués par 0 à 2 R¹² ;
n est égal à 1;
R⁵ est choisi parmi: fluoro, difluoro, =O, alkyle en C₁ à C₃ ou -OR²⁰;
R⁶ est choisi parmi: hydrogène, =O, fluoro, difluoro, alkyle en C₁ à C₃, ou -OR²¹;
R⁵ et R⁶ peuvent aussi se réunir pour former un cycle époxyde; -OCH₂SCH₂O-; -OS(=O)O-; -OC(=O)O-; -OCH₂O-; -OC(=S)O-; -OC(=O)C(=O)O-; -OC(CH₃)₂O-; -OC(OCH₃) (CH₂CH₂CH₃)O-; ou bien un groupe hydrolysable quelconque, qui, après administration à un mammifère, subit un clivage métabolique pour former le diol originel dans lequel R₅ et R⁶ représentent -OH;
R²⁰ et R²¹ sont indépendamment choisis parmi:
hydrogène;
alkyle en C₁ à C₆ substitué par 0 à 3 R₁₁;
alkoxyalkyle en C₃ à C₆ substitué par 0 à 3 R¹¹;
alkylcarbonyle en C₁ à C₆ substitué par 0 à 3 R¹¹;
alkoxycarbonyle en C₁ à C₆ substitué par 0 à 3 R¹¹;
benzoyle substitué par 0 à 3 R¹²;
phénoxycarbonyle substitué par 0 à 3 R¹² ;
phénylaminocarbonyle substitué par 0 à 3 R¹²; ou un groupe hydrolysable quelconque qui après administration à un mammifère subit un clivage métabolique pour former le diol originel dans lequel R⁵ et R⁶ représentent -OH;
R¹¹ est choisi parmi l'un ou plusieurs des groupes suivants:
céto, halogène, cyano, -CH₂NR¹³R¹⁴, -NR¹³R¹⁴, -CO₂R¹³, -OC(=O)R¹³, -OR¹³, alkoxyalkyle en C₂-C₆, -S(O)ₘR¹³, -NHC(=NH)NHR¹³, C(=NH)NHR¹³, C(=O)NR¹³R¹⁴, -NR¹⁴C(=O)R¹³, =NOR¹⁴, -NR¹⁴C(=O)OR¹⁴, -OC(=O)NR¹³R¹⁴, NR¹³C(=O)NR¹³R¹⁴, -NR¹⁴SO₂NR¹³R¹⁴, -NR¹⁴SO₂R¹³, SO₂NR¹³R¹⁴, alkyle en C₁ à C₄, alkényle en C₂ à C₄, cycloalkyle en C₃ à C₆, cycloalkylméthyle en C₃ à C₆ ; 1 à 3 amino acides reliés ensemble par des liaisons amide et reliés à R⁴ ou R⁷ par la terminaison amine ou carboxylate;
un résidu carbocyclique en C₅ à C₁₄ substitué par 0 à 3 R₁₂ ;
aryle substitué par 0 à 3 R₁₂ ; ou
un système cyclique hétérocyclique substitué par 0 à 2 R¹², composé de 5 à 10 atomes comprenant au moins un atome d'azote, d'oxygène ou de soufre;
R¹², lorsqu'il est un substituant sur le carbone, est choisi parmi un ou plusieurs des groupes suivants:
phényle, benzyle, phénéthyle, phénoxy, benzyloxy, halogène, hydroxy, nitro, cyano, alkyle en C₁ à C₄, cycloalkyle en C₃ à C₆, cycloakylméthyle, arylalkyle en C₇ à C₁₀, alkoxy en C₁ à C₄, CO₂H, acide hydroxamique, hydrazide, oxime, acide boronique, sulfonamide, formyle, cycloalkoxy en C₃ à C₆, -OR¹³, alkyle en C₁ à C₄ substitué par -NR¹³R¹⁴, -NR¹³R¹⁴, alkoxyalkyle en C₂ à C₆, hydroxyalkyle en C₁ à C₄, méthylènedioxy, éthylènedioxy, haloalkyle en C₁ à C₄, haloalkoxy en C₁ à C₄, alkoxycarbonyle en C₁ à C₄, alkylcarbonyloxy en C₁ à C₄, alkylcarbonyle en C₁ à C₄, alkylcarbonylamino en C₁ à C₄, -S(O)ₘR¹³, -SO₂NR¹³R¹⁴; -NHSO₂R¹⁴, -OCH₂CO₂H, 2-(1-morpholino) éthoxy; ou
un cycle hétérocyclique à 5 ou 6 éléments contenant de 1 à 4 hétéroatomes choisis parmi oxygène, azote ou soufre;
ou bien R¹² peut être une chaîne de 3 à 4 carbones attachée à des carbones adjacents sur le cycle pour former un cycle condensé à 5 ou 6 éléments, ces cycles à 5 ou 6 éléments étant éventuellement substitués sur les carbones aliphatiques par un atome d'halogène, un alkyle en C₁ à C₄, un alkoxy en C₁ à C₄, hydroxy ou -NR¹³R¹⁴ ; ou bien, lorsque R¹² est rattaché à un atome de carbone saturé, il peut être un radical carbonyle ou thiocarbonyle;
R¹², lorsqu'il constitue un substituant sur l'azote est choisi parmi l'un ou plusieurs des groupes suivants:
phényle, benzyle, phénéthyle, hydroxy, hydroxyalkyle en C₁ à C₄, alkoxy en C₁ à C₄, alkyle en C₁ à C₄, cycloalkyle en C₃ à C₆, cycloalkylméthyle en C₃ à C₆, -NH₂NR¹³R¹⁴, -NR¹³R¹⁴, alkoxyalkyle en C₂ à C₆, haloalkyle en C₁ à C₄, alkoxycarbonyle en C₁ à C₄, -CO₂H, alkylcarbonyloxy en C₁ à C₄, alkylcarbonyle en C₁ à C₄;
R¹³ représente H, un radical phényle, benzyle, alkyle en C₁ à C₆, ou alkoxyalkyle en C₃ à C₆;
R¹⁴ représente OH, H, alkyle en C₁ à C₄, ou benzyle;
R¹³ et R¹⁴ peuvent aussi se réunir pour former -(CH₂)₄-, -(CH₂)₅-, -CH₂CH₂N(R¹⁵)CH₂CH₂-, ou -CH₂CH₂OCH₂CH₂-;
R¹⁵ représente H ou CH₃ ;
m représente 0, 1 ou 2;
w est choisi parmi:
-N(R²²)C(=Z)N(R²³)-;
-N(R²²)S(=O)N(R²³)-;
dans lequel:
Z représente 0, S ou NR²⁴;
R²² et R²³ sont indépendamment choisis parmi les groupes suivants:
hydrogène;
alkyle en C₁ à C₈ substitué par 0 à 3 R³¹;
alkényle en C₂ à C₈ substitué par 0 à 3 R³¹;
alkynyle en C₂ à C₈ substitué par 0 à 3 R³¹;
cycloalkyle en C₃ à C₈ substitué par 0 à 3 R³¹;
bicycloalkyle en C₆ à C₁₀ substitué par 0 à 3 R³¹;
aryle substitué par 0 à 3 R₃₂ ;
un résidu carbocyclique en C₆ à C₁₄ substitué par 0 à 3 R³²;
un système cyclique hétérocyclique substitué par 0 à 2 R³², composé de 5 à 10 atomes y compris au moins d'un atome, d'oxygène et de soufre;
R²⁴ est choisi parmi: hydroxy, amino, alkyle en C₁ à C₄, alkoxy en C₁ à C₄, aminoalkyle en C₁ à C₄, cyano; amino; benzyloxy;
ou encore, R²² peut être réuni avec R⁴ ou R^{4A} pour former un cycle hétérocyclique ou carbocyclique condensé à 5 ou 6 éléments substitués par 0 à 2 R¹² ; et
à titre de variante, R²³ peut être réuni avec R⁷ ou R^{7A} pour former un cycle hétérocyclique ou carbocyclique condensé à 5 ou 6 éléments substitués par 0 à 2 R¹²; et
à tite de variante, W peut être réuni avec R⁵ ou R⁶ pour former un cycle hétérocyclique ou carbocyclique condensé à trois à sept éléments substitués par 0 à 2 R¹² ;
R³¹ est choisi parmi un ou plusieurs des groupes suivants:
céto, halogène, cyano, -CH₂NR¹³R¹⁴, -NR¹³R¹⁴, -CO₂R¹³, -OC(=O)R¹³, -OR¹³, alkoxyalkyle en C₂ à C₆, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, OC(=O)NR¹³R⁴, =NR¹³C(=O)NR¹³R¹⁴, -NR¹⁴SO₂NR¹³R¹⁴, -NR¹⁴SO₂R¹³, -SO₂NR¹³R¹⁴, alkyle en C₁ à C₄, alkényle en C₂ à C₄, cycloalkyle en C₃ à C₆, cycloalkylméthyle en C₃ à C₆ ;
1-3 amino acides reliés ensemble par des liaisons amide et reliés à R⁴ ou R⁷ par la terminaison amine ou carboxylate;
un résidu carbocyclique en C₅ à C₁₄ substitué par 0 à 3 R³² ;
aryle substitué par 0 à 3 R³² ; ou
un système cyclique hétérocyclique substitué par 0 à 2 R³², composé de 5 à 10 atomes comprenant au moins un atome d'azote, d'oxygène ou de soufre;
R³², lorsqu'il représente un substituant sur le carbone, est choisi parmi un ou plusieurs des groupes suivants:
phényle, benzyle, phénéthyle, phénoxy, benzyloxy, halogène, hydroxy, nitro, cyano, alkyle en C₁ à C₄, cycloalkyle en C₃ à C₆, cycloalkylméthyle C₃ à C₆, arylalkyle en C₇ à C₁₀, alkoxy en C₁ à C₄, -CO₂H, acide hydroxamique, hydrazide, oxime, acide boronique, sulfonamide, formyle, cycloalkoxy en C₃ à C₆, -OR¹³, alkyle en C₁ à C₄ substitué par -NR¹³R¹⁴, -NR¹³R¹⁴, alkoxyalkyle en C₂ à C₆, hydroxyalkyle en C₁ à C₄, méthylènedioxy, éthylènedioxy, haloalkyle en C₁ à C₄, haloalkoxy en C₁ à C₄, alkoxycarbonyle en C₁ à C₄, alkylcarbonyloxy en C₁ à C₄, alkylcarbonyle en C₁ à C₄, alkylcarbonylamino en C₁ à C₄; -S(O)ₘR¹³, -SO₂NR¹³R¹⁴, -NHSO₂R¹⁴, -OCH₂CO₂H, 2-(1-morpholino) éthoxy, -C(R¹⁴)=N(OR¹⁴); ou
un cycle hétérocyclique à cinq ou six éléments comportant de 1 à 4 hétéroatomes choisis parmi des atomes d'oxygène, d'azote ou de soufre;
ou R³² peut être une chaîne à 3 ou 4 carbones rattachés à des carbones adjacents sur le cycle pour former un cycle condensé à 5 ou 6 éléments, ledit cycle à 5 à 6 éléments étant éventuellement substitué sur les carbones aliphatiques par un atome d'halogène, alkyle en C₁ à C₄, alkoxy en C₁ à C₄, hydroxy ou -NR¹³R¹⁴; ou bien. lorsque R³² est rattaché à un atome de carbone saturé, il peut être un radical carbonyle ou thiocarbonyle;
R³² lorsqu'il représente un substituant sur l'azote, est choisi parmi un ou plusieurs des groupes suivants;
phényle, benzyle, phénéthyle, hydroxy, hydroxyalkyle en C₁ à C₄, alkyle en C₁ à C₄, cycloalkyle en C₃ à C₆, cycloalkylméthyle en C₃ à C₆, -CH₂NR¹³R¹⁴, -NR¹³R¹⁴, alkoxyalkyle en C₂ à C₆, haloalkyle en C₁ à C₄, alkoxycarbonyle en C₁ à C₄, -CO₂H, alkylcarbonyloxy en C₁ à C₄, alkylcarbonyle en C₁ à C₄, -C(R¹⁴)=N(OR¹⁴);
pourvu que:
R⁴, R^{4A}, R⁷ et R^{7A} ne soient pas tous des atomes d'hydrogène;
lorsque R⁴ et R^{4A} sont tous les deux des atomes d'hydrogène, au moins l'un d'entre les suivants n'est pas un atome d'hydrogène: R²², R²³.

2. Un dérivé selon la revendication 1 de formule (I): 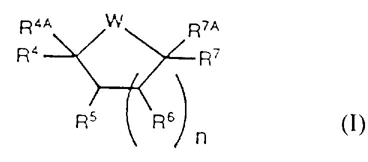 ou un de ses sels ou de ses précurseurs de médicament pharmaceutiquement acceptables dans laquelle :
R⁴ et R⁷ sont indépendamment choisis parmi les groupes suivants:
hydrogène;
alkyle en C₁ à C₄ substitué par 0 à 3 R¹¹;
alkényle en C₃ à C₄ substitué par 0 à 3 R¹¹;
alkynyle en C₃ à C₄ substitué par 0 à 3 R¹¹;
R^{4A} et R^{7A} sont des atomes d'hydrogène;
n est égal à 1;
R⁵ est choisi parmi: fluoro, difluoro, =O, ou -OR²⁰;
R⁶ est choisi parmi: hydrogène, =O, fluoro, difluoro, ou -OR²¹;
R⁵ et R⁶ peuvent aussi se réunir pour former un cycle époxyde; -OCH₂SCH₂O-; -OS(=O)O-; -OC(=O)O-; -OCH₂O-; -OC(=S)O-; -OC(=O)C(=O)O-; -OC(CH₃)₂O-; -OC(OCH₃) (CH₂CH₂CH₃)O-; ou bien un groupe hydrolysable quelconque, qui, lorsqu'on l'administre à un mammifère, subit un clivage métabolique pour former le diol initial dans lequel R⁵ et R⁶ représentent -OH;
R²⁰ et R²¹ sont indépendamment choisis parmi:
hydrogène;
alkylcarbonyle en C₁ à C₆ ;
alkoxycarbonyle en C₁ à C₆ ;
benzoyle, ou tout groupe hydrolysable qui, lorsqu'on l'administre à un mammifère subit un clivage métabolique pour former le diol initial dans lequel R⁵ et R⁶ sont -OH;
R¹¹ est choisi parmi l'un ou plusieurs des groupes suivants:
céto, halogène, cyano, -CH₂NR¹³R¹⁴, -NR¹³R¹⁴, -CO₂R¹³, -OC(=O)R¹³, -OR¹³, alkoxyalkyle en C₂-C₄, -S(O)ₘR¹³, alkyle en C₁ à C₄, alkényle en C₂ à C₄, cycloalkyle en C₃ à C₆;
un résidu carboxylique en C₅ à C₁₄ substitué par 0 à 3 R¹² ;
aryle substitué par 0 à 3 R¹² ; ou
un système cyclique hétérocyclique substitué par 0 à 2 R¹² composé de 5 à 10 atomes, comprenant au moins un atome d'azote, d'oxygène ou de soufre;
R¹², lorsqu'il constitue un substituant sur l'azote, est choisi parmi l'un ou plusieurs des groupes suivants:
phényle, benzyle, phénéthyle, phénoxy, benzyloxy, halogène, hydroxy, nitro, cyano, alkyle en C₁ à C₄, cycloalkyle en C₃ à C₆, cycloalkylméthyle en C₃ à C₆, arylalkyle en C₇ à C₁₀, alkoxy en C₁ à C₄, -CO₂H, acide hydroxamique, hydrazide, oxime, acide boronique, sulfonamide, formyle; cycloalkoxy en C₃ à C₆, -OR¹³, alkyle en C₁ à C₄ substitué par -NR¹³R¹⁴, -NR¹³R¹⁴, alkoxyalkyle en C₂ à C₆, hydroxyalkyle en C₁ à C₄, méthylènedioxy, éthylènedioxy, haloalkyle en C₁ à C₄, haloalkoxy en C₁ à C₄, alkoxycarbonyle en C₁ à C₄, alkylcarbonyloxy en C₁ à C₄, alkylcarbonyle en C₁ à C₄, alkylcarbonylamino en C₁ à C₄, -S(O)ₘR¹³, -SO₂NR¹³R¹⁴, -NHSO₂R¹⁴; ou
un cycle hétérocyclique à 5 à 6 éléments contenant de 1 à 4 hétéroatomes choisis parmi les atomes d'oxygène, d'azote ou de soufre;
ou bien R¹² peut être une chaîne à 3 à 4 carbones rattachées à des carbones adjacents sur le cycle pour former un cycle à 5 à 6 éléments, ledit cycle à 5 ou 6 éléments étant éventuellement substitué sur les carbones aliphatiques par un atome d'halogène, radical alkyle en C₁ à C₄, alkoxy en C₁ à C₄, hydroxy ou -NR¹³R¹⁴; ou bien, lorsque R¹² est rattaché à un atome de carbone saturé, il peut être le radical carbonyle ou thiocarbonyle;
R¹², lorsqu'il est un substituant sur l'azote, est choisi parmi un ou plusieurs des groupes suivants:
phényle, benzyle, phénéthyle, hydroxy, hydroxyalkyle en C₁ à C₄, alkoxy en C₁ à C₄, alkyle en C₁ à C₄, cycloalkyle en C₃ à C₆, cycloalkylméthyle en C₃ à C₆, -CH₂NR¹³R¹⁴, -NR¹³R¹⁴, alkoxyalkyle en C₂ à C₆, haloalkyle en C₁ à C₄, alkoxycarbonyle en C₁ à C₄, alkylcarbonyloxy en C₁ à C₄, alkylcarbonyle en C₁ à C₄, -CO₂H;
R¹³ représente H, un radical alkyle en C₁ à C₆, ou alkoxyalkyle en C₃ à C₆ ;
R¹⁴ est OH, H, un radical en C₁ à C₄, ou benzyle;
R¹³ et R¹⁴ peuvent aussi se réunir pour former -(CH₂)₄-, -(CH₂)₅-, -CH₂CH₂N(R¹⁵)CH₂CH₂-, ou -CH₂CH₂OCH₂CH₂-;
R¹⁵ représente H ou CH₃ ;
m représente 0, 1 ou 2;
w est choisi parmi:
-N(R²²)C(=Z)N(R²³)-;
dans laquelle:
Z représente 0, S, N-CN, N-OH, N-OCH₃;
R²² et R²³ sont indépendamment choisis parmi les groupes suivants:
hydrogène;
alkyle en C₁ à C₈ substitué par 0 à 3 R₃₁;
alkényle en C₃ à C₈ substitué par 0 à 3 R³¹;
alkynyle en C₃ à C₈ substitué par 0 à 3 R³¹;
cycloalkyle en C₃ à C₈ substitué par 0 à 3 R³¹;
R³¹ est choisi parmi un ou plusieurs des suivants:
céto, halogène, cyano, -CH₂NR¹³R¹⁴, -NR¹³R¹⁴, -CO₂R¹³, -OC(=O)R¹³, -OR¹³, alkoxyalkyle en C₂ à C₄, -S(O)ₘR¹³, alkyle en C₁ à C₄, alkényle en C₂ à C₄, cycloalkyle en C₃ à C₆,
un résidu carbocyclique en C₅ à C₁₄ substitué par 0 à 3 R³² ;
aryle substitué par 0 à 3 R³² ; ou
un système cyclique hétérocyclique substitué par 0 à 2 R³², composé de 5 à 10 atomes comprenant au moins un atome, d'azote, d'oxygène ou de soufre;
R³², lorsqu'il représente un substituant sur le carbone, est choisi parmi un ou plusieurs des groupes suivants:
phényle, benzyle, phénéthyle, phénoxy, benzyloxy, halogène, hydroxy, nitro, cyano, alkyle en C₁ à C₄, cycloalkyle en C₃ à C₆, cycloalkylméthyle C₃ à C₆, arylalkyle en C₇ à C₁₀, alkoxy en C₁ à C₄, -CO₂H, acide hydroxamique, hydrazide, oxime, acide boronique, sulfonamide, formyle, cycloalkoxy en C₃ à C₆, -OR¹³, alkyle en C₁ à C₄ substitué par -NR¹³R¹⁴, -NR¹³R¹⁴, alkoxyalkyle en C₂ à C₆, hydroxyalkyle en C₁ à C₄, méthylènedioxy, éthylènedioxy, haloalkyle en C₁ à C₄, haloalkoxy en C₁ à C₄, alkoxycarbonyle en C₁ à C₄, alkylcarbonyloxy en C₁ à C₄, alkylcarbonyle en C₁ à C₄, alkylcarbonylamino en C₁ à C₄; -S(O)ₘR¹³, -SO₂NR¹³R¹⁴, -NHSO₂R¹⁴, -C(R¹⁴)=N(OR¹⁴); ou
un cycle hétérocyclique à cinq ou six éléments contenant de 1 à 4 hétéroatomes choisis parmi des atomes d'oxygène, d'azote ou de soufre;
ou R³² peut être une chaîne à 3 ou 4 carbones rattachés à des carbones adjacents sur le cycle pour former un cycle condensé à 5 ou 6 éléments, ledit cycle à 5 à 6 éléments étant éventuellement substitué sur les carbones aliphatiques par un atome d'halogène, alkyle en C₁ à C₄, alkoxy en C₁ à C₄, hydroxy ou -NR¹³R¹⁴; ou bien, lorsque R³² est rattaché à un atome de carbone saturé, il peut être le radical carbonyle ou thiocarbonyle;
R³² lorsqu'il représente un substituant sur l'azote, est choisi parmi un ou plusieurs des groupes suivants:
phényle, benzyle, phénéthyle, hydroxy, hydroxyalkyle en C₁ à C₄, alkoxy en C₁ à C₄, alkyle en C₁ à C₄, cycloalkyle en C₃ à C₆, cycloalkylméthyle en C₃ à C₆, -CH₂NR¹³R¹⁴, -NR¹³R¹⁴, alkoxyalkyle en C₂ à C₆, haloalkyle en C₁ à C₄, alkoxycarbonyle en C₁ à C₄, alkylcarbonyloxy en C₁ à C₄, alkylcrbonyle en C₁ à C₄, -CO₂H, -C(R¹⁴)=N(OR¹⁴);
pourvu que:
R⁴, R^{4A}, R⁷ et R^{7A} ne soient pas tous des atomes d'hydrogène;
lorsque R⁴ et R^{4A} sont tous les deux des atomes d'hydrogène, au moins l'un d'entre les suivants n'est pas un atome d'hydrogène : R²², R²³.

3. Un dérivé selon la revendication 1 de formule (II): 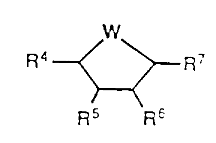 ou un de ses sels ou de ses formes précurseur de médicament pharmaceutiquement acceptables dans laquelle:
R⁴ et R⁷ sont indépendamment choisis parmi les groupes suivants:
hydrogène;
alkyle en C₁ à C₄ substitué par 0 à 3 R¹¹;
alkényle en C₂ à C₄ substitué par 0 à 3 R¹¹;
R⁵ représente -OR²⁰;
R⁶ est un atome d'hydrogène ou -OR²¹;
R²⁰ et R²¹ sont indépendamment un atome d'hydrogène ou un groupe hydrolysable quelconque qui, lorsqu'on l'administre à un mammifère, subit un clivage métabolique pour former le diol initial dans lequel R⁵ et R⁶ représentent -OH;
R¹¹ est choisi parmi un ou plusieurs des groupes suivants:
céto, halogène -CH₂NR¹³R¹⁴, -NR¹³R¹⁴, -OR¹³, alkoxyalkyle en C₂ à C₄, alkyle en C₁ à C₄, alkényle en C₂ à C₄, cycloalkyle en C₃ à C₆,
aryle substitué par 0 à 3 R¹² ; ou
un système cyclique hétérocyclique substitué par 0 à 2 R¹², composé de 5 à 10 atomes comprenant au moins un atome d'azote, d'oxygène ou de soufre;
R¹², lorsqu'il est un substituant sur le carbone, est choisi parmi un ou plusieurs des groupes suivants:
phényle benzyle, phénéthyle, phénoxy, benzyloxy, halogène, alkyle en C₁ à C₄, arylalkyle en C₇ à C₁₀, alkoxy en C₁ à C₄, CO₂H, acide hydroxamique, hydrazide, oxime, acide boronique, sulfonamide, formyle, cycloalkoxy en C₃ à C₆, -OR¹³, alkyle en C₁ à C₄ substitué par -NR¹³R¹⁴, -NR¹³R¹⁴, méthylènedioxy, haloalkyle en C₁ à C₄, alkoxycarbonyle en C₁ à C₄, alkylcarbonylamino en C₁ à C₄, hydroxy, hydroxyméthyle; ou
un cycle hétérocyclique à 5 ou 6 éléments contenant de 1 à 4 hétéroatomes choisis parmi oxygène, azote ou soufre;
R¹², lorsqu'il est un substituant sur un l'azote est choisi parmi les radicaux benzyle ou méthyle;
R¹³ représente H, alkyle en C₁ à C₂, ou alkoxyalkyle en C₃ à C₆;
R¹⁴ représente OH, H ou un radical alkyle en C₁ à C₂;
R¹³ et R¹⁴ peuvent aussi être réunis pour former -(CH₂)₄-, -(CH₂)₅-, -CH₂CH₂N(R¹⁵)CH₂CH₂-, ou -CH₂CH₂OCH₂CH₂-;
W représente -N(R²²)C(=Z)N(R²³)-;
dans laquelle
Z représente O, S ou N-CN;
R²² et R²³ sont indépendamment choisis parmi les groupes suivants:
hydrogène;
alkyle en C₁ à C₄ substitué par 0 à 3 R³¹;
alkényle en C₃ à C₄ substitué par 0 à 3 R³¹;
R³¹ est choisi parmi un ou plusieurs des groupes suivants:
céto, halogène, -CH₂NR¹³R¹⁴, -NR¹³R¹⁴, -OR¹³, alkoxyalkyle en C₂ à C₄, alkyle en C₁ à C₄, alkényle en C₂ à C₄, cycloalkyle en C₃ à C₆ ;
aryle substitué par 0 à 3 R³² ; ou
un système cyclique hétérocyclique substitué par 0 à 2 R³², composé de 5 à 10 atomes comprenant au moins un atome d'azote d'oxygène ou de soufre;
R³², lorsqu'il est un substituant sur le carbone, est choisi parmi un ou plusieurs des groupes suivants:
phényle, benzyle, phénéthyle, phénoxy, benzyloxy, halogène, alkyle en C₁ à C₄, arylalkyle en C₇ à C₁₀, alkoxy en C₁ à C₄, -CO₂H, acide hydroxamique, hydrazide, oxime, acide boronique, sulfonamide, formyle, cycloalkoxy en C₃ à C₆, -OR¹³, alkyle en C₁ à C₄ substitué par -NR¹³R¹⁴, -NR¹³R¹⁴, méthylènedioxy, haloalkyle en C₁ à C₄, alkylcarbonyle en C₁ à C₄, alkylcarbonylamino en C₁ à C₄, hydroxy, hydroxyméthyle, -C(R¹⁴)=N(OR¹⁴); ou
un cycle hétérocyclique à 5 ou 6 éléments contenant de 1 à 4 hétéroatomes choisis parmi oxygène, azote ou soufre;
R³², lorsqu'il est un substituant sur l'azote, est choisi parmi les radicaux benzyle ou méthyle;
pourvu que:
lorsque R⁴ est un atome d'hydrogène, R⁷ n'est pas un atome d'hydrogène;
lorsque R⁴ est un atome d'hydrogène, au moins l'un des suivants n'est pas un atome d'hydrogène: R²², R²³.

4. Un dérivé selon la revendication 3, dans lequel:
R⁴ et R⁷ sont indépendamment choisis parmi les groupes suivants:
hydrogène;
alkyle en C₁ à C₃ substitué par 0 à 1 R¹¹;
R⁵ est -OR²⁰;
R⁶ est un atome d'hydrogène ou -OR²¹;
R²⁰ et R²¹ sont indépendamment un hydrogène ou un groupe hydrolysable quelconque qui, lorsqu'il est administré à un mammifère, subit un clivage métabolique pour former le diol initial dans lequel R⁵ et R⁶ représentent -OH;
R¹¹ est choisi parmi un ou plusieurs des groupes suivants:
halogène, -OR¹³, alkyle en C₁ à C₄, cycloalkyle en C₃ à C₅ ;
aryle substitué par 0 à 2 R¹²; ou
un système cyclique hétérocyclique choisi parmi pyridyle, pyrimidinyle, triazinyle, furanyle, thiényle, pyrrolyle, pyrazolyle, imidazolyle, tétrazolyle, benzofuranyle, indolyle, quinolinyle, isoquinolinyle;
R¹², lorsqu'il est un substituant sur le carbone, est choisi parmi un ou plusieurs des groupes suivants :
benzyloxy, halogène, méthyle, alkoxy en C₁ à C₄, CF₃, 2-(1-morpholino)éthoxy, -CO₂H, acide hydroxamique, hydrazide, oxime, cyano, acide borique, sulfonamide, formyle, cycloalkoxy en C₃ à C₆, alkyle en C₁ à C₄ substitué par -NR¹³R¹⁴, -NR¹³R¹⁴, hydroxy, hydroxyméthyle; ou bien
R¹², lorsqu'il est un substituant sur l'azote représente un radical méthyle;
R¹³ représente H ou un radical méthyle;
R¹⁴ représente OH, H ou un radical méthyle;
R¹³ et R¹⁴ peuvent être réunis pour former (CH₂)₄-, -(CH₂)₅-, -CH₂CH₂N(R¹⁵)CH₂CH₂- ou -CH₂CH₂OCH₂CH₂-;
W représente -N(R²²)C(=O)N(R²³)- ou -N(R²²)C(=N-CN)N(R²³)-;
R²² et R²³ sont indépendamment choisis parmi les groupes suivants:
hydrogène;
alkyle en C₁ à C₄ substitué par 0 à 1 R³¹;
alkényle en C₃ à C₄ substitué par 0 à 1 R³¹;
R³¹ est choisi parmi un ou plusieurs des groupes suivants:
halogène; -OR¹³, alkyle en C₁ à C₄, cycloalkyle en C₃ à C₅;
aryle substitué par 0 à 2 R³² ; ou
un système cyclique hétérocyclique choisis parmi pyridyle, pyrimidinyle, triazinyle, furanyle, thiényle, pyrrolyle, pyrazolyle, imidazolyle, tétrazolyle, benzofuranyle, indolyle, quinolinyle, isoquinolinyle;
R³², lorsqu'il est un substituant sur le carbone, est choisi parmi l'un ou plusieurs des groupes suivants :
benzyloxy, halogène méthyle, alkoxy en C₁ à C₄, CF₃, 2-(1-morpholino)éthoxy, CO₂H, acide hydroxamique, hydrazide, oxime, cyano, acide boronique, sulfonamide, formyle, cycloalkoxy en C₃ à C₆, alkyle en C₁ à C₄ substitué par -NR¹³R¹⁴, -NR¹³R¹⁴, hydroxy, hydroxyméthyle, -C(R¹⁴)=N(OR¹⁴); ou bien
R³², lorsqu'il est un substituant sur l'azote représente un radical méthyle;
pourvu que:
lorsque R⁴ est un atome d'hydrogène, R⁷ n'est pas un atome d'hydrogène;
lorsque R⁴ est un atome d'hydrogène, au moins l'un d'entre les suivants n'est pas un atome d'hydrogène; R²² et R²³.

5. Un dérivé selon la revendication 3 dans lequel:
R⁴ et R⁷ représentent un radical benzyle;
R⁵ est -OH;
R⁶ est un atome d'hydrogène ou -OH;
R¹³ est H ou un méthyle;
R¹⁴ est H ou un méthyle;
W est -N(R²²)C(=O)N(R²³)- ou -N(R²²)C(=N-CN)N(R₂₃)-;
R²² et R²³ sont indépendamment choisis parmi les groupes suivants:
hydrogène;
alkyle en C₁ à C₄ substitué par 0 à 1 R³¹;
R³¹ est choisi parmi un ou plusieurs des groupes suivants:
cycloalkyle en C₃ à C₅;
aryle substitué par 0 à 2 R³² ; ou
un système de cyclique hétérocyclique choisi parmi pyridyle, thiényle, quinolinyle, ou isoquinolinyle;
R³², lorsqu'il est un substituant sur le carbone est choisi parmi un ou plusieurs des groupes suivants:
-CONH₂, -CO₂H, -CHO, -CH₂NHOH, -CH₂NR¹³R¹⁴, -NR¹³R¹⁴, hydroxy, hydroxyméthyle, -C(R¹⁴)=N(OR¹⁴); ou
R³², lorsqu'il est un substituant sur l'azote, représente le radical méthyle.

6. Un dérivé selon la revendication 1 de formule: 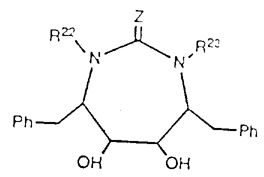 dans laquelle Z est O, S ou N-CN;
dans laquelle R²² et R²³ sont indépendamment choisis parmi le groupe consistant en:
atome d'hydrogène, allyle, propyle, cyclopropylméthyle, n-butyle, isobutyle, pyridylméthyle, méthallyle, n-pentyle, isopentyle, hexyle, benzyle, pyridylméthyle, isoprényle, propargyle, picolinyle, méthoxyéthyle, cyclohexylméthyle; diméthyl-butyle, éthoxyéthyle, méthyloxazolinylméthyle, naphtylméthyle, méthyloxazolinylméthyle, vinyloxyéthyle, pentafluorobenzyle, quinolinylméthyle, carboxybenzyle, chloro-thiényle, picolinyle, benzyloxybenzyle, phénylbenzyle, adamantyléthyle, cyclopropylméthoxybenzyle, éthoxybenzyle, hydroxybenzyle, hydroxyméthylbenzyle, aminobenzyle, formylbenzyle, cyanobenzyle, cinnamyle, allyloxybenzyle, fluorobenzyle, cyclobutylméthyle, formaldoximébenzyle, cyclopentylméthyle, nitrobenzyle, nitrilobenzyle, carboxamidobenzyle, carbométhoxybenzyle, tétrazolylbenzyle et diméthylallyle.

7. Un dérivé selon la revendication 1 de formule (IIa): 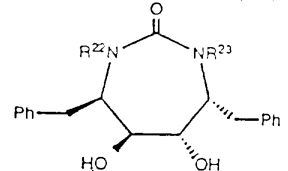 dans laquelle R²² et R²³ sont indépendamment choisis parmi le groupe consistant en:
hydrogène, allyle, propyle, cyclopropylméthyle, n-butyle, iso-butyle, pyridylméthyle, méthallyle, n-pentyle, isopentyle, hexyle, benzyle, pyridylméthyle, iso-prényle, propargyle, picolinyle, méthoxyéthyle, cyclohexylméthyle, diméthyl-butyle, éthoxyéthyle, méthyl-oxazolinylméthyle, naphtylméthyle, méthyloxazolinylméthyle, vinyloxyéthyle, pentafluorobenzyle, quinolinylméthyle, carboxybenzyle, chloro-thiényle, picolinyle, benzyloxybenzyle, phénylbenzyle, adamantyléthyle, cyclopropylméthoxybenzyle, éthoxybenzyle, hydroxybenzyle, hydroxyméthylbenzyle, aminobenzyle, formylbenzyle, cyanobenzyle, cinnamyle, allyloxybenzyle, fluorobenzyle, cyclobutylméthyle, formaldoximébenzyle, cyclopentylméthyle, nitrobenzyle, nitrilobenzyle, carboxamidobenzyle, carbométhoxybenzyle, tétrazolylbenzyle, diméthylallyle, amidinobenzyle et (acide boronique) benzyle.

8. Un dérivé selon la revendication 1 de formule (IIa): 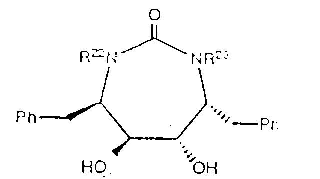 qui est choisi dans le groupe consistant en:
le dérivé de formule (IIa) dans lequel R²² représente un allyle et R²³ un allyle;
le dérivé de formule (IIa) dans lequel R²² représente un propyle et R²³ un propyle;
le dérivé de formule (IIa) dans lequel R²² représente un cyclopropylméthyle et R²³ est un cyclopropylméthyle;
le dérivé de formule (IIa) dans lequel R²² est un n-butyle et R²³ est un n-butyle;
le dérivé de formule (IIa) dans lequel R²² est un iso-pentyle et R²³ est un iso-pentyle;
le dérivé de formule (IIa) dans lequel R²² est un 4-pyridylméthyle et R²³ est un 4-pyridylméthyle;
le dérivé de formule (IIa) dans lequel R²² est un 2-méthallyle et R²³ est un 2-méthallyle;
le dérivé de formule (IIa) dans lequel R²² est un n-pentyle et R²³ est un n-pentyle;
le dérivé de formule (IIa) dans lequel R²² est un isobutyle et R²³ est un isobutyle;
le dérivé de formule (IIa) dans lequel R²² est un benzyle et R²³ est un benzyle;
le dérivé de formule (IIa) dans lequel R²² est un 3-pyridylméthyle et R²³ est un 3-pyridylméthyle;
le dérivé de formule (IIa) dans lequel R²² est un allyle et R²³ est un isoprényle;
le dérivé de formule (IIa) dans lequel R²² est un 3-propargyle et R²³ est un 3-propargyle;
le dérivé de formule (IIa) dans lequel R²² est un 2-picolinyle et R²³ est un 2-picolinyle;
le dérivé de formule (IIa) dans lequel R²² est un 2-méthoxyéthyle et R²³ est un 2-méthoxyéthyle;
le dérivé de formule (IIa) dans lequel R²² est un cyclohexylméthyle et R²³ est un cyclohexylméthyle;
le dérivé de formule (IIa) dans lequel R²² est un 3,3-diméthyl-1-butyle et R²³ est un 3,3-diméthyl-1 butyle;
le dérivé de formule (IIa) dans lequel R²² est un 2-éthoxyéthyle et R²³ est un 2-éthoxyéthyle;
le dérivé de formule (IIa) dans lequel R²² est un 3-méthyl-5-oxazolinylméthyle et R²³ est un hydrogène;
le dérivé de formule (IIa) dans lequel R²² est un 1-naphtylméthyle et R²³ est un 1-naphtylméthyle;
le dérivé de formule (IIa) dans lequel R²² est un 3-méthyloxazolinylméthyle et R²³ est un 3-méthyloxazolinylméthyle;
le dérivé de formule (IIa) dans lequel R²² est un 2-vinyloxyéthyle et R²³ est un 2-vinyloxyéthyle:
le dérivé de formule (IIa) dans lequel R²² est 2,3,4,5,6-pentafluorobenzyle et R²³ est un 2,3,4,5,6-pentafluorobenzyle;
le dérivé de formule (IIa) dans lequel R²² est un benzyle et R²³ est un 2-quinolinylméthyle;
le dérivé de formule (IIa) dans lequel R²² est un 4-carboxybenzyle et R²³ est un 4-carboxybenzyle;
le dérivé de formule (IIa) dans lequel R²² est un 5-chloro-2-thiényle et R²³ est un 5-chlore-2-thiényle;
le dérivé de formule (IIa) dans lequel R²² est un 2-quinolinylméthyle et R₂₃ est un 2-quinolinylméthyle;
le dérivé de formule (IIa) dans lequel R²² est un 2-propyle et R²³ est un 2-picolinyle;
le dérivé de formule (IIa) dans lequel R²² est un 3-benzyloxybenzyle et R²³ est un 3-benzyloxybenzyle;
le dérivé de formule (IIa) dans lequel R²² est un 4-phénylbenzyle et R²³ est un phénylbenzyle;
le dérivé de formule (IIa) dans lequel R²² est un 2-adamantyléthyle et R²³ est un 2-adamantyléthyle;
le dérivé de formule (IIa) dans lequel R²² est un hydrogène et R²³ est un cyclopropylméthyle;
le dérivé de formule (IIa) dans lequel R²² est un 2-picolinyle et R²³ est un 2-naphtylméthyle;
le dérivé de formule (IIa) dans lequel R²² est un 3-allyle et R²³ est un hydrogène;
le dérivé de formule (IIa) dans lequel R²² est un 3-allyle et R²³ est un 2-picolinyle;
le dérivé de formule (IIa) dans lequel R²² est un 3-allyle et R²³ est un 4-picolinyle;
le dérivé de formule (IIa) dans lequel R²² est un 3-benzyloxybenzyle et R²³ est un 3-benzyloxybenzyle;
le dérivé de formule (IIa) dans lequel R²² est un 3-cyclopropylméthoxybenzyle et R²³ est un 3-cyclopropylméthoxybenzyle;
le dérivé de formule (IIa) dans lequel R²² est un 3-éthoxybenzyle et R²³ est un 3-éthoxybenzyle;
le dérivé de formule (IIa) dans lequel R²² est un 4-benzyloxybenzyle et R₂₃ est un 4-benzyloxybenzyle;
le dérivé de formule (IIa) dans lequel R²² est un 3-hydroxybenzyle et R²³ est un 3-hydroxybenzyle;
le dérivé de formule (IIa) dans lequel R²² est un 4-hydroxybenzyle et R²³ est un 4-hydroxybenzyle;
le dérivé de formule (IIa) dans lequel R²² est un 3-hydroxyméthylbenzyle et R²³ est un 3-hydroxyméthylbenzyle;
le dérivé de formule (IIa) dans lequel R²² est un 4-hydroxyméthylbenzyle et R²³ est un 4-hydroxyméthylbenzyle;
le dérivé de formule (IIa) dans lequel R²² est un 3-aminobenzyle et R²³ est un 3-aminobenzyle;
le dérivé de formule (IIa) dans lequel R²² est un 3-carboxylbenzyle et R²³ est un 3-carboxylbenzyle;
le dérivé de formule (IIa) dans lequel R²² est un 3-formylbenzyle et R²³ est un 3-formylbenzyle;
le dérivé de formule (IIa) dans lequel R²² est un 3-cyanobenzyle et R²³ est un 3-cyanobenzyle;
le dérivé de formule (IIa) dans lequel R²² est un 2-naphtylméthyle et R²³ est un 2-naphtylméthyle;
le dérivé de formule (IIa) dans lequel R²² est un n-butyle et R²³ est un benzyle;
le dérivé de formule (IIa) dans lequel R²² est un allyle et R²³ est un cyclopropylméthyle;
le dérivé de formule (IIa) dans lequel R²² est un 3-méthallyle et R²³ est un benzyle;
le dérivé de formule (IIa) dans lequel R²² est un benzyle et R²³ est un éthyle;
le dérivé de formule (IIa) dans lequel R²² est un benzyle et R²³ est un 4-picolinyle;
le dérivé de formule (IIa) dans lequel R²² est un cyclopropylméthyle et R²³ est un 4-picolinyle;
le dérivé de formule (IIa) dans lequel R²² est un benzyle et R²³ est un cyclopentylméthyle;
le dérivé de formule (IIa) dans lequel R²² est un cyclopropylméthyle et R²³ est un cyclopentylméthyle;
le dérivé de formule (IIa) dans lequel R²² est un benzyle et R²³ est un n-propyle;
le dérivé de formule (IIa) dans lequel R²² est un cyclopropylméthyle et R²³ est un cinnamyle;
le dérivé de formule (IIa) dans lequel R²² est un cyclopropylméthyle et R₂₃ est 2-naphtylméthyle;
le dérivé de formule (IIa) dans lequel R²² est un cyclopentylméthyle et R²³ est 2-naphtylméthyle;
le dérivé de formule (IIa) dans lequel R²² est un benzyle et R²³ est 2-naphtylméthyle;
le dérivé de formule (IIa) dans lequel R²² est un cyclopropylméthyle et R²³ est un 2-picolinyle;
le dérivé de formule (IIa) dans lequel R²² est un 3-cyanobenzyle et R²³ est un 3-cyanobenzyle;
le dérivé de formule (IIa) dans lequel R²² est un 3-allyle et R²³ est un 2-napthylméthyle;
le dérivé de formule (IIa) dans lequel R²² est un n-propyle et R²³ est un 2-naphtylméthyle;
le dérivé de formule (IIa) dans lequel R²² est un n-butyle et R²³ est un 2-naphtylméthyle;
le dérivé de formule (IIa) dans lequel R²² est un H et R²³ est un 2-naphtylméthyle;
le dérivé de formule (IIa) dans lequel R²² est un 4-picolinyle et R²³ est un 2-naphtylméthyle;
le dérivé de formule (IIa) dans lequel R²² est un 3-allyle et R²³ est un cyclopentylméthyle;
le dérivé de formule (IIa) dans lequel R²² est un 3-allyle et R²³ est un 2-quinolinylméthyle;
le dérivé de formule (IIa) dans lequel R²² est un 3-picolinyle et R²³ est un cyclopropylméthyle;
le dérivé de formule (IIa) dans lequel R²² est un 3-picolinyle et R²³ est un 2-naphtylméthyle;
le dérivé de formule (IIa) dans lequel R²² est un 3-allyloxybenzyle et R²³ est un 3-allyloxybenzyle;
le dérivé de formule (IIa) dans lequel R²² est un 3-allyloxybenzyle et R²³ est un 3-hydroxybenzyle;
le dérivé de formule (IIa) dans lequel R²² est un 3-picolinyle et R²³ est un 3-picolinyle;
le dérivé de formule (IIa) dans lequel R²² est un 2-napthylméthyle et R²³ est un 4-fluorobenzyle;
le dérivé de formule (IIa) dans lequel R²² est un 3-carbométhoxybenzyle et R²³ est un 3-carbométhyloxybenzyle;
le dérivé de formule (IIa) dans lequel R²² est un 4-formylbenzyle et R²³ est un 4-formylbenzyle;
le dérivé de formule (IIa) dans lequel R²² est un 4-cyanobenzyle et R²³ est un 4-cyanobenzyle;
le dérivé de formule (IIa) dans lequel R²² est un 4-hydroxybenzyle et R²³ est un n-propyle;
le dérivé de formule (IIa) dans lequel R²² est un 3-hydroxybenzyle et R²³ est un n-propyle;
le dérivé de formule (IIa) dans lequel R²² est un 3-carboxybenzyle et R²³ est un 3-carboxybenzyle;
le dérivé de formule (IIa) dans lequel R²² est un cyclobutylméthyle et R²³ est un cyclobutylméthyle;
le dérivé de formule (IIa) dans lequel R²² est un cyclopentylméthyle et R²³ est un cyclopentylméthyle;
le dérivé de formule (IIa) dans lequel R²² est un n-butyle et R²³ est un 3-méthallyle;
le dérivé de formule (IIa) dans lequel R²² est un n-butyle et R²³ est un cyclopentylméthyle;
le dérivé de formule (IIa) dans lequel R²² est un 3-formaldoximébenzyle et R₂₃ est un 3-formaldoximébenzyle;
le dérivé de formule (IIa) dans lequel R²² est un cyclopropylméthyle et R²³ est un 3-hydroxybenzyle;
le dérivé de formule (IIa) dans lequel R²² est un cyclopropylméthyle et R²³ est un 4-hydroxybenzyle;
le dérivé de formule (IIa) dans lequel R²² est un 3-(N-méthylamino)benzyle et R²³ est un 3-(N-méthylaminobenzyle;
le dérivé de formule (IIa) dans lequel R²² est un 3-acétylbenzyle et R²³ est un 3-acétylbenzyle;
le dérivé de formule (IIa) dans lequel R²² est un 3-hydroxylaminobenzyle et R²³ est un 3-hydroxylaminobenzyle;
le dérivé de formule (IIa) dans lequel R²² est un 2-napthylméthyle et R²³ est un 3-hydroxybenzyle;
le dérivé de formule (IIa) dans lequel R²² est un 4-hydroxyméthylbenzyle et R²³ est un 3-hydroxybenzyle;
le dérivé de formule (IIa) dans lequel R²² est un N-méthyl-(3-amido)benzyle et R²³ est un N-méthyl-(3-amido)benzyle;
le dérivé de formule (IIa) dans lequel R²² est un N-méthyl-(3-amido)benzyle et R²³ est un 3-(amidino)benzyle;
le dérivé de formule (IIa) dans lequel R²² est un 3-(5-tétrazolyl)benzyle et R²³ est un cyclopropylméthyle;
le dérivé de formule (IIa) dans lequel R²² est un 3-(5-tétrazolyl)benzyle et R²³ est un 3-(5-tétrazolyl)benzyle;
le dérivé de formule (IIa) dans lequel R²² est un phénylméthyl-3 acide boronique et R²³ est un phénylméthyl-3-acide boronique.

9. Un dérivé selon la revendication 1 de formule (IIa): 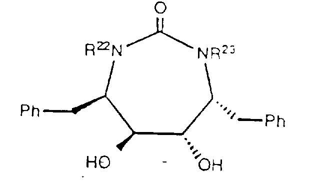 qui est choisi dans le groupe constitué par:
le dérivé de formule (IIa) dans lequel R²² est un allyle et R₂₃ un allyle;
le dérivé de formule (IIa) dans lequel R²² est un propyle et R²³ un propyle;
le dérivé de formule (IIa) dans lequel R₂₂ est un cyclopropylméthyle et R²³ est un cycloplopylméthyle;
le dérivé de formule (IIa) dans lequel R²² est un n-butyle et R₂₃ est un n-butyle;
le dérivé de formule (IIa) dans lequel R²² est un isopentyle et R²³ est un isopentyle;
le dérivé de formule (IIa) dans lequel R²² est un 2-méthallyle et R²³ est un 2-méthallyle;
le dérivé de formule (IIa) dans lequel R²² est un n-pentyle et R²³ est un n-pentyle;
le dérivé de formule (IIa) dans lequel R²² est un benzyle et R²³ est un benzyle;
le dérivé de formule (IIa) dans lequel R²² est un allyle et R²³ est un isoprényle;
le dérivé de formule (IIa) dans lequel R²² est un 3-hydroxybenzyle et R²³ est un 3-hydroxybenzyle;
le dérivé de formule (IIa) dans lequel R²² est un 4-hydroxybenzyle et R²³ est un 4-hydroxybenzyle;
le dérivé de formule (IIa) dans lequel R²² est un 3-hydroxyméthylbenzyle et R²³ est un 3-hydroxyméthylbenzyle;
le dérivé de formule (IIa) dans lequel R²² est un 4-hydroxyméthylbenzyle et R²³ est un 4-hydroxyméthylbenzyle;
le dérivé de formule (IIa) dans lequel R²² est un 3-aminobenzyle et R²³ est un 3-aminobenzyle;
le dérivé de formule (IIa) dans lequel R²² est un 3-carboxylbenzyle et R²³ est un 3-carboxylbenzyle;
le dérivé de formule (IIa) dans lequel R²² est un 3-formylbenzyle et R²³ est un 3-formylbenzyle;
le dérivé de formule (IIa) dans lequel R²² est un 3-cyanobenzyle et R²³ est un 3-cyanobenzyle;
le dérivé de formule (IIa) dans lequel R²² est un 2-naphtylméthyle et R²³ est un 2-naphtylméthyle;
le dérivé de formule (IIa) dans lequel R²² est un n-butyle et R²³ est un benzyle;
le dérivé de formule (IIa) dans lequel R²² est un allyle et R²³ est un cyclopropylméthyle;
le dérivé de formule (IIa) dans lequel R²² est un n-butyle et R₂₃ est un cyclopropylméthyle;
le dérivé de formule (IIa) dans lequel R²² est un 3-méthallyle et R²³ est un benzyle;
le dérivé de formule (IIa) dans lequel R²² est un benzyle et R²³ est un éthyle; le dérivé de formule (IIa) dans lequel R²² est un benzyle et R²³ est un 4-picolinyle;
le dérivé de formule (IIa) dans lequel R²² est un cyclopropylméthyle et R²³ est un 4-picolinyle:
le dérivé de formule (IIa) dans lequel R²² est un benzyle et R²³ est un cyclopentylméthyle;
le dérivé de formule (IIa) dans lequel R²² est un cyclopropylméthyle et R²³ est un cyclopentylméthyle;
le dérivé de formule (IIa) dans lequel R²² est un benzyle et R²³ est un n-propyle:
le dérivé de formule (IIa) dans lequel R²² est un cyclopropylméthyle et R²³ est un cinnamyle;
le dérivé de formule (IIa) dans lequel R²² est un cyclopropylméthyle et R₂₃ est 2-naphtylméthyle;
le dérivé de formule (IIa) dans lequel R²² est un cyclopentylméthyle et R²³ est 2-naphtylméthyle;
le dérivé de formule (IIa) dans lequel R²² est un benzyle et R²³ est 2-naphtylméthyle;
le dérivé de formule (IIa) dans lequel R²² est un cyclopropylméthyle et R²³ est un 2-picolinyle;
le dérivé de formule (IIa) dans lequel R²² est un 3-cyanobenzyle et R²³ est un 3-cyanobenzyle;
le dérivé de formule (IIa) dans lequel R²² est un 3-allyle et R²³ est un 2-napthylméthyle;
le dérivé de formule (IIa) dans lequel R²² est un n-propyle et R²³ est un 2-naphtylméthyle;
le dérivé de formule (IIa) dans lequel R²² est un n-butyle et R²³ est un 2-naphtylméthyle;
le dérivé de formule (IIa) dans lequel R²² est un H et R²³ est un 2-naphtylméthyle;
le dérivé de formule (IIa) dans lequel R²² est un 4-picolinyle et R²³ est un 2-naphtylméthyle;
le dérivé de formule (IIa) dans lequel R²² est un 3-allyle et R²³ est un cyclopentylméthyle;
le dérivé de formule (IIa) dans lequel R²² est un 3-allyle et R²³ est un 2-quinolinylméthyle;
le dérivé de formule (IIa) dans lequel R²² est un 3-picolinyle et R²³ est un cyclopropylméthyle;
le dérivé de formule (IIa) dans lequel R²² est un 3-picolinyle et R²³ est un 2-naphtylméthyle;
le dérivé de formule (IIa) dans lequel R²² est un 3-allyloxybenzyle et R²³ est un 3-allyloxybenzyle;
le dérivé de formule (IIa) dans lequel R²² est un 3-allyloxybenzyle et R²³ est un 3-hydroxybenzyle;
le dérivé de formule (IIa) dans lequel R²² est un 3-picolinyle et R²³ est un 3-picolinyle:
le dérivé de formule (IIa) dans lequel R²² est un 2-napthylméthyle et R²³ est un 4-fluorobenzyle;
le dérivé de formule (IIa) dans lequel R²² est un 3-carbométhoxybenzyle et R²³ est un 3-carbométhyloxybenzyle;
le dérivé de formule (IIa) dans lequel R²² est un 4-formylbenzyle et R²³ est un 4-formylbenzyle;
le dérivé de formule (IIa) dans lequel R²² est un 4-cyanobenzyle et R²³ est un 4-cyanobenzyle;
le dérivé de formule (IIa) dans lequel R²² est un 4-hydroxybenzyle et R²³ est un n-propyle;
le dérivé de formule (IIa) dans lequel R²² est un 3-hydroxybenzyle et R²³ est un n-propyle;
le dérivé de formule (IIa) dans lequel R²² est un 3-carboxybenzyle et R²³ est un 3-carboxybenzyle;
le dérivé de formule (IIa) dans lequel R²² est un cyclobutylméthyle et R²³ est un cyclobutylméthyle;
le dérivé de formule (IIa) dans lequel R²² est un cyclopentylméthyle et R²³ est un cyclopentylméthyle;
le dérivé de formule (IIa) dans lequel R²² est un n-butyle et R²³ est un 3-méthallyle;
le dérivé de formule (IIa) dans lequel R²² est un n-butyle et R²³ est un cyclopentylméthyle;
le dérivé de formule (IIa) dans lequel R²² est un 3-formaldoximébenzyle et R²³ est un 3-formaldoximébenzyle;
le dérivé de formule (IIa) dans lequel R²² est un cyclopropylméthyle et R²³ est un 3-hydroxybenzyle;
le dérivé de formule (IIa) dans lequel R²² est un cyclopropylméthyle et R²³ est un 4-hydroxybenzyle;
le dérivé de formule (IIa) dans lequel R²² est un 3-(N-méthylamino)benzyle et R²³ est un 3-(N-méthylaminobenzyle;
le dérivé de formule (IIa) dans lequel R²² est un 3-acétylbenzyle et R²³ est un 3-acétylbenzyle;
le dérivé de formule (IIa) dans lequel R²² est un 3-hydroxylaminobenzyle et R²³ est un 3-hydroxylaminobenzyle;
le dérivé de formule (IIa) dans lequel R²² est un 2-napthylméthyle et R²³ est un 3-hydroxybenzyle;
le dérivé de formule (IIa) dans lequel R²² est un 4-hydroxyméthylbenzyle et R²³ est un 3-hydroxybenzyle;
le dérivé de formule (IIa) dans lequel R²² est un méthyl-(3-amido)benzyle et R²³ est un N-méthyl-(3-amido)benzyle;
le dérivé de formule (IIa) dans lequel R²² est un N-méthyl-(3-amido)benzyle et R²³ est un 3-(amidino)benzyle;
le dérivé de formule (IIa) dans lequel R²² est un 3-(5-tétrazolyl)benzyle et R²³ est un cyclopropylméthyle;
le dérivé de formule (IIa) dans lequel R²² est un 3-(5-tétrazolyl)benzyle et R²³ est un 3-(5-tétrazolyl)benzyle;
le dérivé de formule (IIa) dans lequel R²² est un phénylméthyl-3 acide boronique et R²³ est un phénylméthyl-3-acide boronique;

10. Le dérivé selon la revendication 1 de formule (IIa): 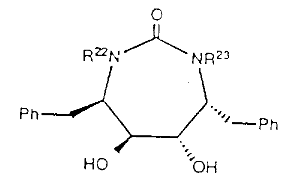 qui est choisi dans le groupe consistant en :
le dérivé de formule (IIa) dans lequel R²² est un allyle et R²³ est un allyle;
le dérivé de formule (IIa) dans lequel R²² est un cyclopropylméthyle et R²³ est un cyclopropylméthyle;
le dérivé de formule (IIa) dans lequel R²² est n-butyle et R²³ est un n-butyle;
le dérivé de formule (IIa) dans lequel R²² est un propyle et R²³ est un propyle:
le dérivé de formule (IIa) dans lequel R²² est un isopentyle et R²³ est un isopentyle;
le dérivé de formule (IIa) dans lequel R²² est un benzyle et R²³ est un benzyle;
le dérivé de formule (IIa) dans lequel R²² est un 3-hydroxybenzyle et R²³ est un 3-hydroxybenzyle;
le dérivé de formule (IIa) dans lequel R²² est un 4-hydroxybenzyle et R²³ est un 4-hydroxybenzyle;
le dérivé de formule (IIa) dans lequel R²² est un 3-hydroxyméthylbenzyle et R²³ est un 3-hydroxyméthylbenzyle;
le dérivé de formule (IIa) dans lequel R²² est un 4-hydroxyméthylbenzyle et R²³ est un 4-hydroxyméthylbenzyle;
le dérivé de formule (IIa) dans lequel R²² est un 3-aminobenzyle et R²³ est un 3-aminobenzyle;
le dérivé de formule (IIa) dans lequel R²² est un 3-carboxylbenzyle et R²³ est un 3-carboxylbenzyle;
le dérivé de formule (IIa) dans lequel R²² est un 3-formybenzyle et R²³ est un 3-formylbenzyle;
le dérivé de formule (IIa) dans lequel R²² est un 3-formaldoximébenzyle et R²³ est un 3-formaldoximébenzyle;
le dérivé de formule (IIa) dans lequel R²² est un 3-(N-méthylamino)benzyle et R²³ est un 3-(N-méthylamino)benzyle;
le dérivé de formule (IIa) dans lequel R²² est un 3-acétylbenzyle et R²³ est un 3-acétylbenzyle;
le dérivé de formule (IIa) dans lequel R²² est un 3-hydroxylaminobenzyle et R²³ est un 3-hydroxylaminobenzyle;
le dérivé de formule (IIa) dans lequel R²² est un 2-naphtylméthyle et R²³ est un 3-hydroxybenzyle;
le dérivé de formule (IIa) dans lequel R²² est un 4-hydroxyméthylbenzyle et R²³ est un 3-hydroxybenzyle;
le dérivé de formule (IIa) dans lequel R²² est un N-méthyl-(3-amido)benzyle et R²³ est un N-méthyl-(3-amido)benzyle;
le dérivé de formule (IIa) dans lequel R²² est un N-méthyl-(3-amido)benzyle et R₂₃ est un 3-(amidino)benzyle;
le dérivé de formule (IIa) dans lequel R²² est un 3-(5-tétrazolyl)benzyle et R²³ est un cyclopropylméthyle;
le dérivé de formule (IIa) dans lequel R²² est un 3-(5-tétrazolyl)benzyle et R²³ est un 3-(5-tétrazolyl)benzyle;
le dérivé de formule (IIa) dans lequel R²² est un phénylméthyl-3-acide boronique et R²³ est un phénylméthyl-3-acide boronique.

11. Un dérivé selon la revendication 1 de formule (IIb): 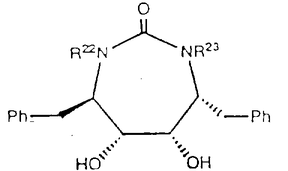 ou un de ses sels ou de ses précurseurs de médicament, pharmaceutiquement acceptables dans laquelle :
R²² et R²³ sont indépendamment choisis dans le groupe consistant en : hydrogène, cyclopropylméthyle, CH₂(C₆H₄)-p-OCH₂C₆H₅, CH₂(C₆H₄)-p-OH, cyclopentylméthyle, allyle, n-butyle, béta-napthylméthyle, benzyle, CH₂(C₆H₄)-m-OCH₂C₆H₅, p-nitrobenzyle, m-nitrobenzyle, CH₂(C₆H₄)-m-OH, CH₂(C₆H₄)-m-(CH₂OH), p-aminobenzyle, m-aminobenzyle, p-nitrilobenzyle, m-nitrilobenzyle, diméthylallyle, cyclohexylméthyle, cyclobutylméthyle, propyle, 3-méthyl-1-butyle, carboxamidobenzyle, et formaldoximébenzyle.

12. Un dérivé selon la revendication 1 de formule (Ib): 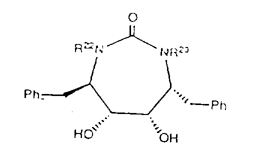 ou de ses sels ou de ses précurseurs de médicaments, pharmaceutiquement acceptables, dans laquelle:
R²² et R²³ sont indépendamment choisis dans le groupe consistant en :
hydrogène, cyclopropylméthyle, CH₂(C₆H₄)-p-OCH₂C₆H₅, CH₂(C₆H₄)-p-OH, cyclopentylméthyle, allyle, n-butyle, béta-napthylméthyle, benzyle, CH₂(C₆H₄)-m-OCH₂C₆H₅, p-nitrobenzyle, m-nitrobenzyle, CH₂(C₆H₄)-m-OH, p-aminobenzyle, m-aminobenzyle, p-nitrilobenzyle, m-nitrilobenzyle, diméthylallyle, cyclohexylméthyle, cyclobutylméthyle, propyle, 3-méthyl-1-butyle, carboxamidobenzyle, et formaldoximébenzyle.

13. Utilisation d'un dérivé selon une quelconque des revendications 1 à 12 pour la préparation d'un médicament destiné au traitement d'infections virales.

14. Composition pharmaceutique comprenant un support pharmaceutiquement acceptable et au moins un dérivé selon l'une quelconque des revendications 1 à 12.
